# EUROPEAN PATENT APPLICATION

(11) **EP 1 215 587 A2**
(43) Date of publication of application: **19.06.2002**
(21) Application number: 01309996.5
(22) Date of filing: 29.11.2001
(51) Int. Cl.: G06F 17/00, C12N 9/78

(54) **Adenosine monophosphate deaminase crystal structure**

(30) Priority: 13.12.2000 GB 0030424
(71) Applicant: Pfizer Limited, Sandwich, Kent CT13 9NJ (GB); PFIZER INC., New York, N.Y. 10017 (US)
(72) Inventor: Bazin, Richard J., c/o Pfizer Global Res. & Dev., Sandwich, Kent CT13 9NJ (GB); McDonald, Graeme A., c/o Pfizer Global Res. & Dev., Sandwich, Kent CT13 9NJ (GB); Phillips, Christopher, c/o Pfizer Global Res.&Dev., Sandwich, Kent CT13 9NJ (GB)
(74) Representative: Hayles, James Richard

(57) **Abstract**

A 2.2 Å crystal structure of rabbit AMP deaminase, an integral enzyme of purine nucleotide interconversion, has been determined, in an unligated state and with an inhibitor bound. The present invention further discloses the use of x-ray crystallographic data for identification and construction of possible therapeutic compounds in the treatment of various disease conditions. The sequence of rabbit AMP deaminase is also disclosed.

## Description

### Field of the Invention

The present invention relates to crystals of adenosine monophosphate deaminase (AMPDA), and more particularly to the high resolution structure of AMPDA obtained by X-ray diffraction. The invention further relates to the use of the three-dimensional structure so determined to identify, design or select compounds that bind to AMPDA or its active site.

### Background to the invention

AMP deaminase (AMPDA; EC 3.5.4.6) is an integral enzyme of purine nucleotide interconversion. The zinc dependent enzyme catalyses the irreversible hydrolysis of 5' adenosine monophosphate (AMP) to form inosine monophosphate (IMP) and ammonia. This reaction represents a branch-point in the energy-generating adenylate catabolic pathway regulating the availability of adenosine nucleotides, including ATP, within the cell. The enzyme is a target e.g. for the treatment of ischemia-related diseases. Blocking the pathway reduces the depletion of the total adenine pool observed in ischemic tissues (Erion, M.D., *et al.* (1999) J. Am. Chem. Soc. 121, 308-319; Skladanowski, A.C. in Myocardial Energy Metabolism (ed. de Jong, J.W.) 53-65 (Dordrecht, 1998)), and thus the metabolically expensive requirement for the *de novo* synthesis of ATP. Patients with congestive heart failure (CHF) have been found to have a significantly longer survival period without heart transplant if they have a genetic deficiency in skeletal muscle AMPDA (Loh, E., et al. (1999) Circulation 99, 1422-1425); similarly, survival in patients with coronary artery disease (CAD) is improved if they have a common variant of the *ampd*1 gene, which encodes a truncated, inactive enzyme (Anderson, J.L. et al (2000) J. Am Coll Cardiol 36, 1248-1252). The therapeutic potential of specific AMPDA inhibitors is not limited to CHF and CAD, but extends to other diseases, including to all ischemia-related diseases. These include peripheral vascular disease and chronic obstructive pulmonary disease. As recently reported, AMPDA inhibitors could also be useful in Alzheimer's disease (Sims, B. et al. (1998) Neurobiol. Aging 19, 385-391).

AMPDA is a diverse, oligomeric, and highly regulated enzyme ubiquitous in eukaryotic cells. Multiple isoforms have been purified and characterised from rat and human tissues, which are encoded by transcripts from a multigene family (Morisaki, T., Sabina, R.L. & Holmes, E.W. (1990) J. Biol. Chem. 265, 11482-11486; Baush-Jurken, M.T., et al (1992) J. Biol. Chem. 267, 22407-22413). Three mammalian genes encoding AMP deaminase have been identified; *ampd*1, which encodes the isoenzyme expressed at high levels in skeletal muscle (M-AMPDA), *ampd*2 and *ampd*3 which are expressed in a wide range of tissues and are called the smooth and cardiac muscle isoenzymes (or L-AMPDA and E-AMPDA) respectively. Sequence analysis demonstrates that these isoenzymes have a highly conserved C-terminal domain and a smaller divergent N-terminal domain. The skeletal enzyme is closely associated with muscle fibers binding the myosin heavy chain and is regulated by a number of factors. ATP acts as an allosteric inhibitor. K⁺ ion concentration modulates the enzyme activity which has also been shown to increase under the mildly acidic (pH6.5) conditions found in exercising muscle (Sabina, R.L. & Mahnke-Zizelman, D.K. (2000) Pharmacol. Ther. 87, 279-283; Ranieri-Raggi, M. & Raggi, A. (1990) Biochem. J. 272, 755-759; Ranieri-Raggi, M. & Raggi, A. (1980) Biochem. J. 189, 367-368; Ranieri-Raggi, M. & Raggi, A. (1979) FEBS Lett. 102, 59-63; Thakkar, J.K., et al (1993) Biochem. J. 290, 335-341; Hisatome, I., et al. (1998) Am J. Physiol. 275, C870-C881).

The purification of the enzyme from rabbit muscle has been reported by Smiley, K.L., Berry, A.J. & Suelter, C.H. ((1967) J. Biol. Chem. 242, 2502-2506). The protein is unstable and on storage the N-terminal domain (7kDa) is removed, an effect that can be reproduced by limited trypsin proteolysis cleaving the enzyme after lysine 95 (Sabina, R.L. & Mahnke-Zizelman, D.K. (2000) Pharmacol. Ther. 87, 279-283). The C-terminal domain retains catalytic activity. However, in the truncated state the enzyme is less tightly regulated and ATP no longer inhibits the enzyme allosterically, indicating that the small N-terminal domain is regulatory and binds ATP. Enzyme has since been produced from a number of sources including chicken (Chilson, O.P., Kelly-Chilson, A.E. & Siegel, N.R. (1997) Comp. Biochem. Physiol. 116B, 371-377) and rat (Coffee, C.J. & Kofke, W.A. (1975), J. Biol. Chem. 250, 6653-6658) skeletal-muscle, while the human enzyme has been produced in a baculovirus expression system (Mahnke-Zizelman, D.K., Tullson, P.C. & Sabina, R.L. (1998) J. Biol. Chem. 273, 35118-35125). Like the rabbit enzyme, each of these proteins is unstable which has led to a range of molecular weights for the enzymes being reported and some debate over the oligomeric make-up of the protein. Trimers have been reported for the truncated form of the enzyme, but controlled limited proteolysis has demonstrated that the enzymes purified from mammalian skeletal muscle are most likely homo-tetramers of the truncated enzyme (Sabina, R.L. & Mahnke-Zizelman, D.K. (2000) Pharmacol. Ther. 87, 279-283).

Despite its early identification and purification, structural information on the enzyme is very limited. The hexagonal bipyramidal crystals obtained by Smiley, Berry & Suelter ((1967) J. Biol. Chem. 242, 2502-2506) are not suitable for X-ray crystallography, and the structure of the enzyme has not been elucidated. Sequence analysis by conventional techniques fails to identify any close homologues; however, an extensive analysis of amidohydrolase sequences using a combination of database searching, conserved functional properties and sequence threading techniques suggests that the enzyme may share the same fold, an ellipsoidal (βα)₈ barrel with a conserved metal binding site, as the urease and adenosine deaminase (ADA) family of proteins (Holm, L. & Sander, C. (1997) Proteins 28, 72-82). In contrast, a second study has proposed that the catalytic C-terminal domain of AMPDA contains two pleckstrin homology (PH) domains, a prediction which is functionally supported by the observation that AMPDA binds phosphoinositides (Sims, B., et al. (1999) J. Biol. Chem. 274, 25701-25707).

Various AMPDA inhibitors have been identified; examples are coformycin (Nakamura H et al (1974) J. Am. Chem. Soc. 96, 4327-8), as well as inhibitors based around coformycin, (Erion, M.D. et al (1999) J. Am. Chem. Soc. 121, 308-319; Bookser, B.C. et al. (2000) J. Med. Chem. 43, 1495-1507), and a series of compounds as exemplified in WO 94/18200, including 3-(2'-(3"-carboxynaphthyl)ethyl)coformycin aglycone (compound 1p), herein called UK-384,858. AMPDA is also inhibited by alkylsulfonate compounds (Yoshino, M. & Murakami, K. (1998) Env Tox Pharmacol 5, 215-217). However, without the structure of the enzyme and detailed knowledge at the atomic level of how the inhibitors bind to the enzyme, the design and optimisation of such compounds is difficult.

### Aspects of the invention

In a broad aspect, the invention relates to novel crystals of AMPDA which are of tetragonal form. Preferred embodiments include crystals
- wherein the AMPDA consists of the catalytic domain,
- wherein the AMPDA is from a mammal,
- wherein the AMPDA is from a rabbit,
- wherein the AMPDA has the sequence as in SEQ ID NO: 2 but starting at position Leu96;
- wherein the crystal has been grown with citric acid as the precipitating agent;
- wherein the crystal has been grown in the pH range of 7.80-8.20;
- wherein the crystal has been grown in the presence of imidazole;
- wherein the crystal has a space group P4₂2₁2;
- wherein the crystal has unit cell dimensions of a=b=149Å +/-3Å, c=159Å +/-3Å, more preferably wherein the crystal has unit cell dimensions of a=b=148.7Å, c=158.6Å,
- wherein there is a dimer of two AMPDA molecules in the asymmetric unit;
- wherein two AMPDA dimers form tightly associated tetramers, each monomer having a (βα)₈ barrel fold, where the intersubunit contacts are almost exclusively made by the helices additional to the (βα)₈ barrel;
- wherein the AMPDA has a Zn²⁺ coordination site involving residues His303, His305, His 572, and Asp649, with a further coordination site contributed by an activated water molecule required for catalysis;
- wherein the active site of AMPDA is contained in a cleft formed by the additional helices between the first and second strand of the (βα)₈ barrel fold, and the helix immediately following the third strand;
- wherein the AMPDA has a pocket which can accommodate the adenosine group of AMP, which pocket is formed by amino acid residues including, but not limited to, residues His305, Phe372, Phe375, Asp513, Glu575, His594, and Asp650;
- wherein the AMPDA has a pocket which can accommodate the ribose and phosphate groups of AMP, which pocket is formed by amino acid residues including, but not limited to, residues His305, Ala306, Ala307, Ala308, Phe375, Asn376, Tyr379, Arg388, Lys393, Ser427, Tyr429, Pro460, Ile462, Val512, and Asp513;
- which diffracts X-rays to 3.5Å, preferably to 2.8Å, 2.5Å, or 2.2Å resolution;
- which has the atomic coordinates set out in Table 2, or a derivative set as expressed in any reference frame.

A preferred embodiment is a heavy atom derivative of the above mentioned crystals; preferably, the heavy atoms are lead, silver or xenon.

Further preferred embodiments are the above mentioned AMPDA crystals with inhibitors soaked in, more preferably wherein the inhibitors are AMPDA transition state analogues, preferably coformycin analogues, even more preferably wherein the inhibitor is coformycin or wherein the inhibitor is UK-384,858. A preferred embodiment of the invention is an AMPDA crystal with coformycin soaked in, having the atomic coordinates as set out in Table 3, or a derivative set as expressed in any reference frame; another preferred embodiment is an AMPDA crystal with UK-384,858 soaked in, having the atomic coordinates as set out in Table 4, or a derivative set as expressed in any reference frame.

A further embodiment of the invention is a crystal of AMPDA wherein the primary sequence of the AMPDA has 90%, preferably 95%, even more preferably 98% identity at amino acid level to the sequence shown in SEQ ID NO:2.

A further embodiment of the invention is the use of atomic coordinates obtained by X-ray diffraction studies of the above mentioned crystals for deriving the three-dimensional structure of AMPDA.

Preferred embodiments of the invention are the use of the three-dimensional structure of AMPDA so determined for computationally or otherwise evaluating the binding interactions of a chemical compound with AMPDA, preferably the active site of AMPDA; and for designing compounds, preferably inhibitors of AMPDA, capable of associating with the enzyme, or preferably with the active site of the enzyme.

A preferred embodiment is a compound evaluated or designed as described above.

Another embodiment of the invention is a method of selecting an AMPDA inhibitor from a group of potential AMPDA inhibitor compounds by creating a three-dimensional representation of the enzyme, preferably the active site cavity of AMPDA, as derived from the three-dimensional structure determined above, in a suitable computer program; displaying and superimposing the model of said test compound on the model of said enzyme, or preferably the active site of it, and assessing whether the test compound model fits the enzyme, or preferably its active site. The method can further comprise incorporating the test compound in a biological AMPDA activity assay, and determining whether the test compound inhibits AMPDA activity in this assay. A compound selected by such method is also an embodiment of the invention.

Further embodiments of the invention are pharmaceutical compositions of any of the compounds evaluated, designed, or selected as described above, as well as their use in the manufacture of a medicament for the treatment of diseases where AMPDA is implicated. These diseases include but are not limited to, ischemia-related diseases such as congestive heart failure, peripheral vascular disease, and chronic obstructive pulmonary disease. The diseases also include coronary artery disease (CAD) and Alzheimer's disease.

Another embodiment of the invention is the use of the atomic coordinates of AMPDA as mentioned above, or portions thereof, to solve a crystal form of a mutant, homologue, or co-complex of AMPDA, e.g. by molecular replacement or difference Fourier analysis, as well as the use of the atomic coordinates to produce a model of the three-dimensional structure of related enzymes.

A further embodiment of the invention is an isolated and/or purified polynucleotide comprising a polynucleotide encoding the polypeptide as set forth in SEQ ID NO: 2; a polynucleotide comprising a nucleotide sequence of SEQ ID NO: 1; a polynucleotide comprising a nucleotide sequence that has at least 91%, preferably 95%, even more preferably 98% identity to the polynucleotide mentioned above; a polynucleotide comprising a nucleotide sequence which is capable of hybridising to the polynucleotide mentioned above under high stringency conditions (washing with 0.1x SSC, 0.1% SDS at 65°C); a complement to any of the polynucleotide mentioned above, or a polynucleotide fragment of any of these polynucleotides.

A further embodiment of the invention is a polypeptide having the deduced amino acid sequence translated from the polynucleotide sequence in SEQ ID NO: 1 and variants, fragments, homologues, analogues and derivatives thereof; or a polypeptide of SEQ ID NO: 2 and variants, fragments, homologues, analogues and derivatives thereof. A preferred embodiment is a polypeptide of SEQ ID NO: 2 but starting at position Leu96.

### Definitions

AMPDA: AMP deaminase (E.C. 3.5.4.6) - catalyzes the deamination of AMP to IMP as part of the purine catabolic pathway.

AMPDA catalytic domain: the C-terminal part of the enzyme, sufficient for catalytic activity (usually lacking the N-terminal 80-100 residues).

M-AMPDA, L-AMPDA and E-AMPDA isoenzymes: Products of different genes (*ampd*1, *ampd*2 and *ampd*3), but all encoding AMP deaminase, i.e. the same activity; the isoenzymes are differentially expressed in different tissues.

Polynucleotide: DNA or RNA in isolated form, made by recombinant or synthetic routes.

Polypeptide: interchangeable with the term protein, includes single-chain polypeptides as well as complexes of single-chain polypeptides linked by covalent or non-covalent means.

The terms "variant", "homologue", "fragment", "analogue" or "derivative" in relation to the amino acid sequence for the polypeptide of the present invention include any substitution of, variation of, modification of, replacement of, deletion of or addition of one (or more) amino acid from or to the sequence providing the resultant polypeptide retains 90%, preferably 95%, even more preferably 98% sequence identity to the sequence shown in SEQ ID No:2. Sequence identity is determined by standard bioinformatics software tools such as Blast2 or Fasta, which generate an optimal alignment between two sequences and then calculate the % identity on this basis, using default parameters.

Mutant of AMPDA refers to AMPDA where changes in one or more amino acid residues have been introduced artificially by processes like site-directed mutagenesis, or where deletions or insertions have been introduced into the AMPDA sequence, whether or not the resulting proteins retain enzyme activity. It also refers to naturally occurring variants of AMPDA, whether or not they retain enzyme activity.

Related protein refers to a protein the structure of which has not yet been determined, which displays significant sequence similarity (>30% sequence identity at protein level), or a significantly large portion of such a protein which would retain some of the structural features of the protein, or such a protein which does not display any similarity at the sequence level, but has a similar (βα)₈ fold as AMPDA.

For human use, the compounds of the invention, and their pharmaceutically acceptable salts, can be administered alone but will generally be administered in admixture with a suitable pharmaceutical excipient, diluent or carrier selected with regard to the intended route of administration and standard pharmaceutical practice.

For example, the compounds, and their pharmaceutically acceptable salts, can be administered orally, buccally or sublingually in the form of tablets, capsules, ovules, elixirs, solutions or suspensions, which may contain flavouring or colouring agents, for immediate-, delayed-, modified-, sustained-, pulsed- or controlled-release applications.

The compounds can also be administered parenterally, for example, intravenously, intra-arterially, intraperitoneally, intrathecally, intraventricularly, intraurethrally, intrasternally, intracranially, intramuscularly or subcutaneously, or they may be administered by infusion or needleless injection techniques. For such parenteral administration they are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

### Examples

The invention will now be illustrated by the following non-limiting examples and accompanying figures, in which:
**SEQ ID NO: 1** illustrates the rabbit AMPDA cDNA sequence;
**SEQ ID NO: 2** illustrates the rabbit AMPDA protein sequence;
**SEQ ID NOs: 3-6** give the primer sequences used for amplification of the rabbit AMPDA coding sequence;

**Figure 1** illustrates the monomer structure of AMPDA; coloured by greyscale from the N-terminus to the C-terminus (dark to light). Helices and sheets additional to the core (βα)₈ barrel region are labelled. The zinc is depicted as a grey sphere.
**Figure 2** illustrates the tetramer structure of AMPDA;
**Figure 3** shows the structures of coformycin (A) and UK-384,858 (B); atom positions are numbered for the seven membered ring system of coformycin;
**Figure 4** shows the structure of the AMPDA-coformycin complex;
**Figure 5** shows the structure of the AMPDA-UK-384,858 complex; and
**Figure 6** shows the transition state model.

### Example 1 - Cloning of rabbit AMPDA

A λgt10 rabbit chest muscle cDNA library was purchased (Clontech TL3000a) and used as the source of cDNA for the amplification of rabbit AMPDA by polymerase chain reaction (PCR). Four primers were designed using the human skeletal muscle AMPDA sequence (Sabina R.L. et al (1992) Neurology 42, 170-179); two of these primers, AMPDA1 and AMPDA2, were complementary to the 5' and 3' ends of the coding sequence of human AMPDA, respectively; primers AMPDA3 and AMPDA4 were internal primers, designed to enable the sequence to be amplified in two halves. Primers AMPDA1 and AMPDA4 were used to amplify the N-terminal portion, and AMPDA2 and AMPDA3 to amplify the C-terminal portion of the coding sequence:

AMPDA-1 5'-ATGCCTCTGTTCAAACTCCC-3' (SEQ ID NO: 3)

AMPDA-2 5'-TTCTGTTGATTTAAGACCCTC-3' (SEQ ID NO: 4)

AMPDA-3  5'-ATGAACCAGAAACATCTGCTG-3'  (SEQ ID NO: 5)

AMPDA-4 5'-CAGCAGATGTTTCTGGTTCATG-3' (SEQ ID NO: 6)

Amplification was carried out using the above mentioned cDNA library as template, Taq polymerase (Life Technologies) and a MasterAmp™ PCR optimisation kit from Epicentre Technologies (Cambio). The amplification products were TA-cloned into pCRII (Invitrogen) as per manufacturer's protocol, and the DNA sequence determined by Lark Technologies (Saffron Walden, UK).

The rabbit cDNA sequence and the resulting translation product are shown in SEQ ID NOs: 1 and 2.

### Example 2 - Purification of rabbit AMPDA

Purification of rabbit AMPDA was carried out following a procedure modified from Smiley, K.L., Berry, A.J. & Suelter, C.H. ((1967) J. Biol. Chem. 242, 2502-2506). Frozen rabbit muscle was obtained from Pel-Freez Biologicals Inc., Rogers, Arkansas.

To one kilogram of thawed muscle was added 3 times its weight of extraction buffer composed of 0.18 M potassium chloride (KCl), 0.054 M potassium dihydrogen phosphate (KH₂PO₄), 0.035 M potassium hydrogen phosphate (K₂HPO₄) pH 6.50. After homogenisation for 30 seconds in a Waring blender, the homogenate was centrifuged at 14,000 x g for 15 minutes, the resultant pellets being discarded. The supernatant fraction was decanted through synthetic cheese cloth to remove lipid particles prior to cellulose phosphate chromatography.

10 g of cellulose phosphate (Whatman) was prepared according to the manufacturer's instructions. The prepared cellulose phosphate was added directly to the supernatant fraction and stirred at room temperature. After one hour the cellulose phosphate was recovered by means of a sintered glass suction funnel, the filtrate being discarded. The cellulose phosphate was transferred to an XK 50/20 column and washed with 0.45 M potassium chloride, pH 7.0, 5 mM β-mercaptoethanol. When a stable UV absorbance was achieved, the column was eluted with 1.0 M KCl, pH 7.0, 5 mM β-mercaptoethanol. The eluted protein was stored at 4°C for at least two weeks. During this period a mass loss of aproximately 10kDa was observed from the N-terminus. The new N-terminus, determined by N-terminal sequencing and verified by mass spectroscopy, begins at Leu96, indicating that the N-terminal domain had been lost. This protein was then purified to homogeneity by means of gel filtration chromatography. A Superdex 200 HiLoad 26/60 column (Pharmacia) was equilibrated with 20 mM Tris-hydrochloride, pH 7.00, 10% glycerol, 200 mM sodium chloride, 5 mM β-mercaptoethanol. Several chromatography runs were performed, loading a maximum of 13ml protein solution per run. Eluted fractions were analysed by 4-20% gradient sodium dodecyl sulphate polyacrylamide gel electrophoresis (SDS-PAGE). Those containing a single protein band, of molecular weight c. 70 kDa, were combined and concentrated.

### Example 3 - Crystallisation of rabbit AMPDA

For crystallisation trials the purified protein was concentrated to 15 mg/ml using Ultra-free Tangental membranes (Millipore) with a molecular weight cut-off of 30kDa. Crystallisation was performed, at 20°C, using the vapour-diffusion technique, with drops containing 2 µl of protein and 2 µl of reservoir solution. A number of different crystal morphologies grew in similar conditions, the best diffracting crystal form, tetragonal rods, were obtained using 1.0 M citric acid (tri-sodium salt, dihydrate) as precipitant and 0.10 M imidazole, in the range pH 7.80 - 8.20, as buffer. The crystals belong to space group P4₂2₁2 with cell dimensions of a=148.7Å, c=158.6Å, and have a dimer in the asymmetric unit.

### Example 4 - Structure Solution

Native data, to 2.2Å resolution, were collected at station 9.6 of the S.R.S. Daresbury, from crystals frozen at liquid nitrogen temperatures in a cryo-protecting solution comprising of mother liquor plus 20% glycerol. The structure was solved by multiple isomorphous replacement with anomalous scattering using three heavy atom derivatives. Table 1 details data collection and heavy atom statistics; the HKL package (Otwinowski, Z & Minor, W (1997) Methods Enzymol 276, 307-326), and the CCP4 (Acta Crystallogr D 50 (1994) 760-763) software suite were used for data processing and analysis. Heavy atom positions were derived from difference Patterson and difference Fourier calculations. The SHARP program (Fortelle E.d.-L. & Bricogne, G. (1997) Methods Enzymol 276, 472-494) was used to refine the heavy atom parameters and obtain initial phases, further heavy atom sites were identified from the residual map analysis available in SHARP. The maps were solvent flattened using SOLOMON (Abrahams, J.P. & Leslie, A.G.W. (1996) Acta Crystallogr. D52, 30-42) and 2-fold averaged with the DM program (Cowtan, K. (1994) Joint CCP4 and ESF-EACBM Newsletter on Protein Crystallography 31, 34-38).

The initial model of AMPDA was traced using the QUANTA package (Molecular Simulations Inc) and refined using all data to 2.2Å with X-PLOR (Brünger, A.T. (1993) X-PLOR Version 3.1 A system for X-ray crystallography and NMR, Yale University Press, New Haven, CT). The torsion angle refinement procedure was used to escape initial local minima. Strict NCS constraints were applied through several cycles of model building and refinement. Individual B-factors were refined. The current model has a R_{cryst} = 21.2% and a R_{free} = 25.4%, calculated on 5% of the data set (Brünger, A.T. (1992) Nature 355, 472-475).

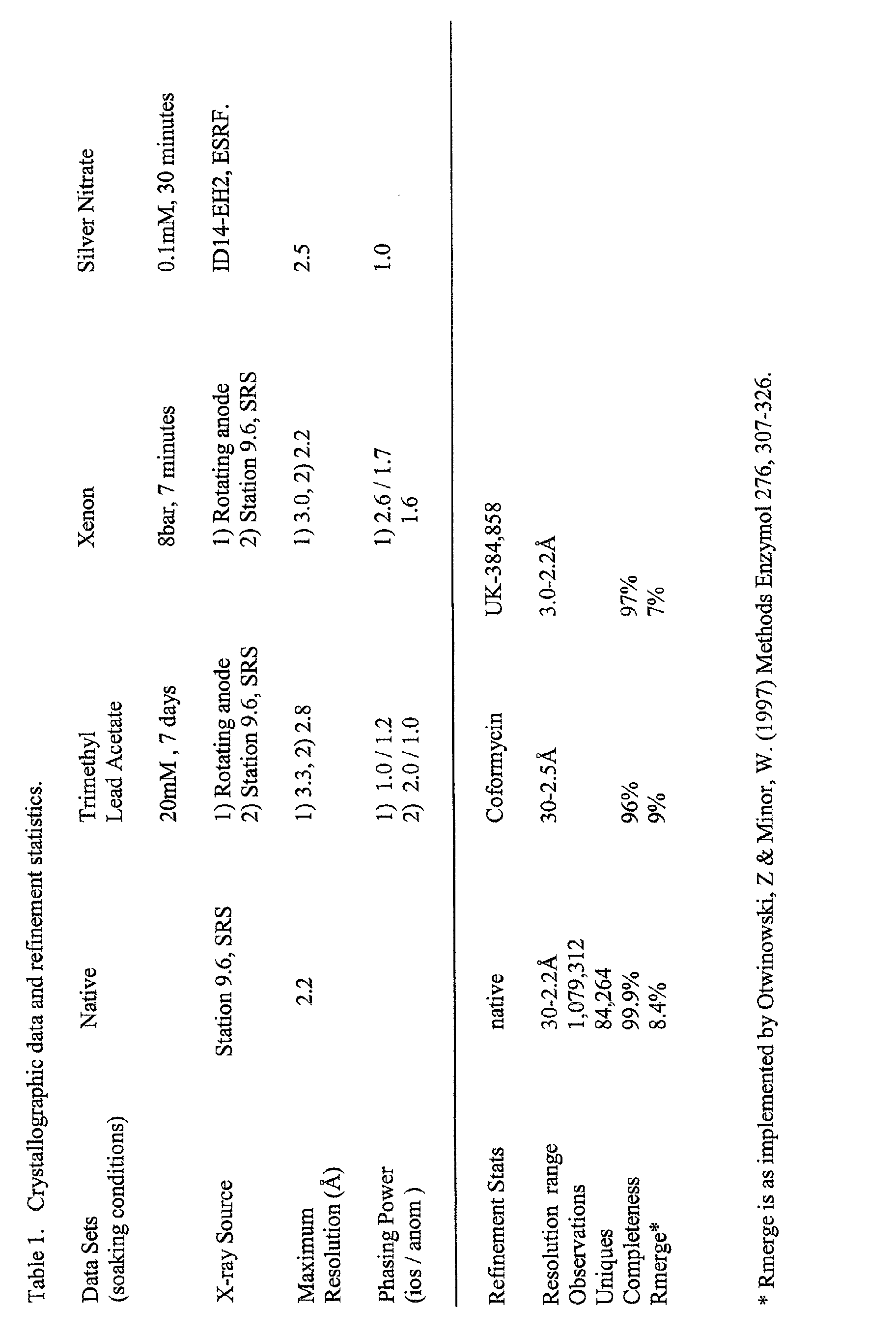

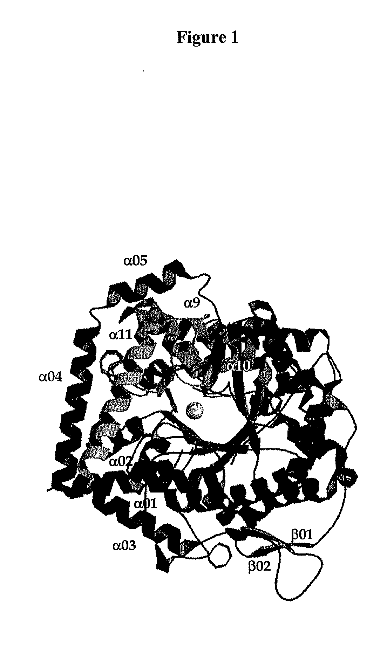

The monomer structure can be described as follows. The catalytic domain of AMPDA is constructed around a core comprising the well precedented (βα)₈ barrel fold (Bränden, C.-I. (1991) Curr. Op. Struc. Biol. 1, 978-983). N-terminal to this barrel are five helices, a large loop and two short anti-parallel strands. These features wrap around the barrel forming an outer layer to the domain. Several additional secondary structural features are inserted between the secondary structural elements that comprise the barrel. Three helices are inserted in the loop between the first strand and helix of the barrel, and two short helices are inserted immediately after the barrel's third strand and third helix respectively. C-terminal to the (βα)₈ motif are three further helices, the first lies across the NH₂-terminal of the β-barrel, the second is short and packs anti-parallel to the first, while the third makes extensive contacts with the additional N-terminal helices. In total the enzyme contains 21 helices and 10 strands. By precedent the helices and strands of the (βα)₈―barrel are identified α1 through α8 and β1 through β8. The additional N-terminal helices and strands are labelled α01 through α05, β01 and β02 respectively. Helices inserted within the barrel-fold are termed α1'1 through α1'3, α3' and α4' to indicate their position in the fold, while the C-terminal helices are referred to as α9 through α11 (The nomenclature scheme is adapted from that of Wilson, D.K., Rudolph, F.B. & Quiocho, F.A. ((1991) Science 252, 1278-1284). A Zn²⁺ ion is bound at the C-terminus of the barrel at the bottom of a large cleft in the enzyme surface. The sides of this cleft are formed by the additional helices between the first and second strands of the barrel and the helix immediately following the third strand. This cleft contains the enzyme's active site. The structure is depicted in figure 1.

The Zn²⁺ co-ordination geometry best fits the trigonal bipyramid class (Alberts, I.L., Nadassy, K. & Wodak, S.J. (1998) Protein Science 7, 1700-1716), involving four residues, three histidines (His303, 305 and 572) and Asp649. The remaining coordination site can be accounted for as a discrete density peak about 1.8Å from the Zn²⁺ was observed throughout refinement and was modelled as the activated water molecule required for catalysis.

Four disordered regions have not been modelled in the apo-structure. Density is not observed for the N-terminal 10 residues, a six residue loop immediately prior to the first helix cannot be modelled, 11 residues forming a loop close to the active site are disordered and the two C-terminal residues are absent.

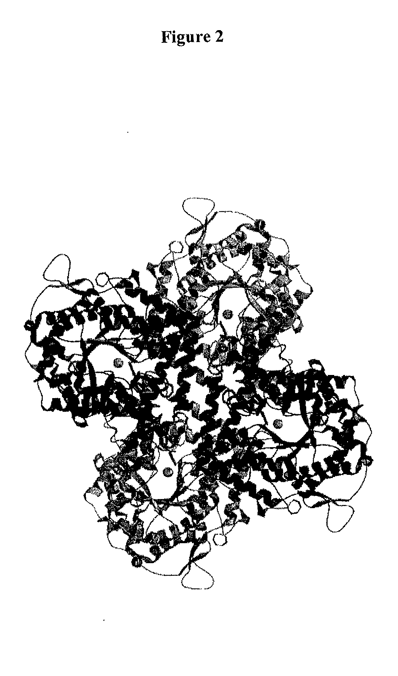

A tetramer is observed in the crystals (figure 2). Two sub-units are packed around a non-crystallographic diad axis forming the asymmetric unit. This dimer is packed around the crystallographic 2-fold axis in z to give the physiologically observed tetramer. An extensive buried surface indicates that the tetramer is tightly associated, and inter-subunit contacts are made almost exclusively by the helices additional to the (βα)₈ barrel.

The atomic coordinates are given in Table 2.

### Example 5 - Soaking of AMPDA with inhibitors and structure solution of enzyme-inhibitor complexes

The coformycin and UK-384,858 enzyme inhibitor complexes were prepared by soaking the crystals in a solution corresponding to the well solution plus 10mM inhibitor. The crystals were shown to tolerate DMSO concentrations of 10% and higher, allowing inhibitors with a relatively low solubility, such as UK-384,858, to be used. Data were collected as described above, the inhibitor structures were modelled into initial difference maps, using the QUANTA software, and have been refined in X-PLOR to give R_{cryst}=20.7% (R_{free}=26.1%), and R_{cryst} =22.9% (R_{free}=25.8%) respectively. All three structures presented have good stereochemistry as judged by the PROCHECK program (Laskowski, R.A. et al (1993) J. Appl. Crystallogr. 26, 283-291). Figures were prepared using the software packages MOLSCRIPT (Kraulis, P.J. (1991) J. Appl. Crystallogr. 24, 946-950) and RASTER3D (Merrit, E.A. & Murphy, M.E.P. (1994) Acta Crystallogr. D50, 869-873).

### a) The complex with Coformycin

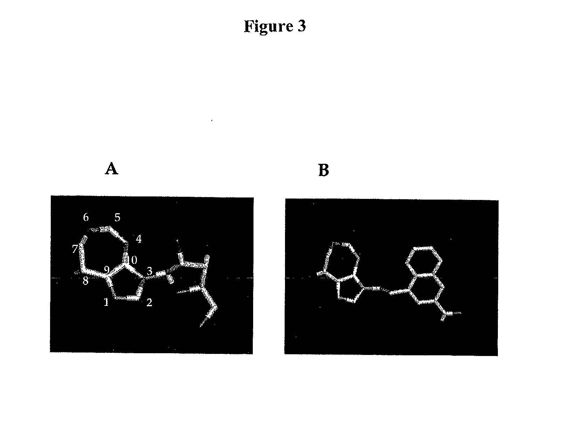

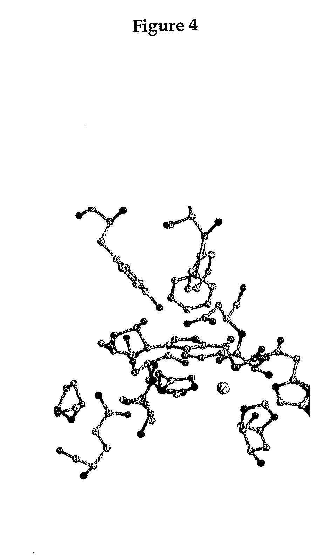

The structure of the enzyme complexed with coformycin (the structure of which is shown in figure 3) at 2.5Å resolution shows the inhibitor bound at the base of the active site cleft. The seven-membered ring binds in a pocket which can accommodate the substrate's adenosine group. The 8-hydroxyl displaces the activated water seen in the apo-structure and hydrogen bonds the Zn²⁺. The hydroxyl to Zn²⁺ distance is 1.8Å. The tetrahedral geometry observed at the C8 position is consistent with coformycin being a transition-state analogue inhibitor of the enzyme. The 11 residues (366-377), disordered in the apo-structure, pack against the bicyclic ring system forming a loop and short (three turn) helix motif, termed the 370 helix. The 370 helix is amphipathic and the hydrophobic face contains two phenylalanine residues (Phe372 and Phe375) which pack against the inhibitor entirely enclosing this portion of the binding site from solvent. The stacking interactions made between the inhibitor ring system and these aromatic residues stabilise the disordered loop. Key inhibitor recognition features include two hydrogen bonds made by acidic residues in the binding site. Asp650 forms a hydrogen bond with N1 of coformycin (D650 Oδ1 - N1, 2.3Å) and is co-planar with the ring, while Glu575 is 2.9Å from N6 and has good geometry to accept a proton. The main chain amide group of Asp513 is positioned to act as a hydrogen bond donating group with the N4 atom of the inhibitor as the acceptor, the distance between these two atoms is however too large (4.4Å) for a good hydrogen bond to be made. Additional residues involved in van der Waals interactions in the purine binding pocket include the Zn²⁺ co-ordinating residues His305 and His594. The bicyclic ring system of the inhibitor packs against the plane of these histidine residues and is essentially sandwiched between these aromatic side chains and the two ordered phenylalanine residues of the 370 helix (figure 4).

The ribose binding site is less tightly defined. Two hydrogen bonds are made by hydroxyl groups, the 2'-OH is 3.0Å from Asn376 Nδ2 and the 5'-OH group is 3.2Å from Gln458 Nδ1. The orientation of Gln458 is fixed in a position to form this hydrogen bond by a hydrogen bonding network through His305 to the catalytic zinc. van der Waals interactions are made by the ribose ring to four hydrophobic side-chains, namely Pro460, Val512, Phe375 and Tyr379 (figure 4). The location of the phosphate binding site can be predicted from the structure of the complex with coformycin, the basic residues Lys393 and Arg388 are ideally positioned to co-ordinate a phosphate group as are the main-chain NH groups of the flexible loop of alanine residues 306-308.

The atomic coordinates are shown in Table 3.

### b) The complex with UK-384,858

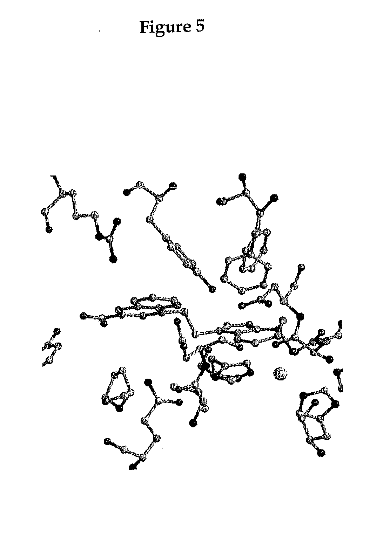

A number of AMPDA specific inhibitors, based around coformycin, have been reported (Erion, M.D. et al (1999) J. Am. Chem. Soc. 121, 308-319; Bookser, B.C. et al. (2000) J. Med. Chem. 43, 1495-1507). UK-384,858 (figure 3) is a potent of example of these inhibitors in which the ribose unit of coformycin has been replaced by an ethyl linked naphthalene group with a carbocylic acid substitutent. The crystal structure of the enzyme inhibitor complex has been solved and refined at 2.2Å resolution. The complex is consistent with that observed with coformycin; the ordering of the active site, tetrahedral transition state geometry binding of the Zn²⁺, and all previously described interactions in the purine binding site are observed. The naphthalene group is seen to pack in a highly hydrophobic environment making van der Waals interactions with residues Pro460, Ile462, Phe372 and the Cα atom of Asp513. The ethyl linker group is also involved in hydrophobic contacts with Phe375. The acidic group is shown to be within hydrogen bonding distance of Tyr429, Ser427 and Arg388 (figure 5).

The atomic coordinates for this structure are shown in Table 4.

### Example 6 - Homology with adenosine deaminase: mechanism conservation

Despite having diverse sequences, AMPDA shares a conserved catalytic mechanism with adenosine deaminase (ADA). ADA is a monomeric, 40 kDalton, protein which catalyses the hydrolysis of adenosine to inosine (Wilson, D.K., Rudolph, F.B. & Quiocho, F.A. (1991) Science 252, 1278-1284). In common with AMPDA, ADA co-ordinates a catalytic zinc atom within a (βα)₈ barrel. The inserted loop, containing 3 helices, between the first stand and helix of the barrel is common to both enzymes and forms part of a highly conserved purine binding site (Sharff, A.J. et al (1992) J. Mol. Biol. 226, 917-921).

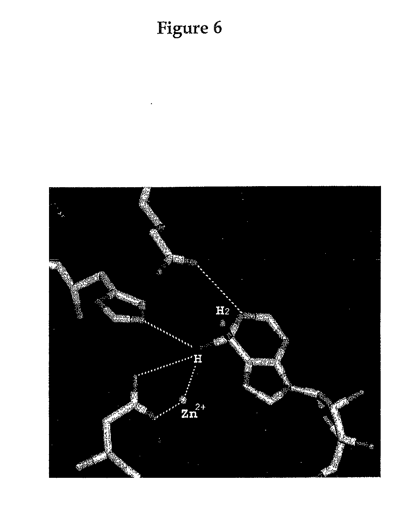

The mechanism of ADA has been examined extensively by a combination of crystallographic (Wang, Z. & Quiocho, F.A. (1998) Biochemistry 37, 8314-8324) and site-directed mutagenesis studies (Sideraki, V. et al (1996) Biochemistry 35, 15019-15028). All the residues identified as being important for the mechanism of ADA are equivalent in AMPDA and it is clear that the mechanism has been conserved in evolution. The reaction has been described in two stages: an initial stereo-specific hydroxide addition to form a tetrahedral (at the substrate C6 position) transition-state intermediate, and a final ammonia elimination to form the inosine product. The Zn²⁺ acts as a powerful electrophile, as demonstrated by the presence of the hydroxide ion (activated water) in the apo-AMPDA structure and the analogous observation reported in an ADA inhibitor complex structure (Wilson, D.K. & Quiocho, F.A. (1993) Biochemistry 32, 1689-1694). The transition state is stabilised by the Glu575 - substrate N6 interaction. His238 in ADA, which is equivalent to His594 in AMPDA, has been shown to promote hydroxide formation, while Asp649 is positioned to orientate the hydroxide oxygen in line for addition to C6 (figure 6). The position of the Zn²⁺ ion and Asp649 dictate the pro-S stereo-specificity of the reaction.

The second stage, the elimination of ammonia, is less well understood. Two pathways have been postulated, either Asp649 or Glu575 acts as the shuttle for the proton from the 6-OH to the 6-NH₂ of the tetrahedral intermediate (Wilson, D.K. & Quiocho, F.A. (1993) Biochemistry 32, 1689-1694).

### Example 7 - Assay for AMPDA activity

There are many assays for AMPDA activity in the prior art (e.g. as in Smiley, K.L., Berry, A.J. & Suelter, C.H. (1967) J. Biol. Chem. 242, 2502-2506). Typically, the assay was performed in microtitre plates. To each well, 10 µl of test compound, diluted in sodium citrate buffer (10mM, pH 6.5) with 10% DMSO and 70 µl of sodium citrate buffer were added; the plate was mixed with a plate shaker. Then 10 µl of enzyme stock solution in 1M KCl were added, and the plate was mixed again, and incubated for 5 minutes. This was followed by the addition of 10 µl substrate solution (10 mM 5'-AMP in sodium citrate buffer). After mixing and 5 minutes incubation, 150 µl of Ninhydrin reagent (Sigma, N1632) were added to each well to stop the reaction. The plate was then covered and left overnight for the colour to develop. The absorbance at 570 nm was measured with an appropriate standard plate reader (e.g. Molecular Devices Spectramax Plus).

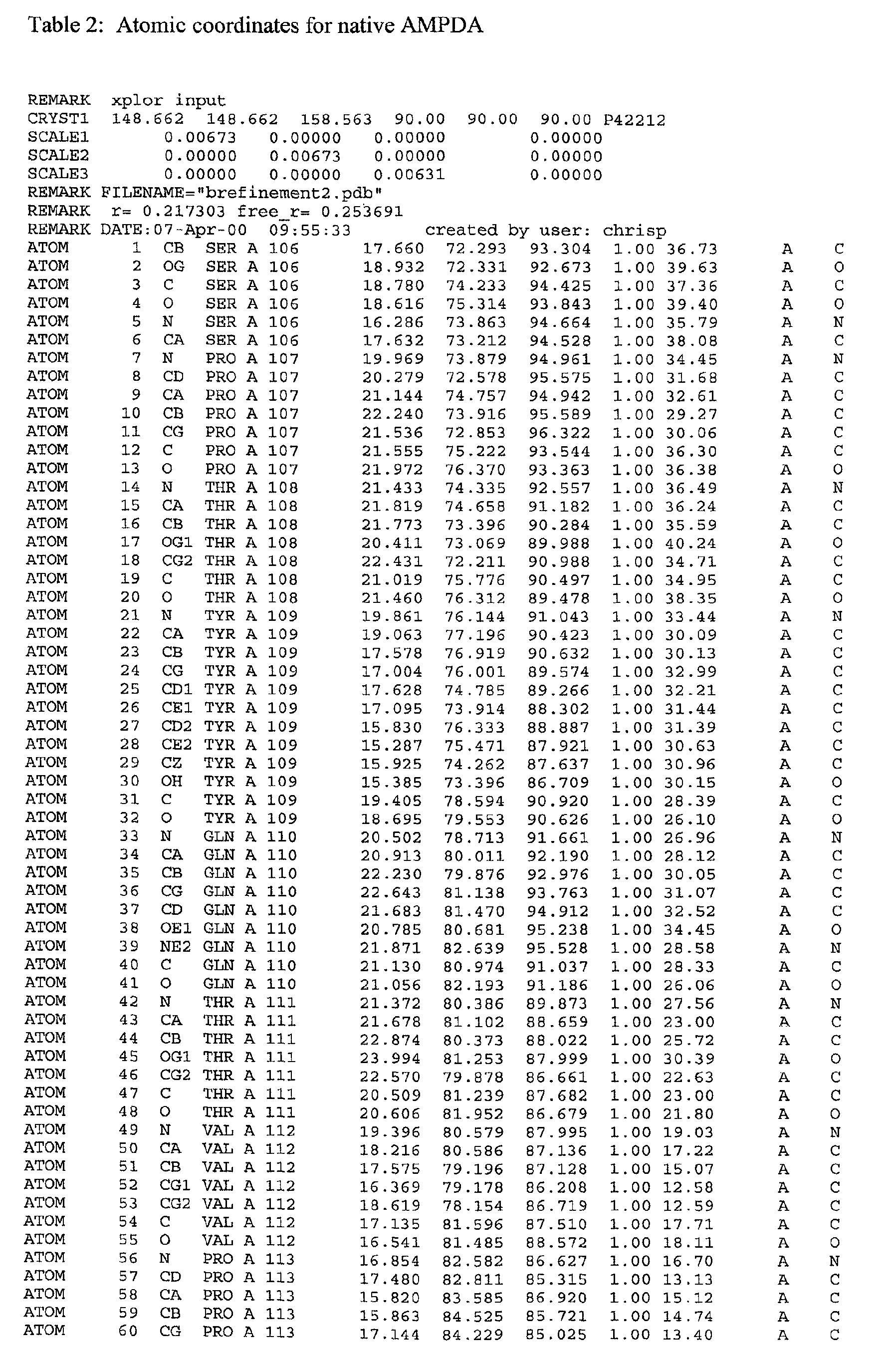

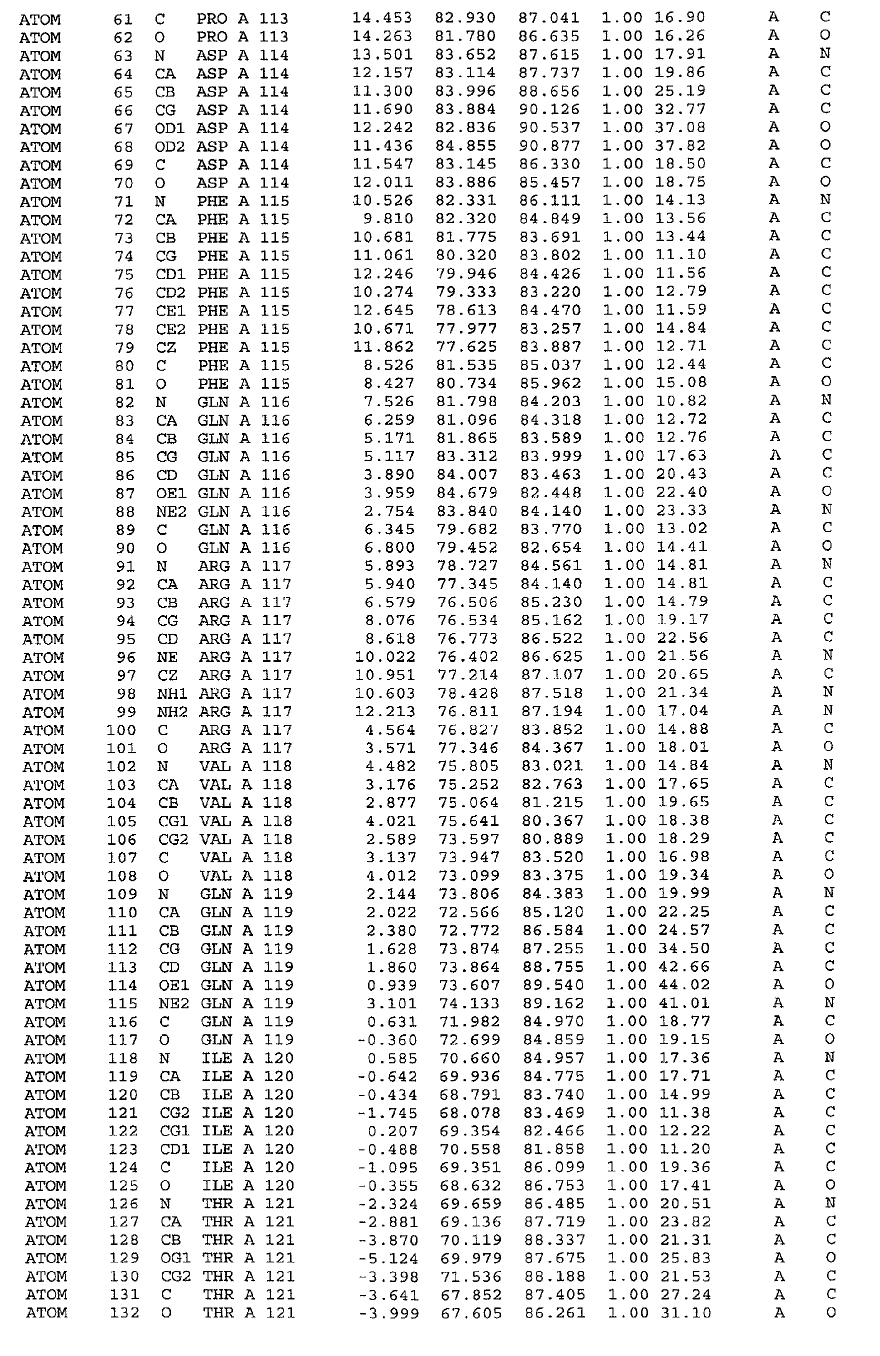

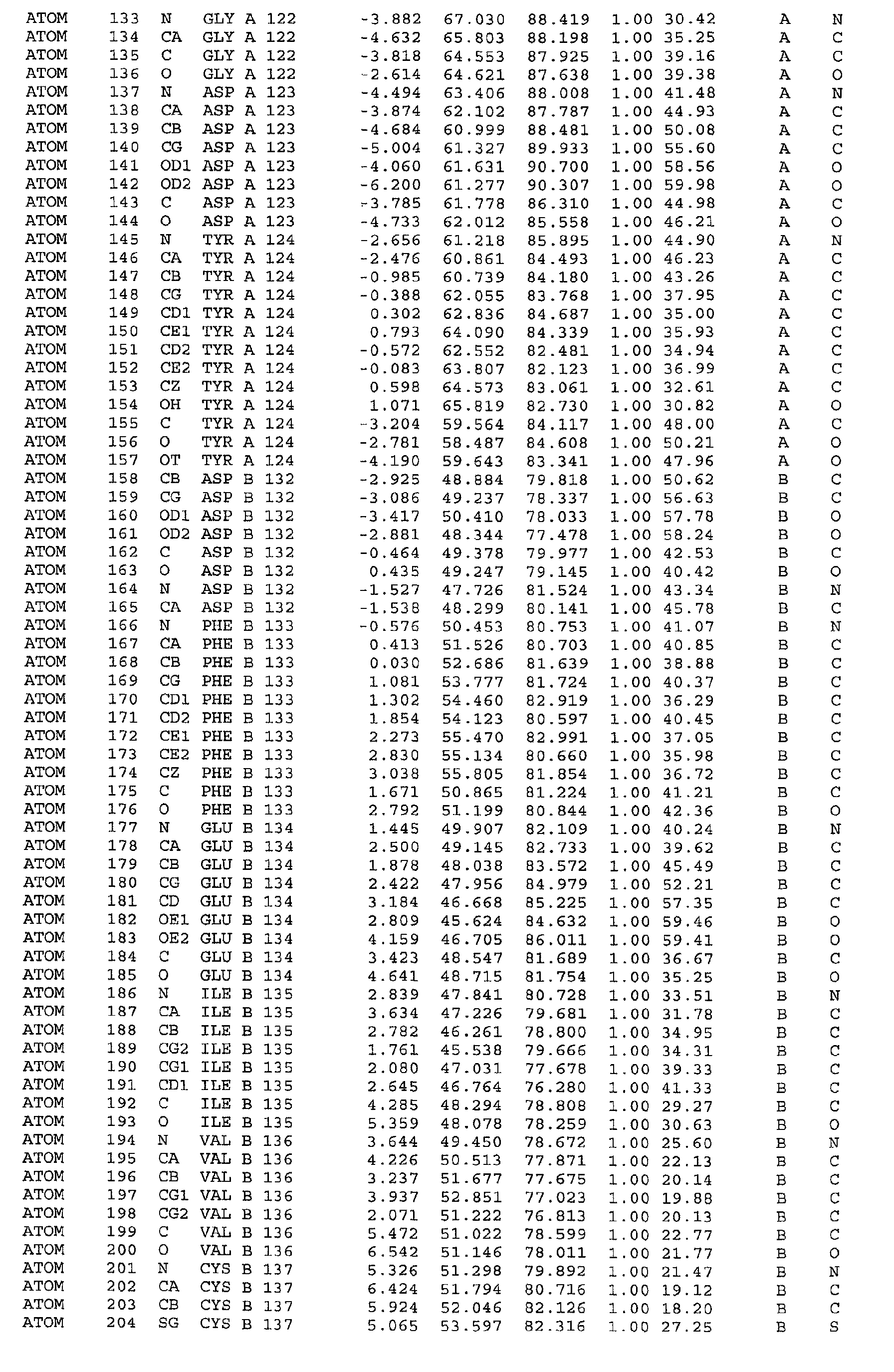

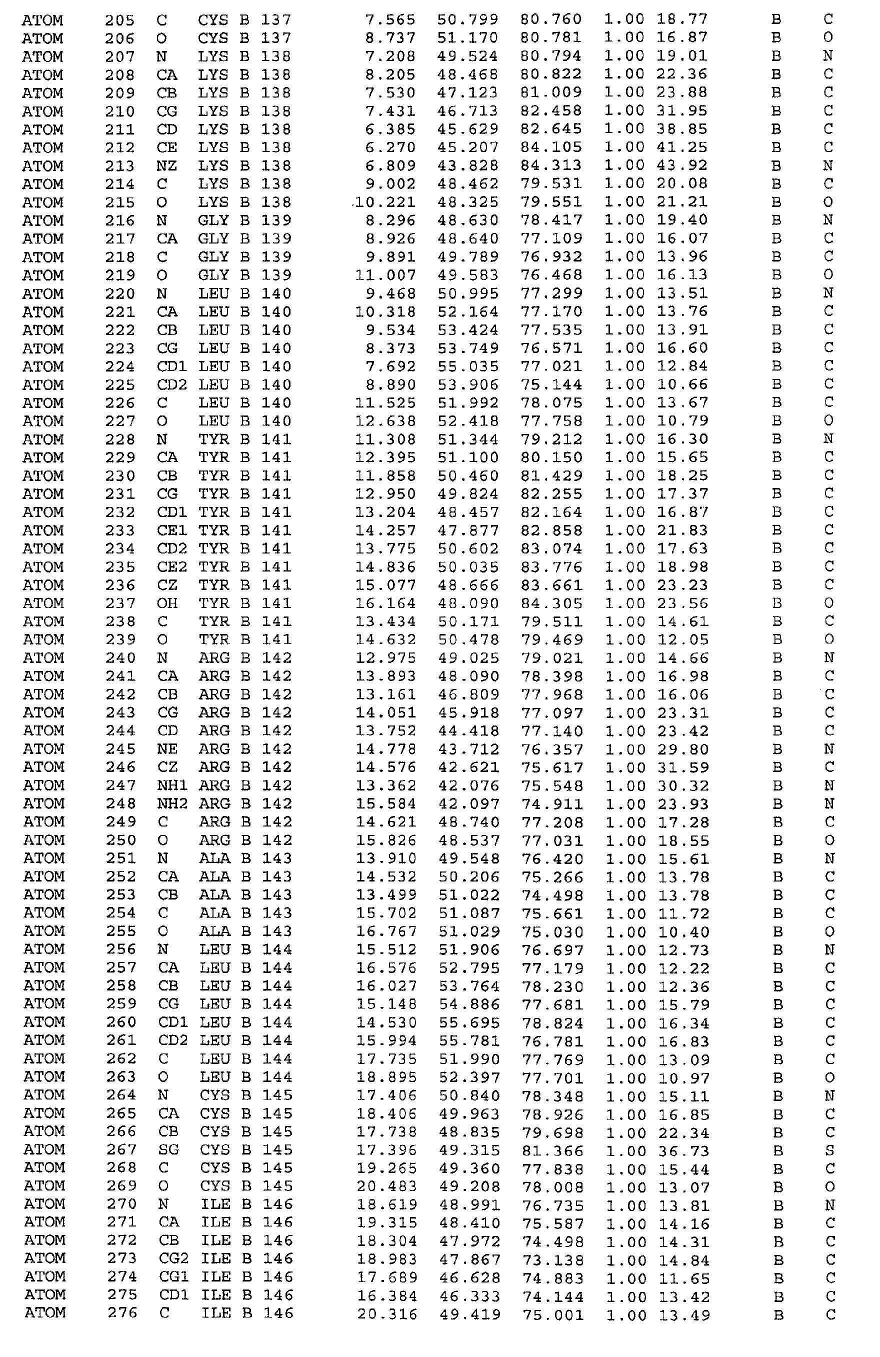

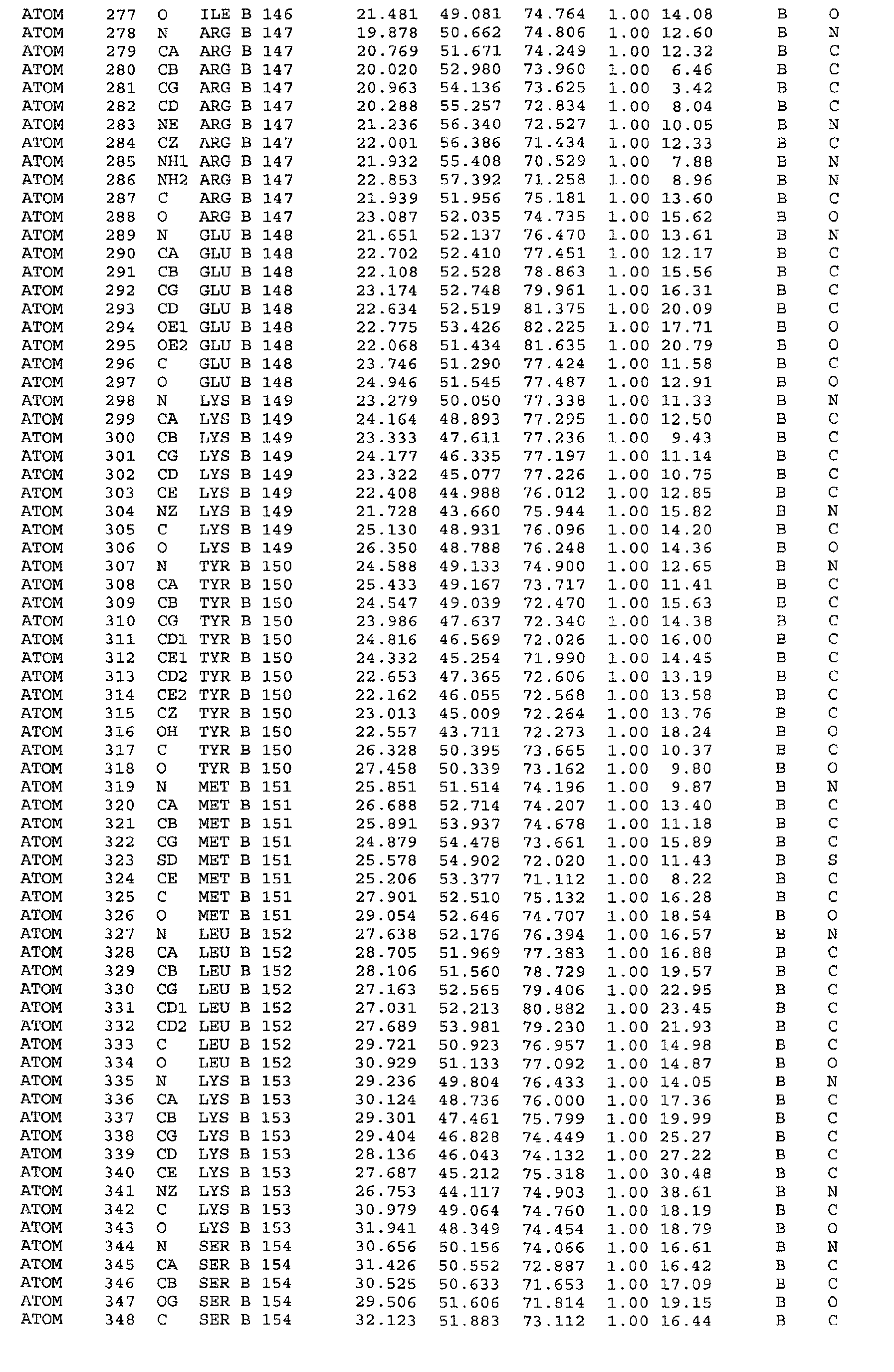

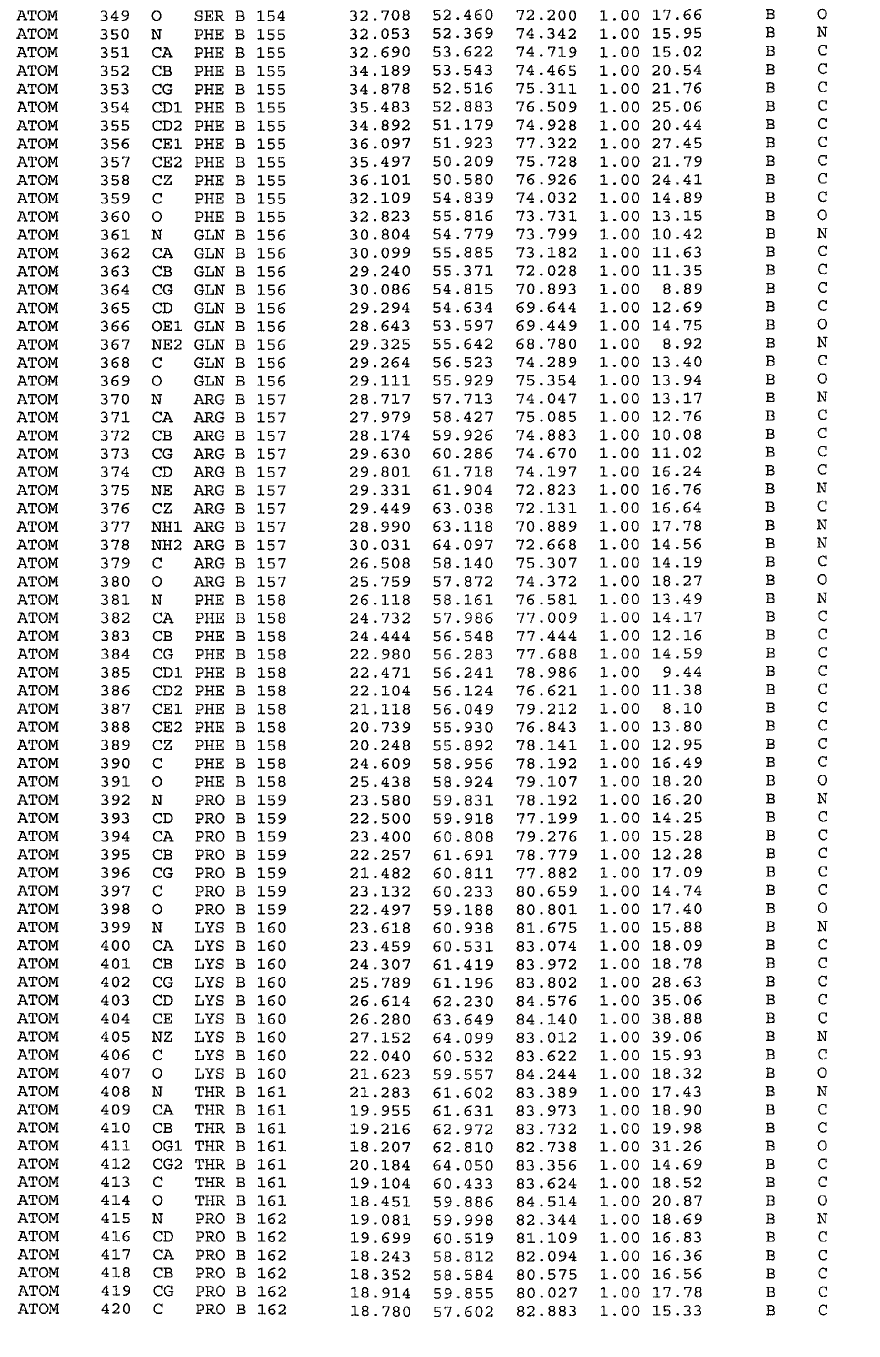

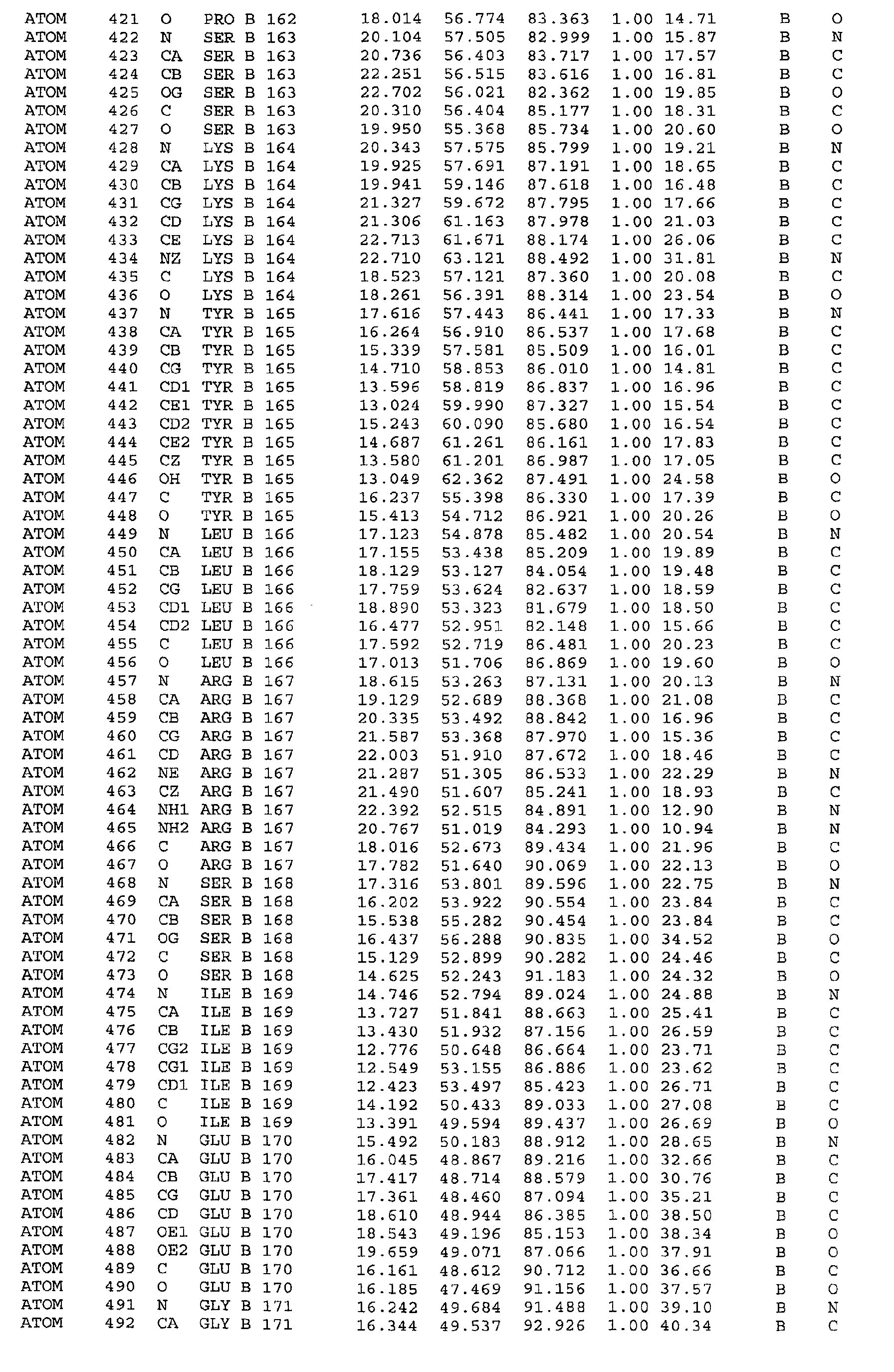

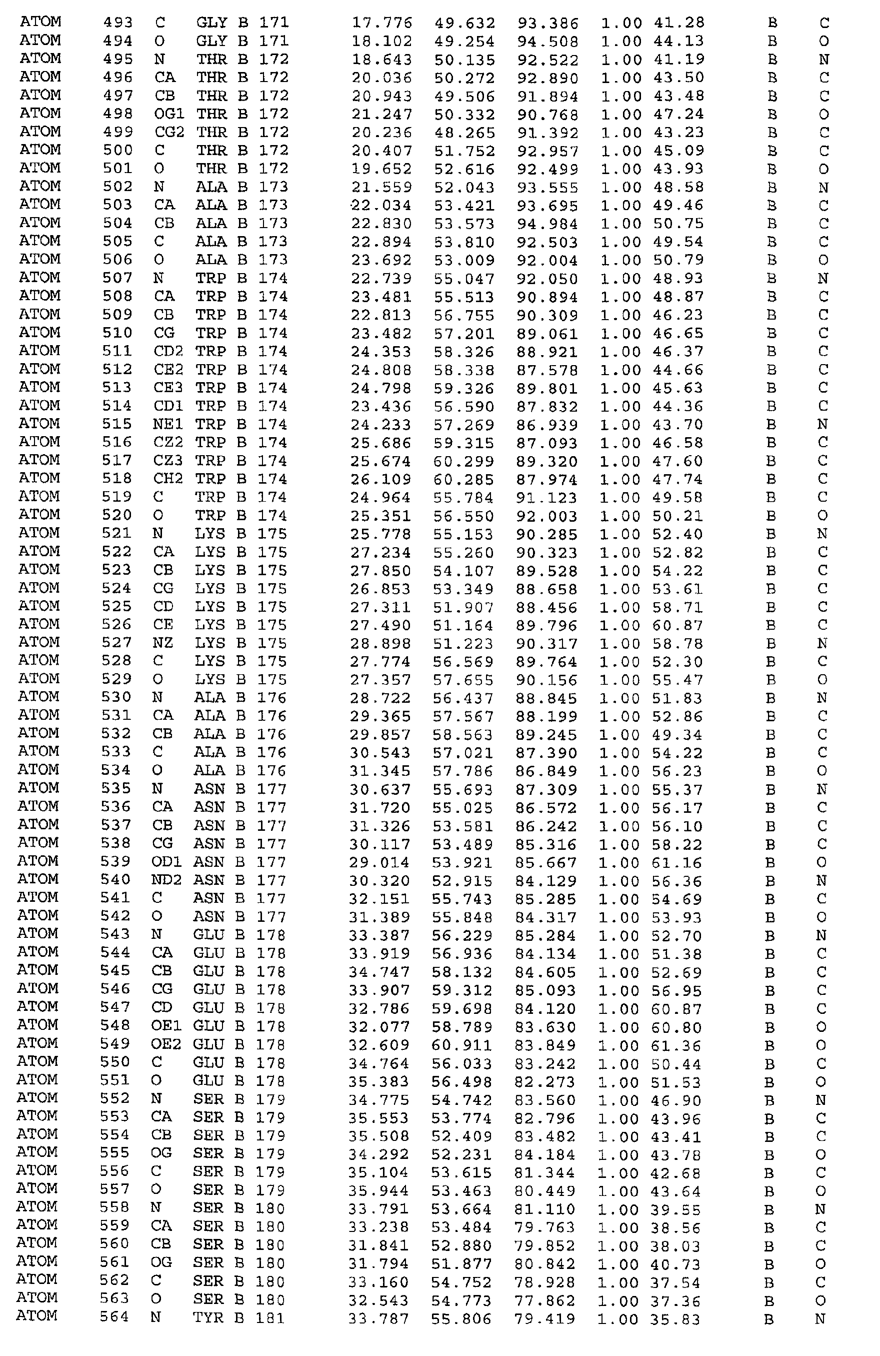

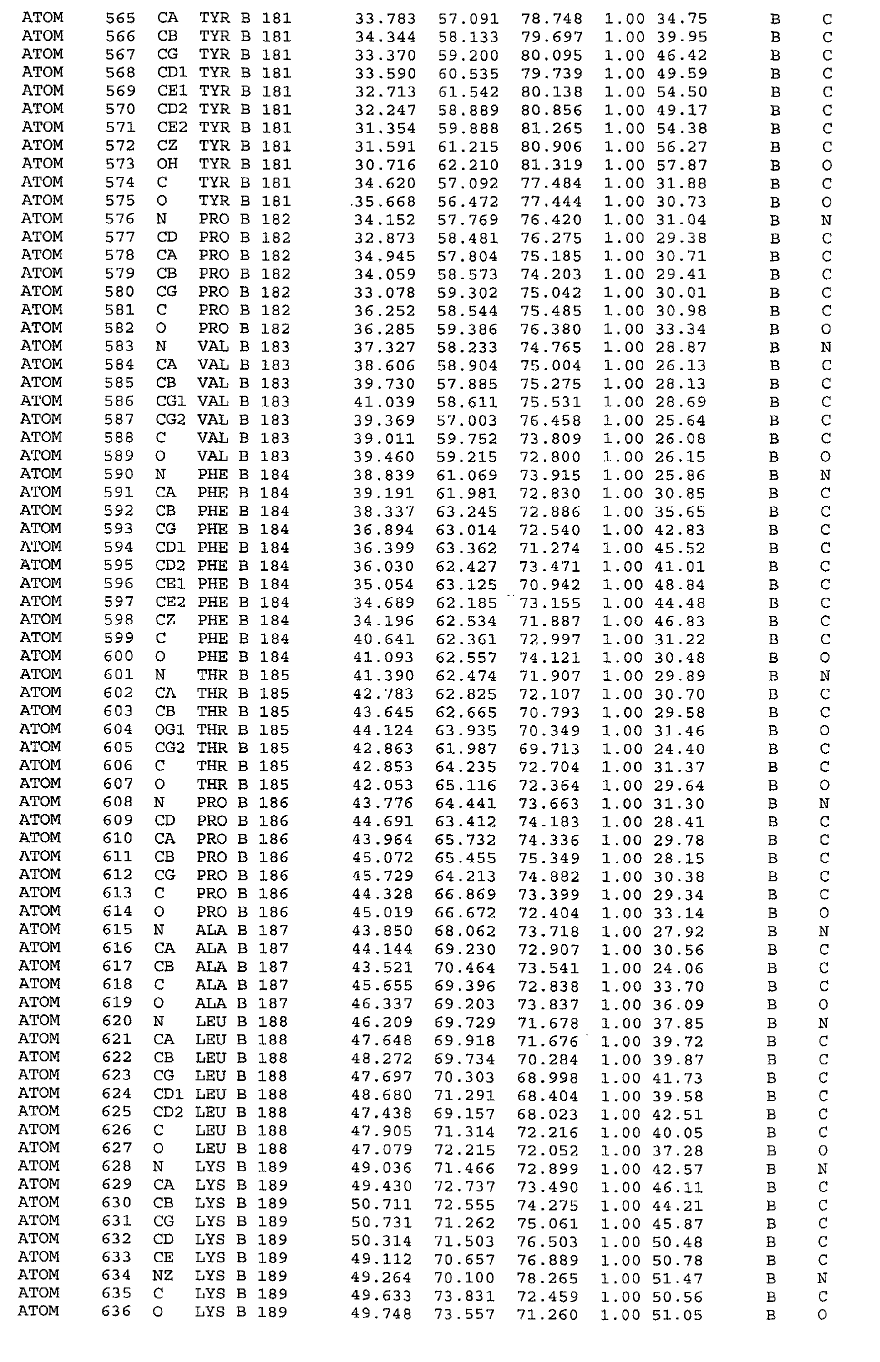

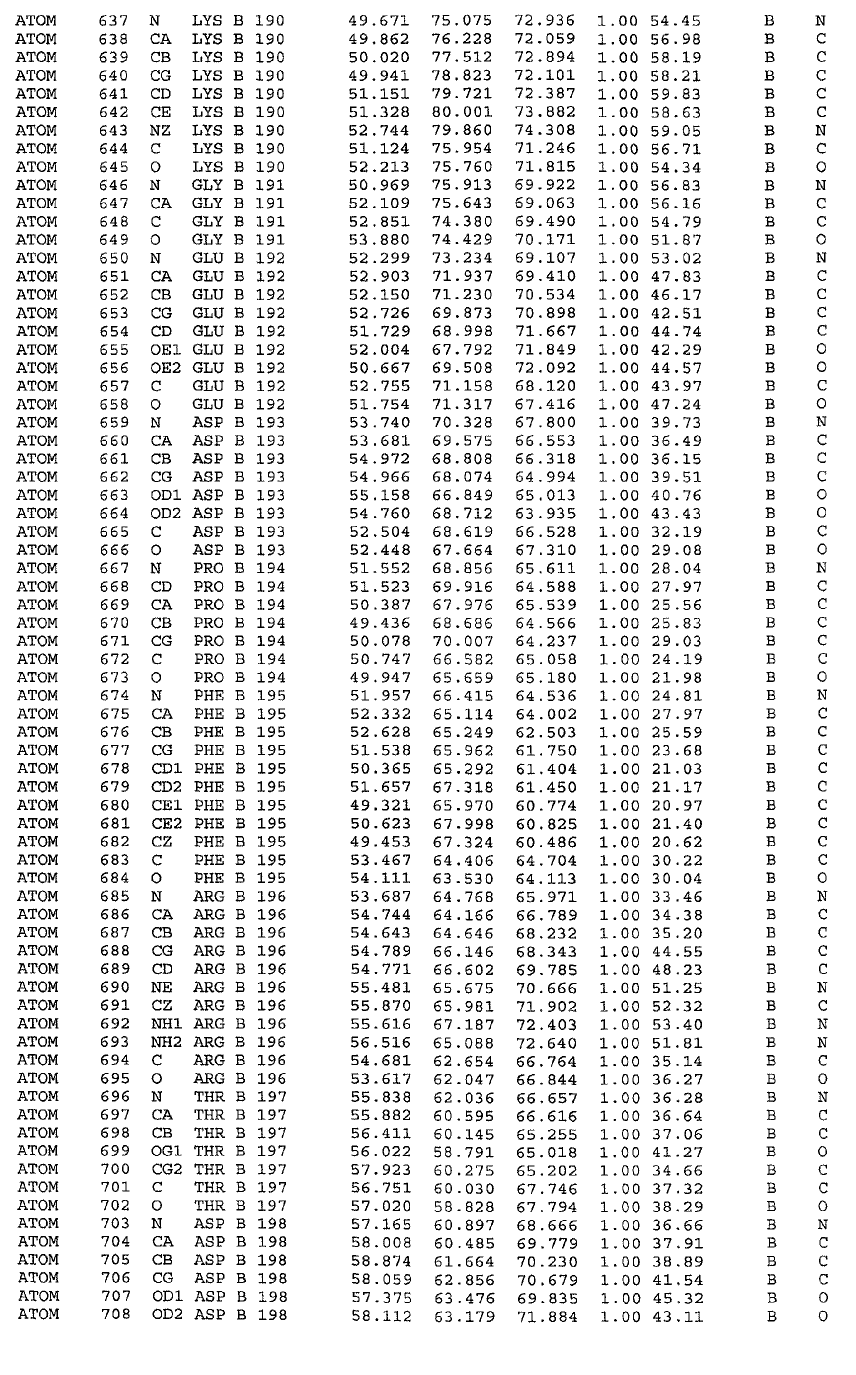

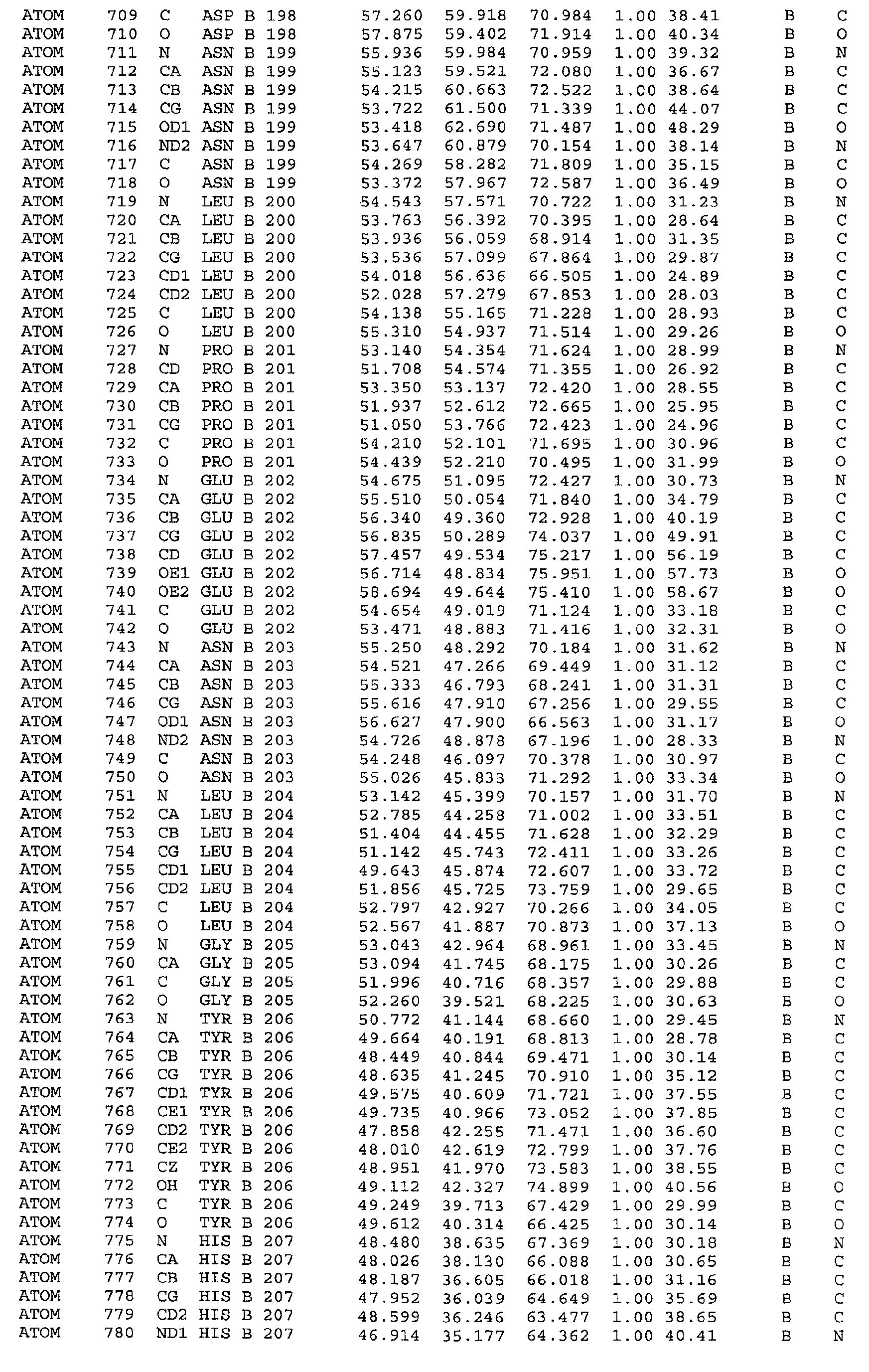

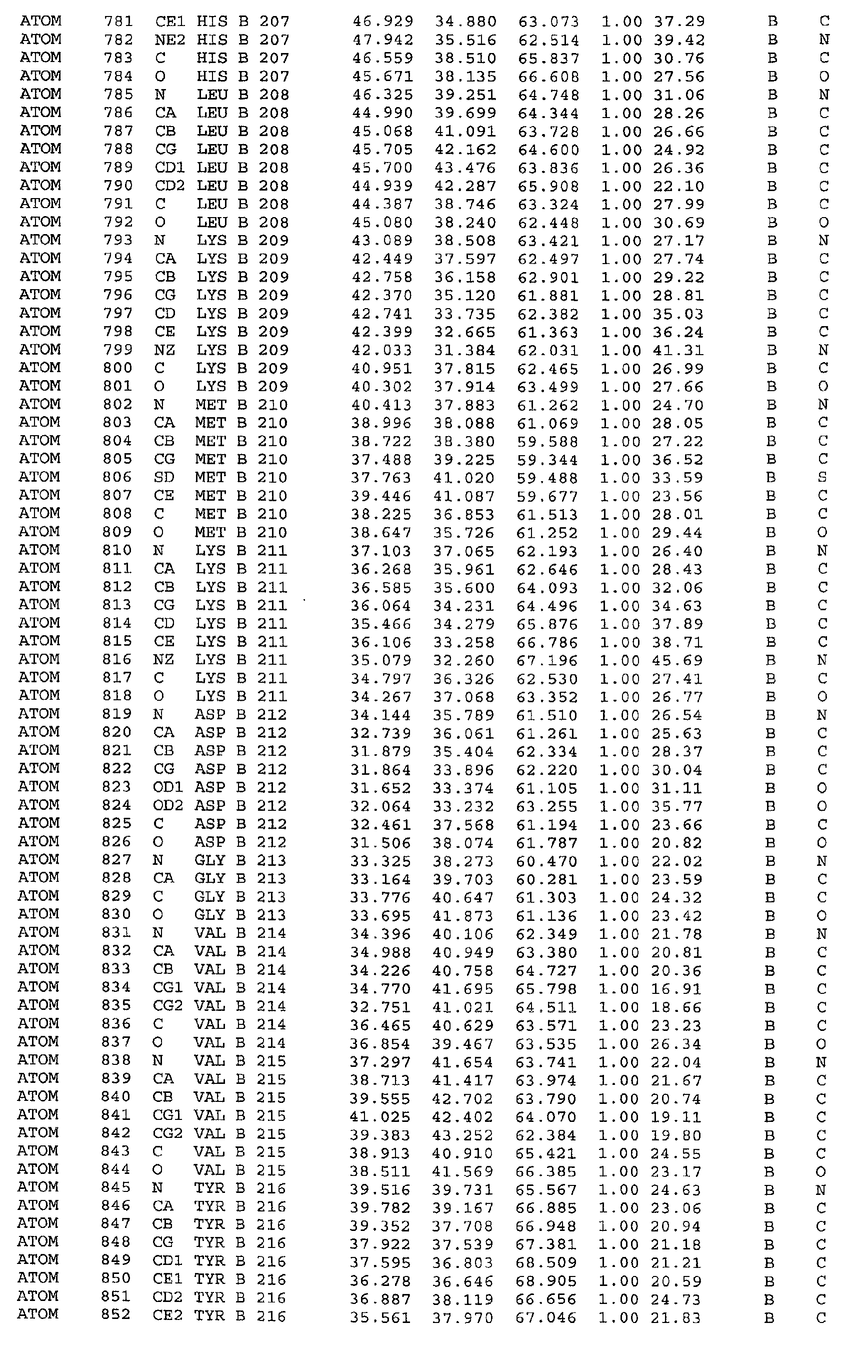

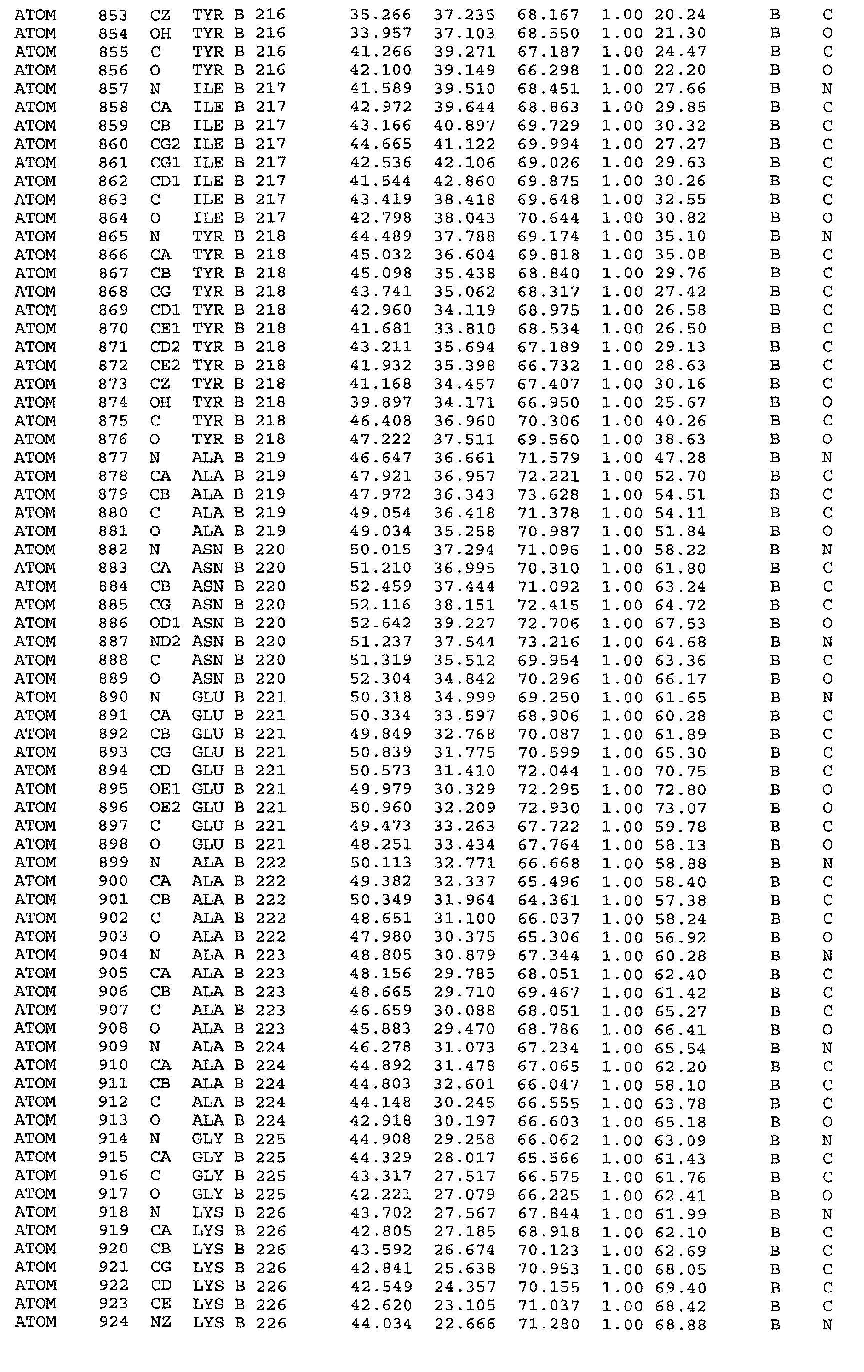

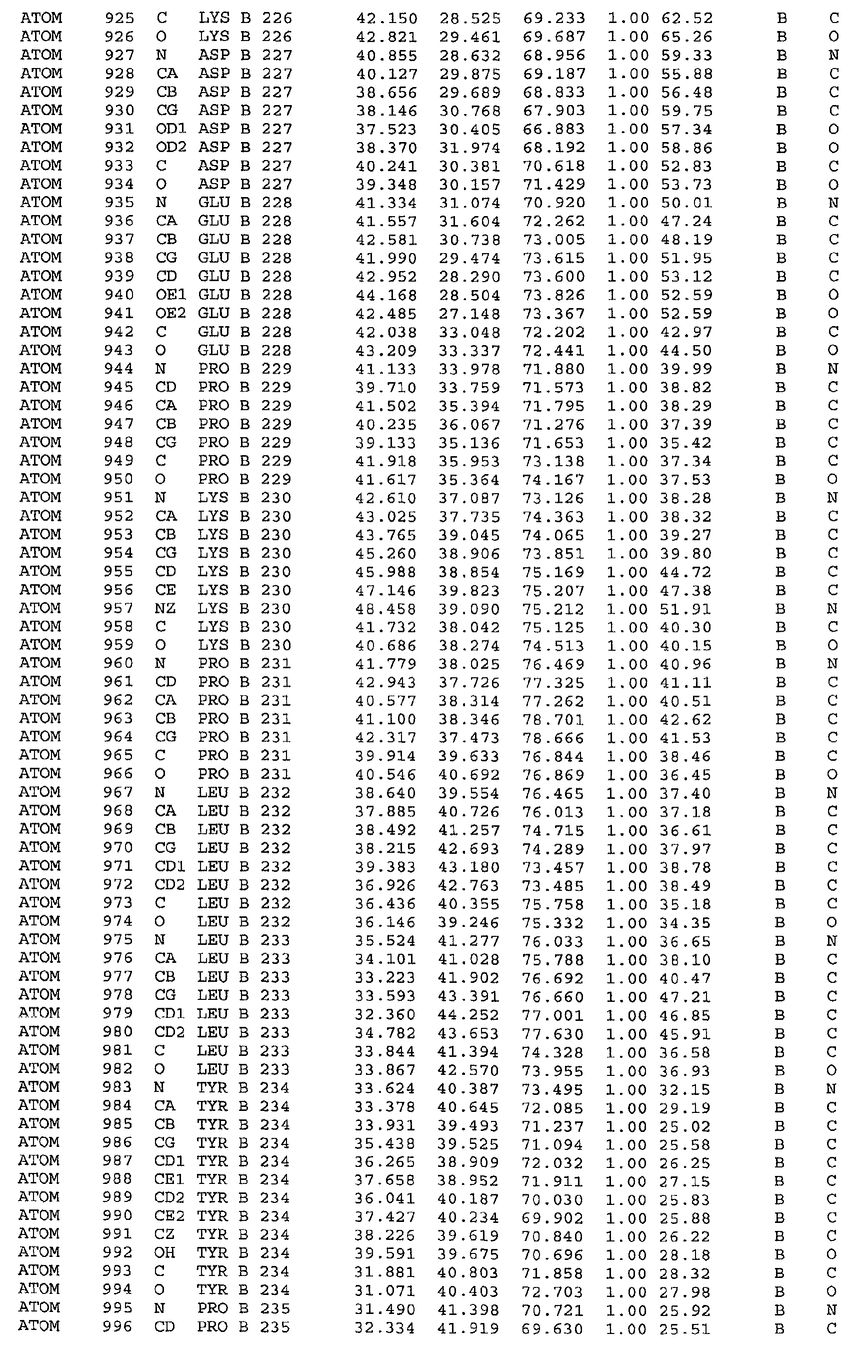

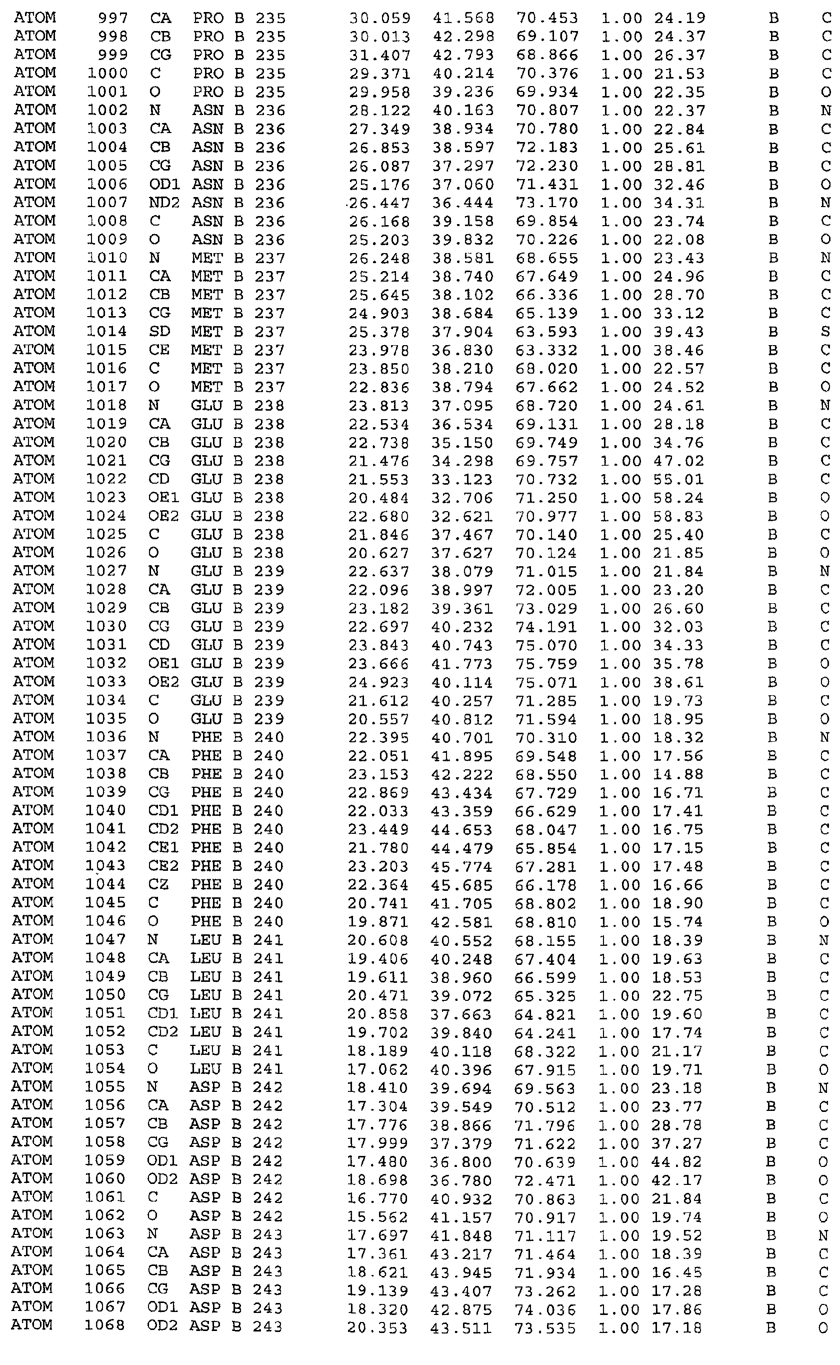

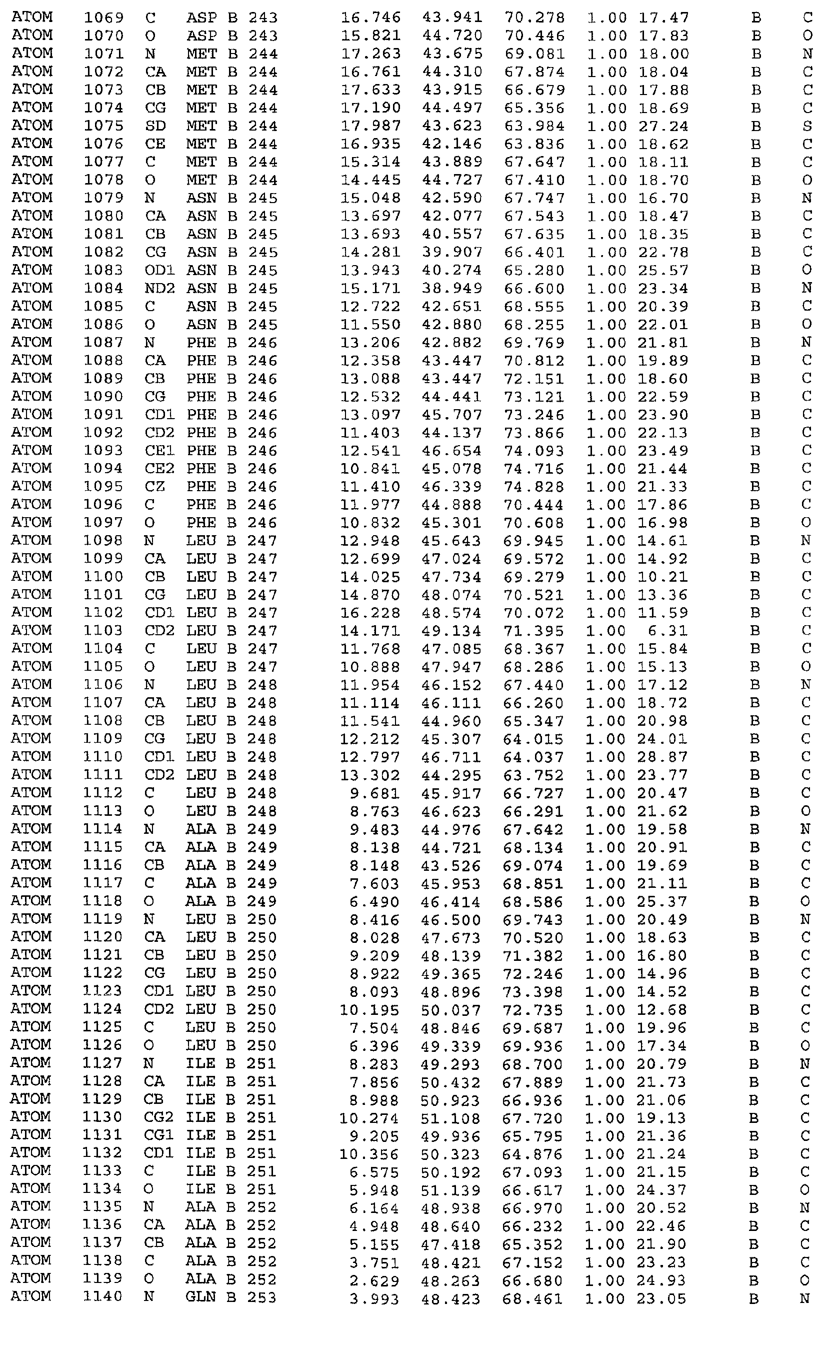

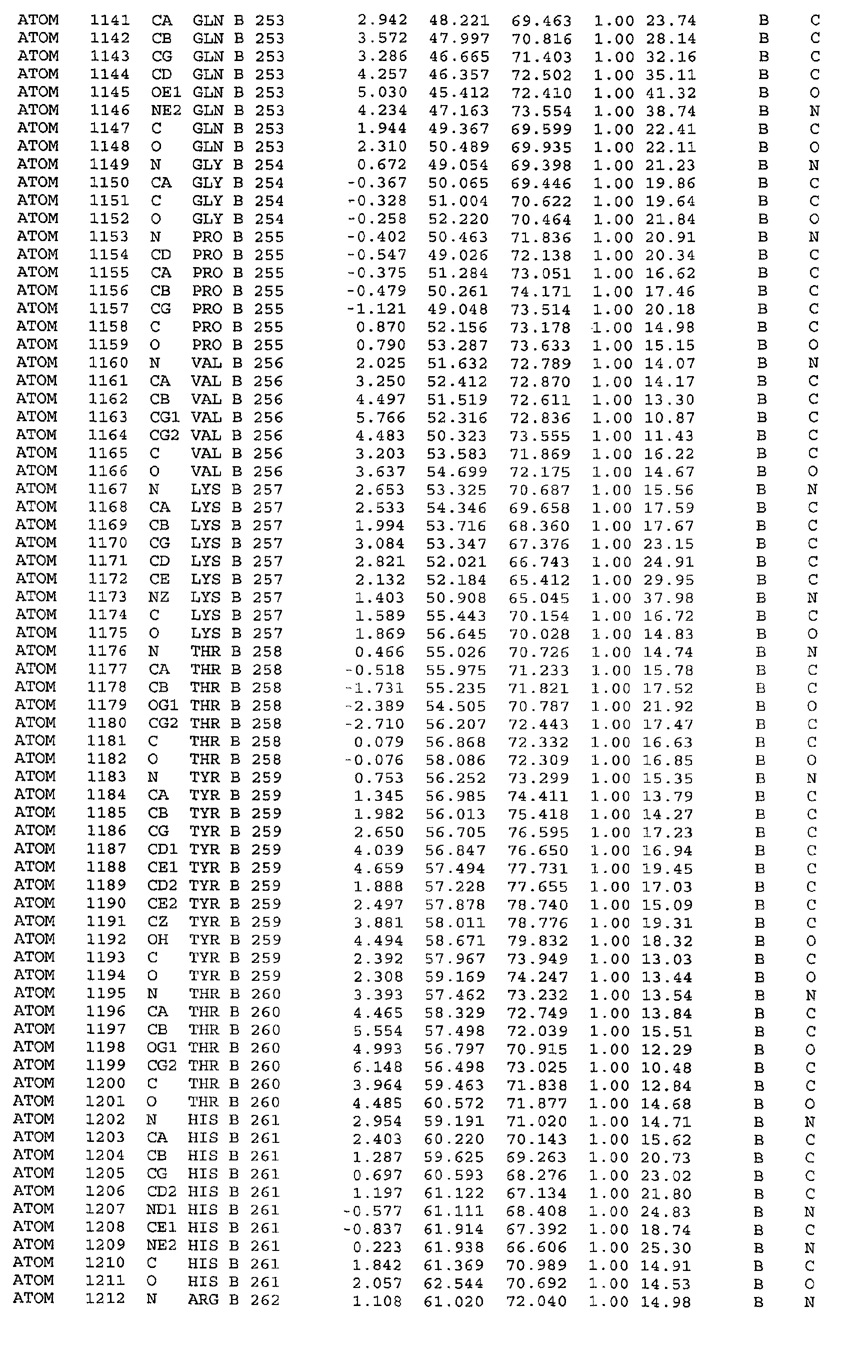

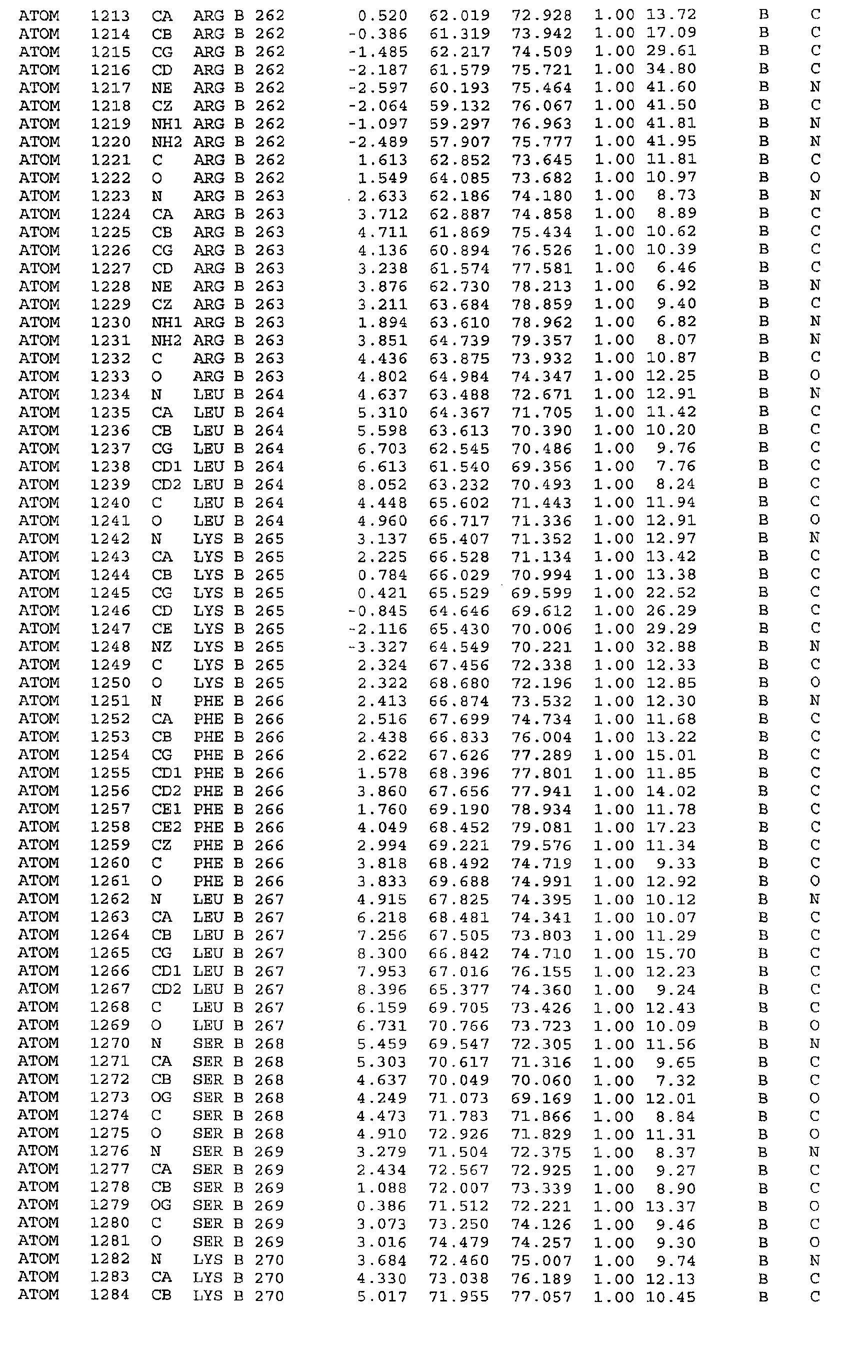

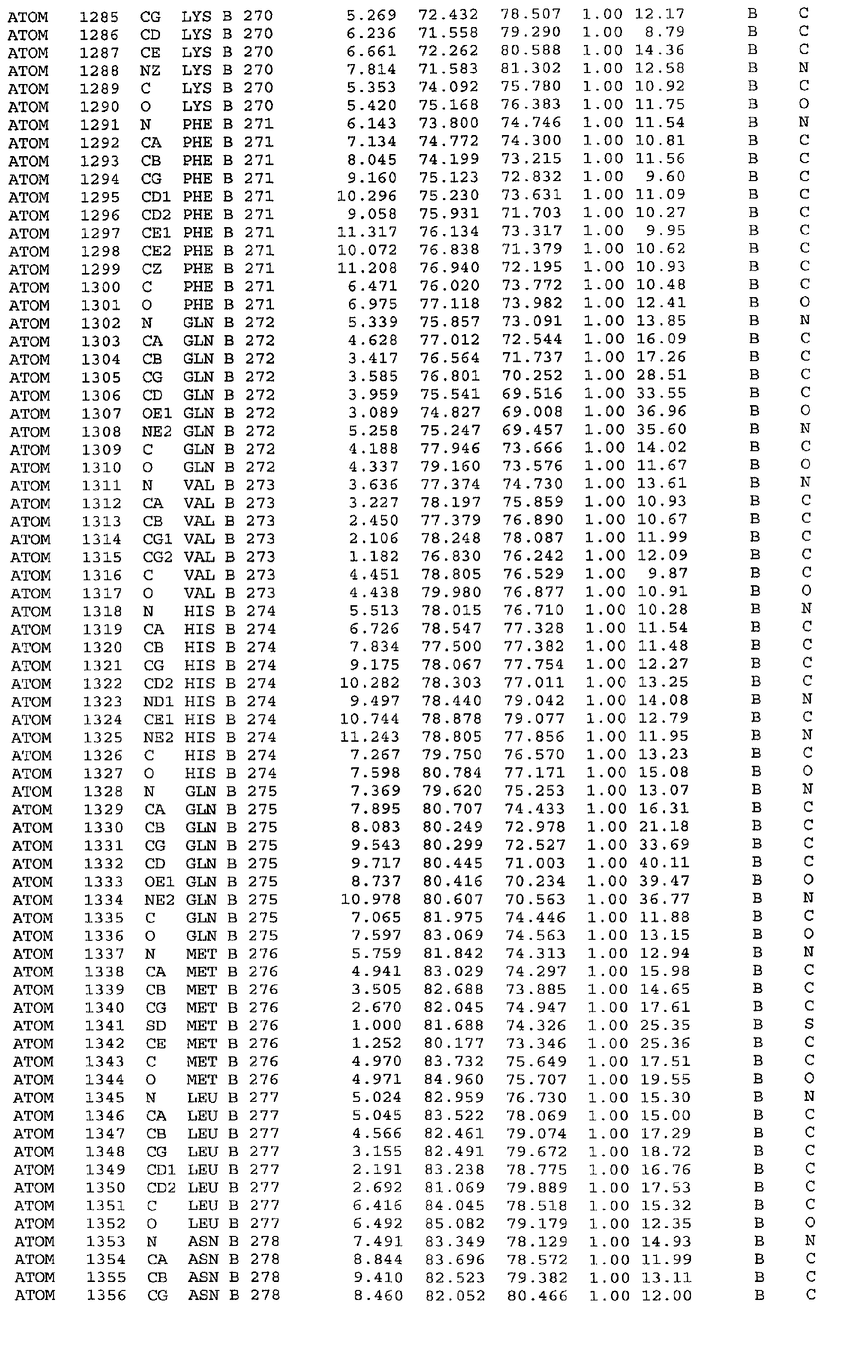

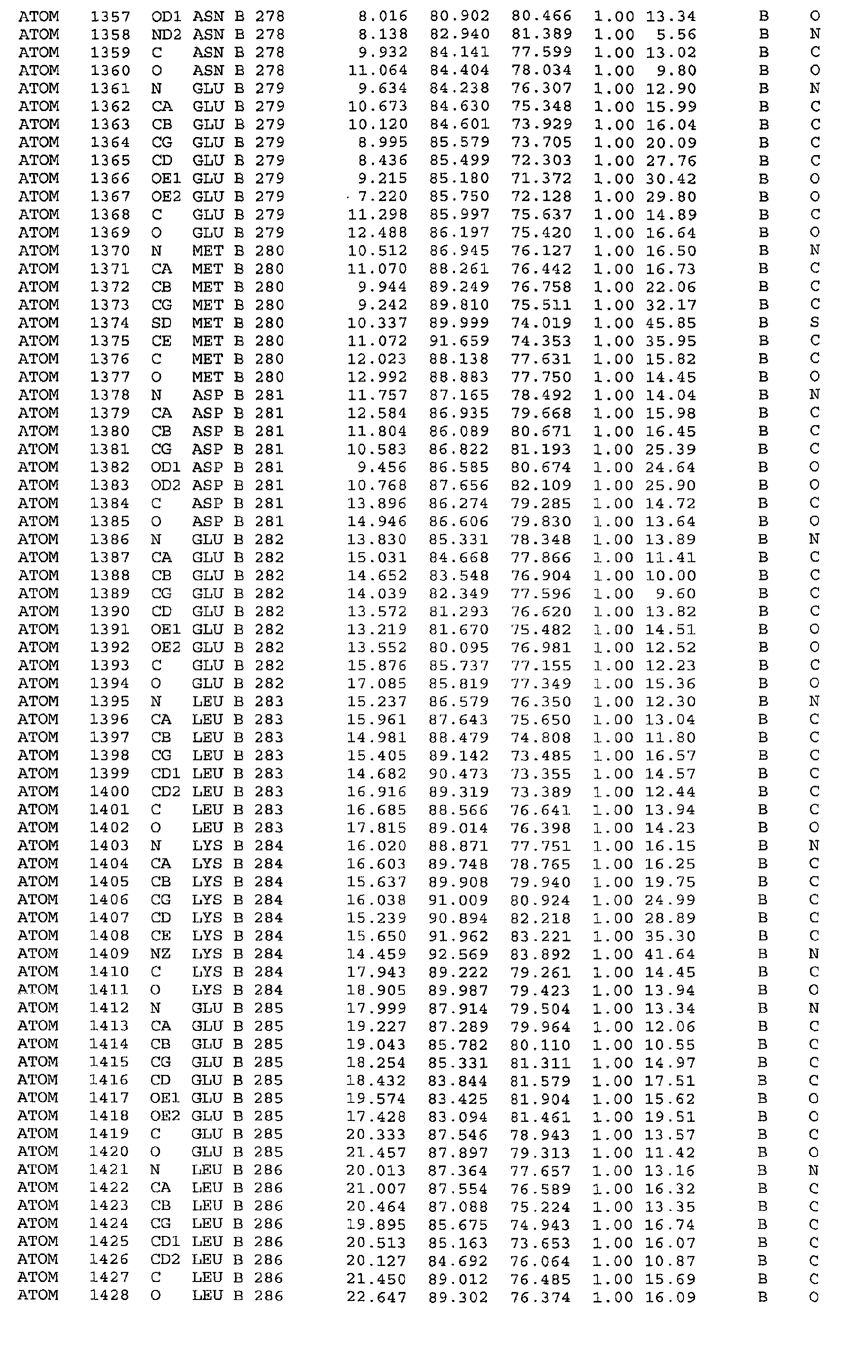

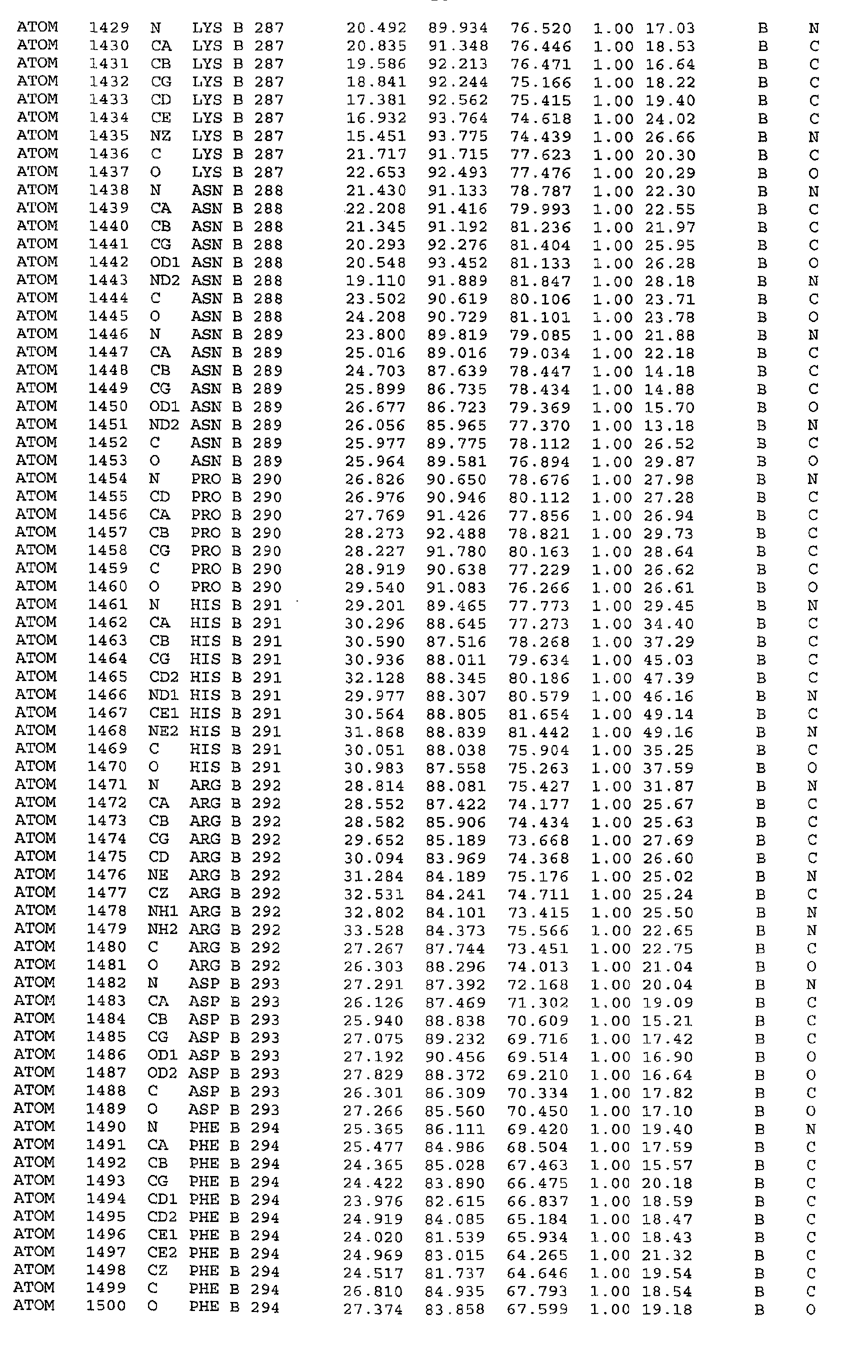

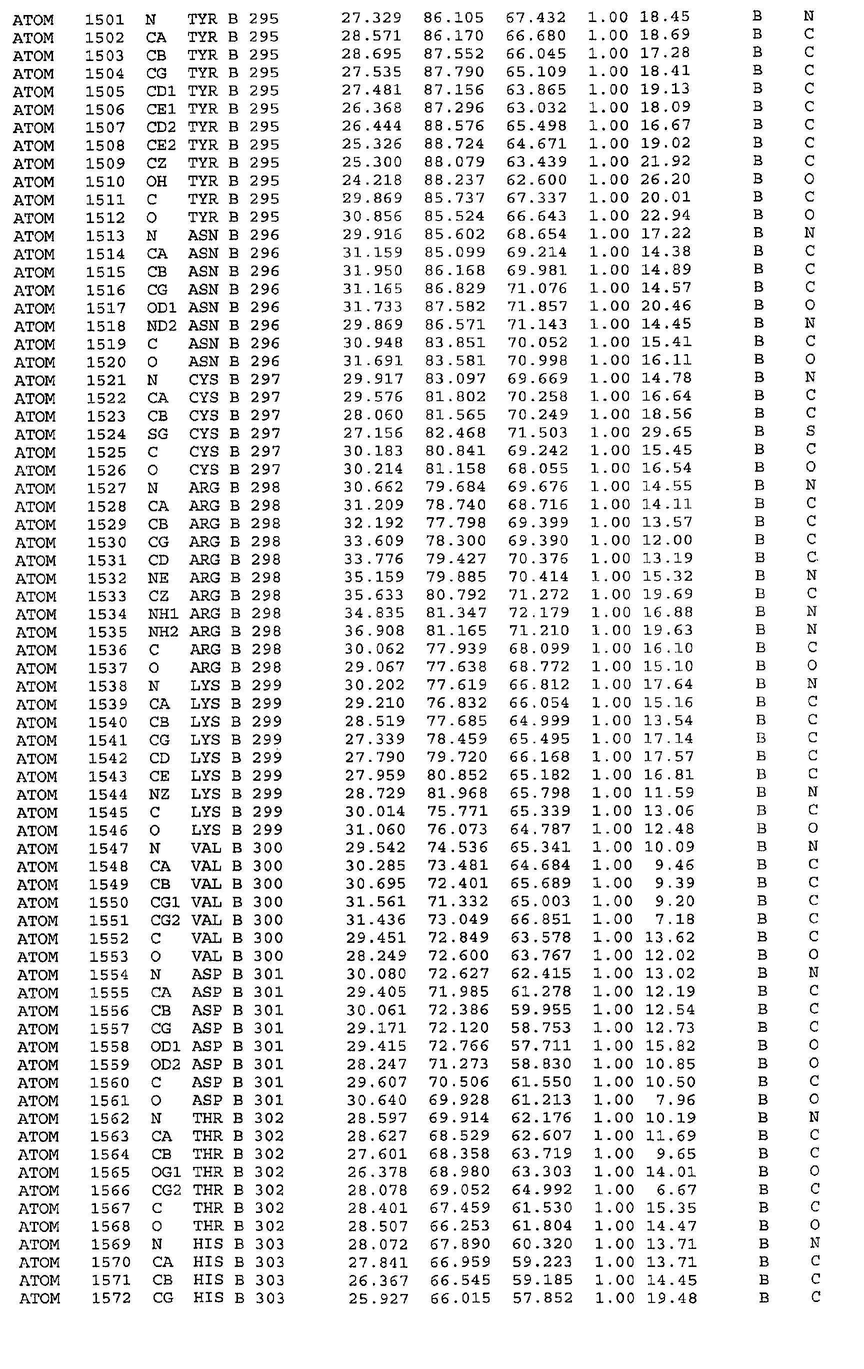

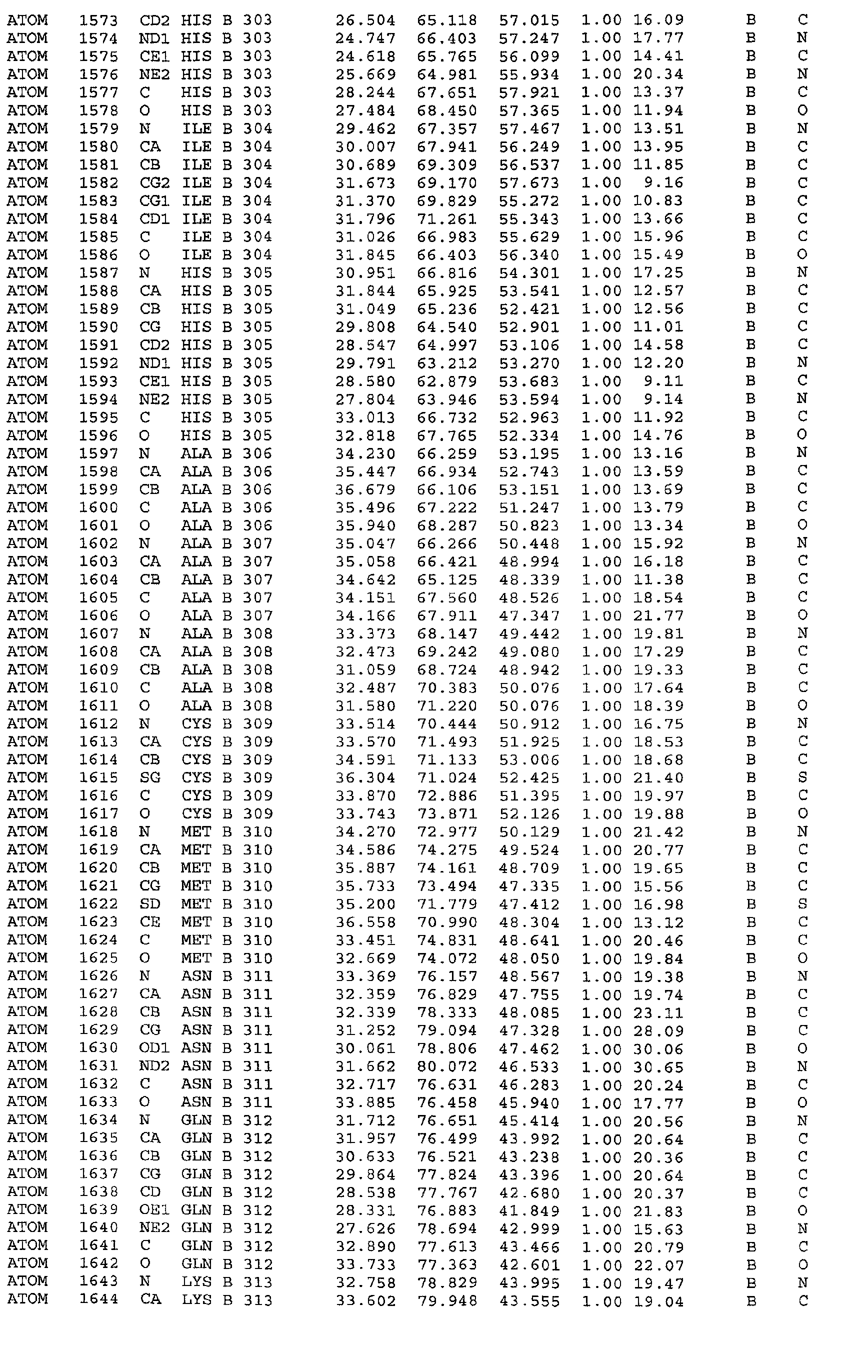

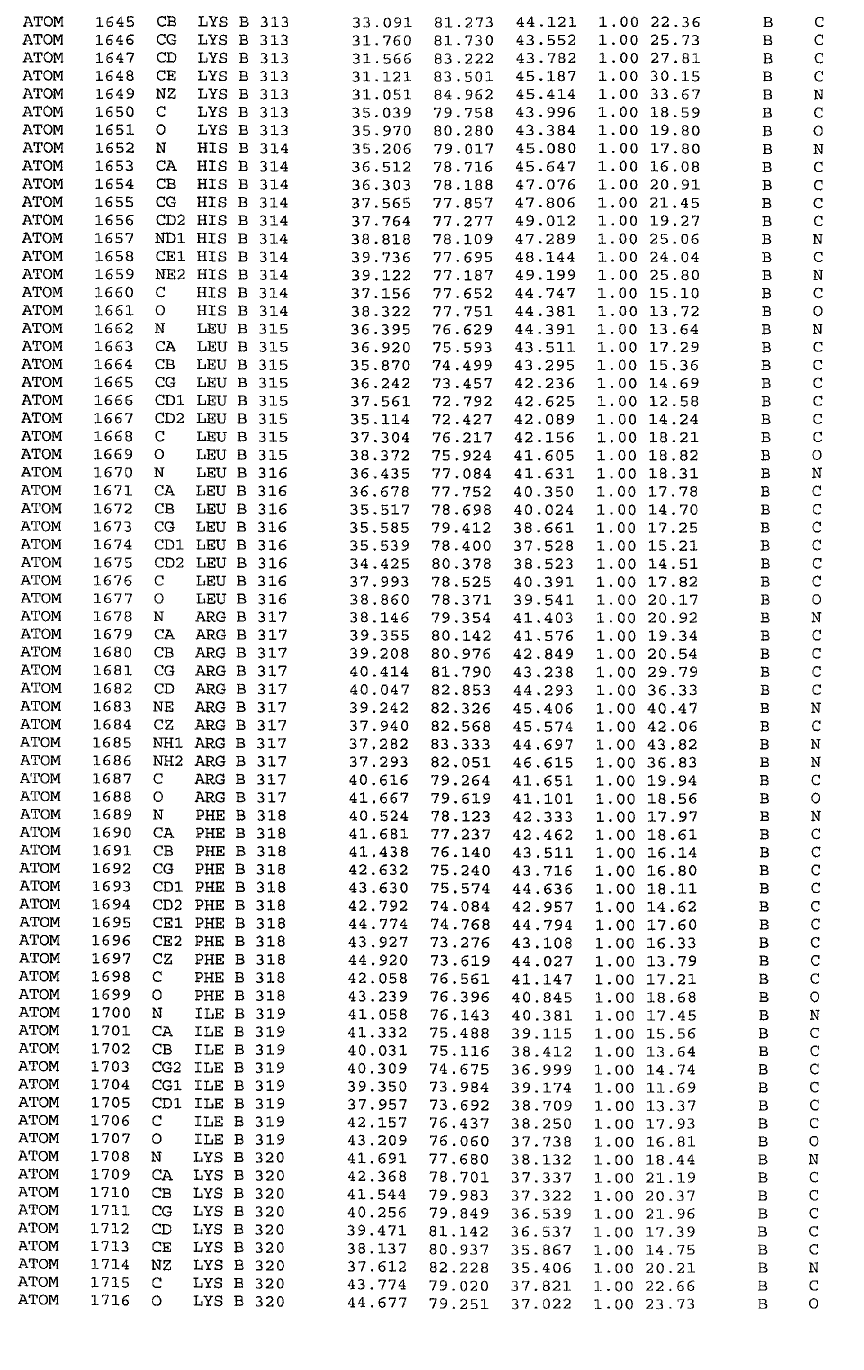

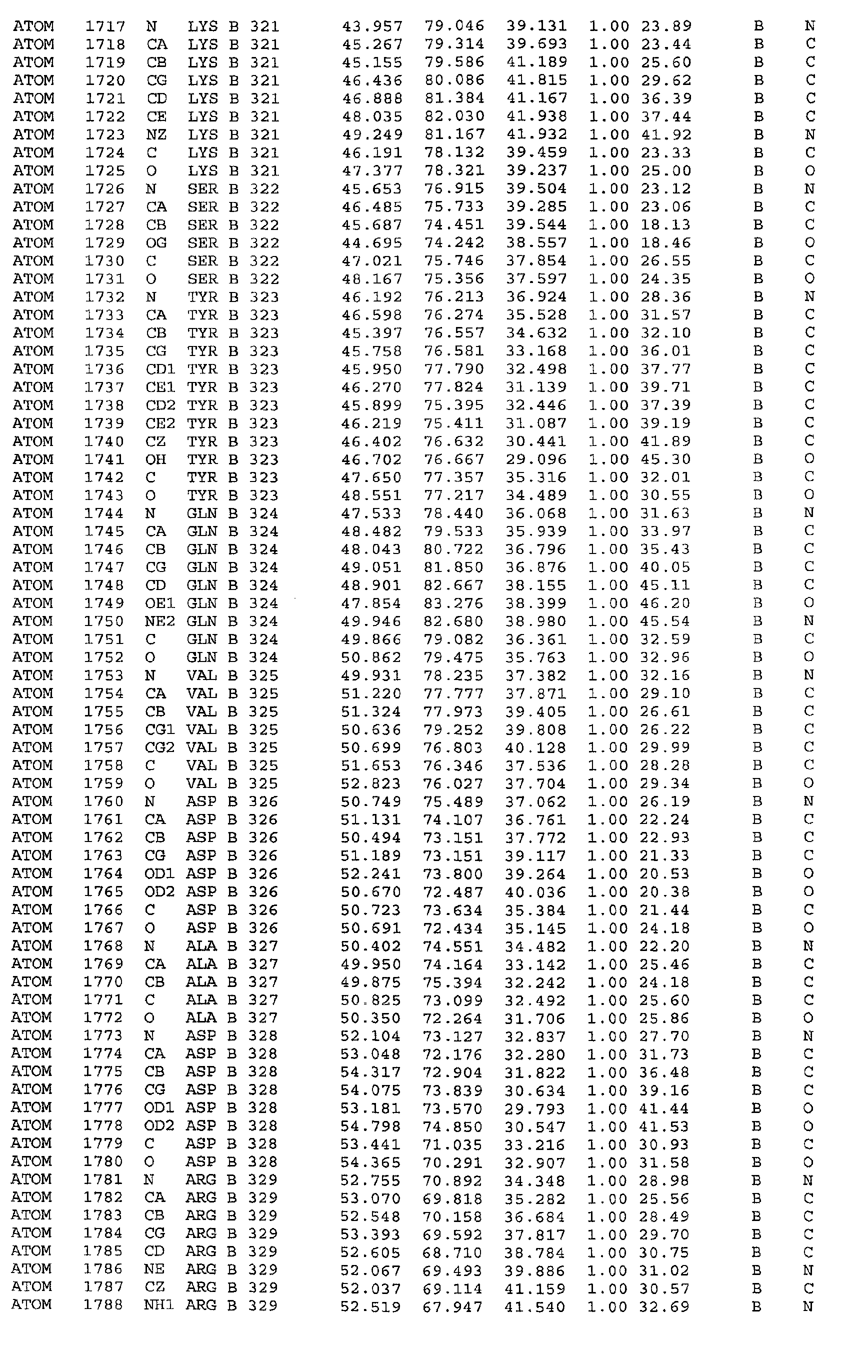

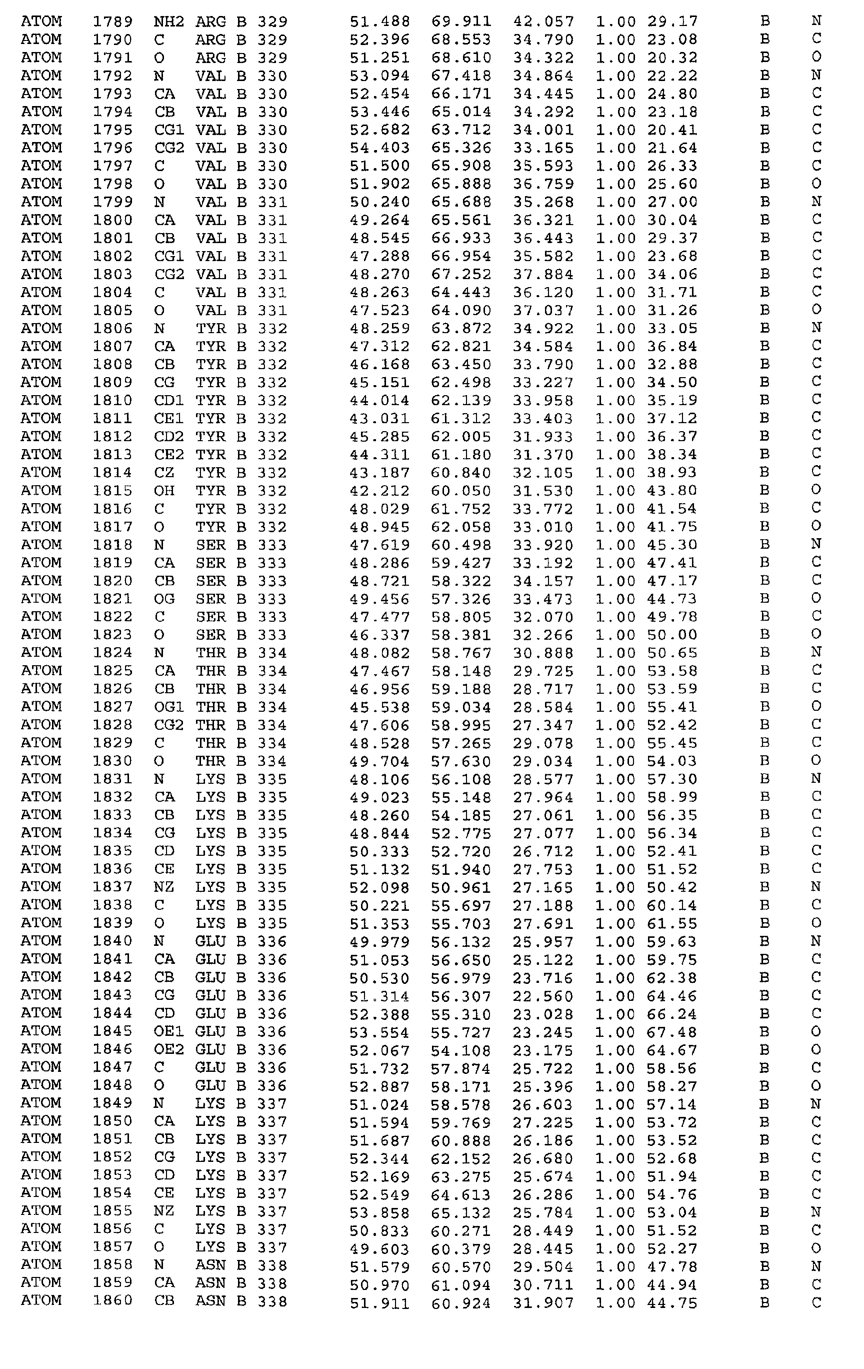

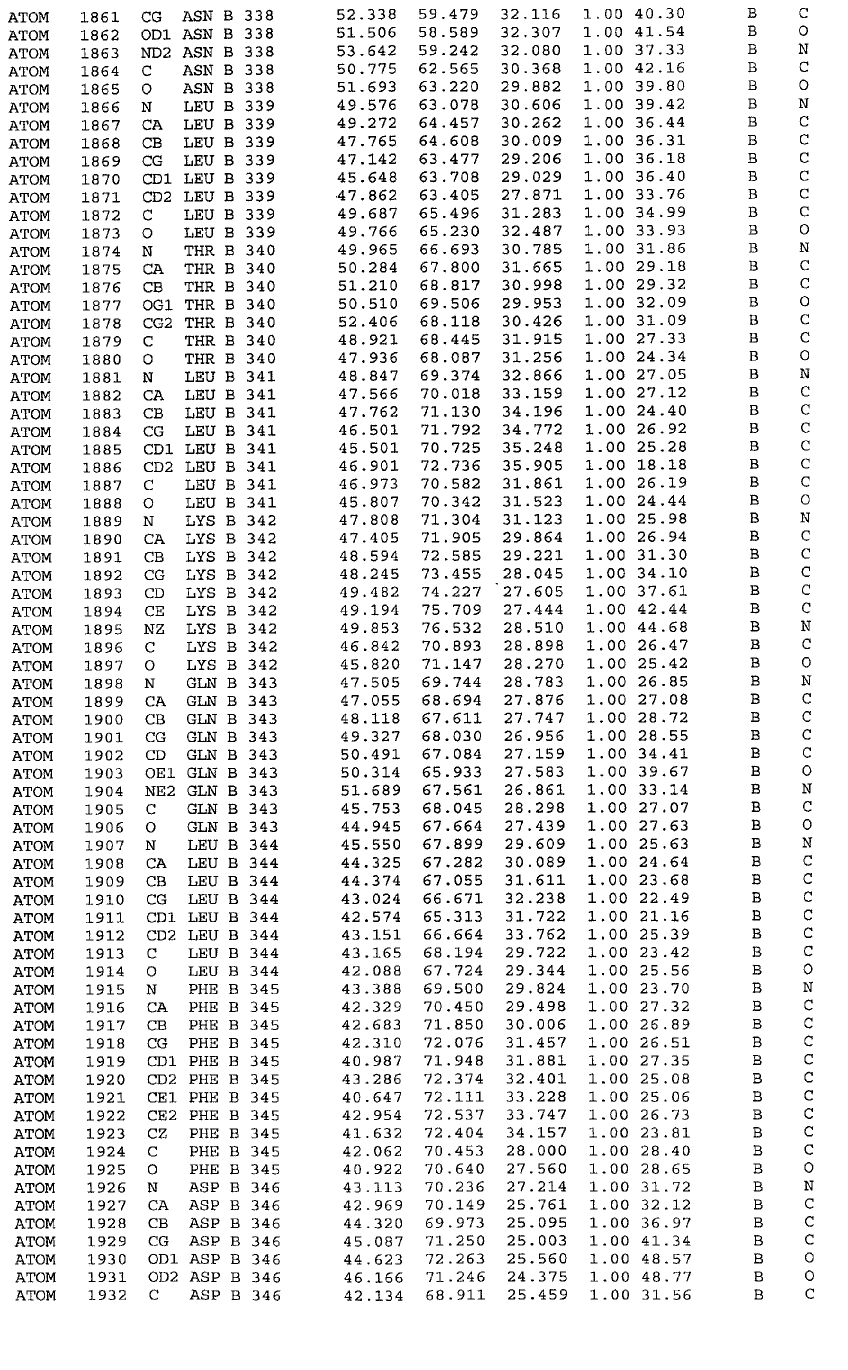

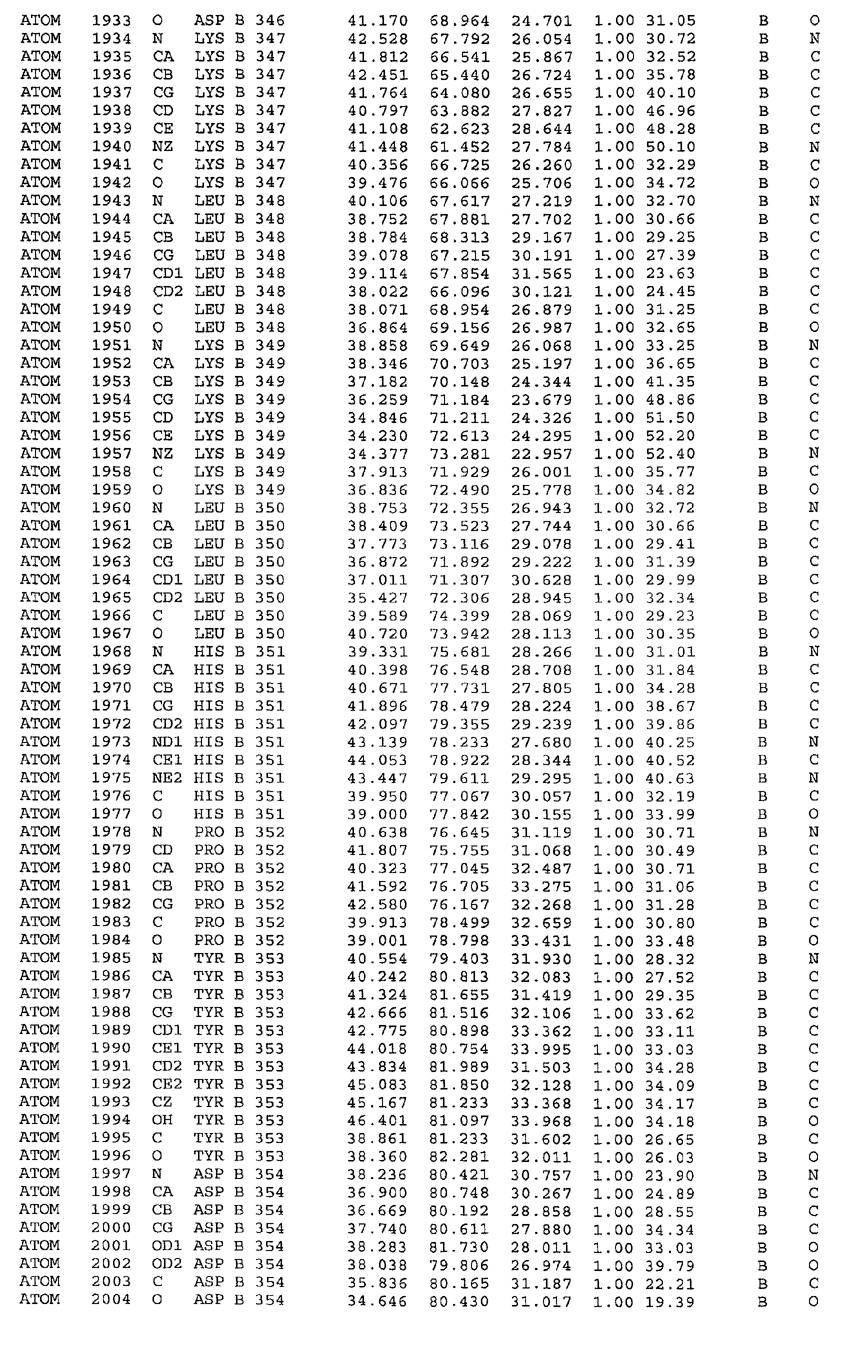

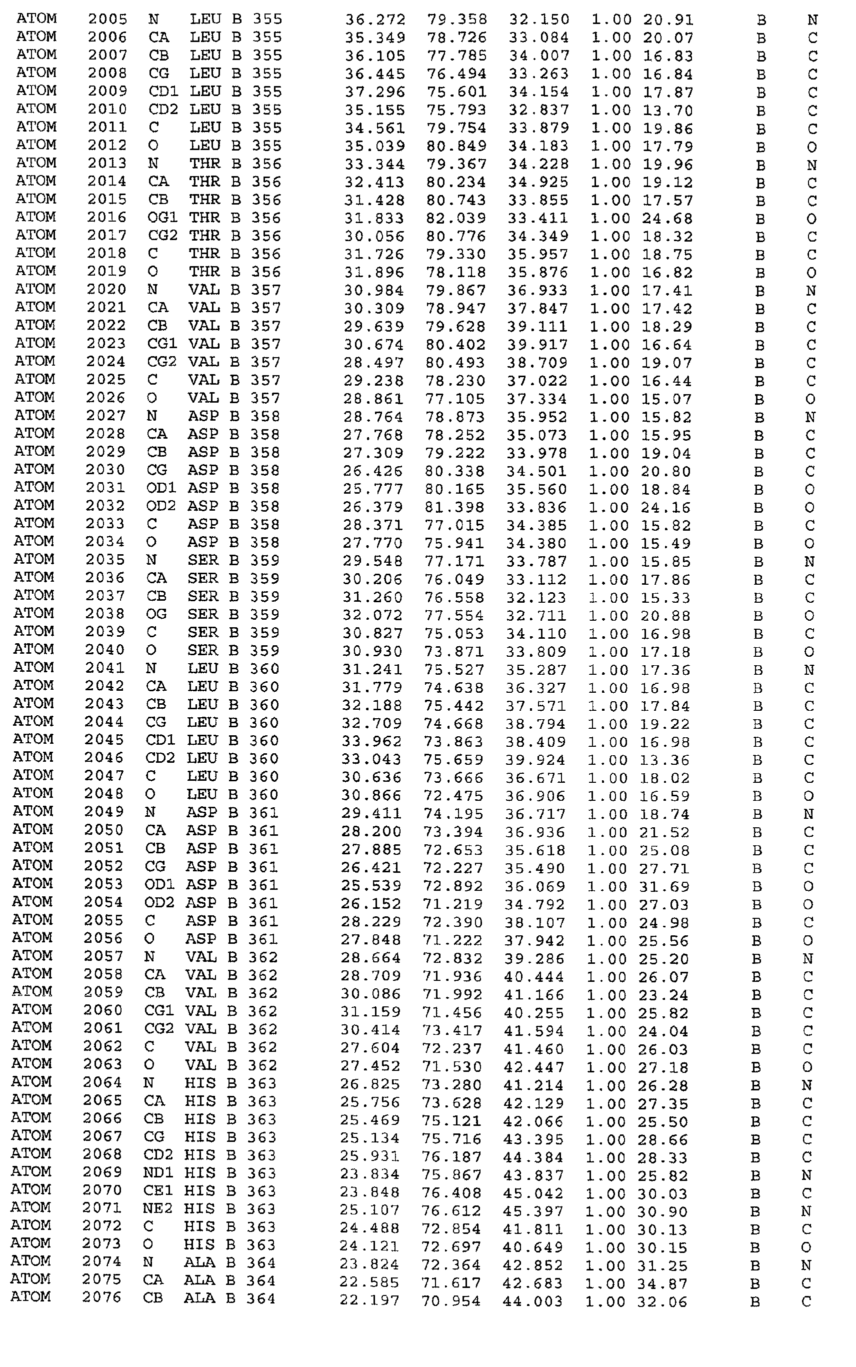

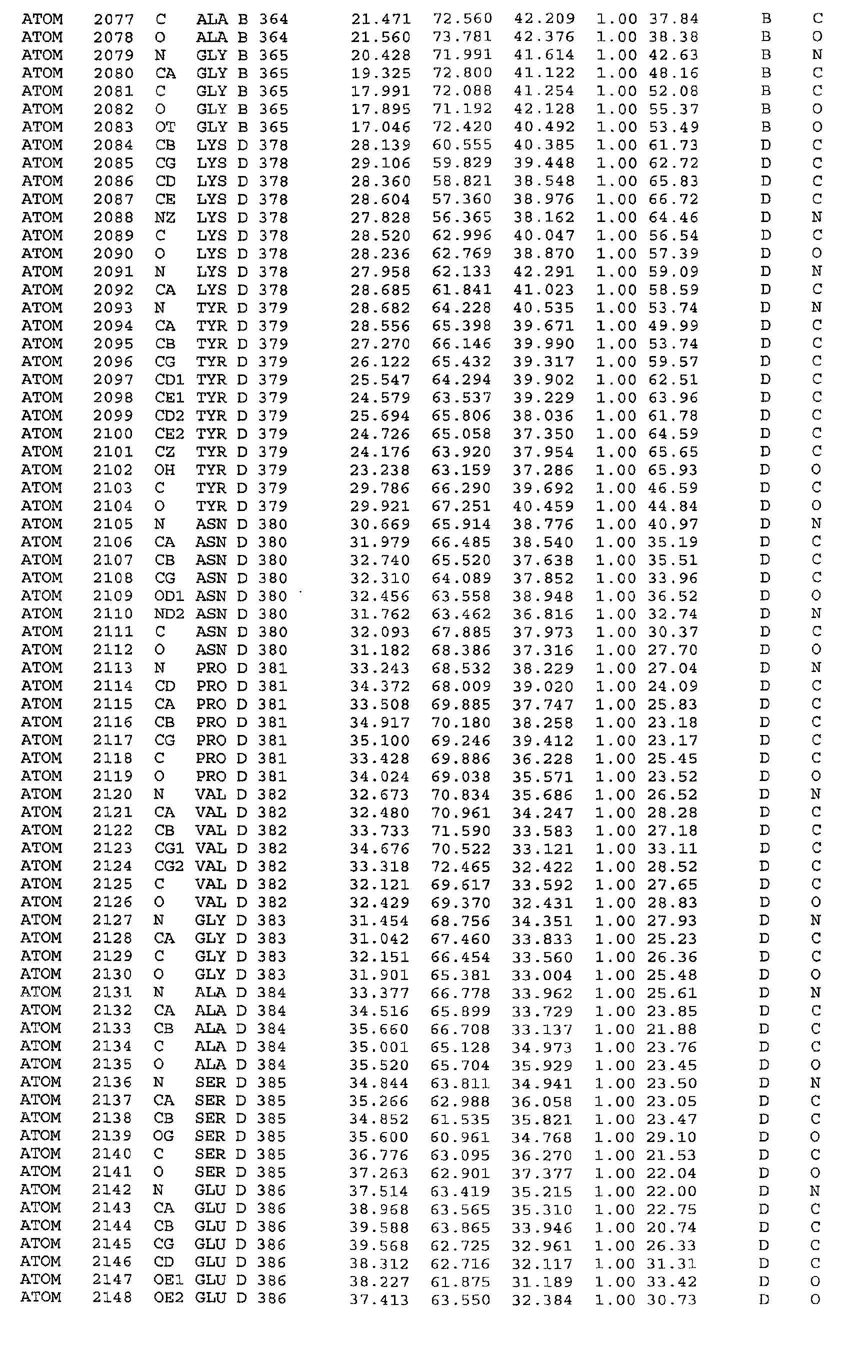

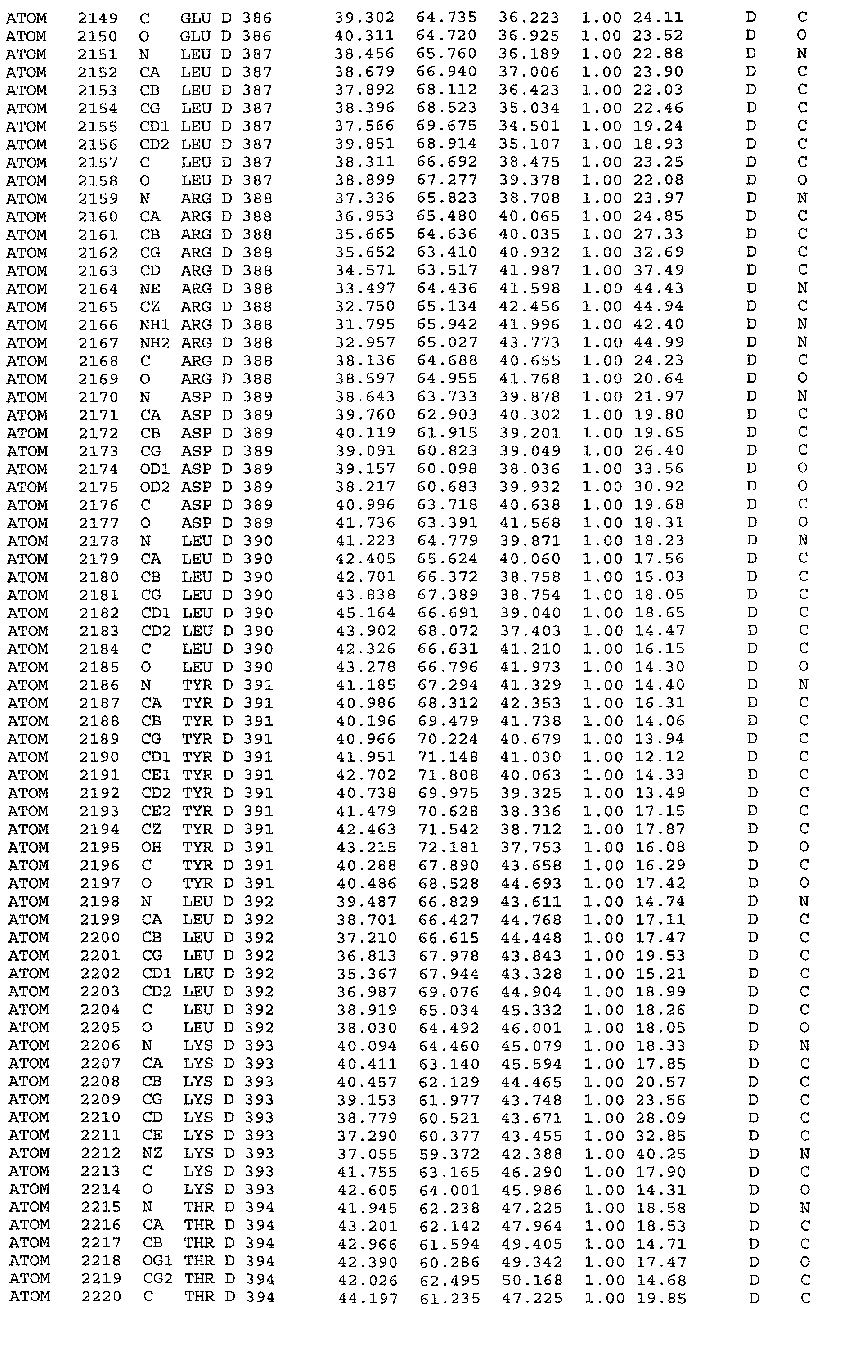

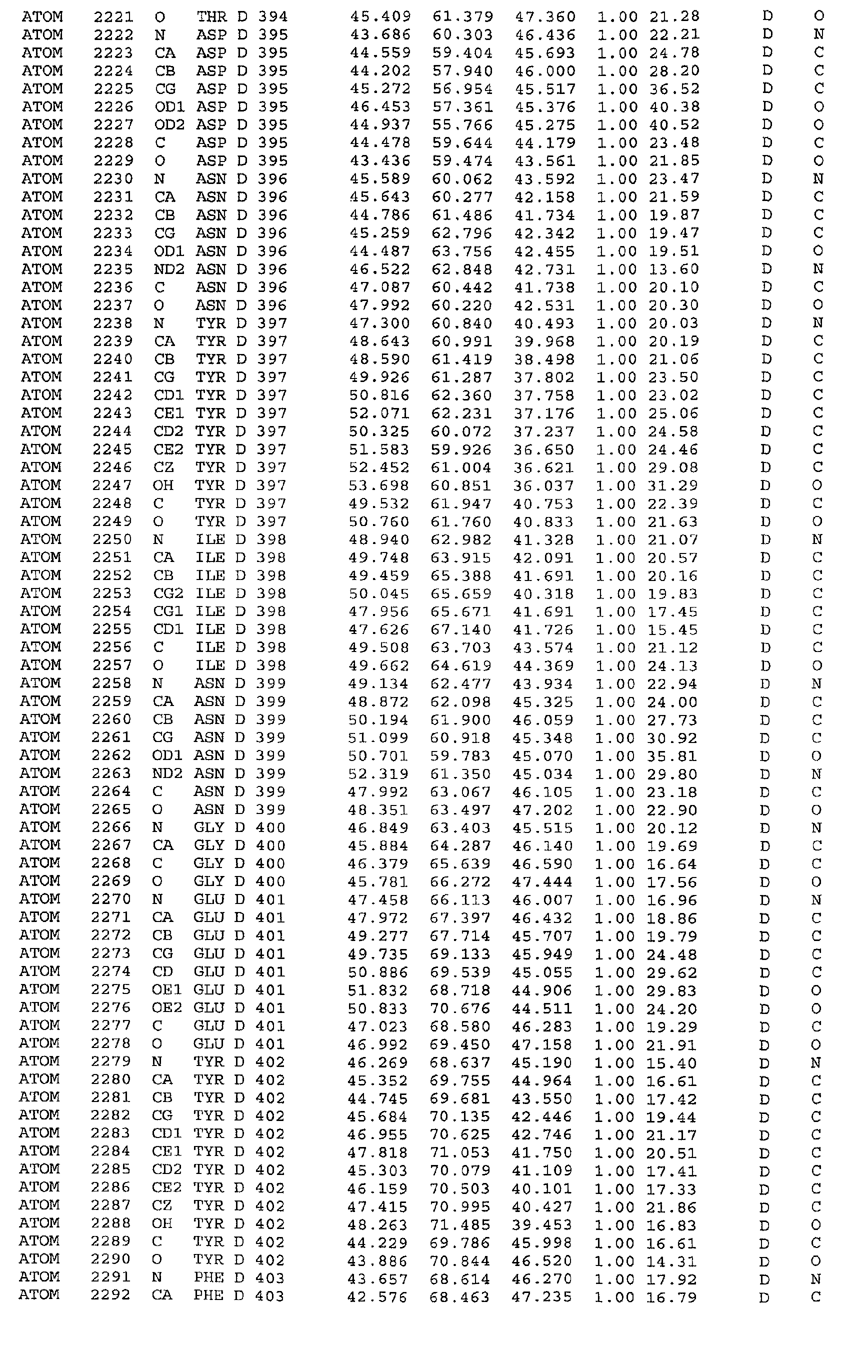

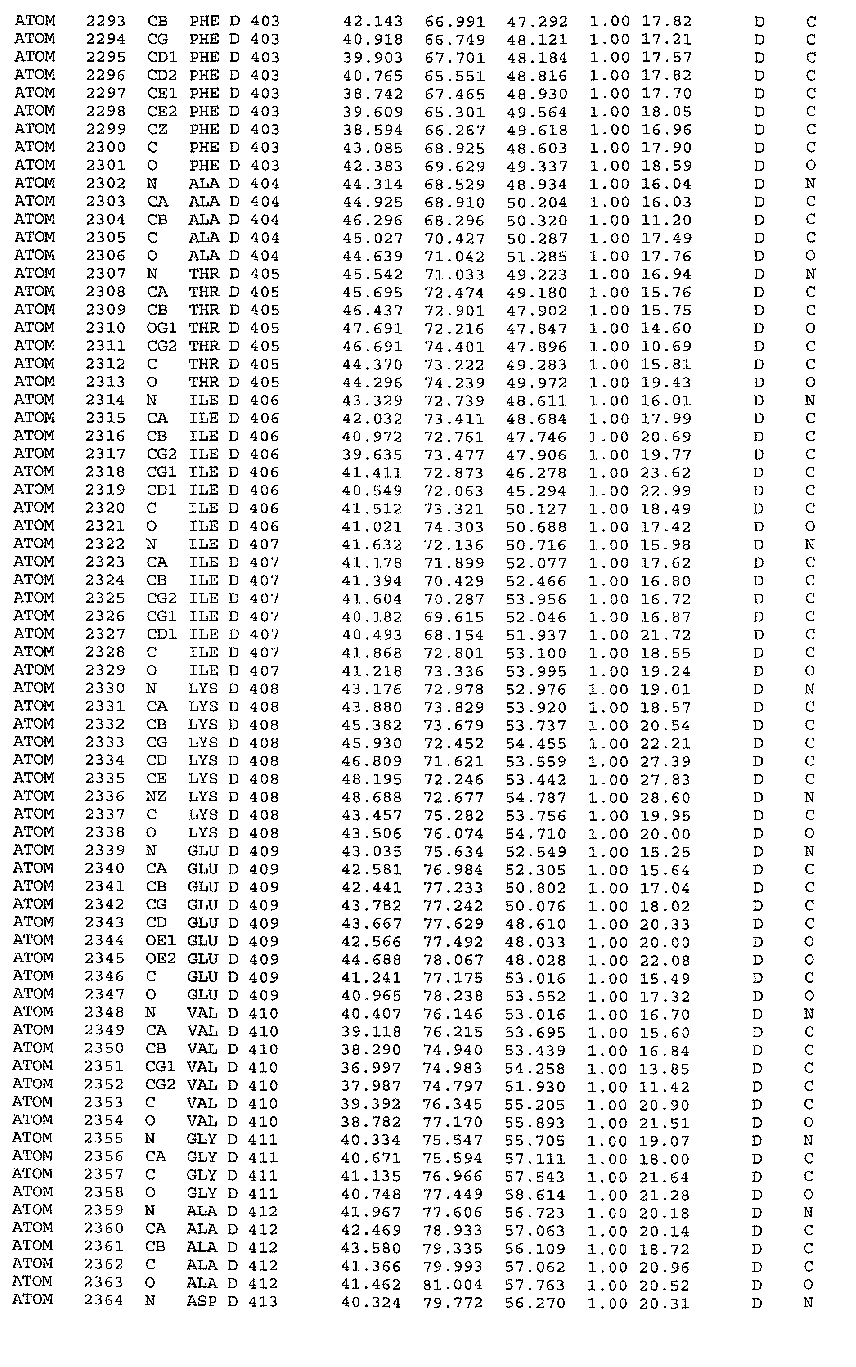

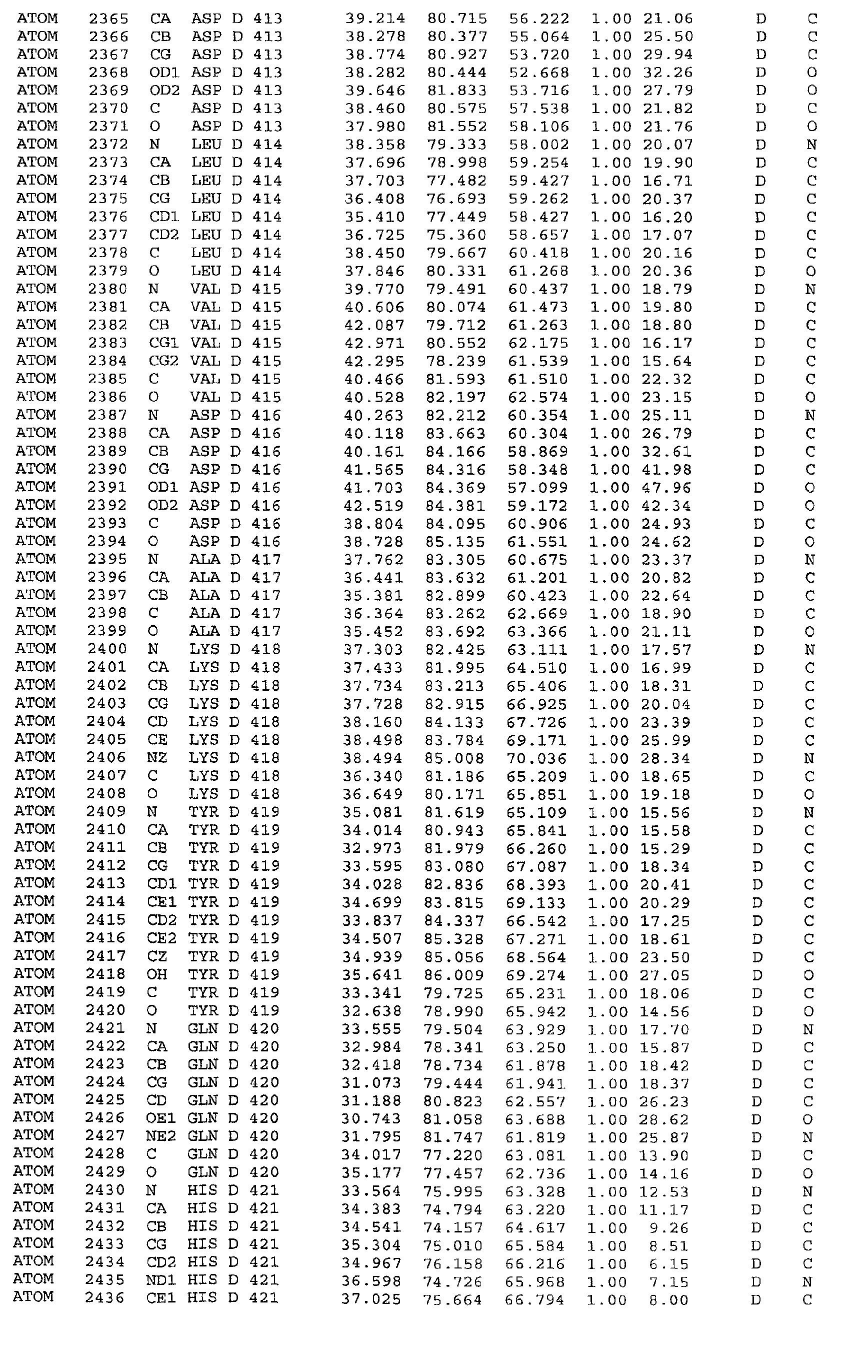

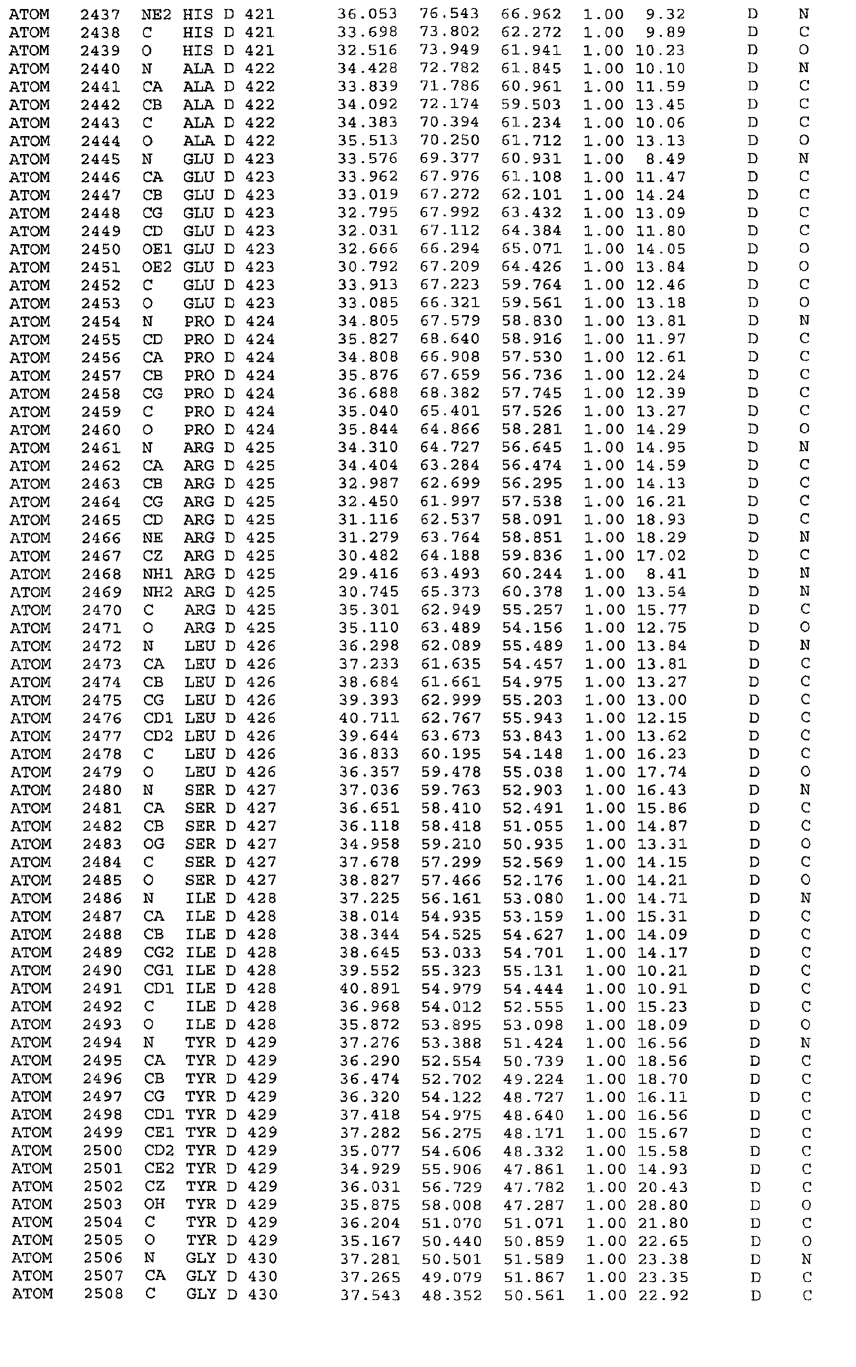

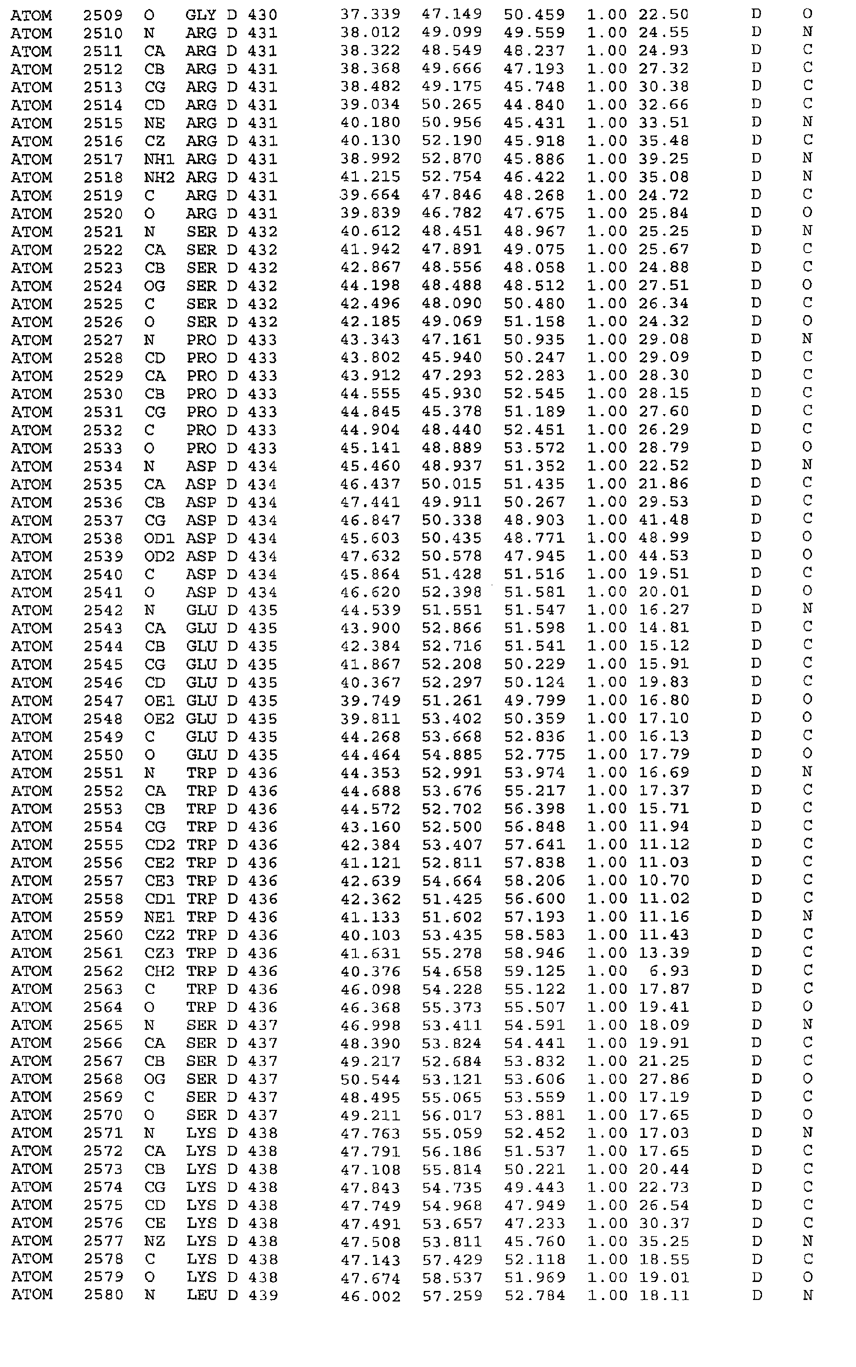

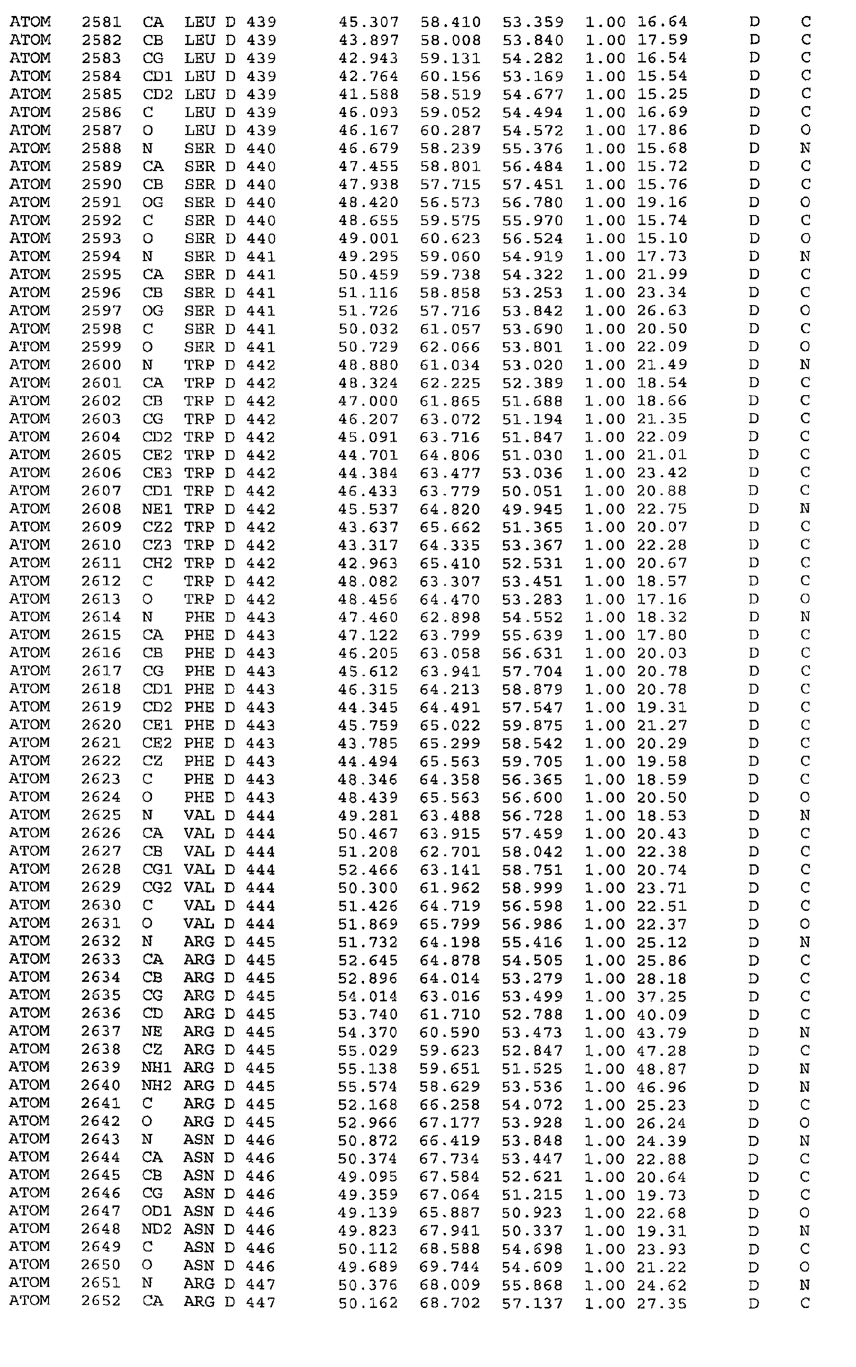

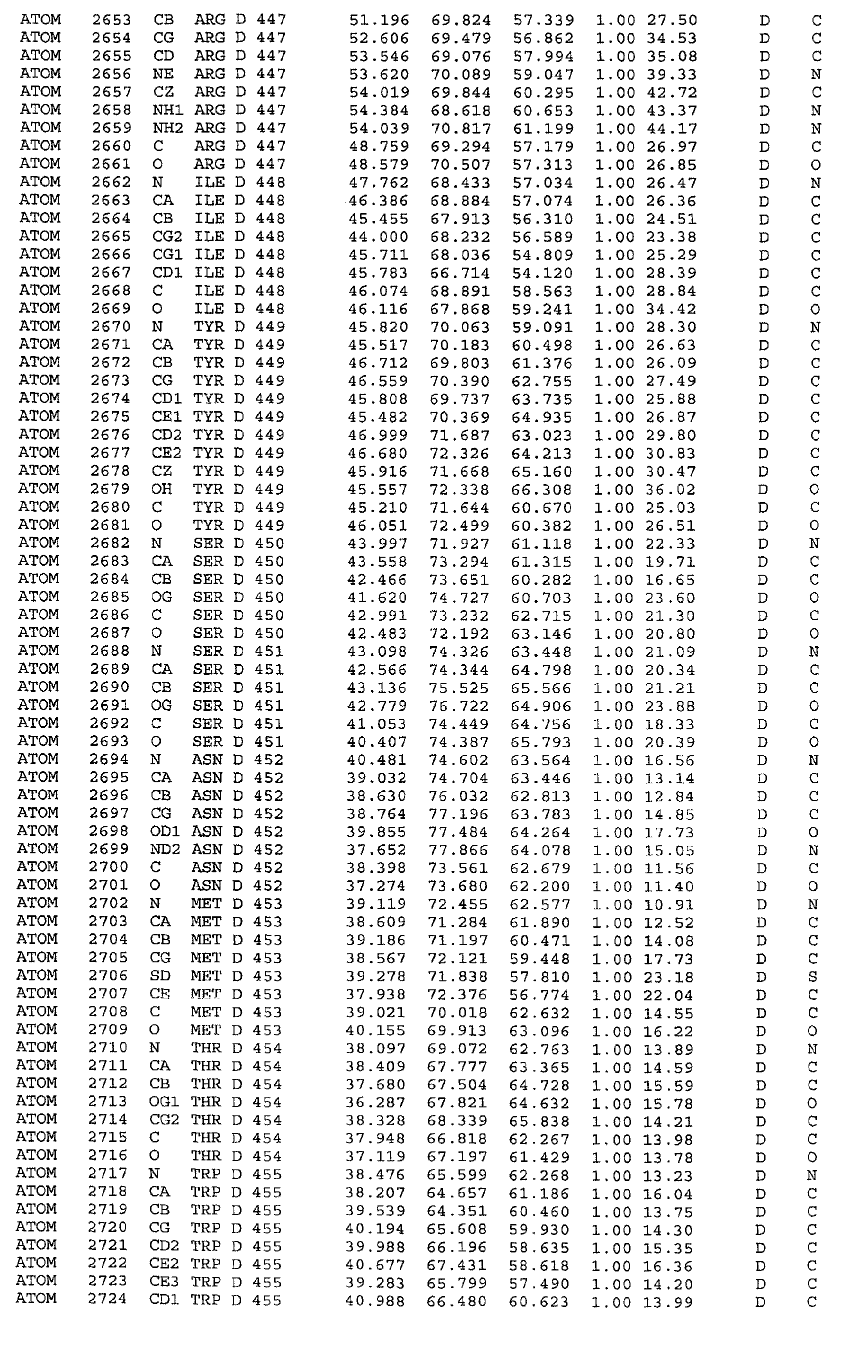

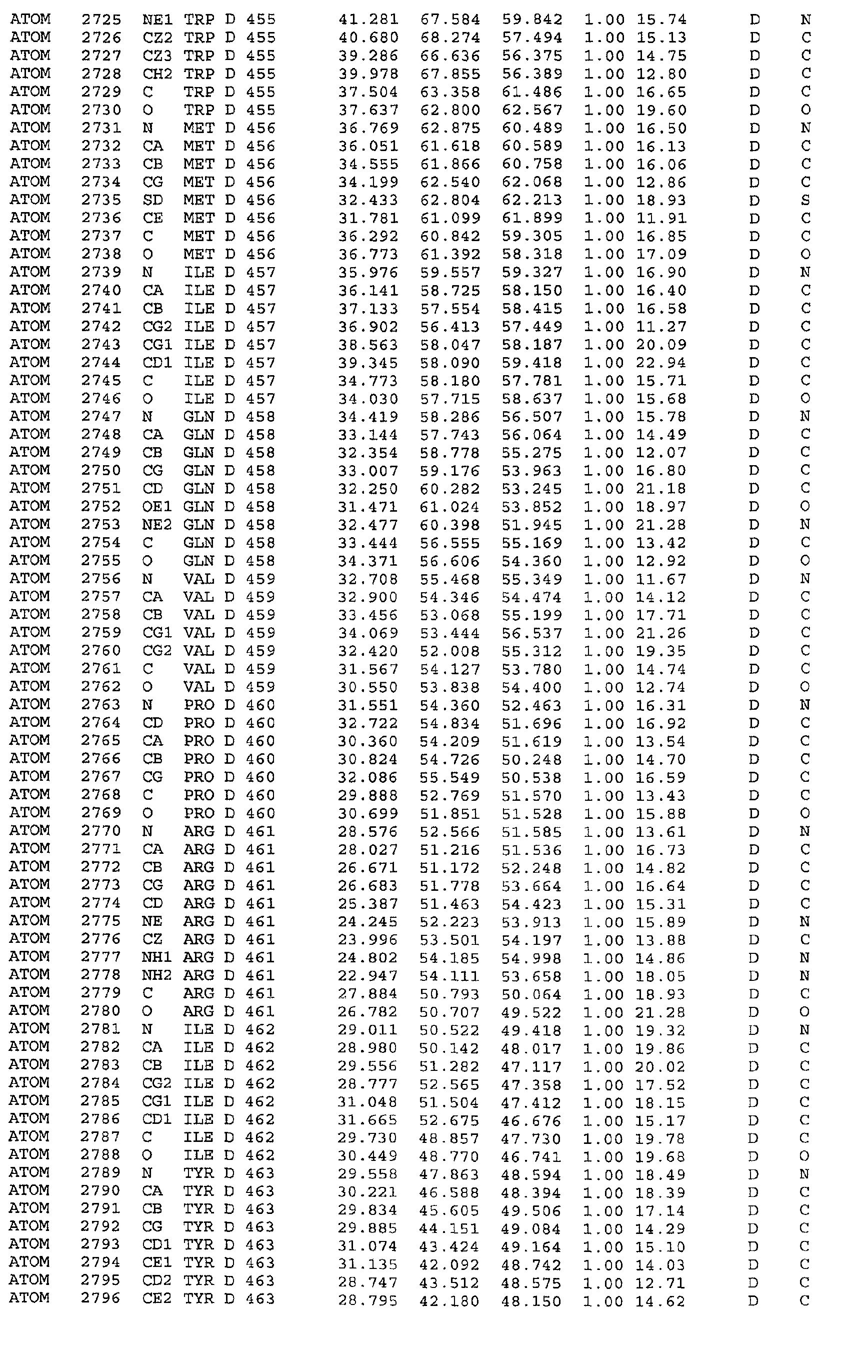

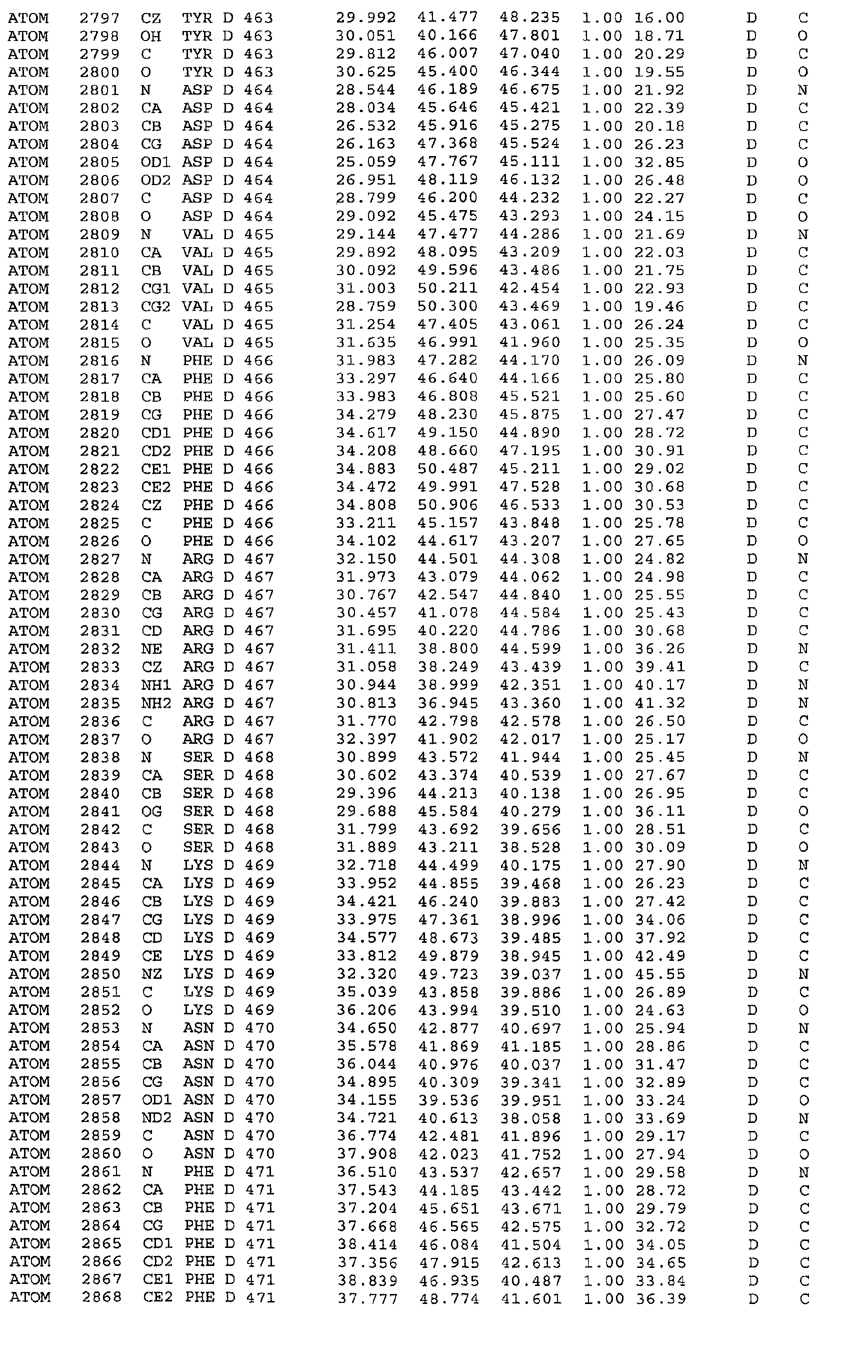

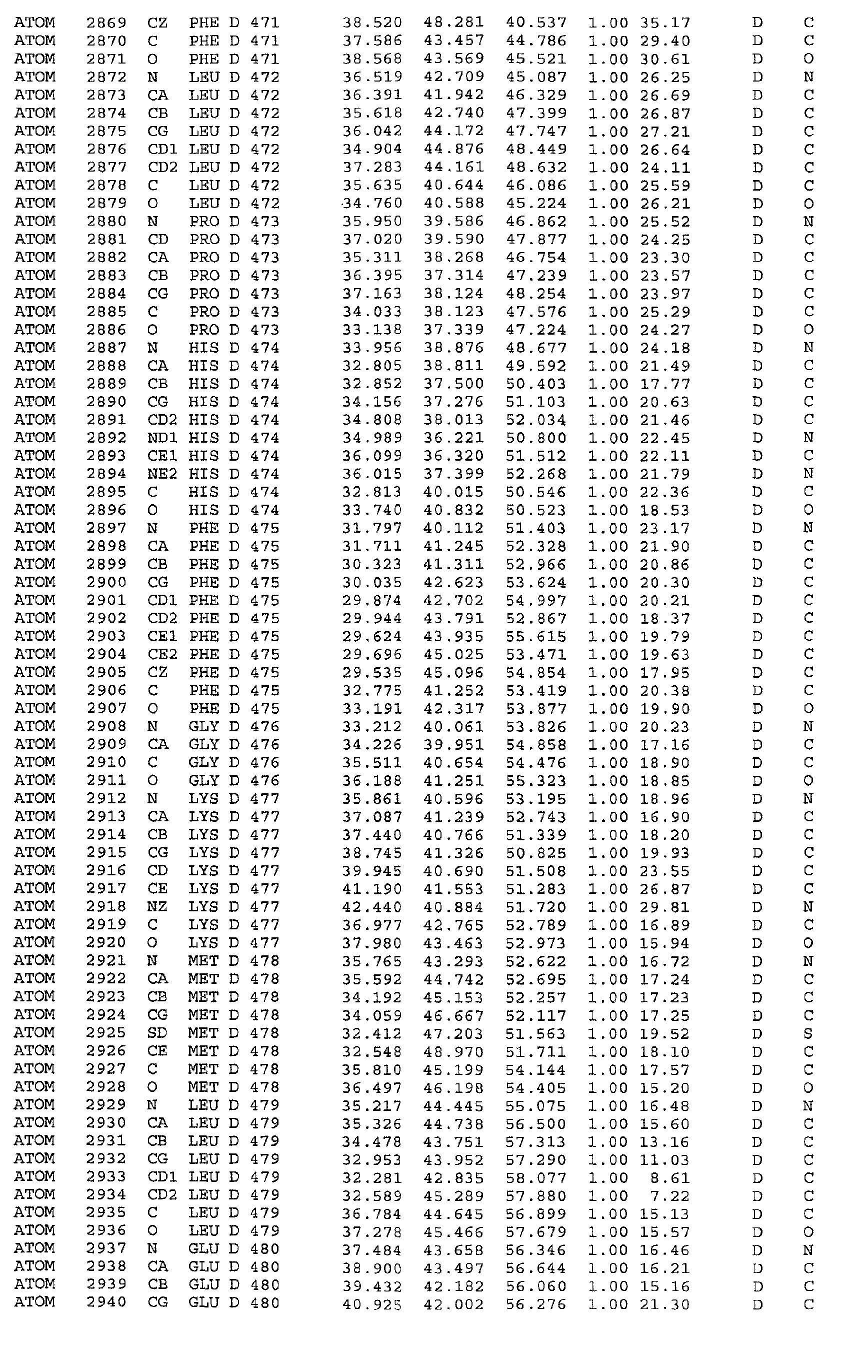

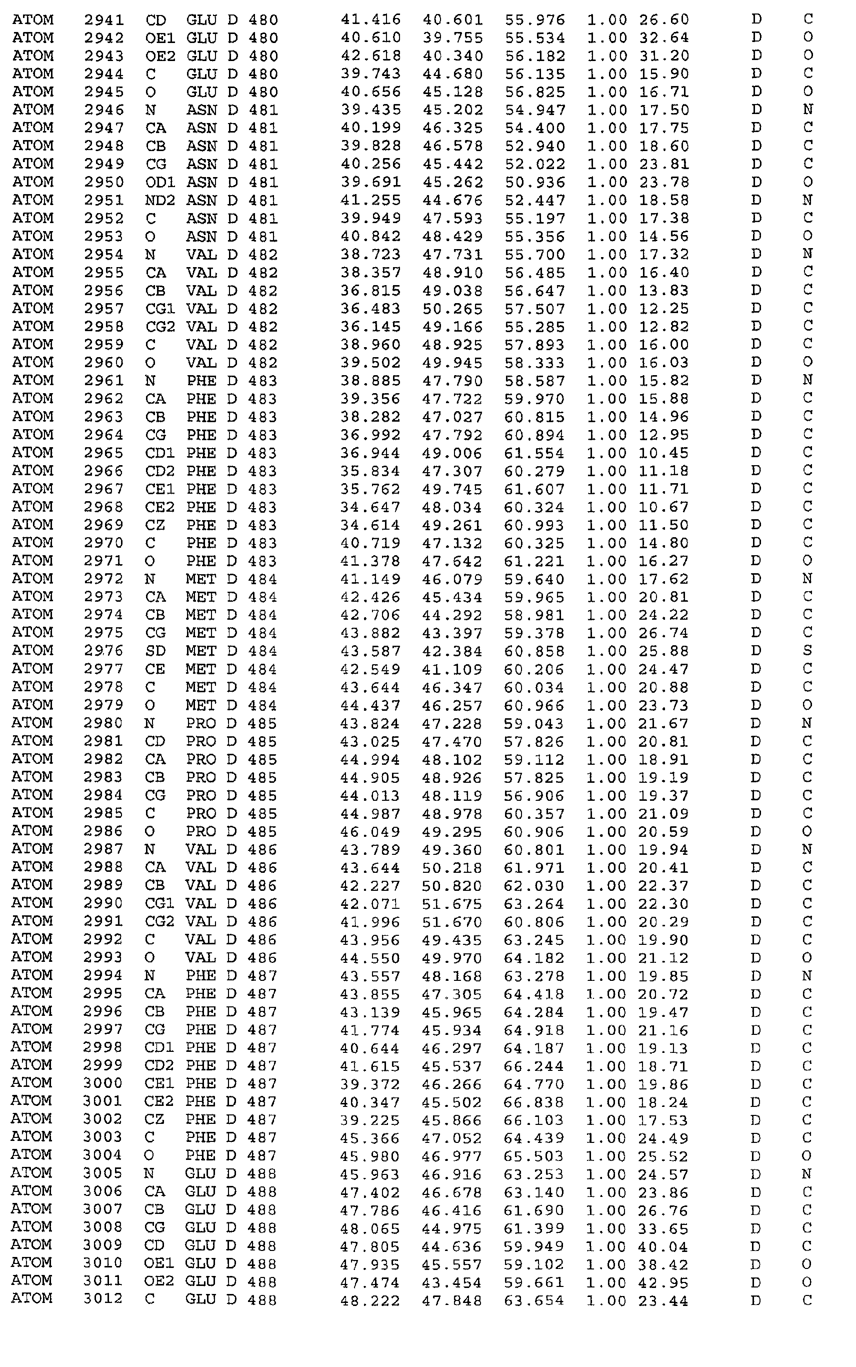

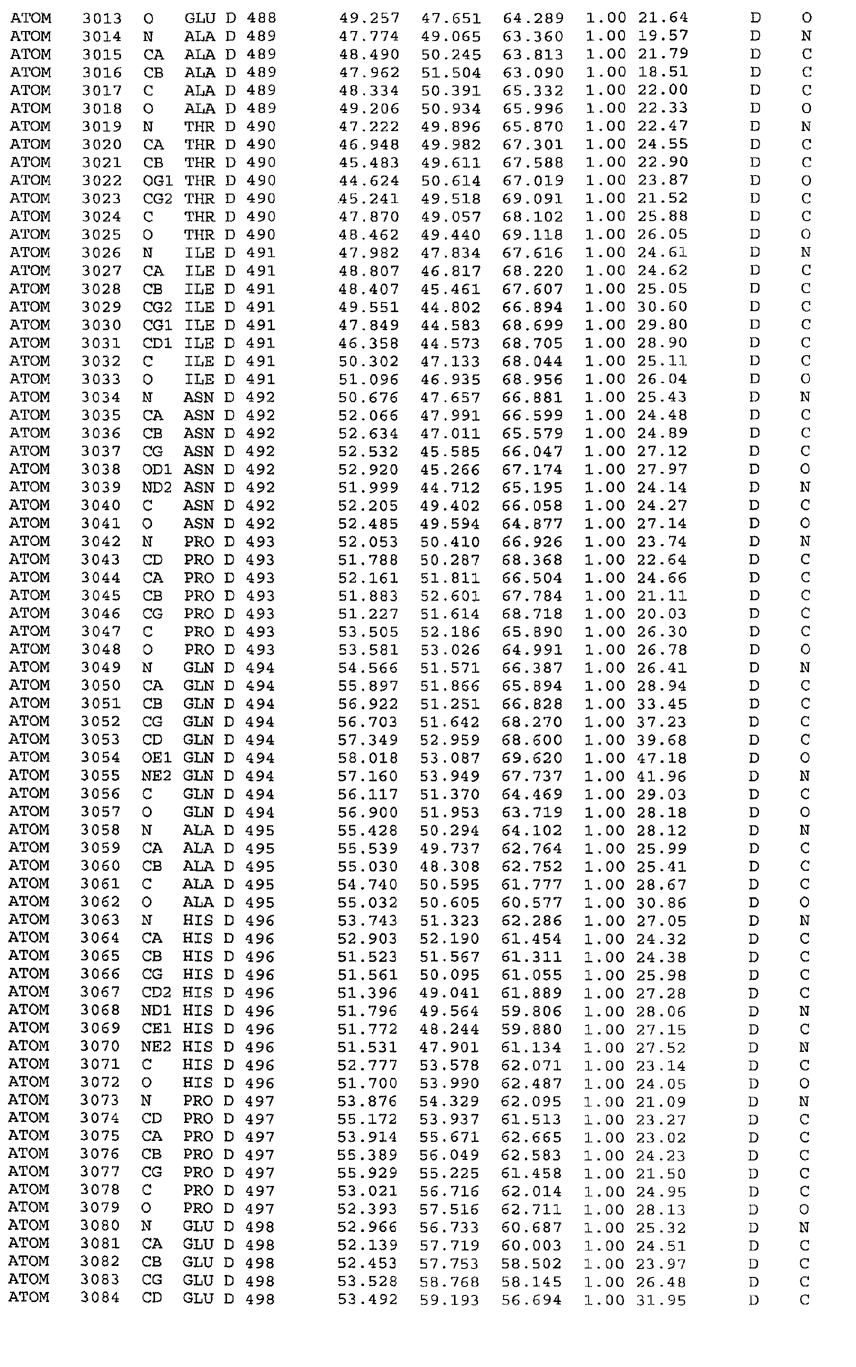

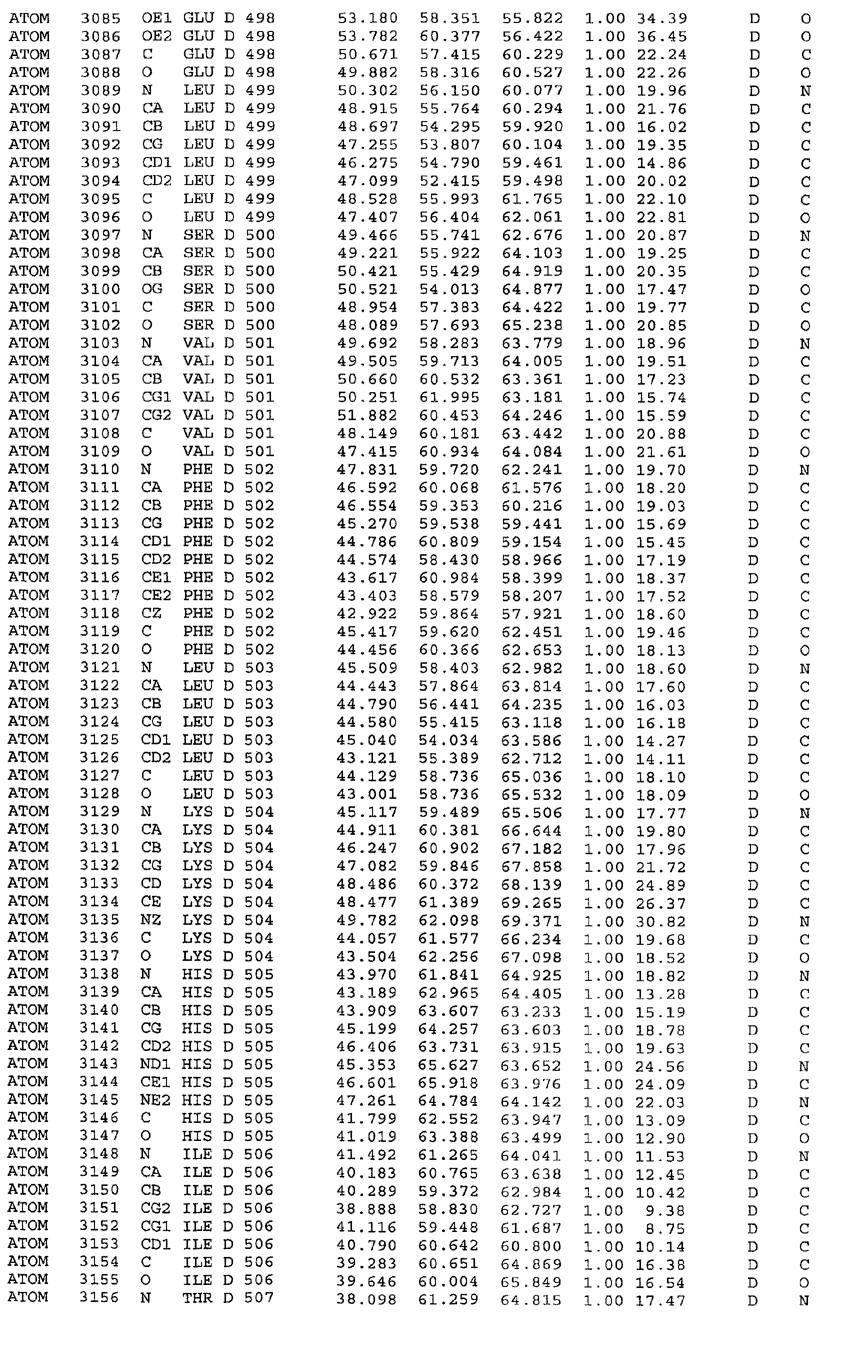

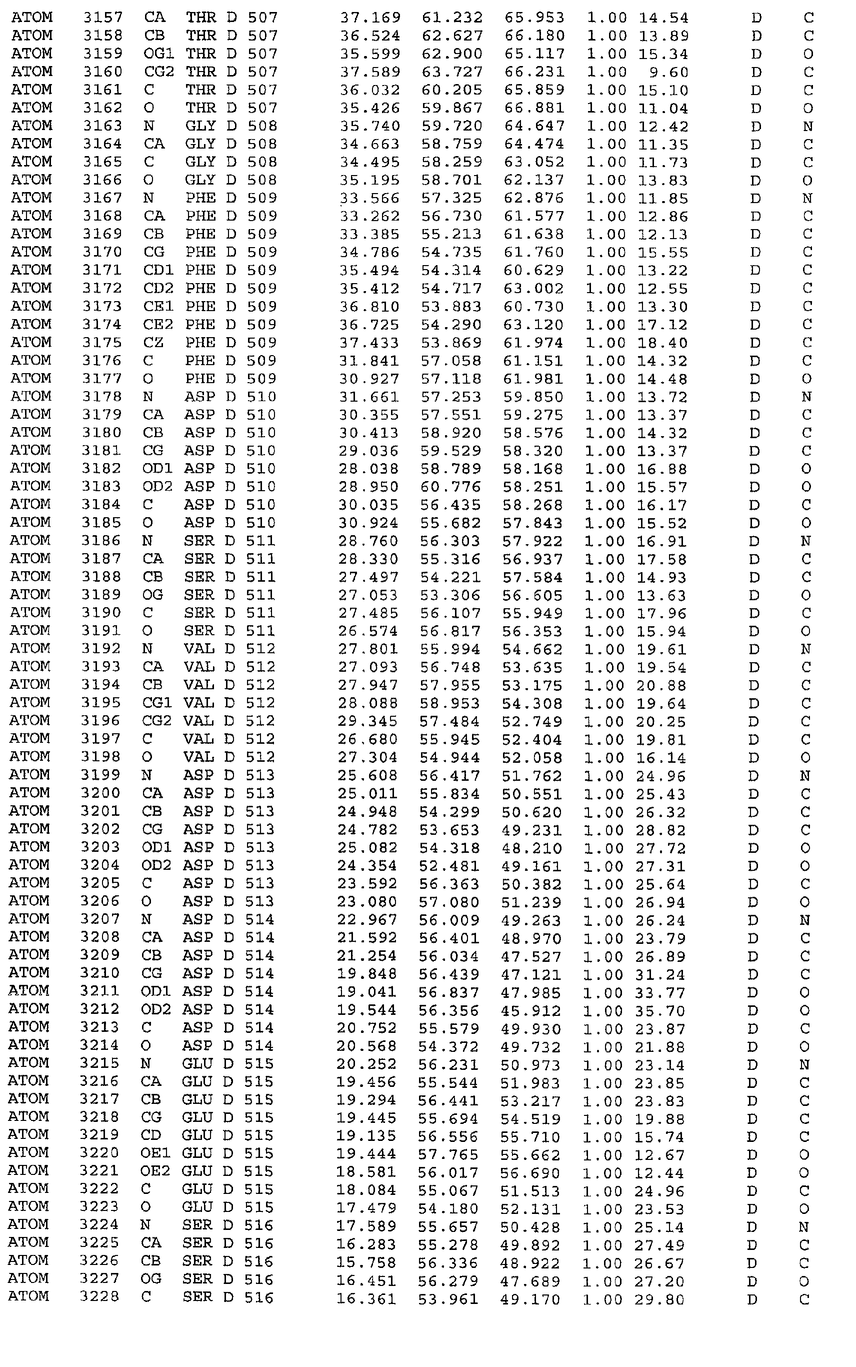

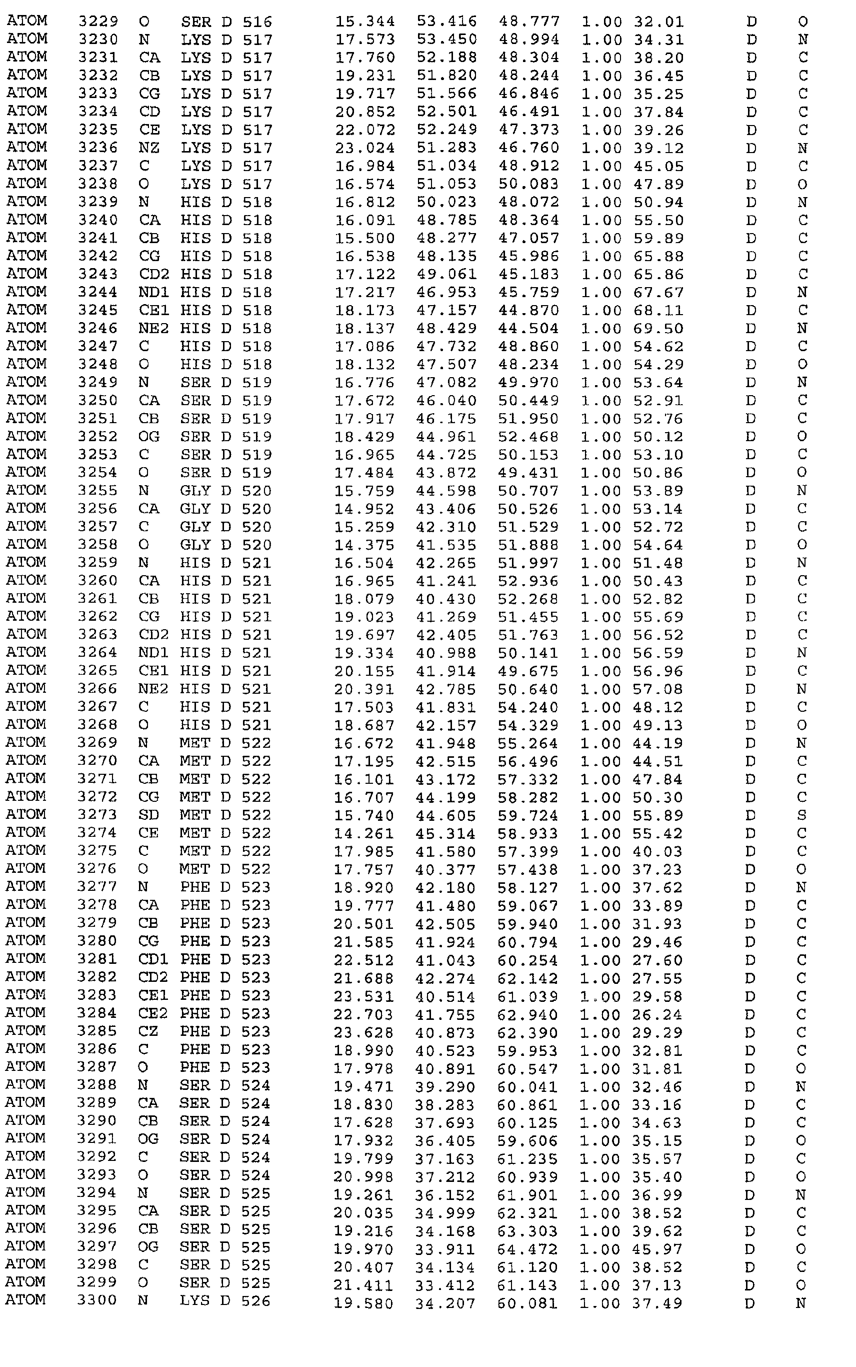

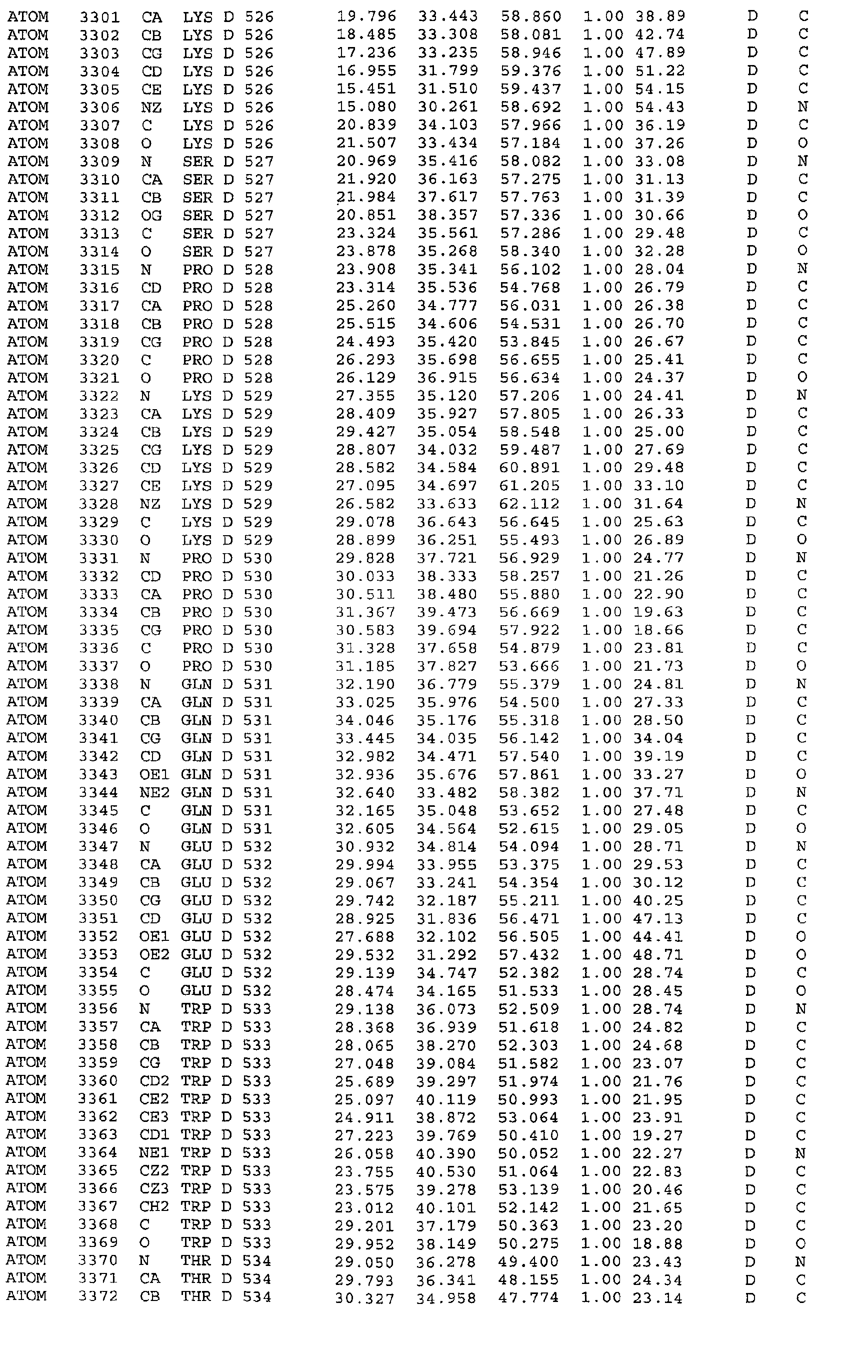

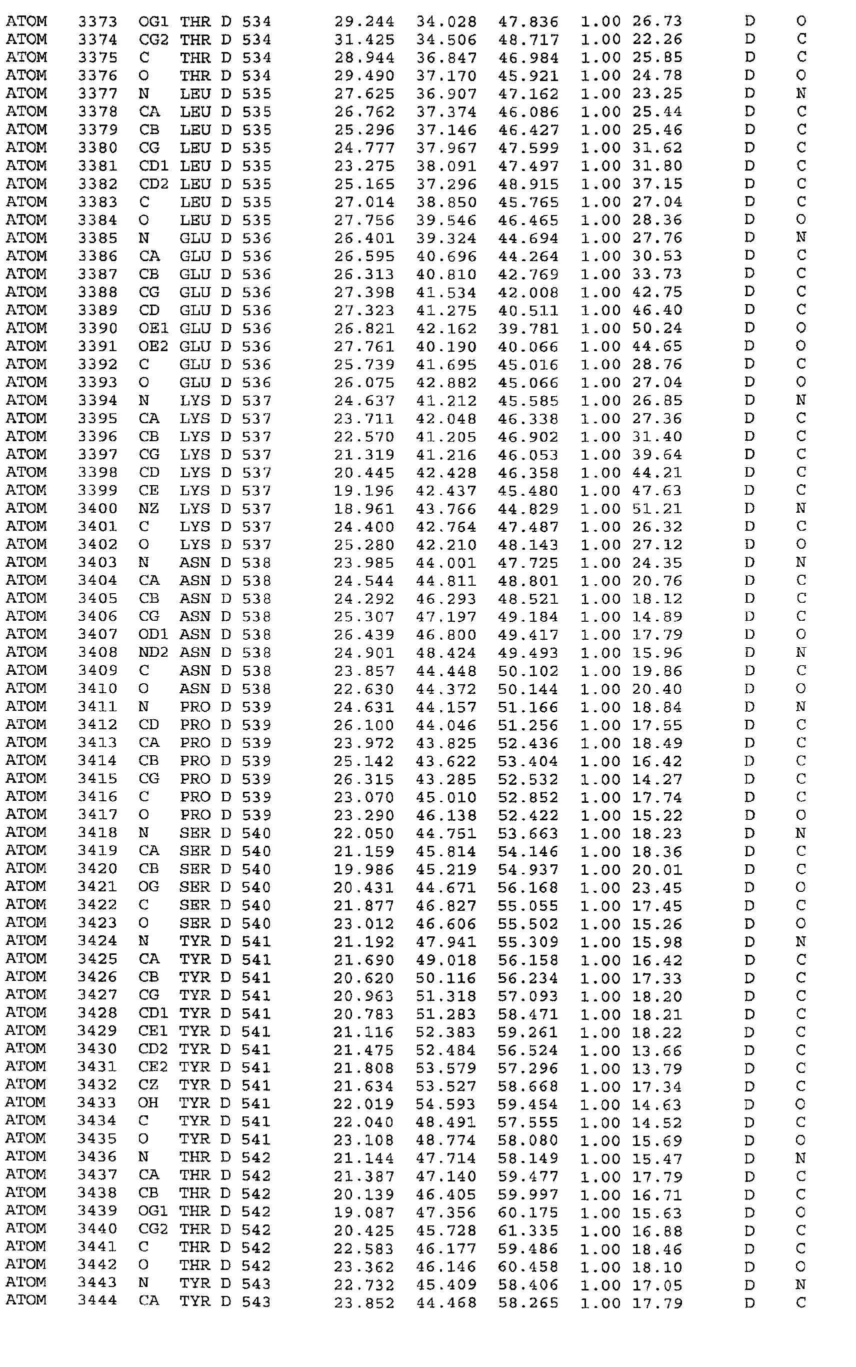

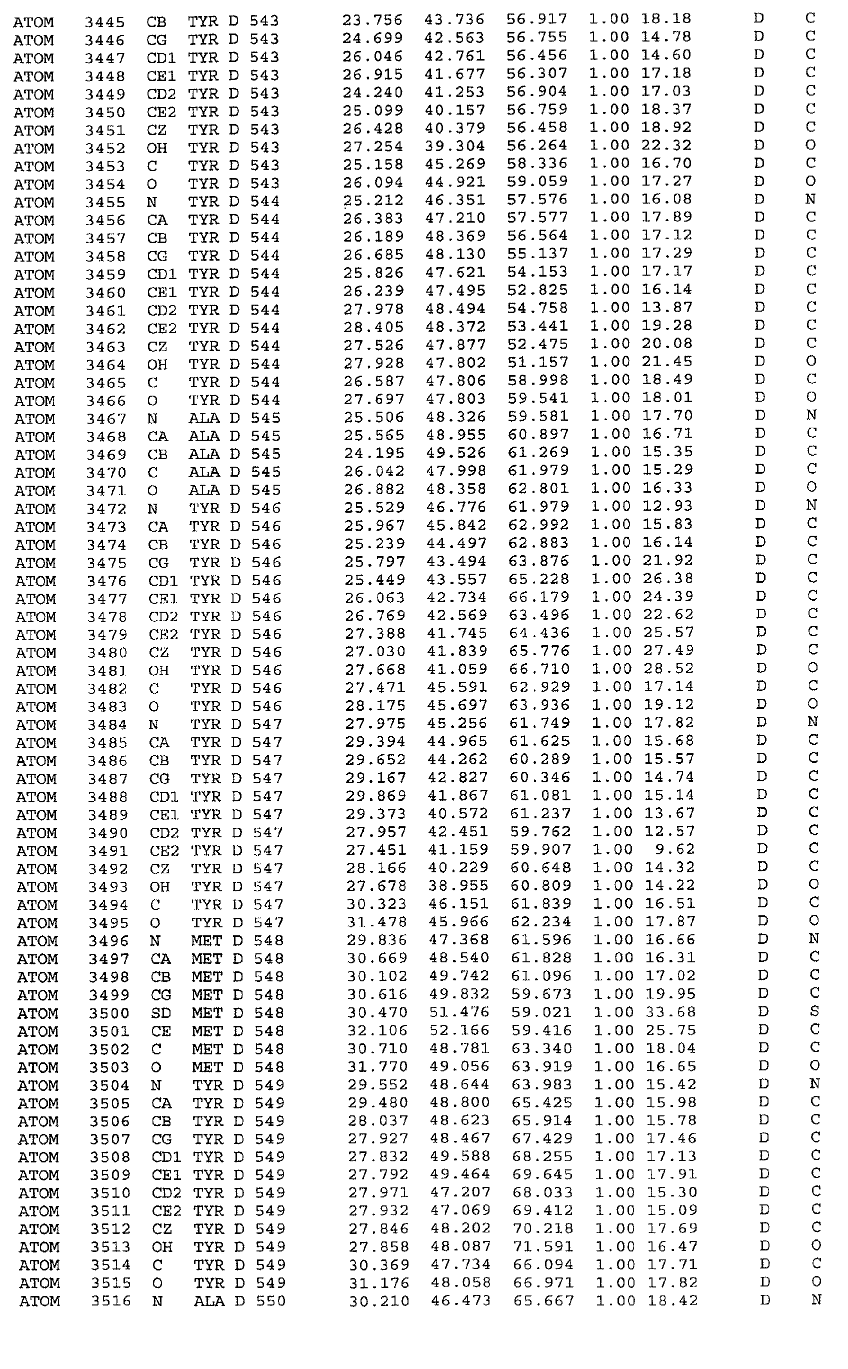

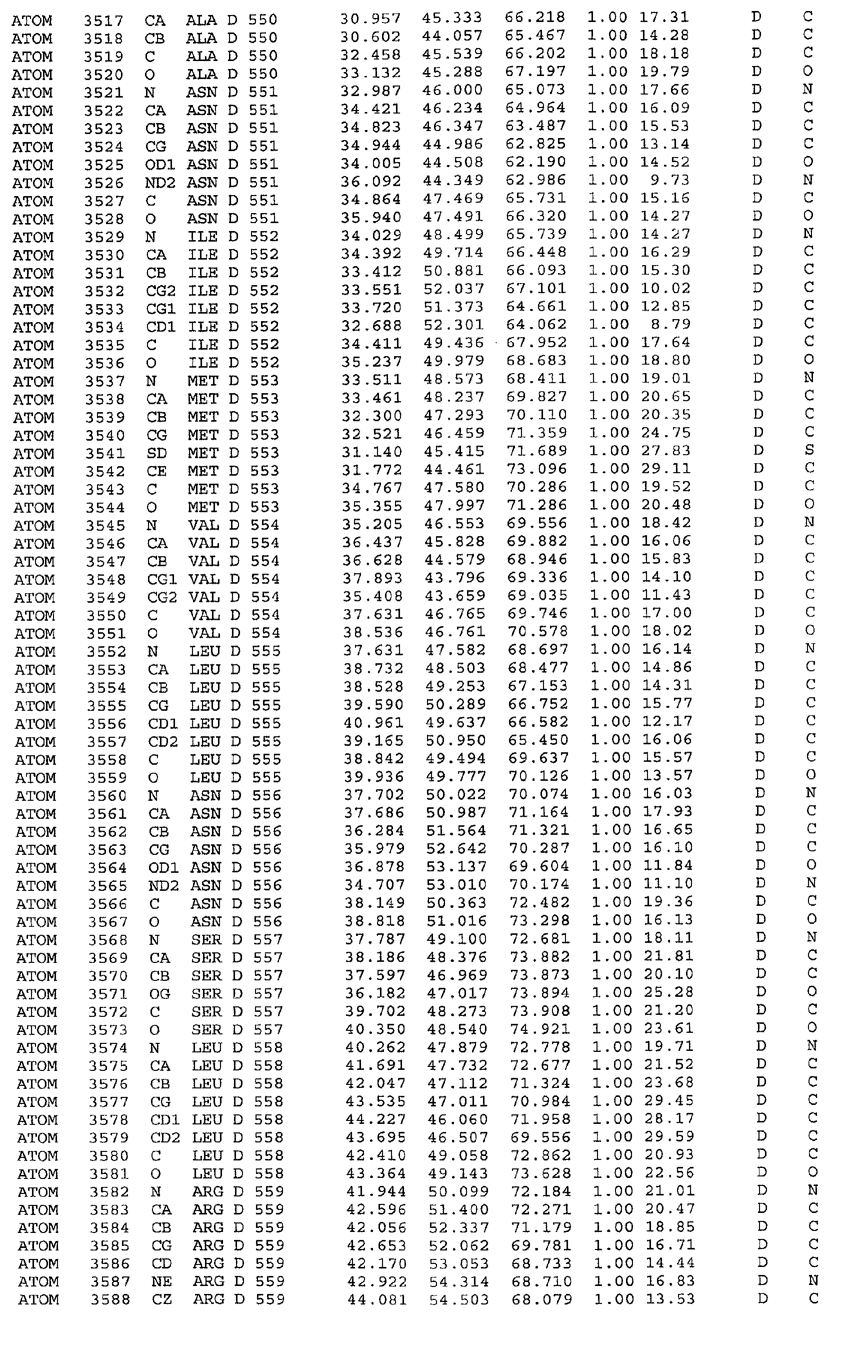

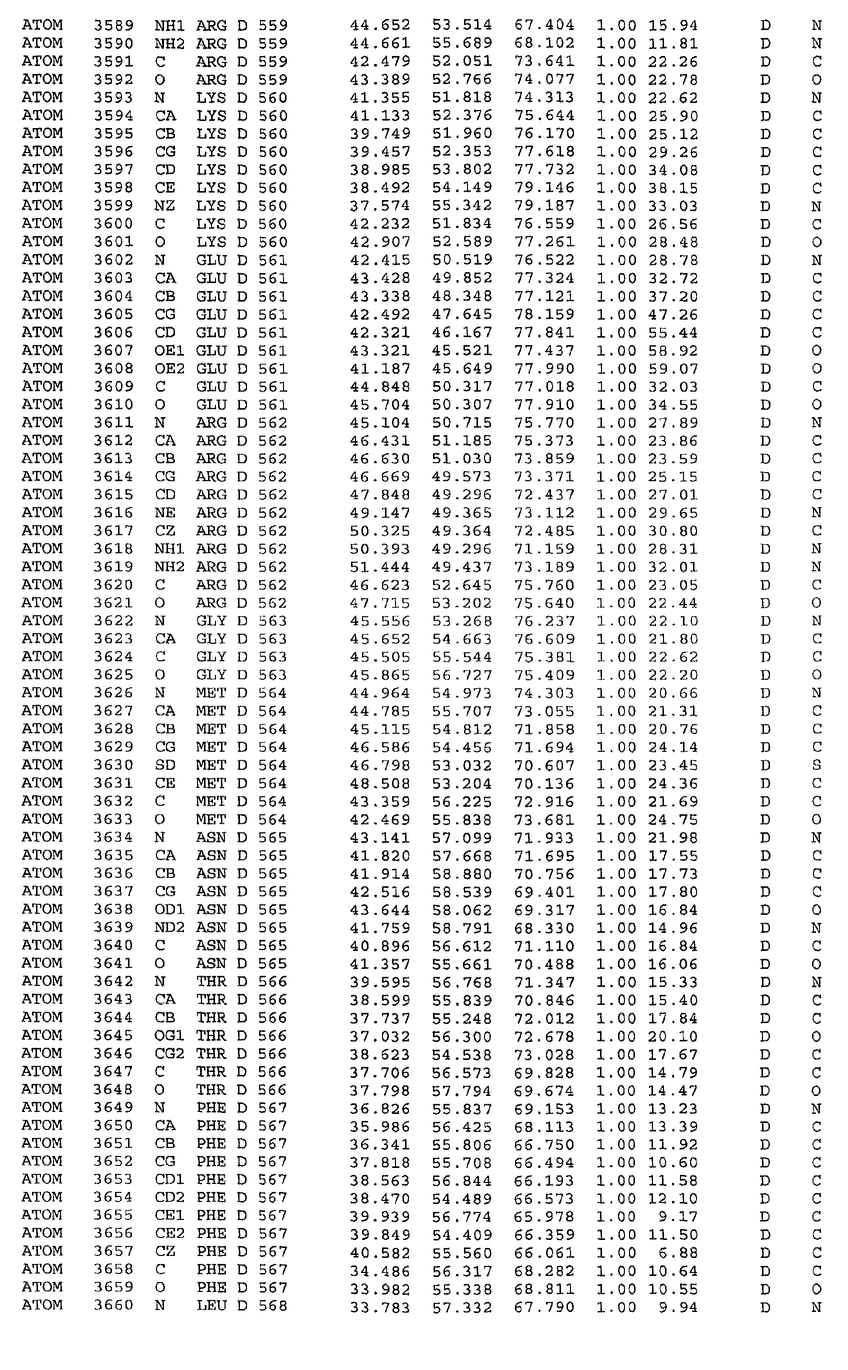

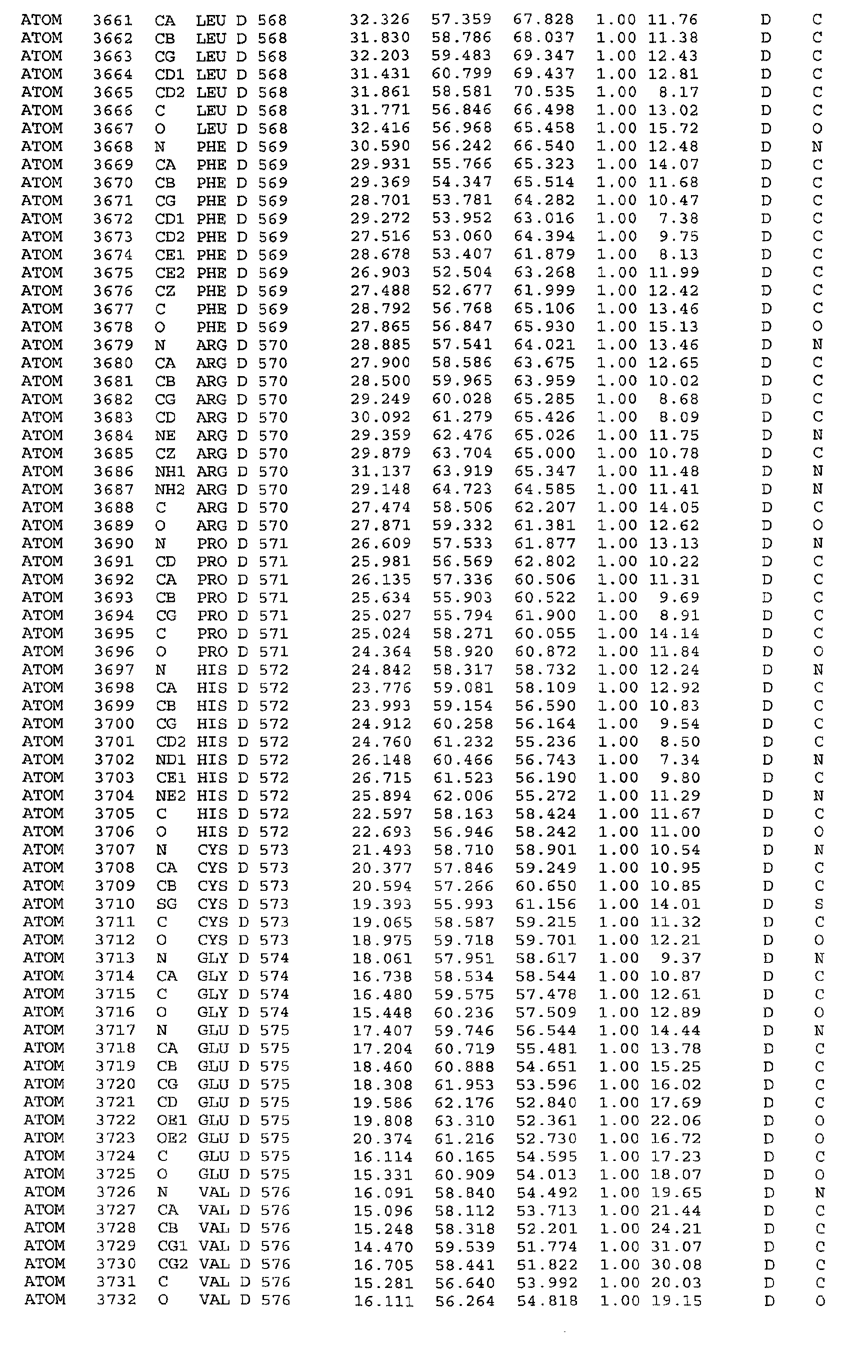

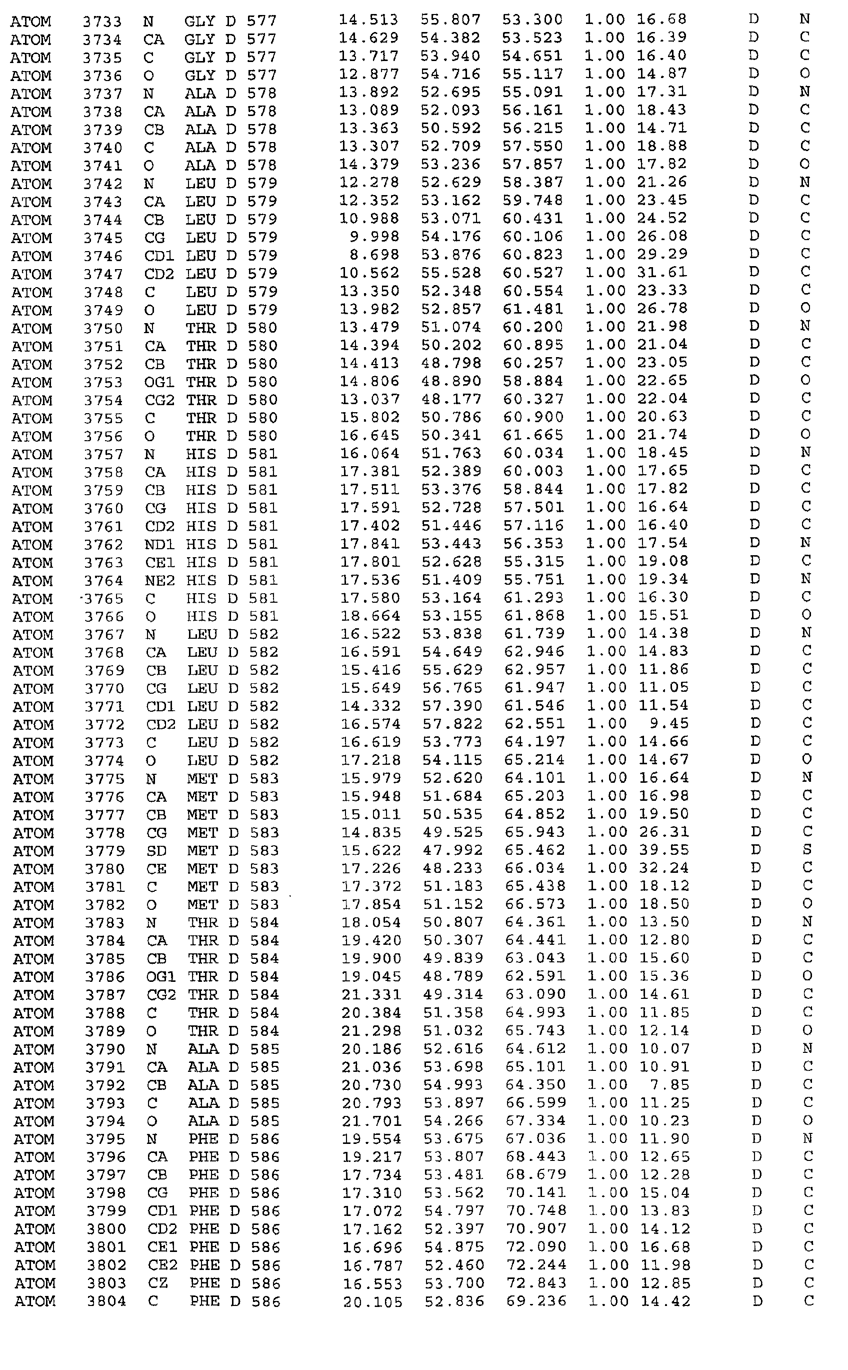

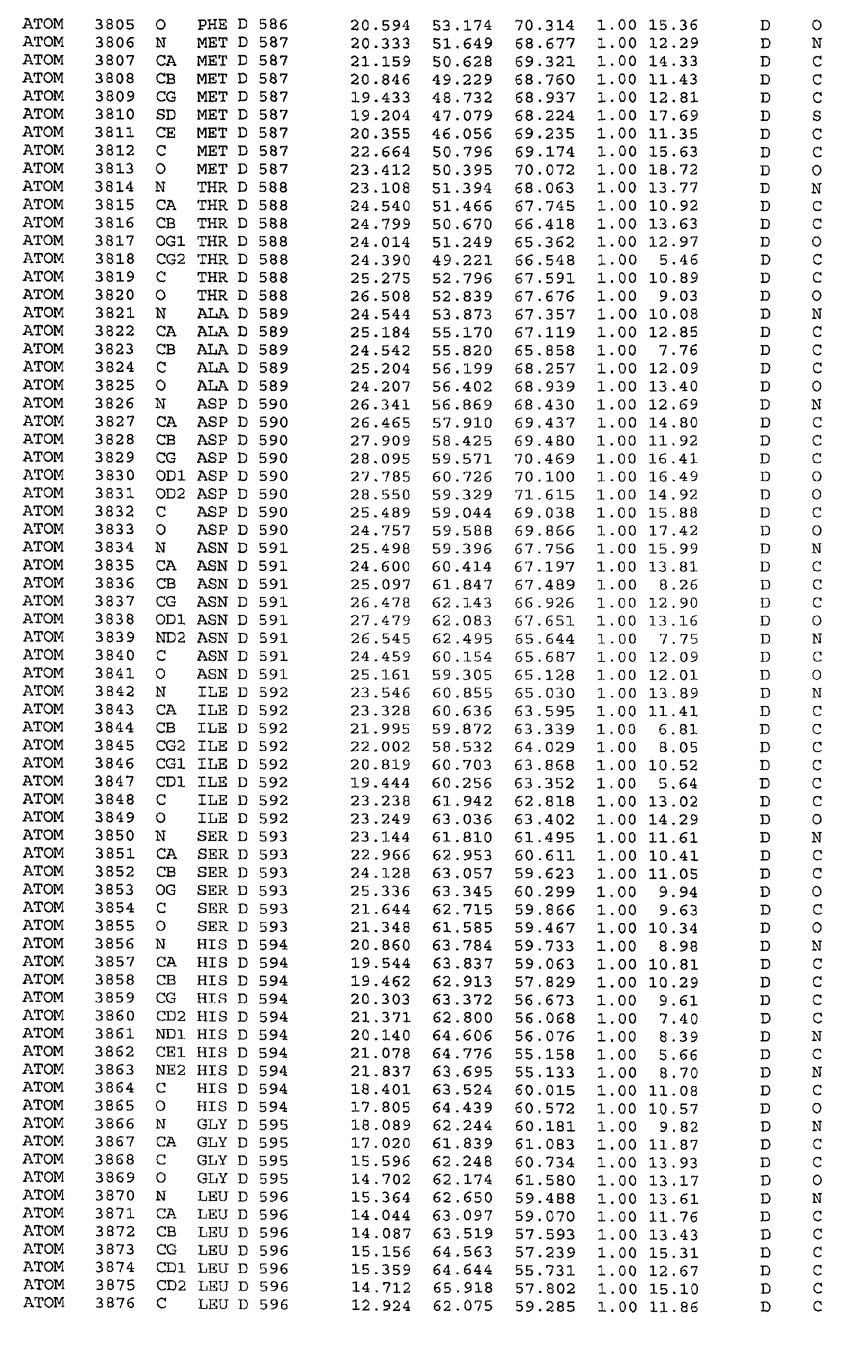

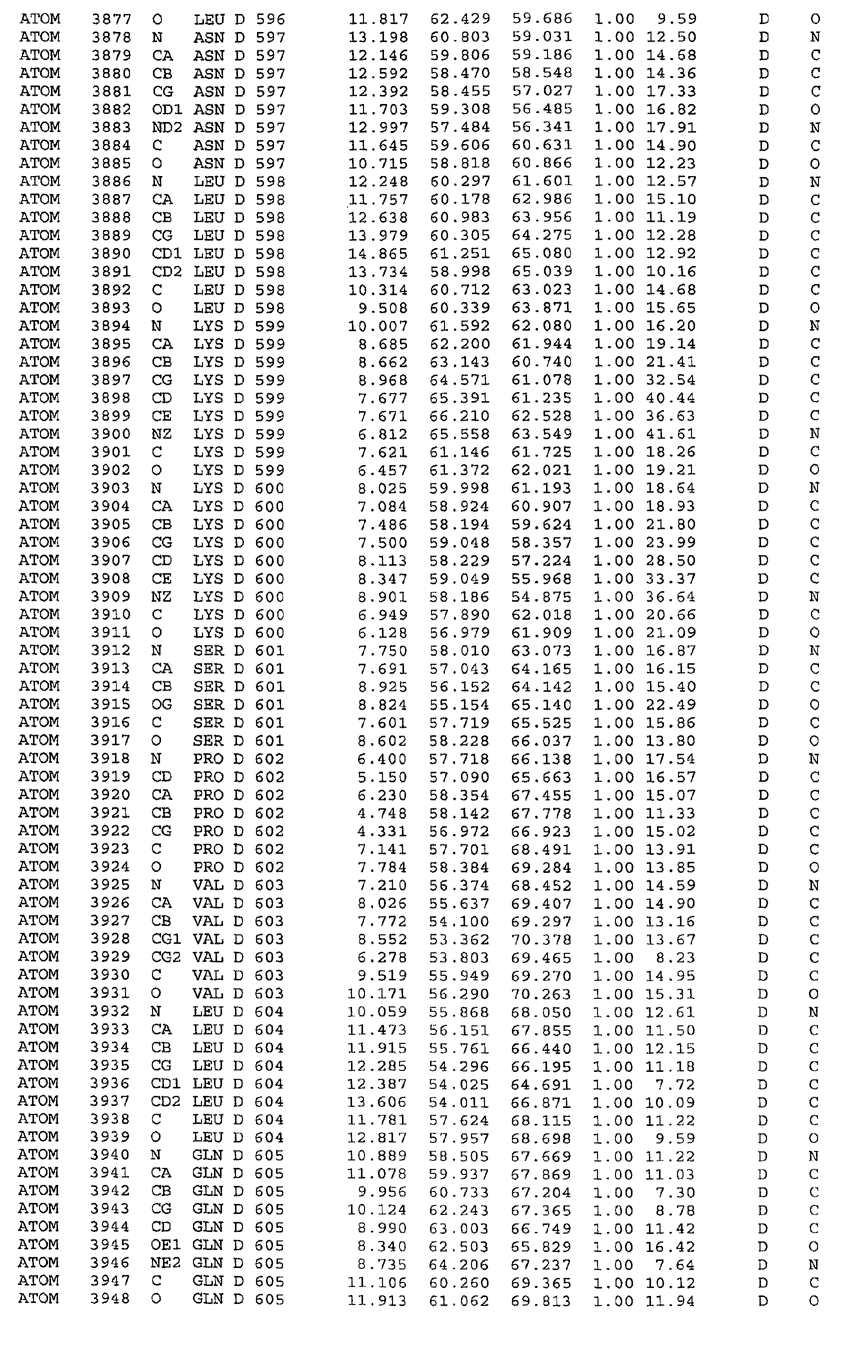

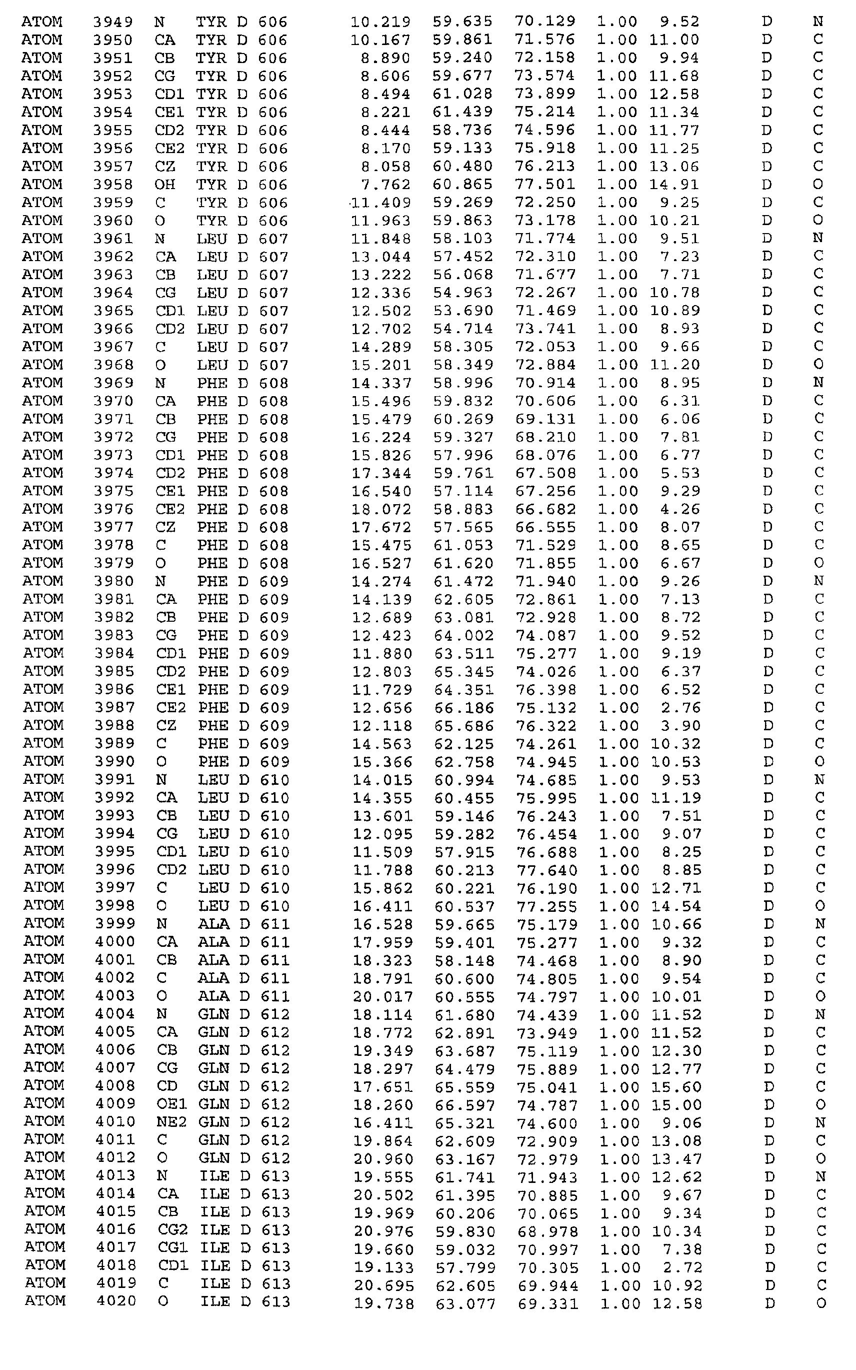

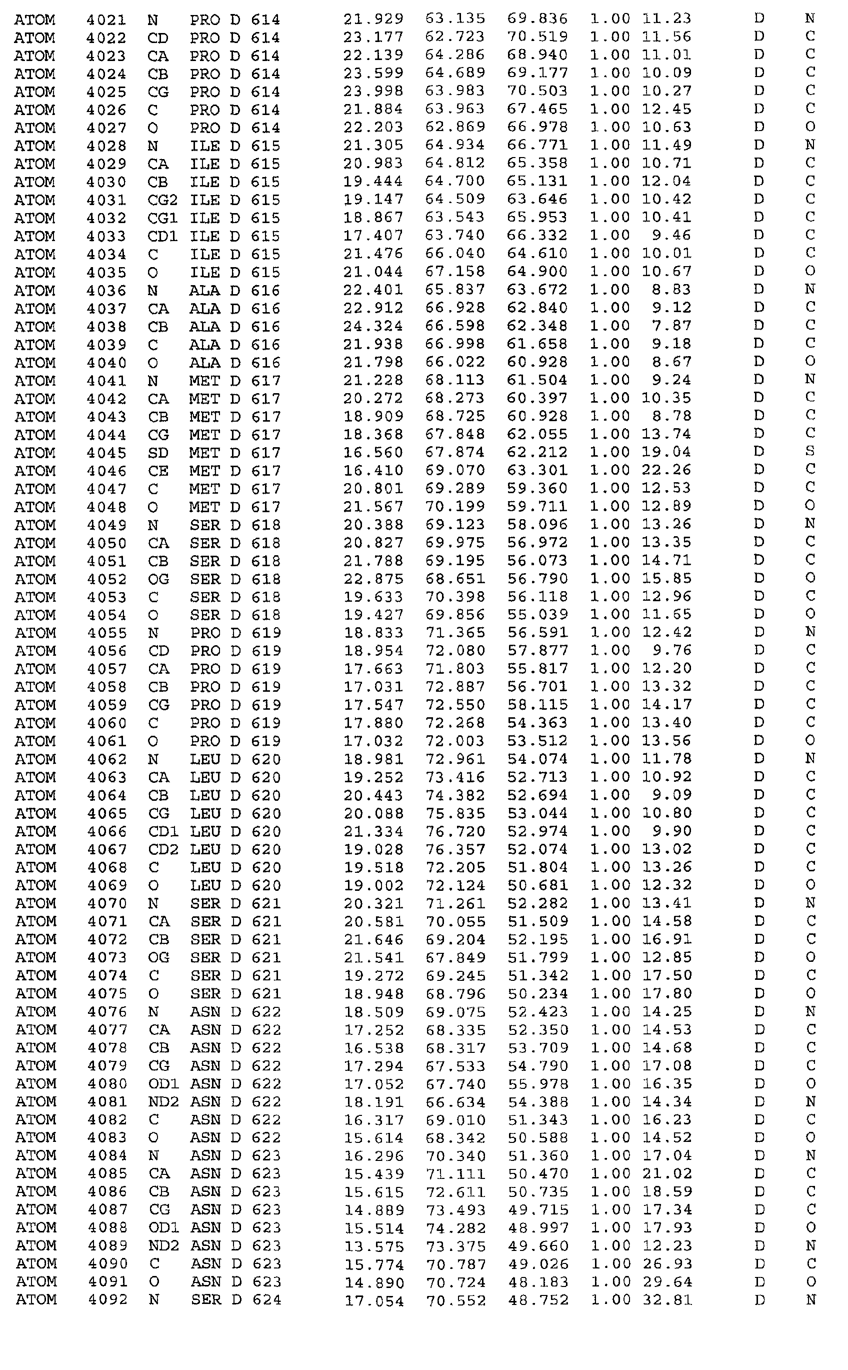

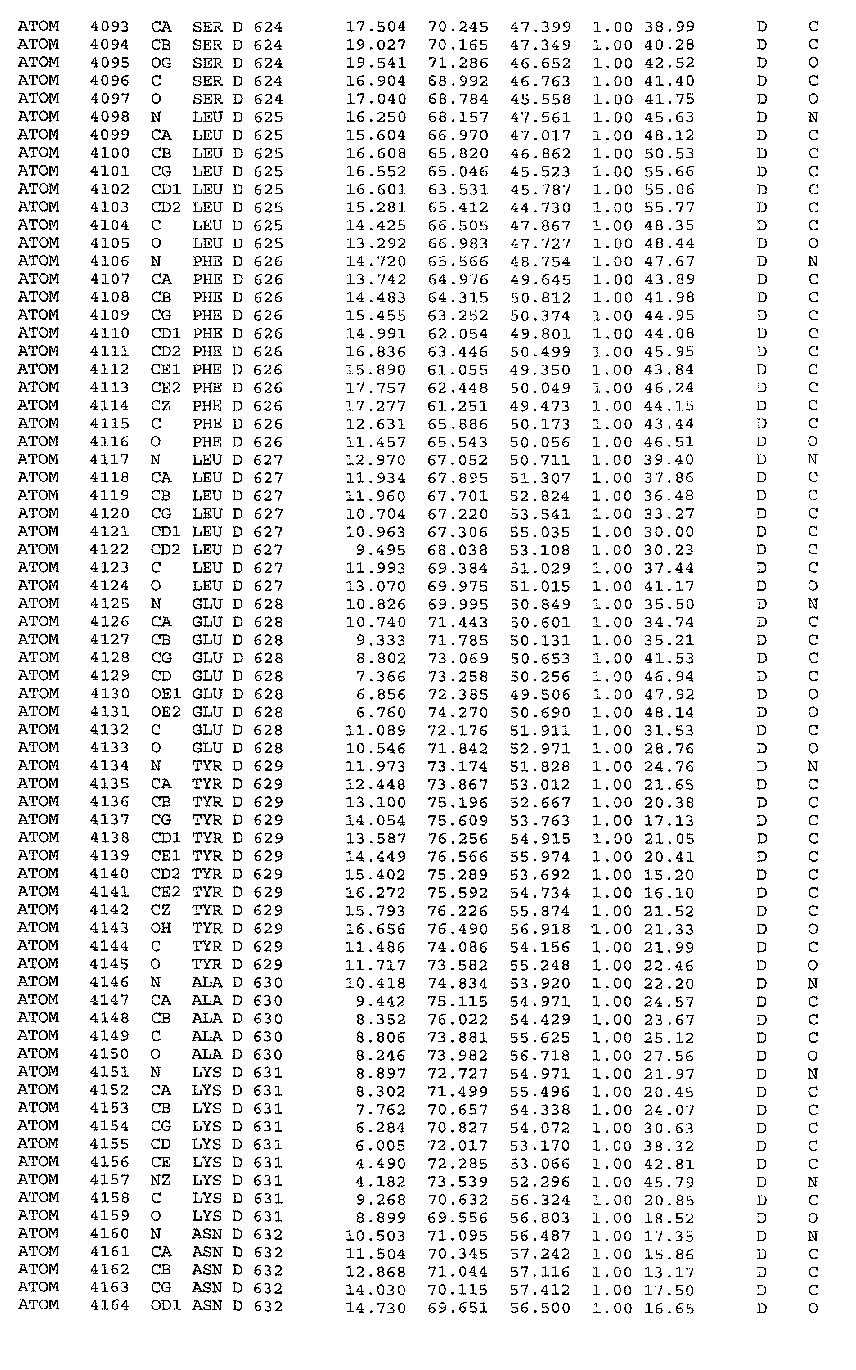

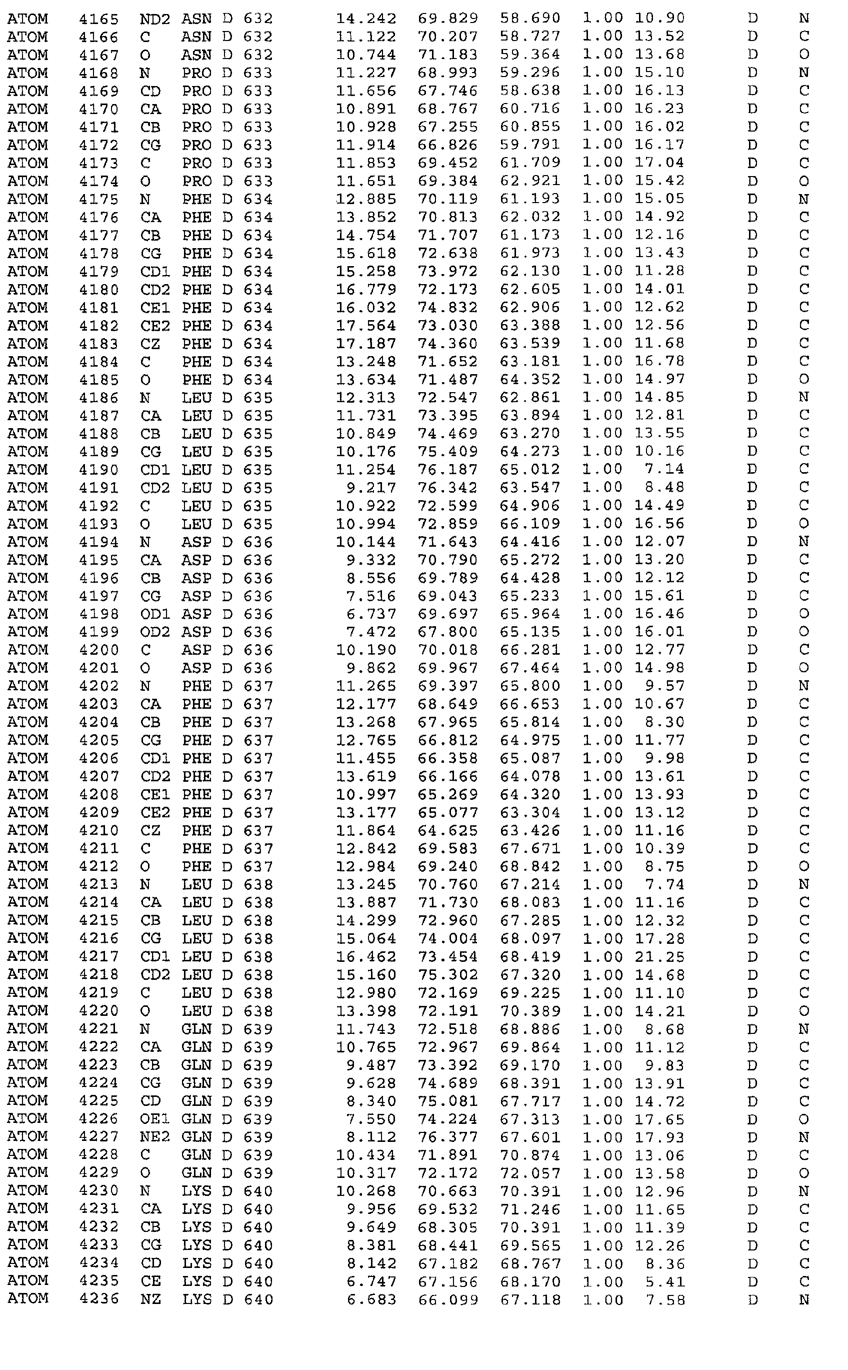

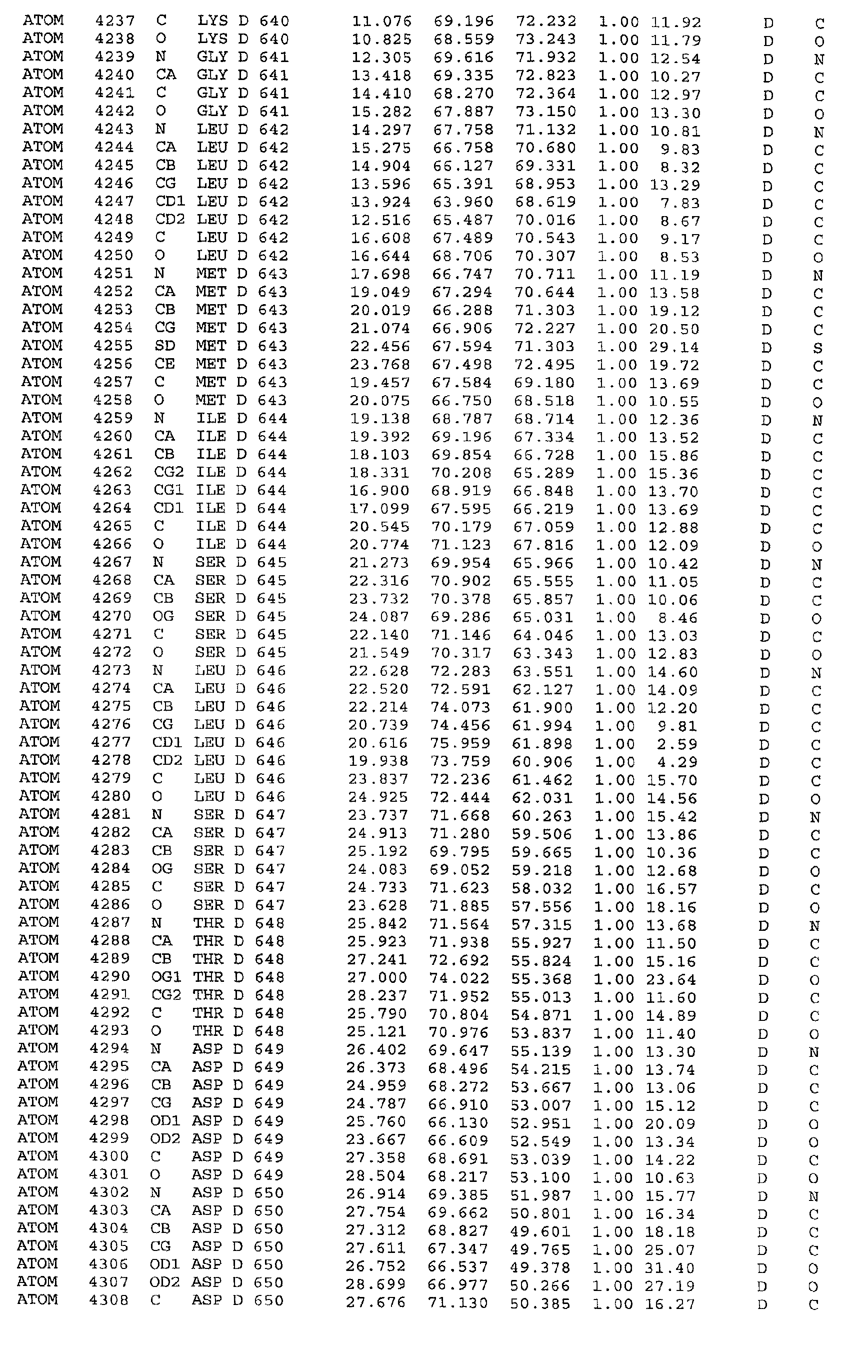

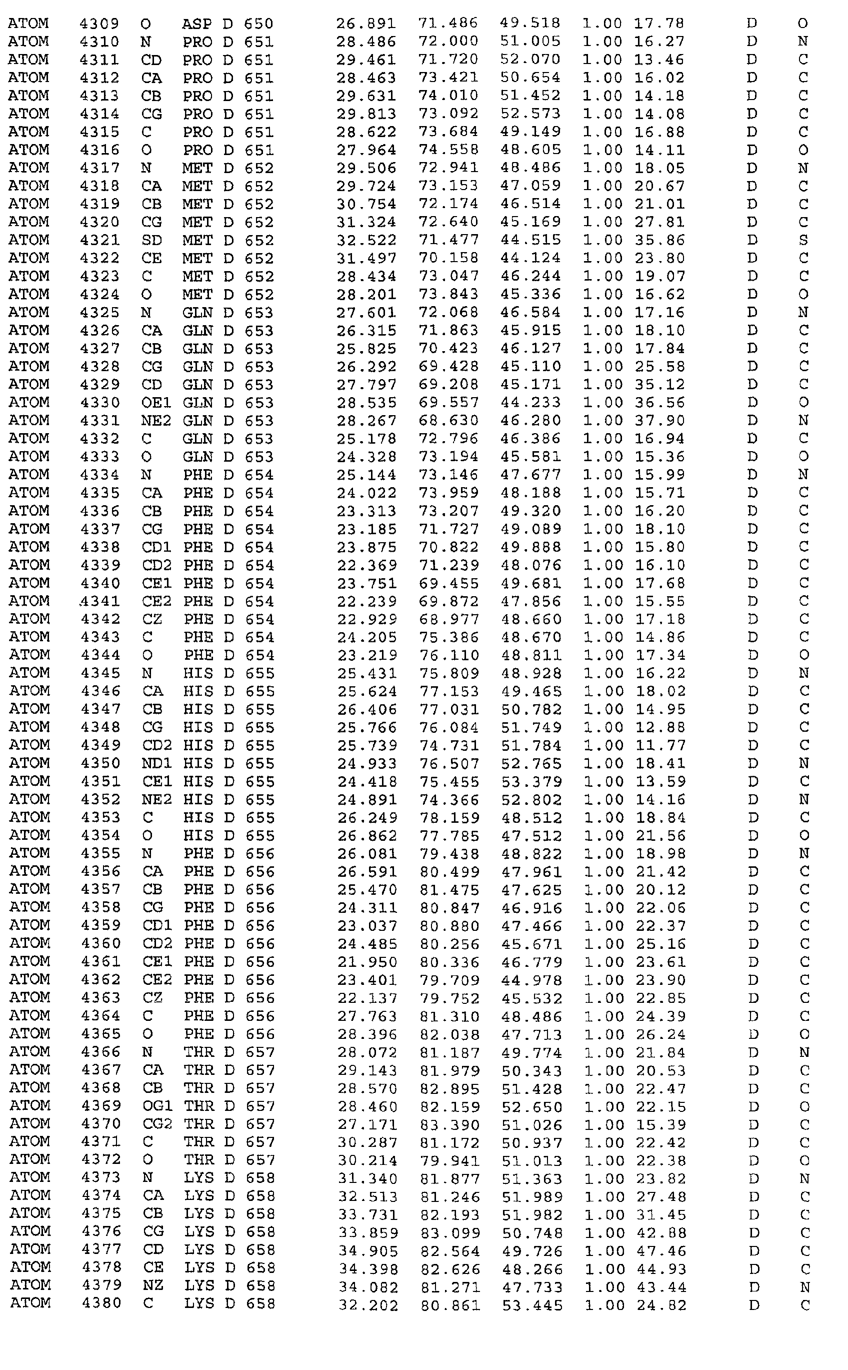

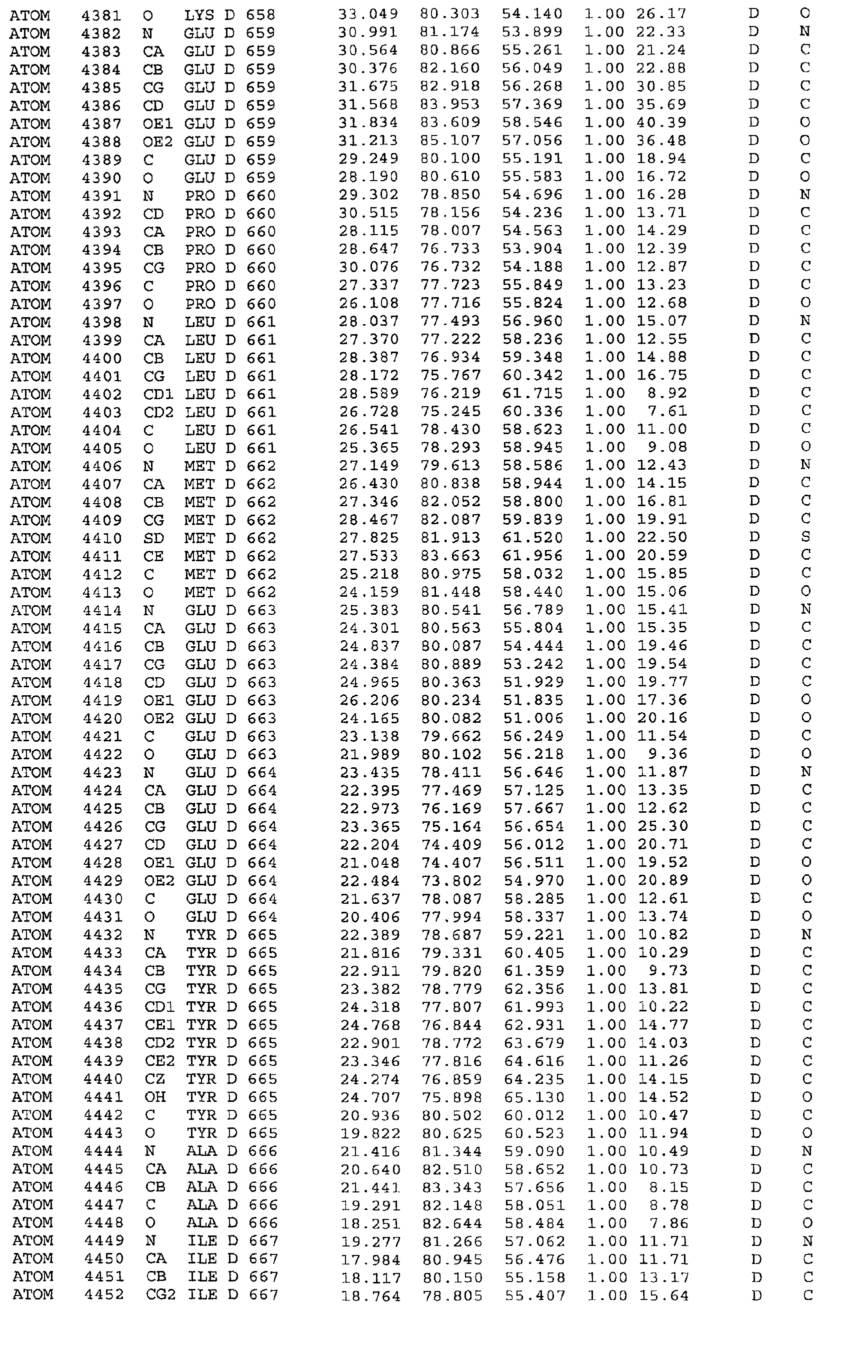

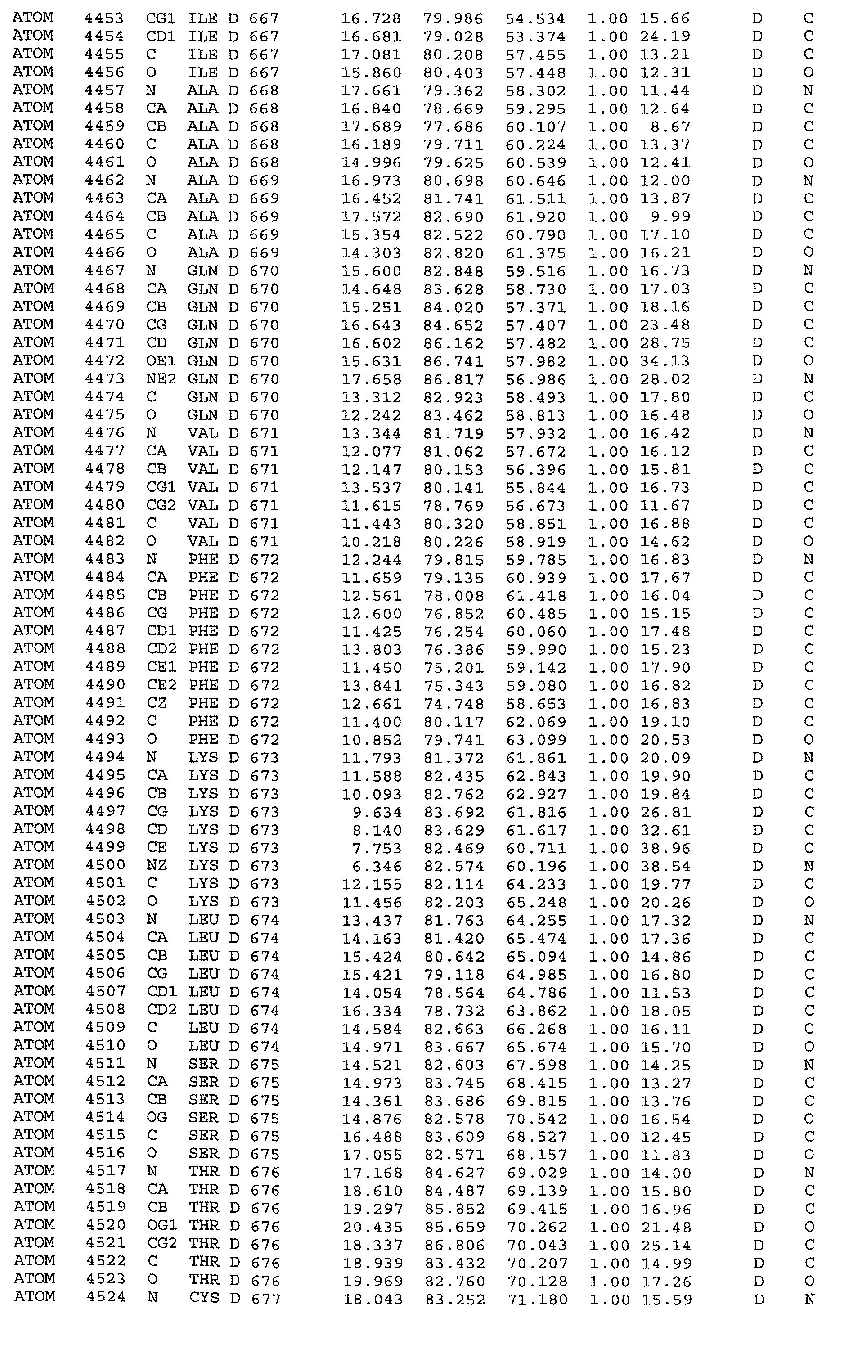

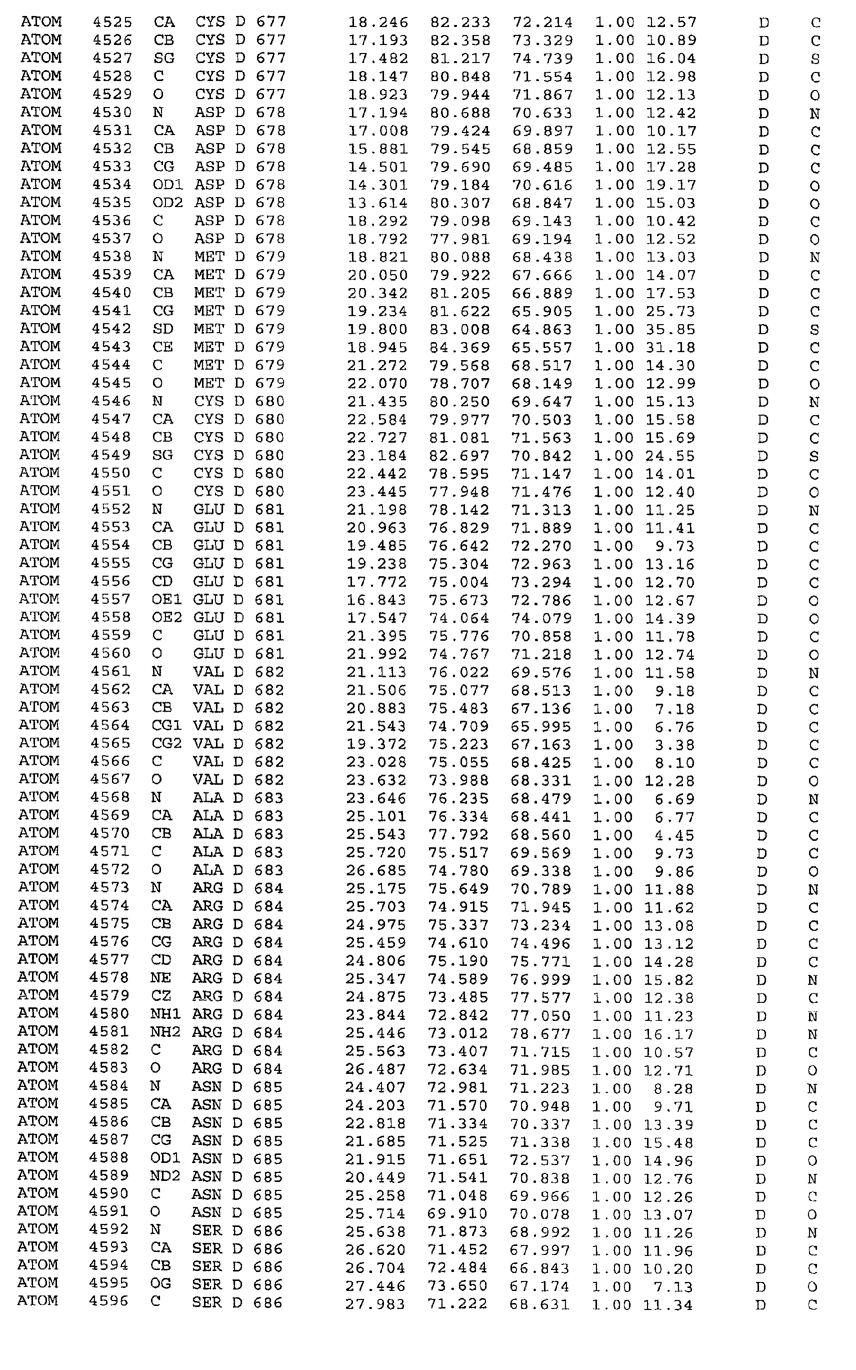

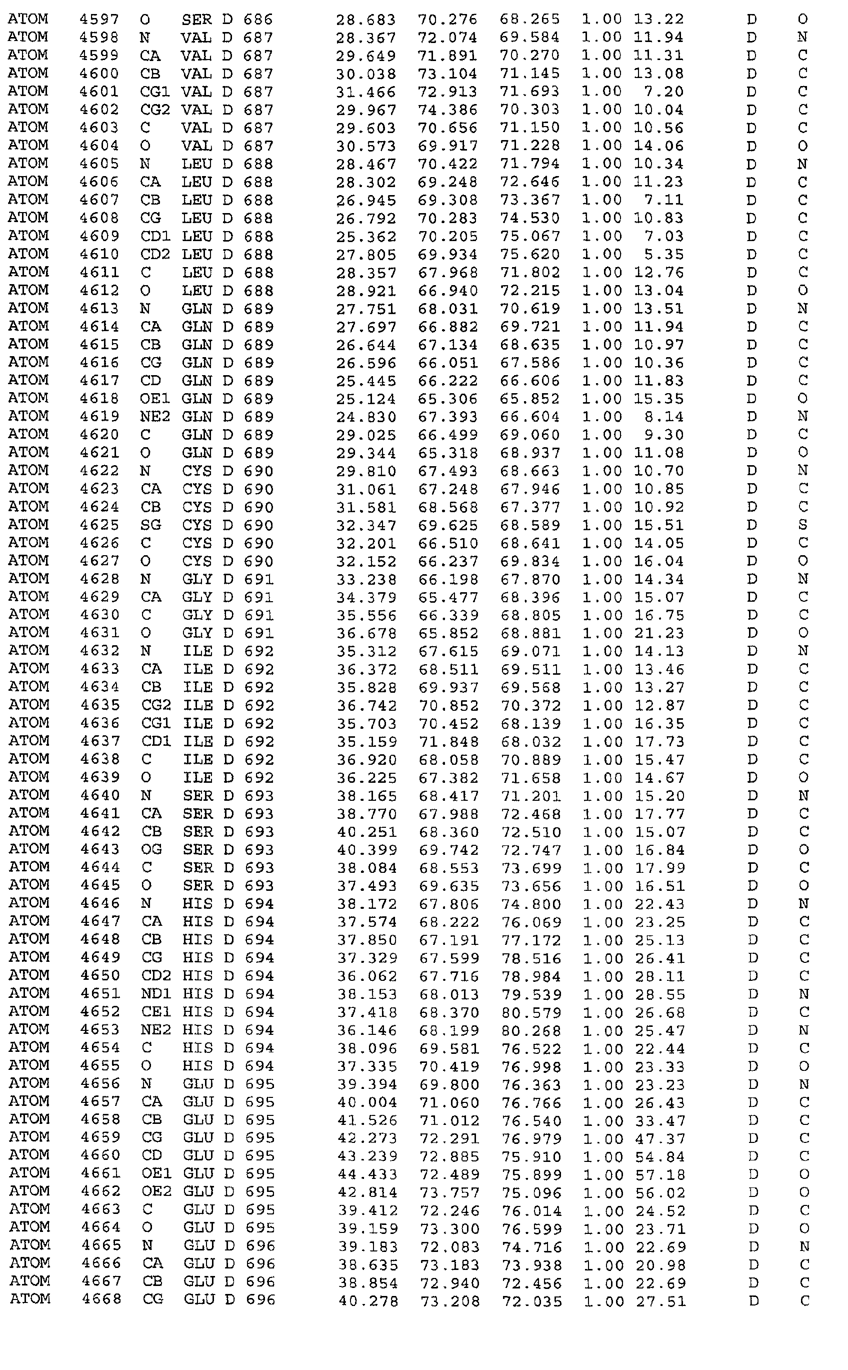

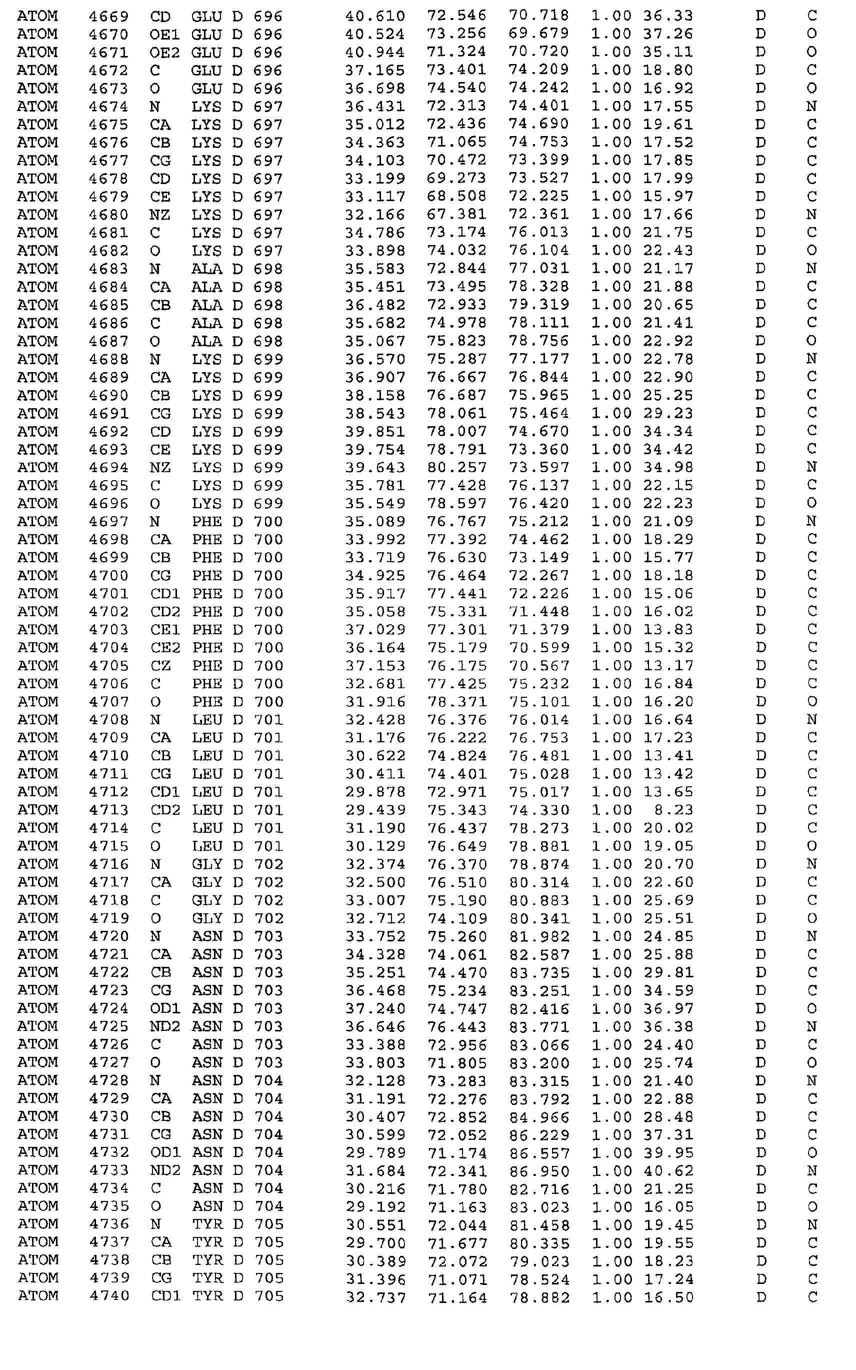

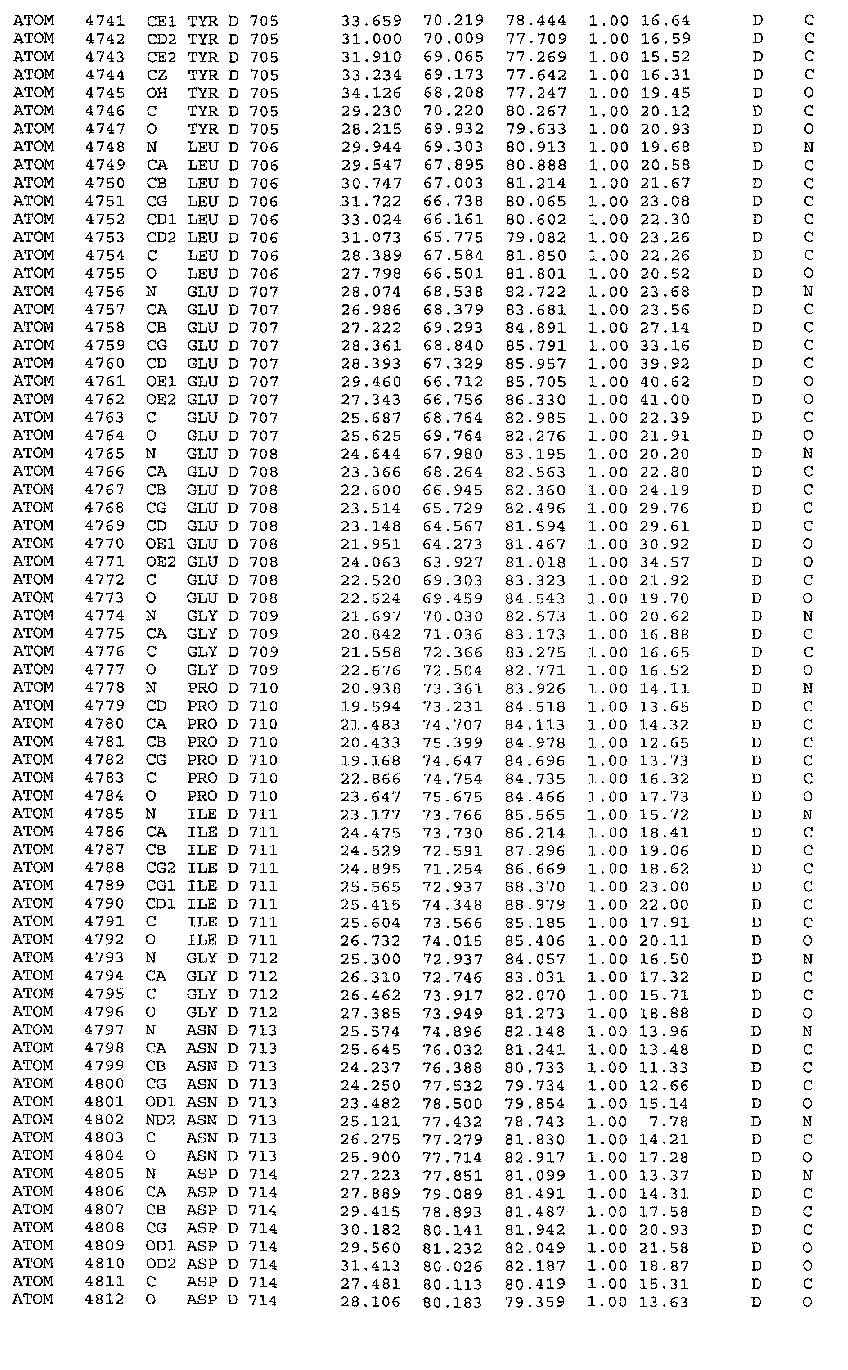

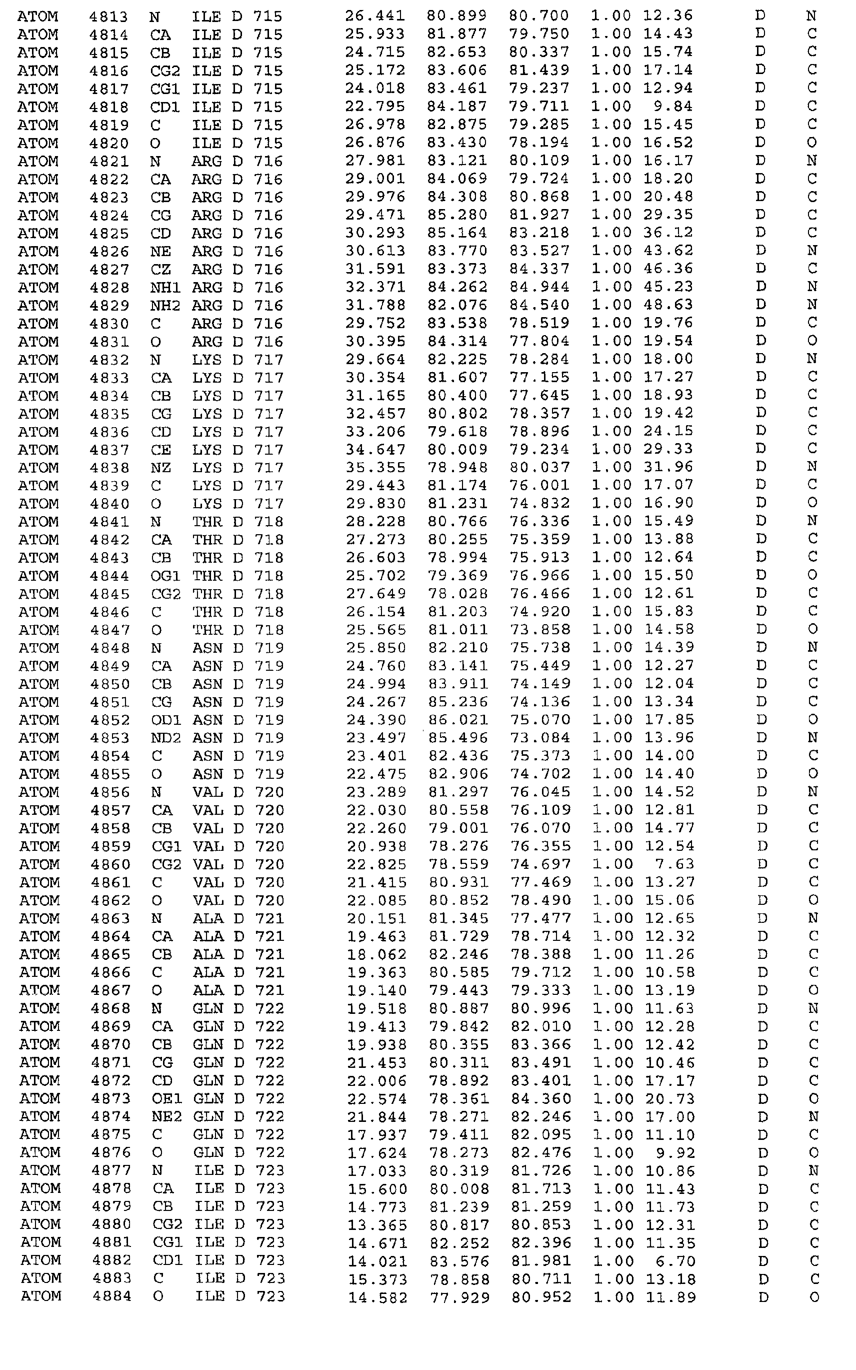

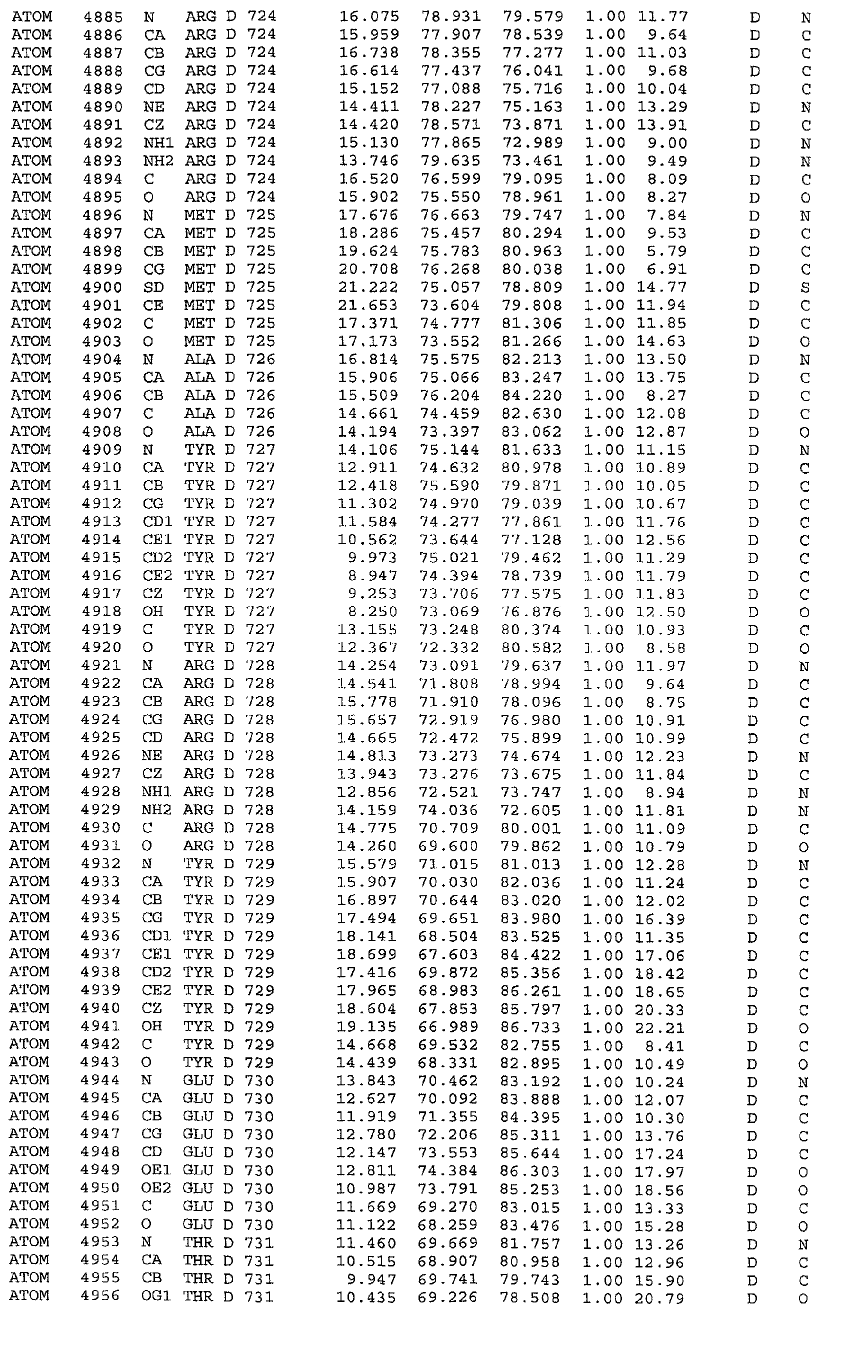

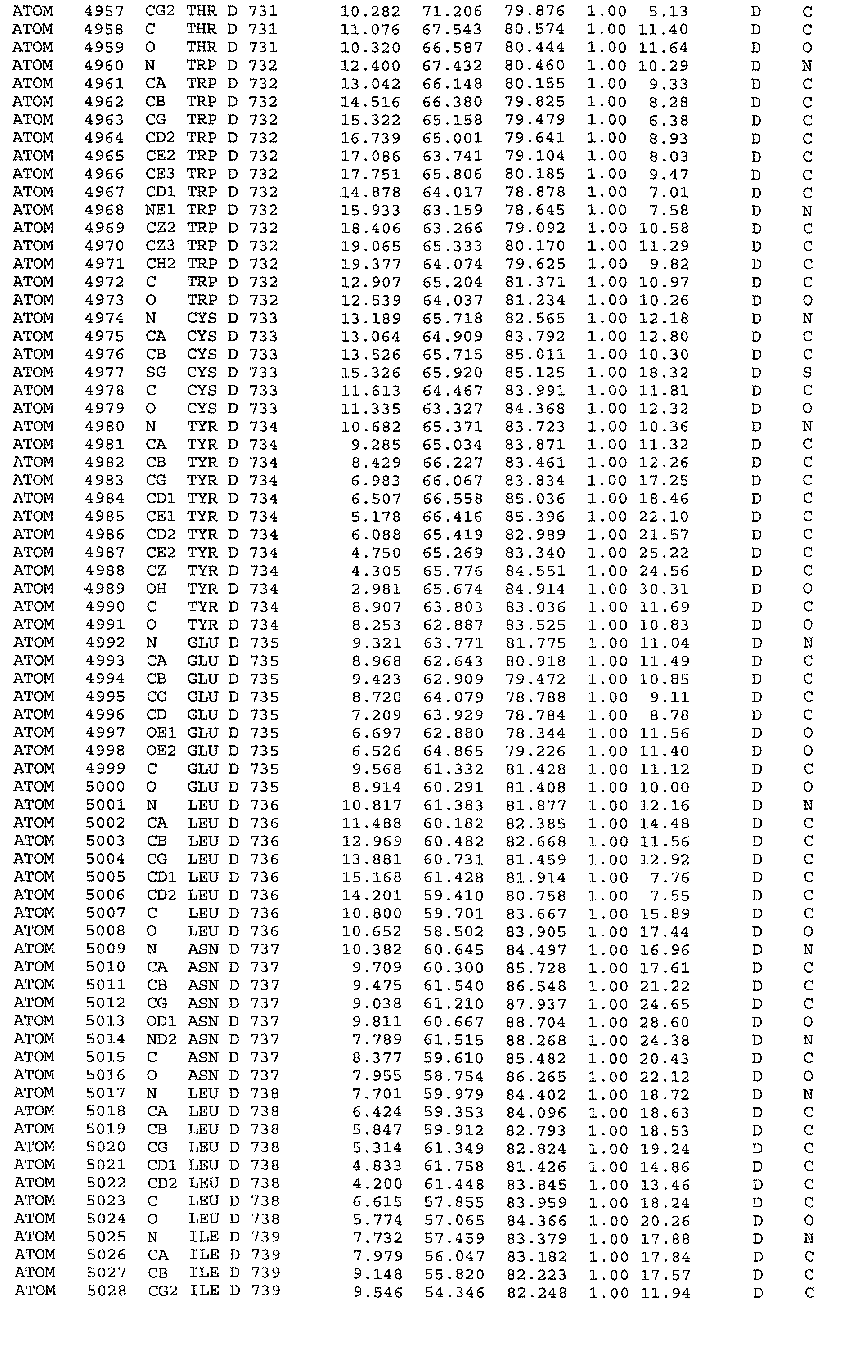

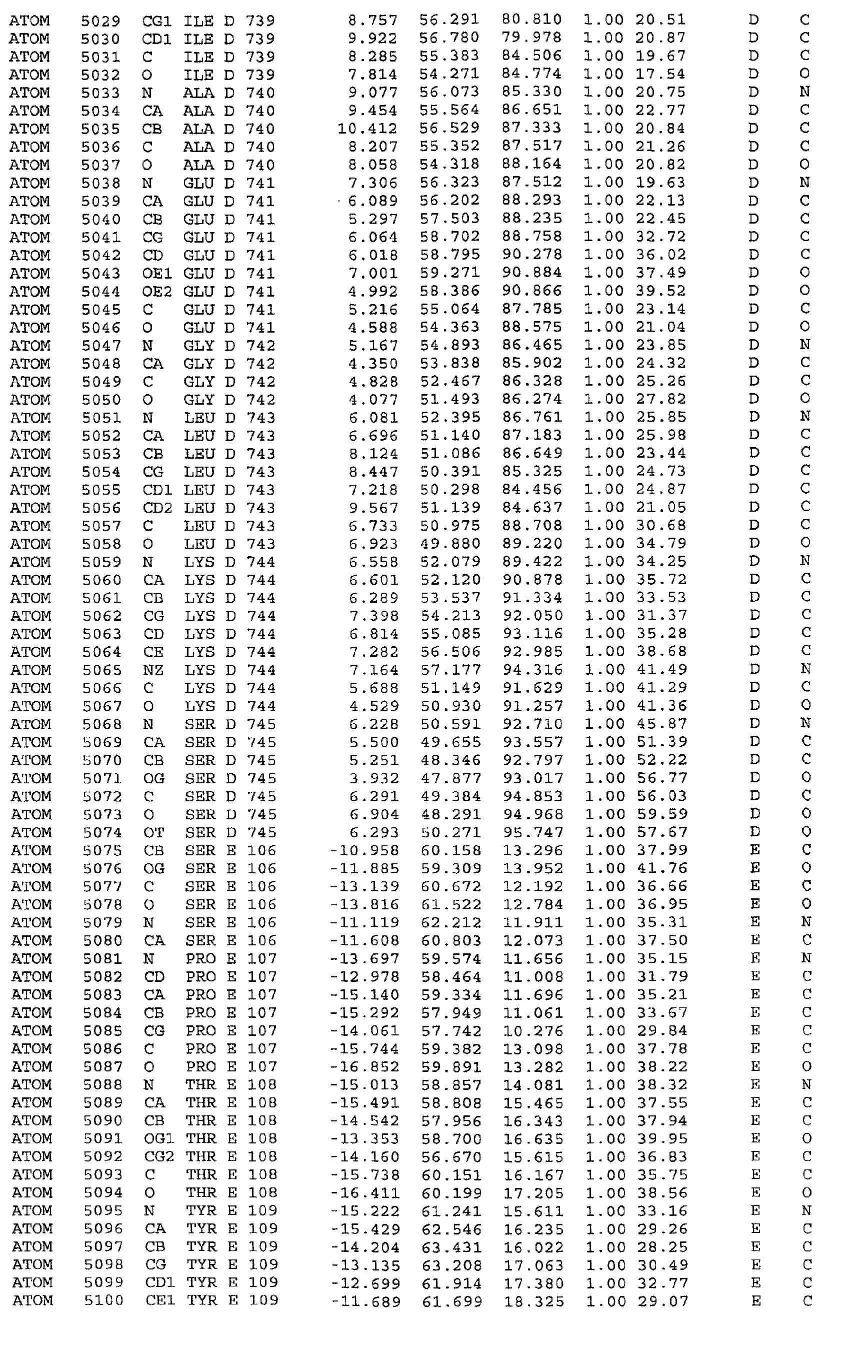

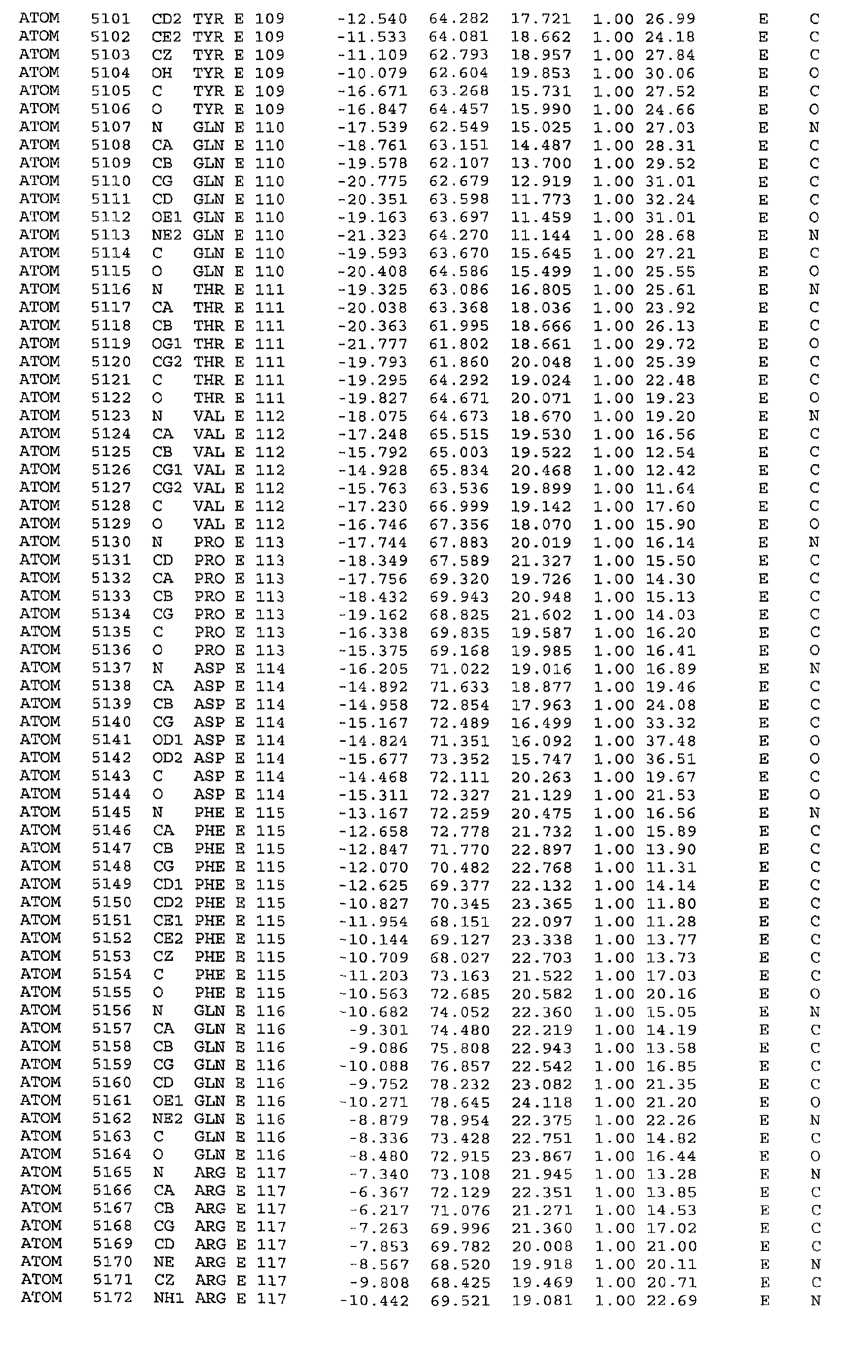

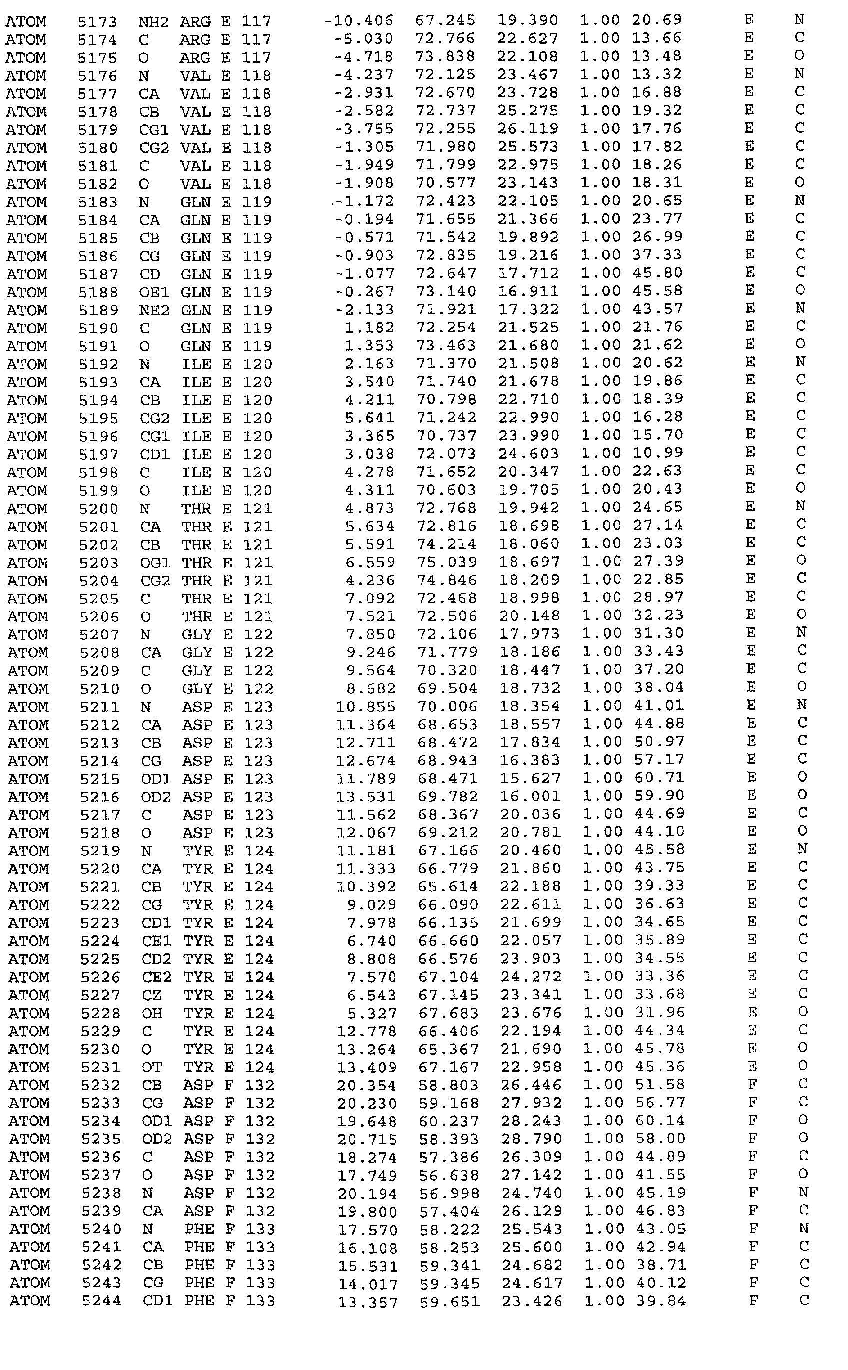

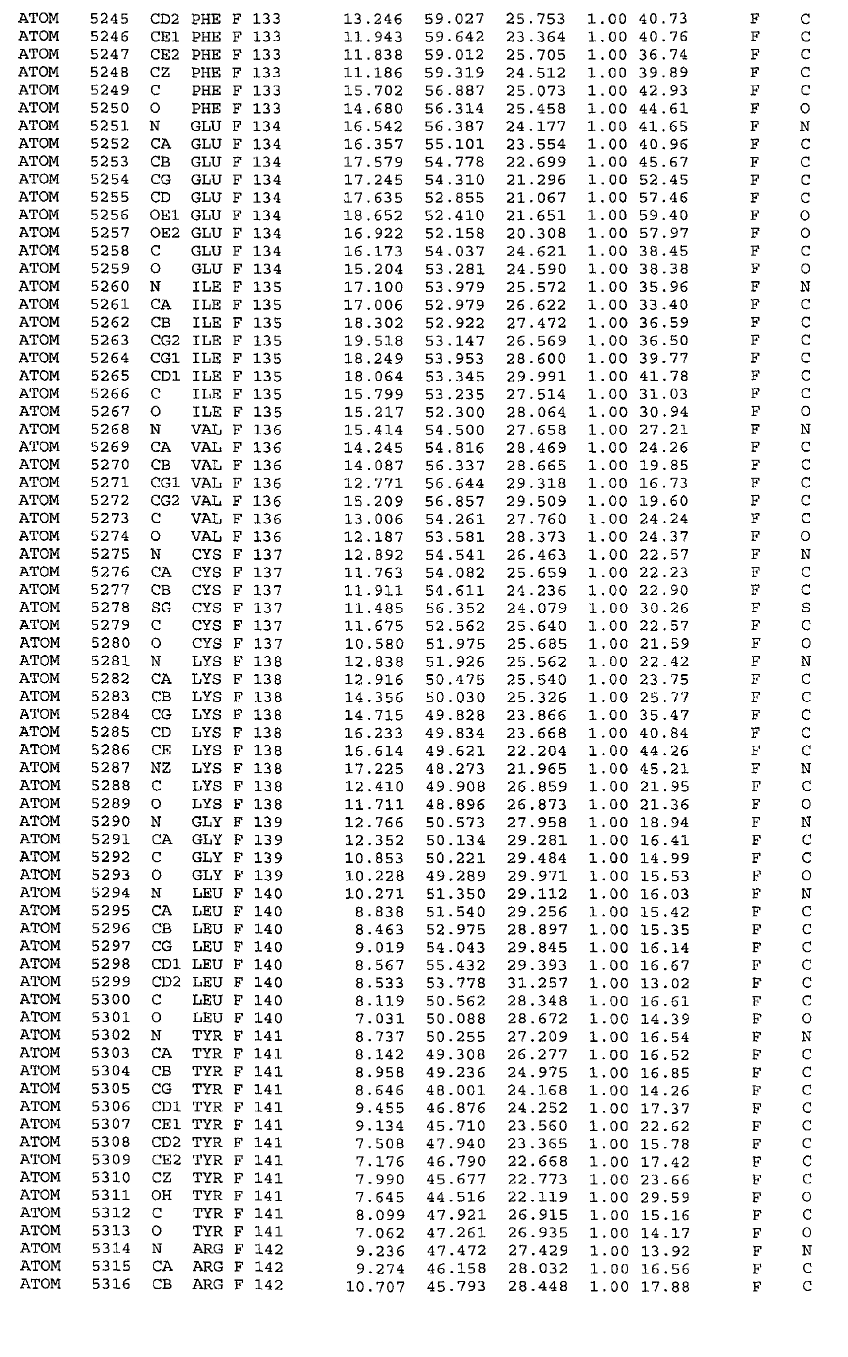

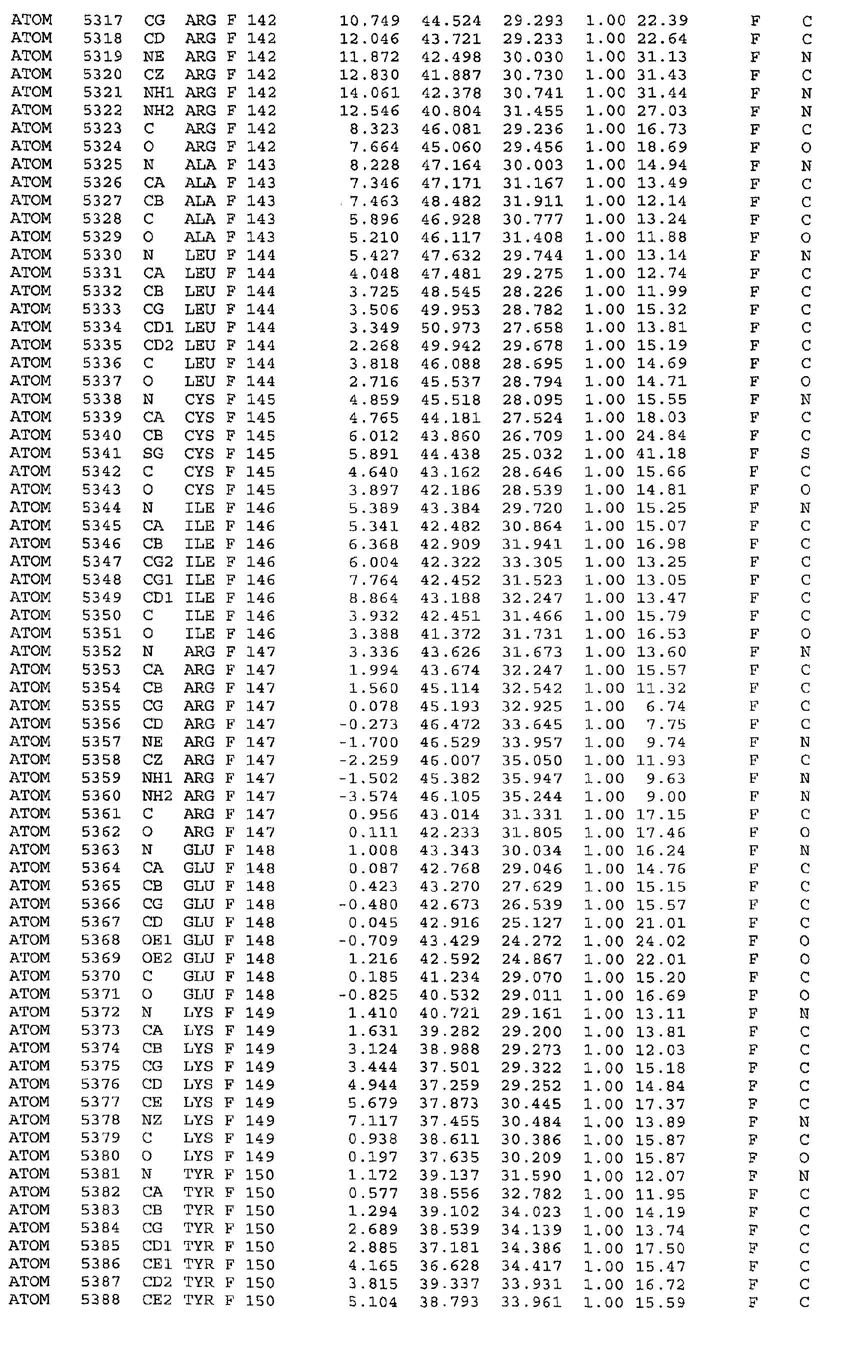

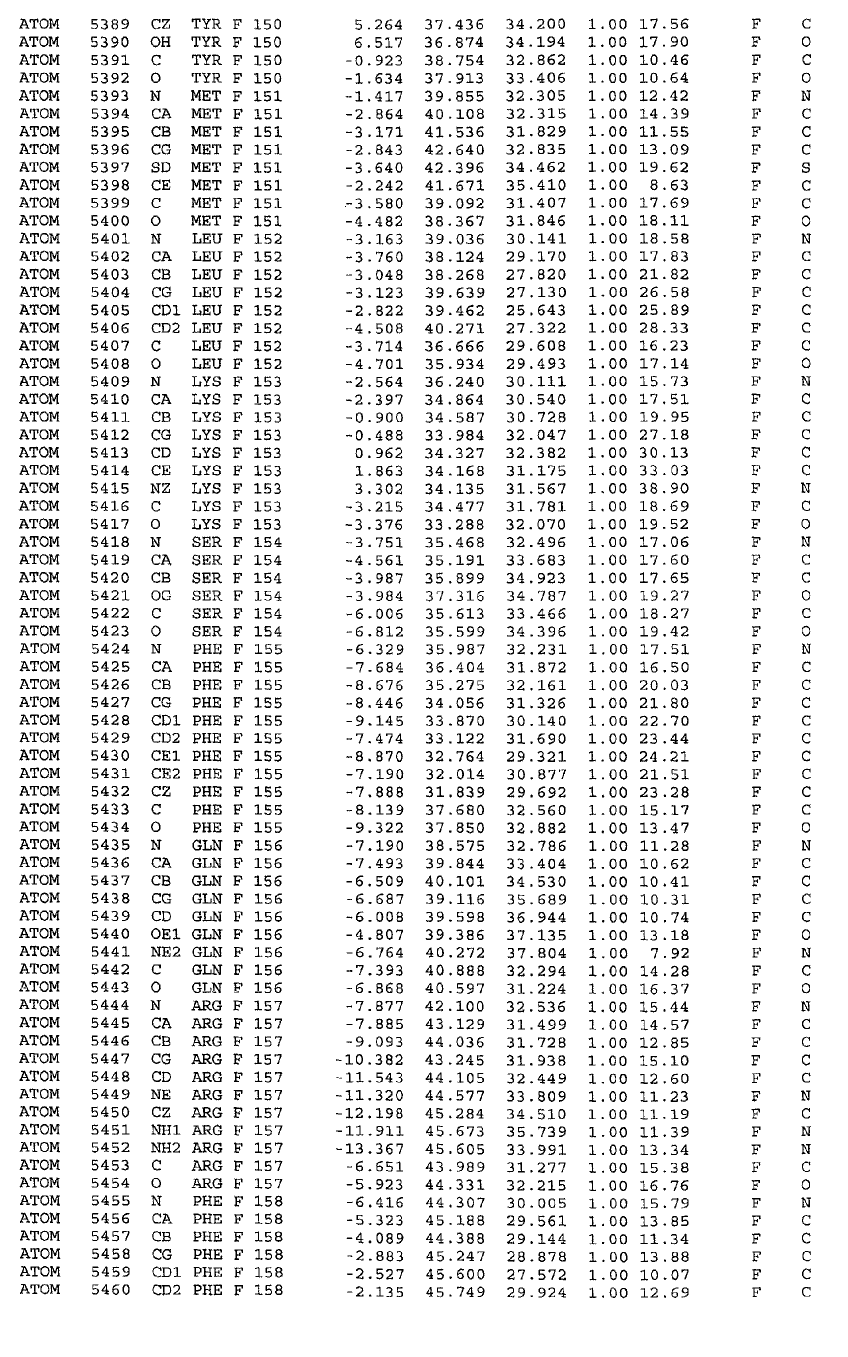

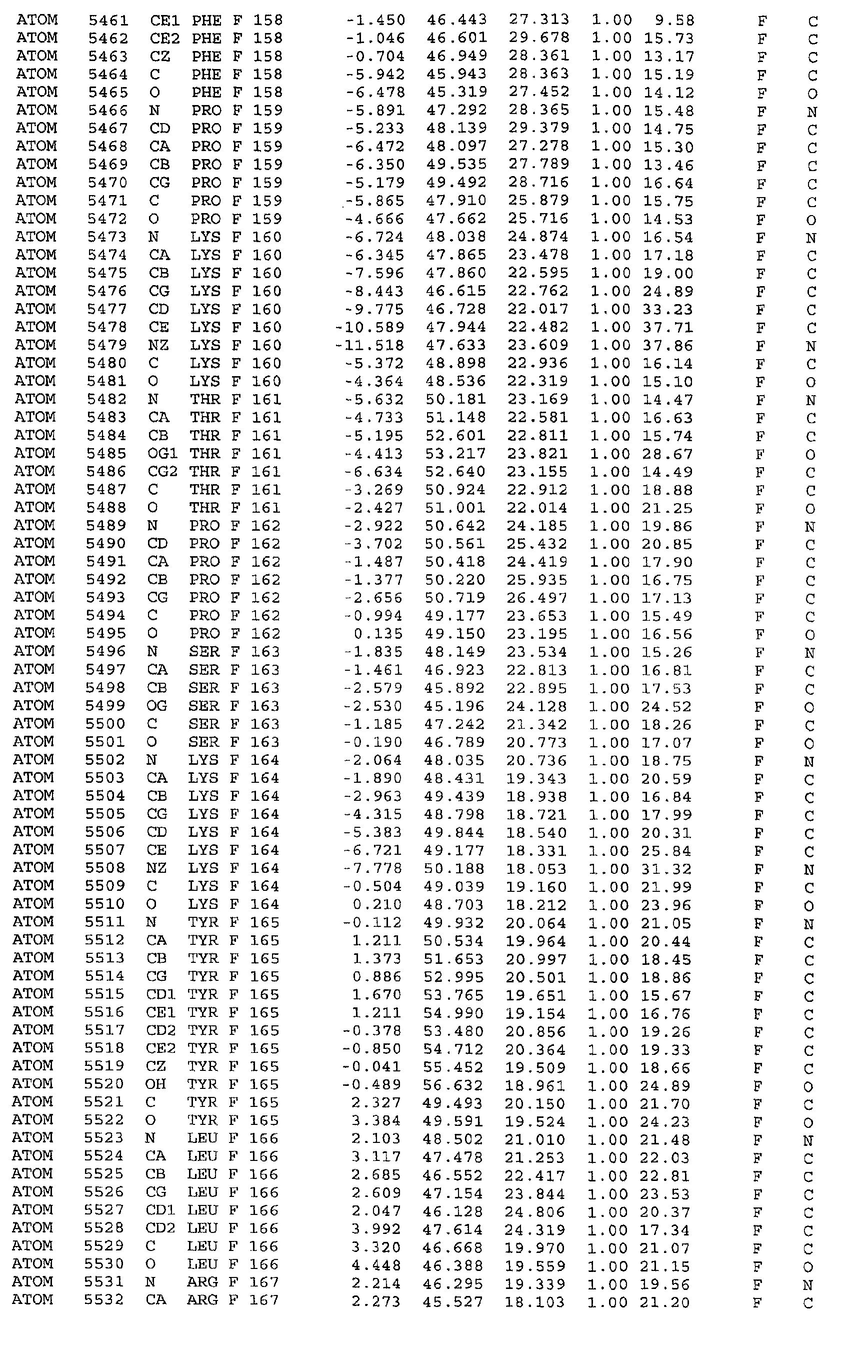

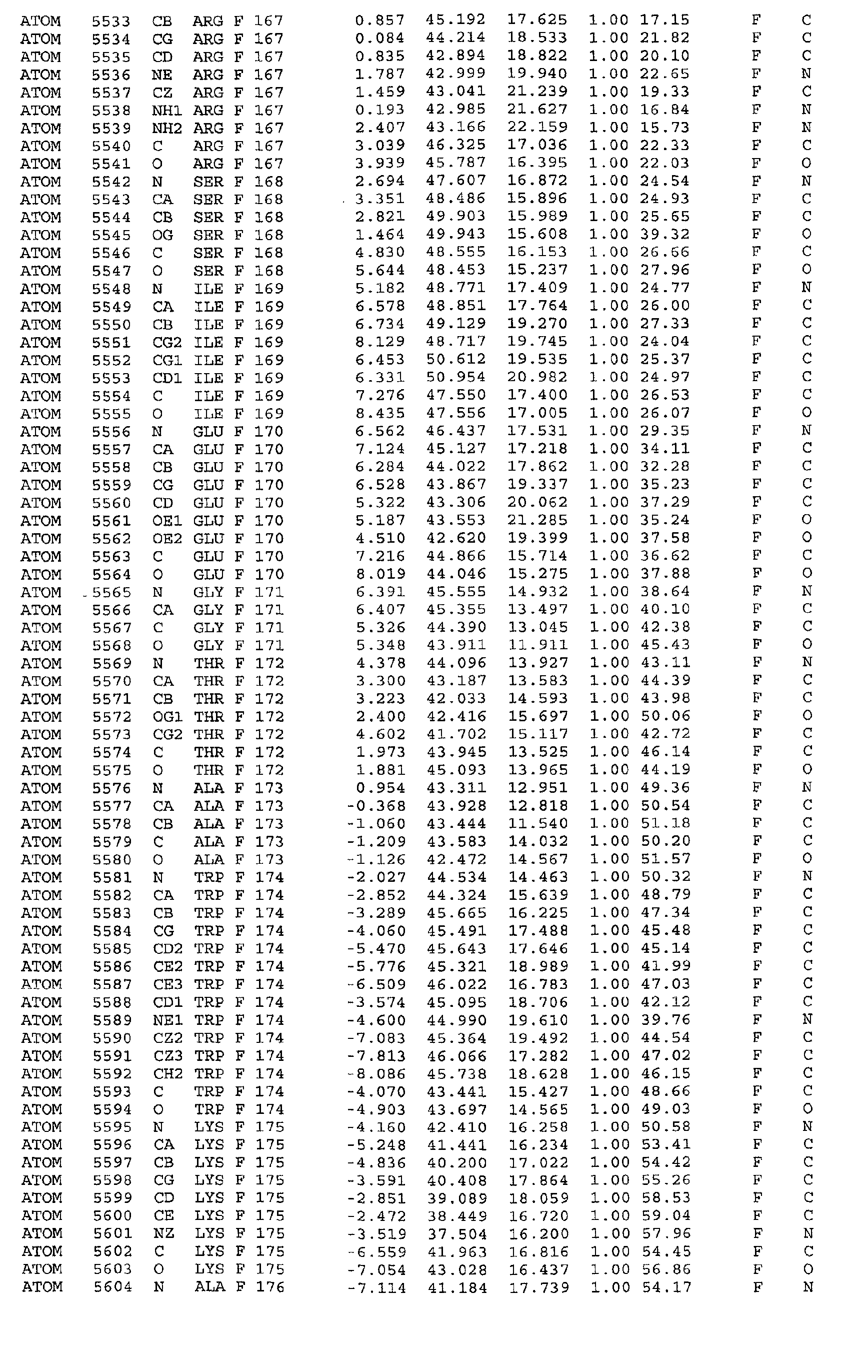

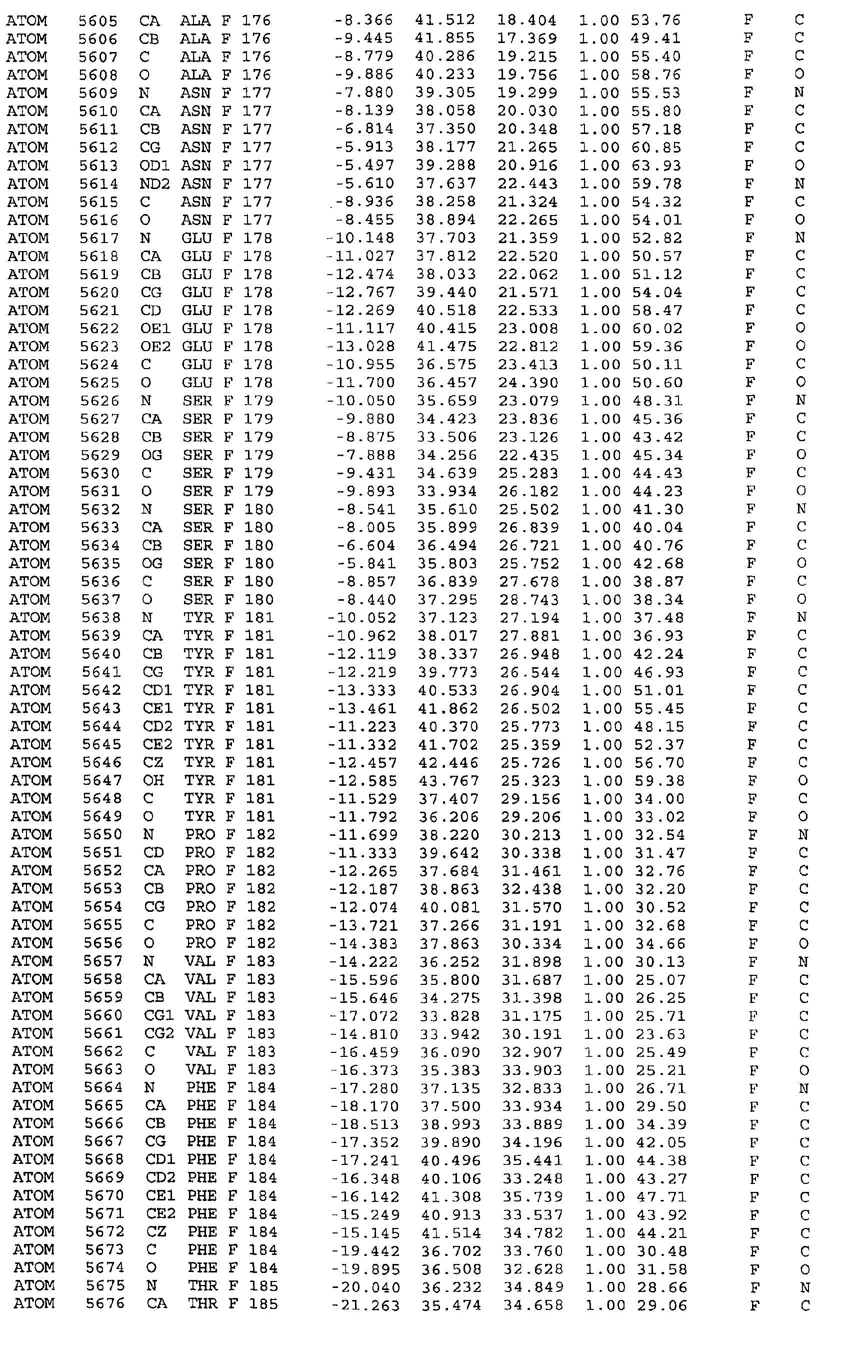

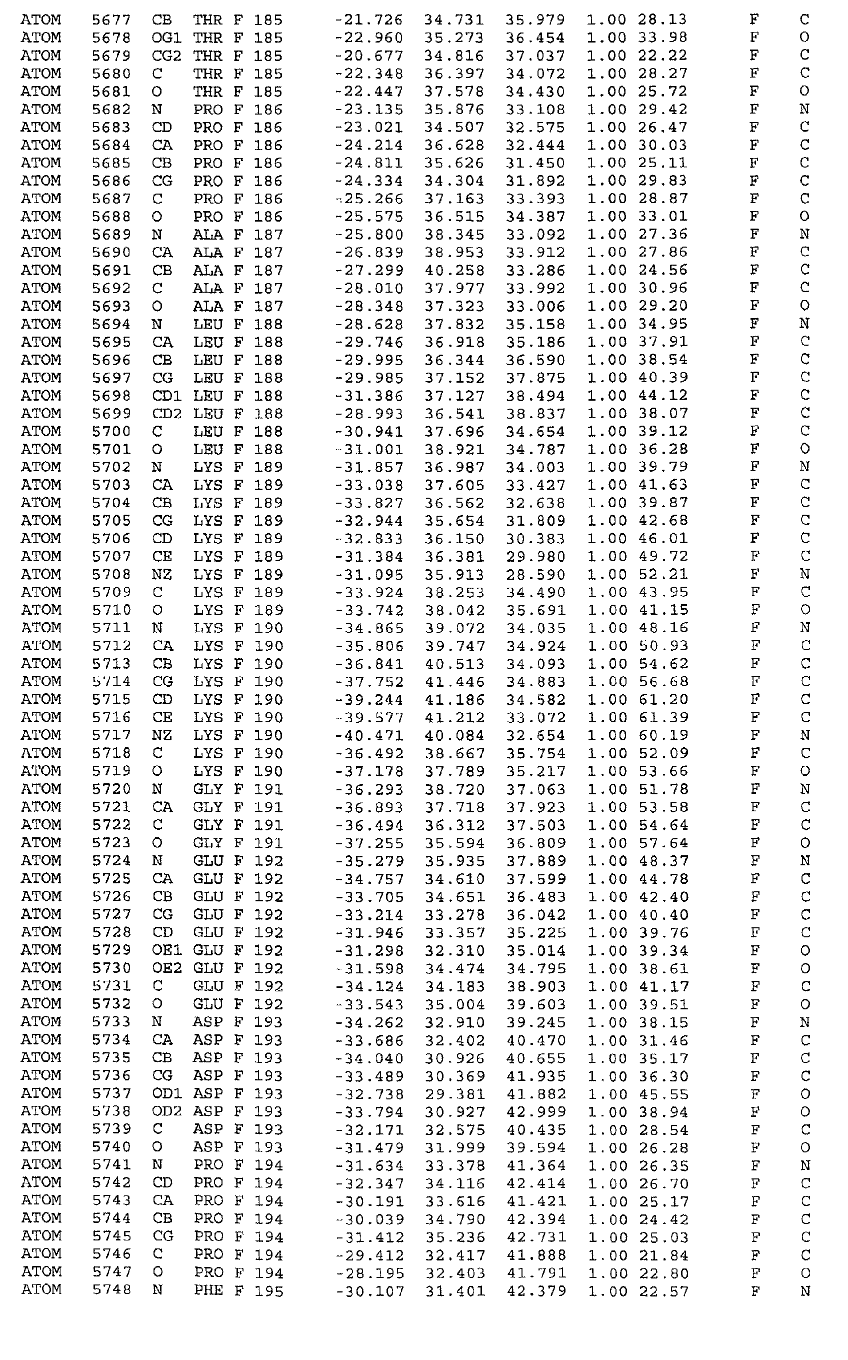

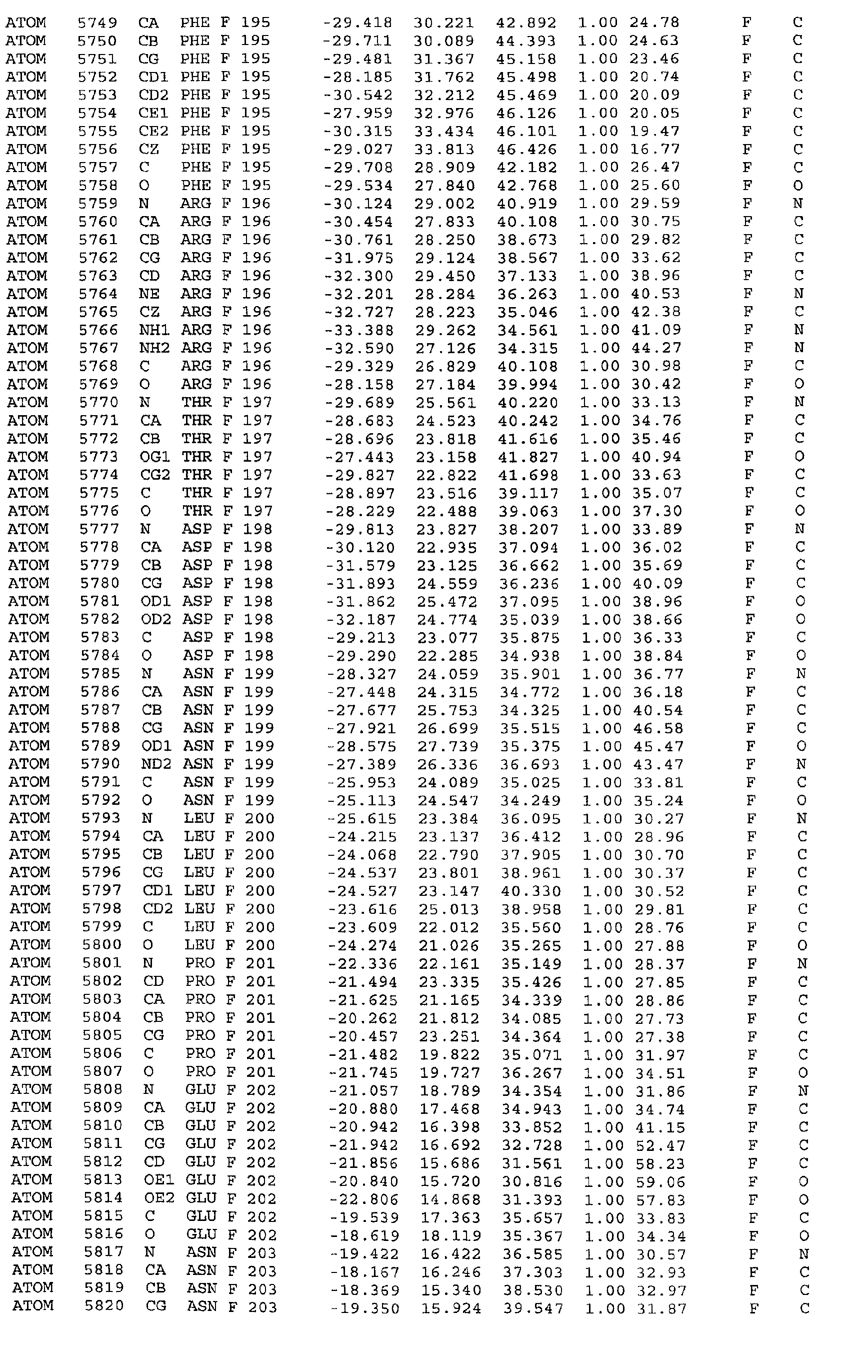

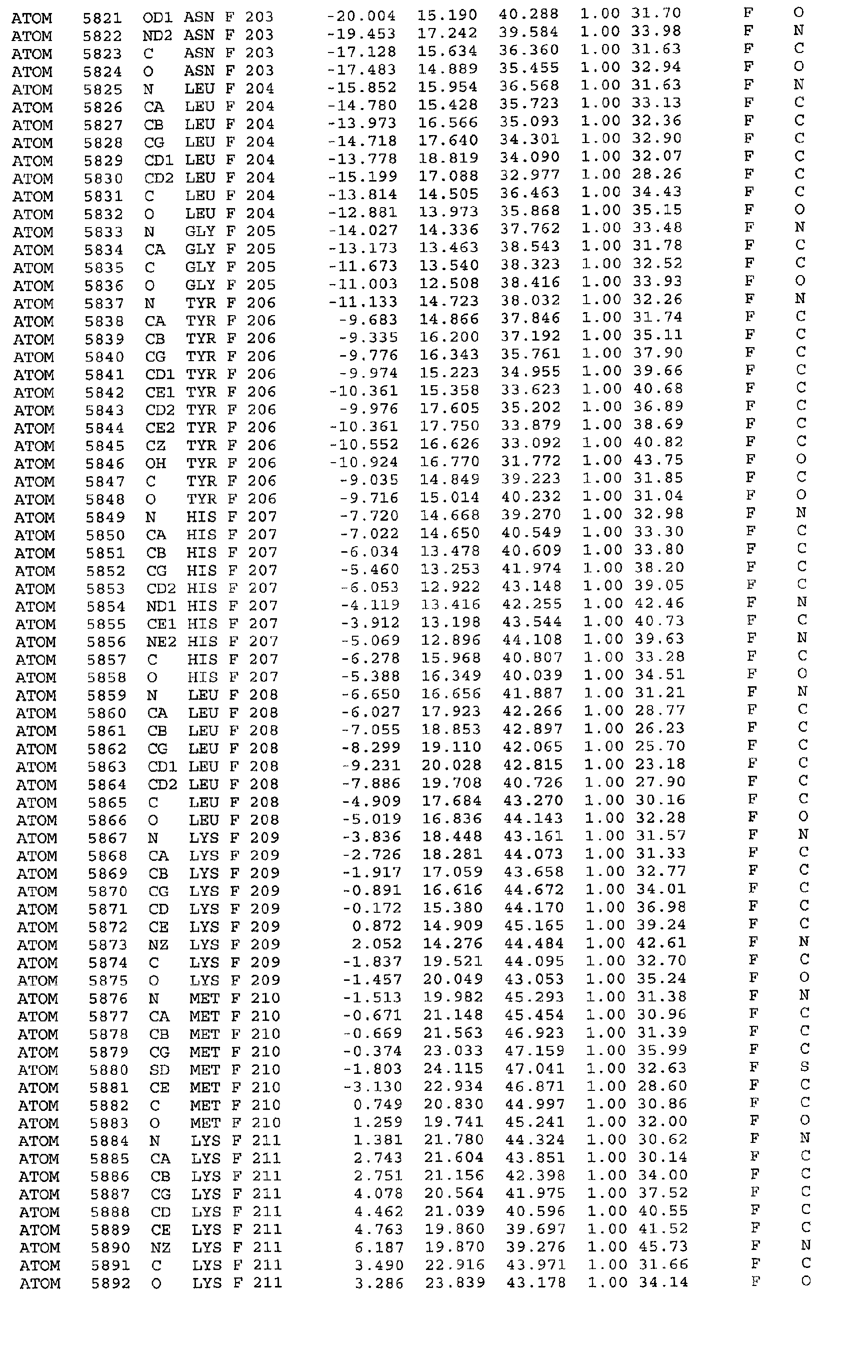

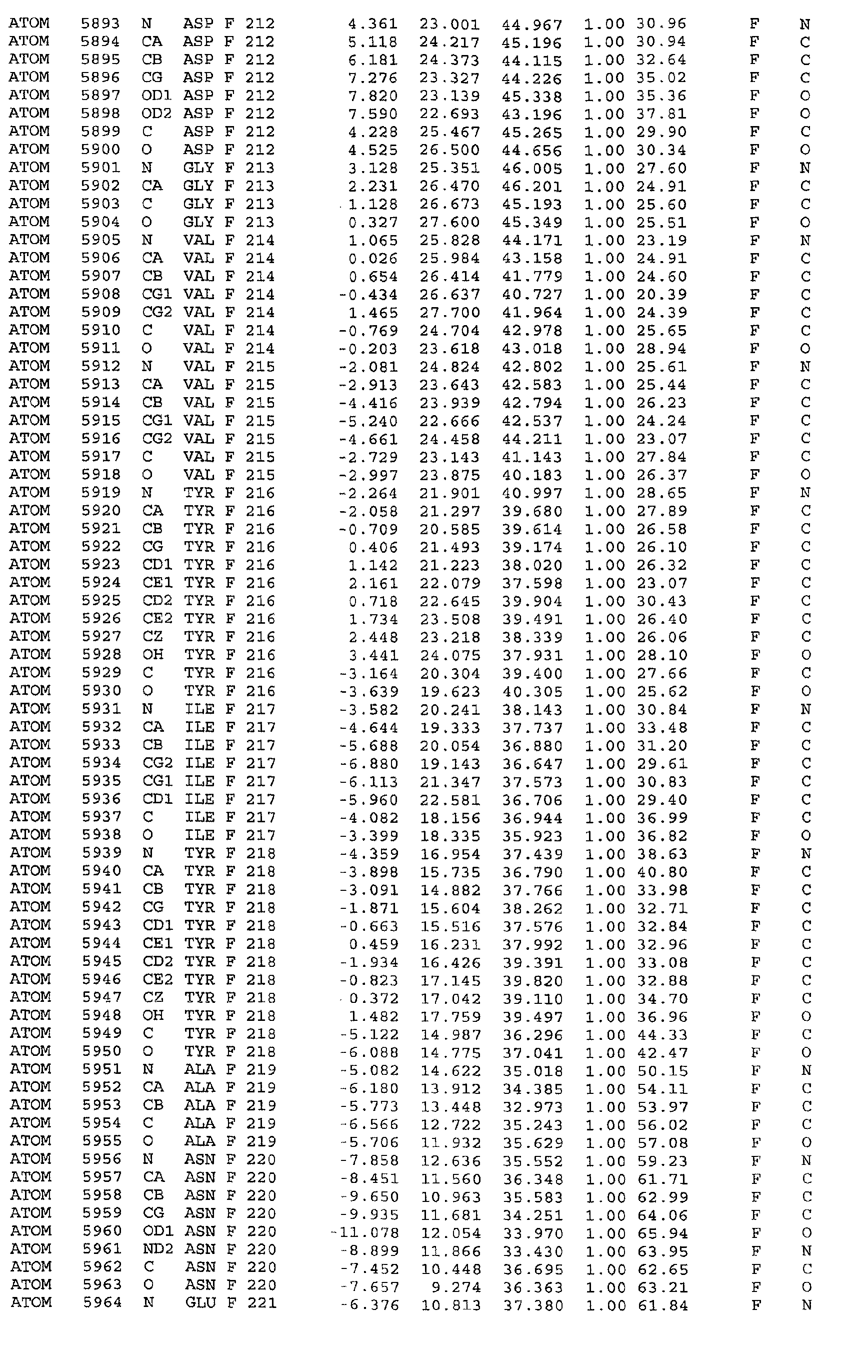

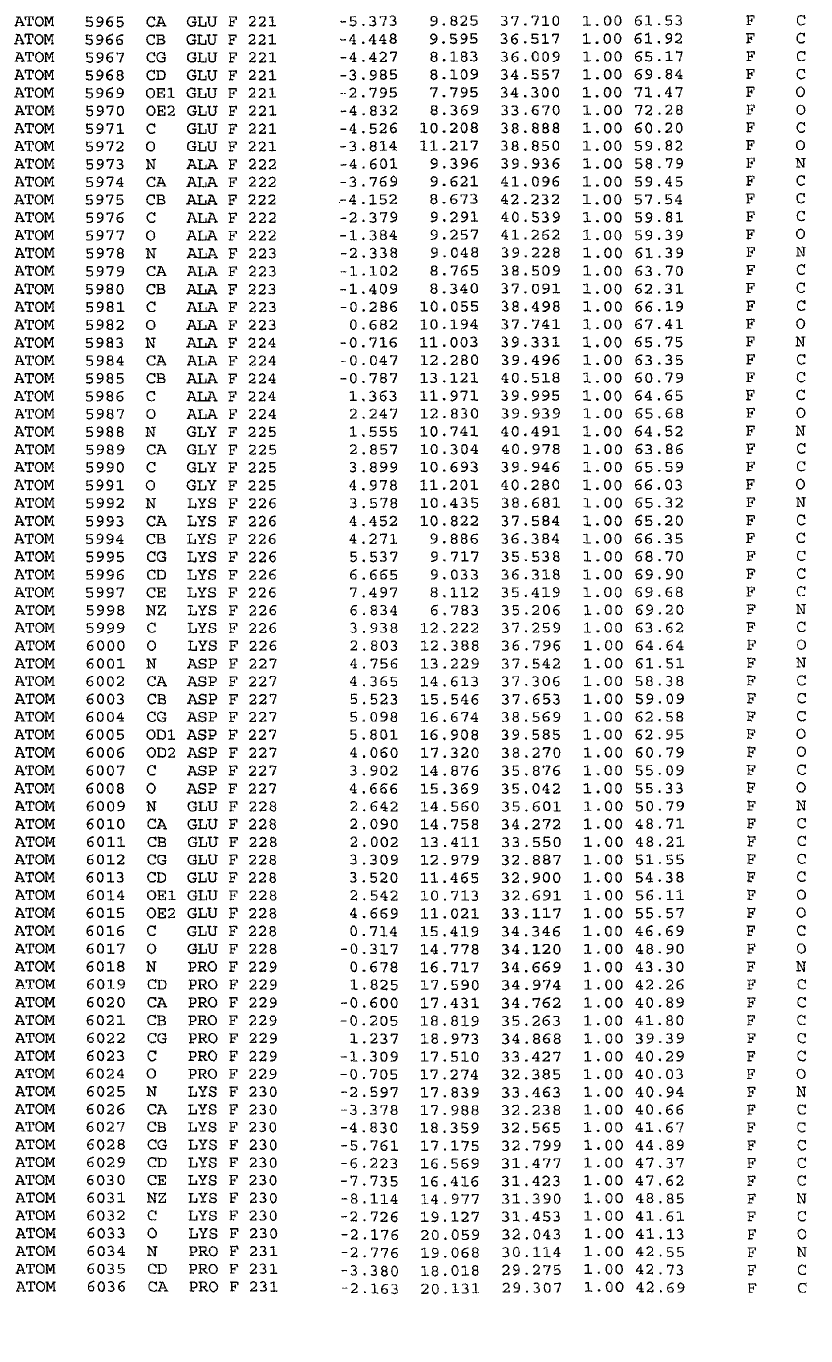

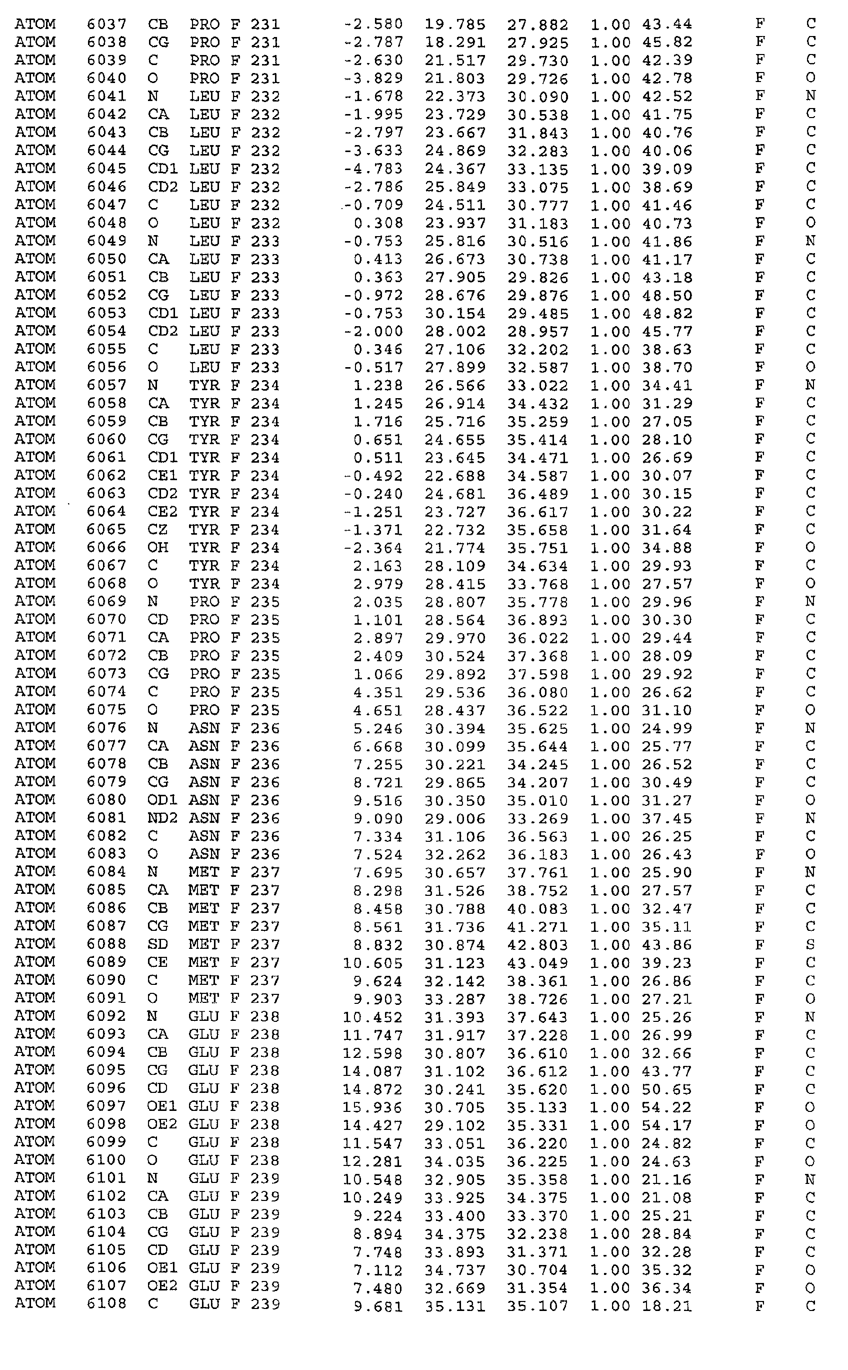

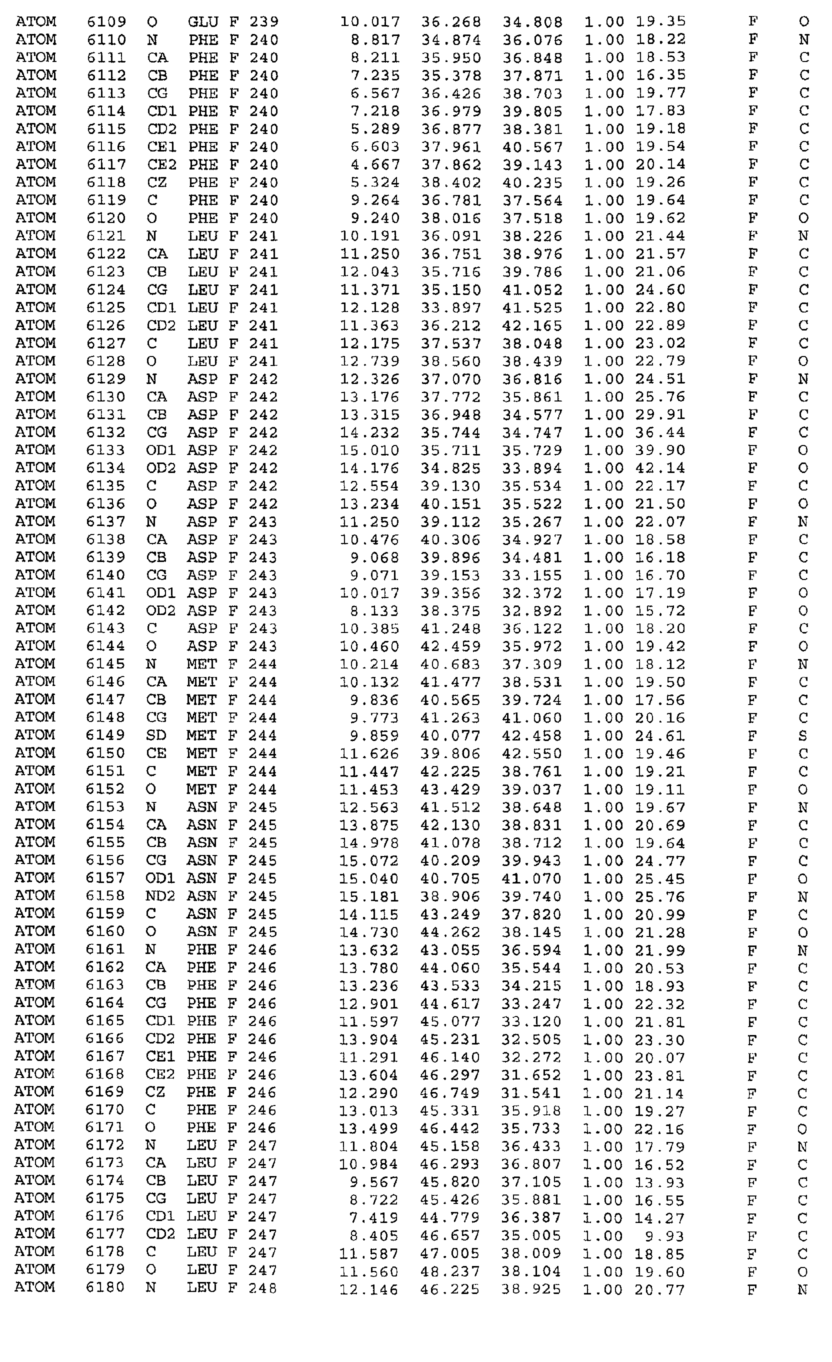

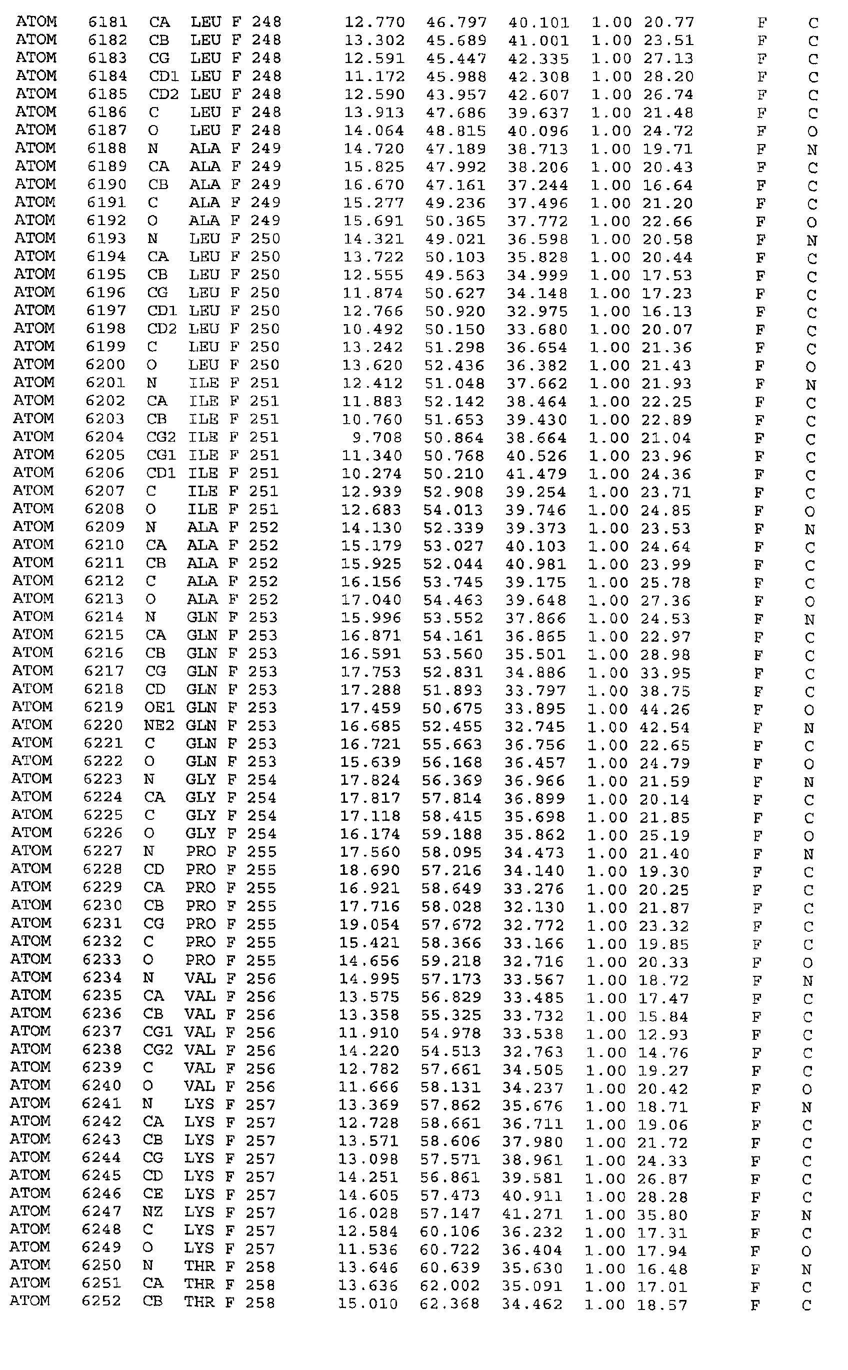

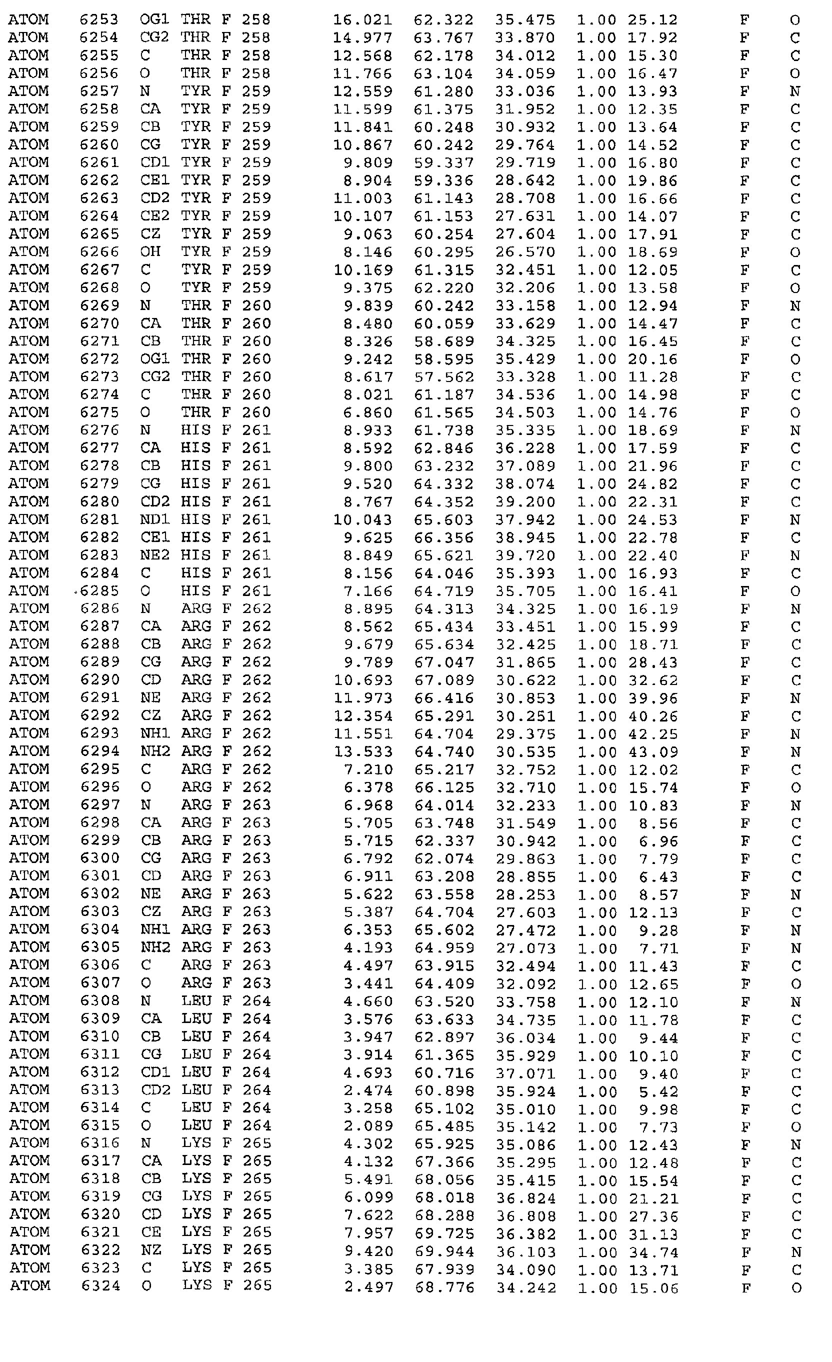

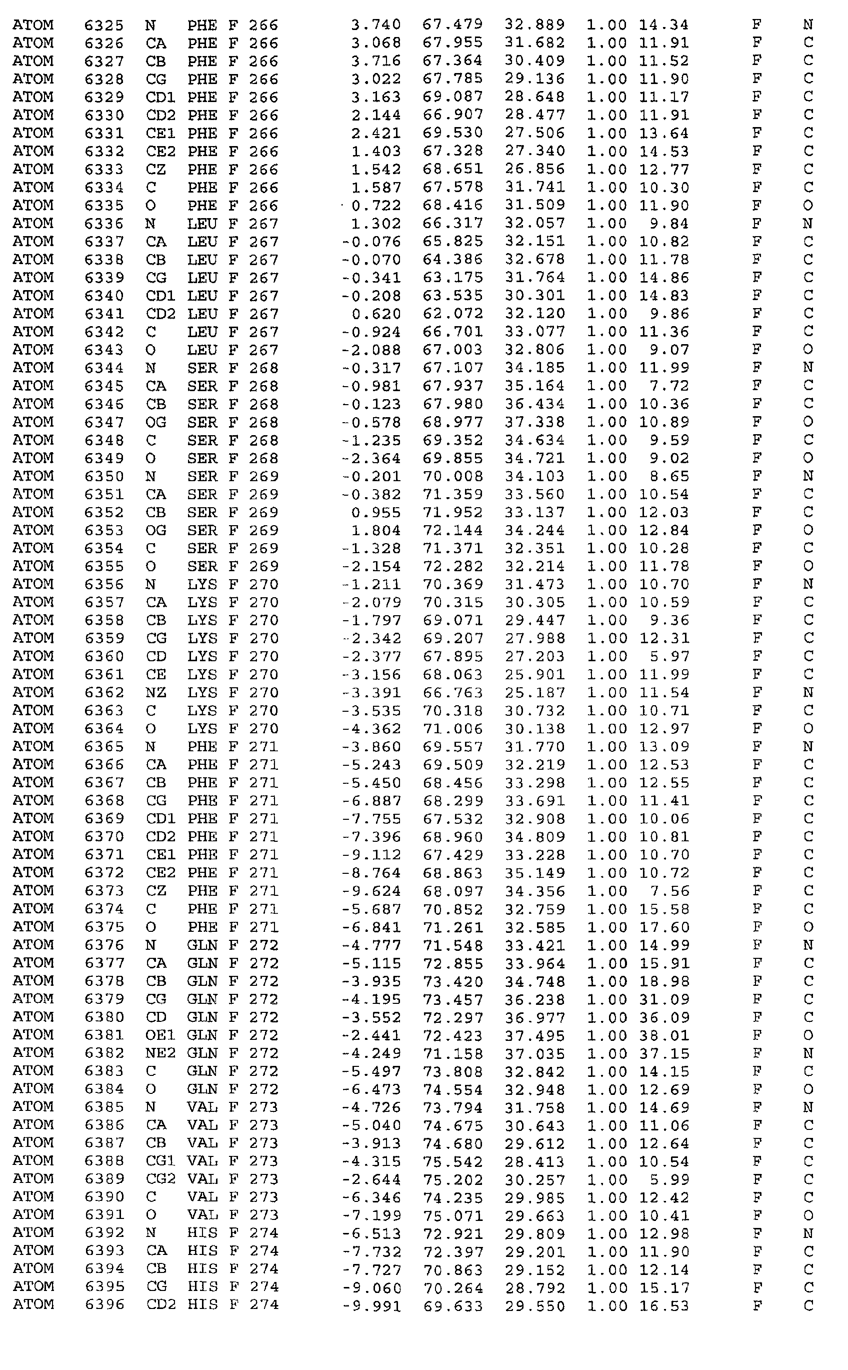

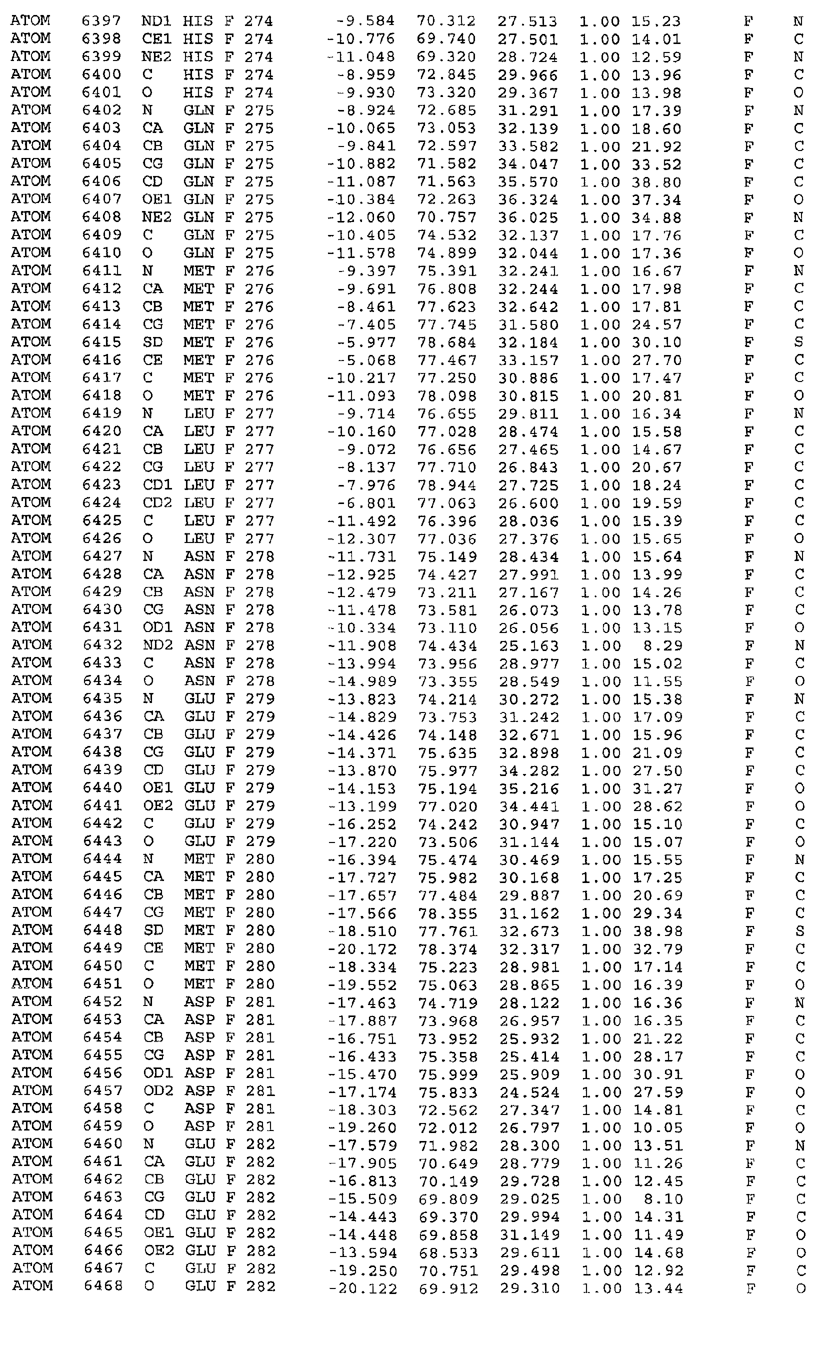

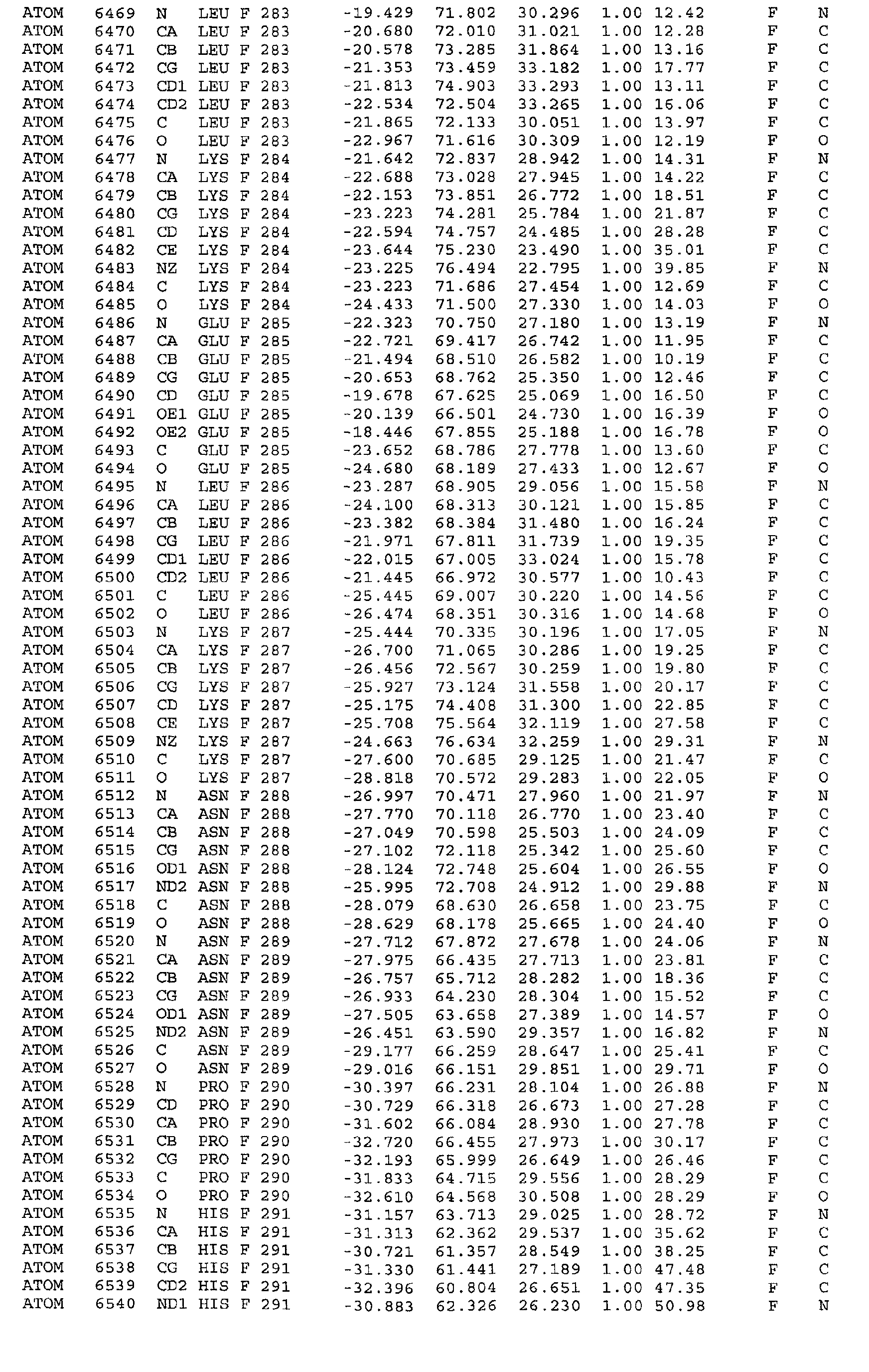

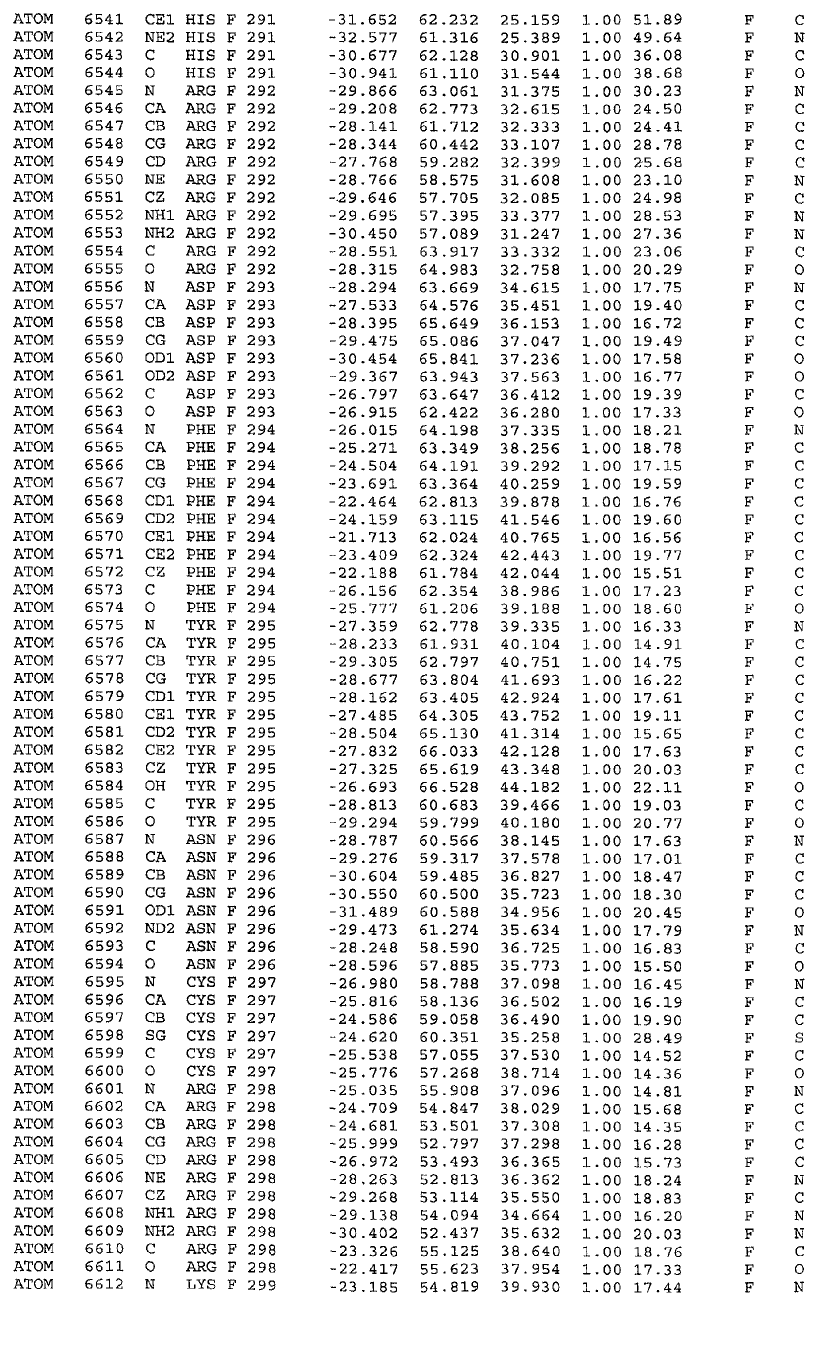

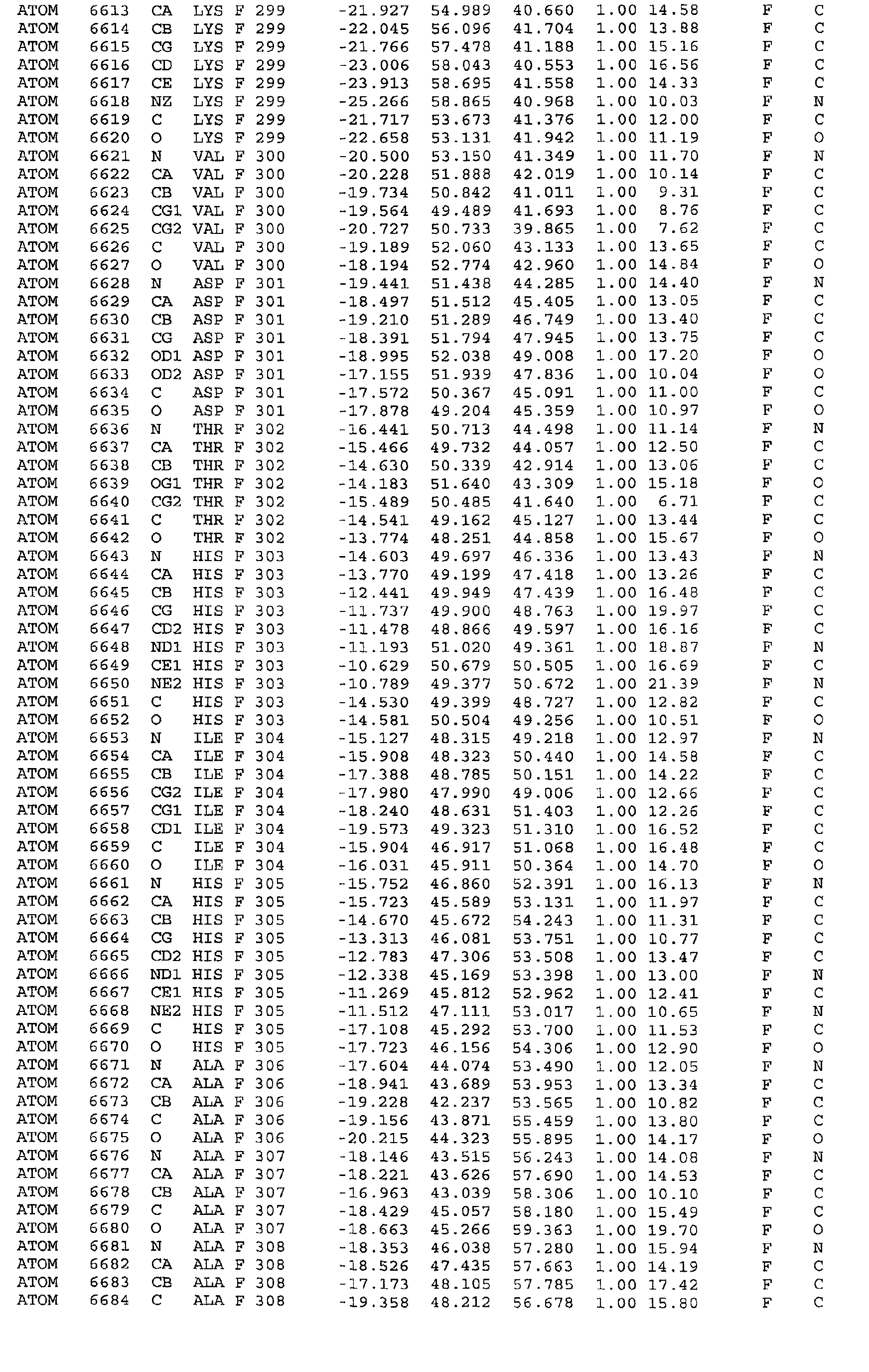

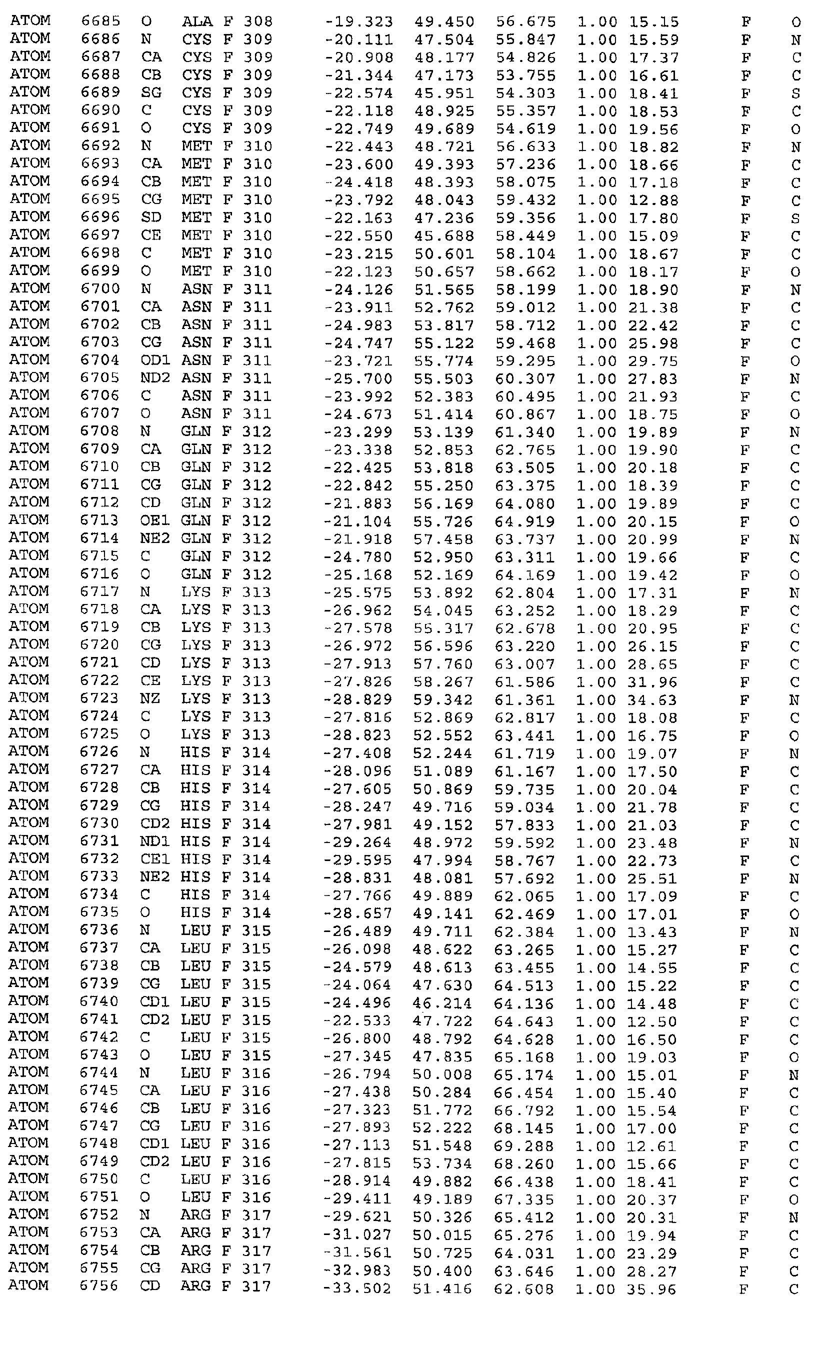

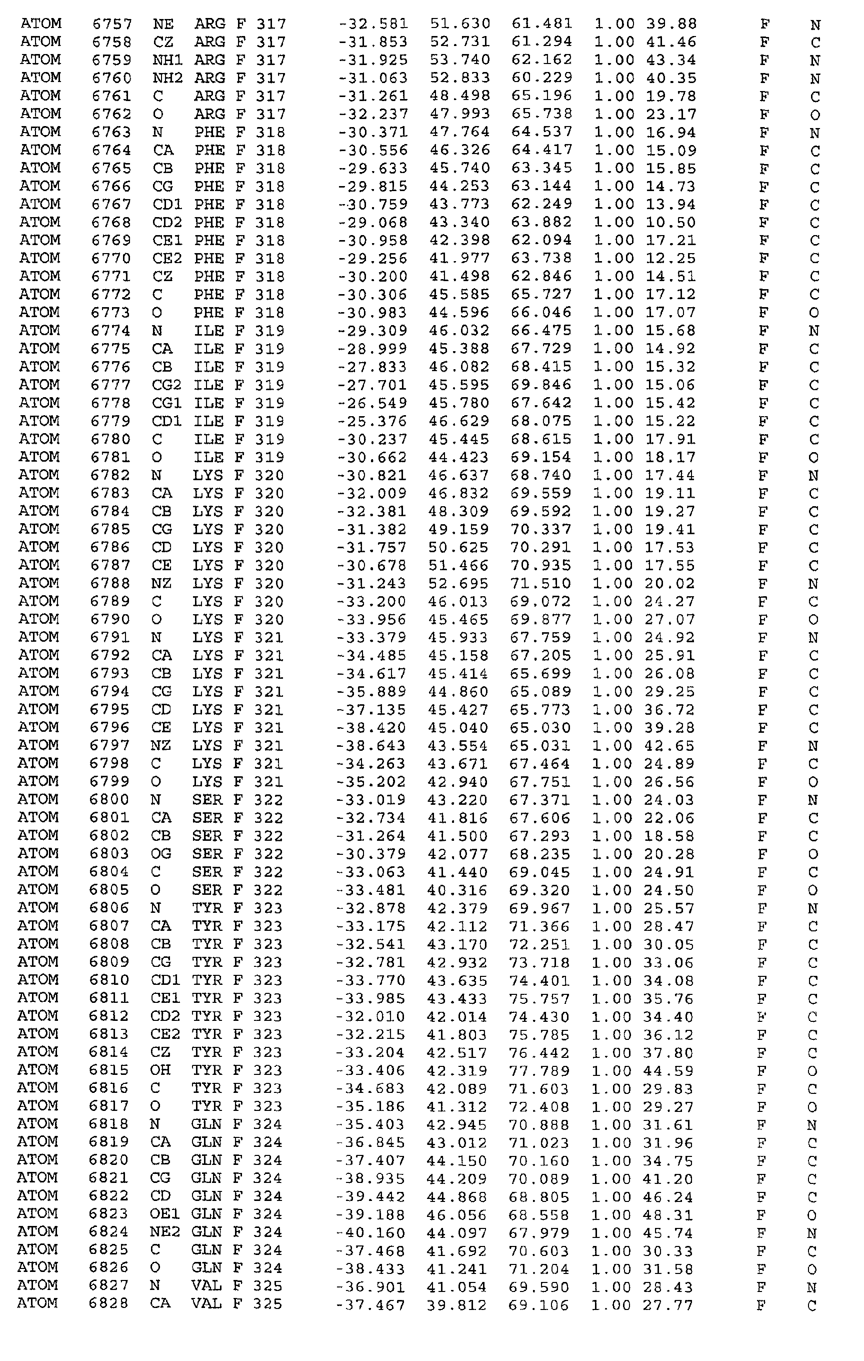

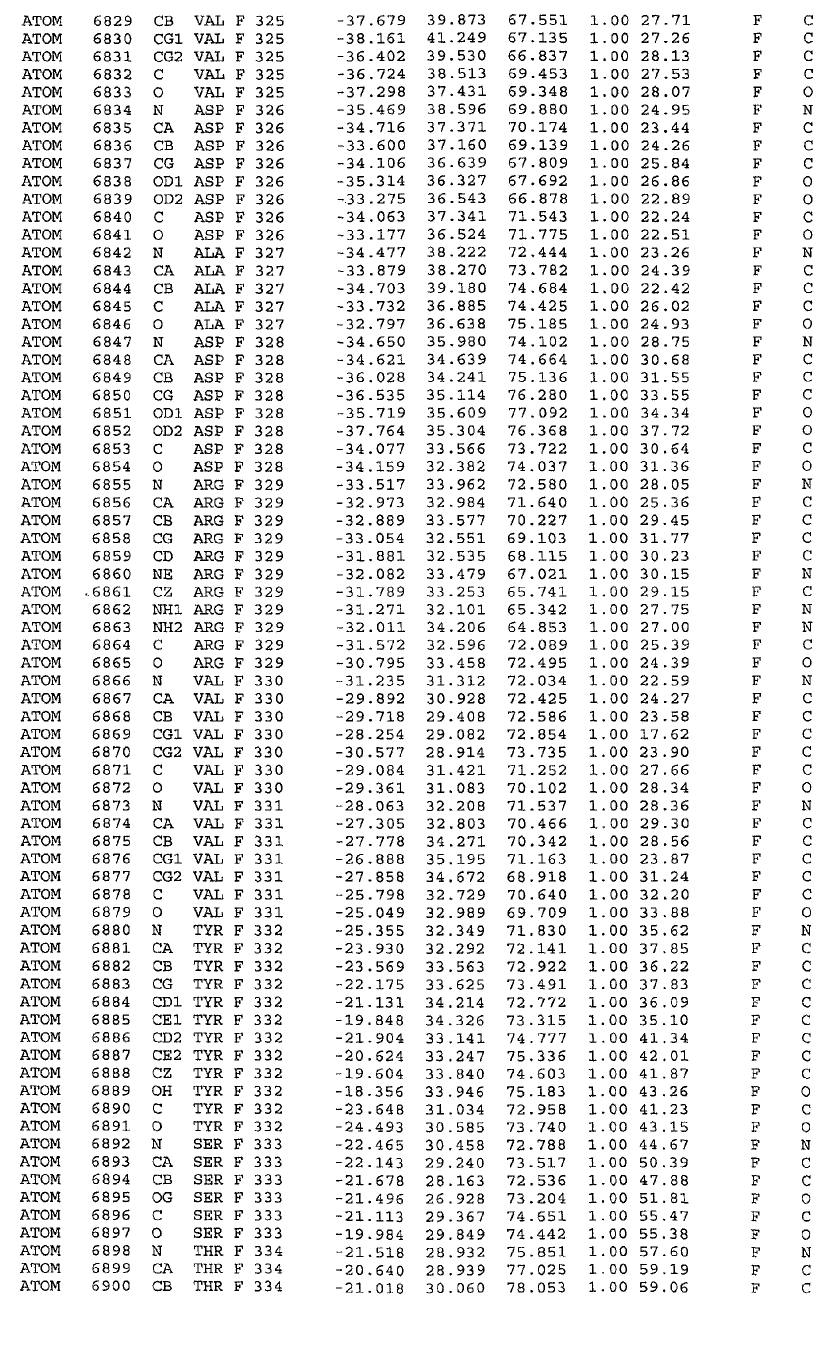

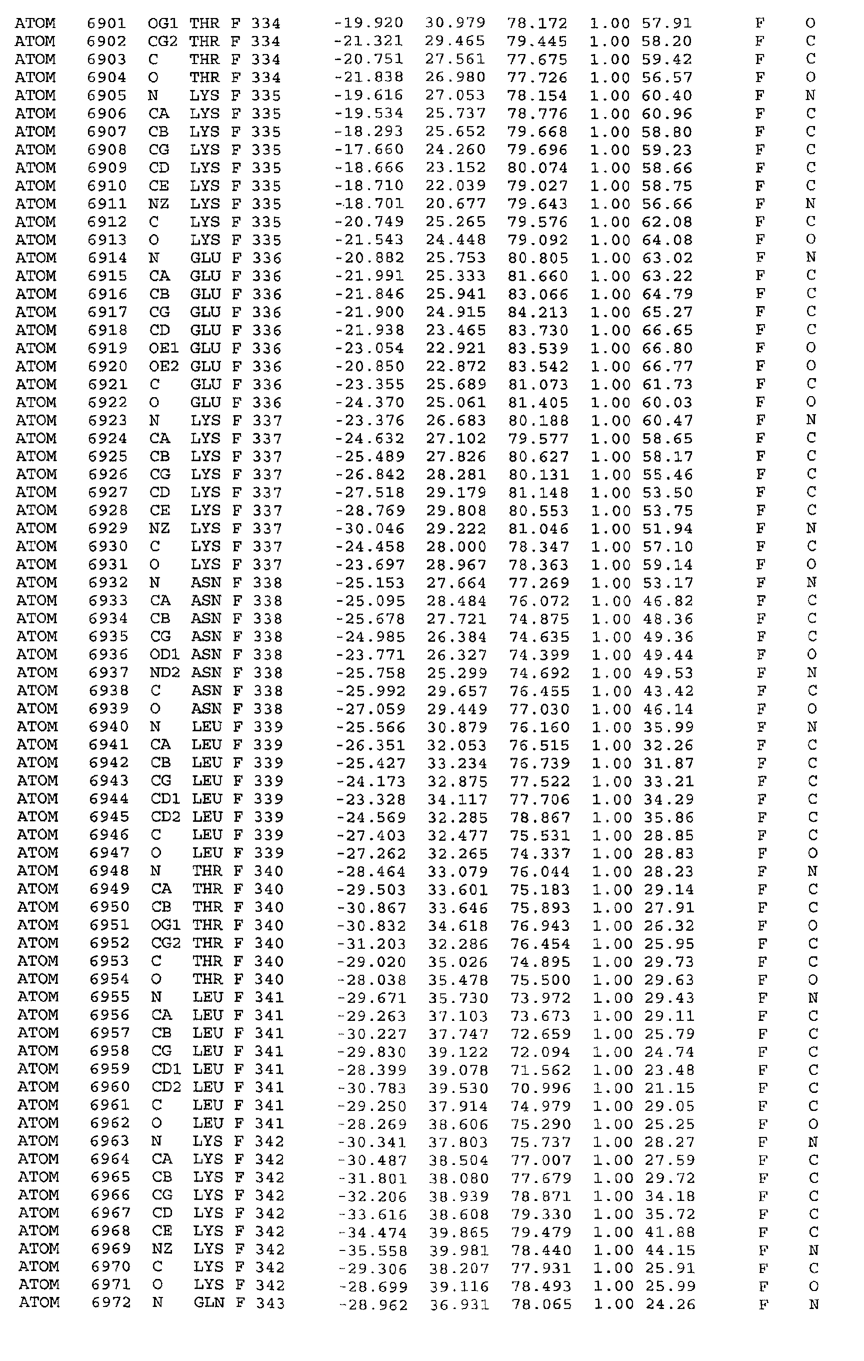

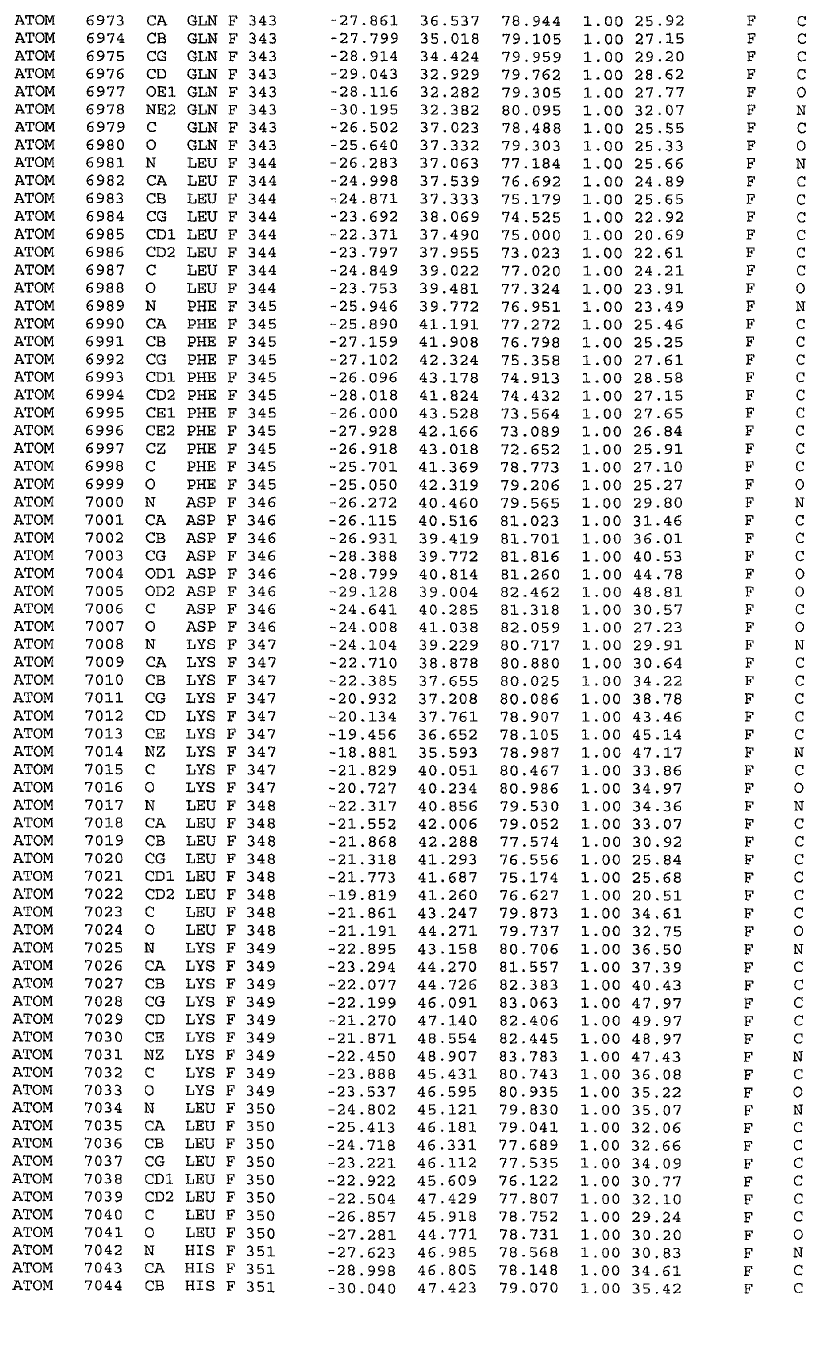

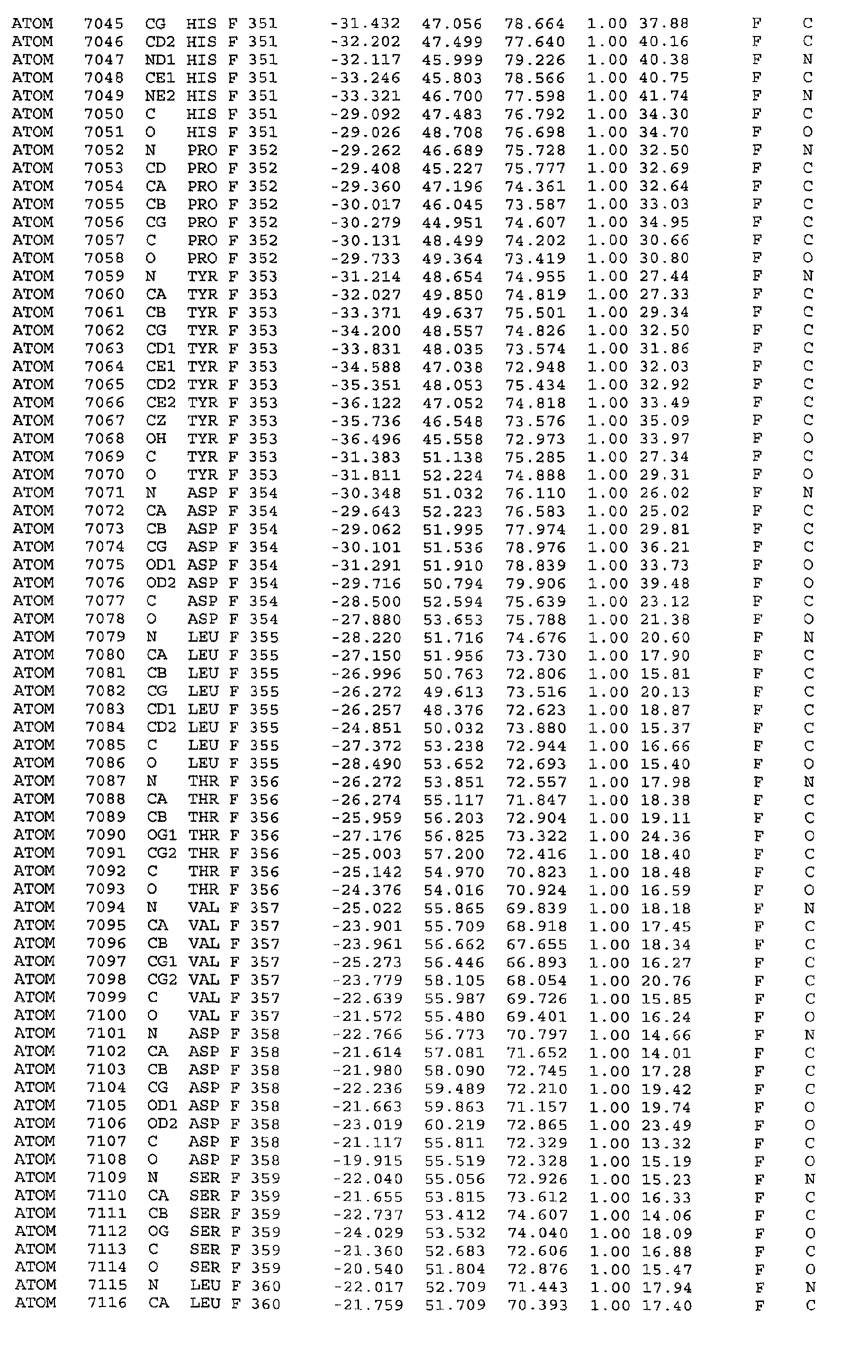

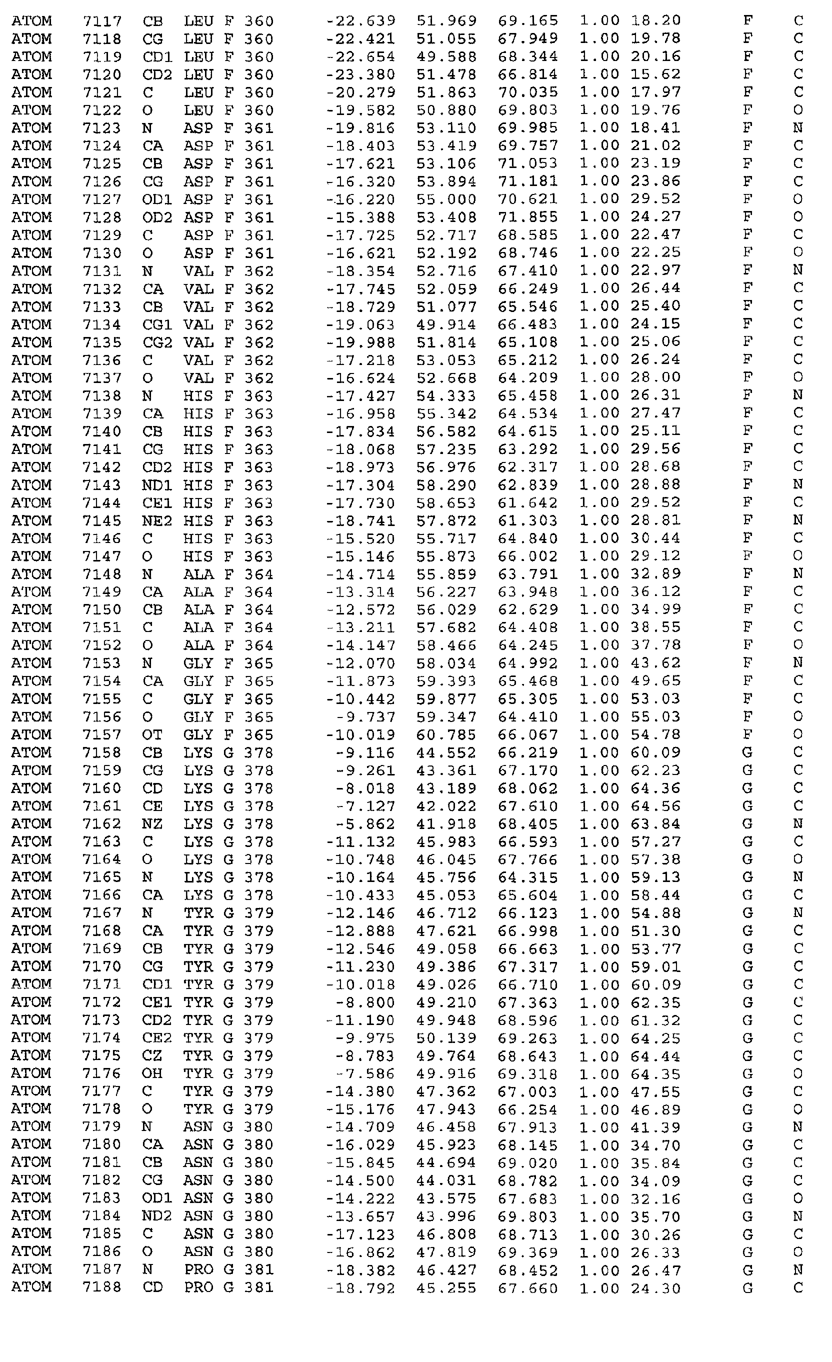

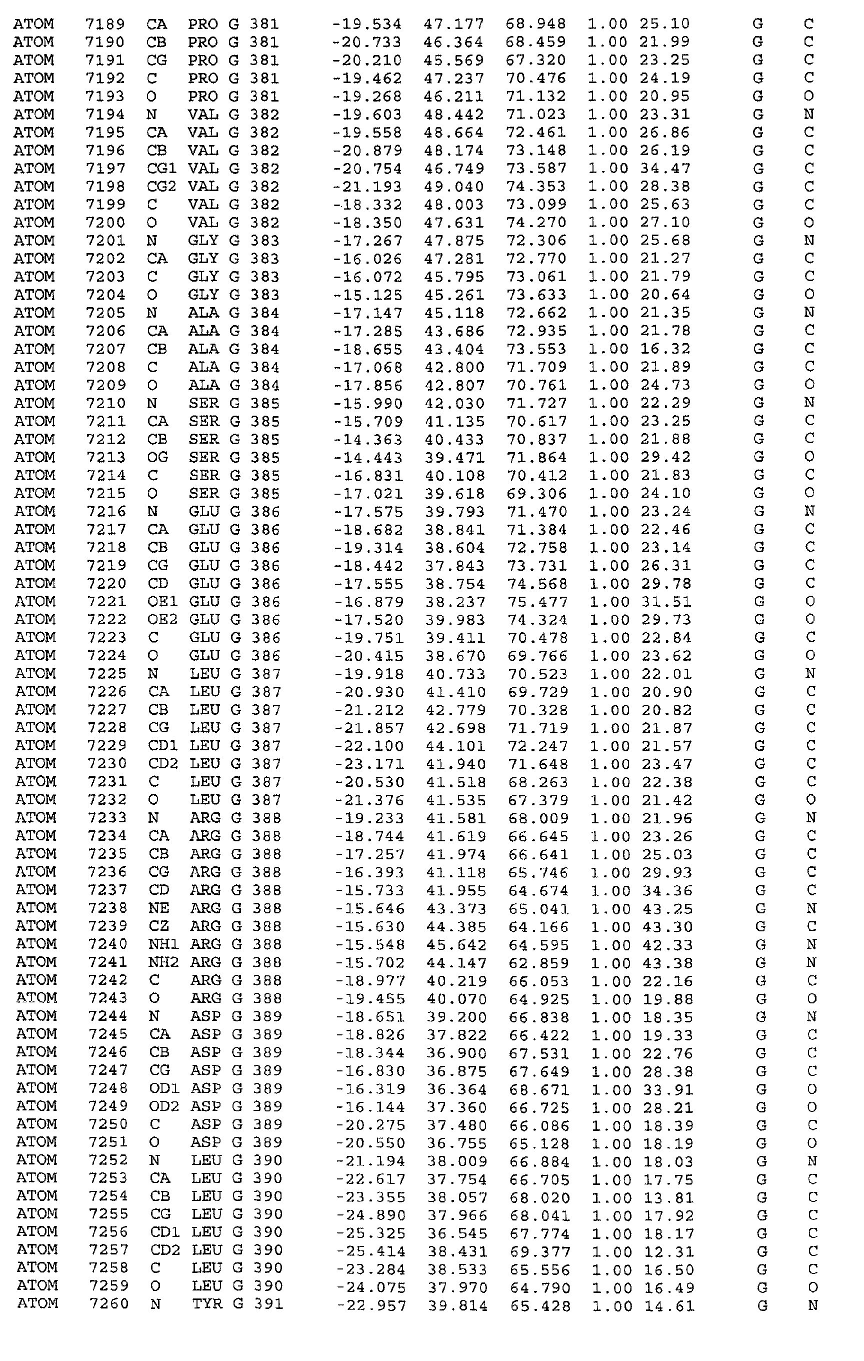

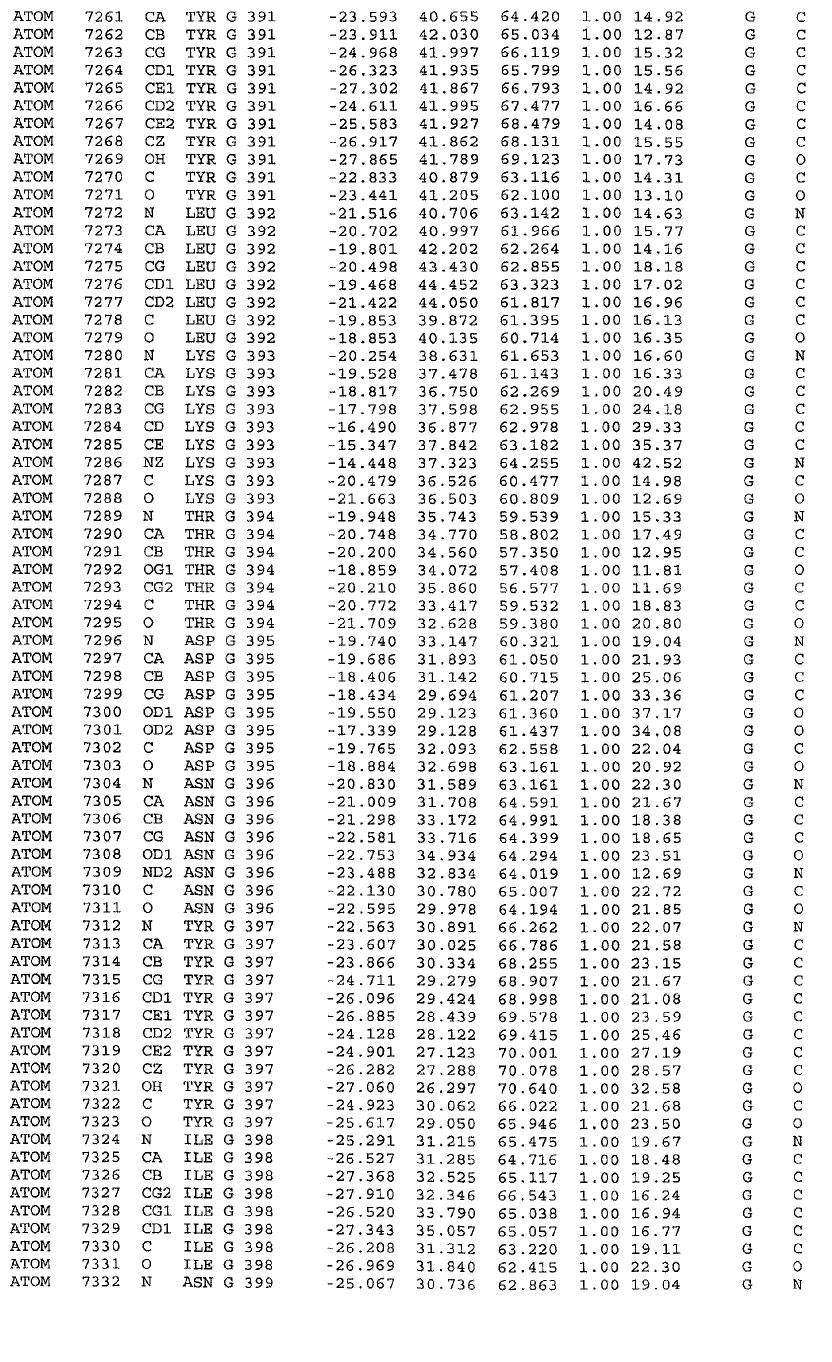

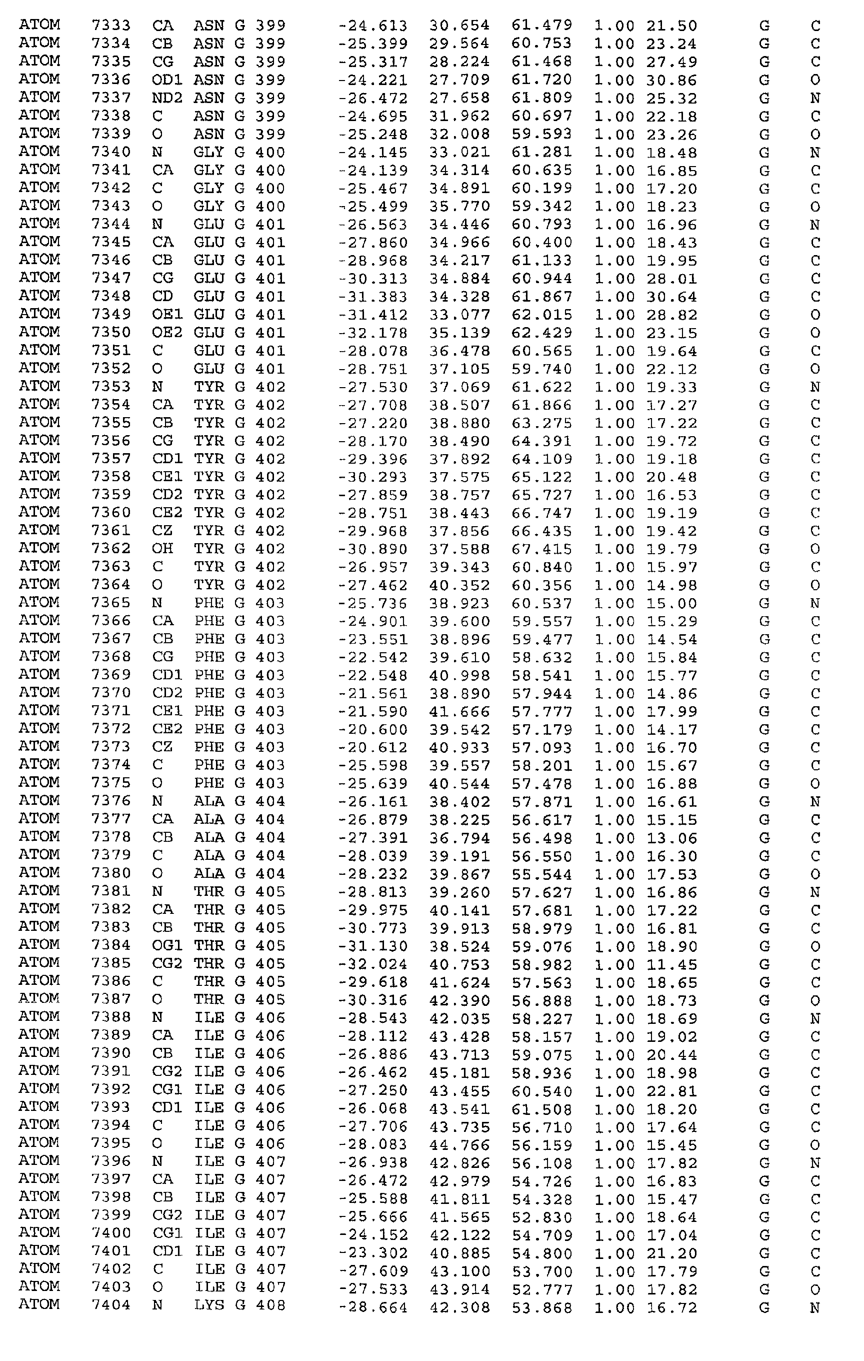

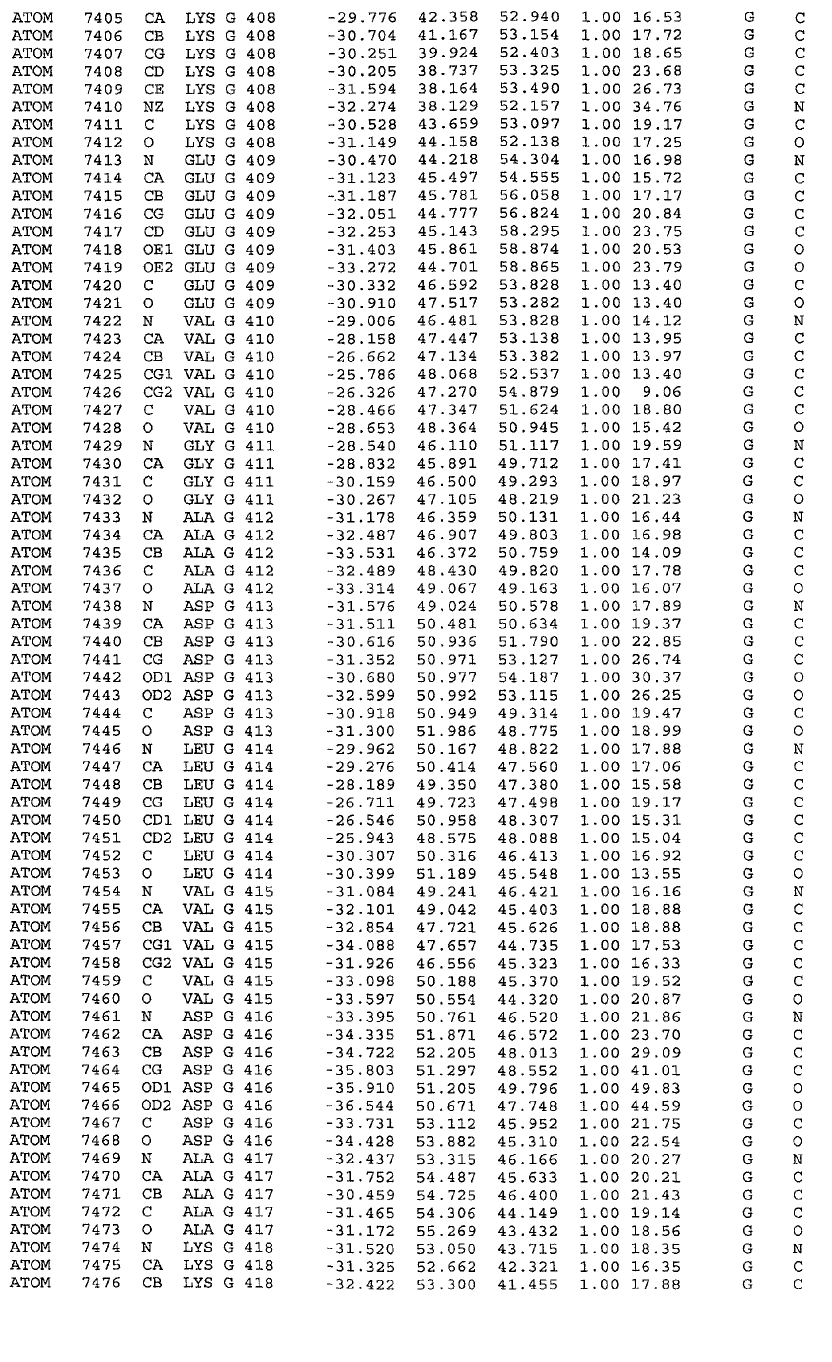

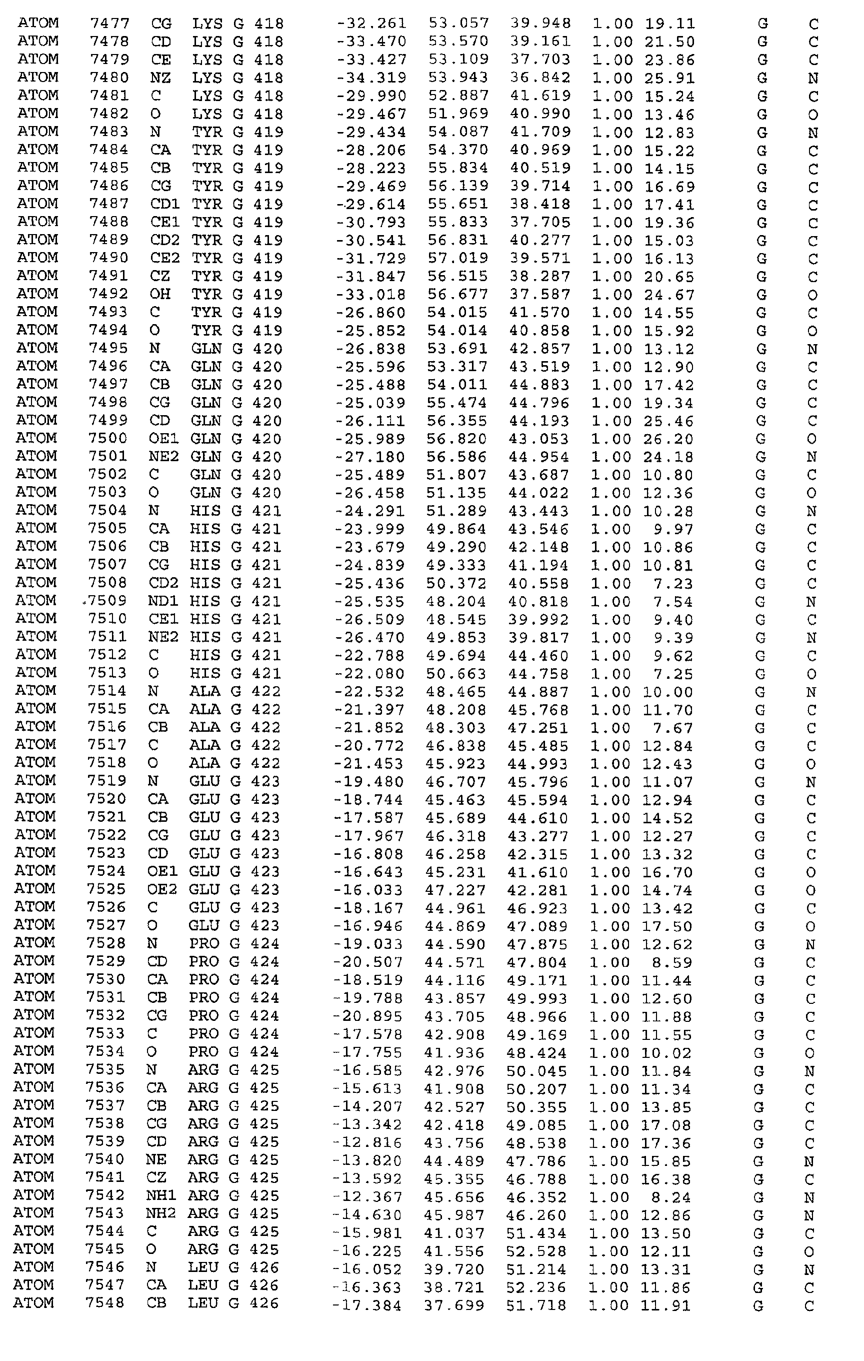

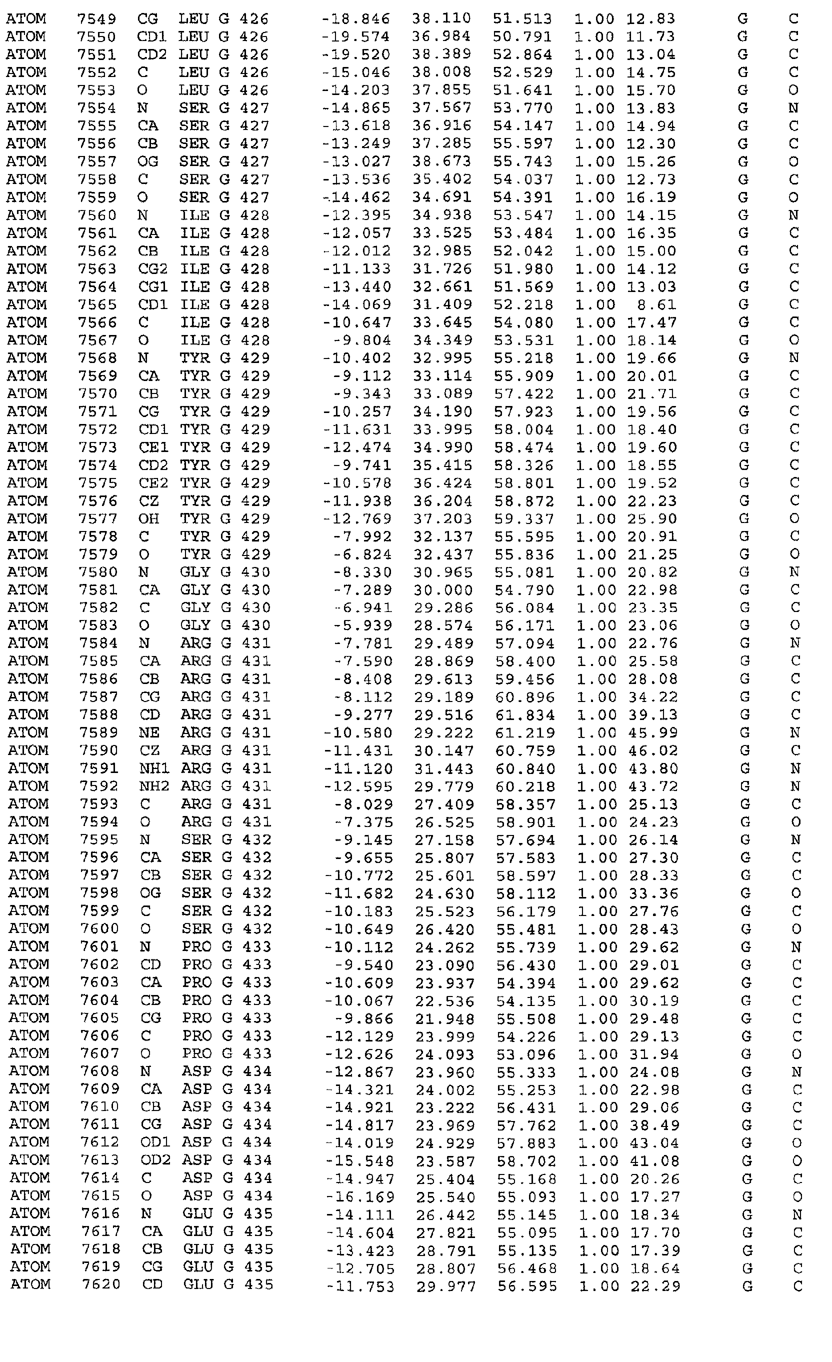

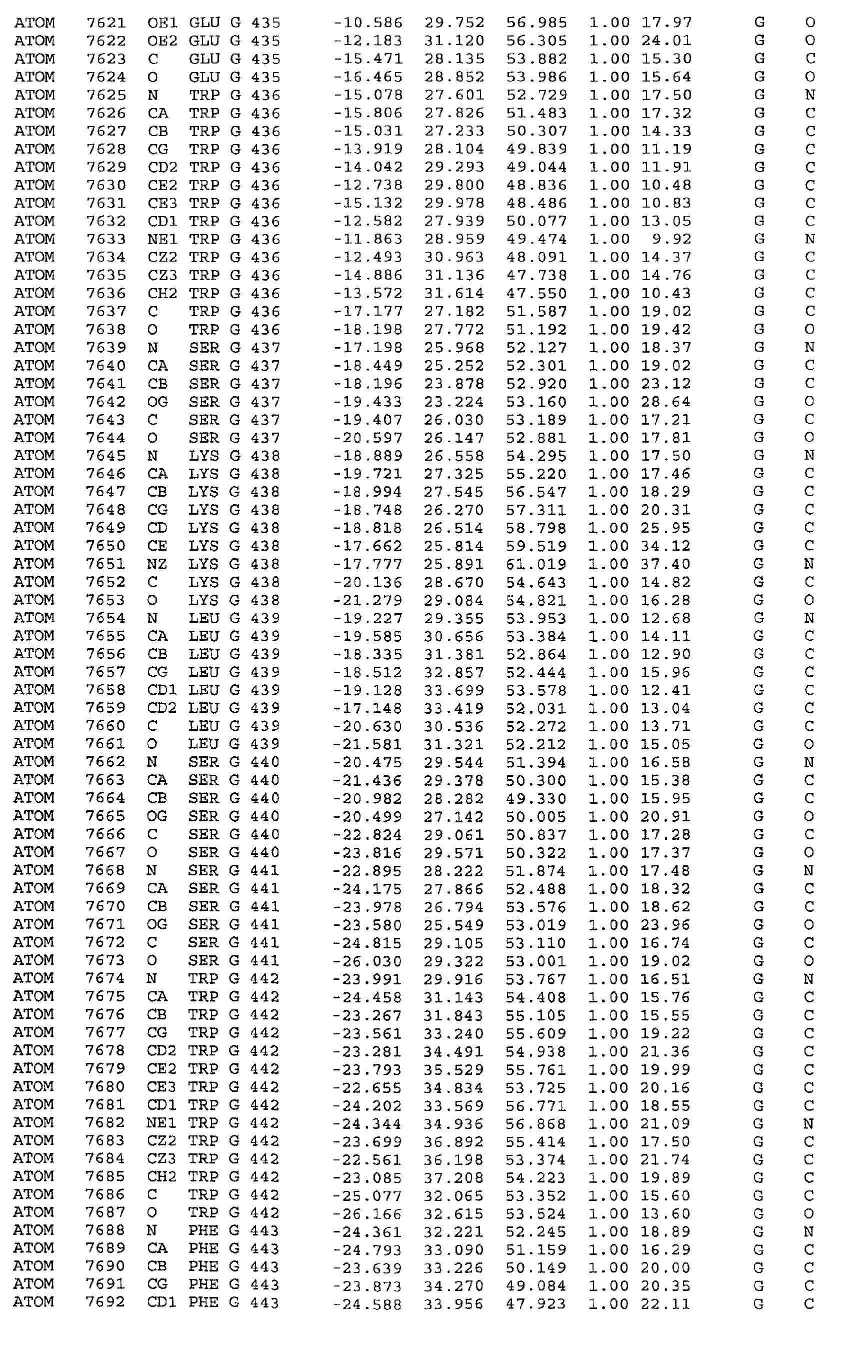

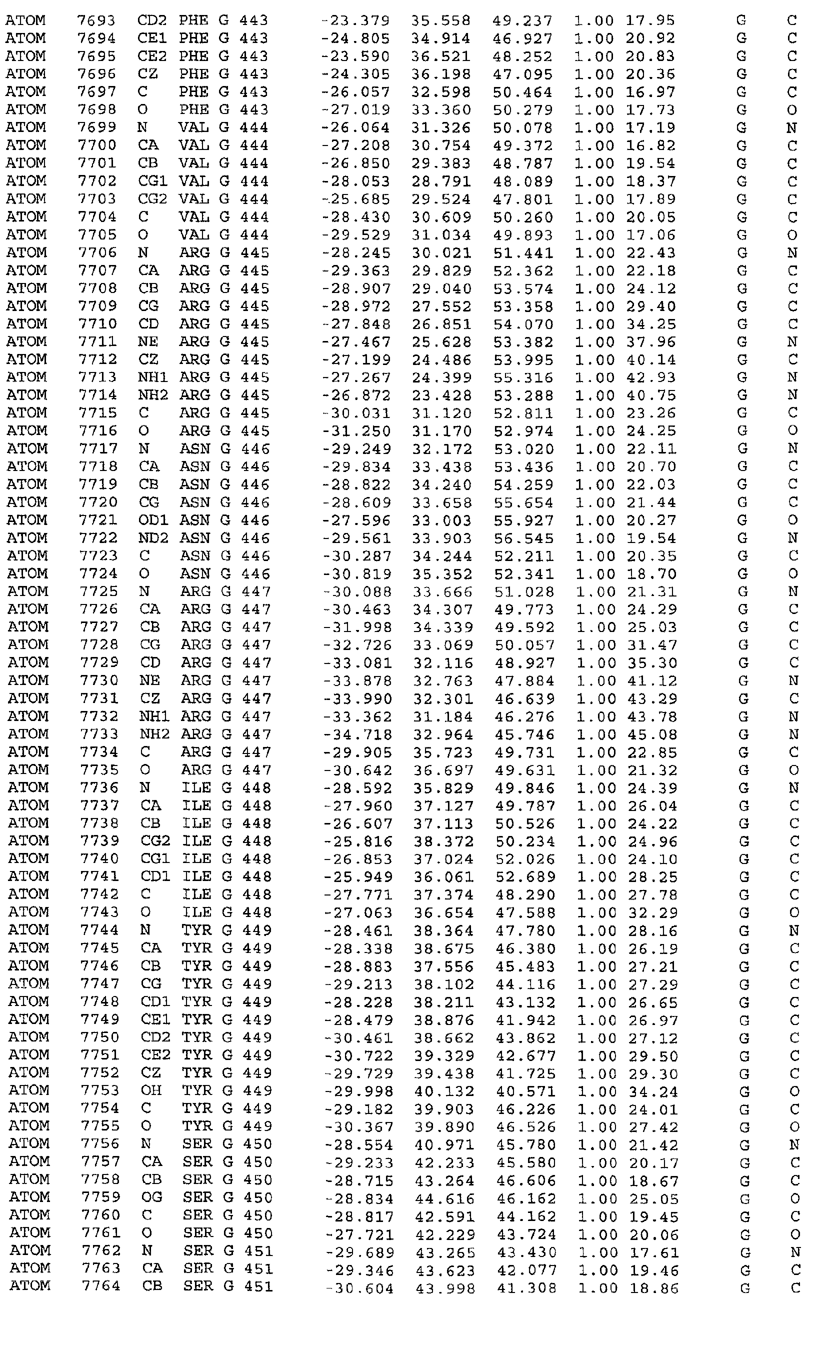

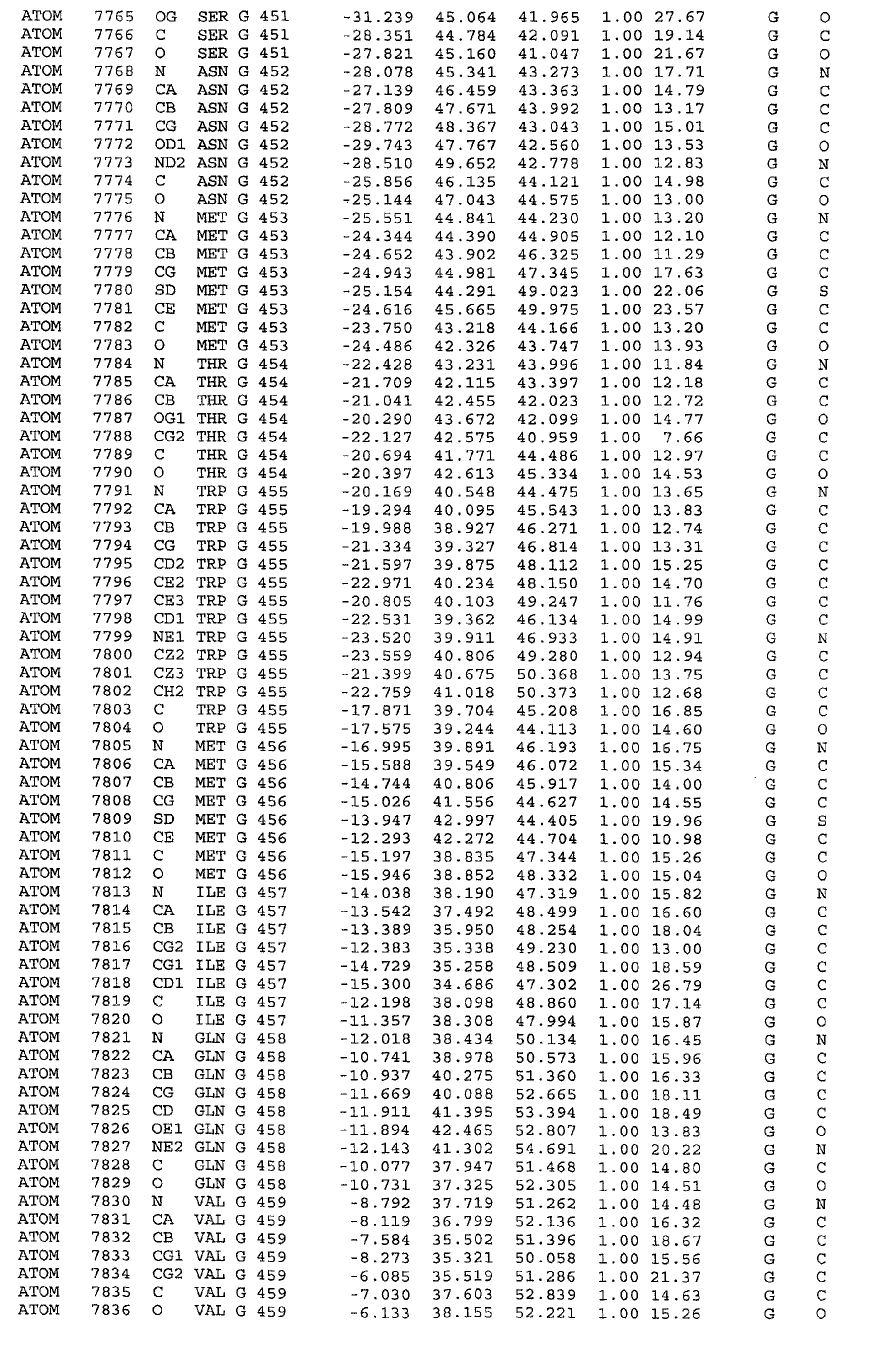

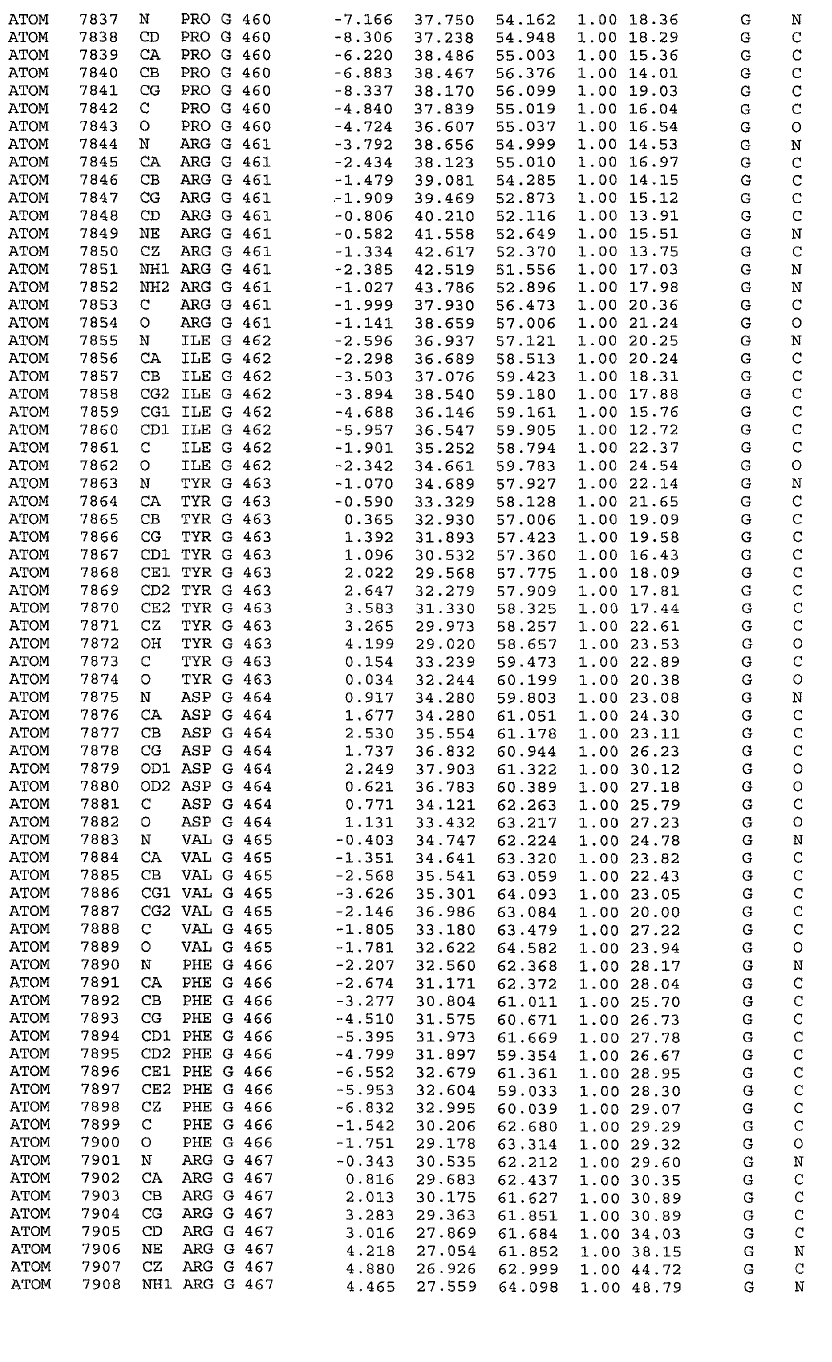

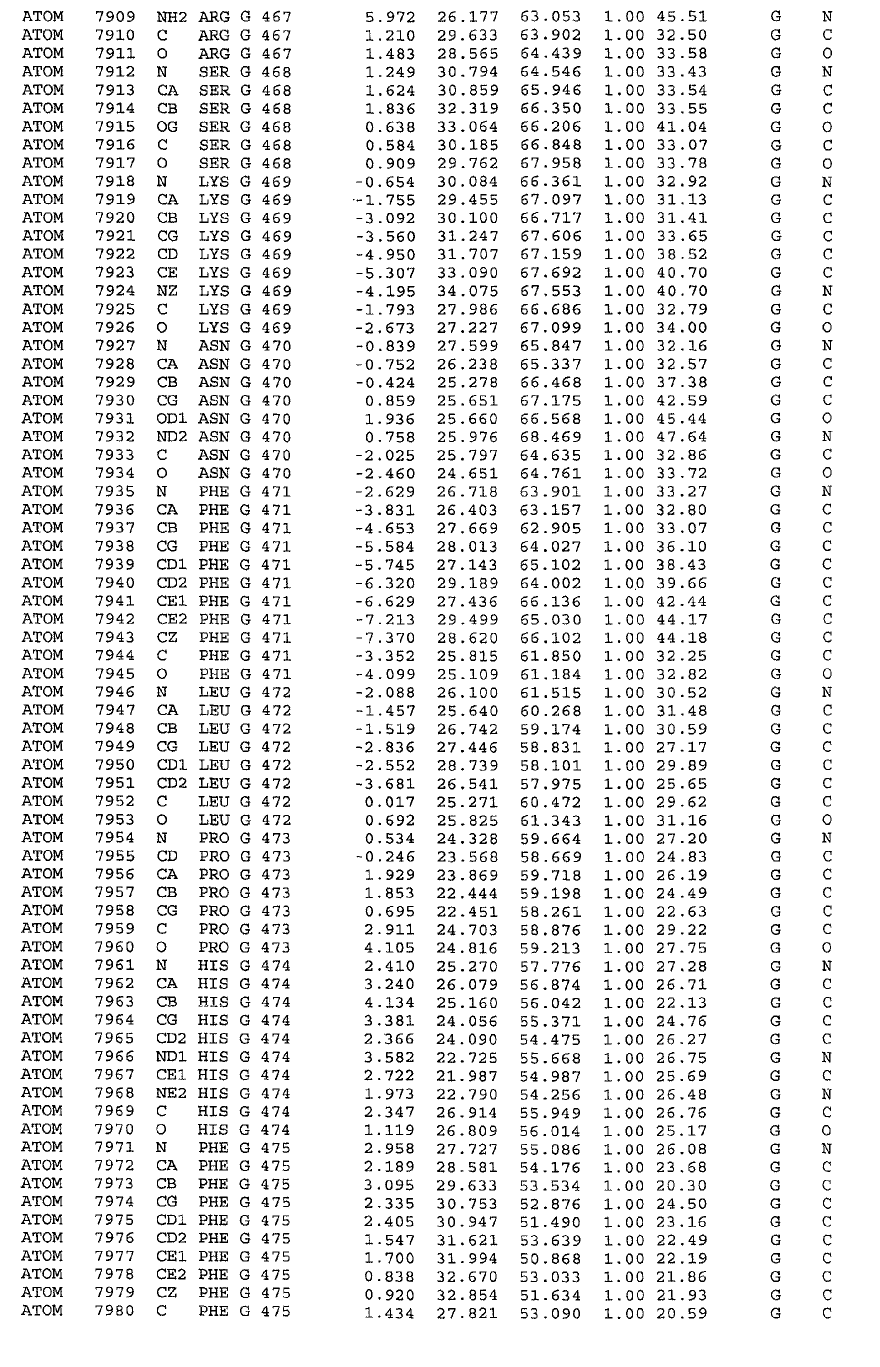

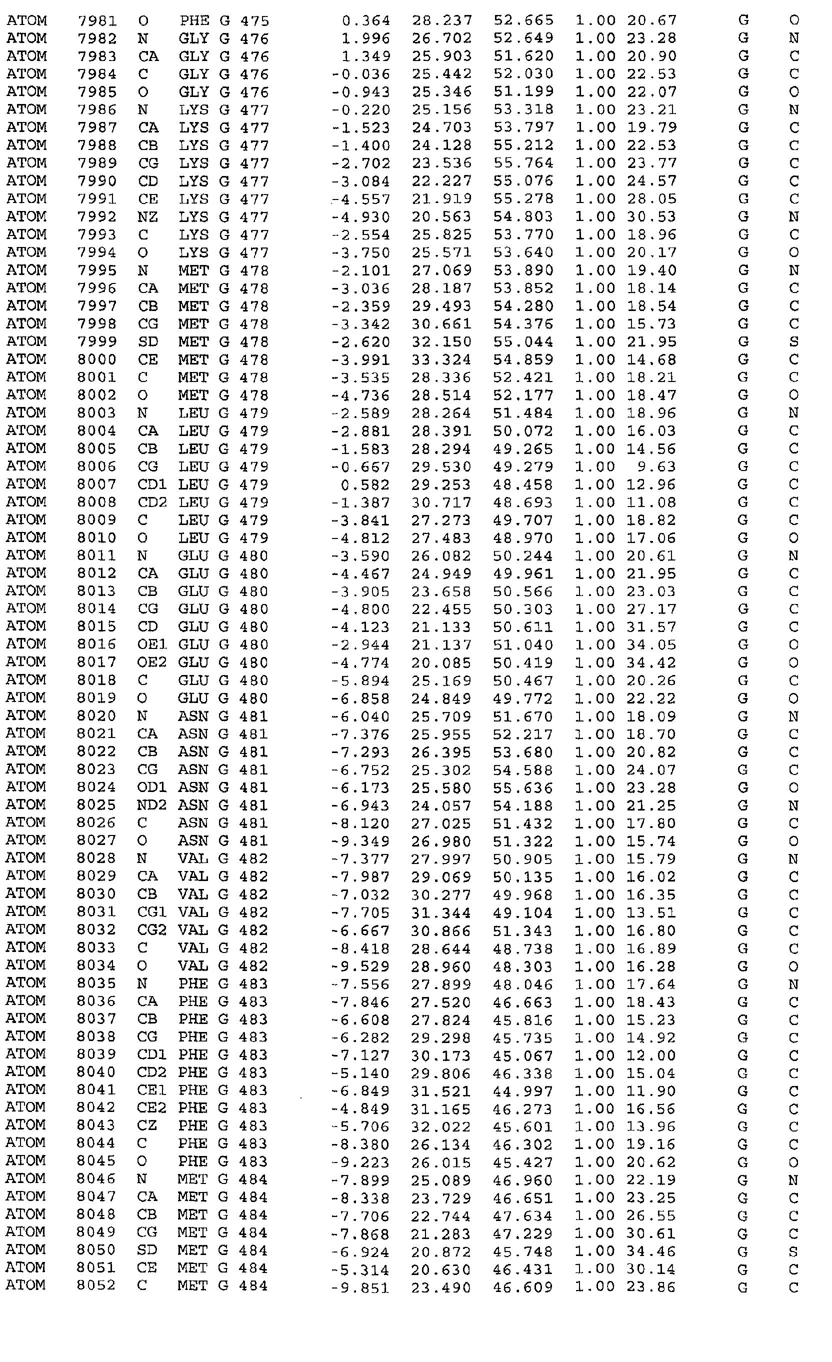

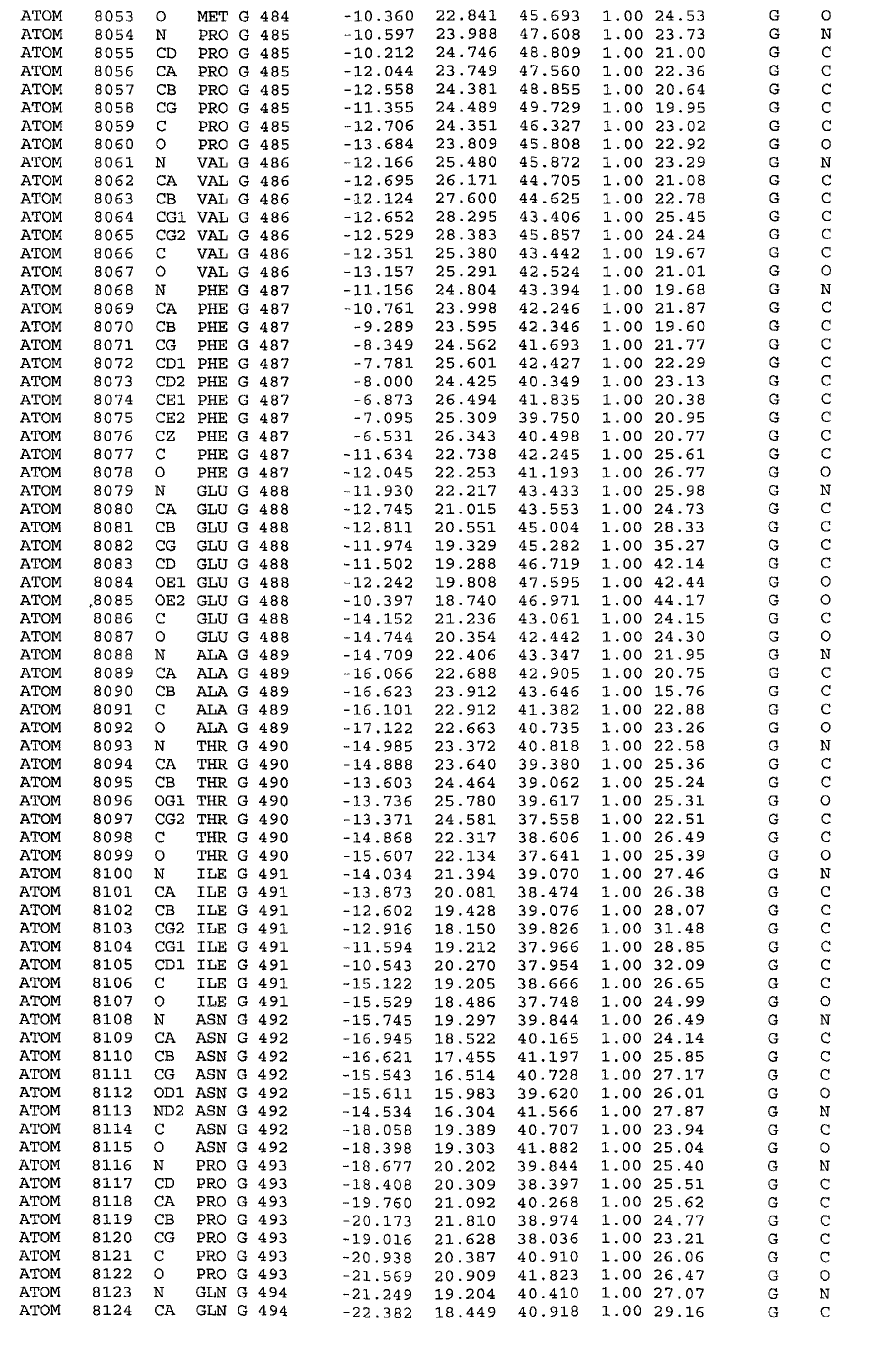

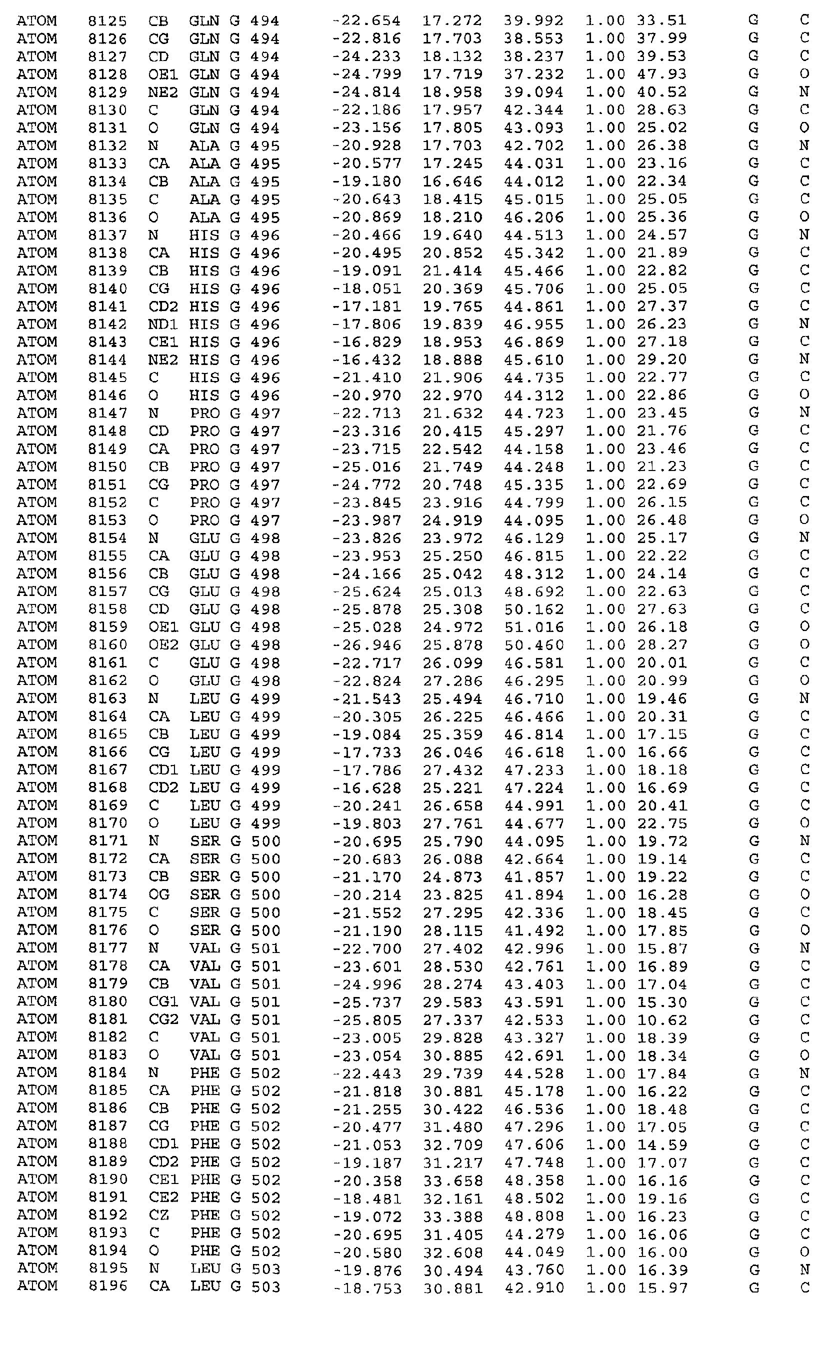

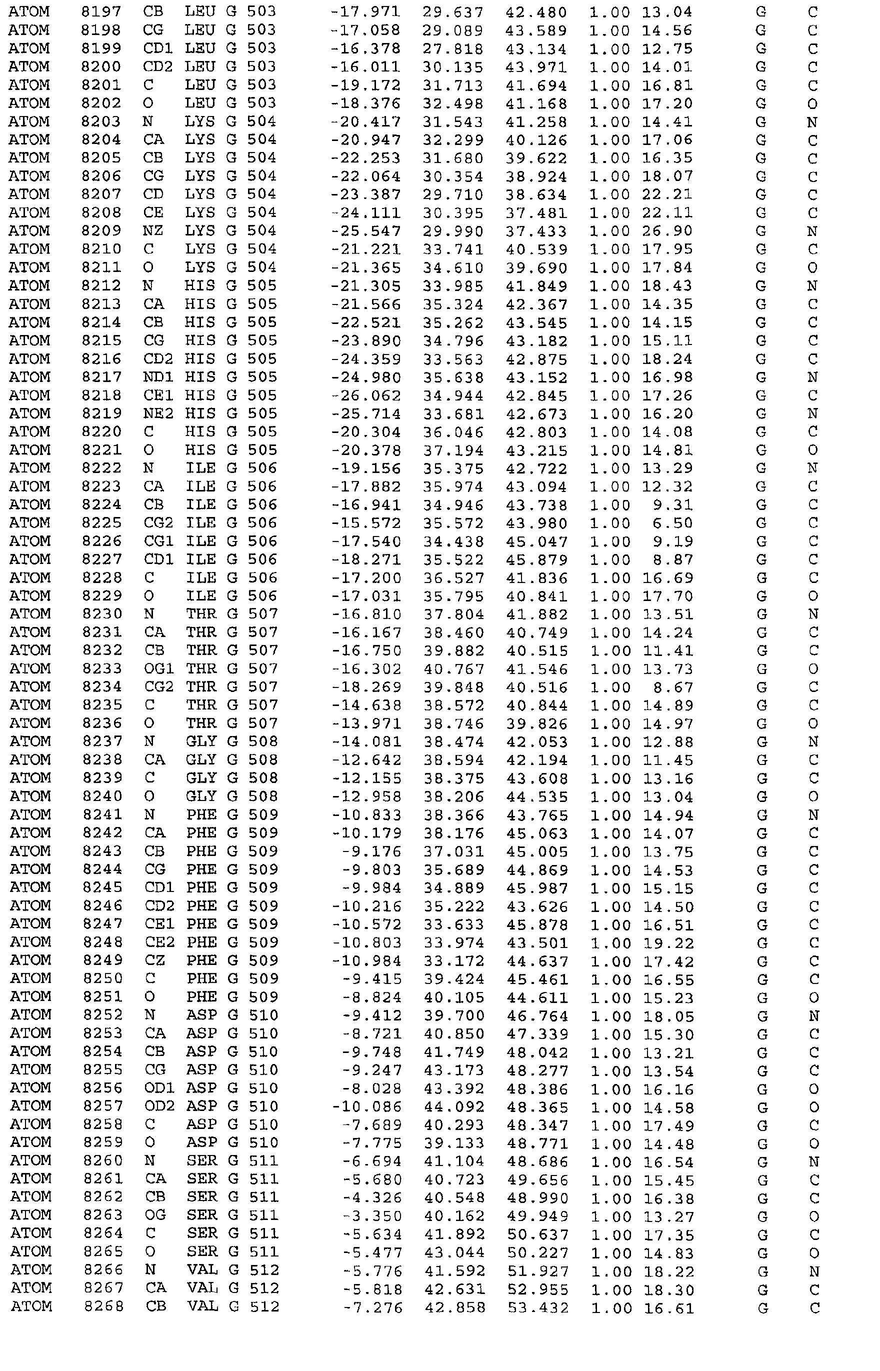

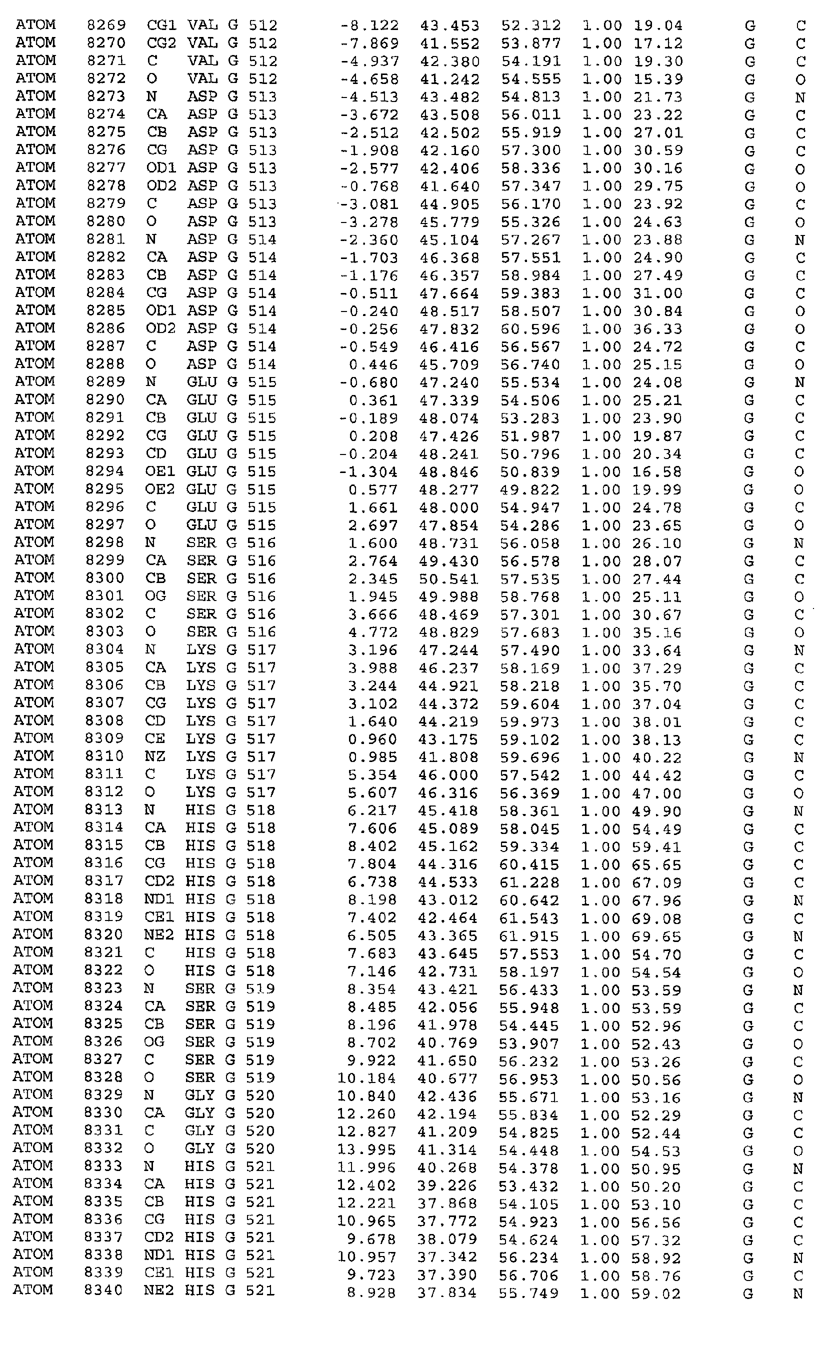

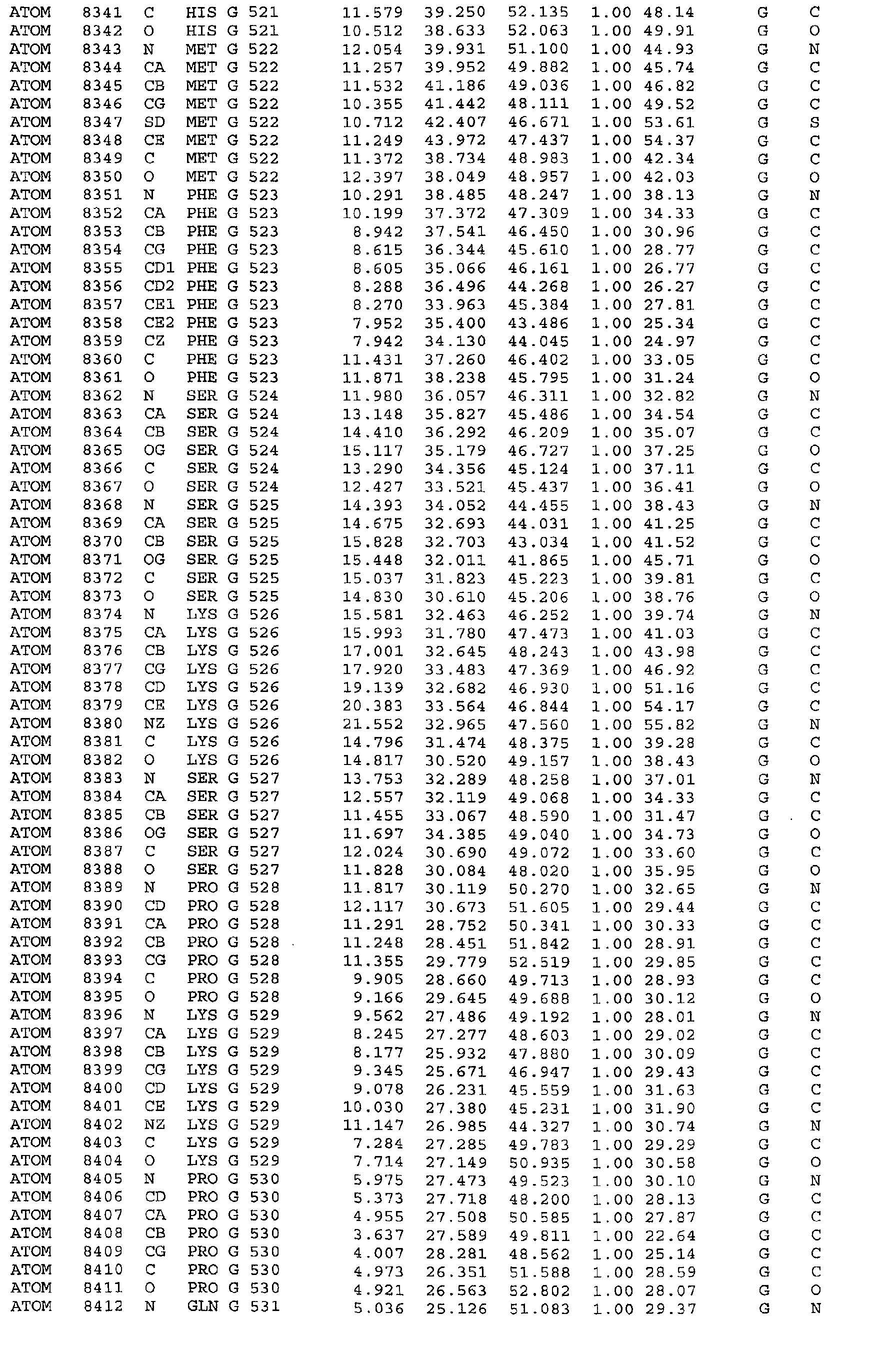

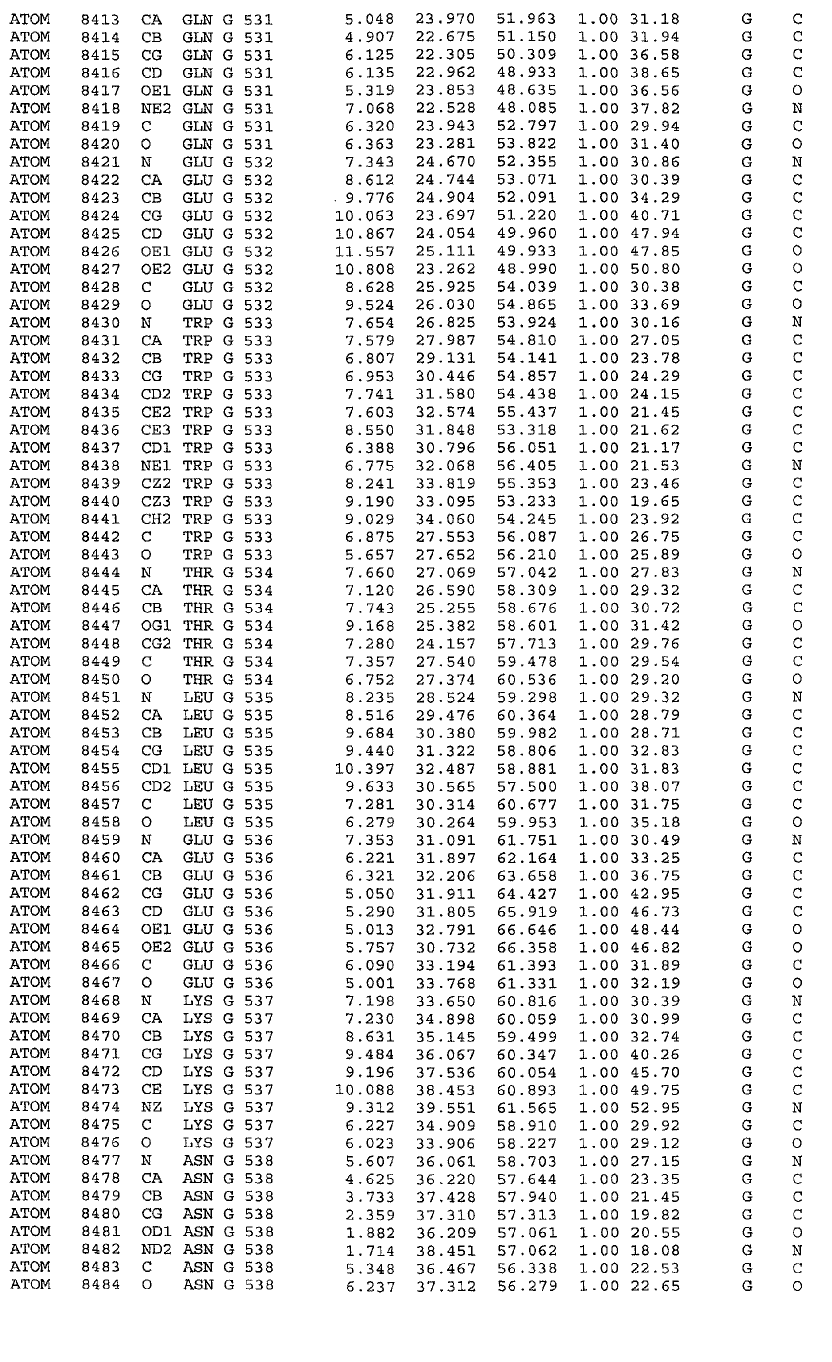

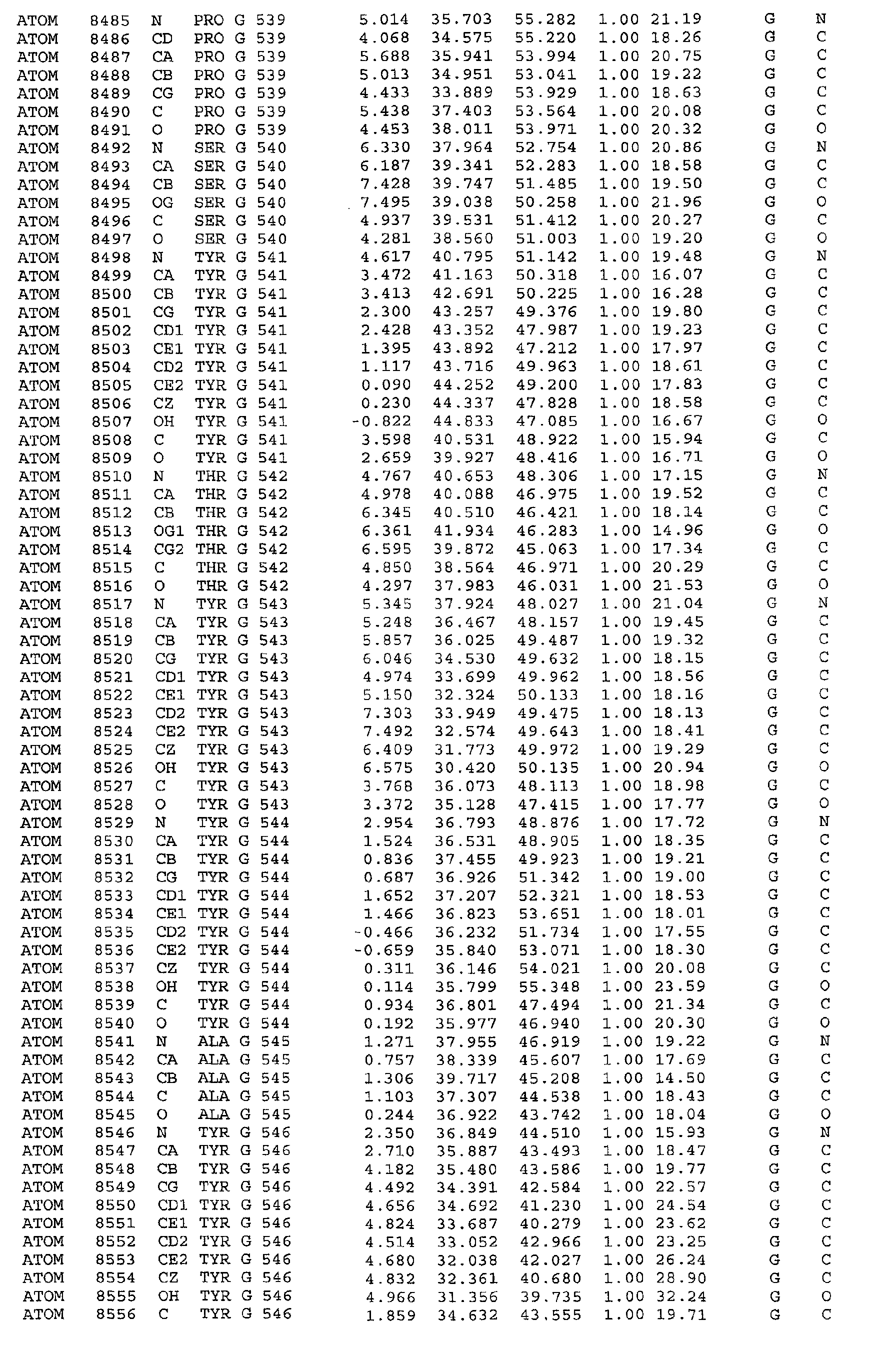

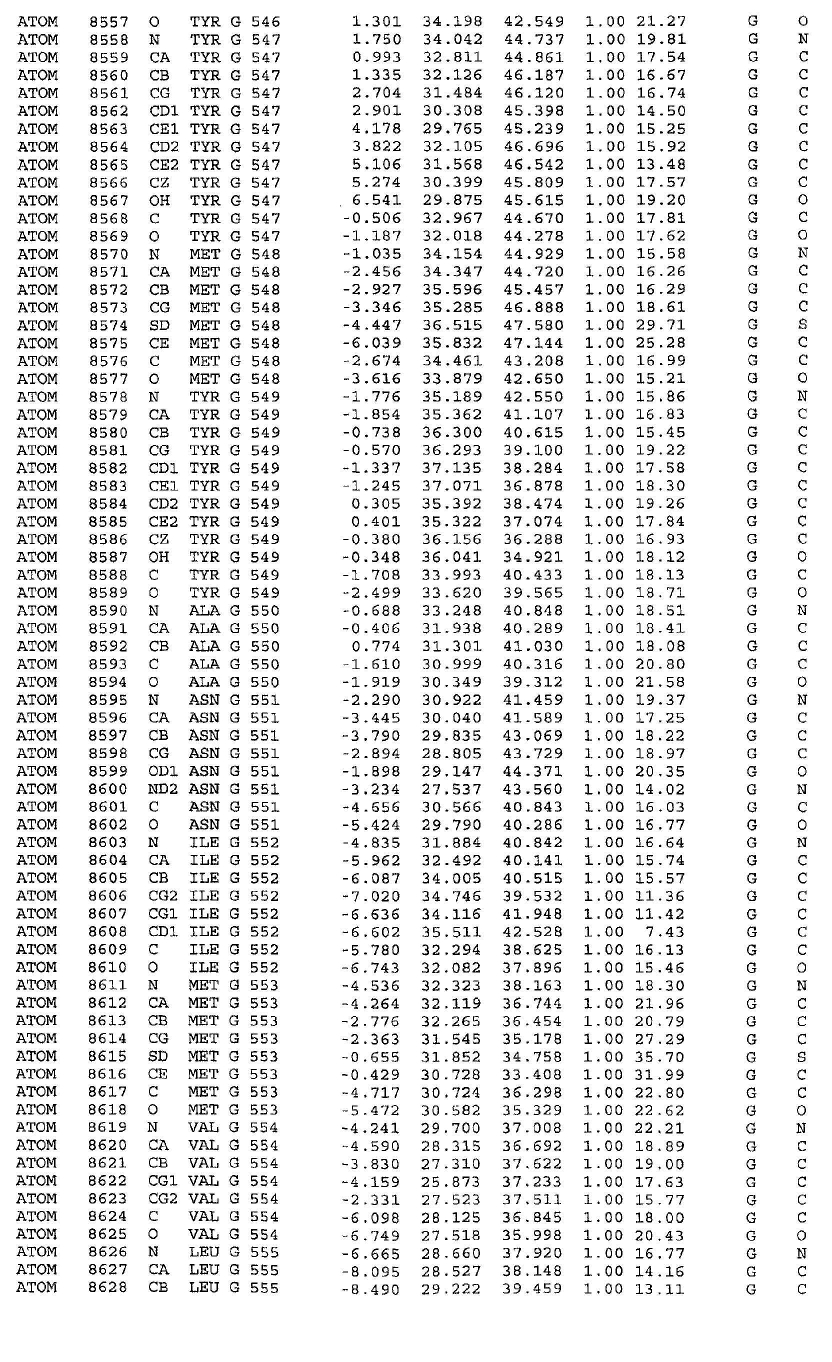

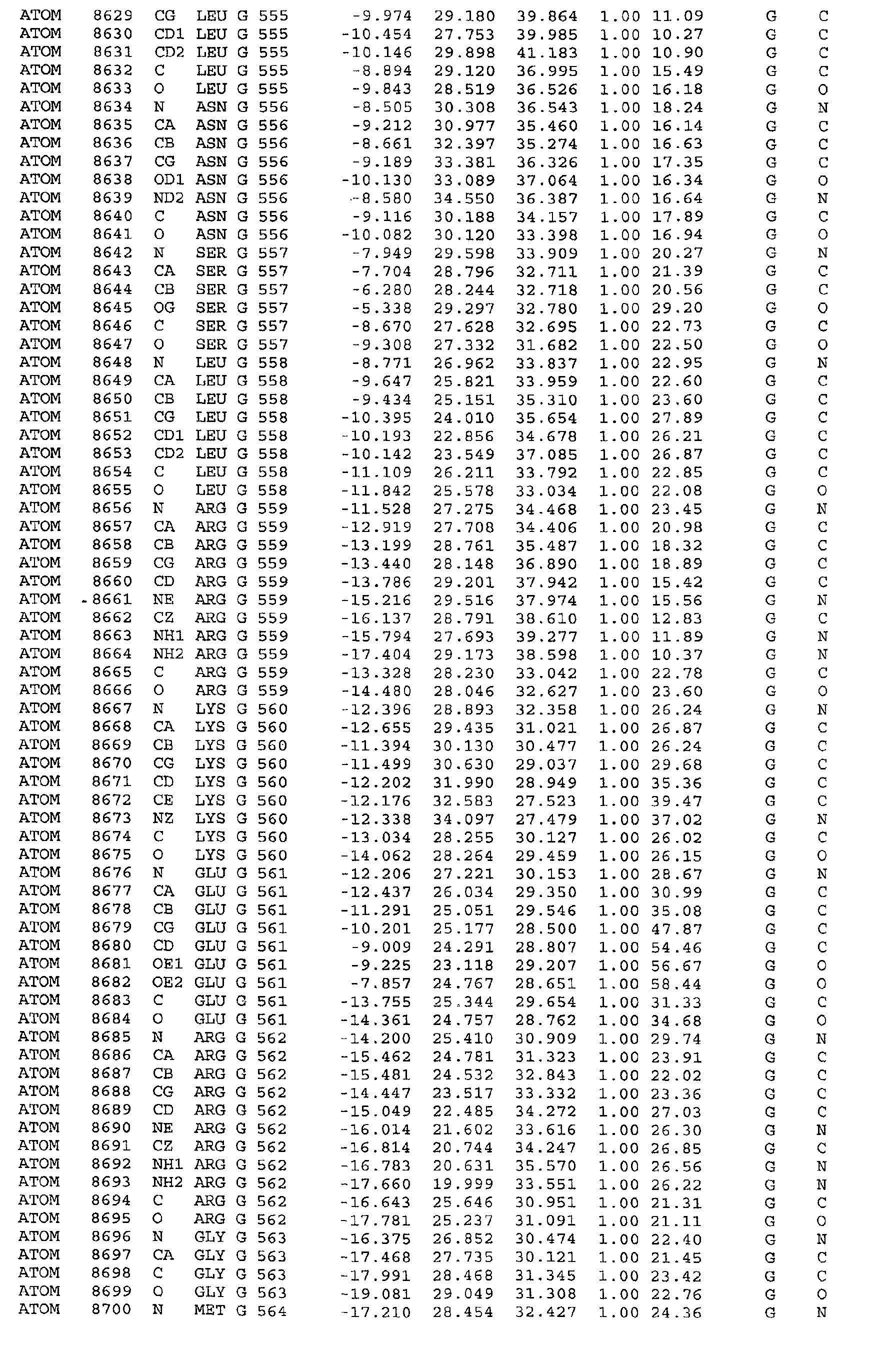

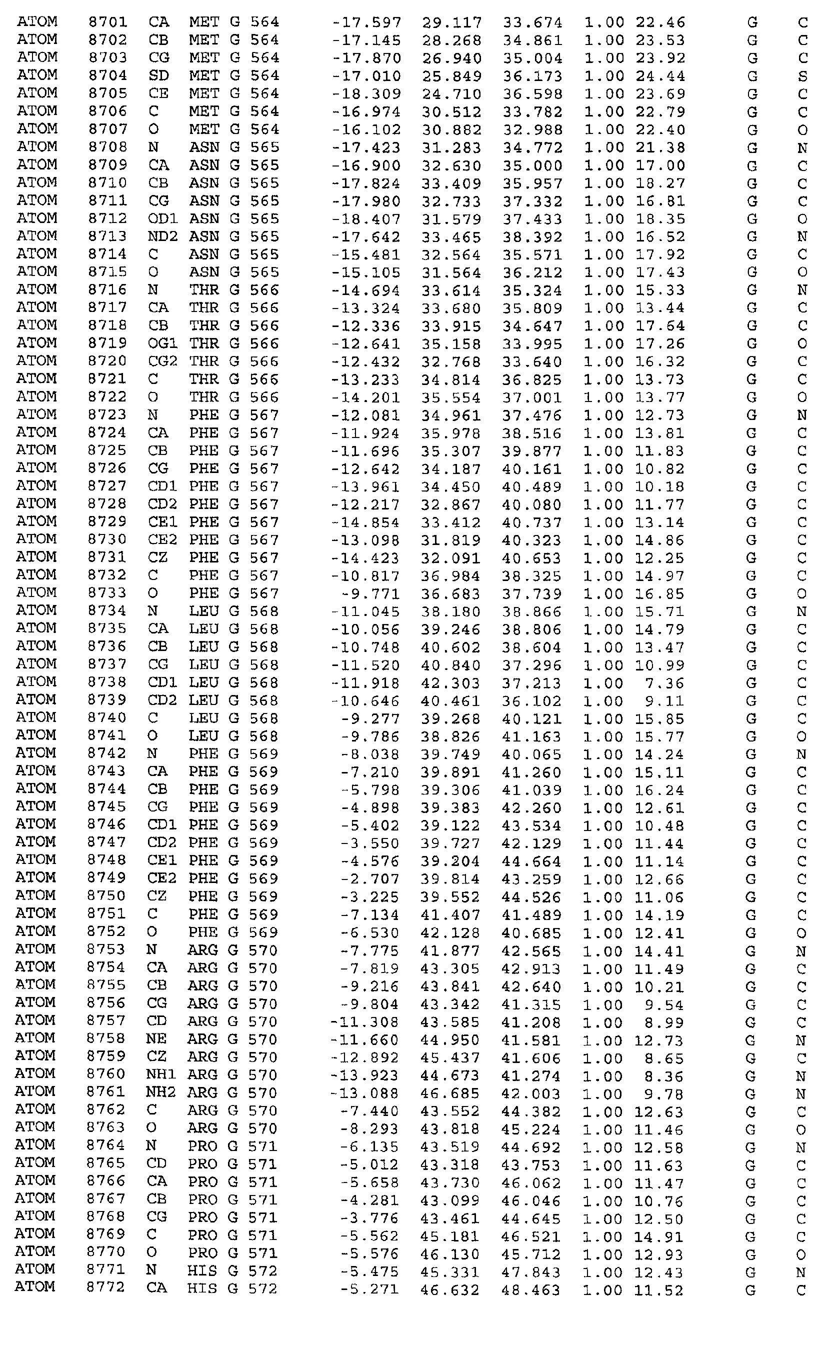

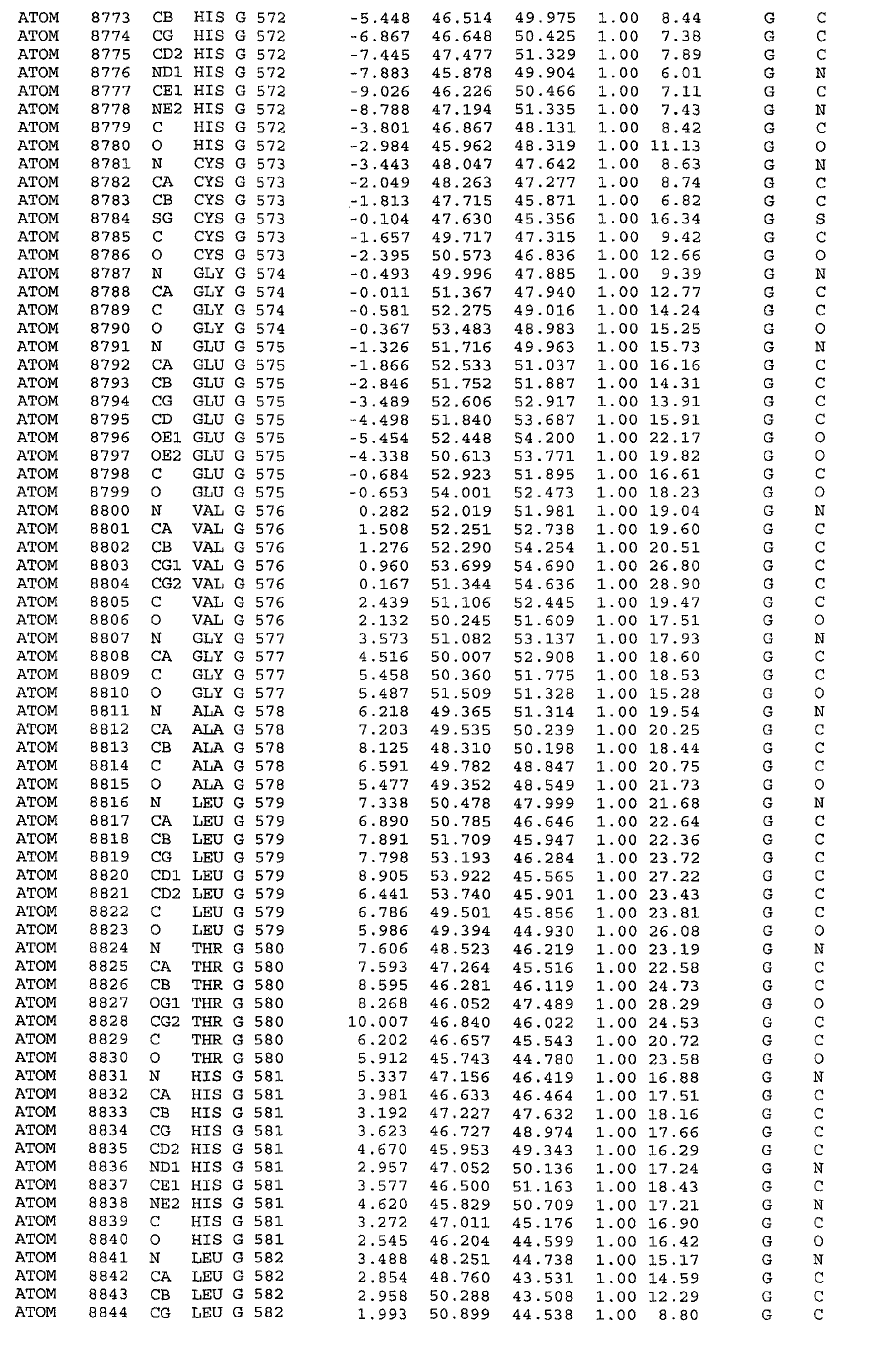

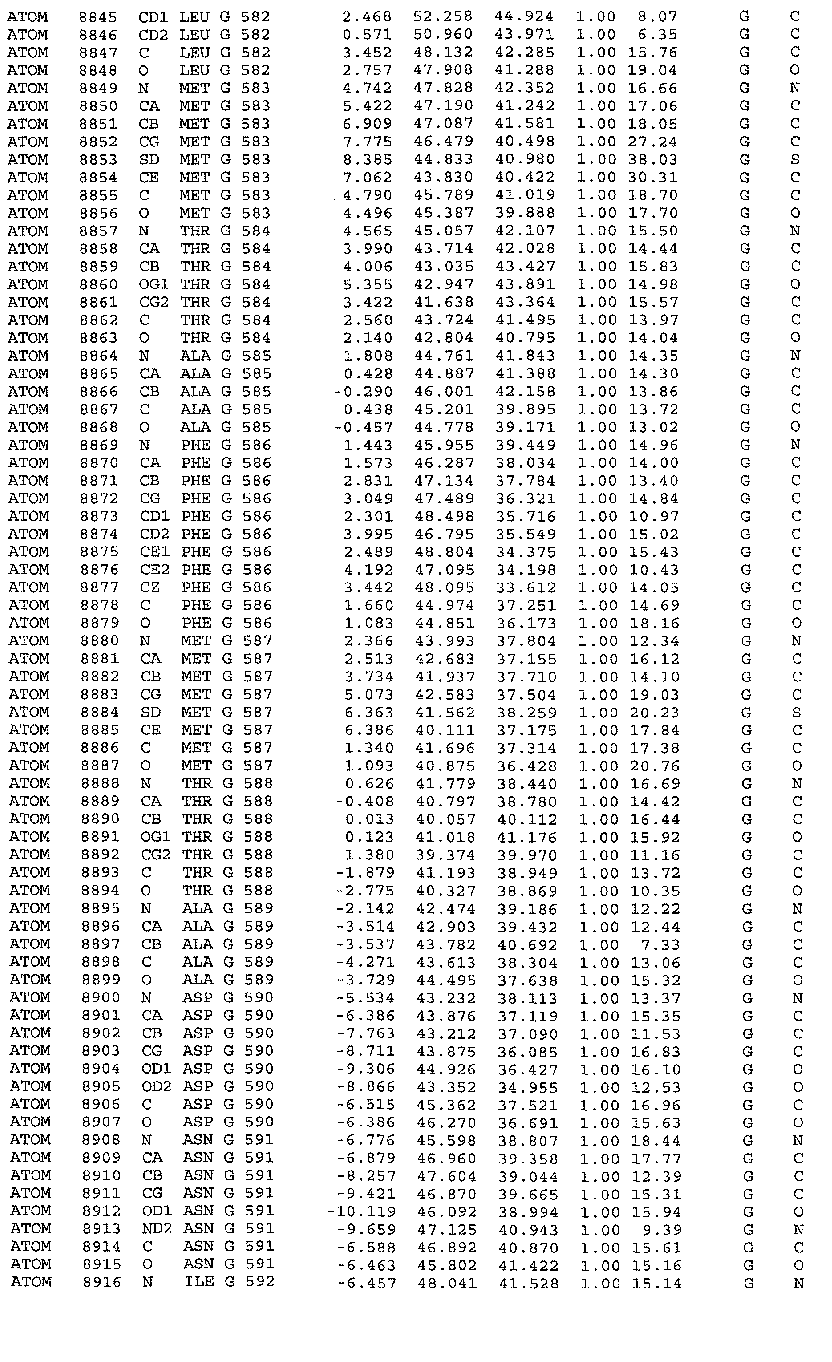

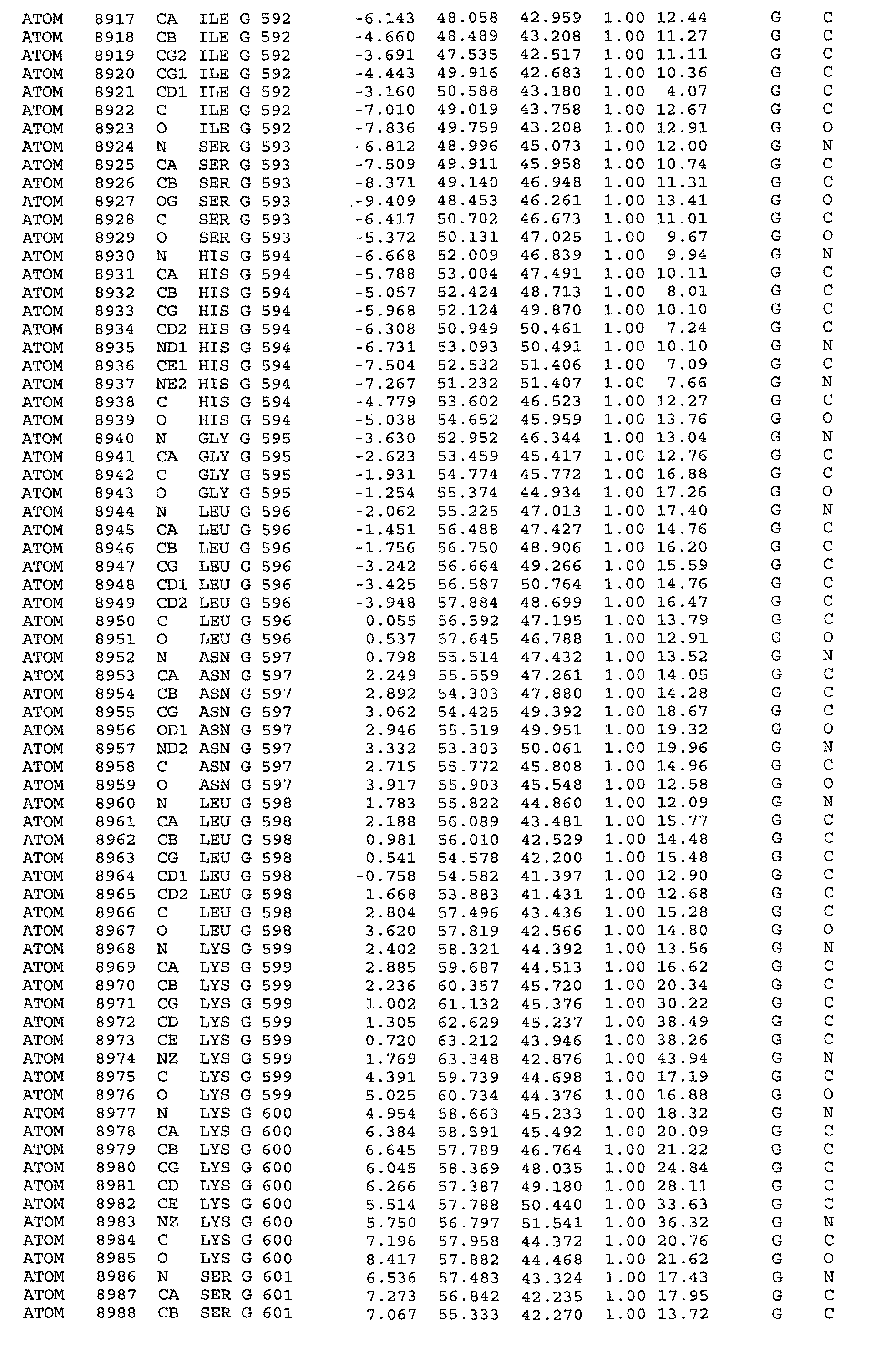

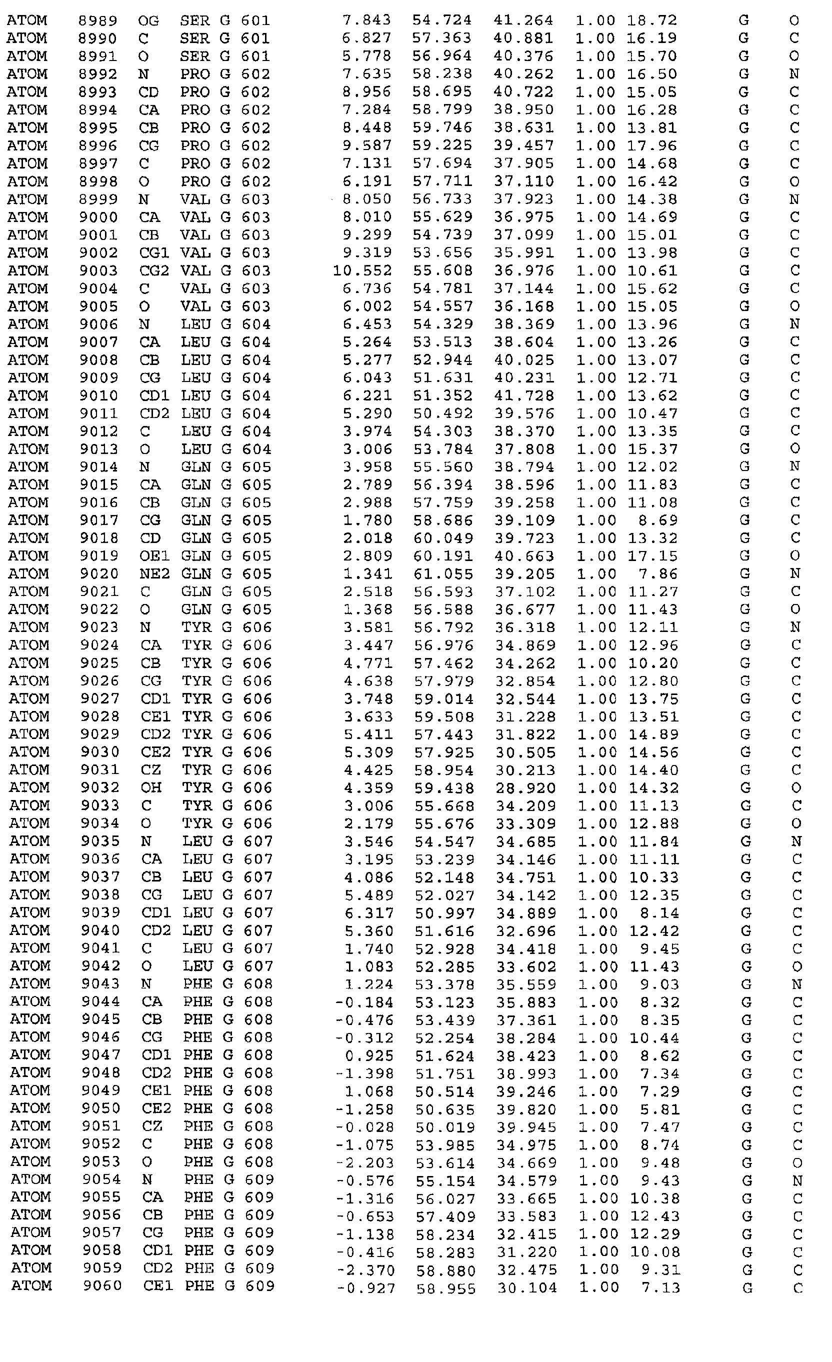

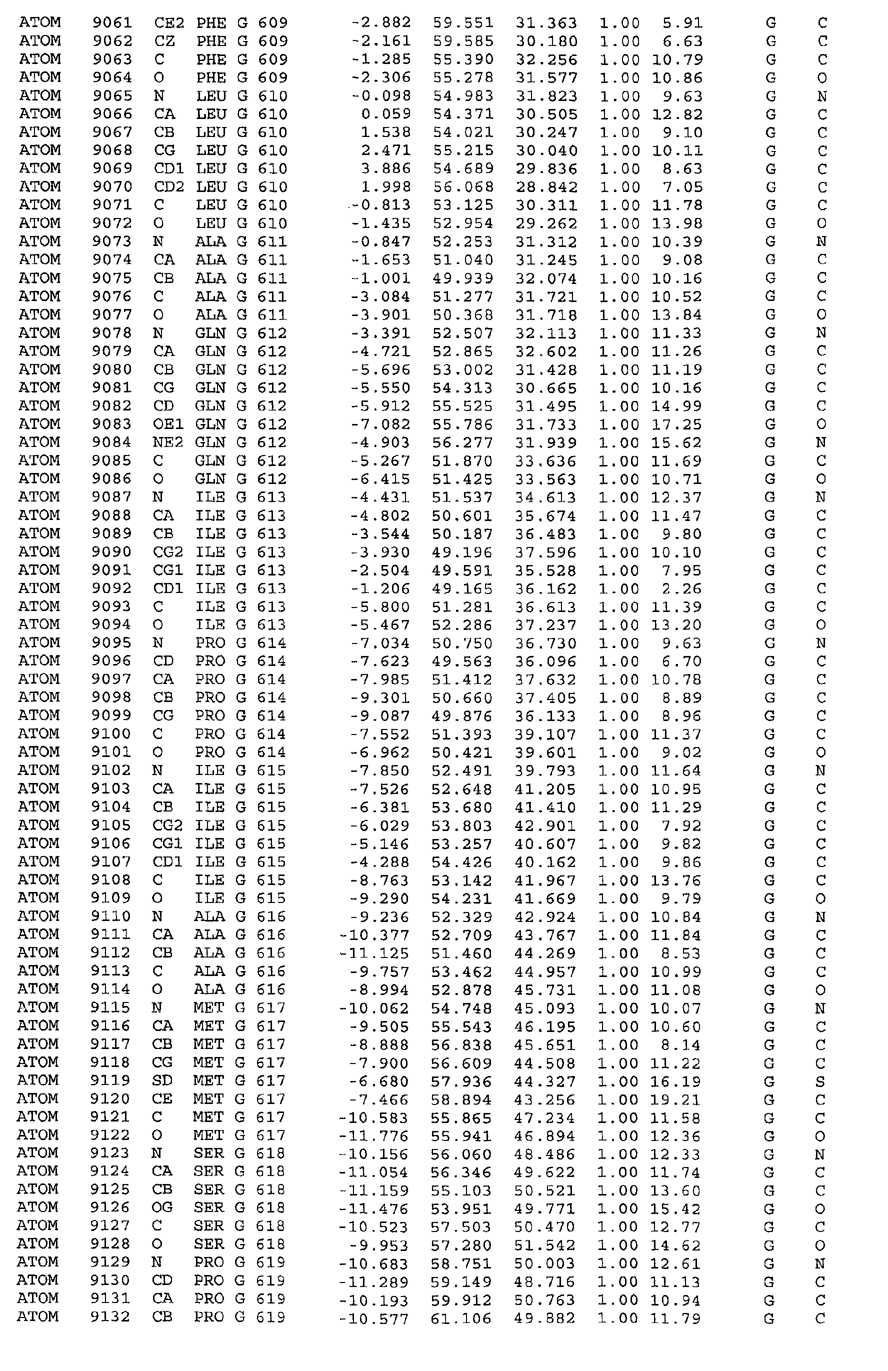

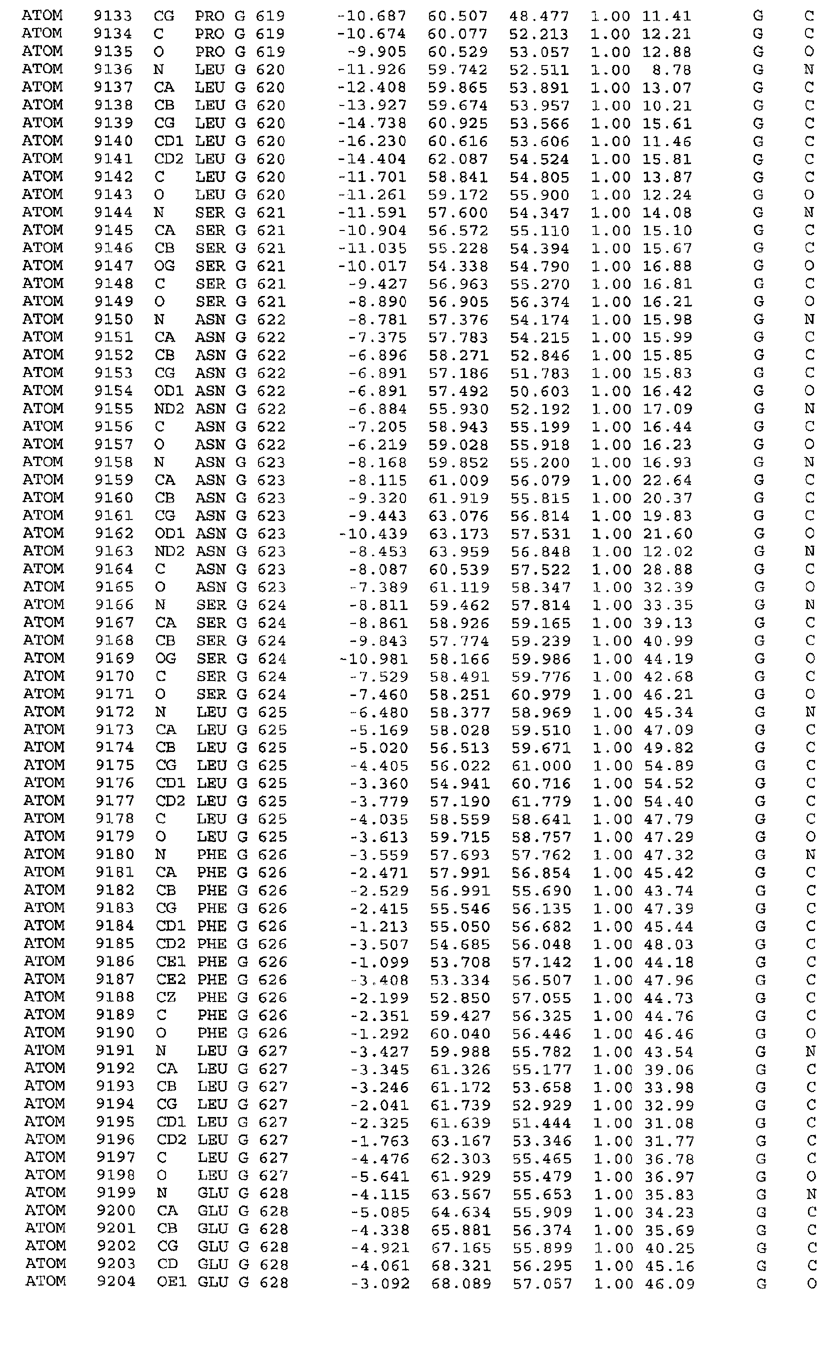

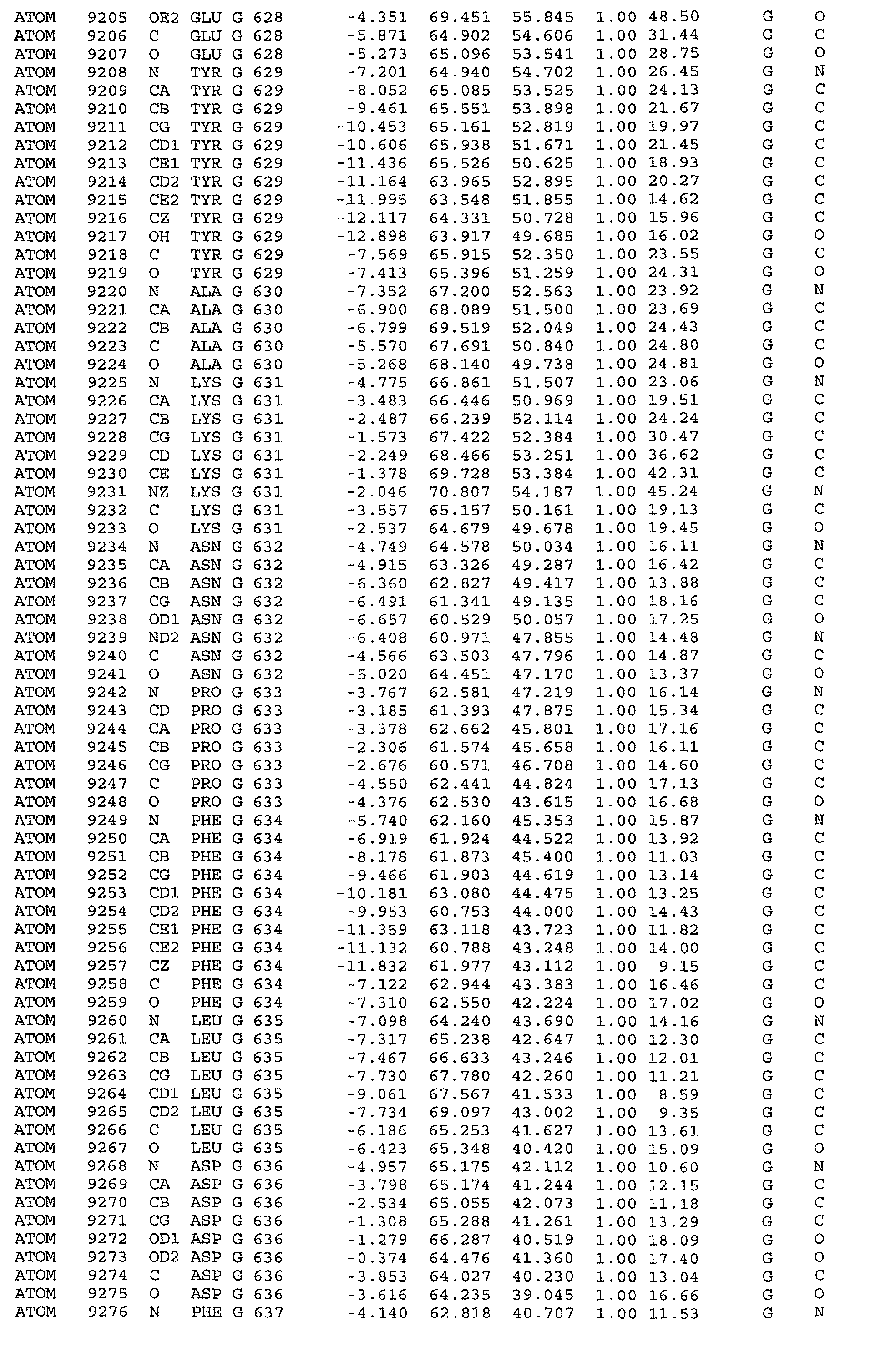

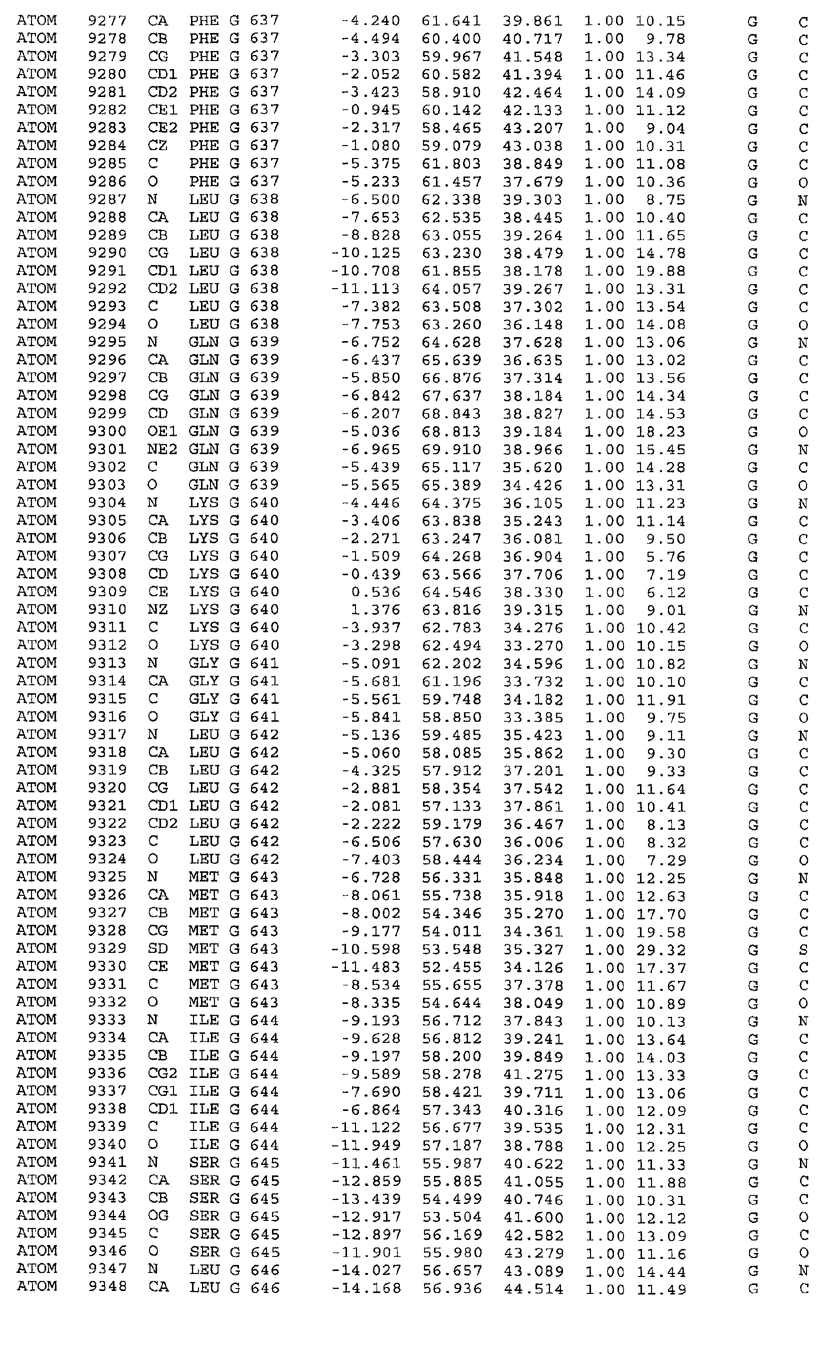

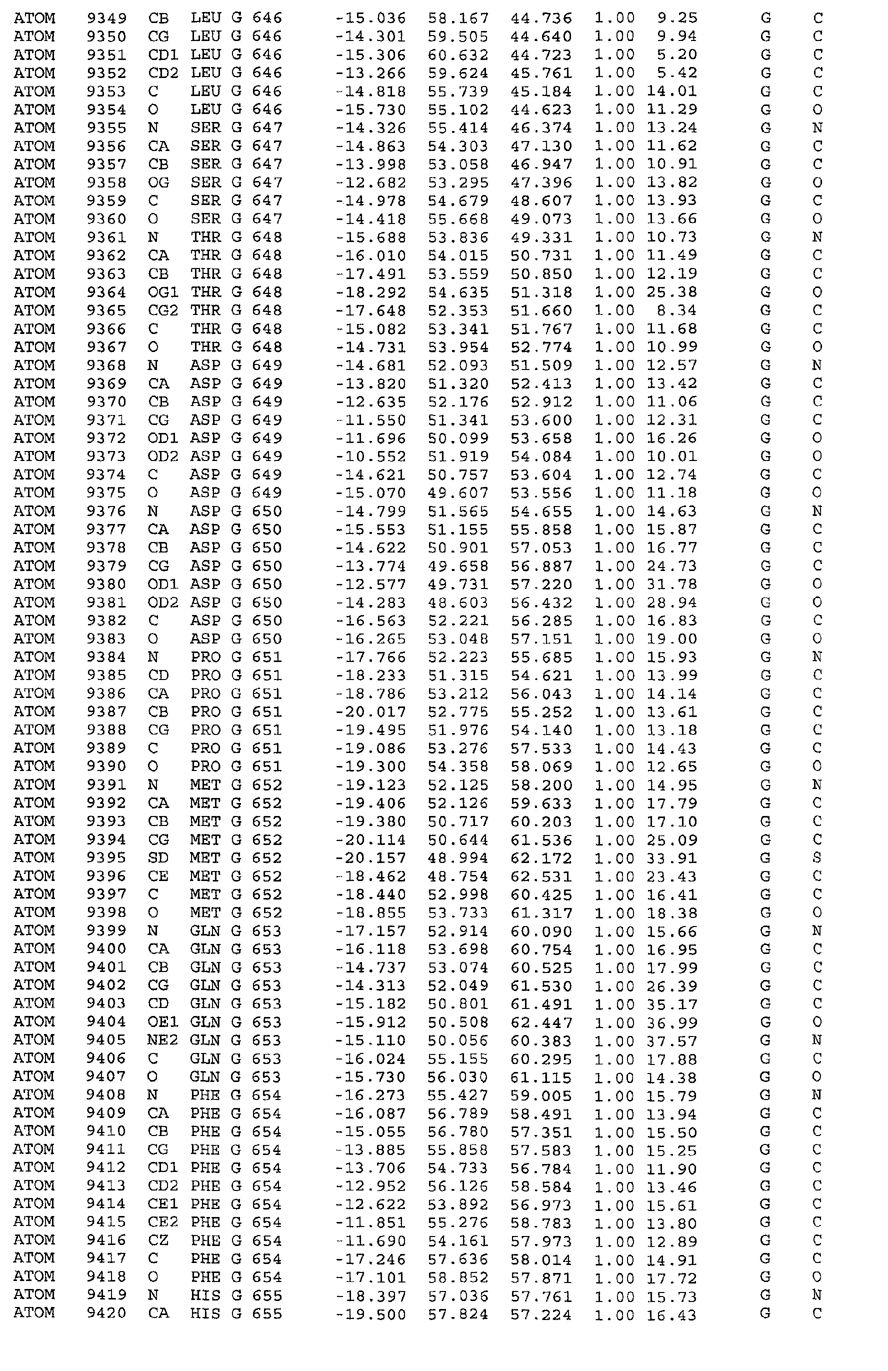

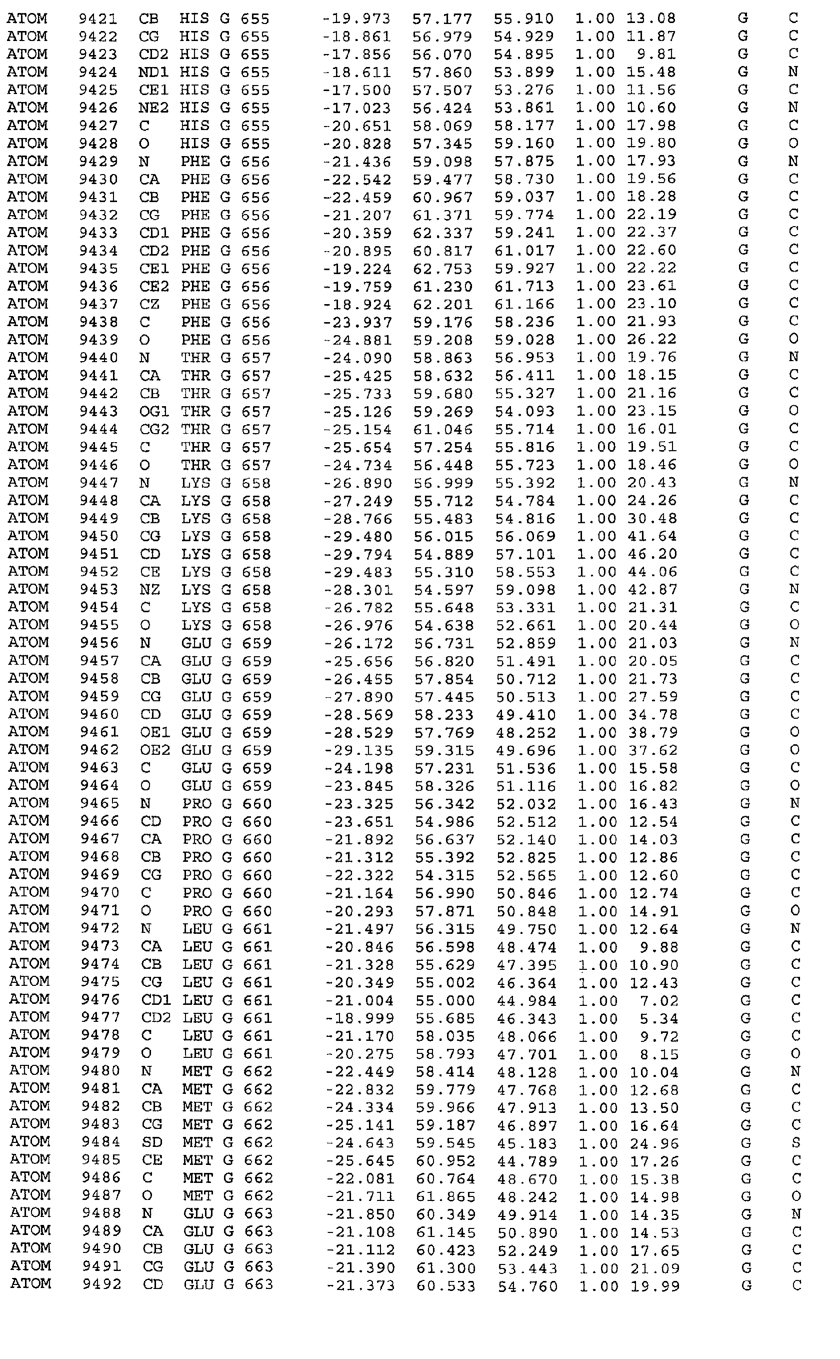

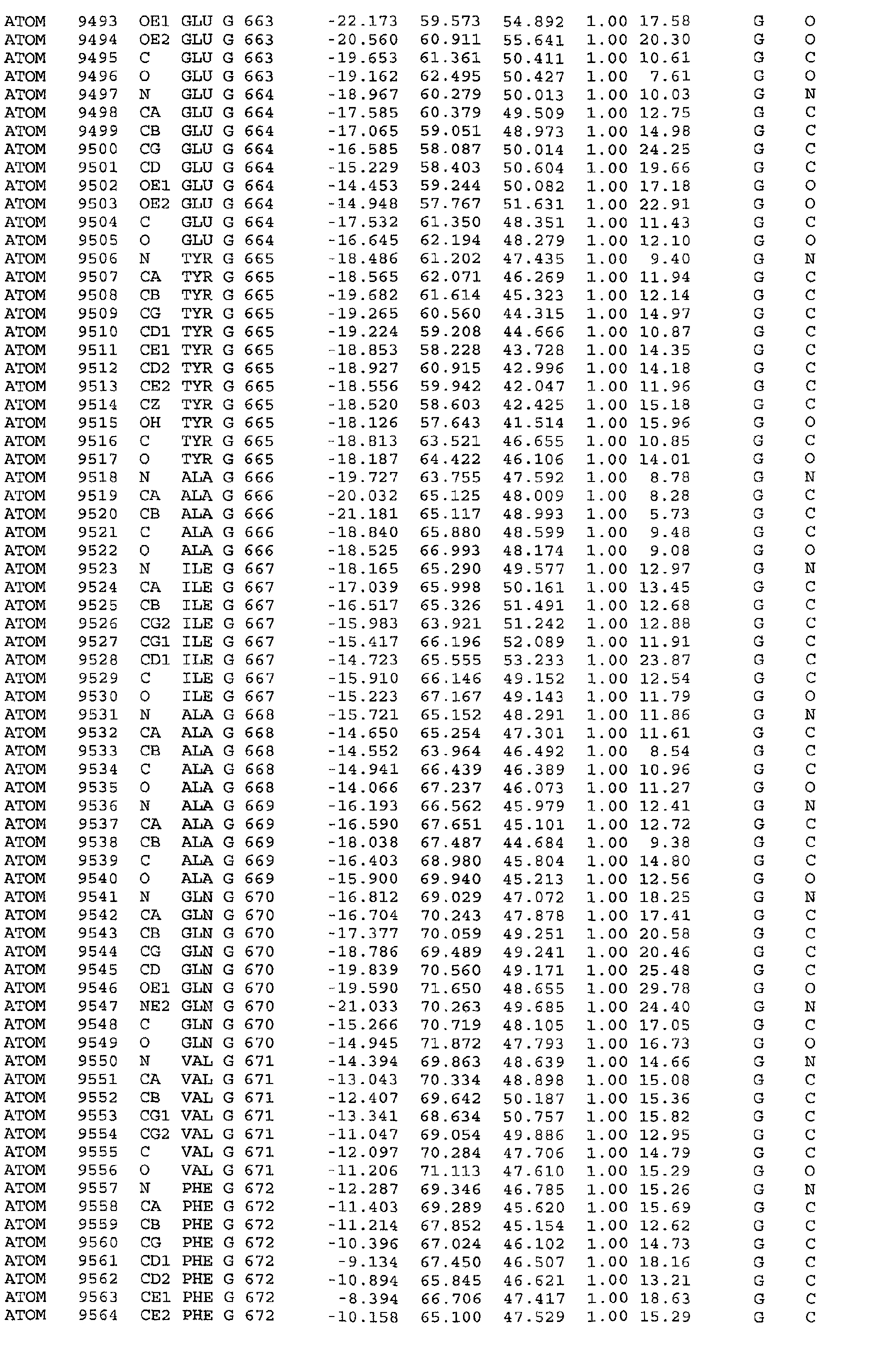

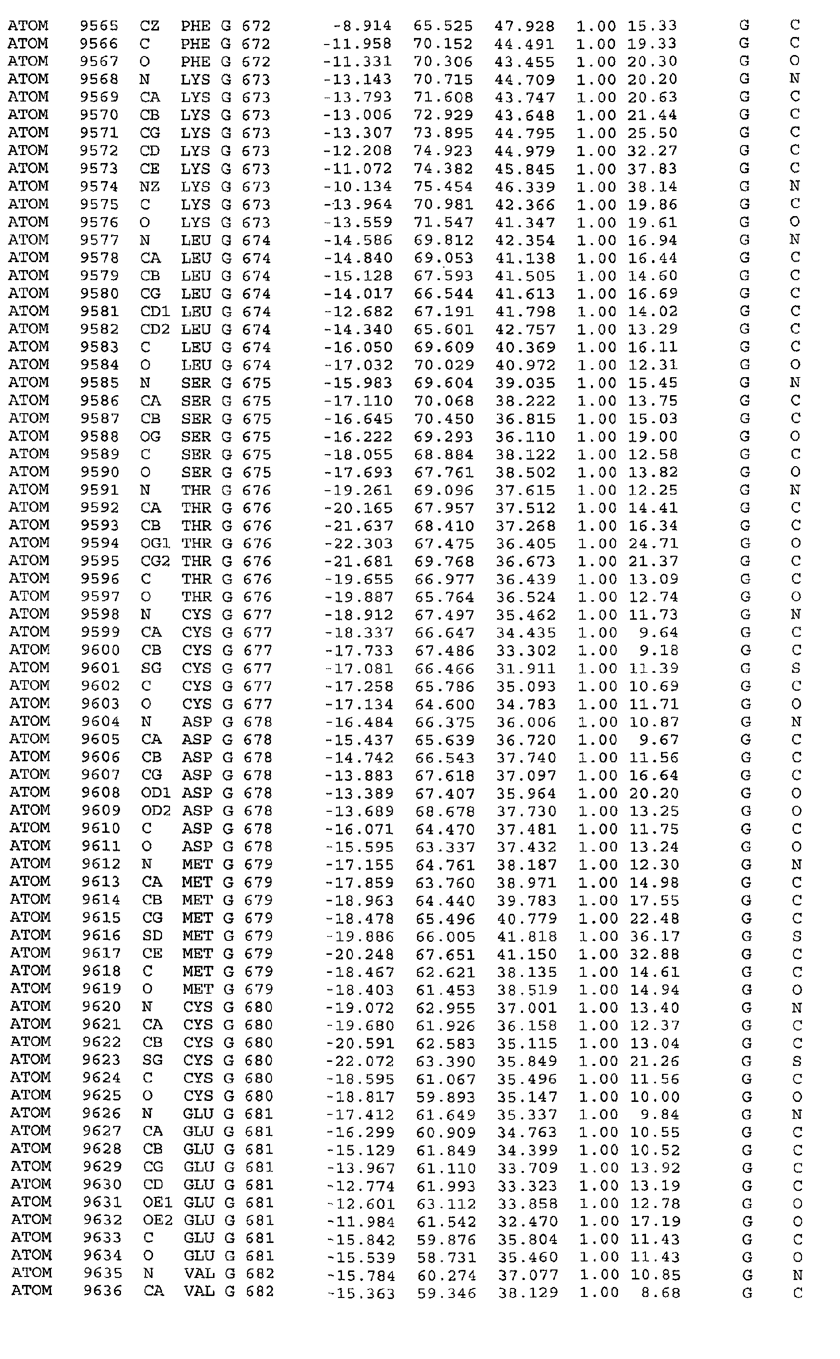

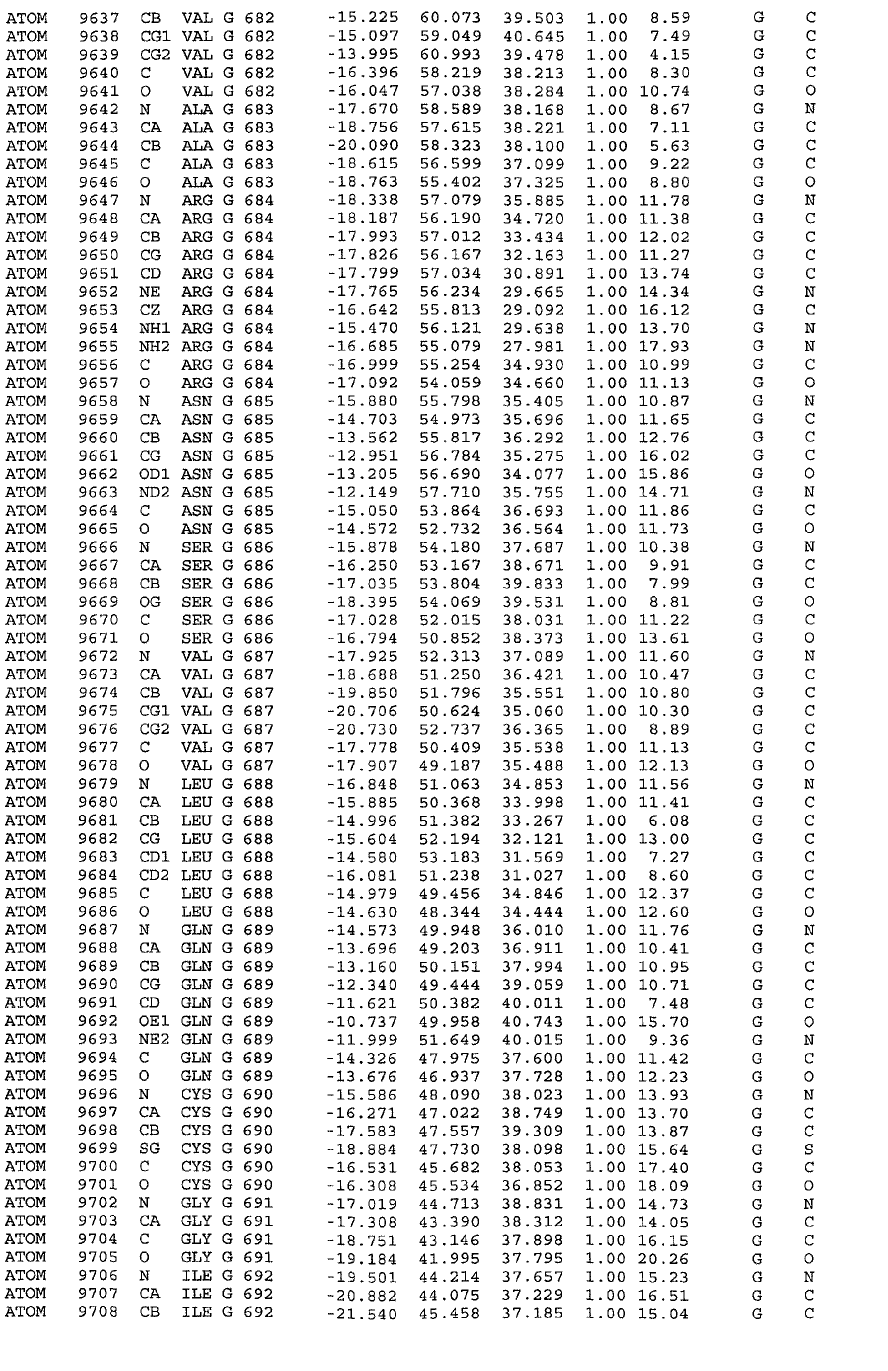

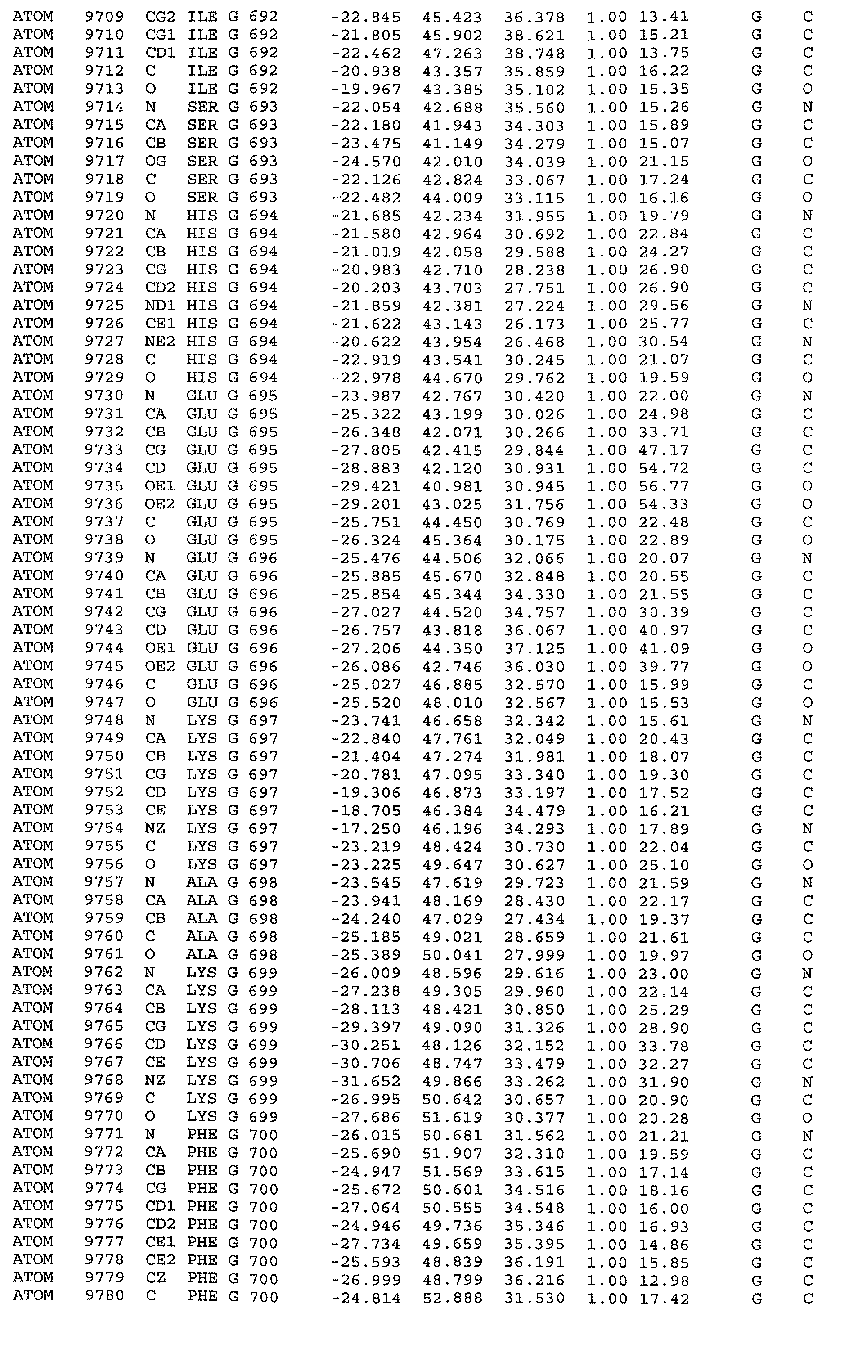

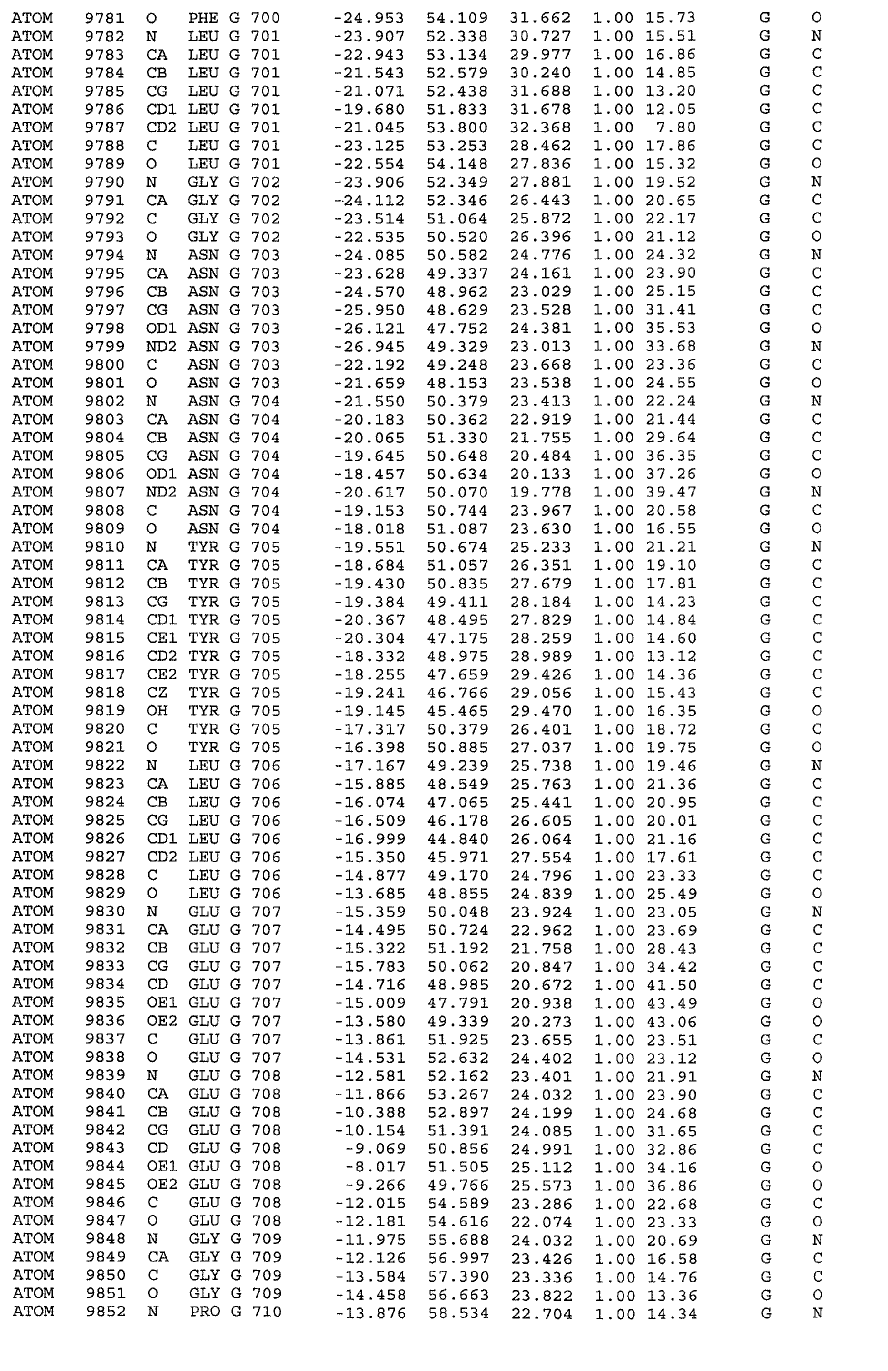

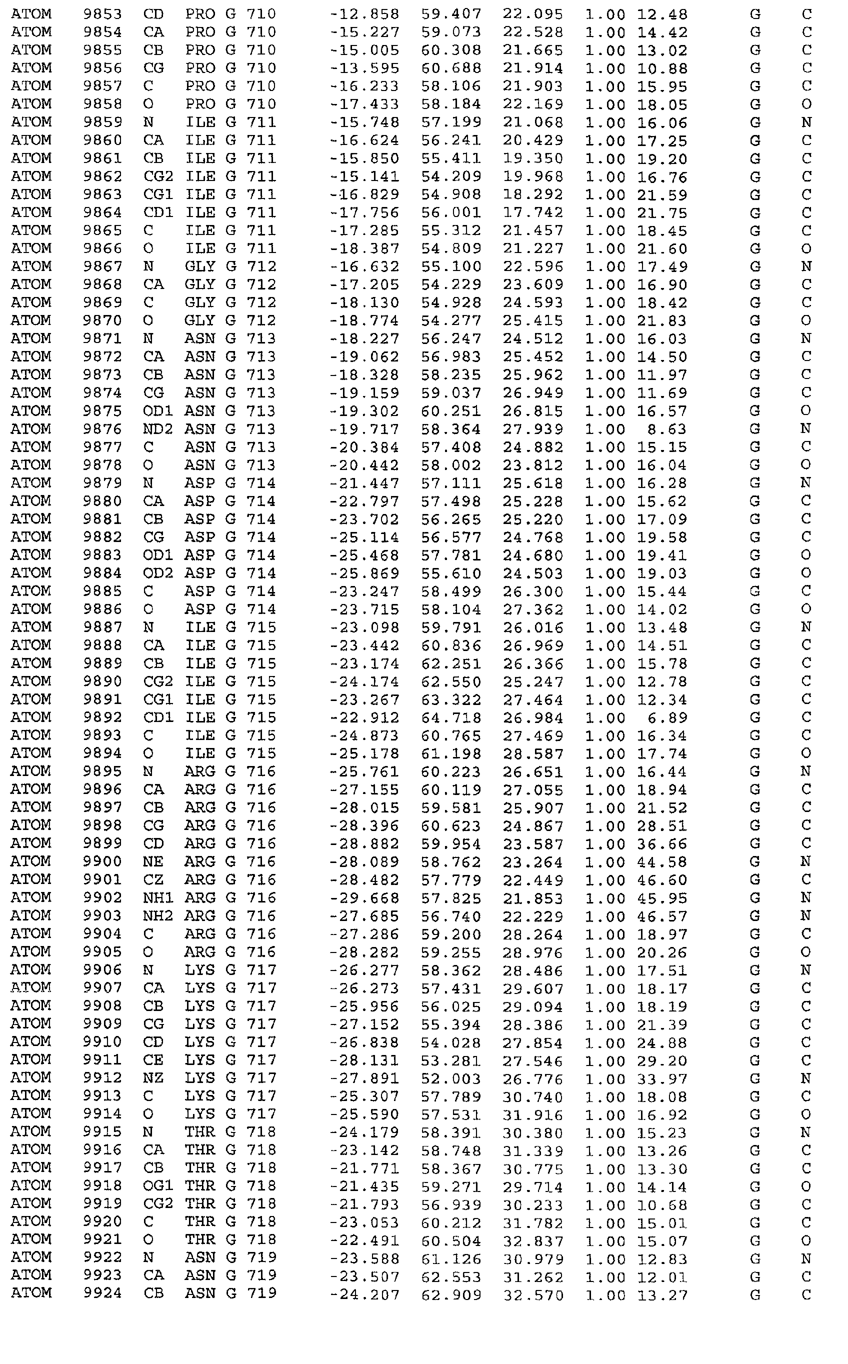

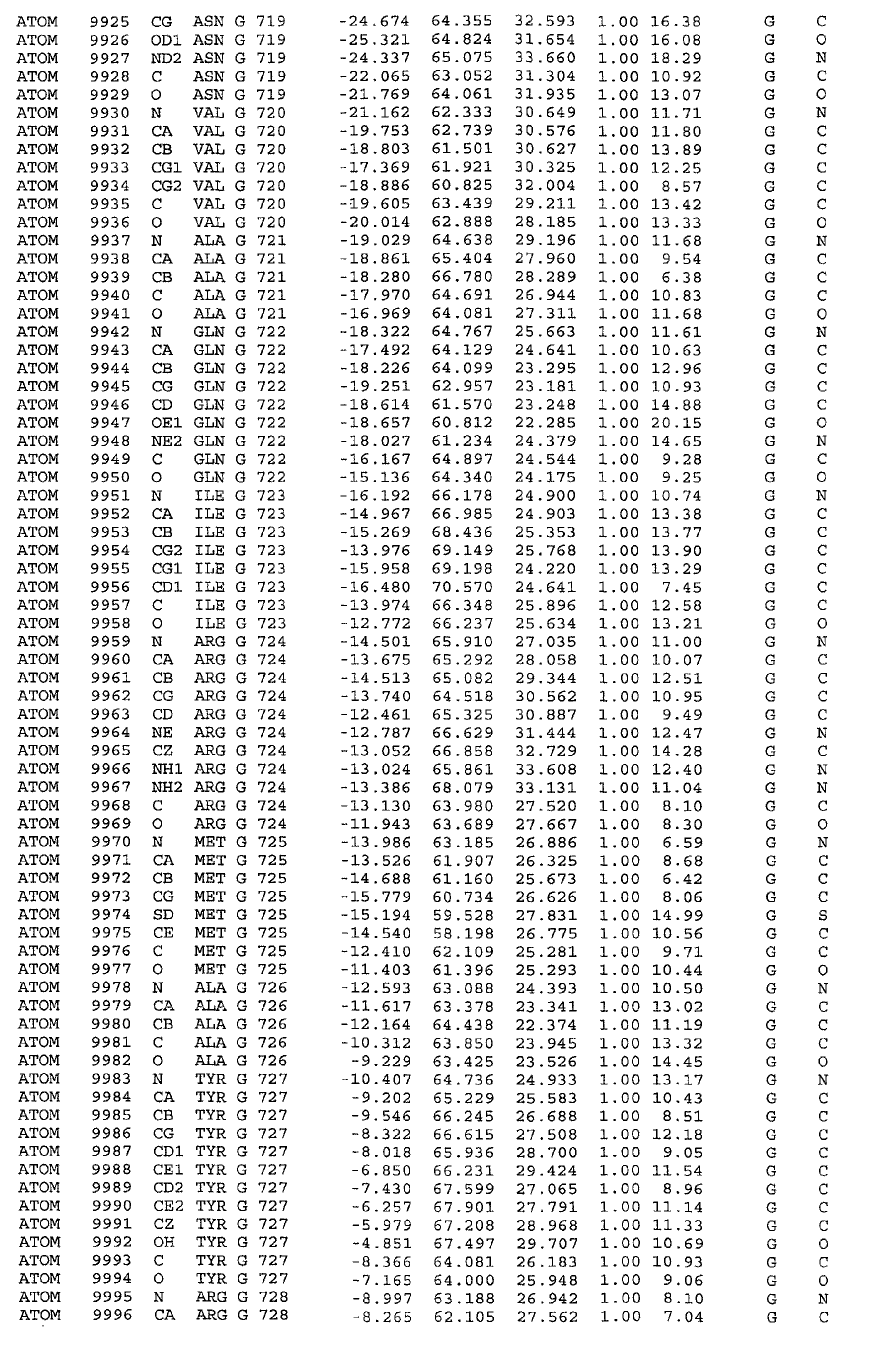

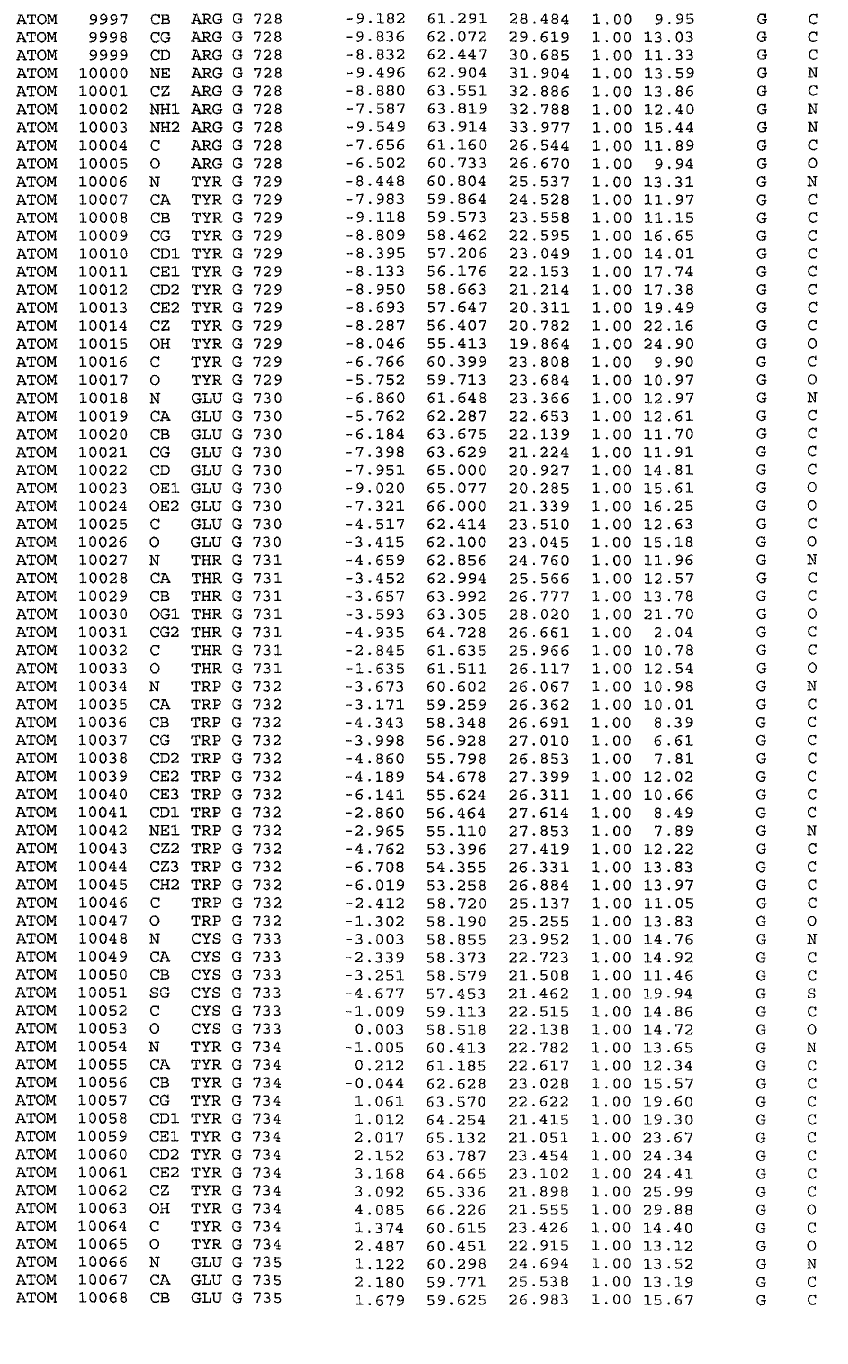

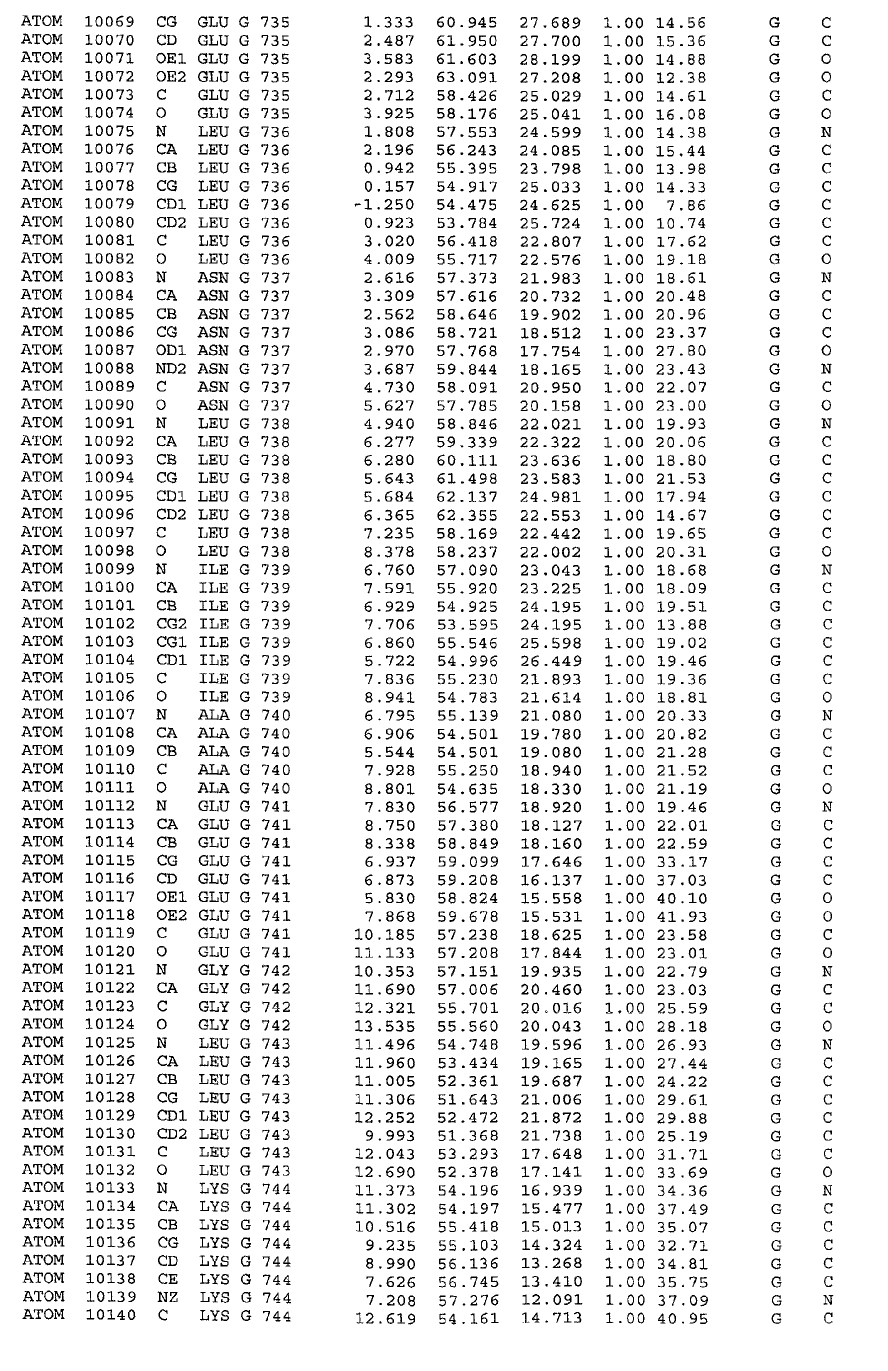

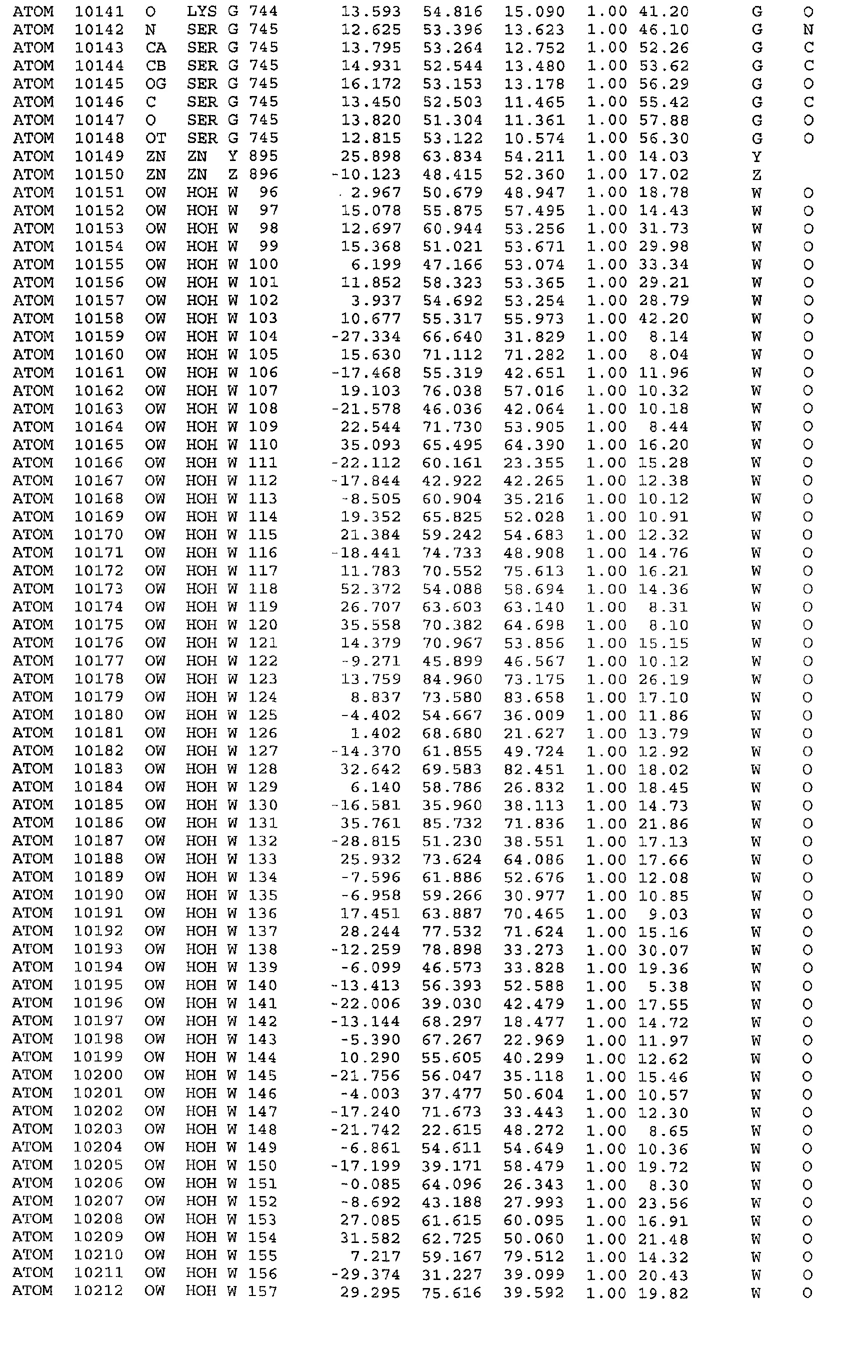

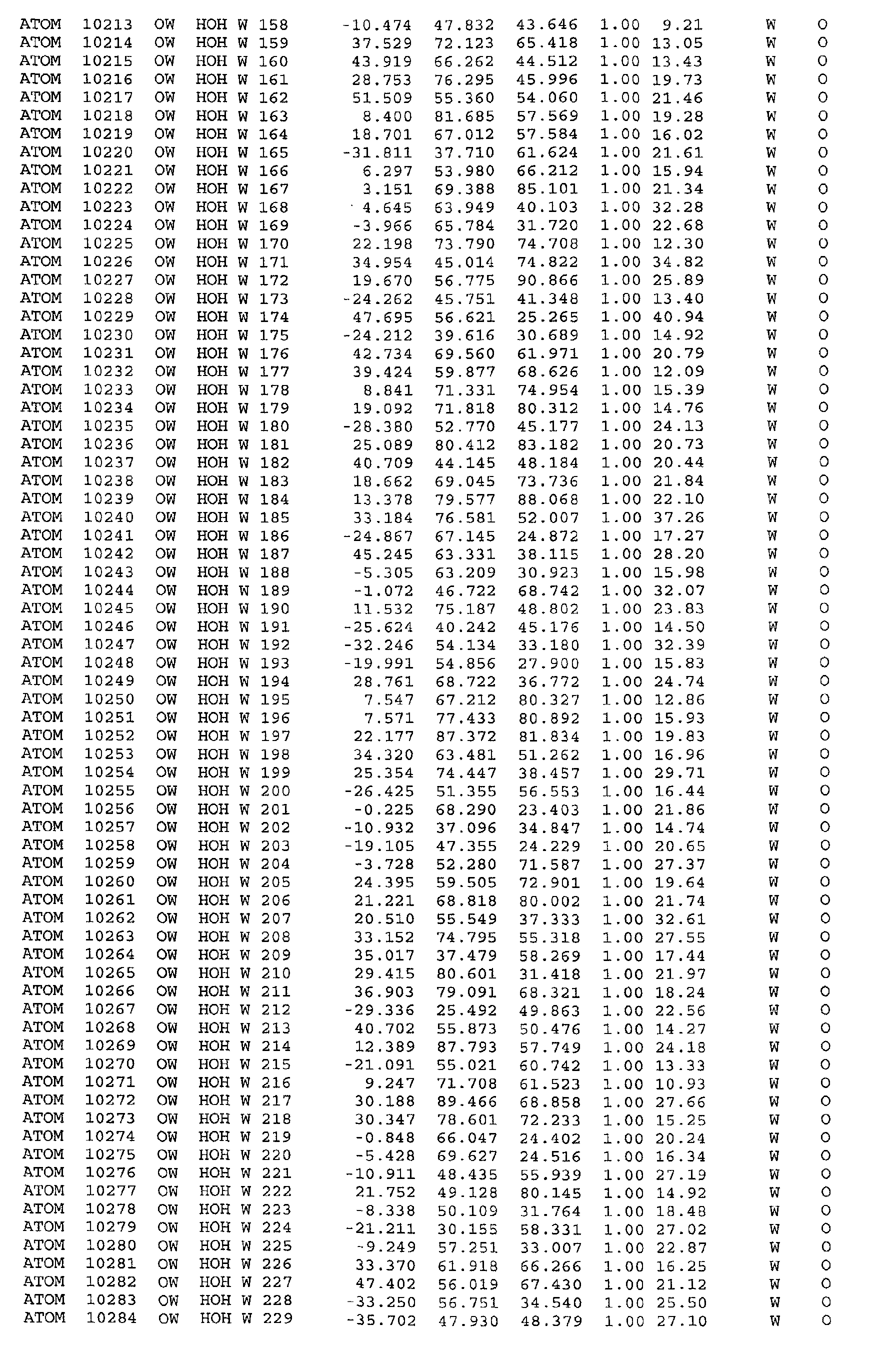

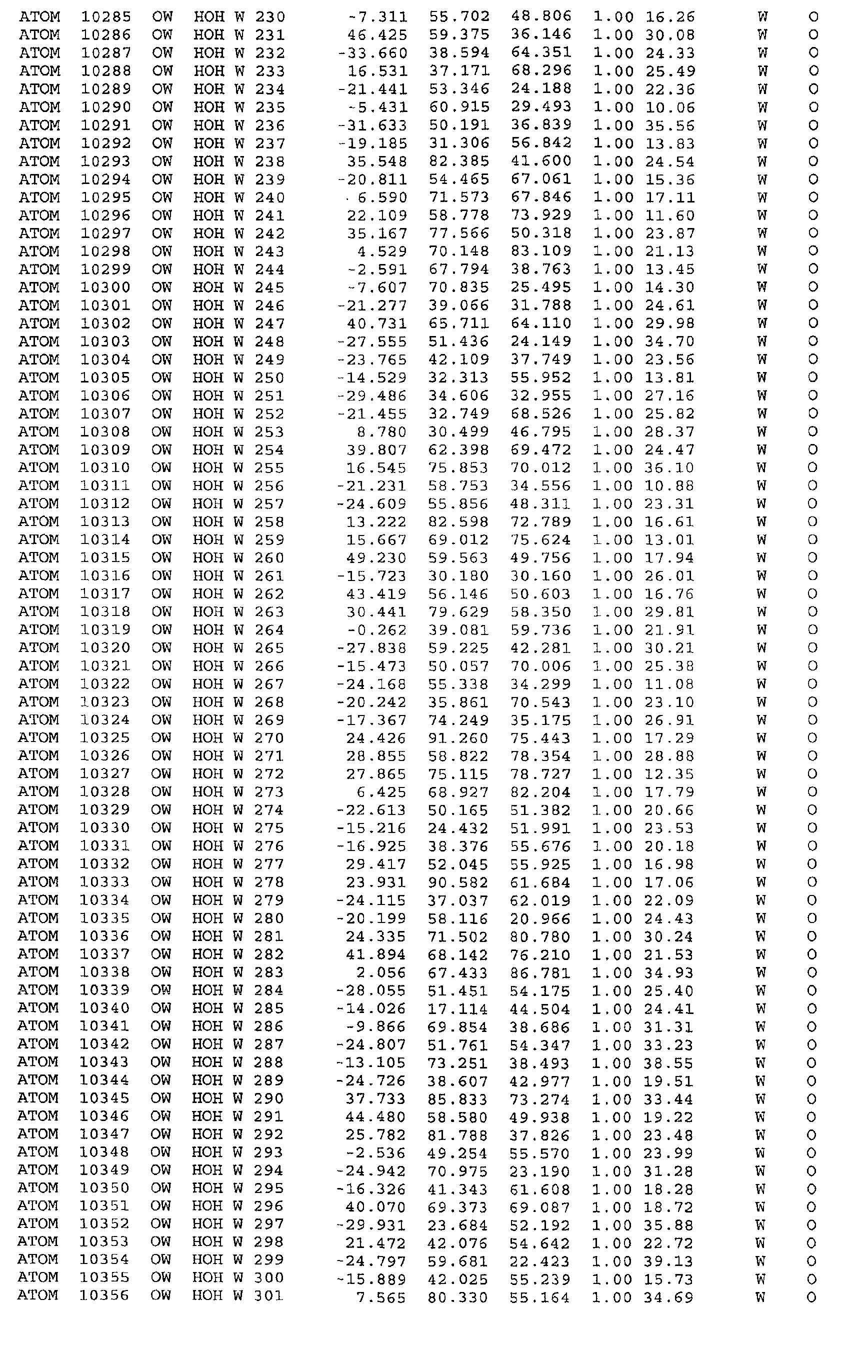

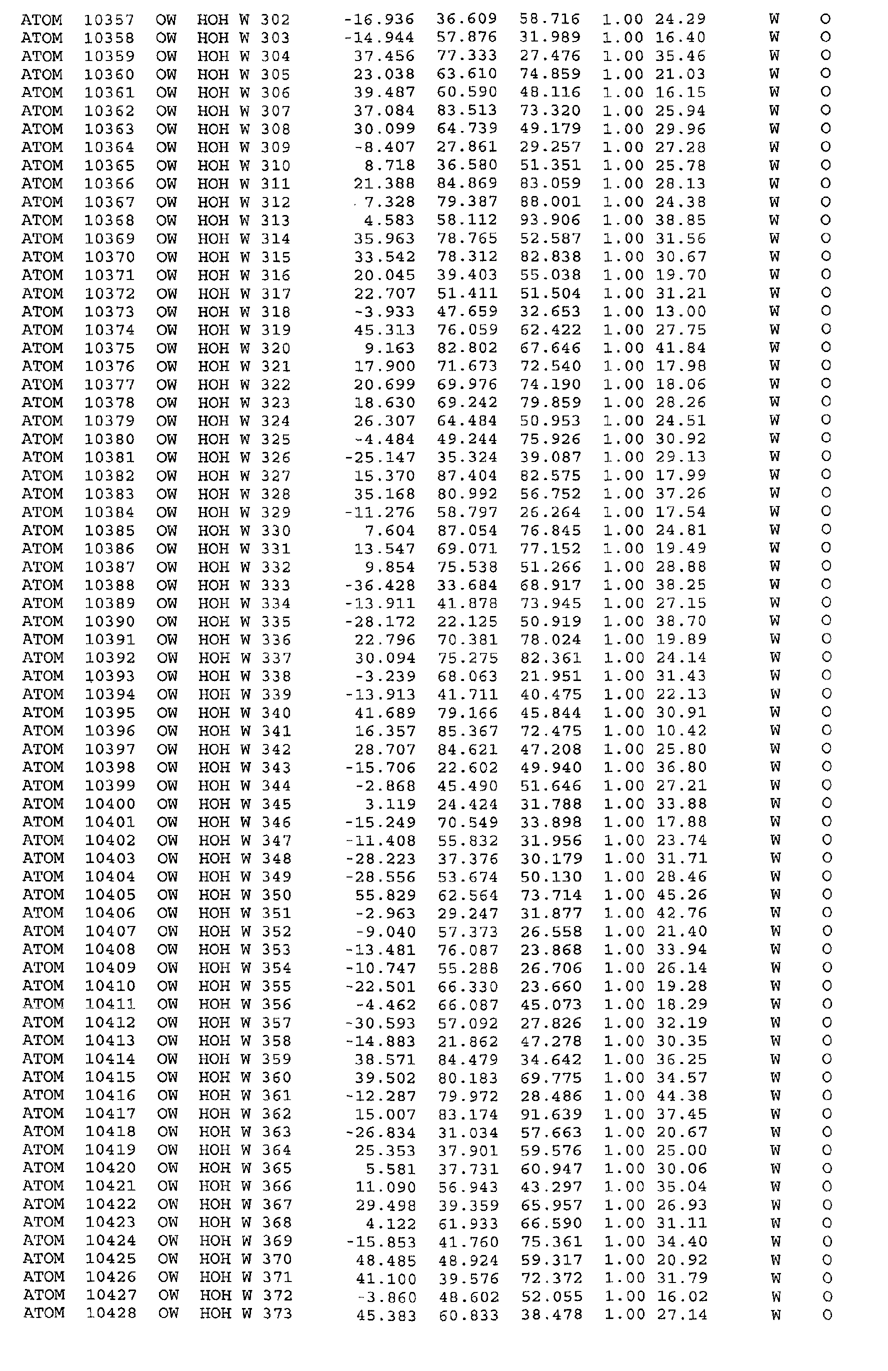

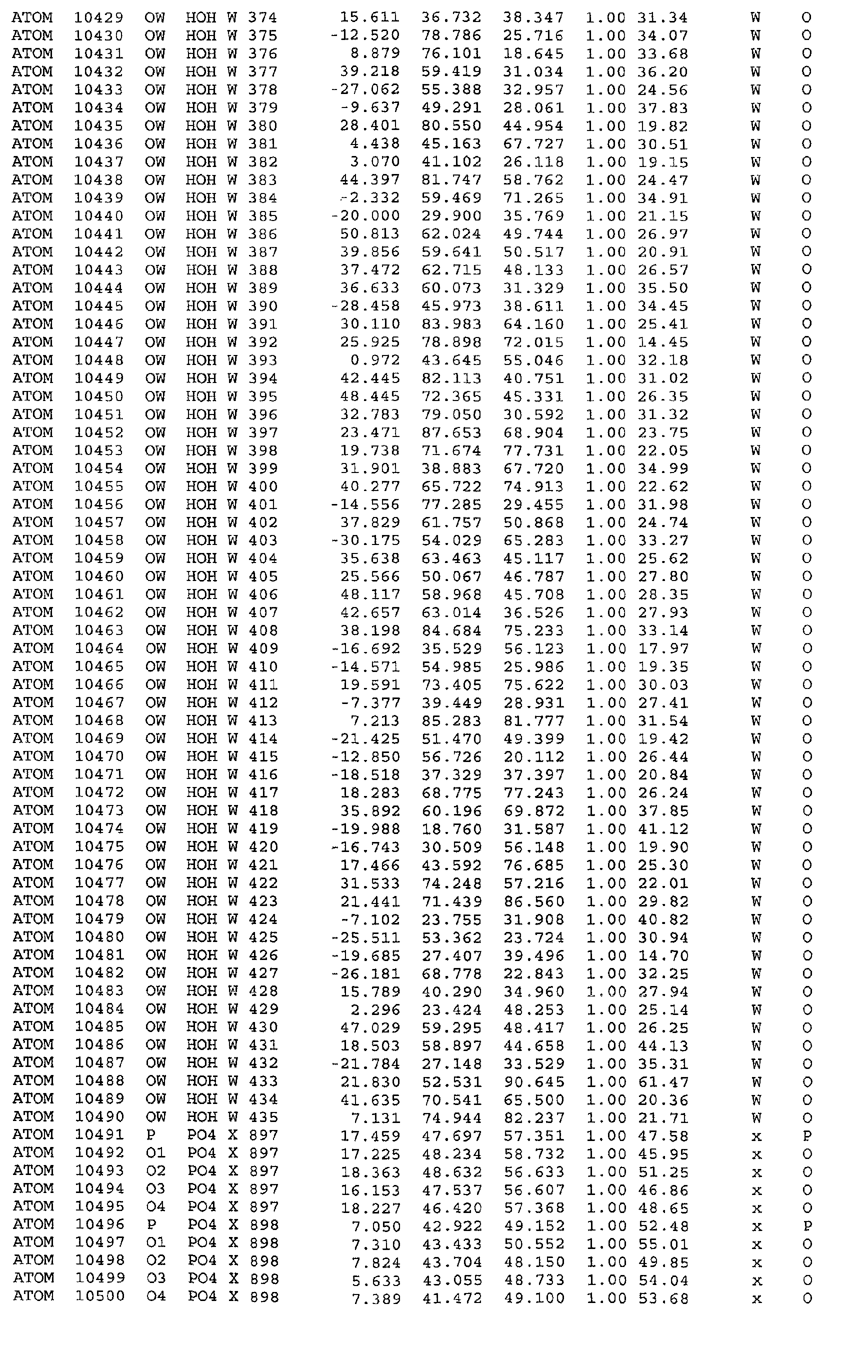

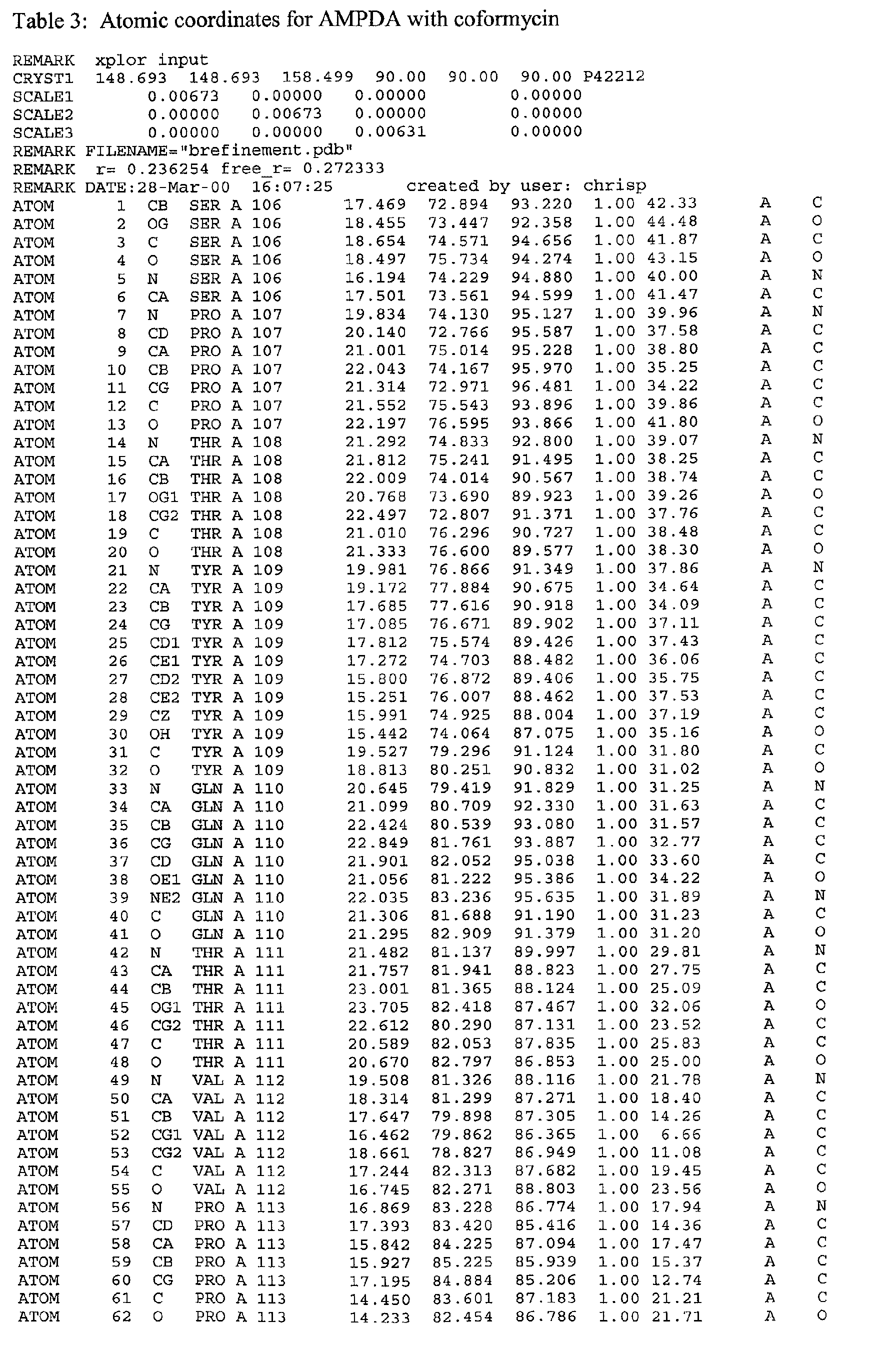

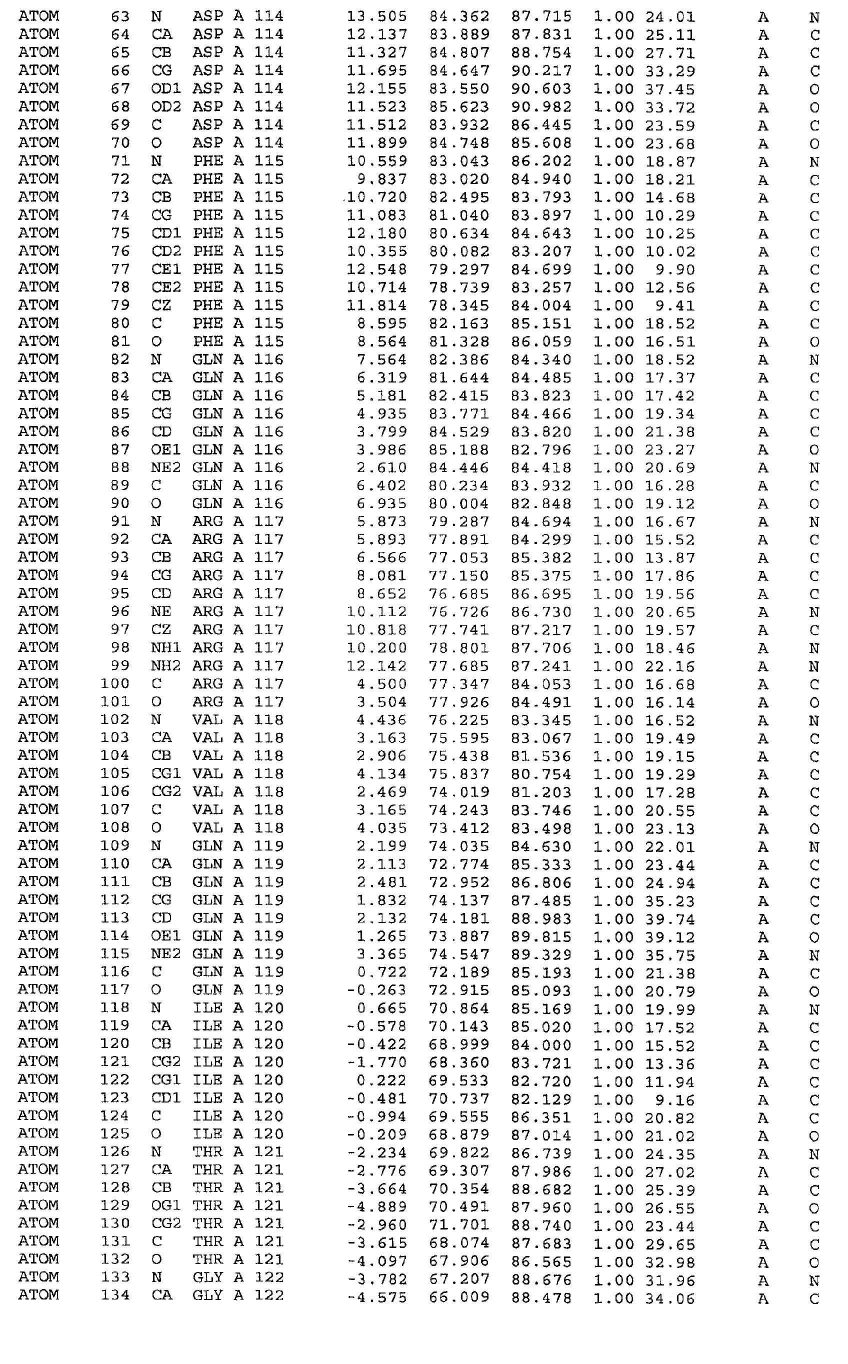

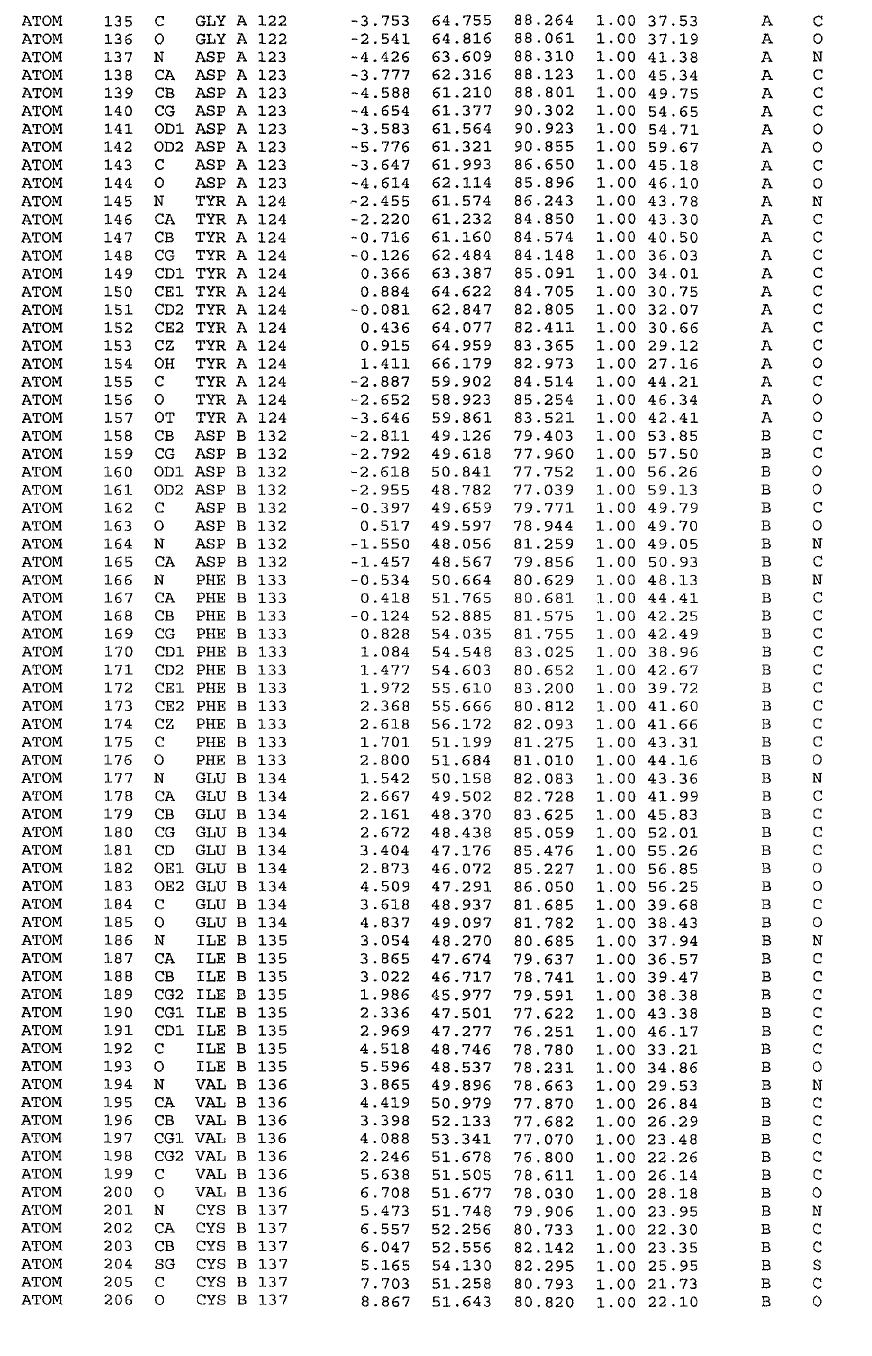

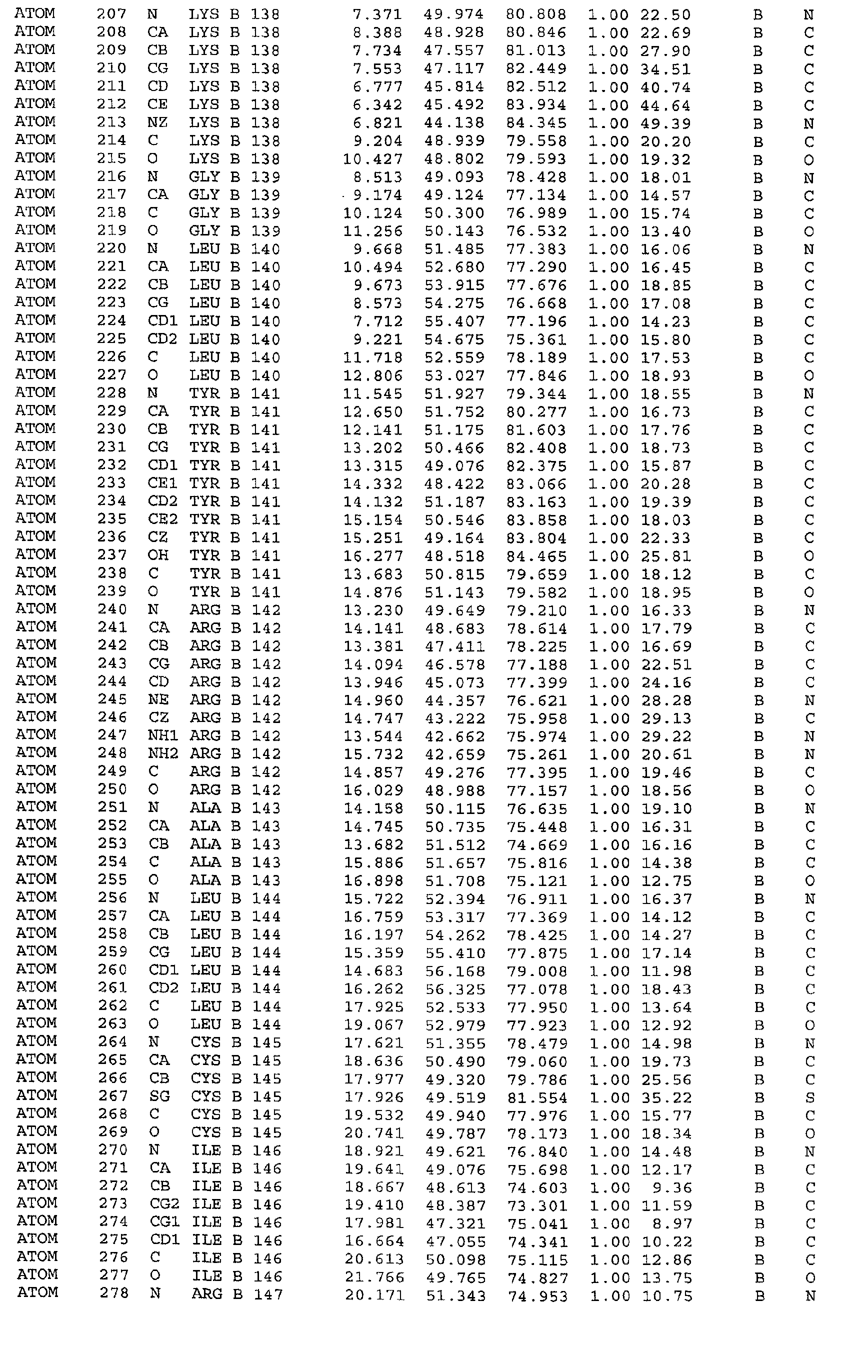

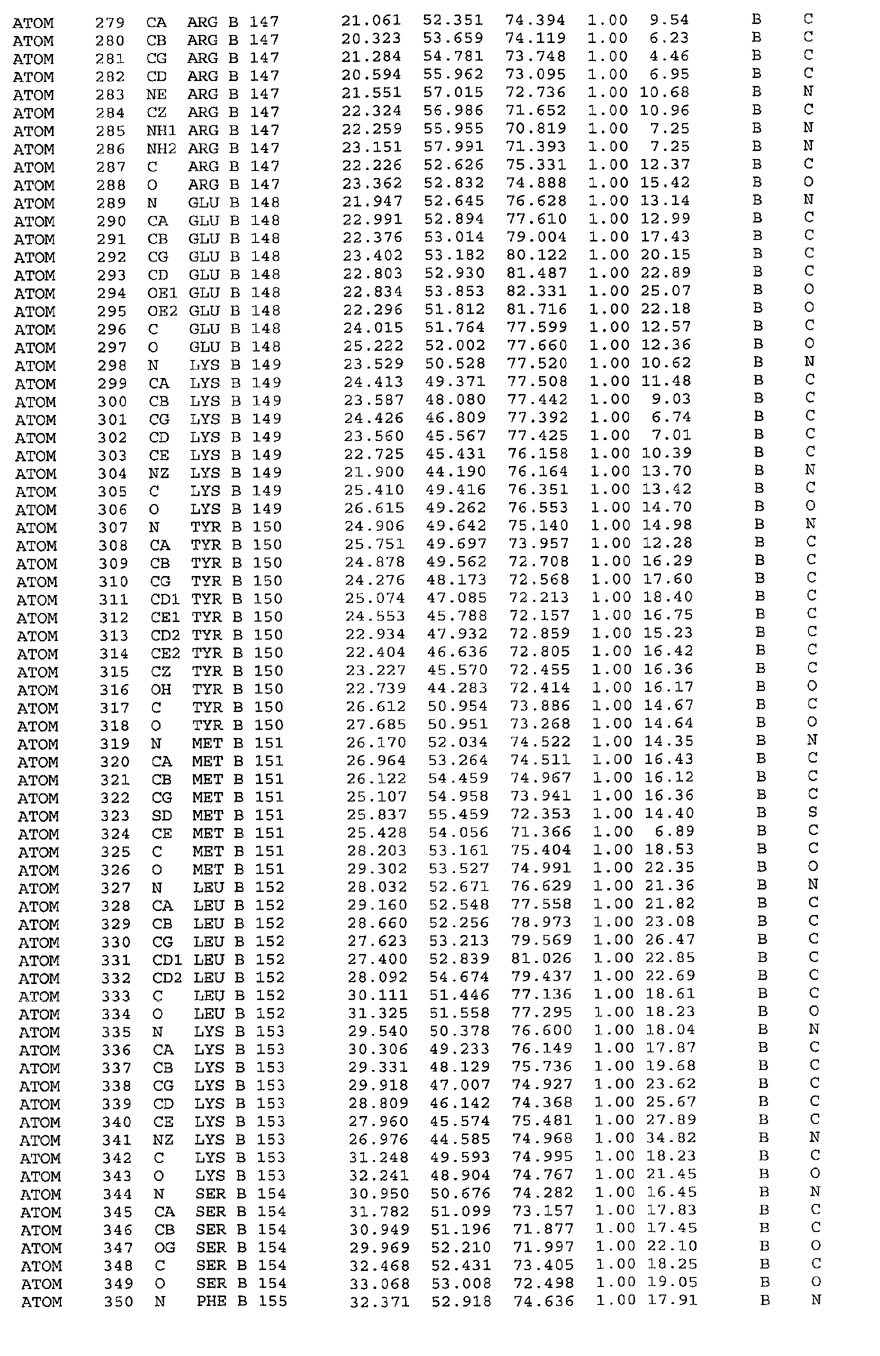

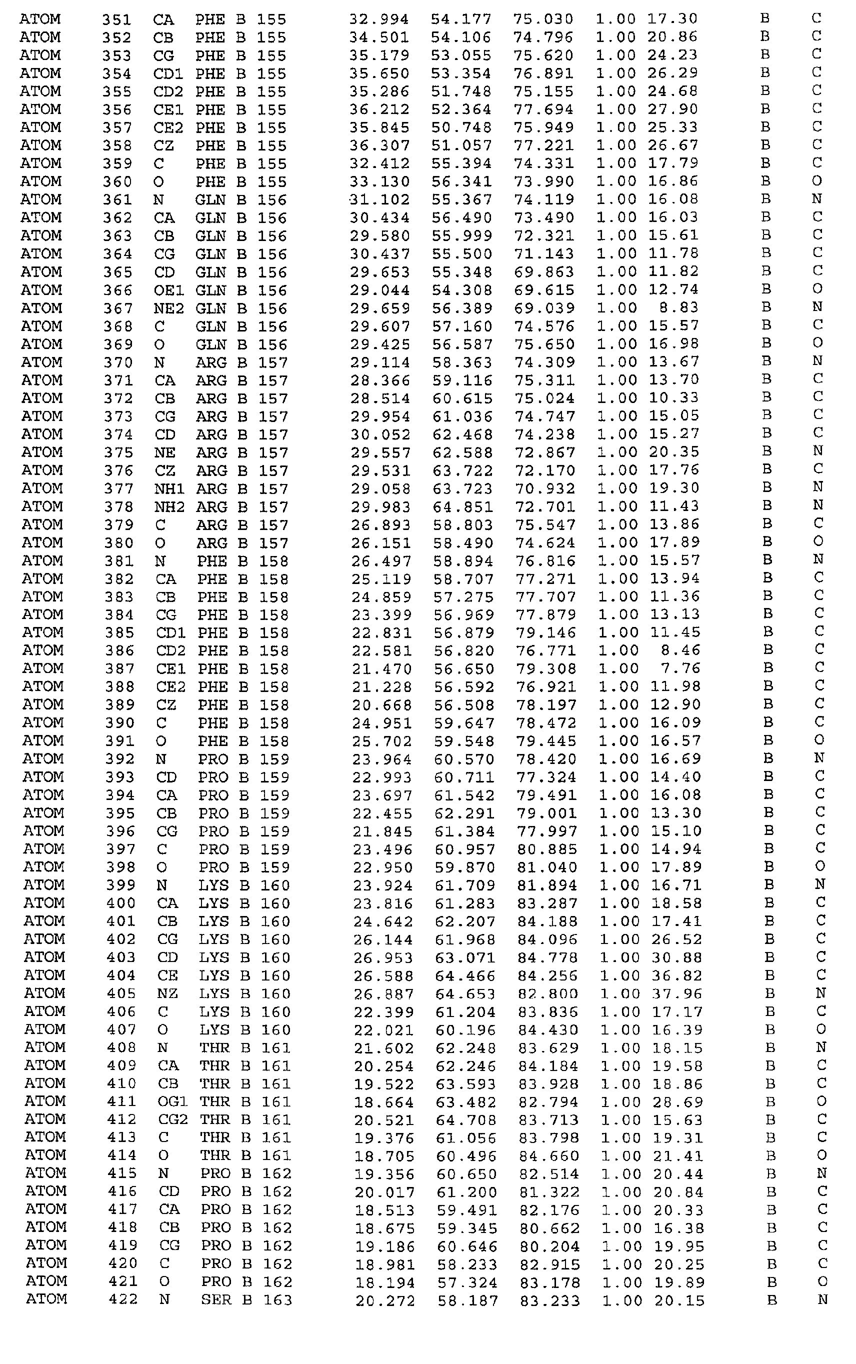

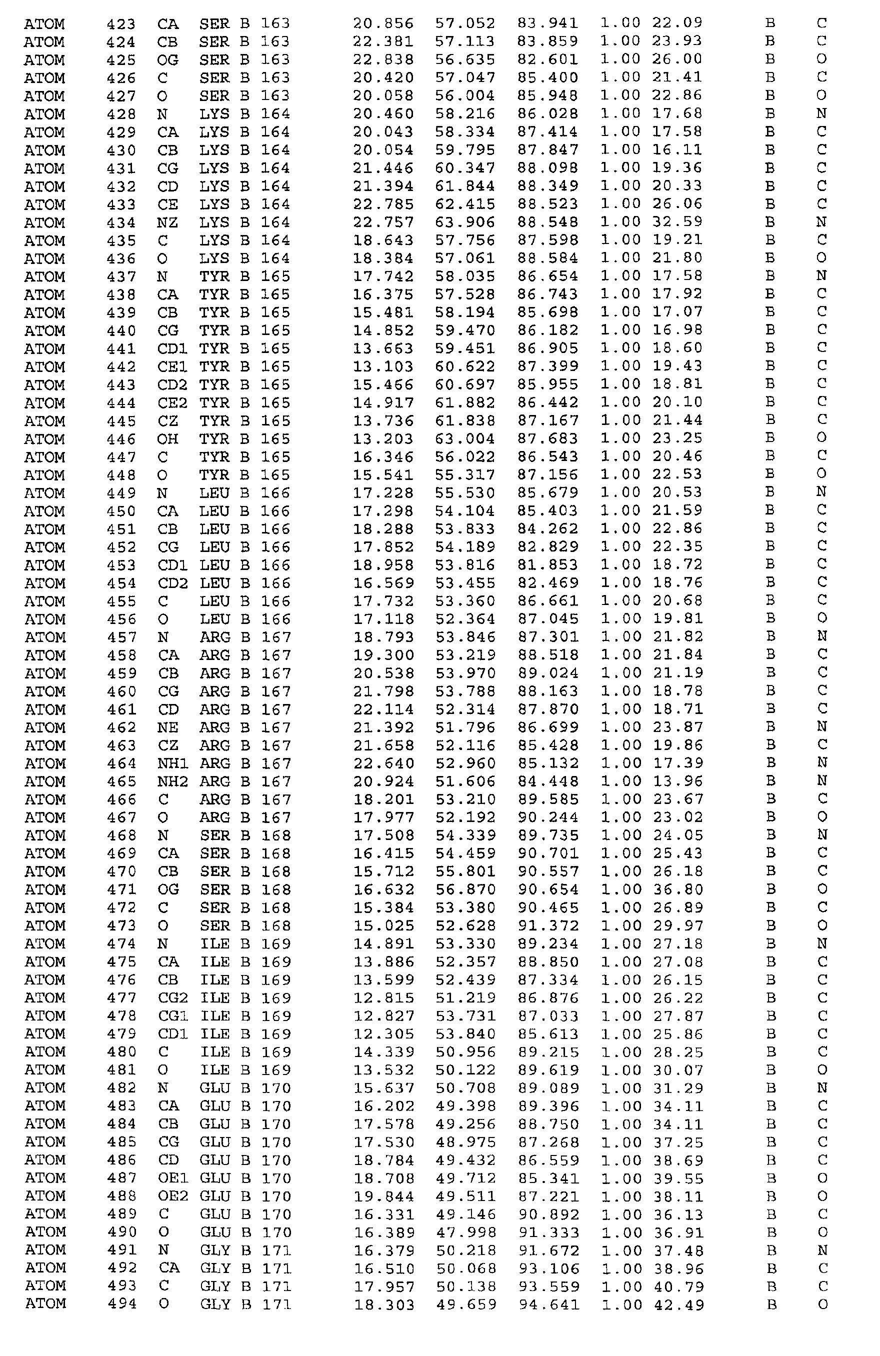

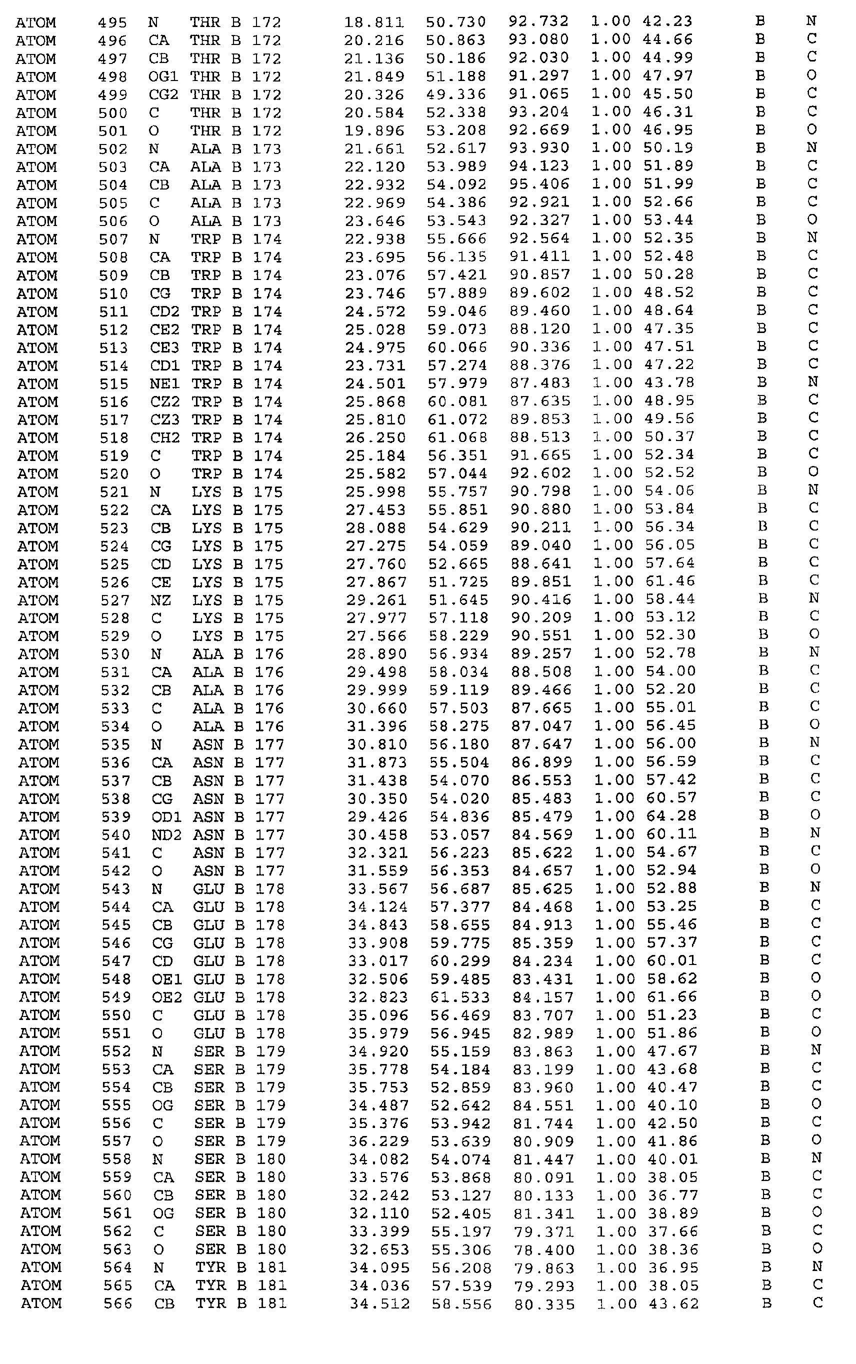

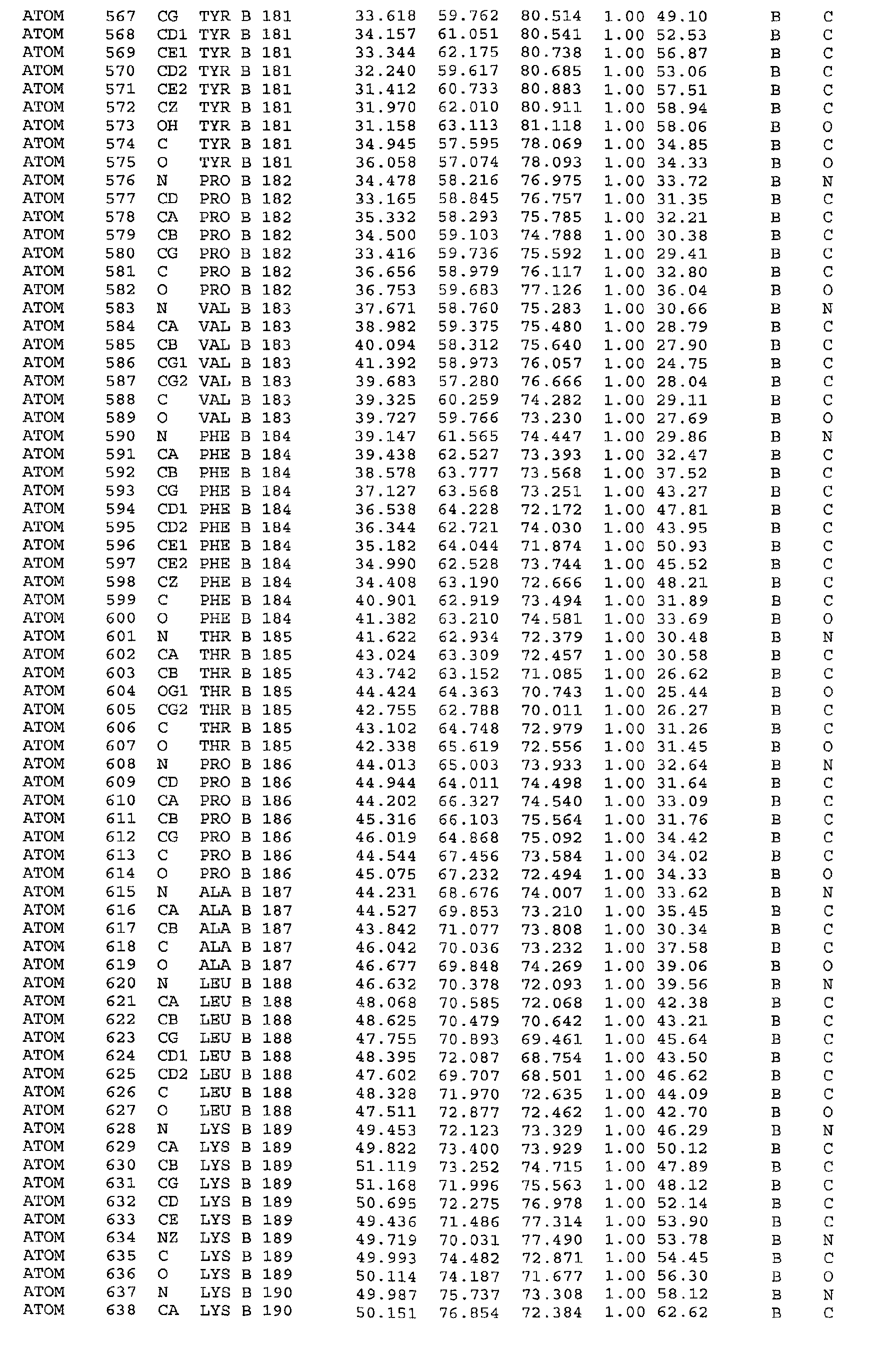

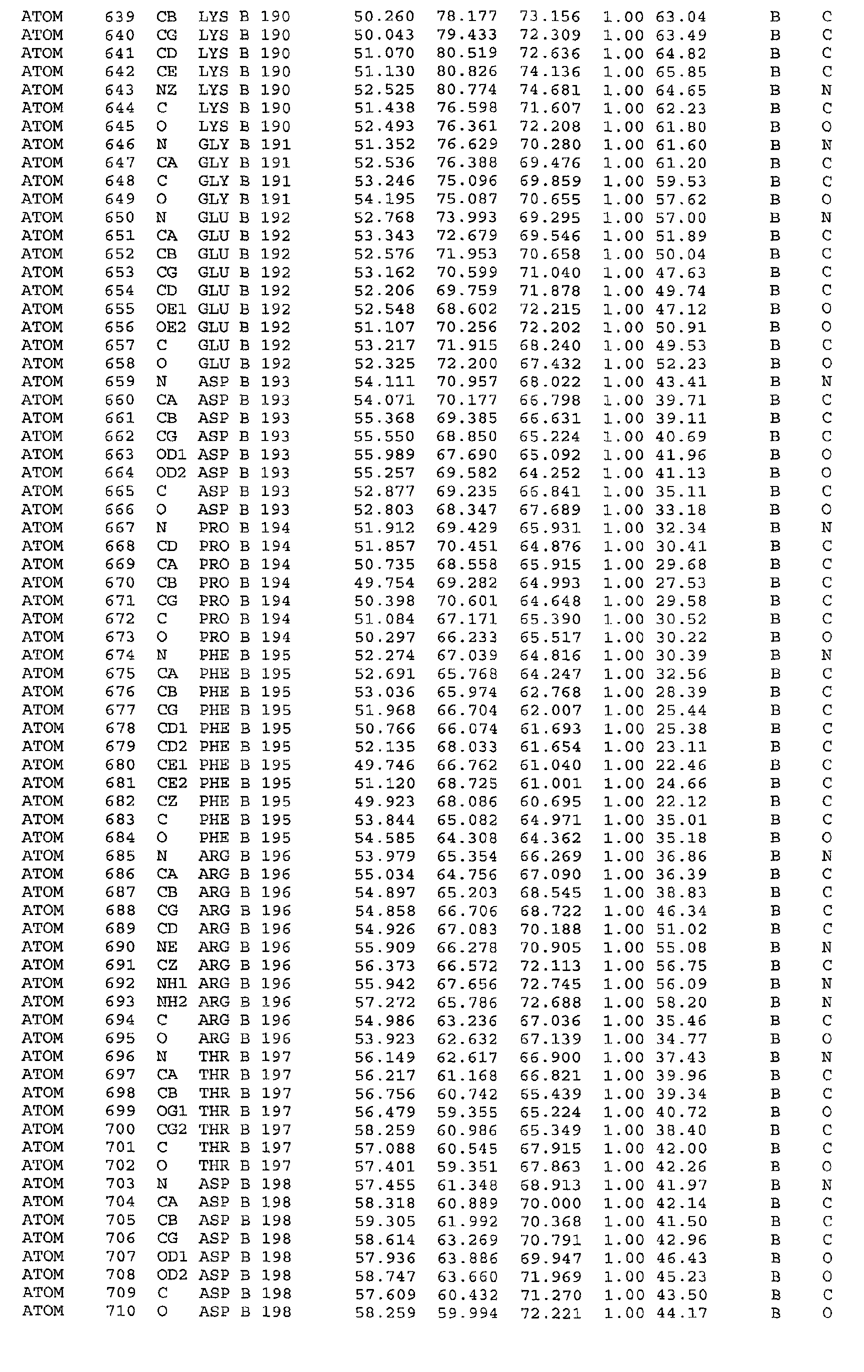

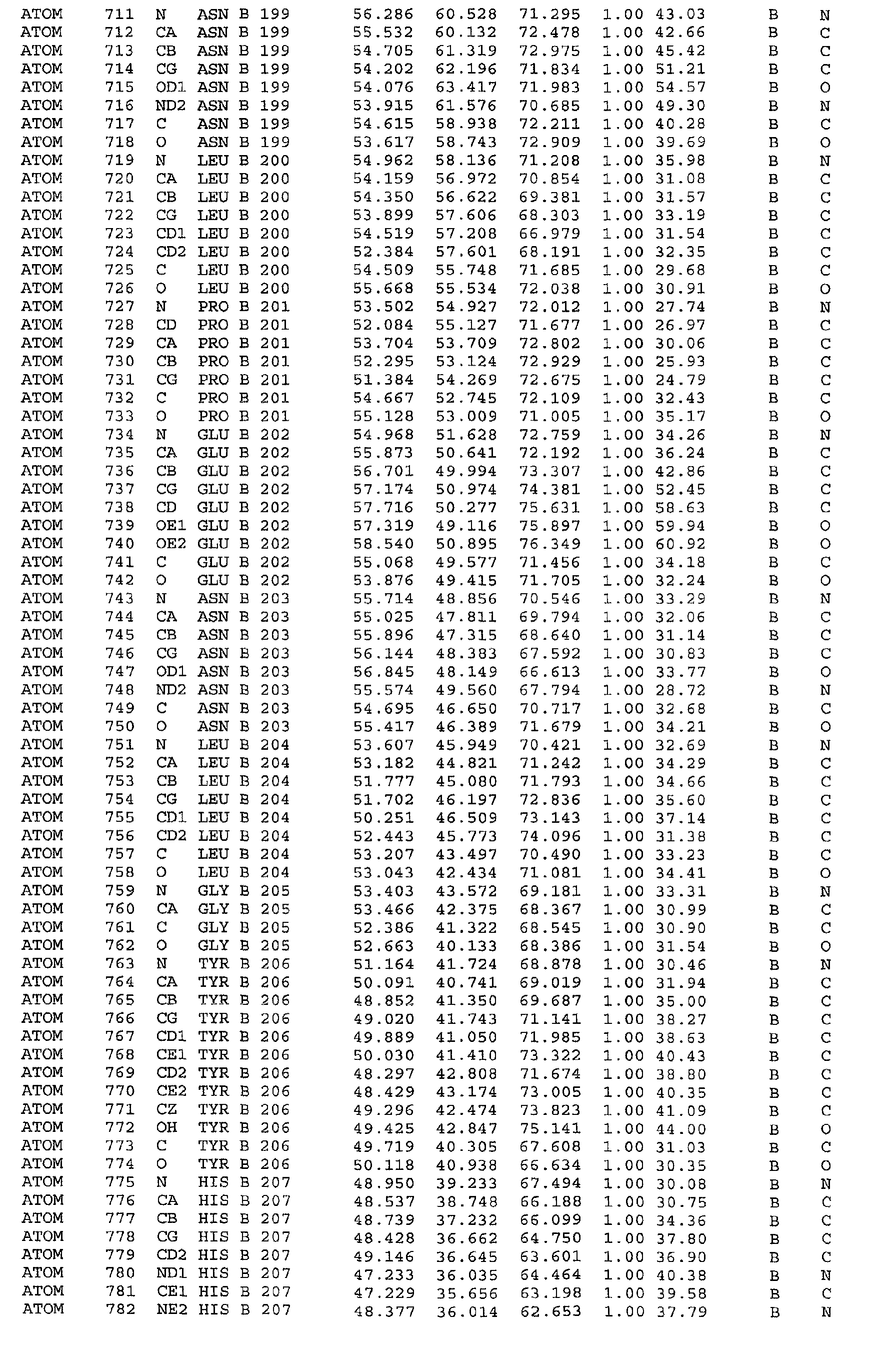

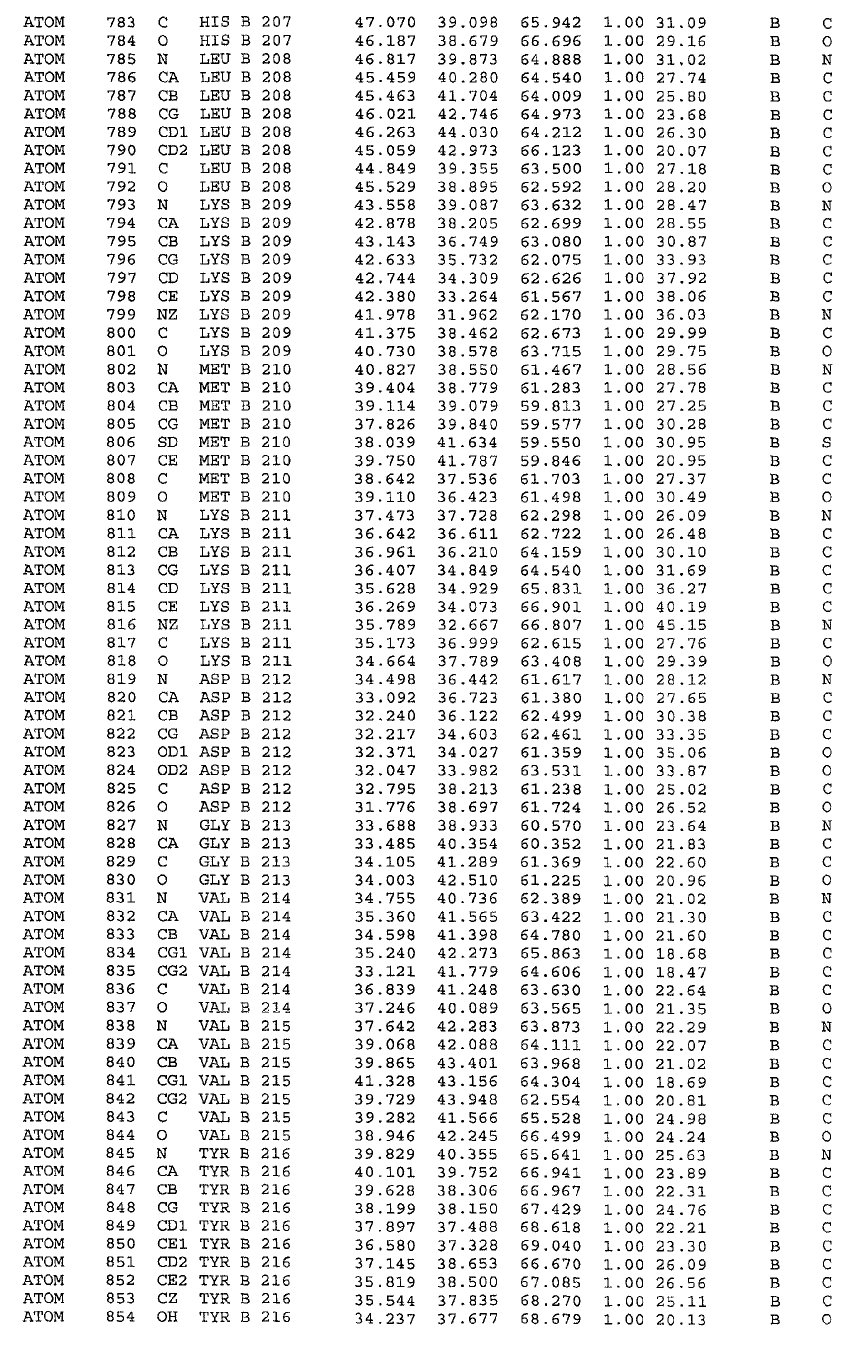

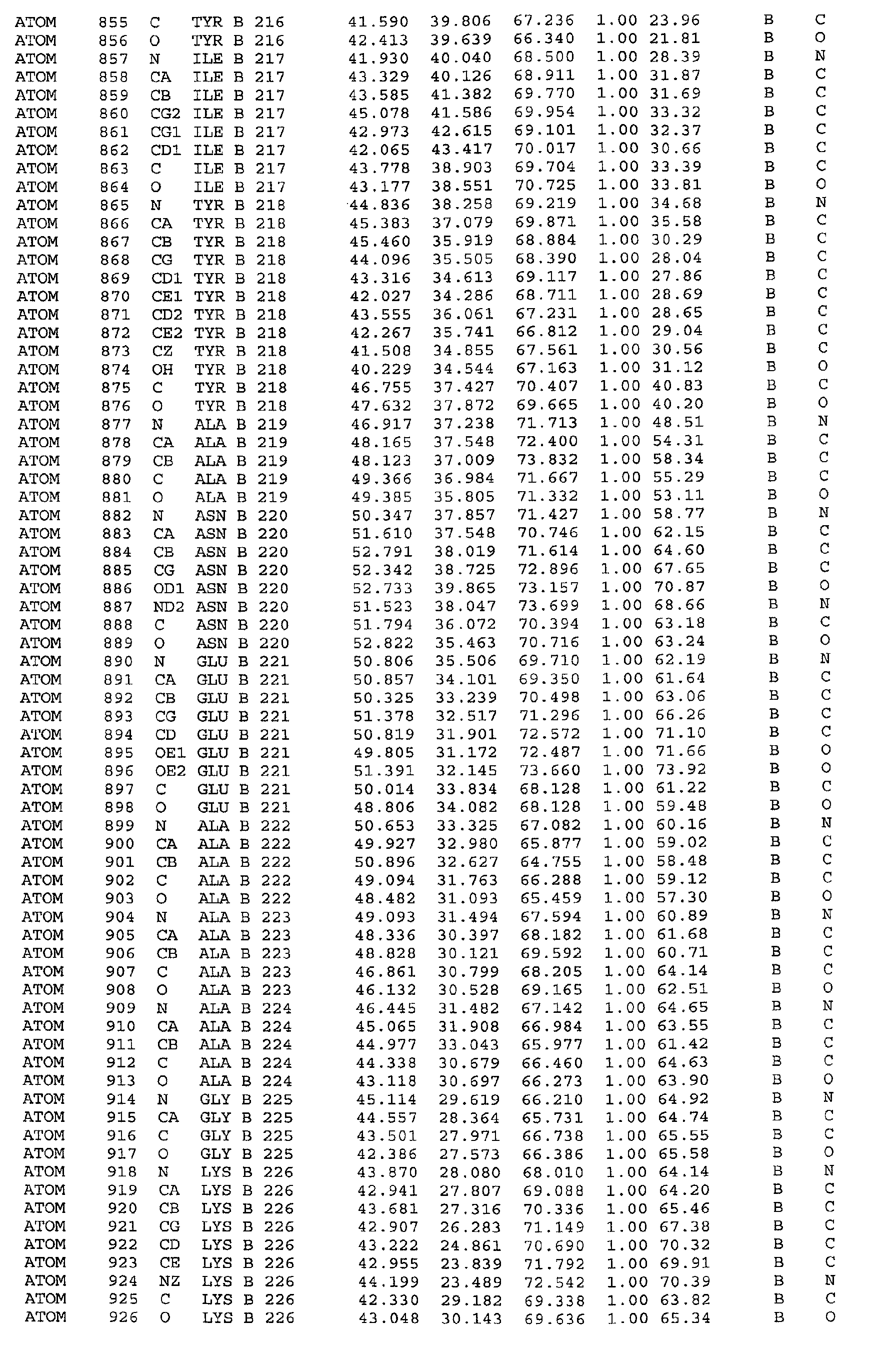

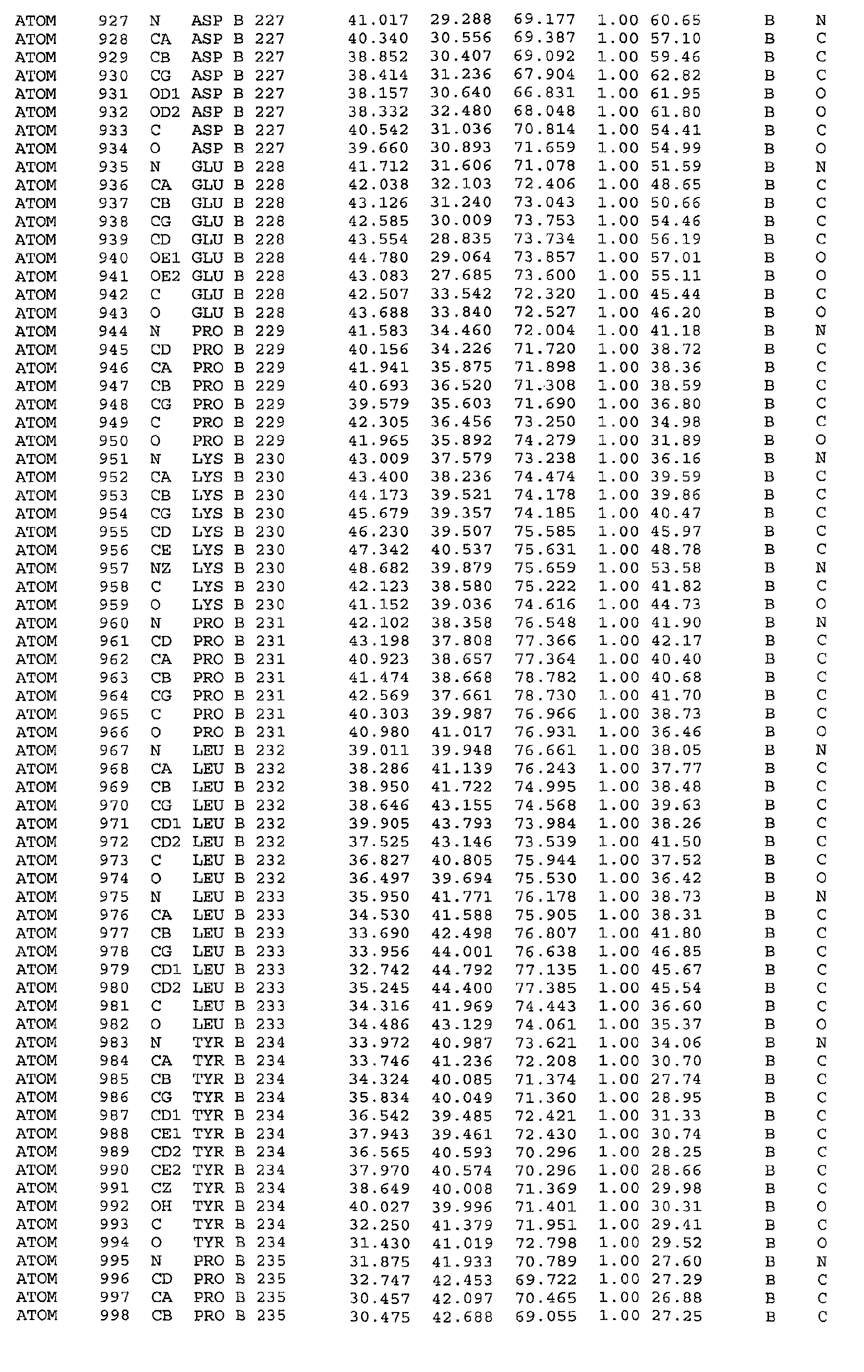

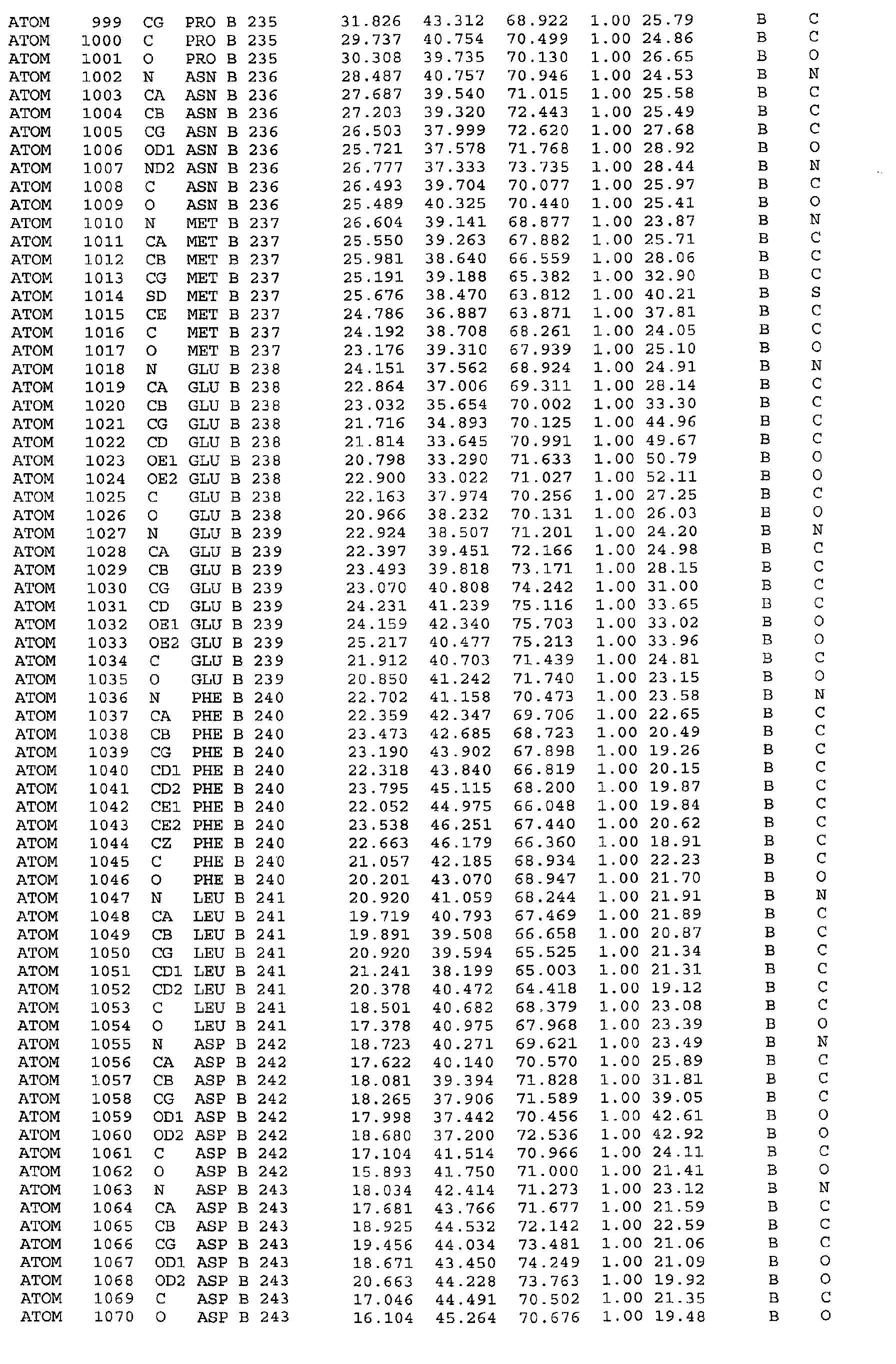

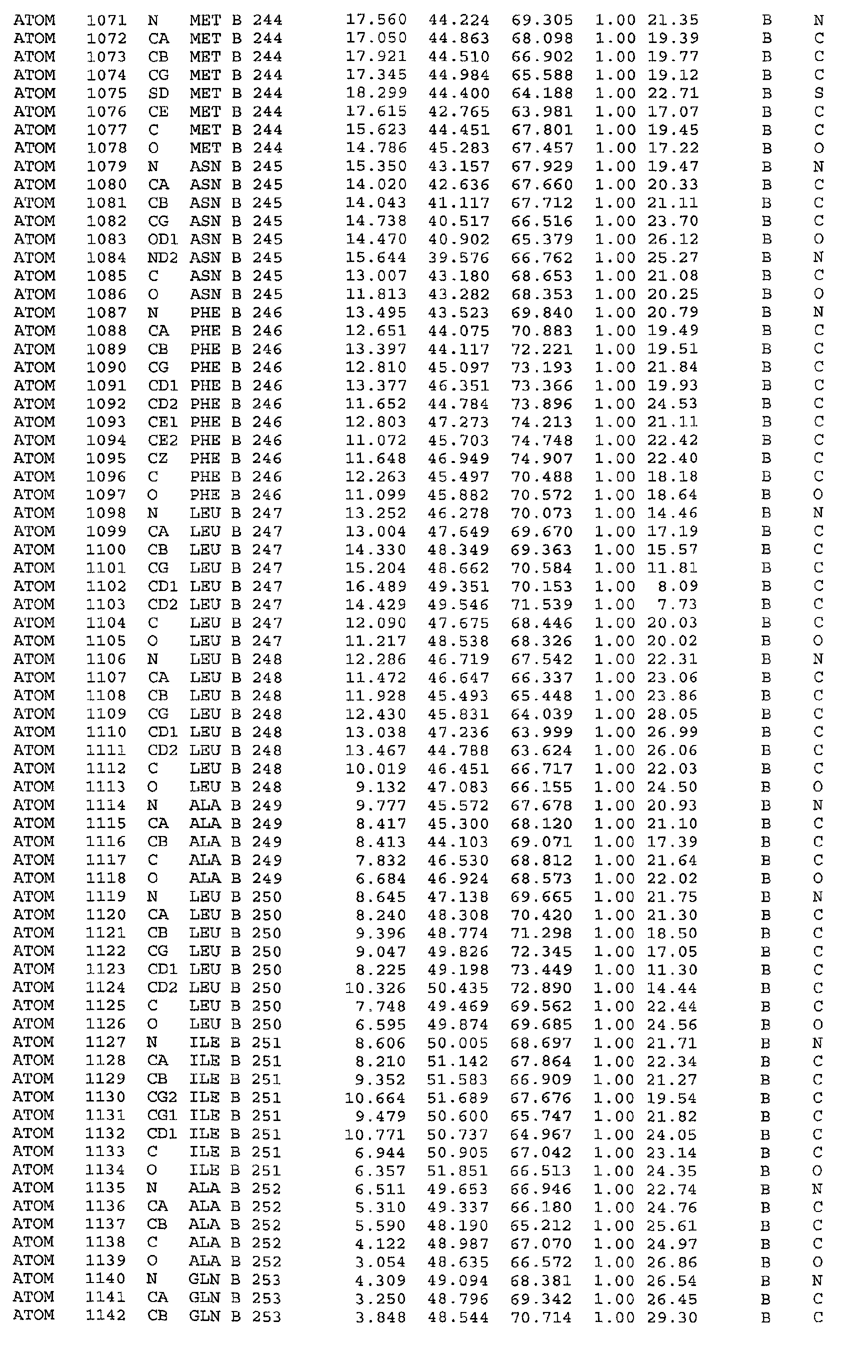

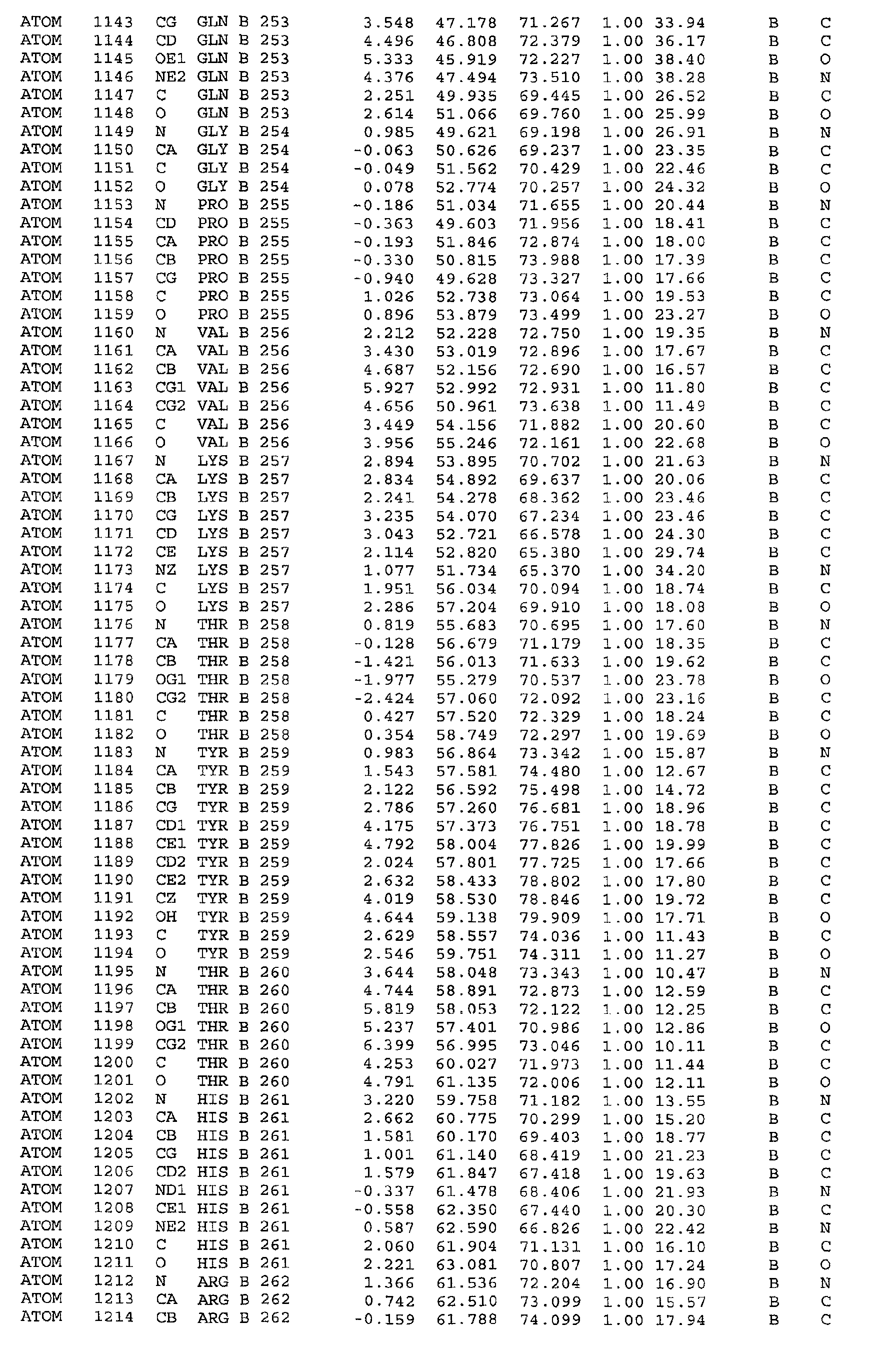

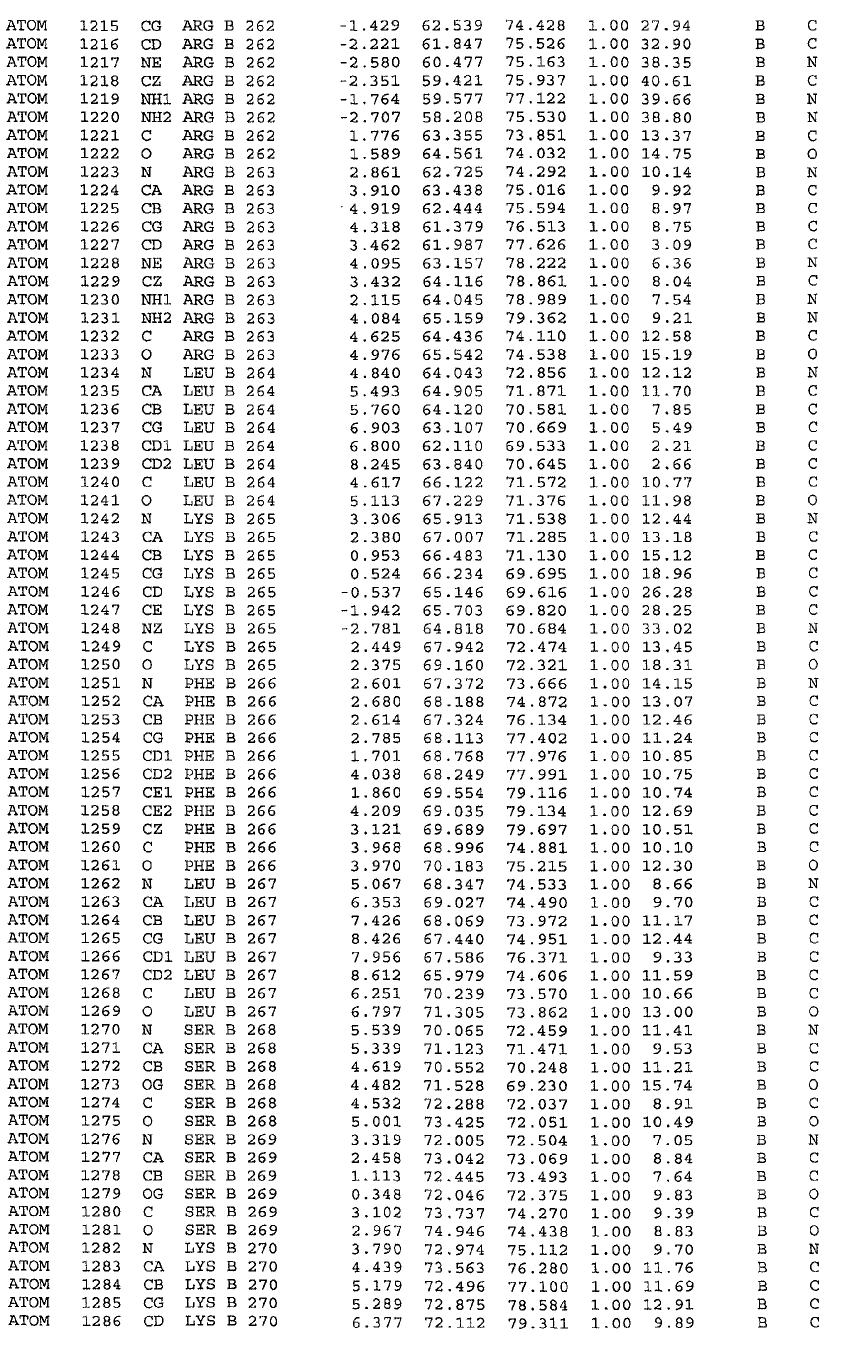

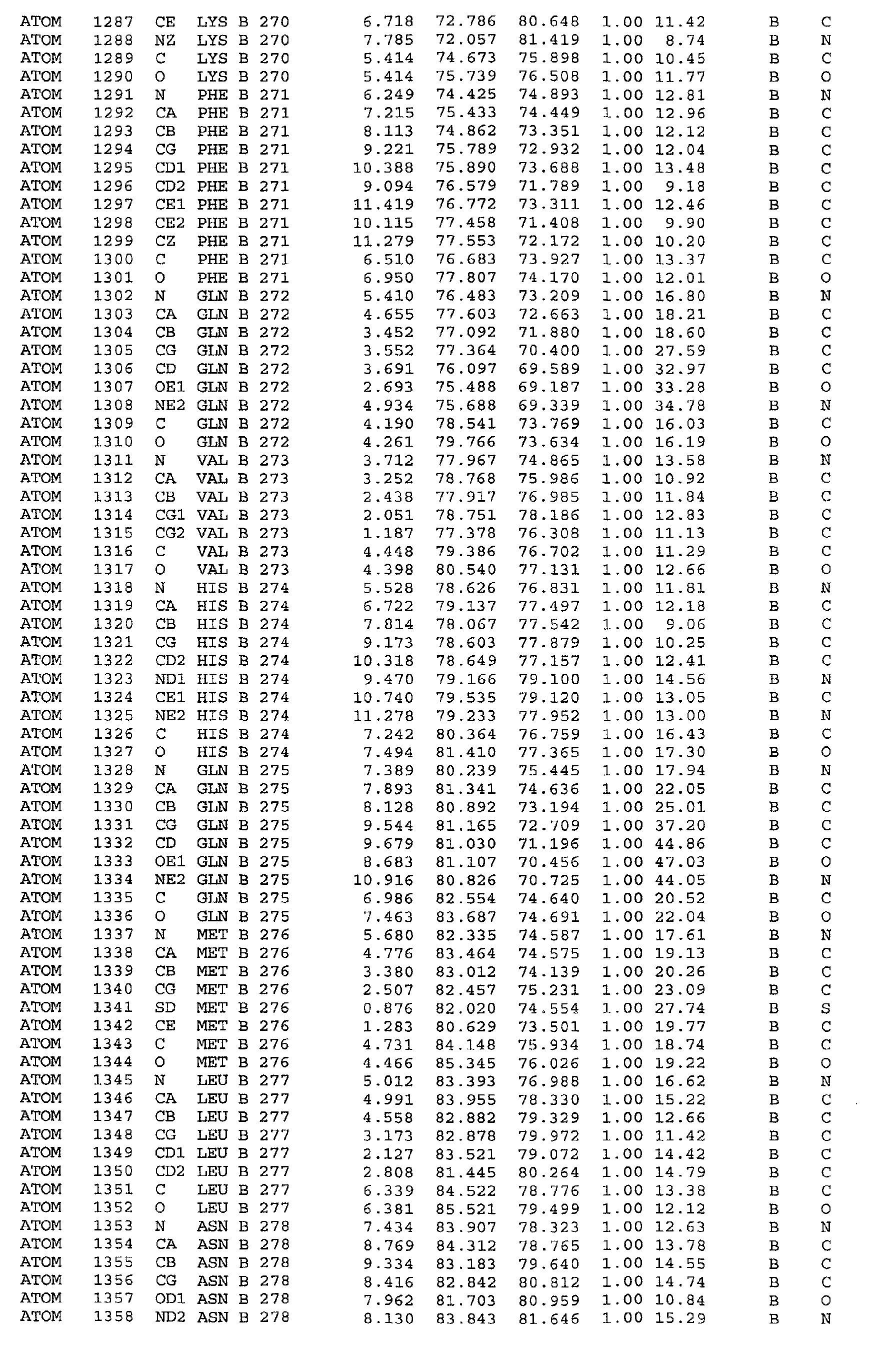

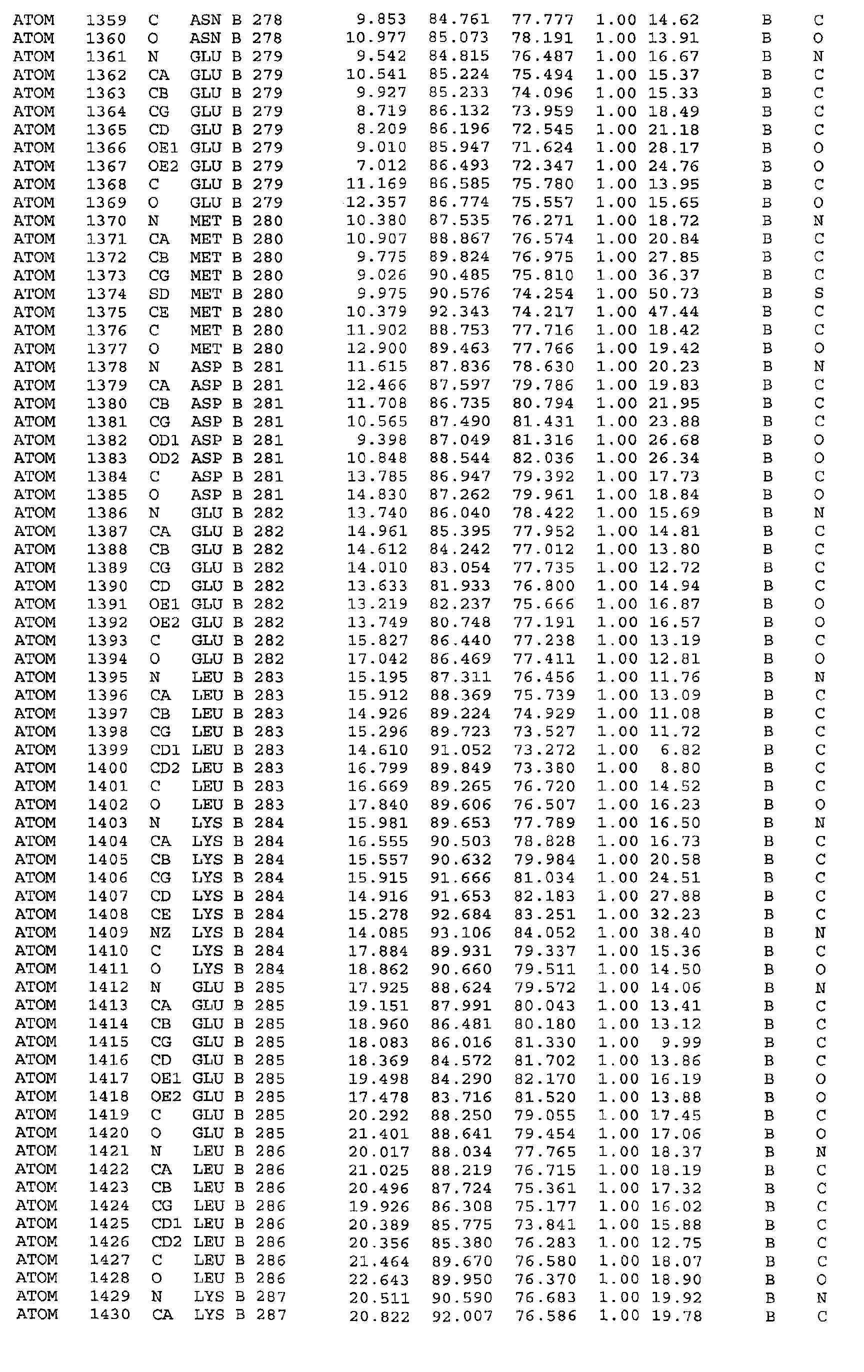

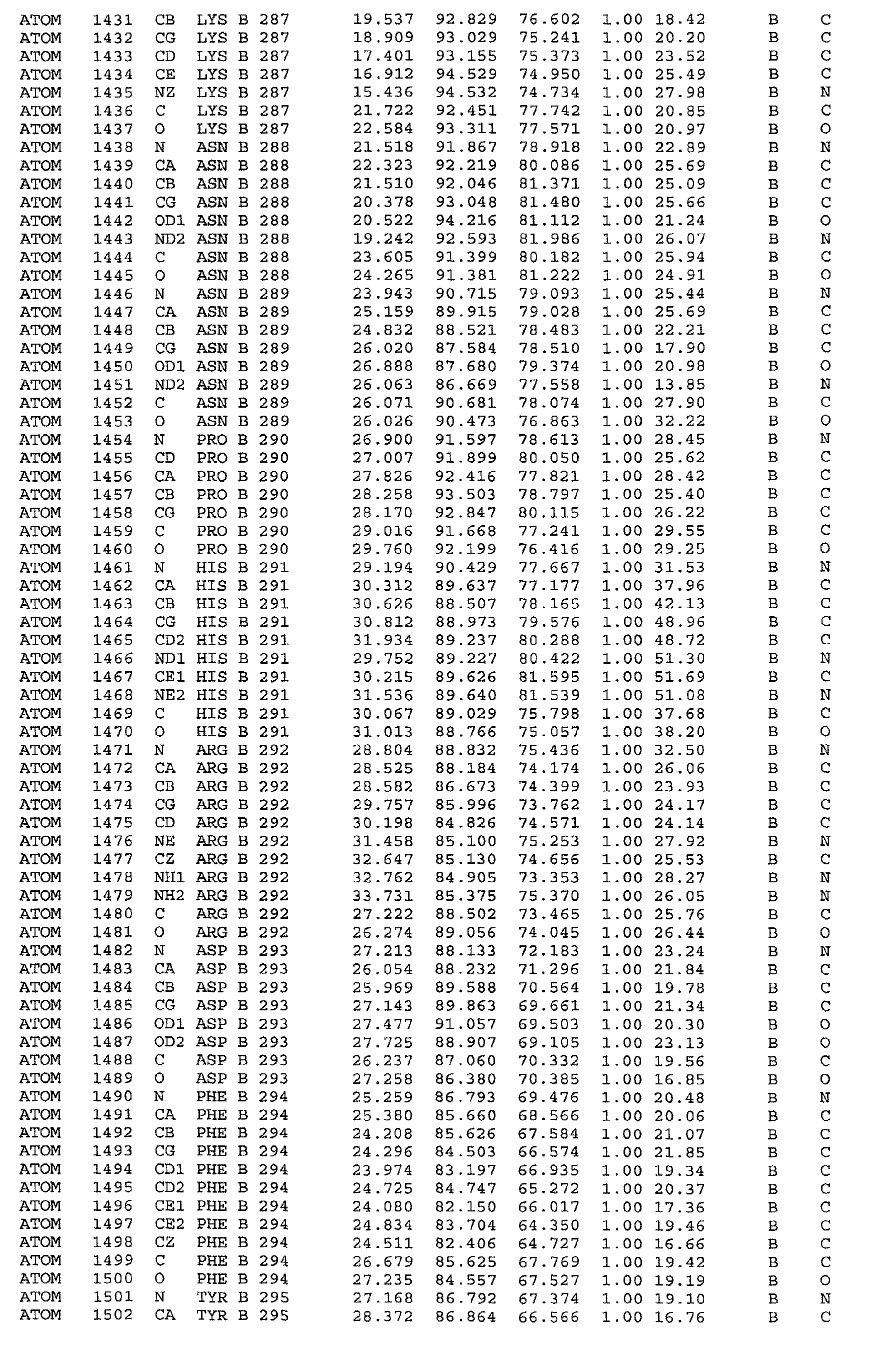

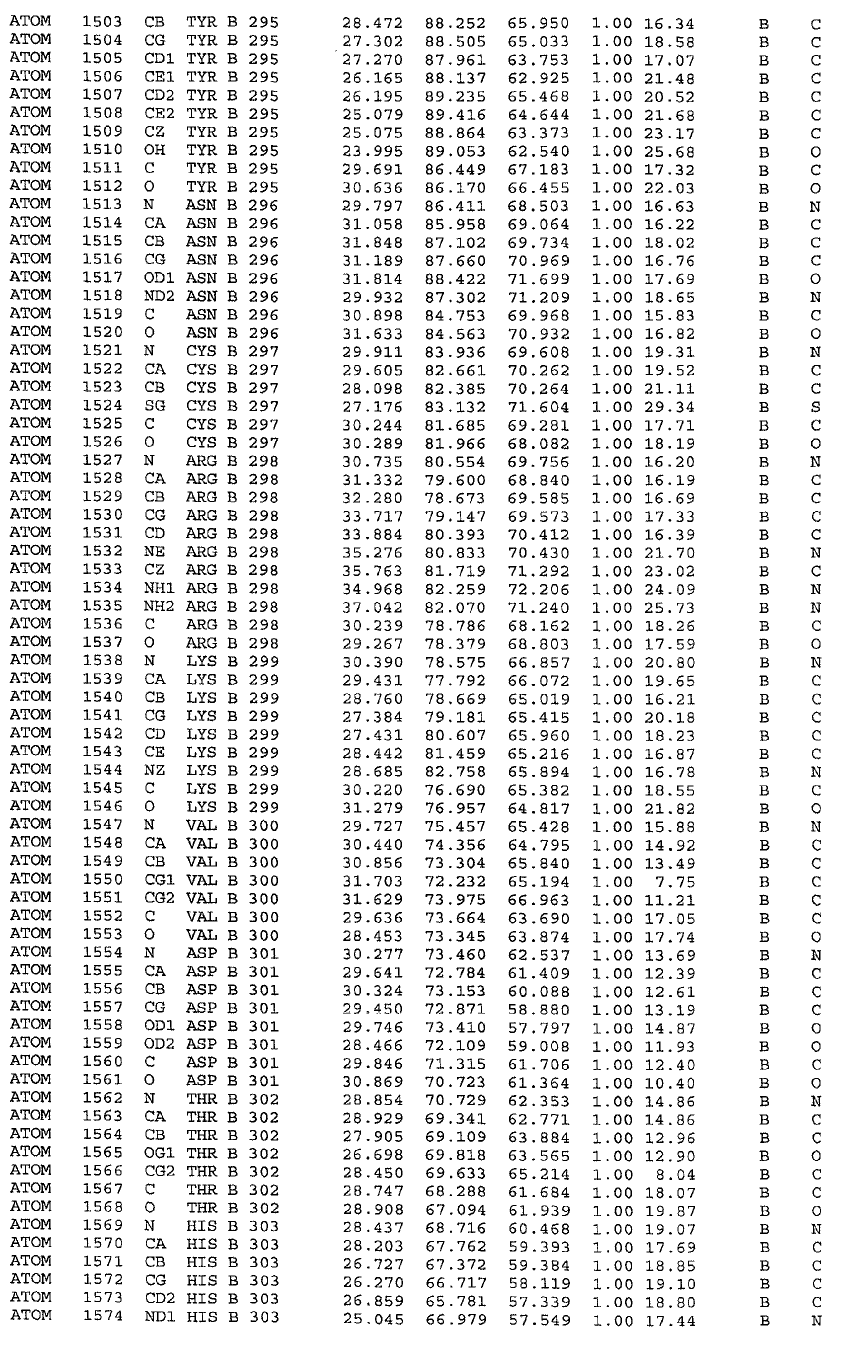

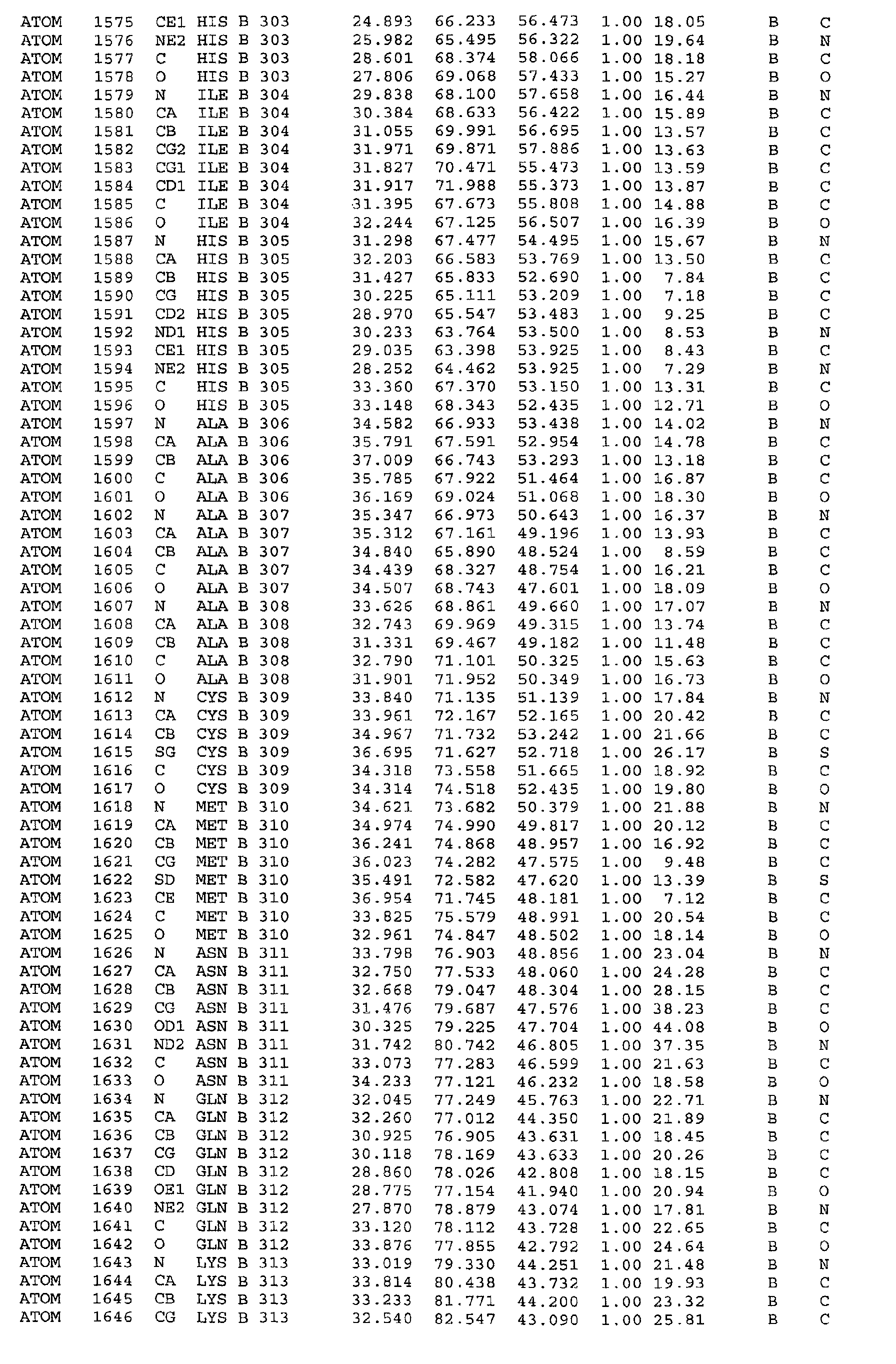

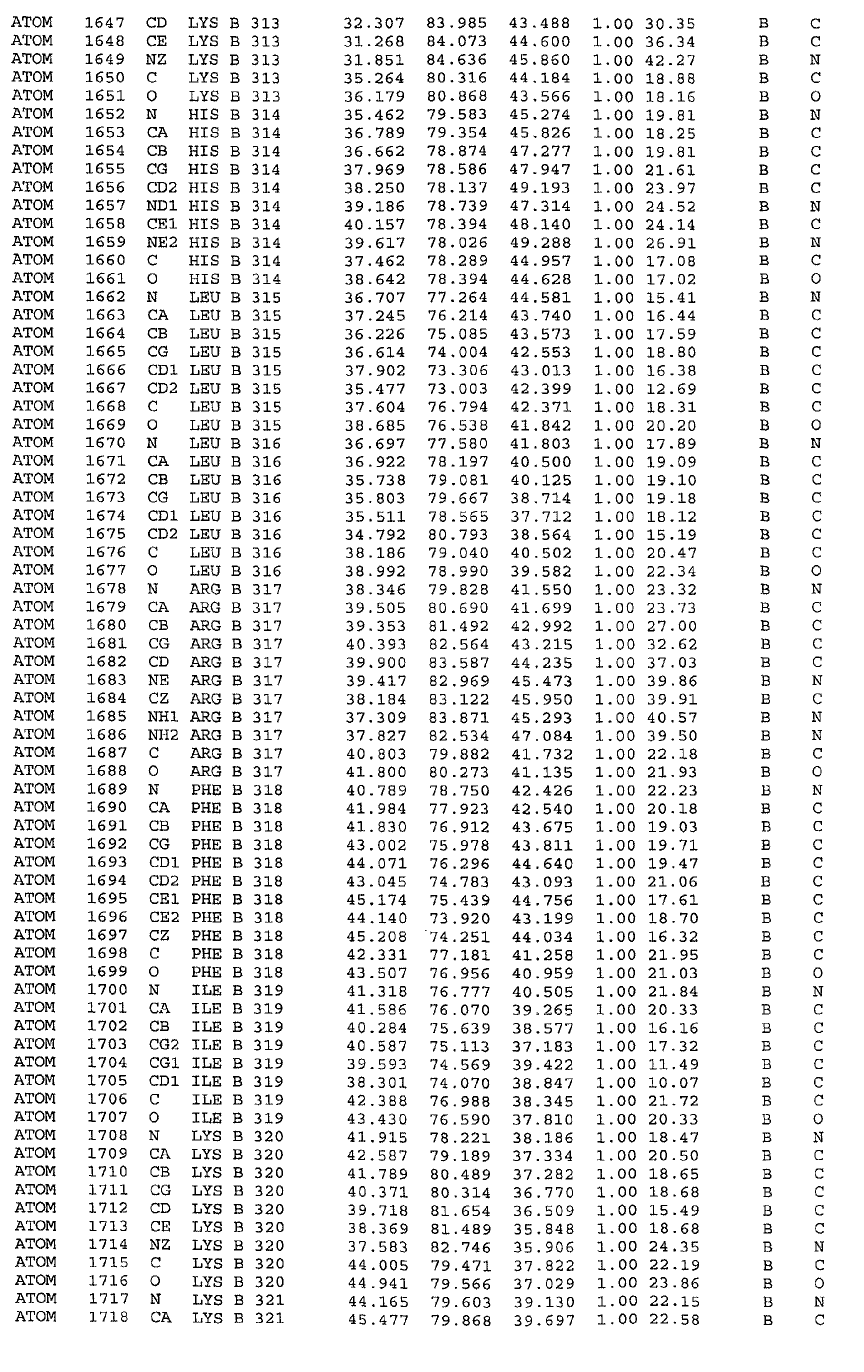

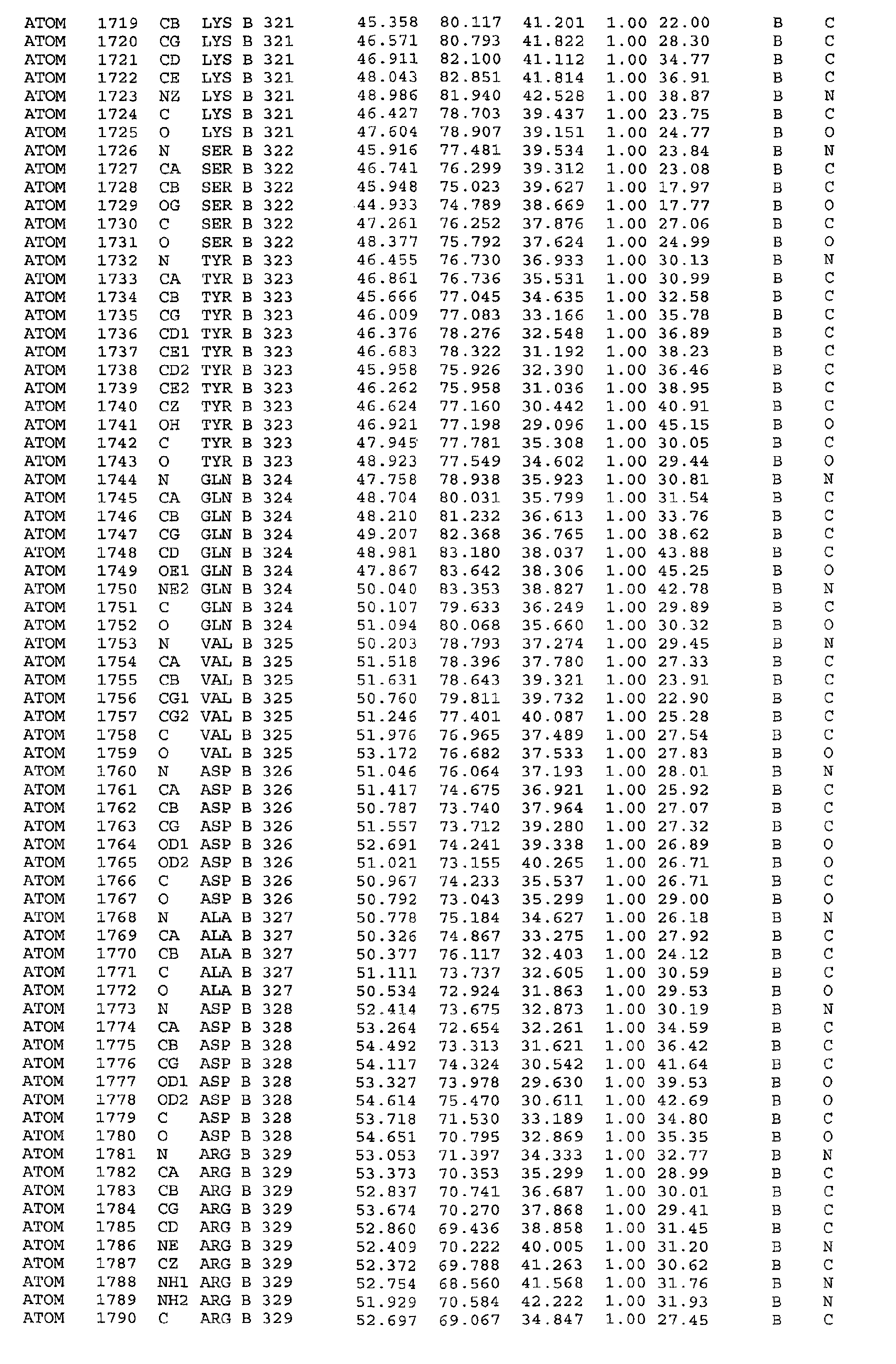

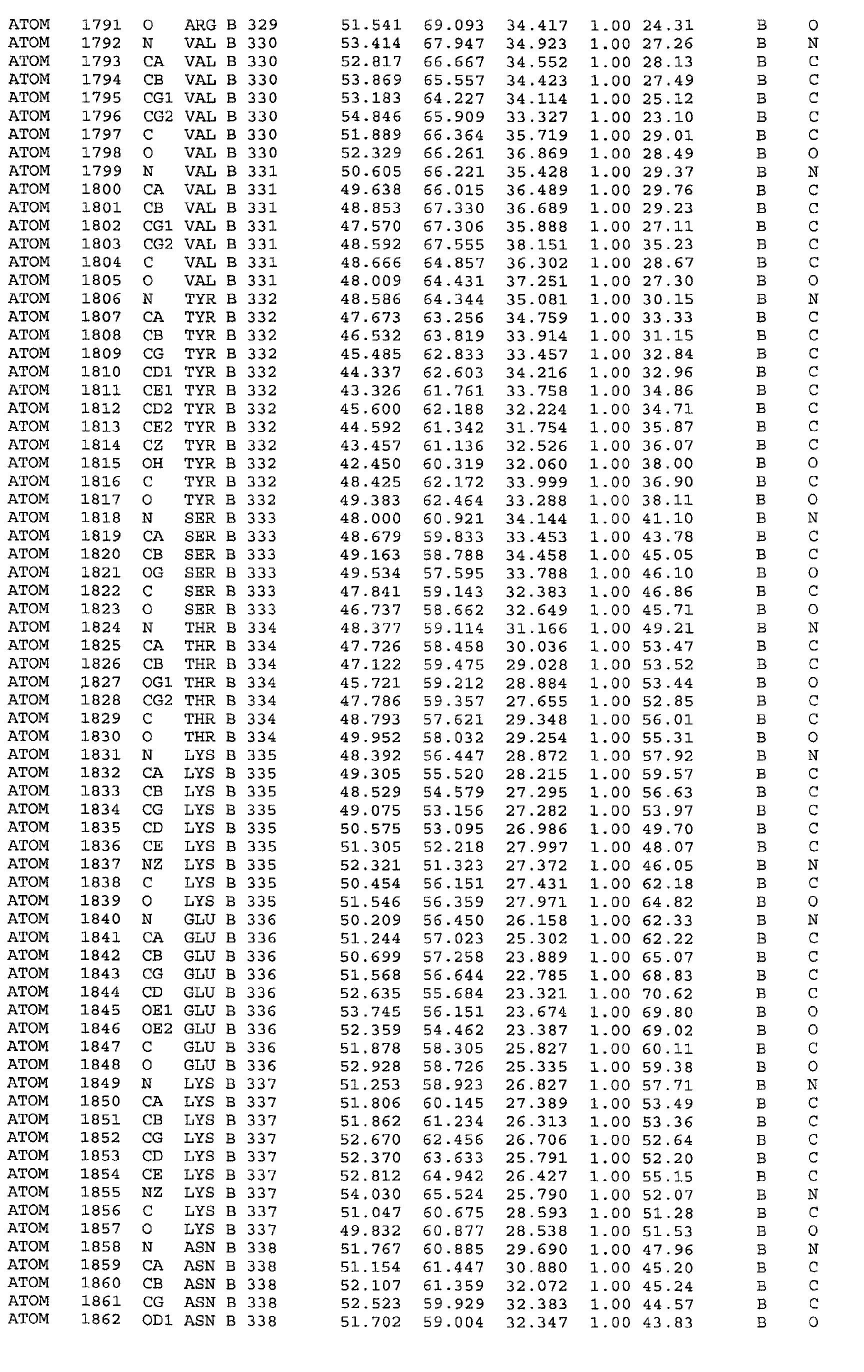

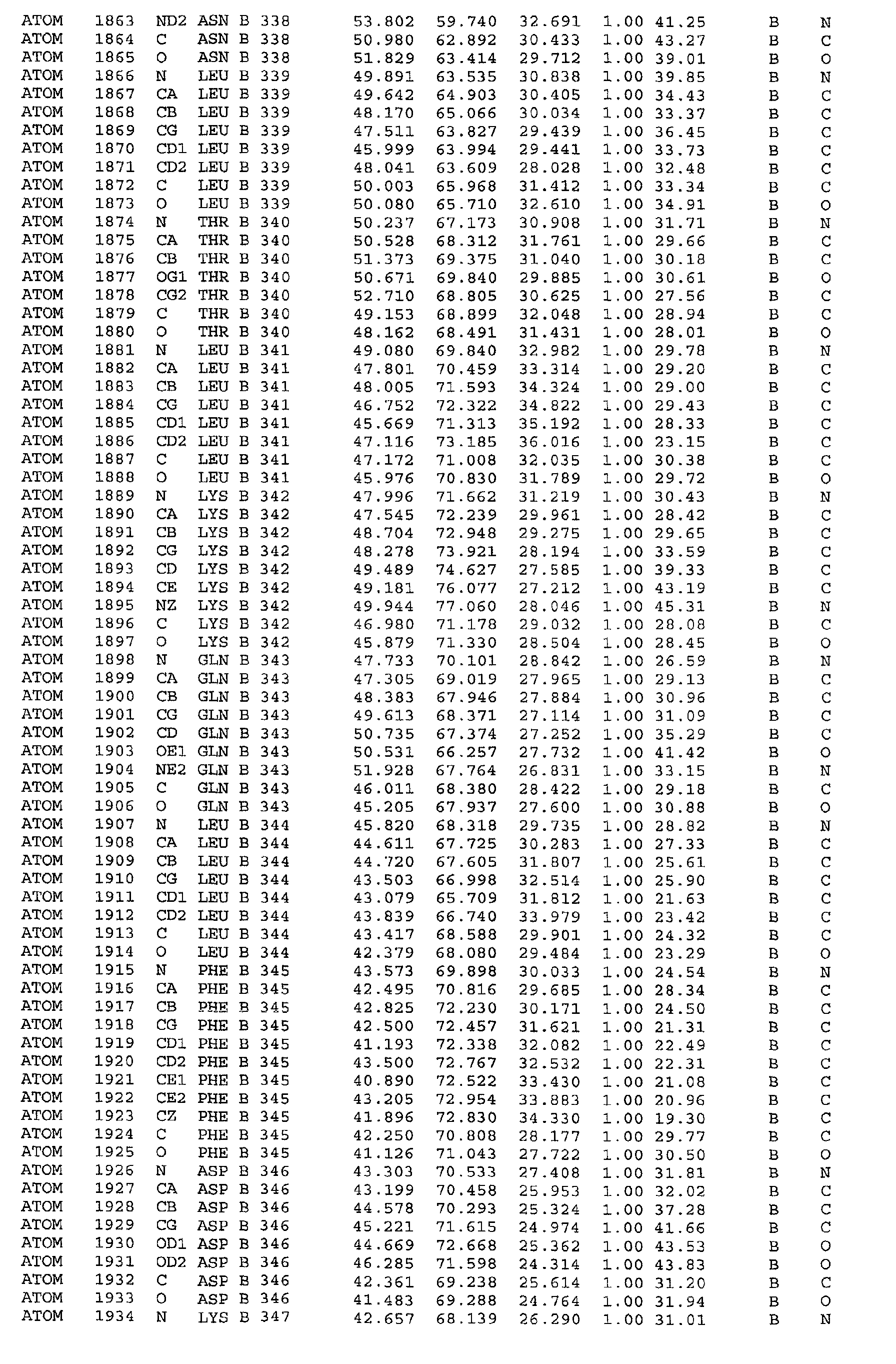

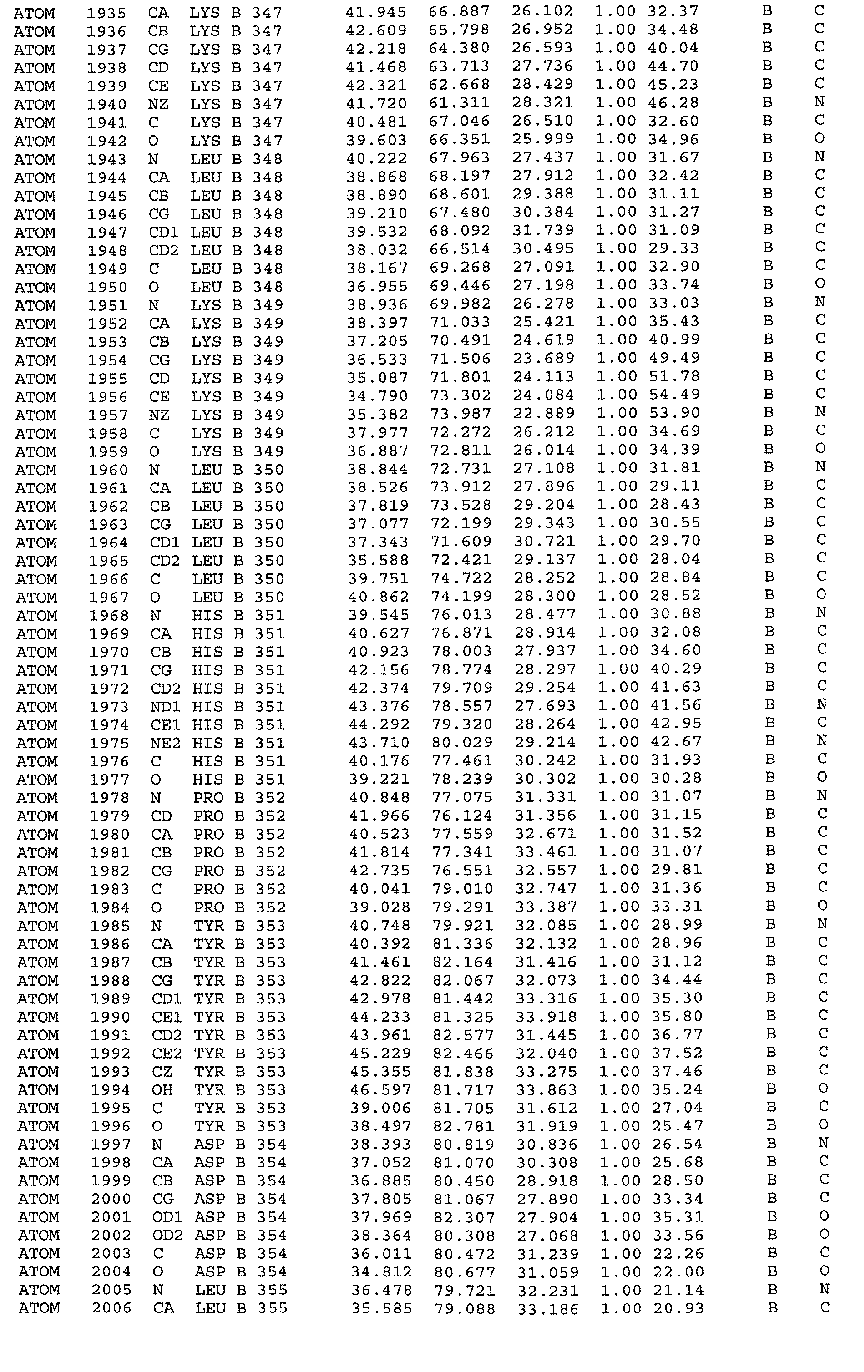

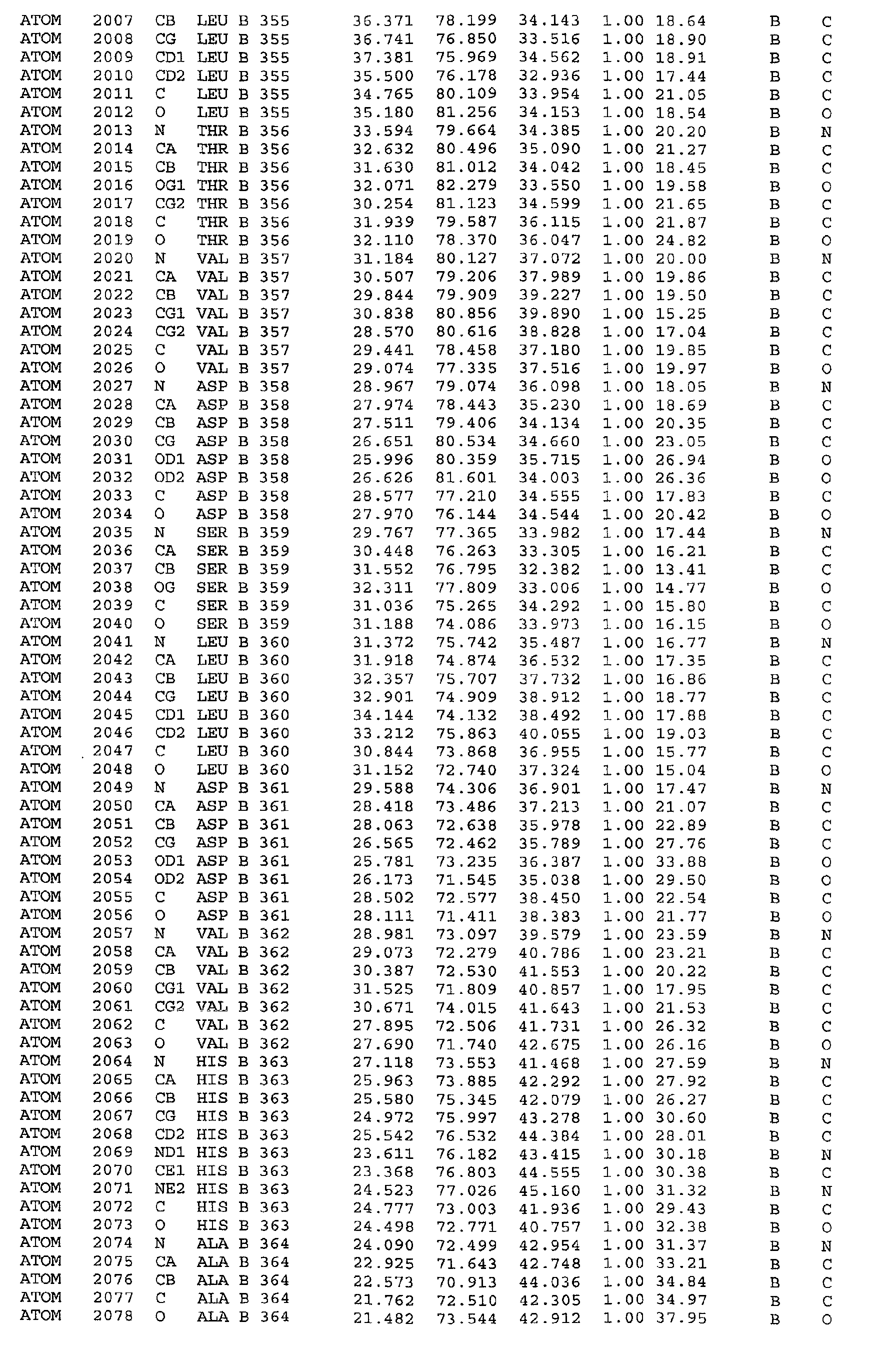

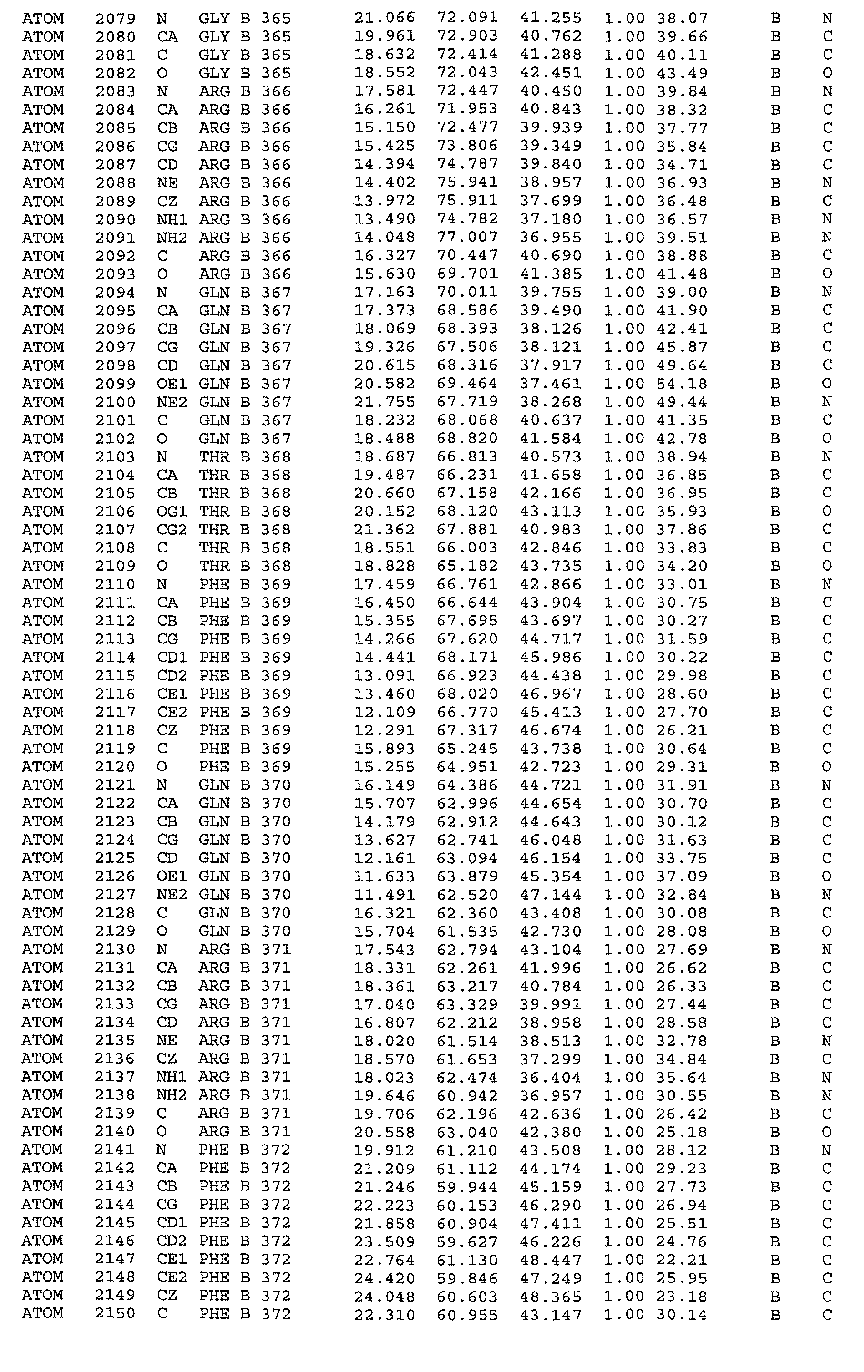

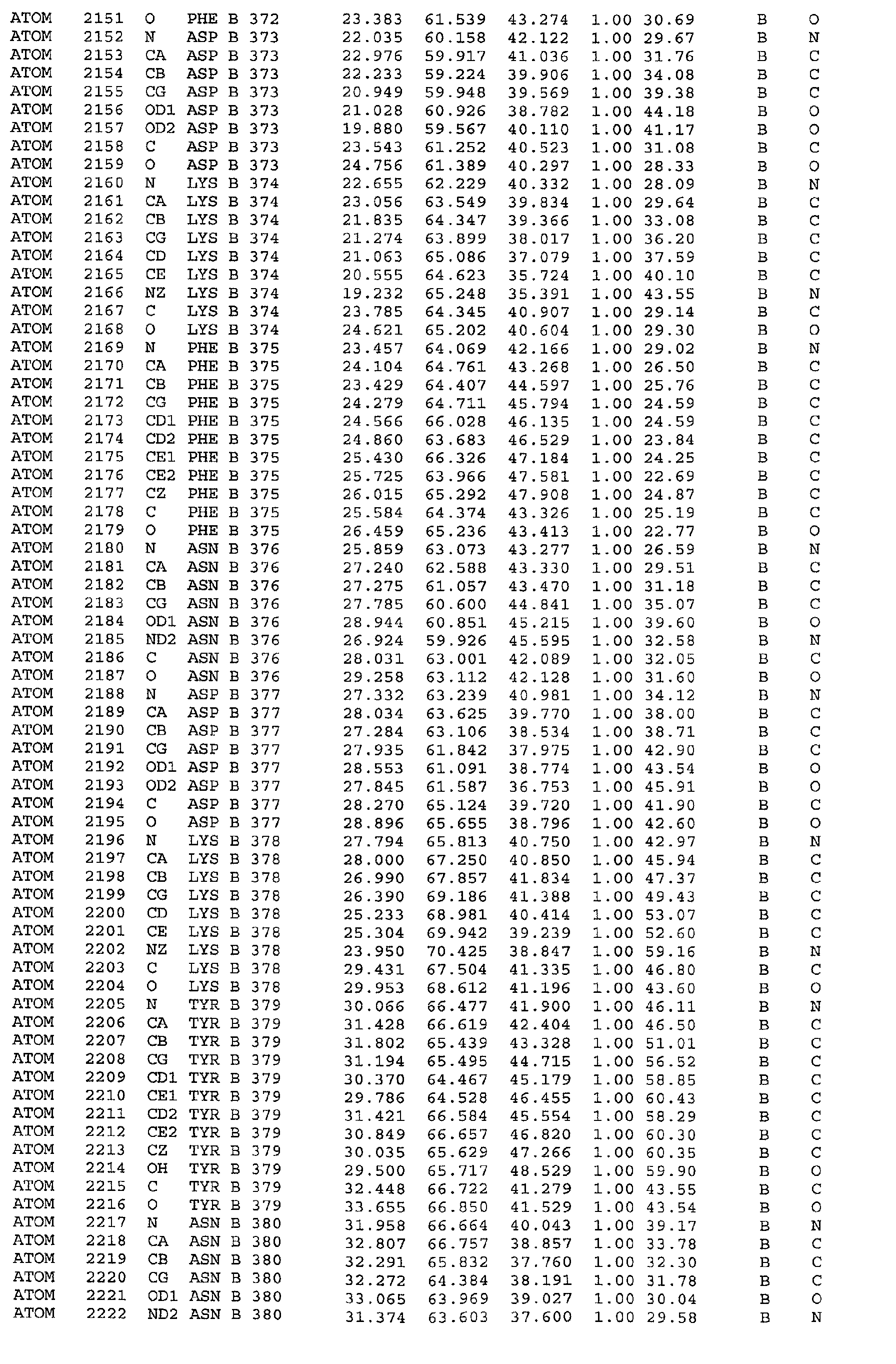

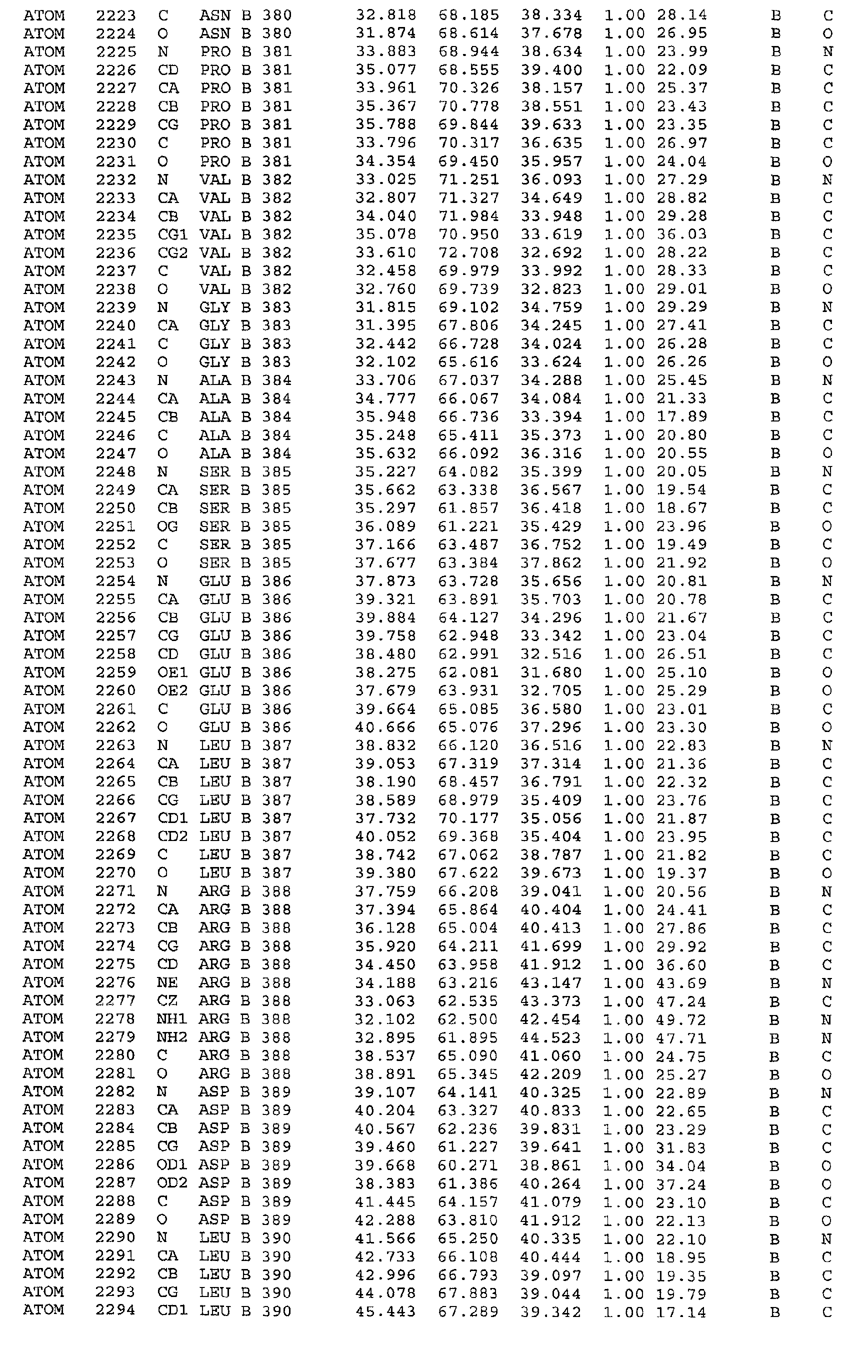

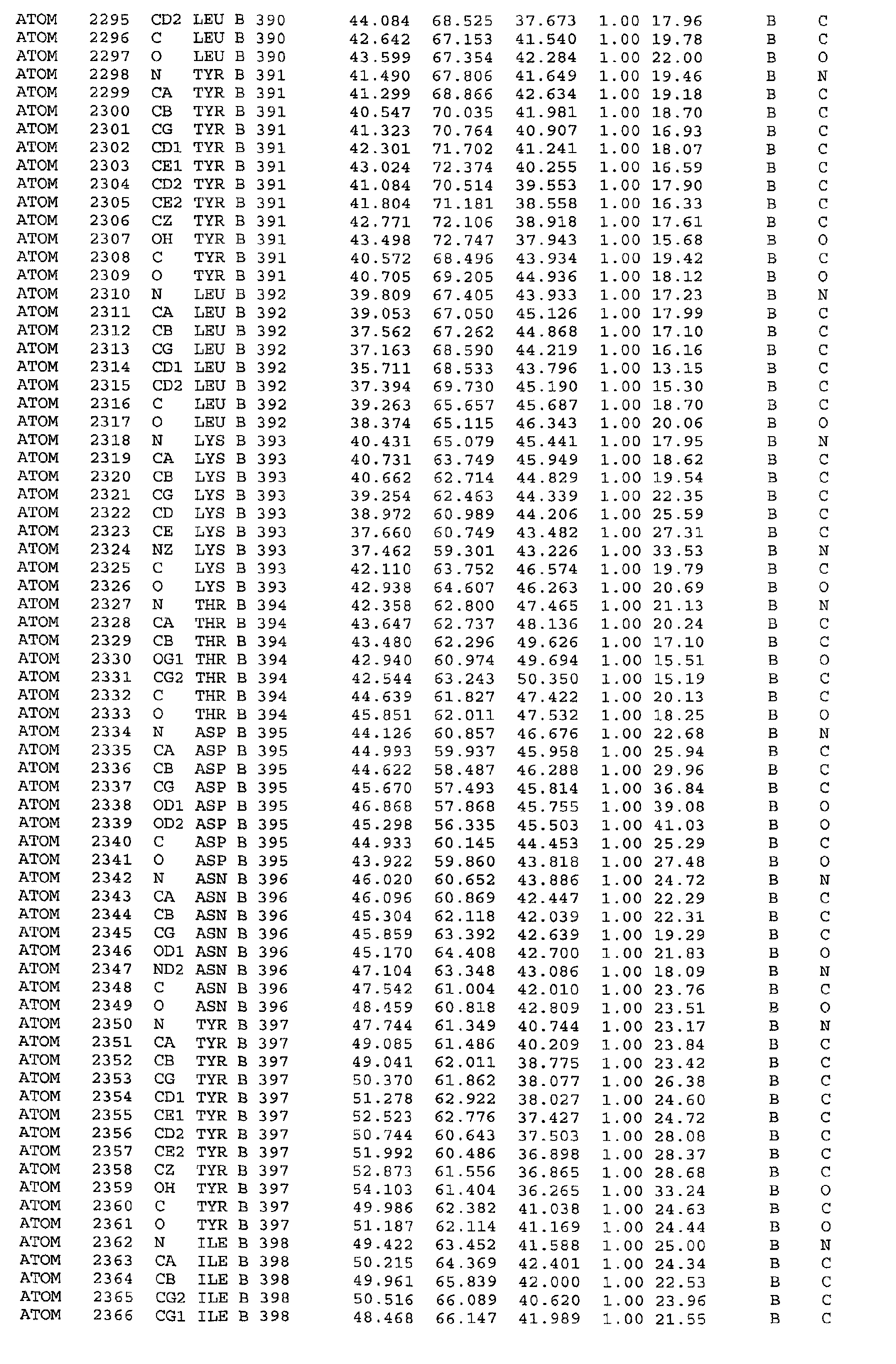

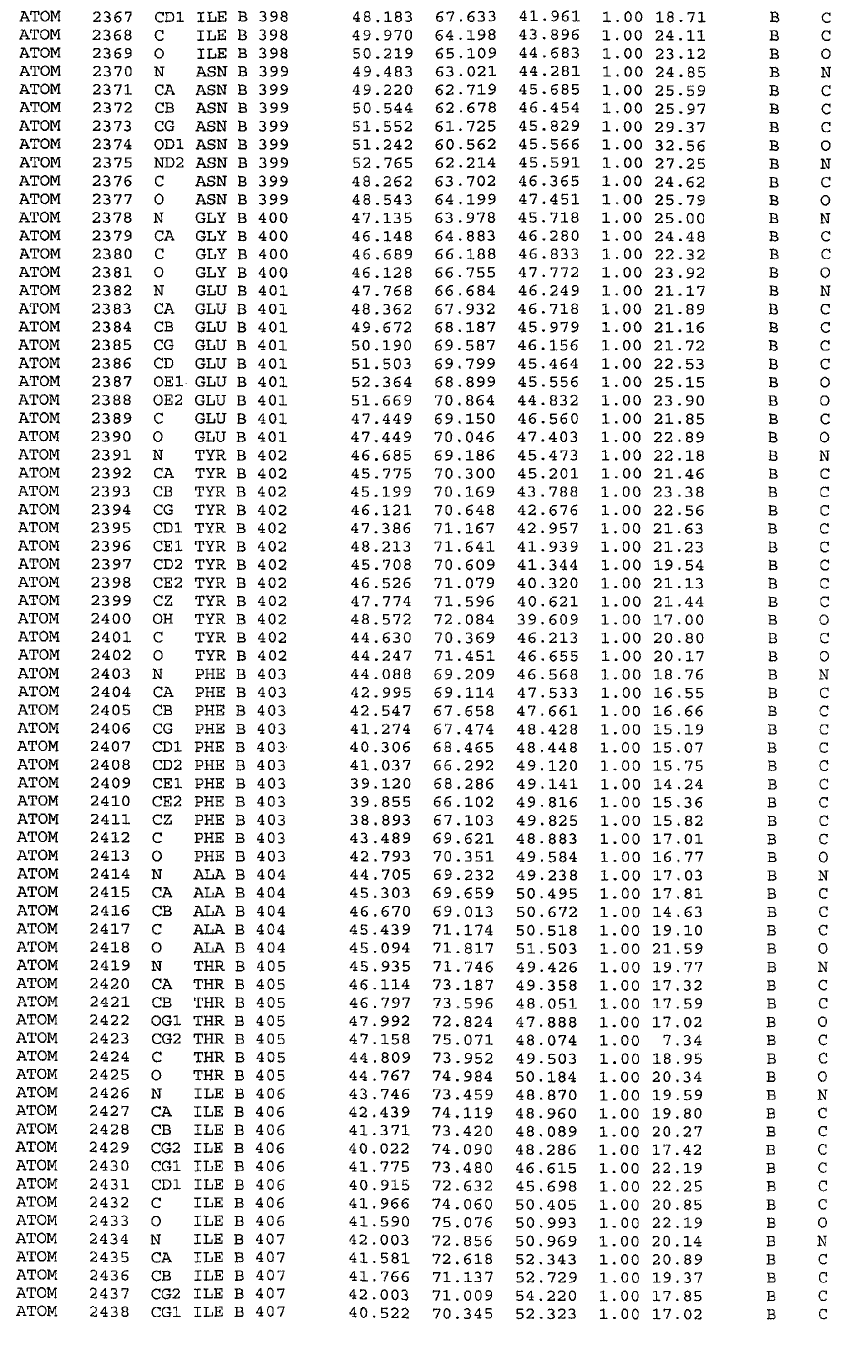

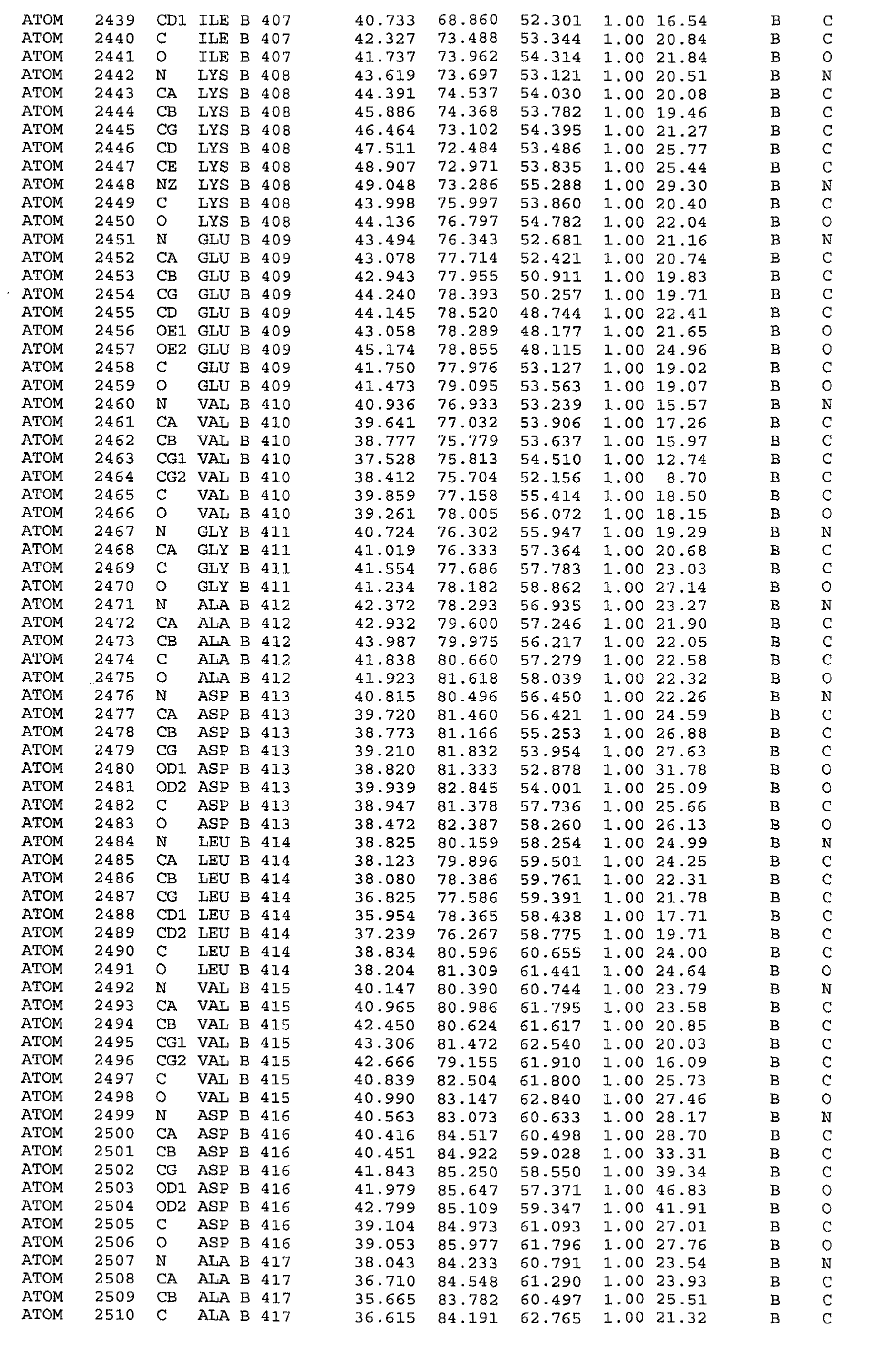

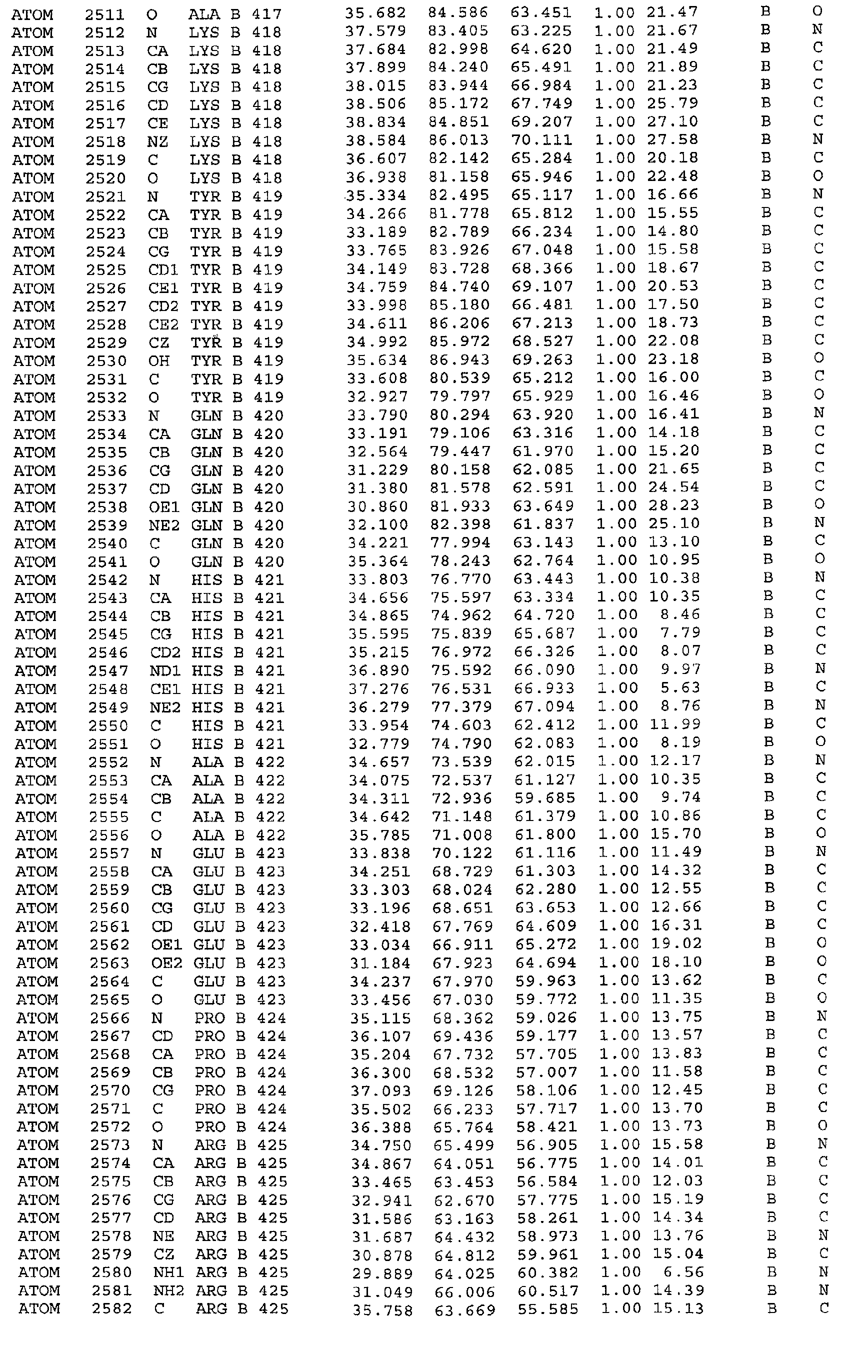

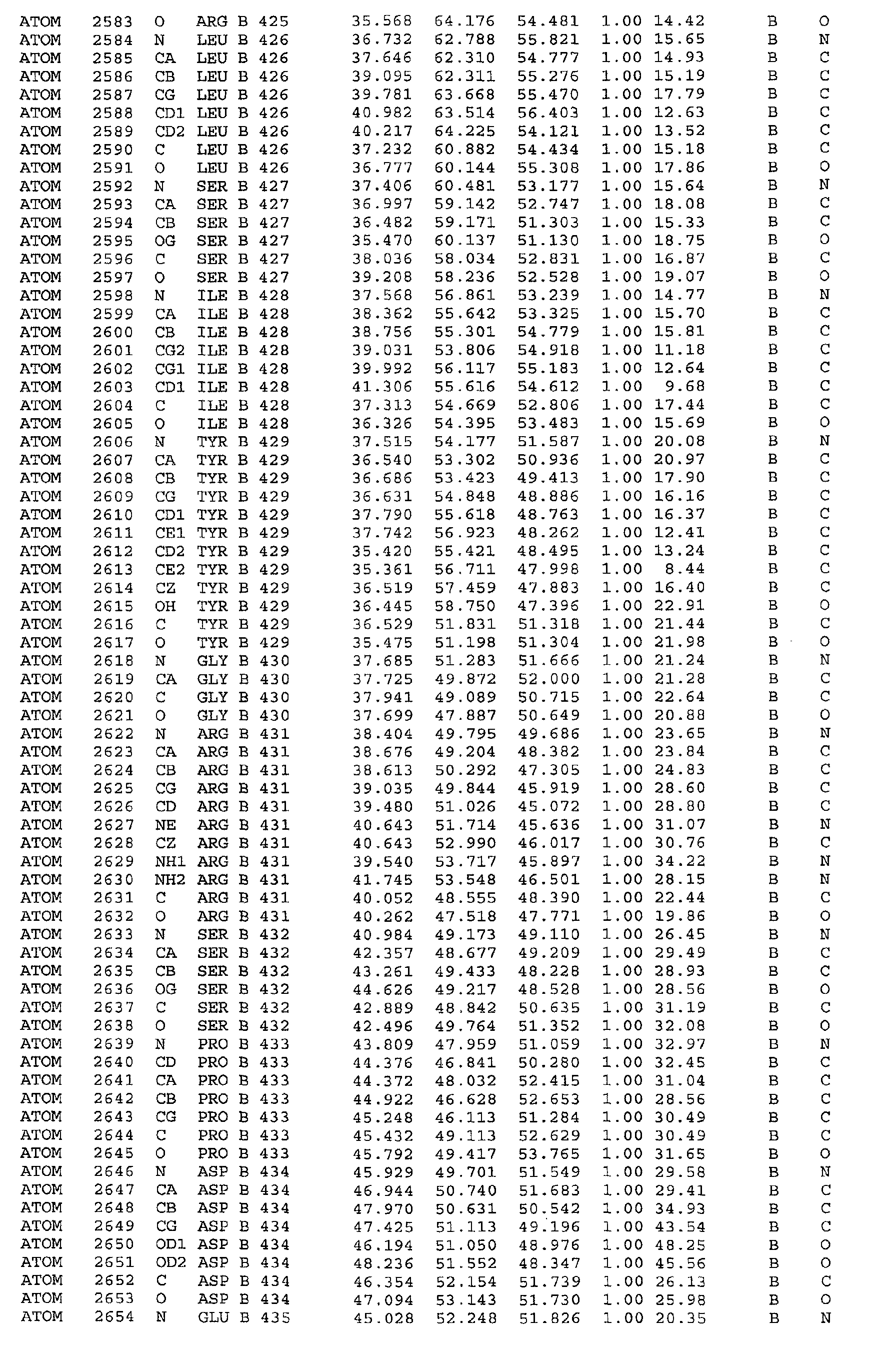

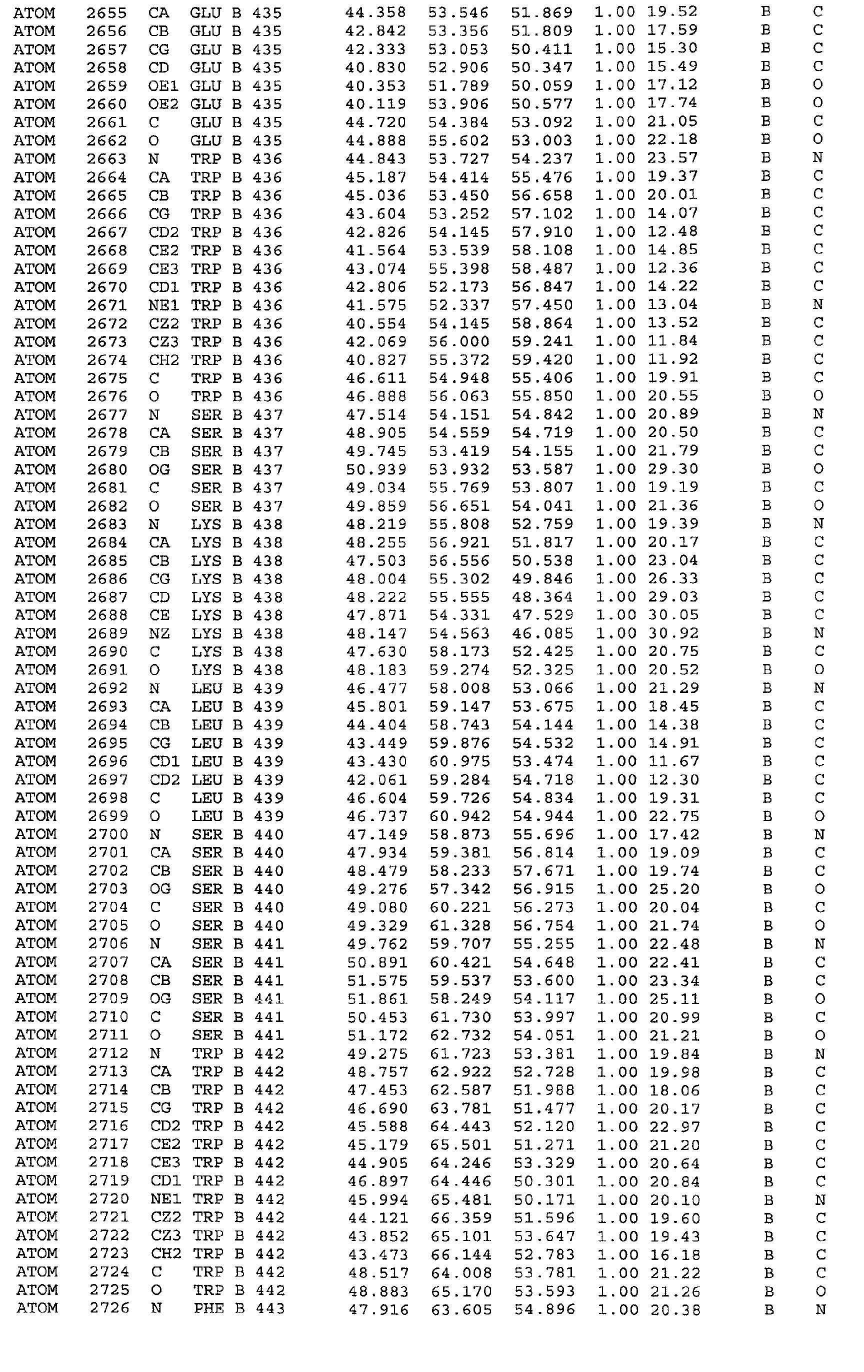

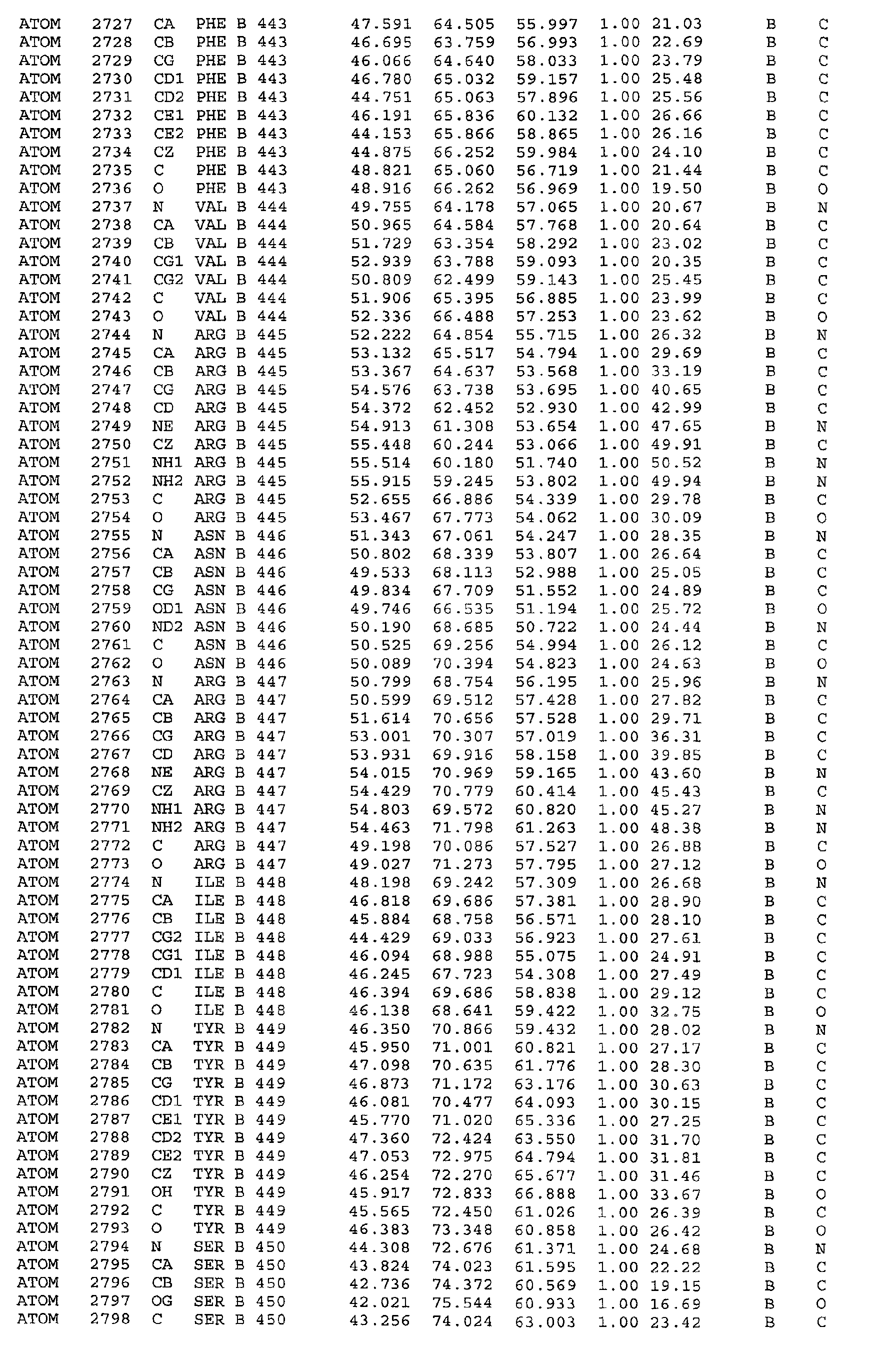

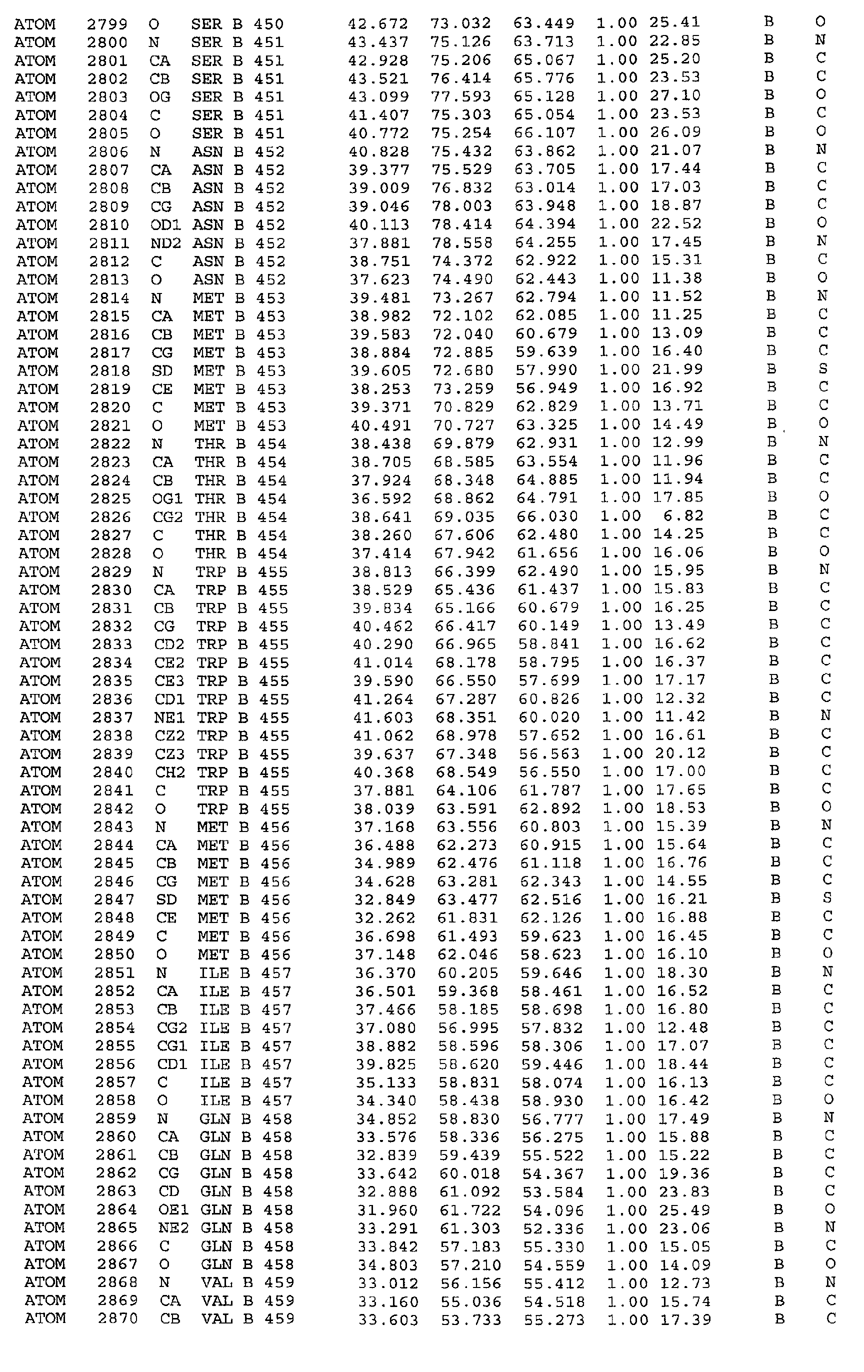

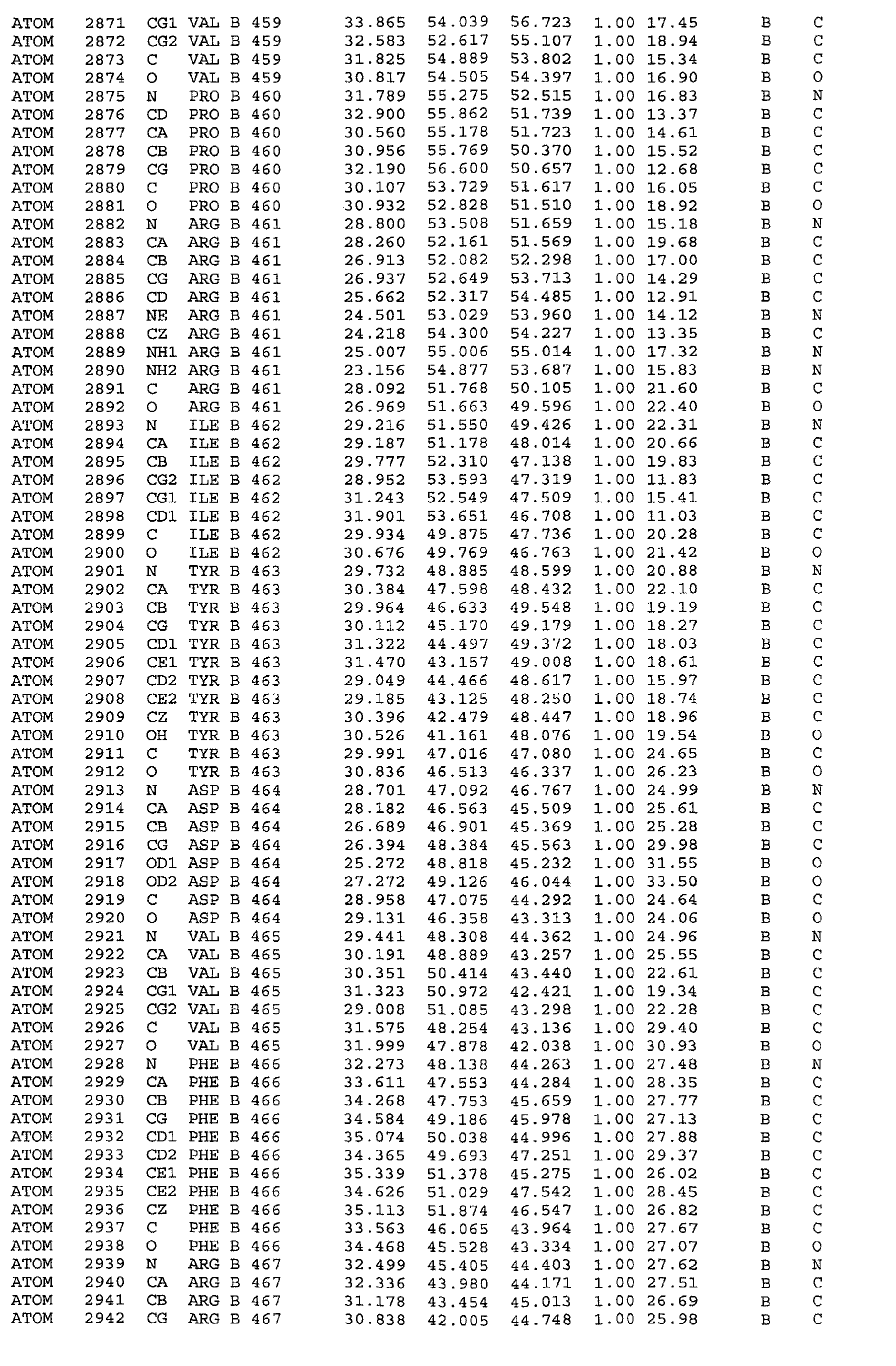

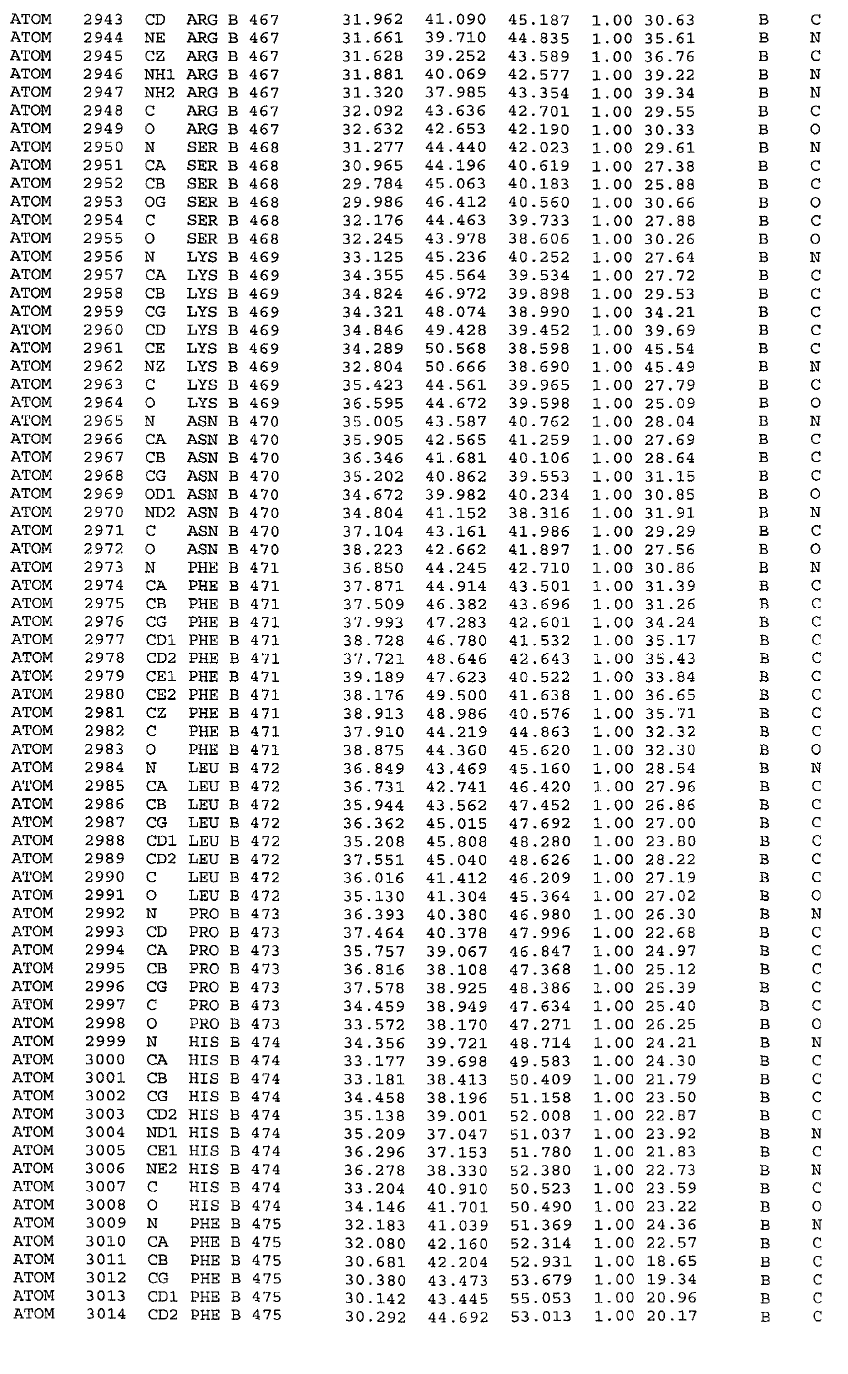

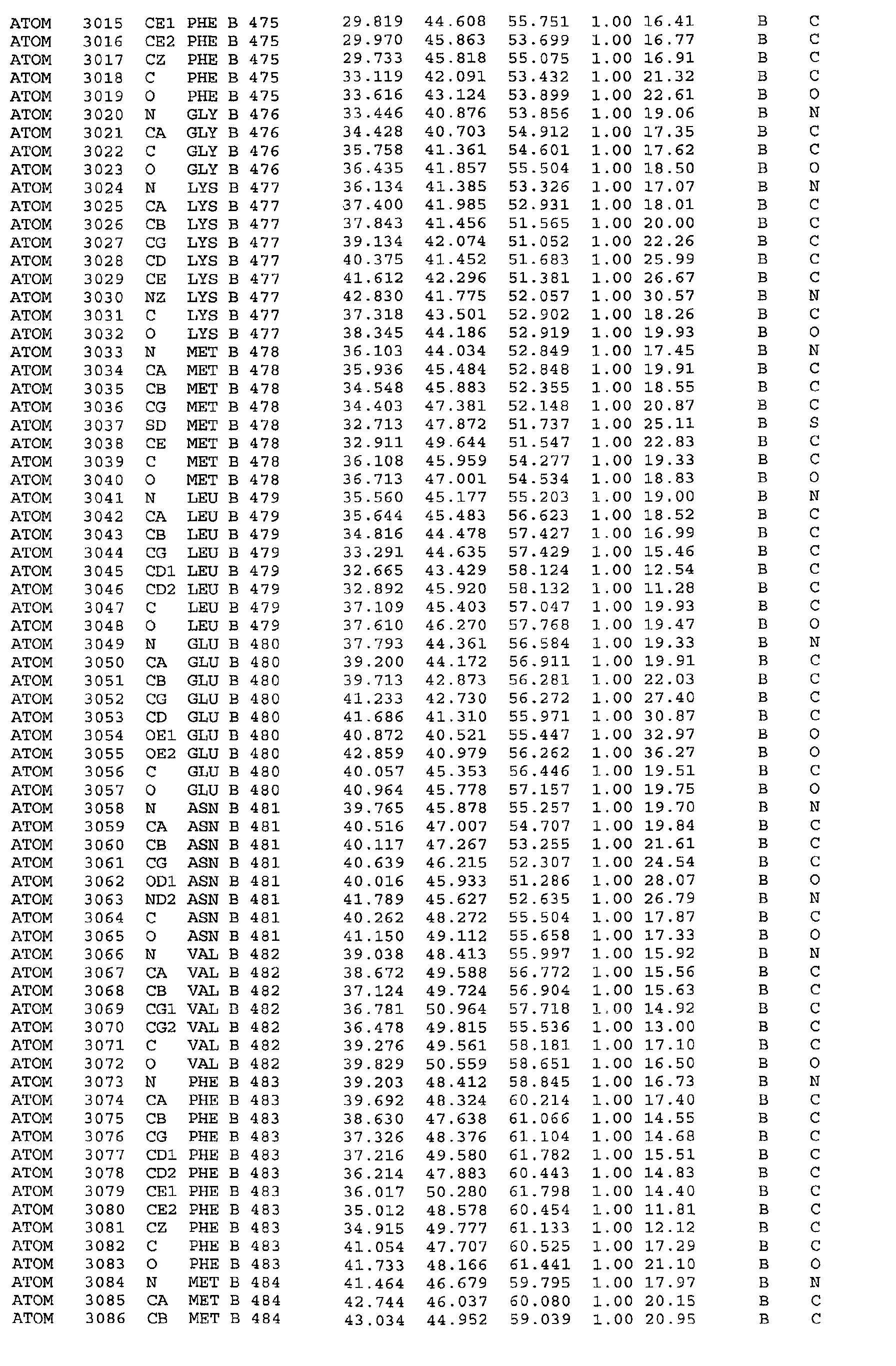

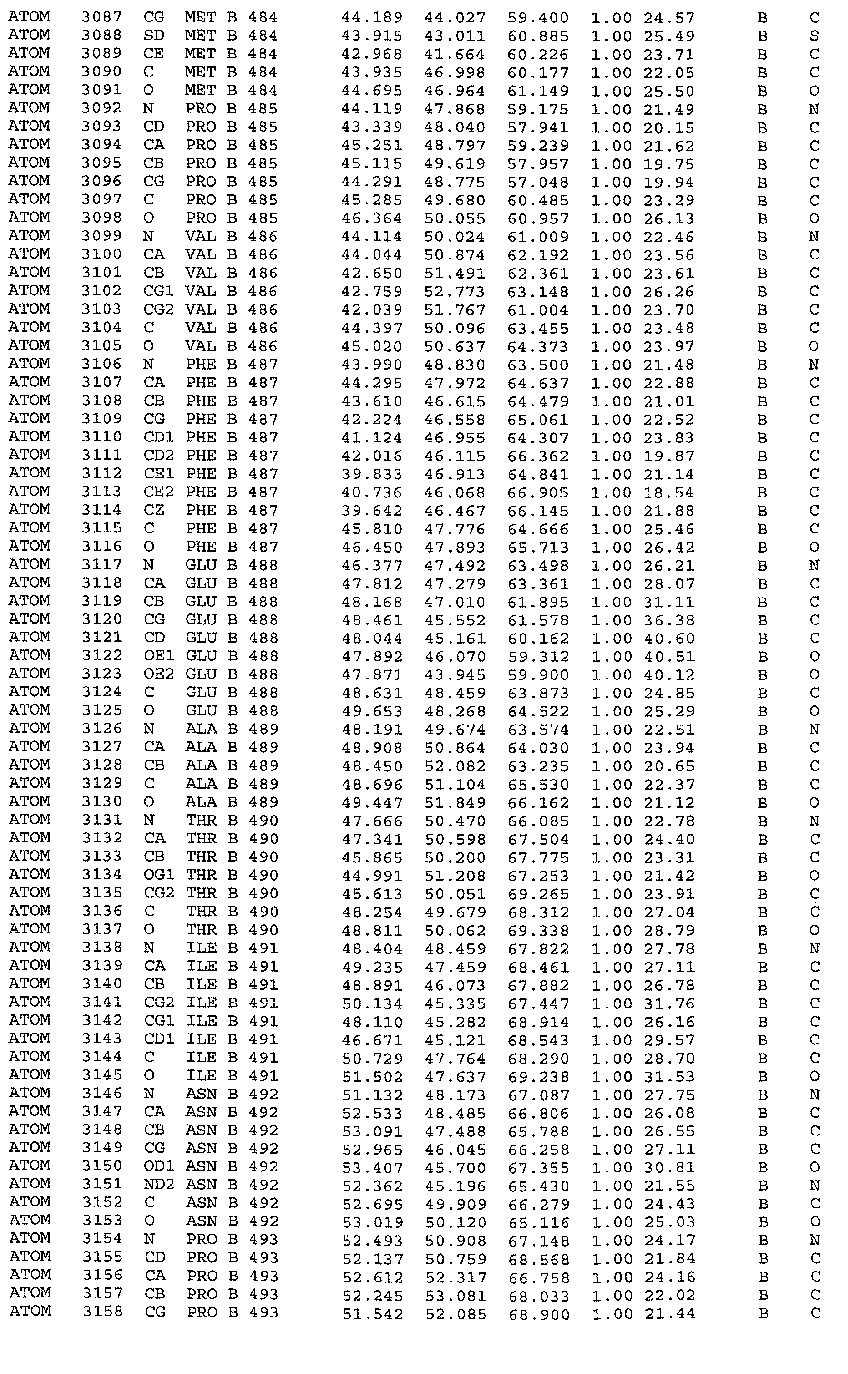

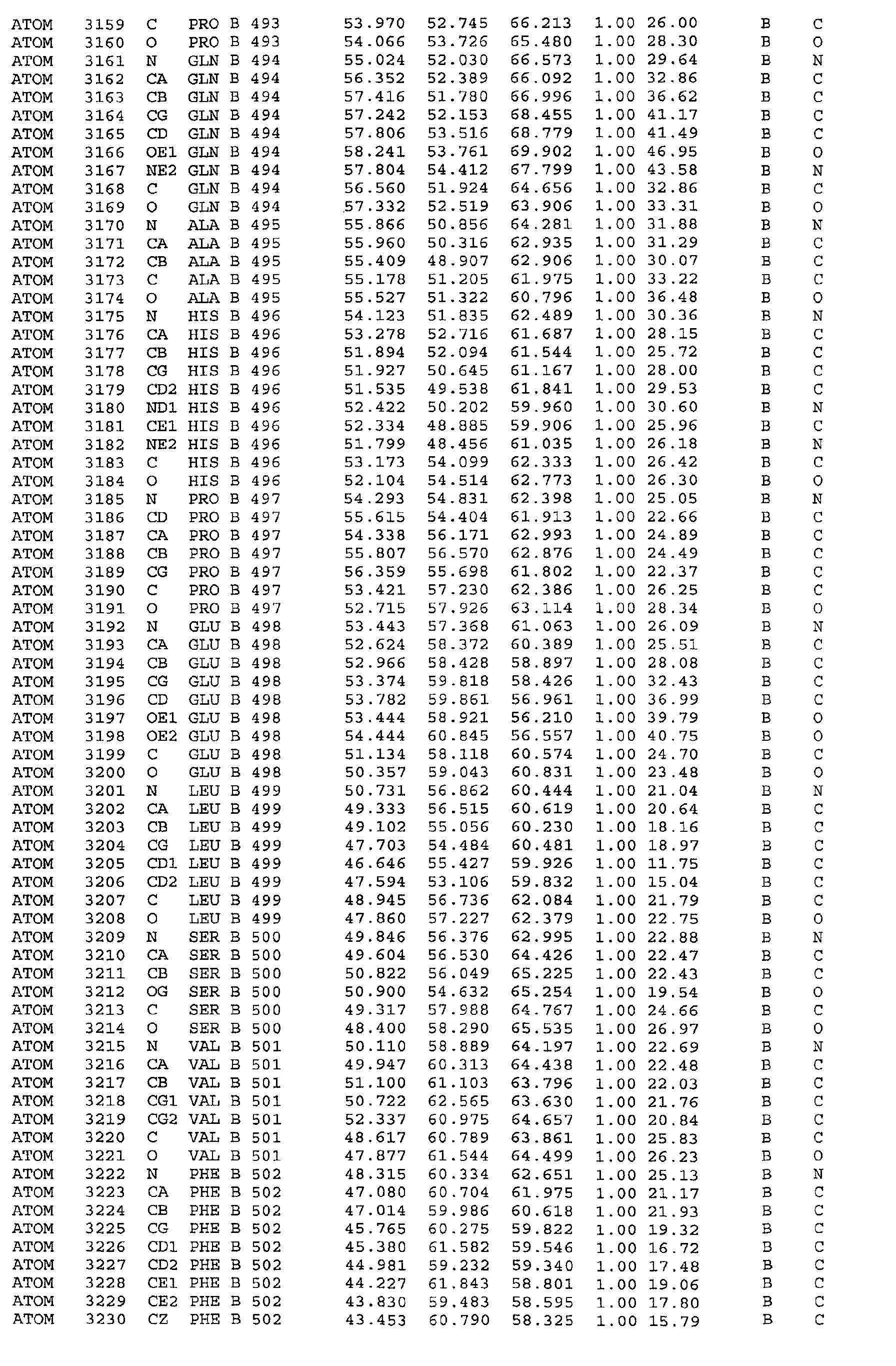

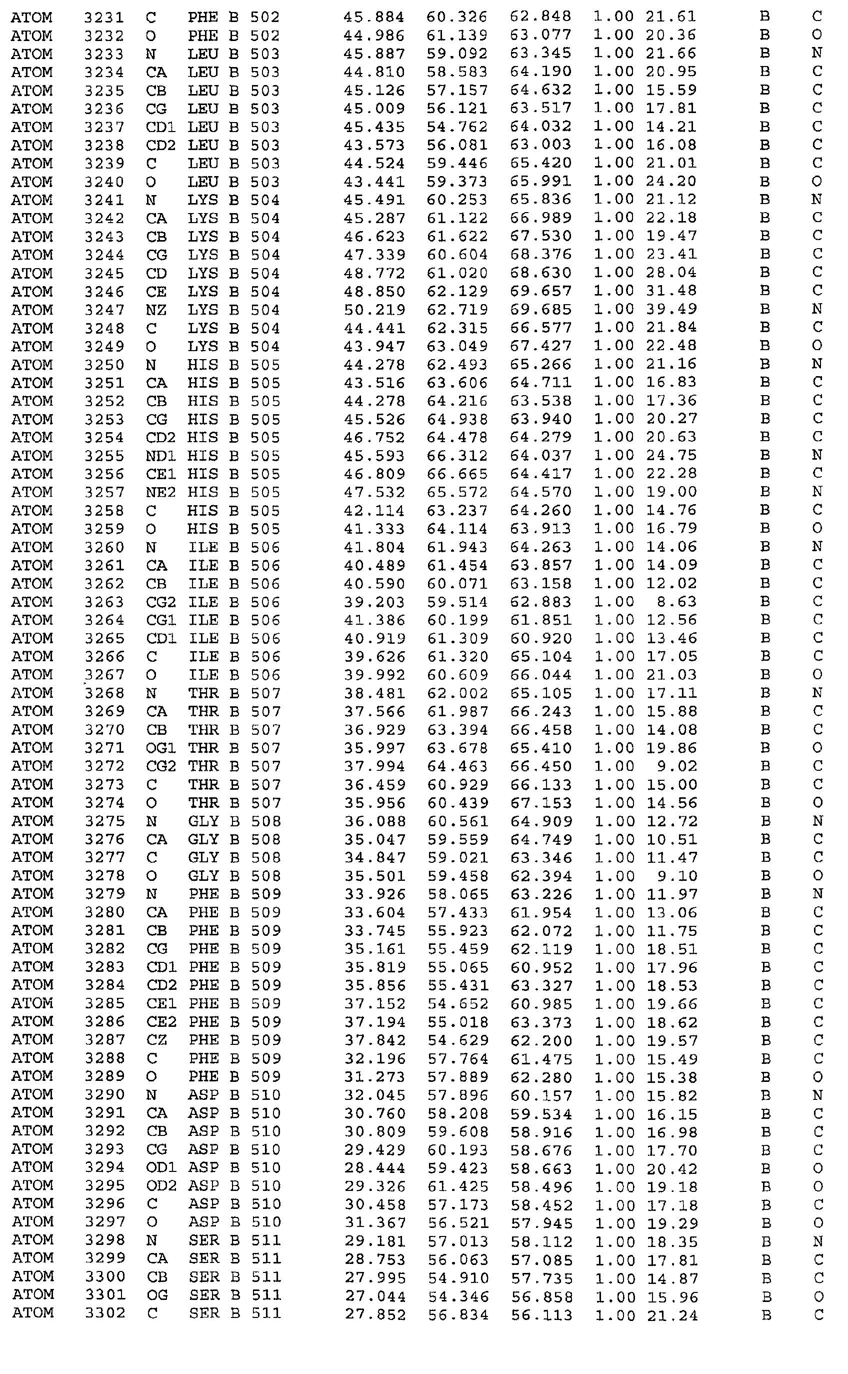

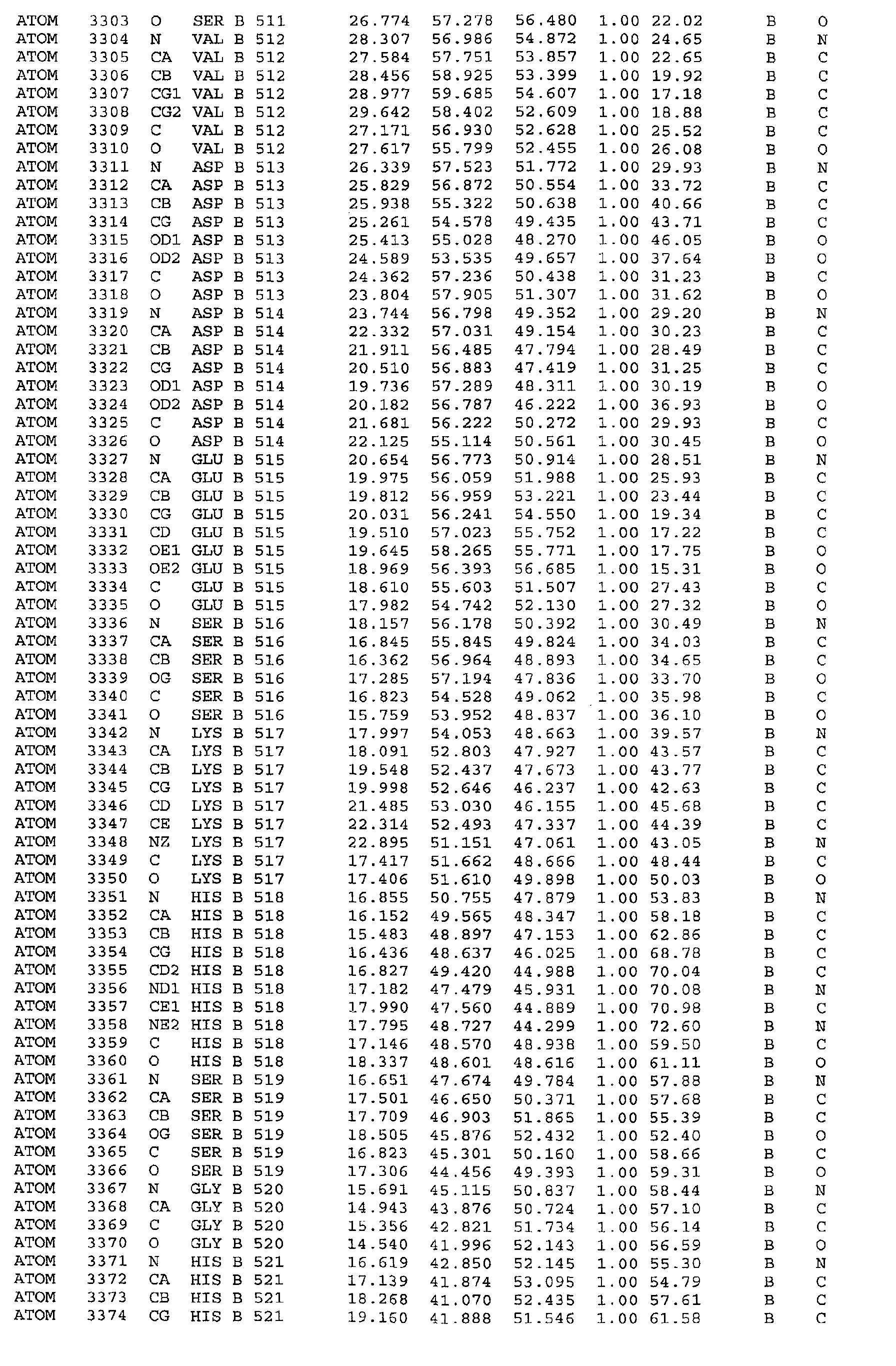

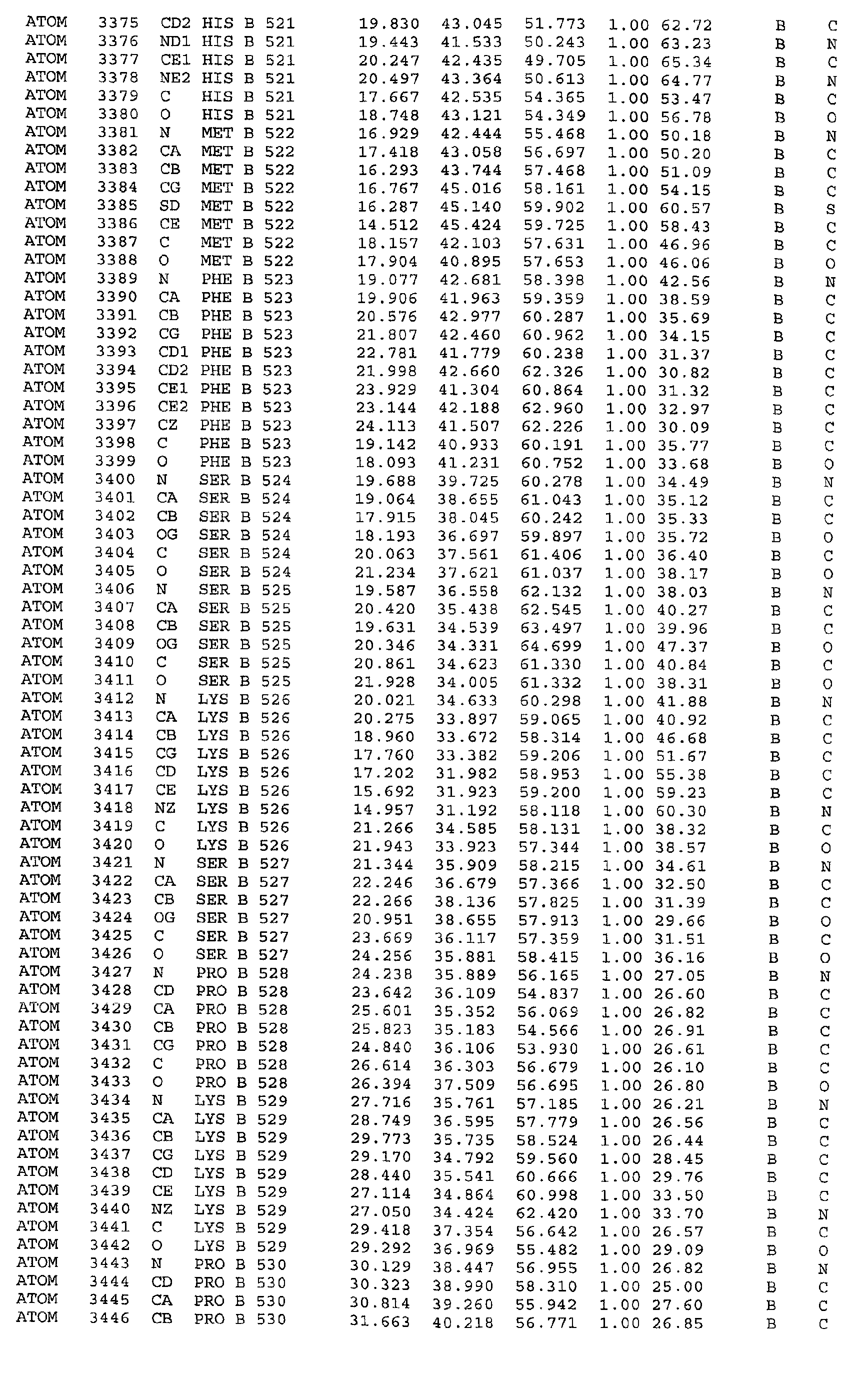

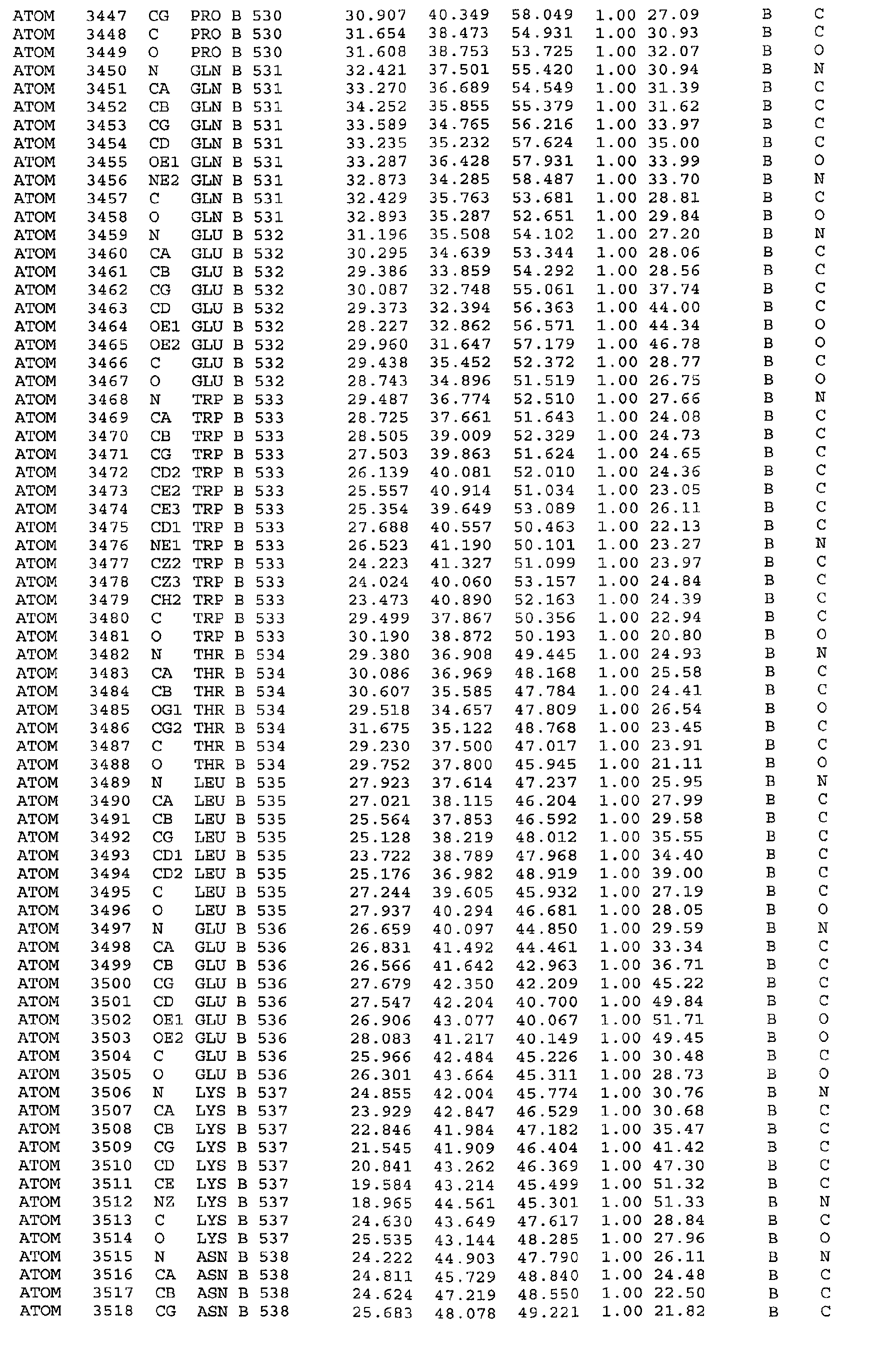

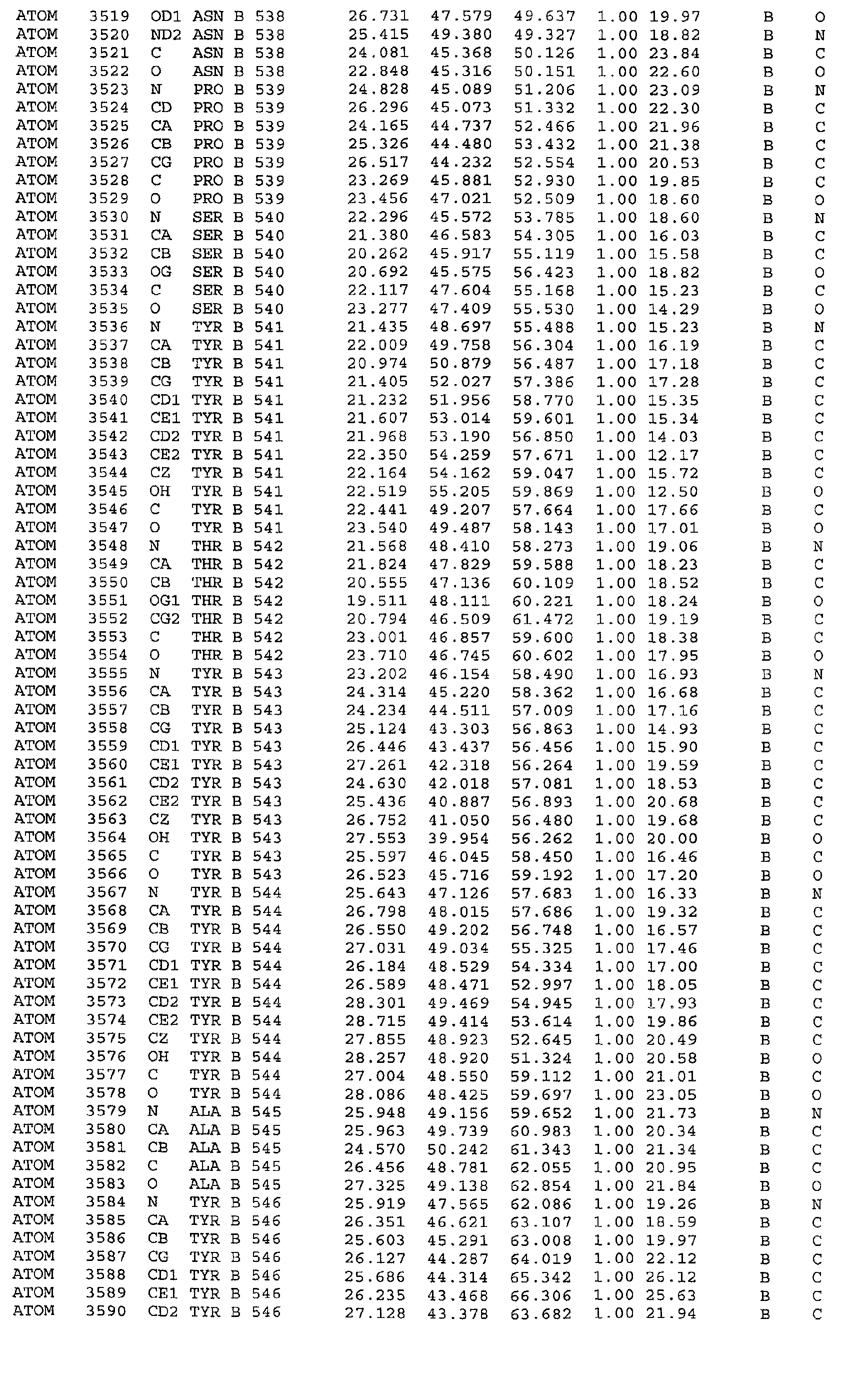

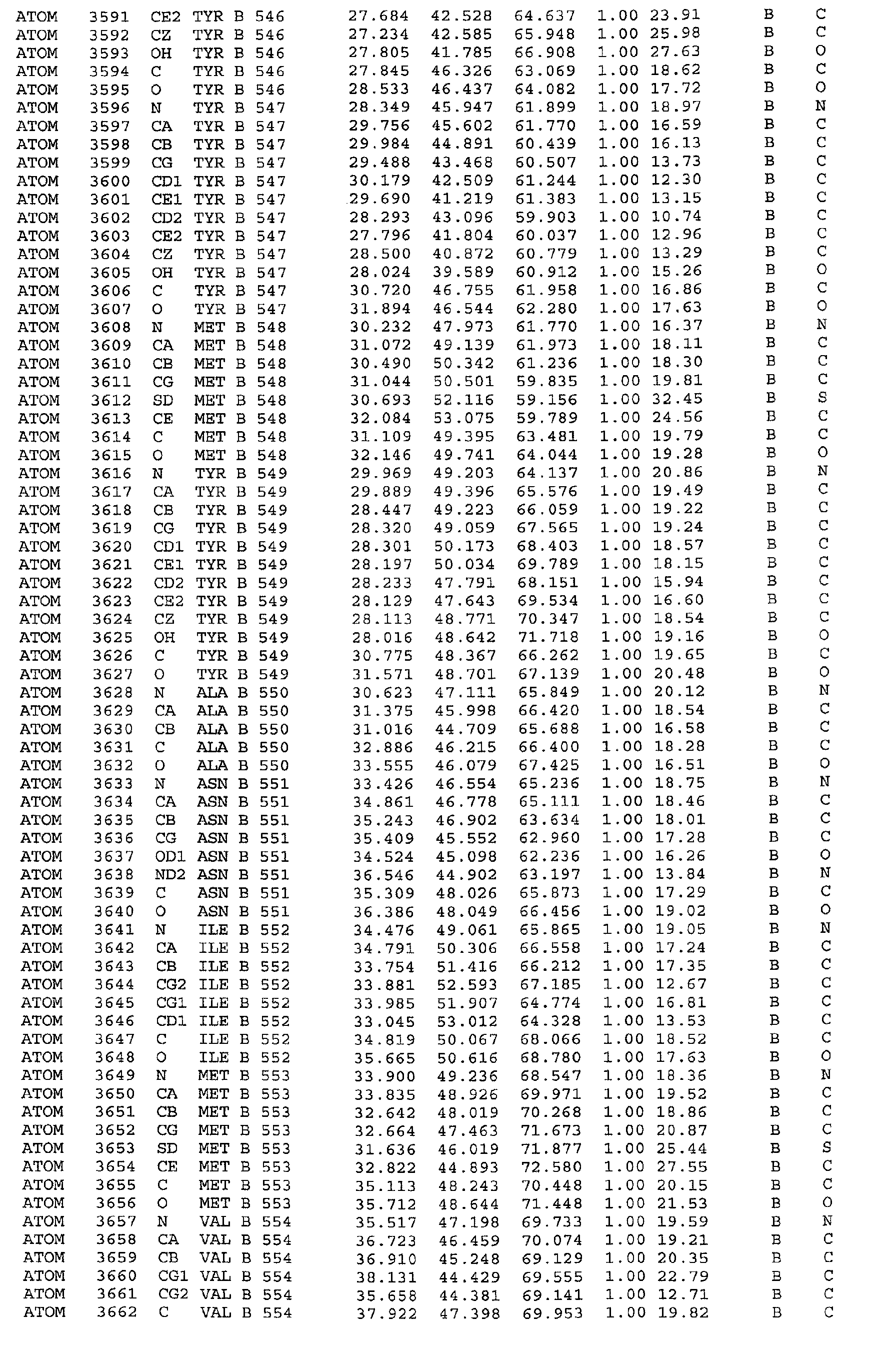

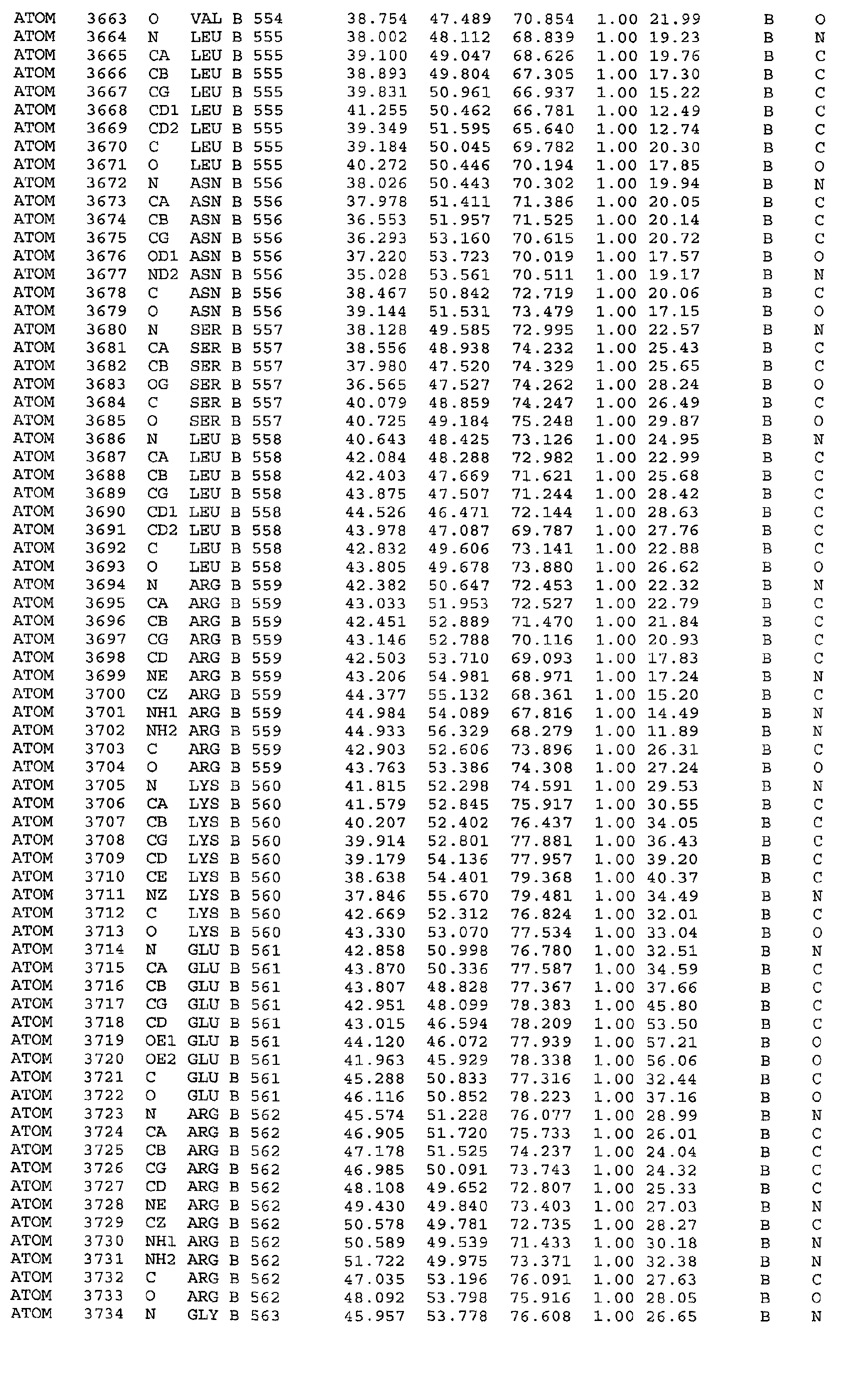

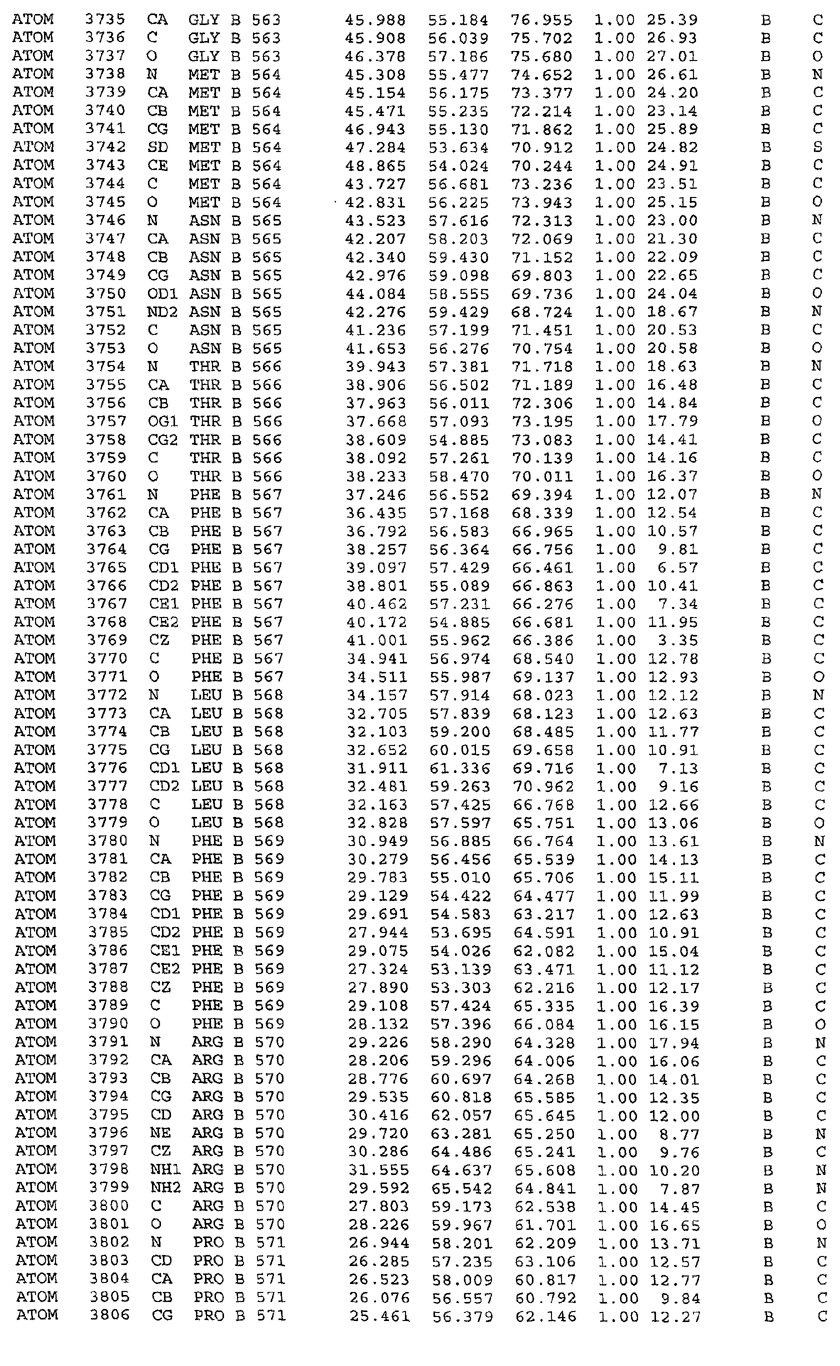

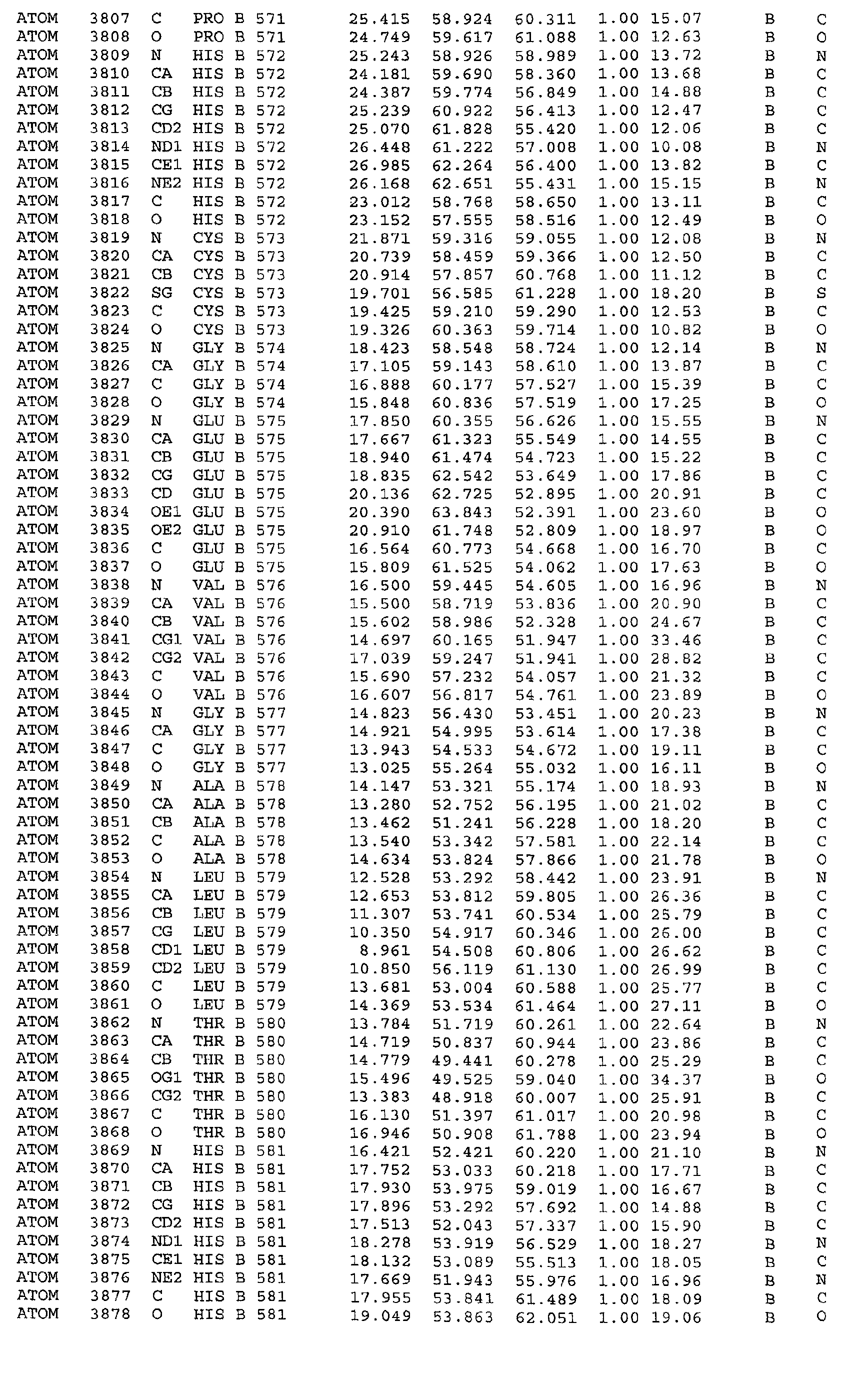

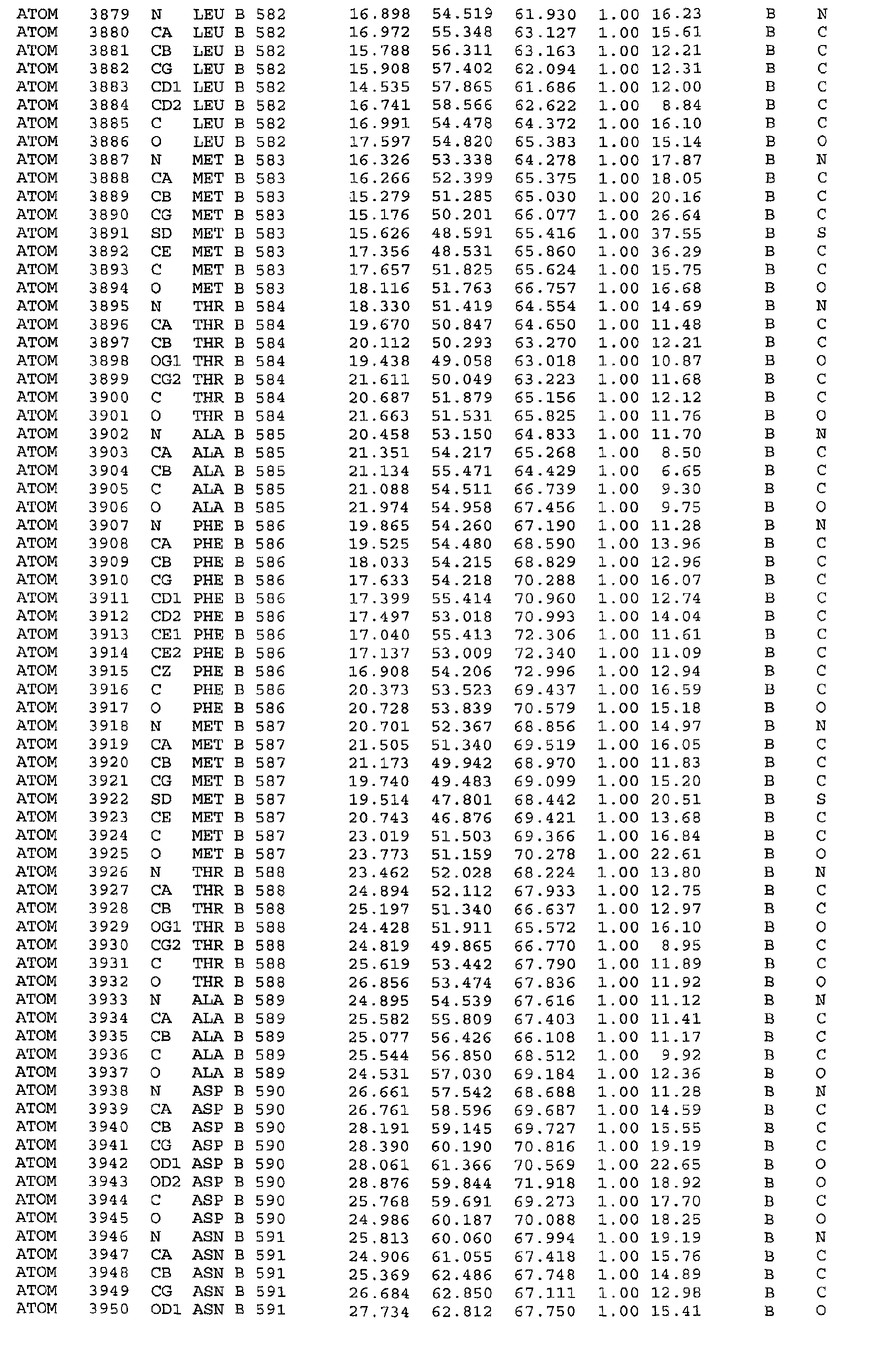

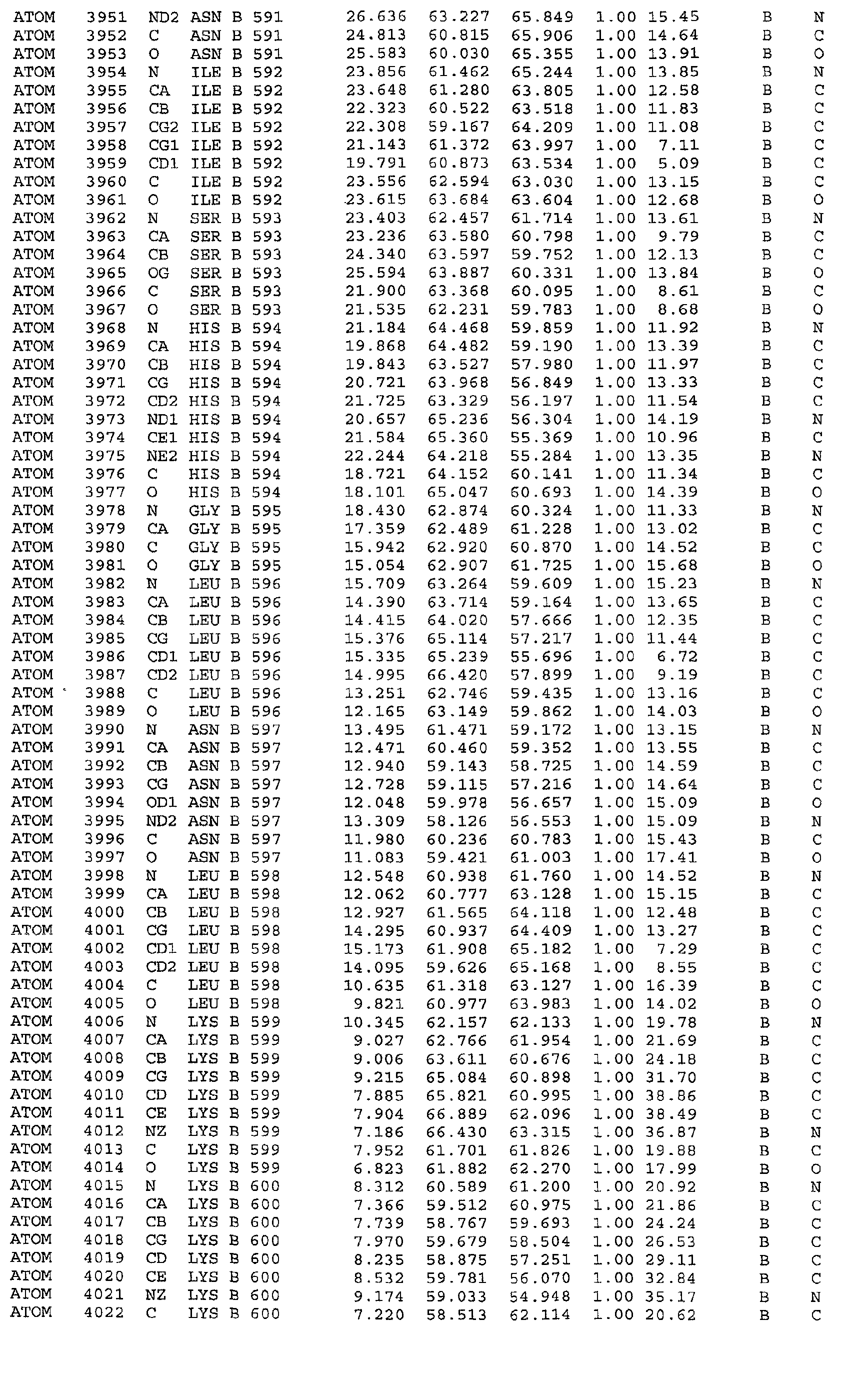

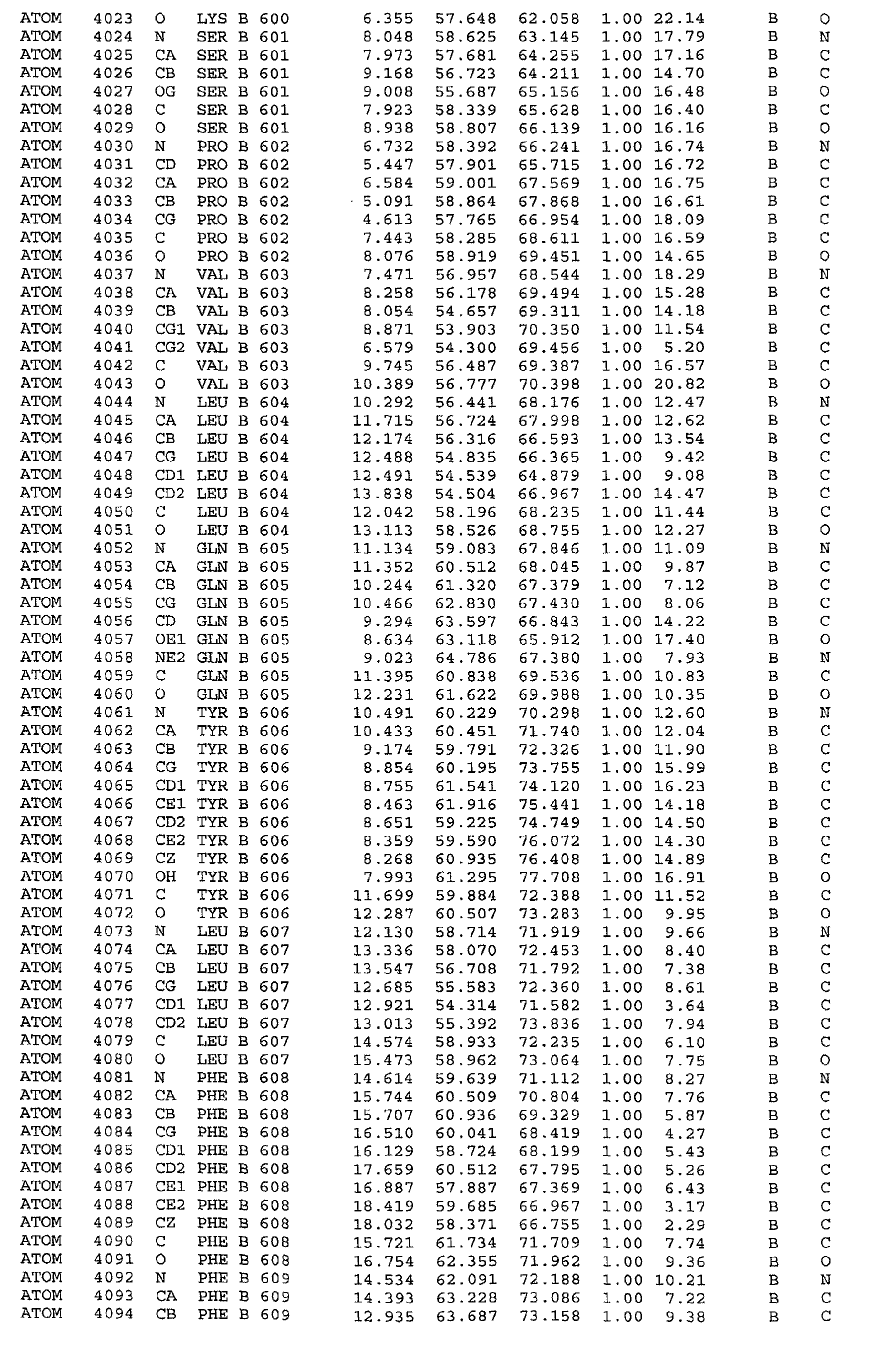

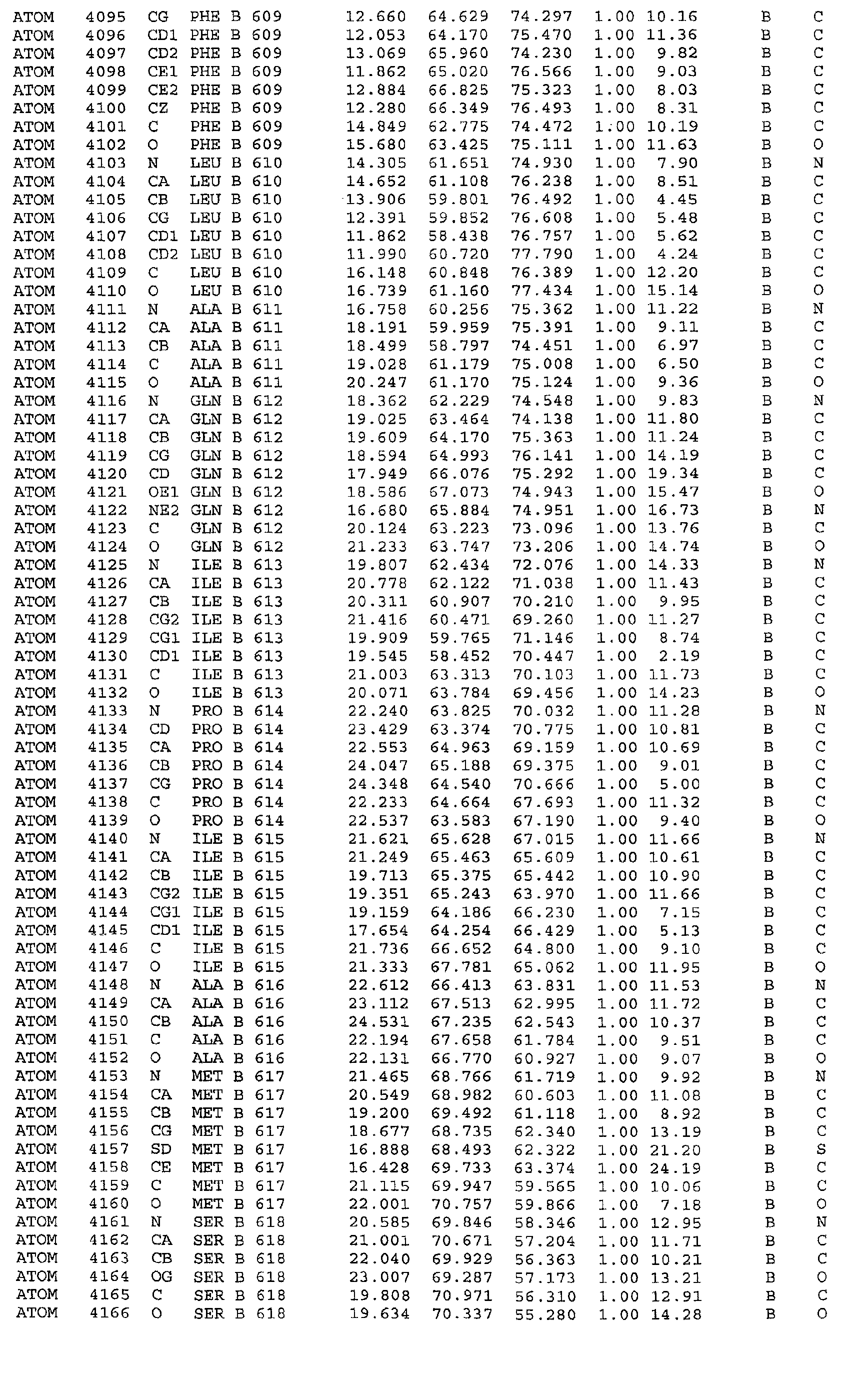

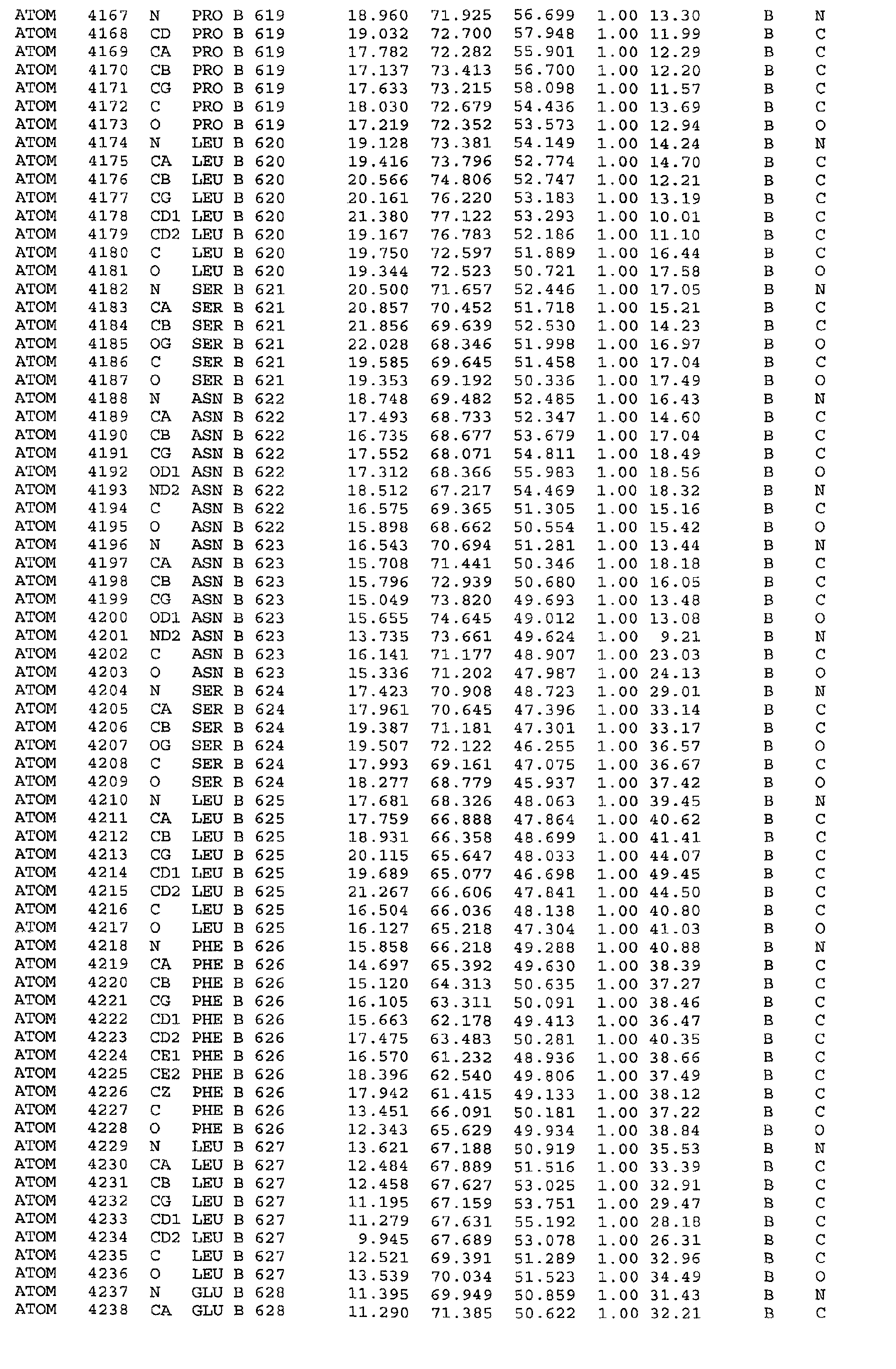

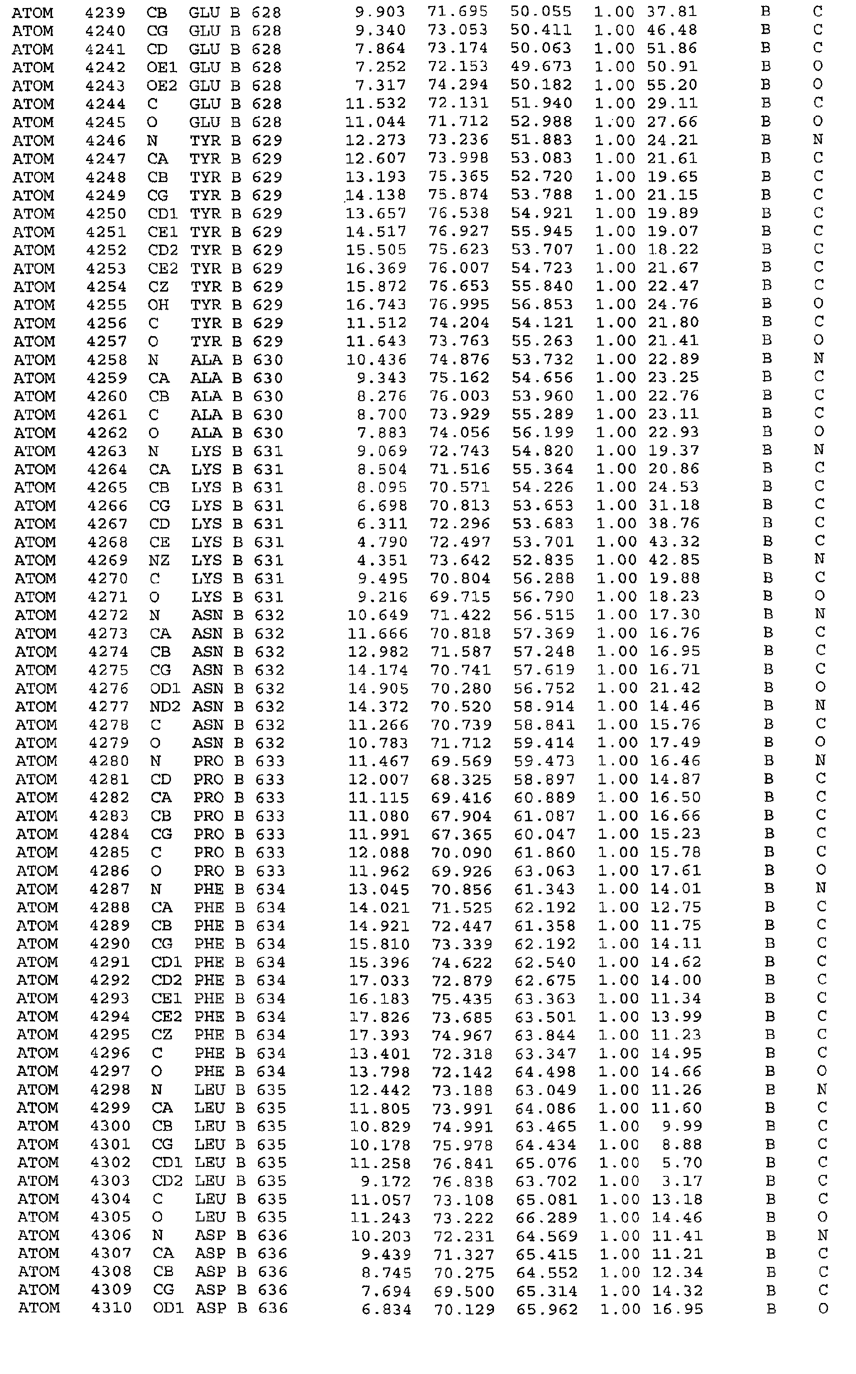

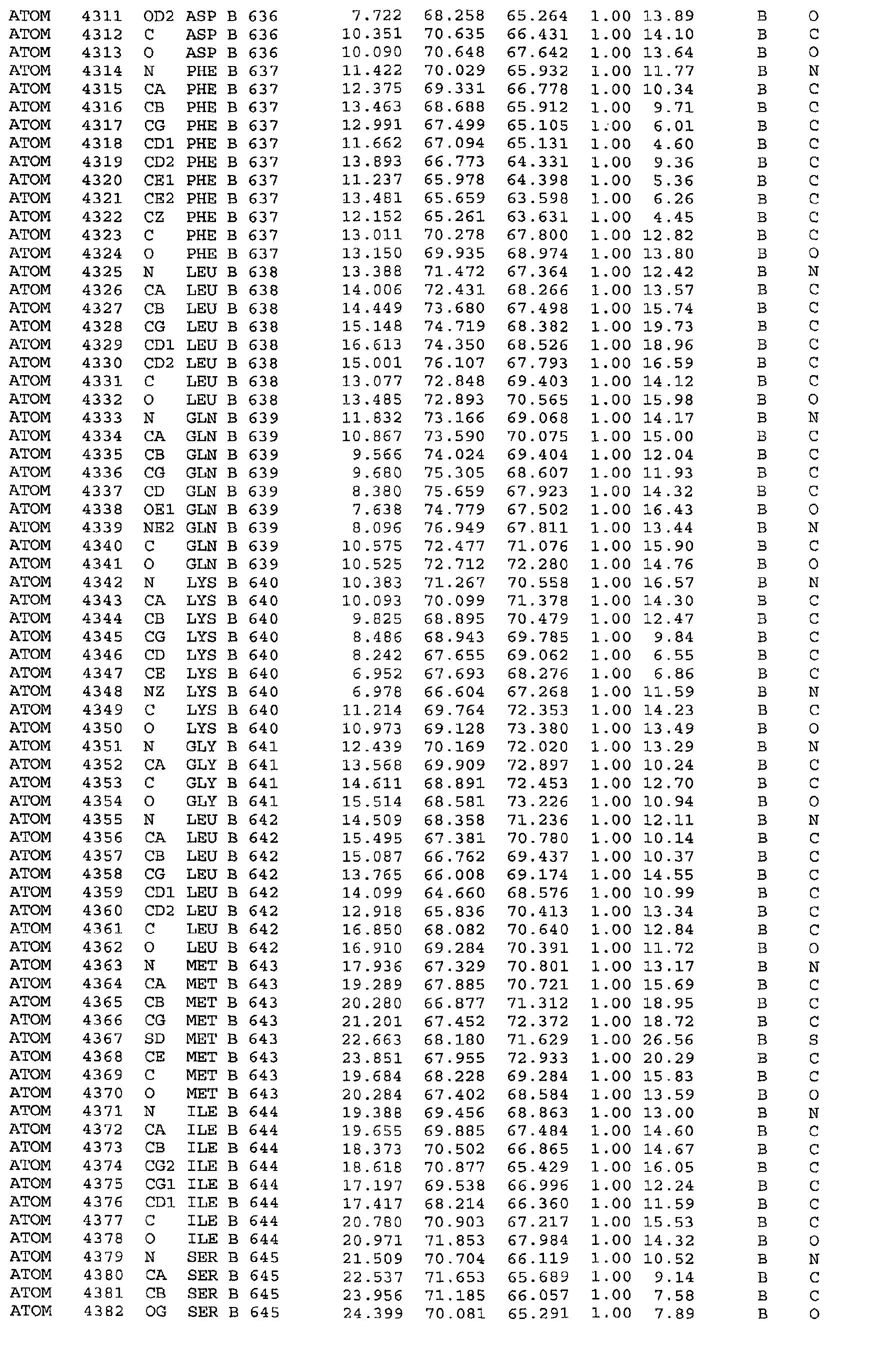

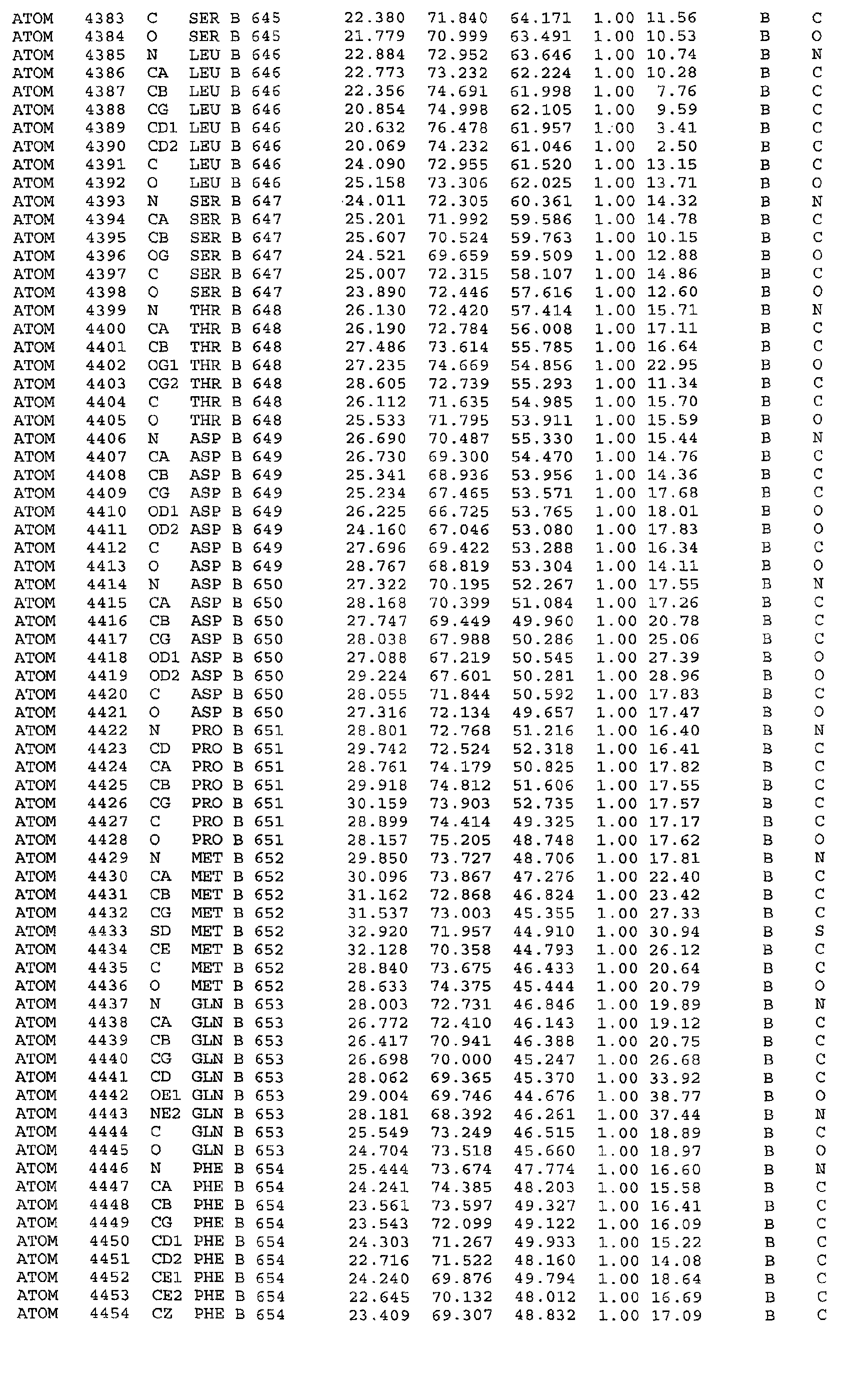

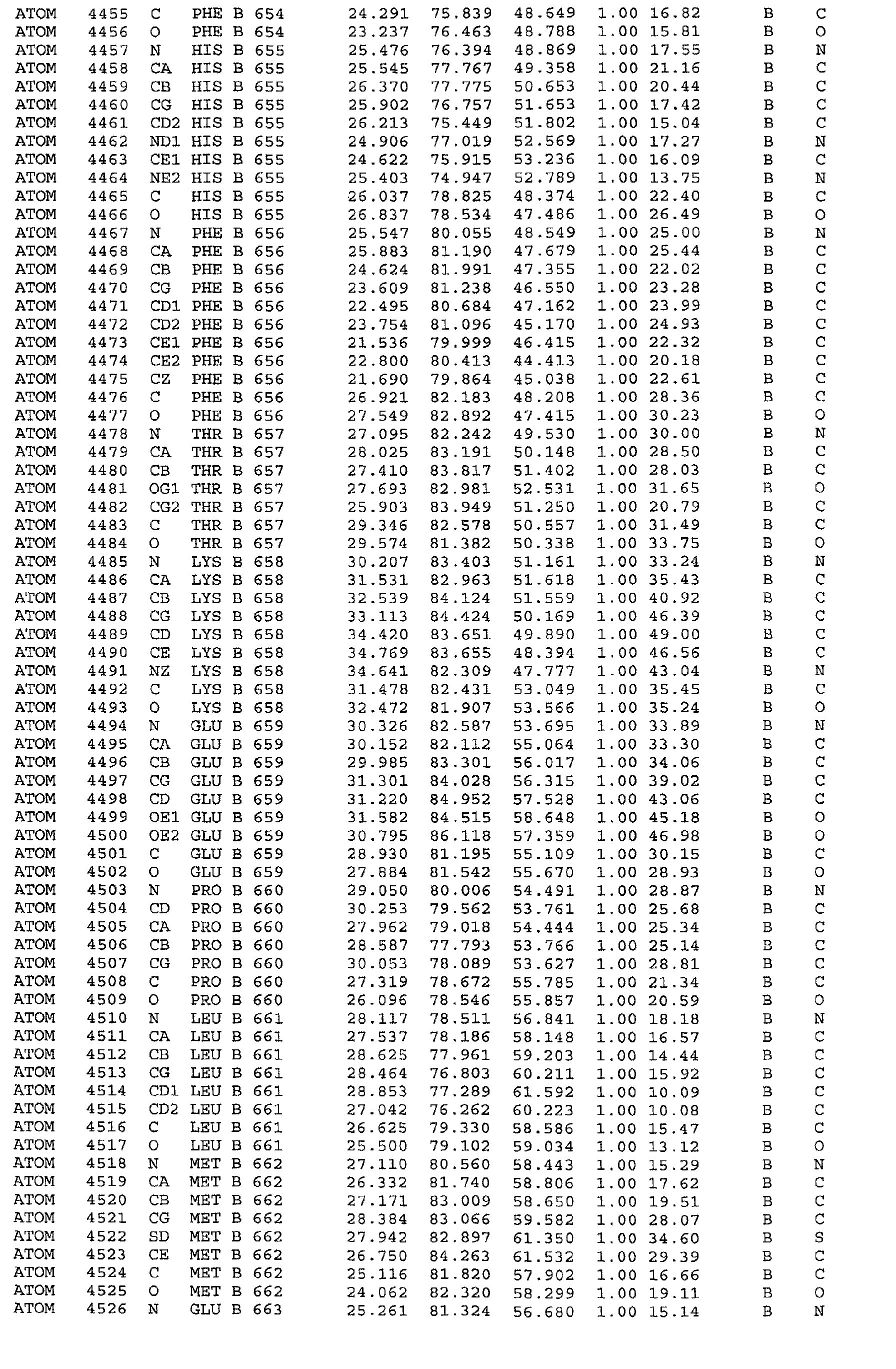

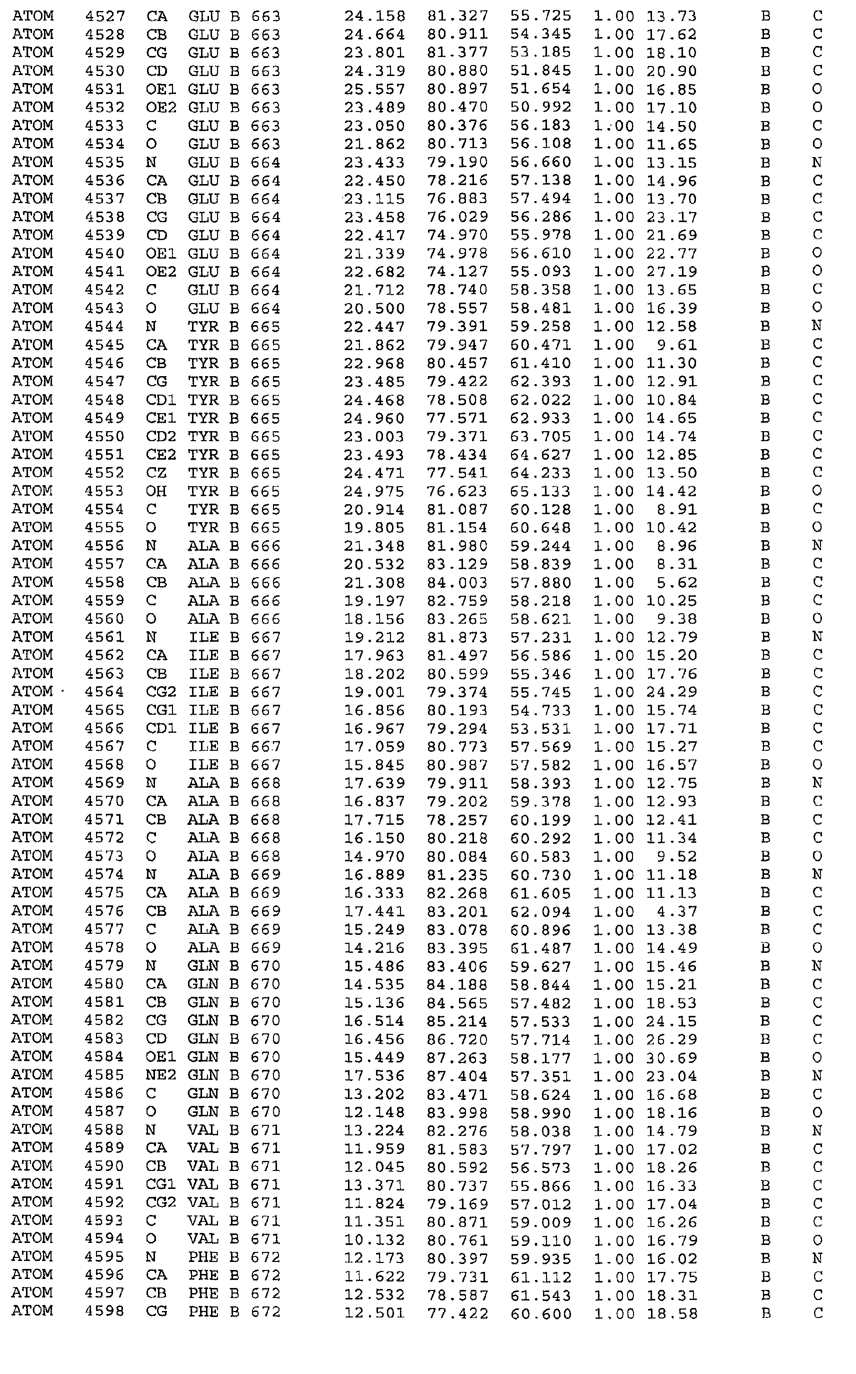

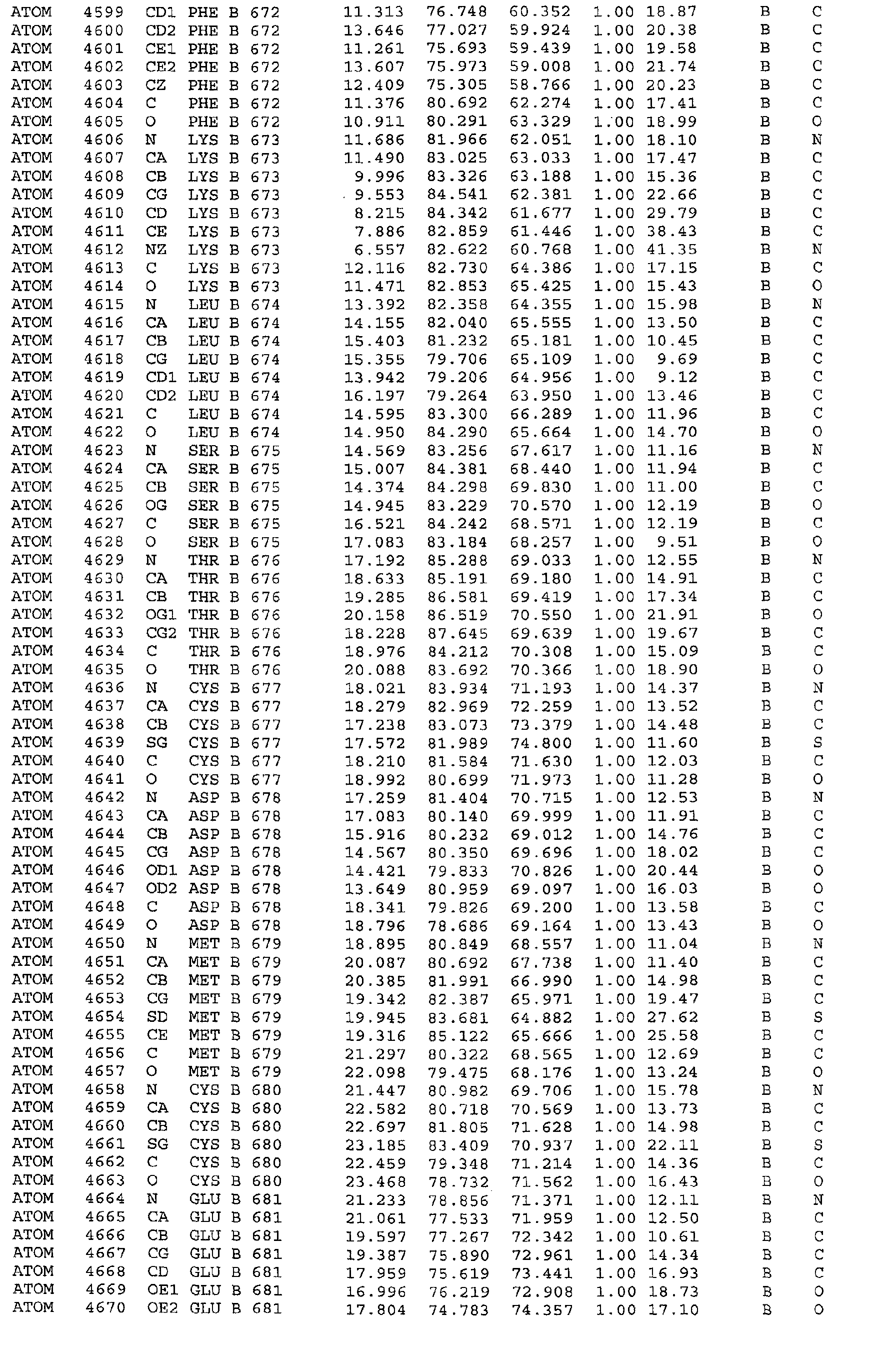

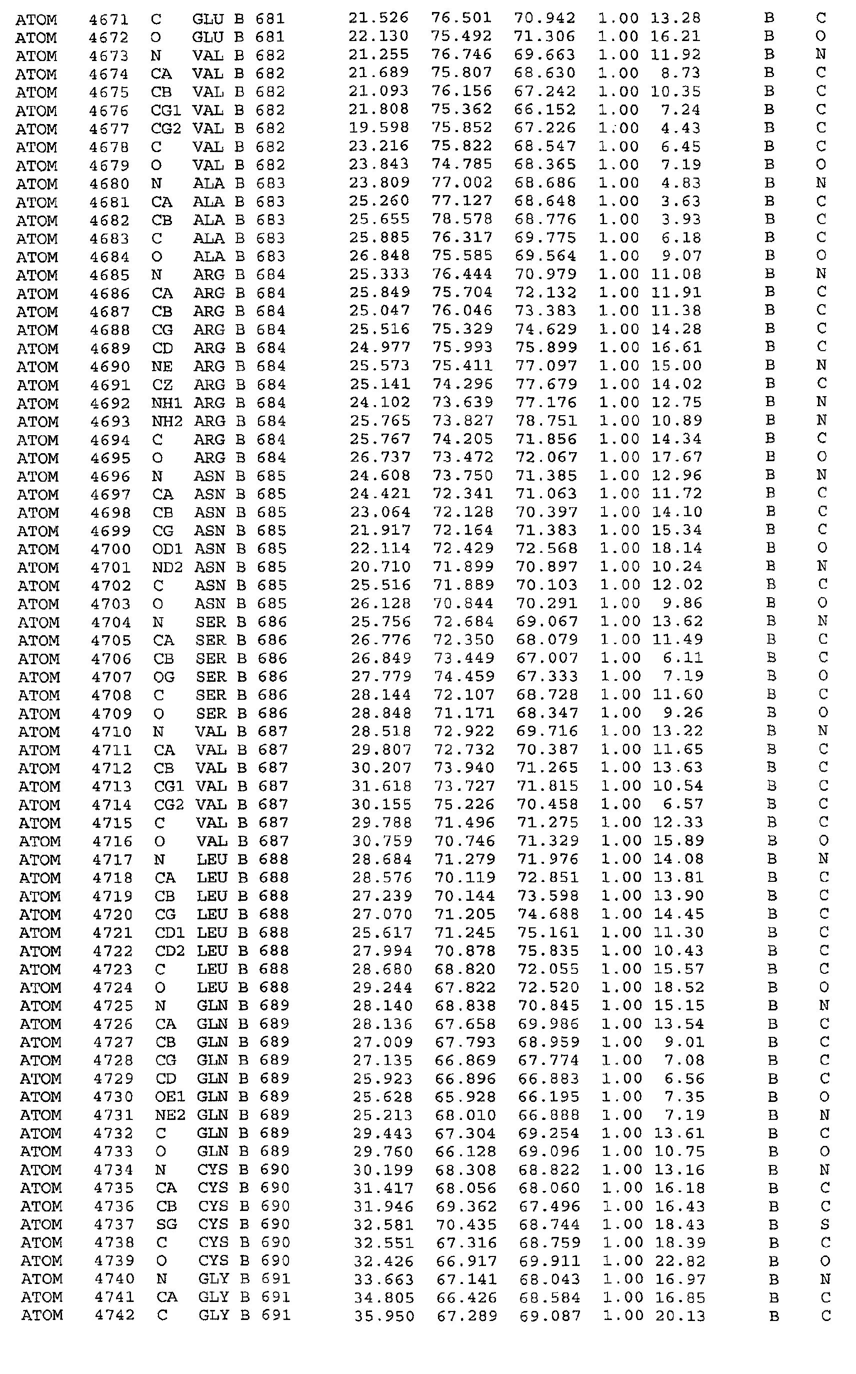

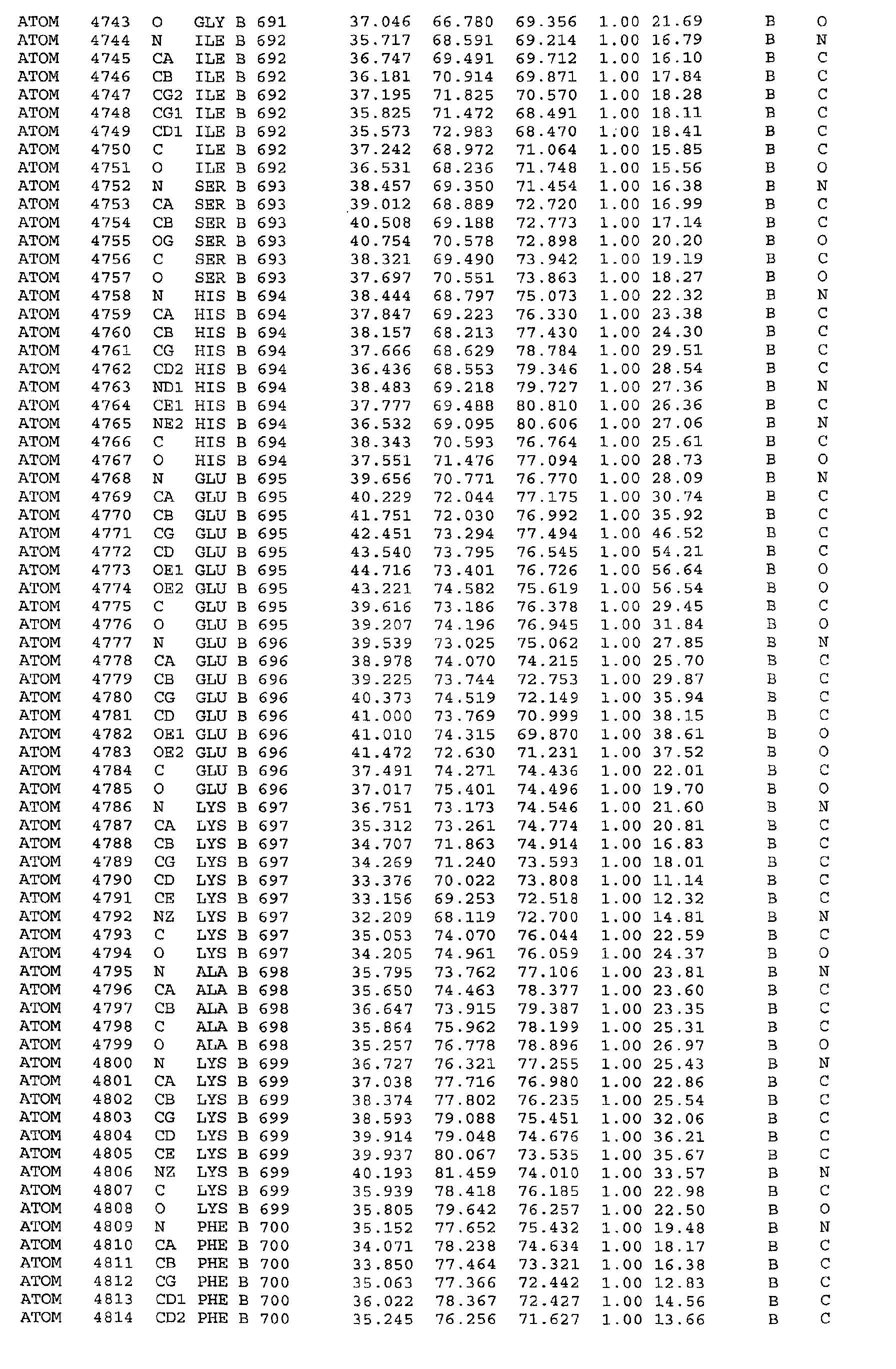

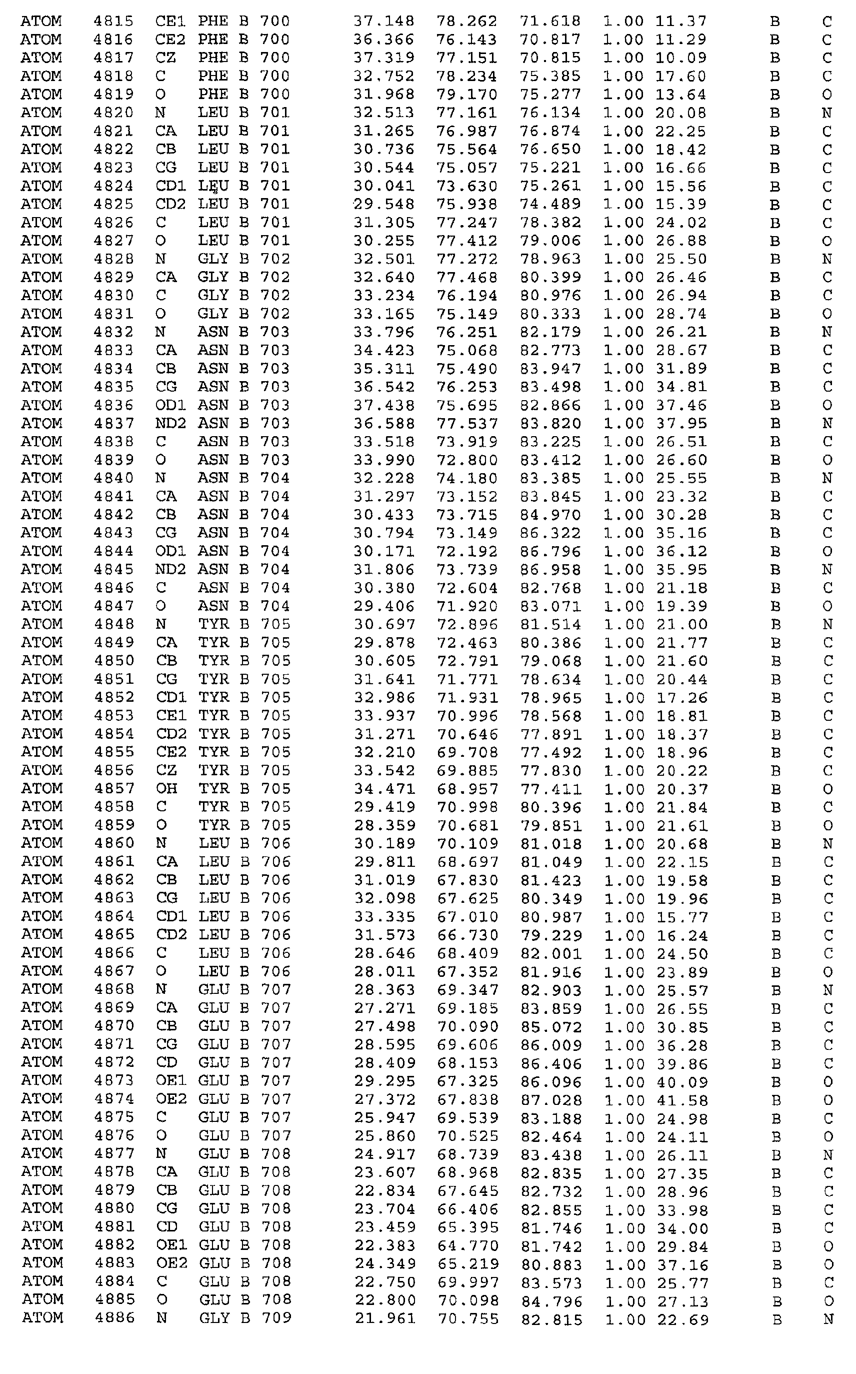

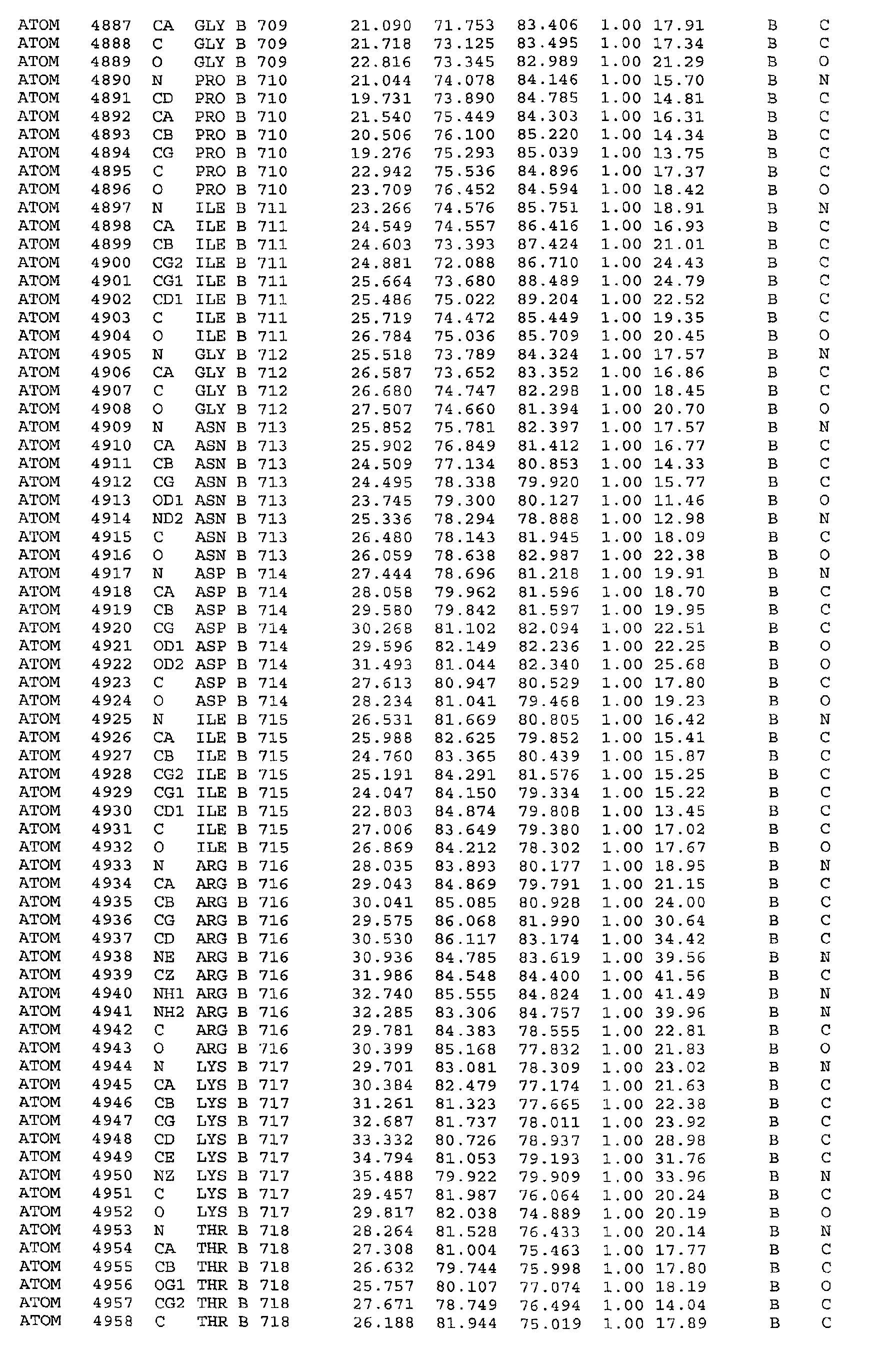

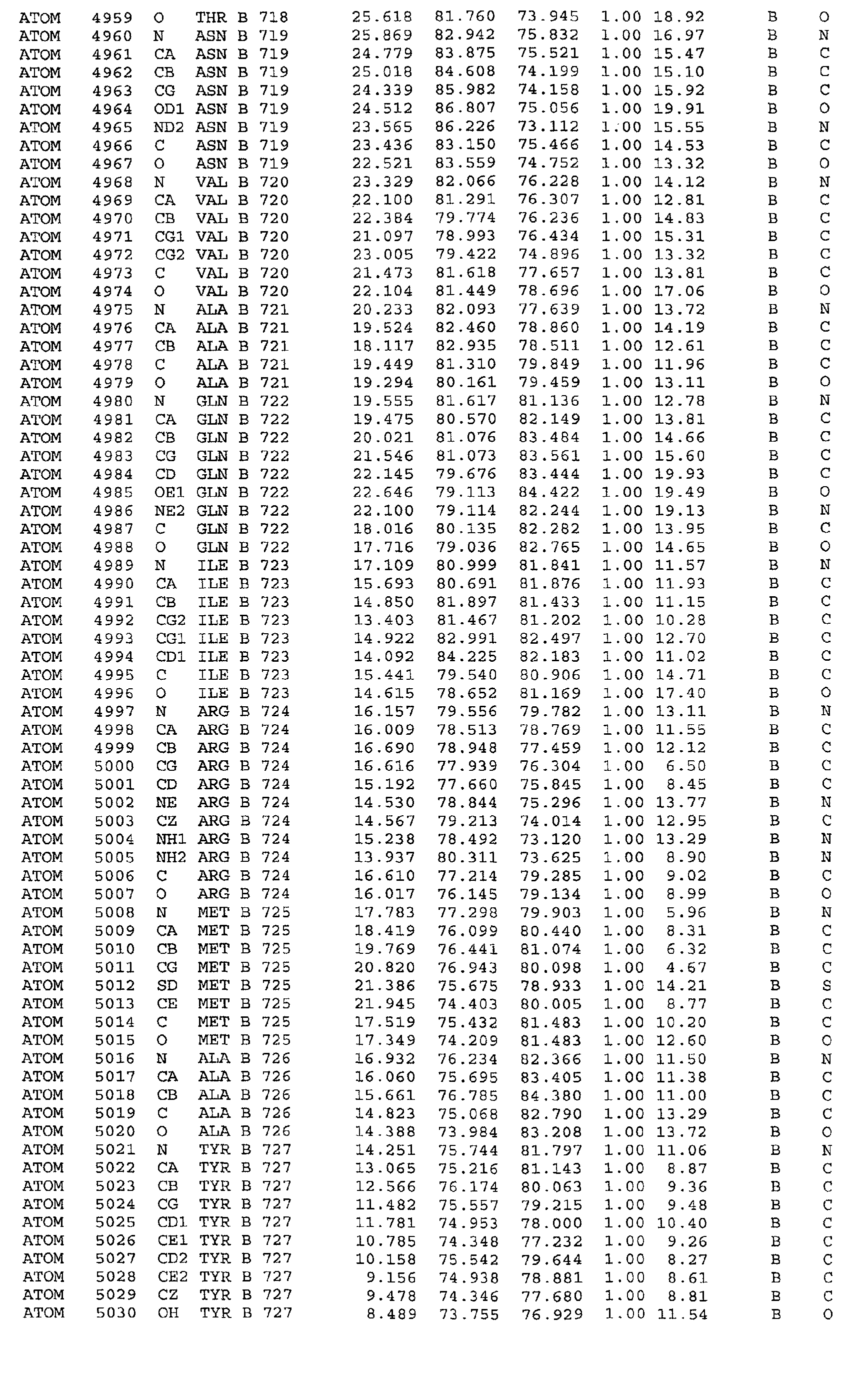

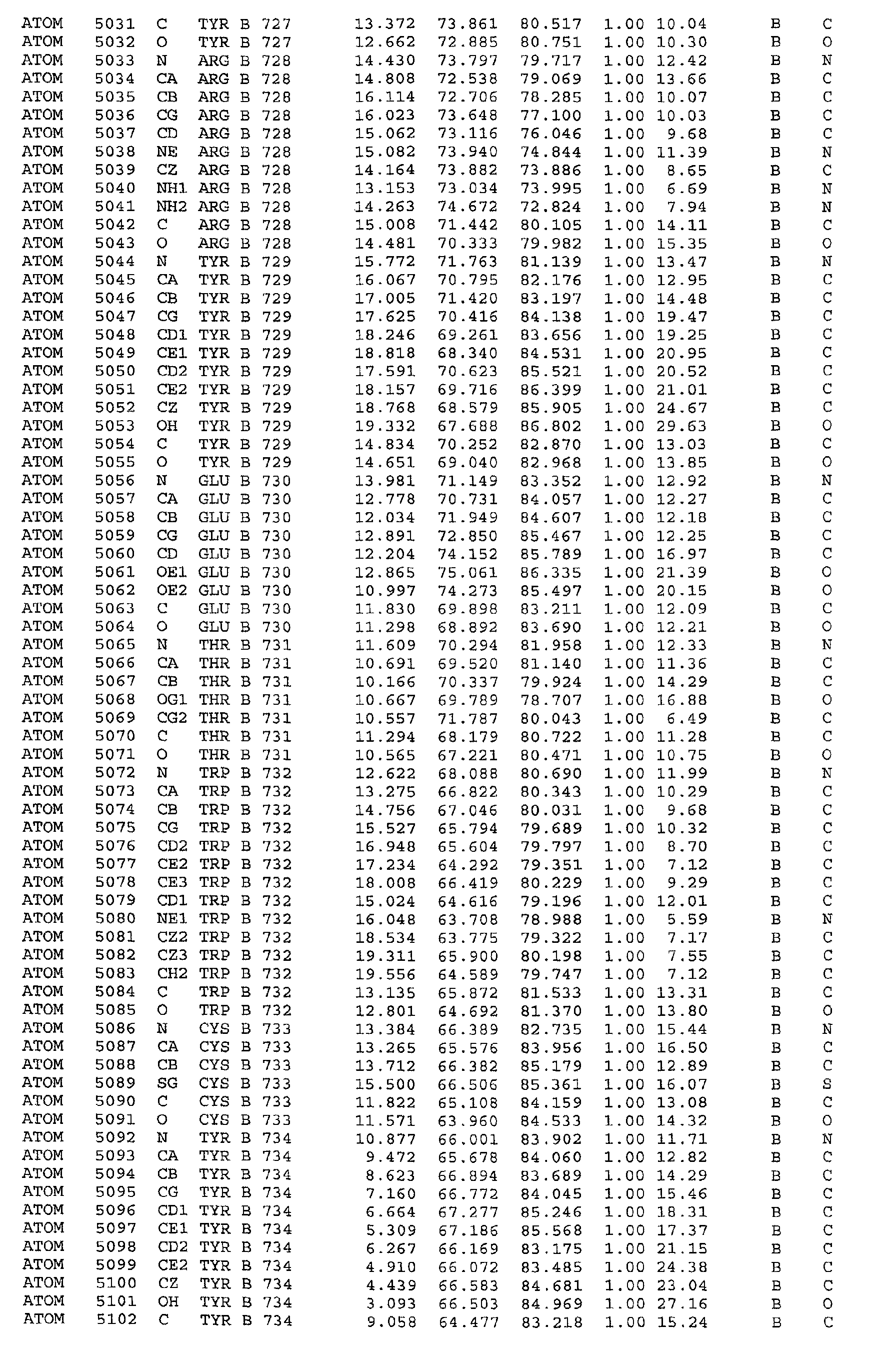

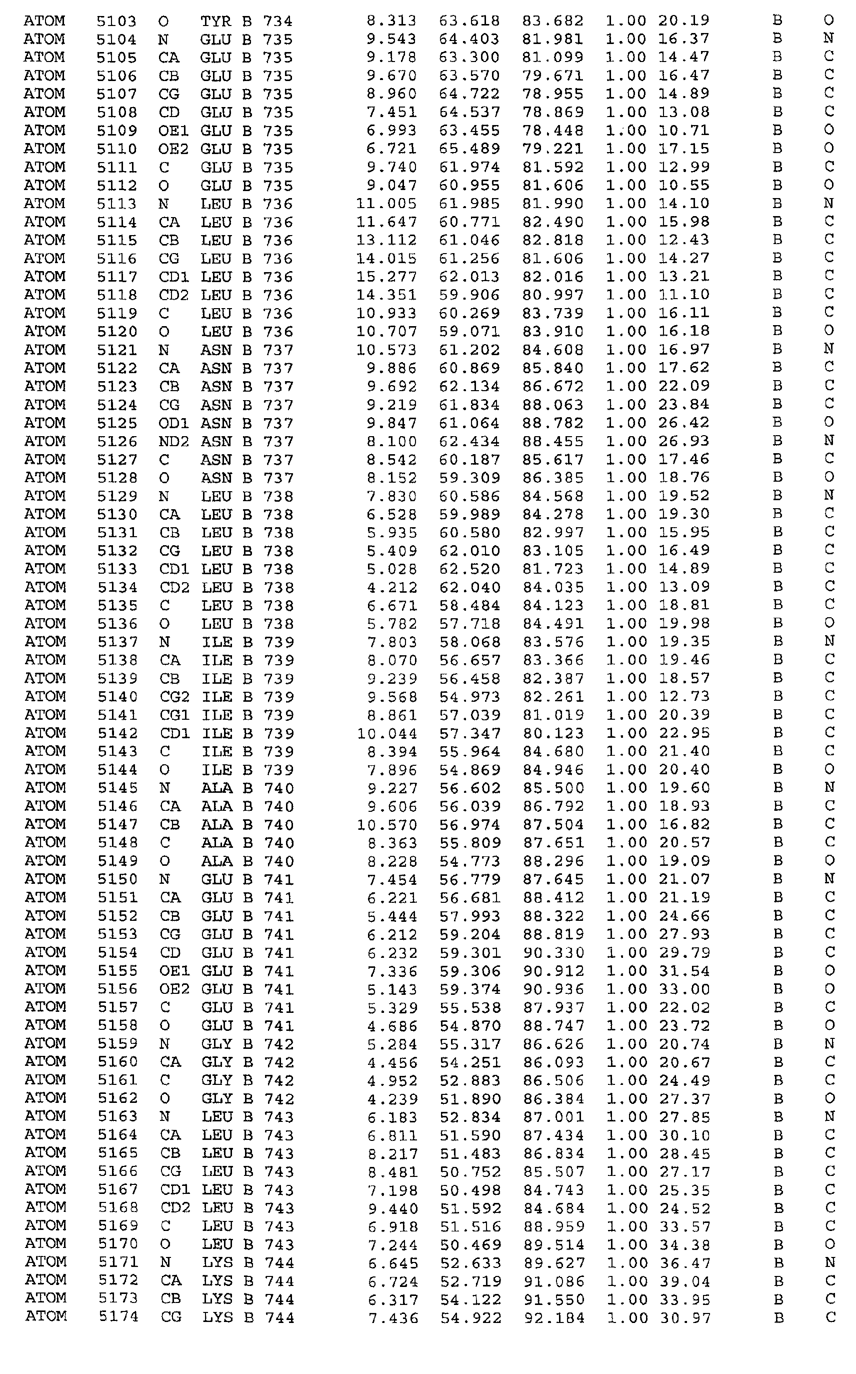

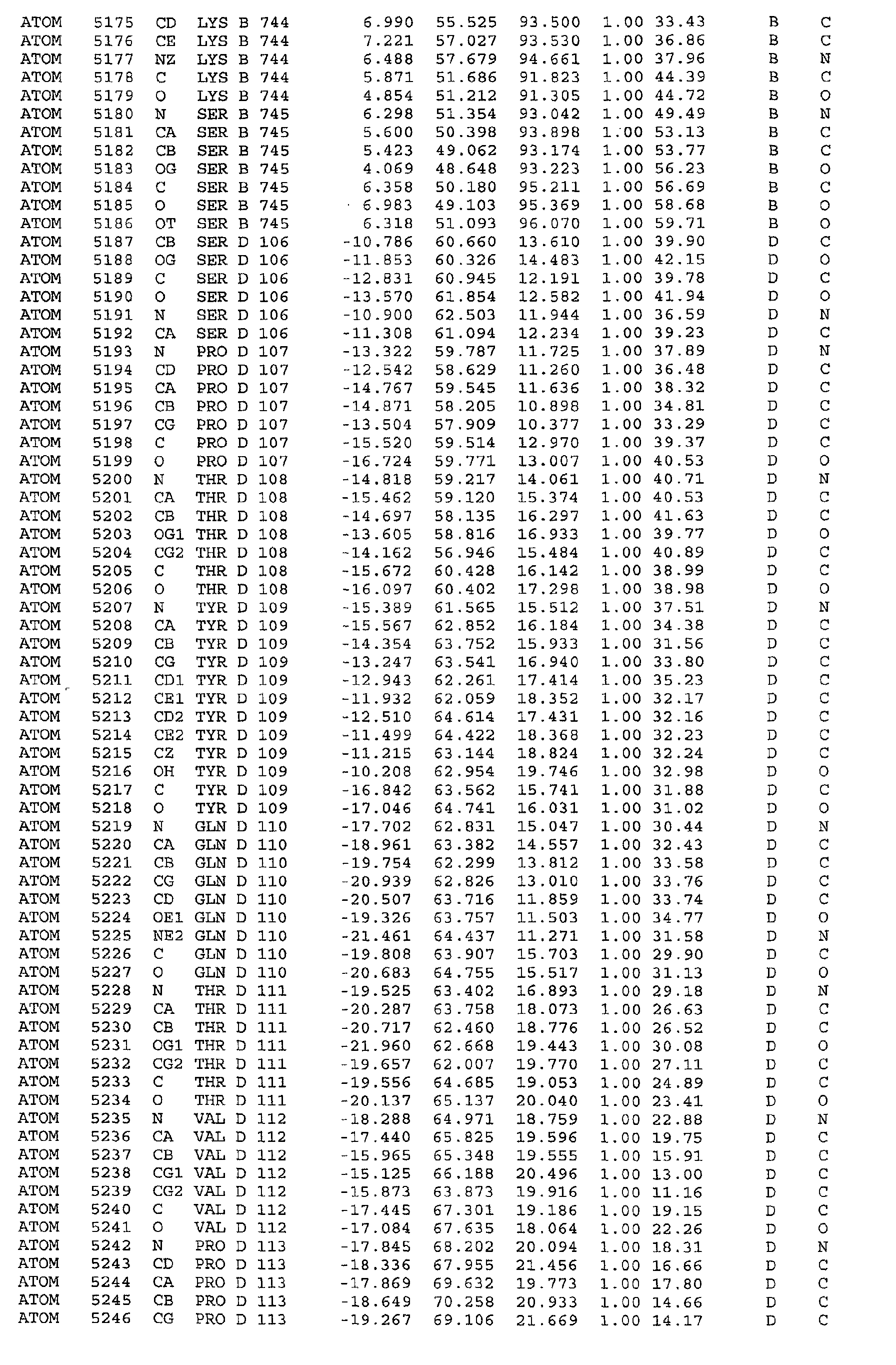

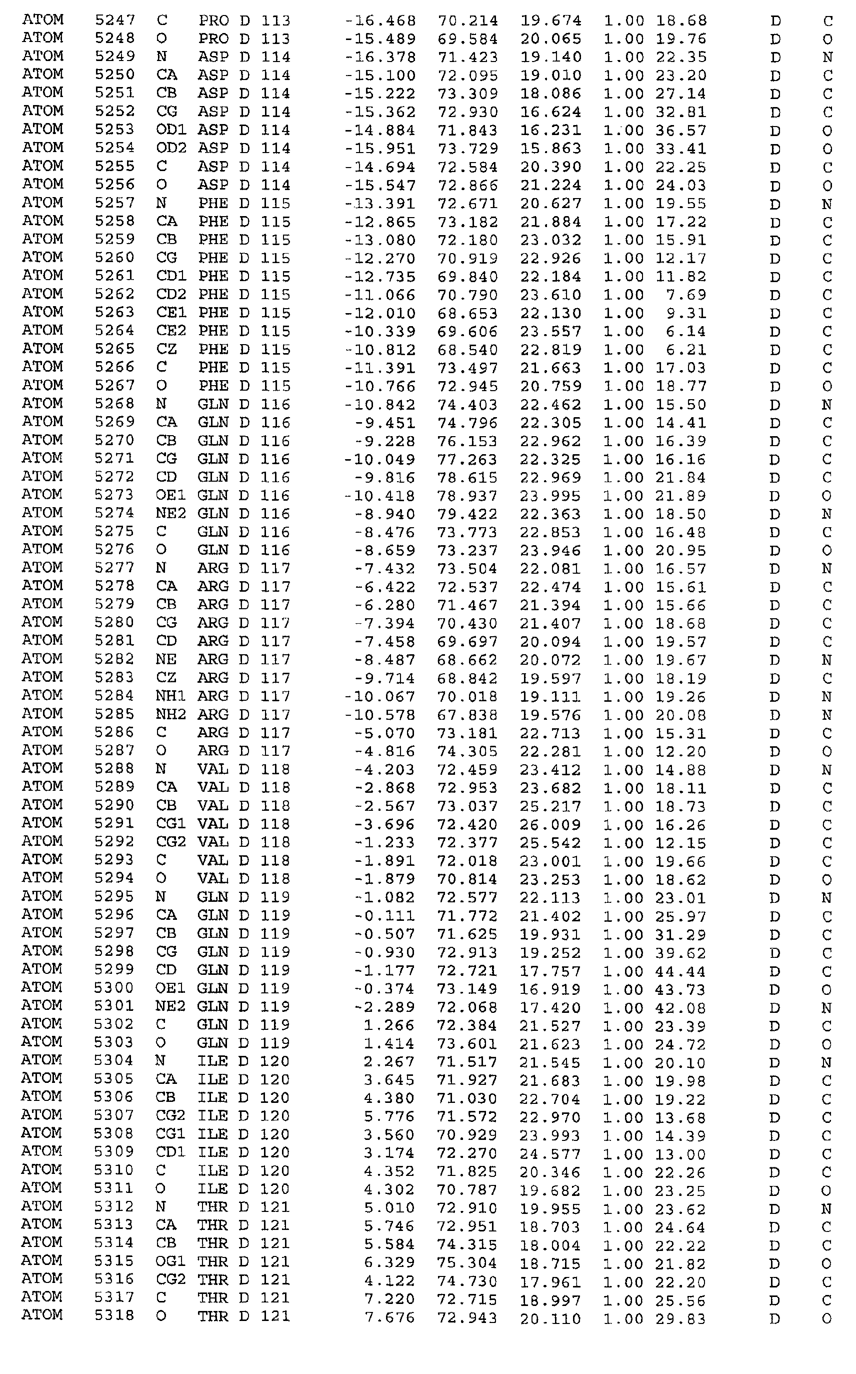

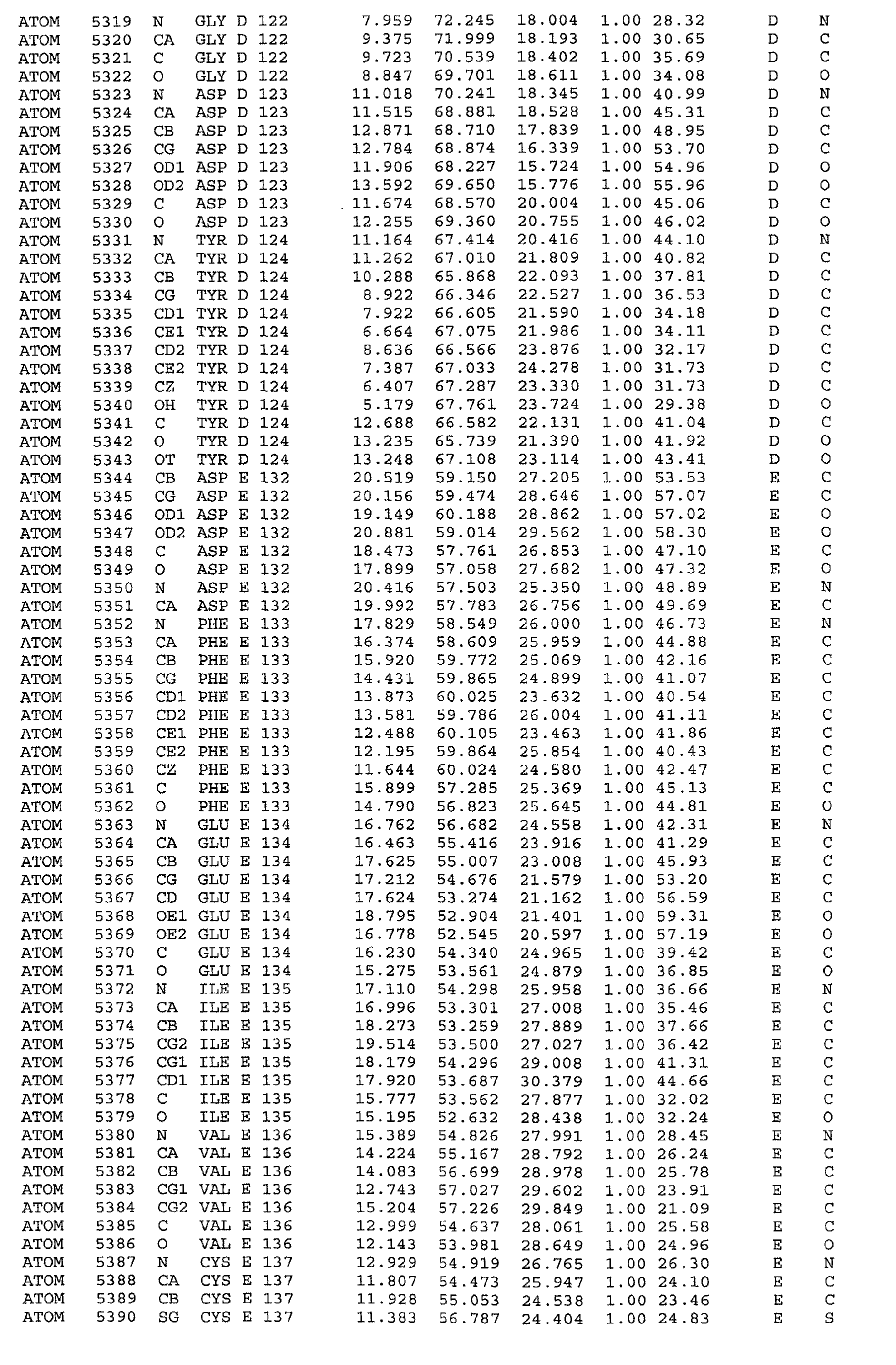

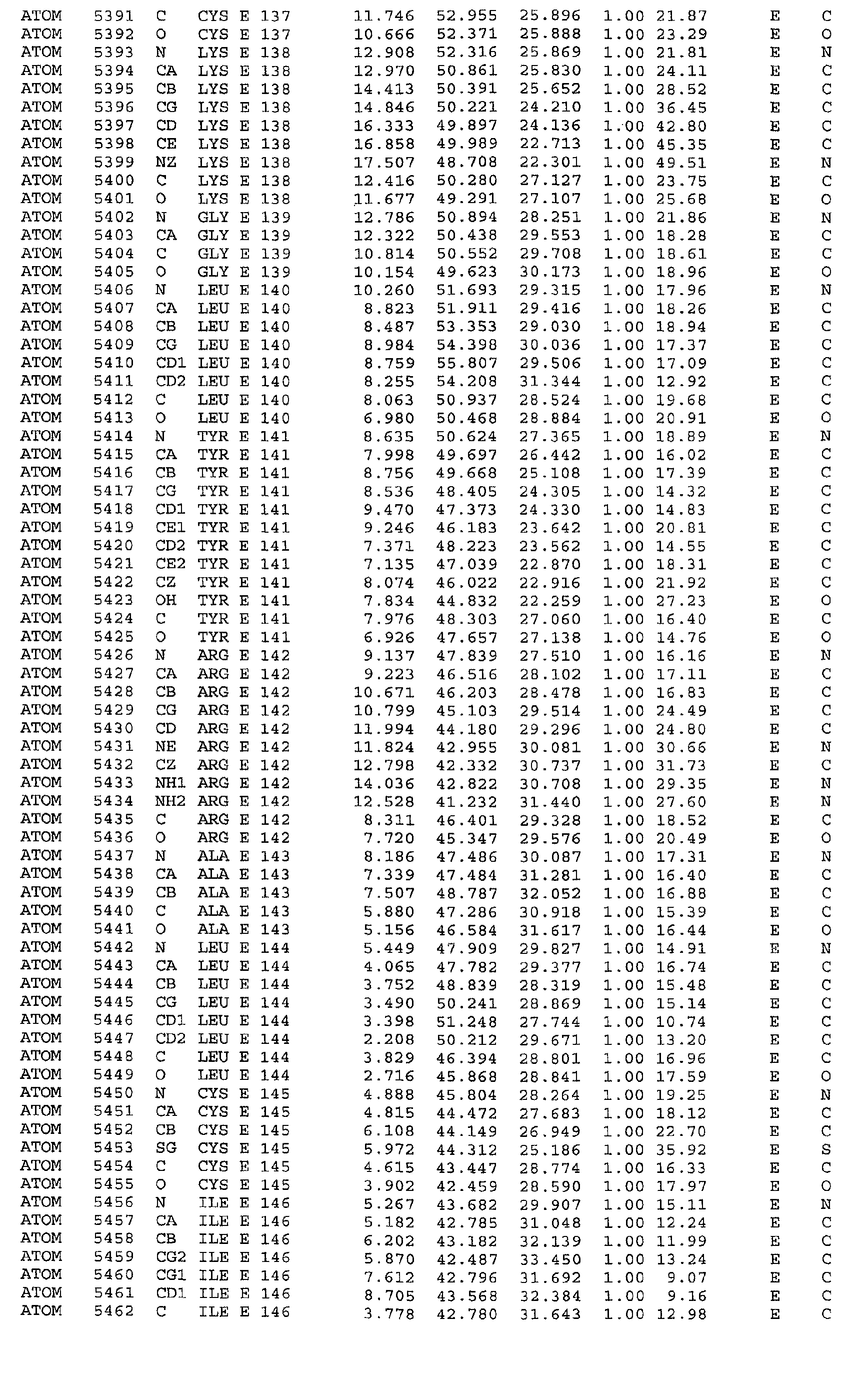

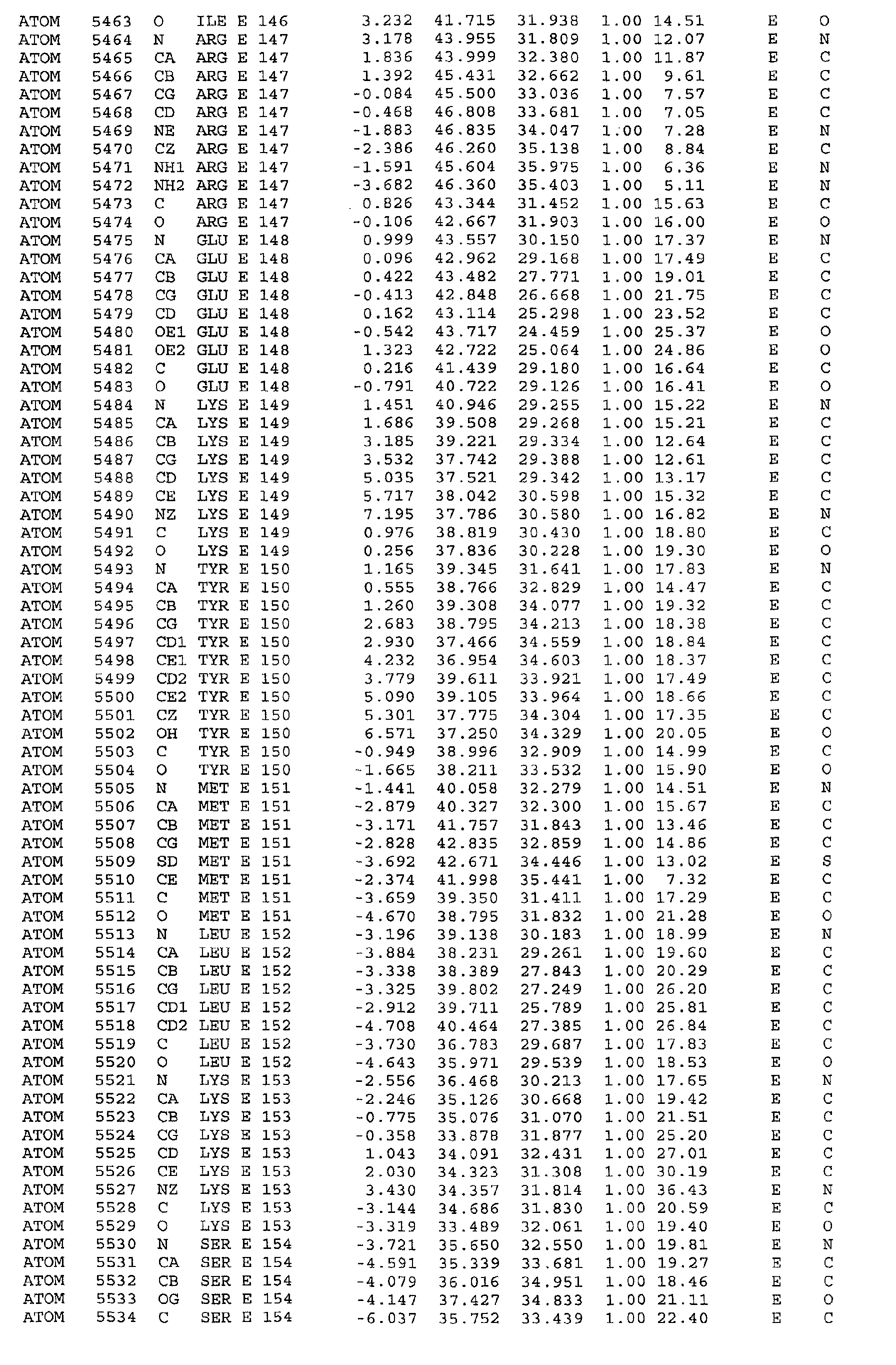

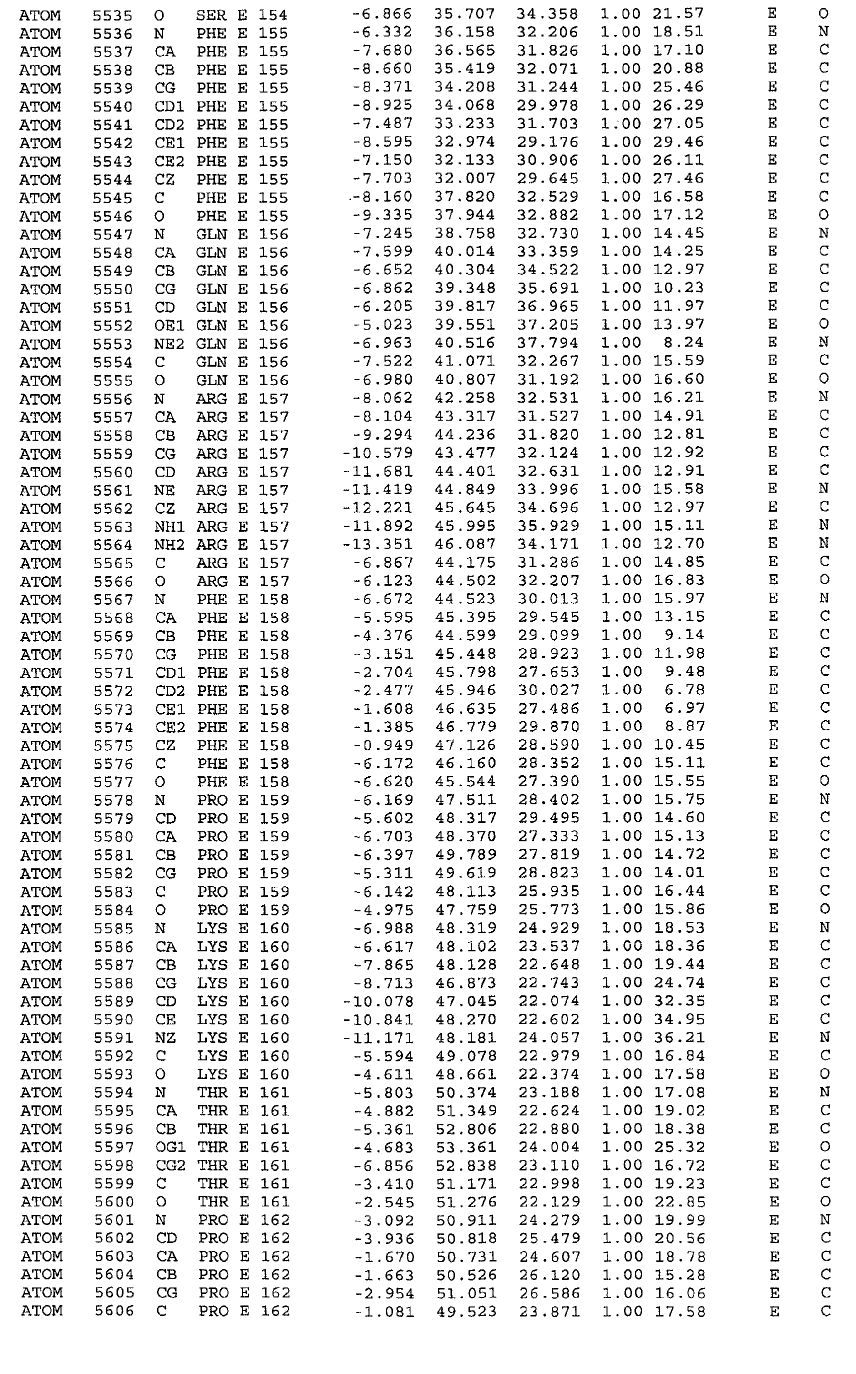

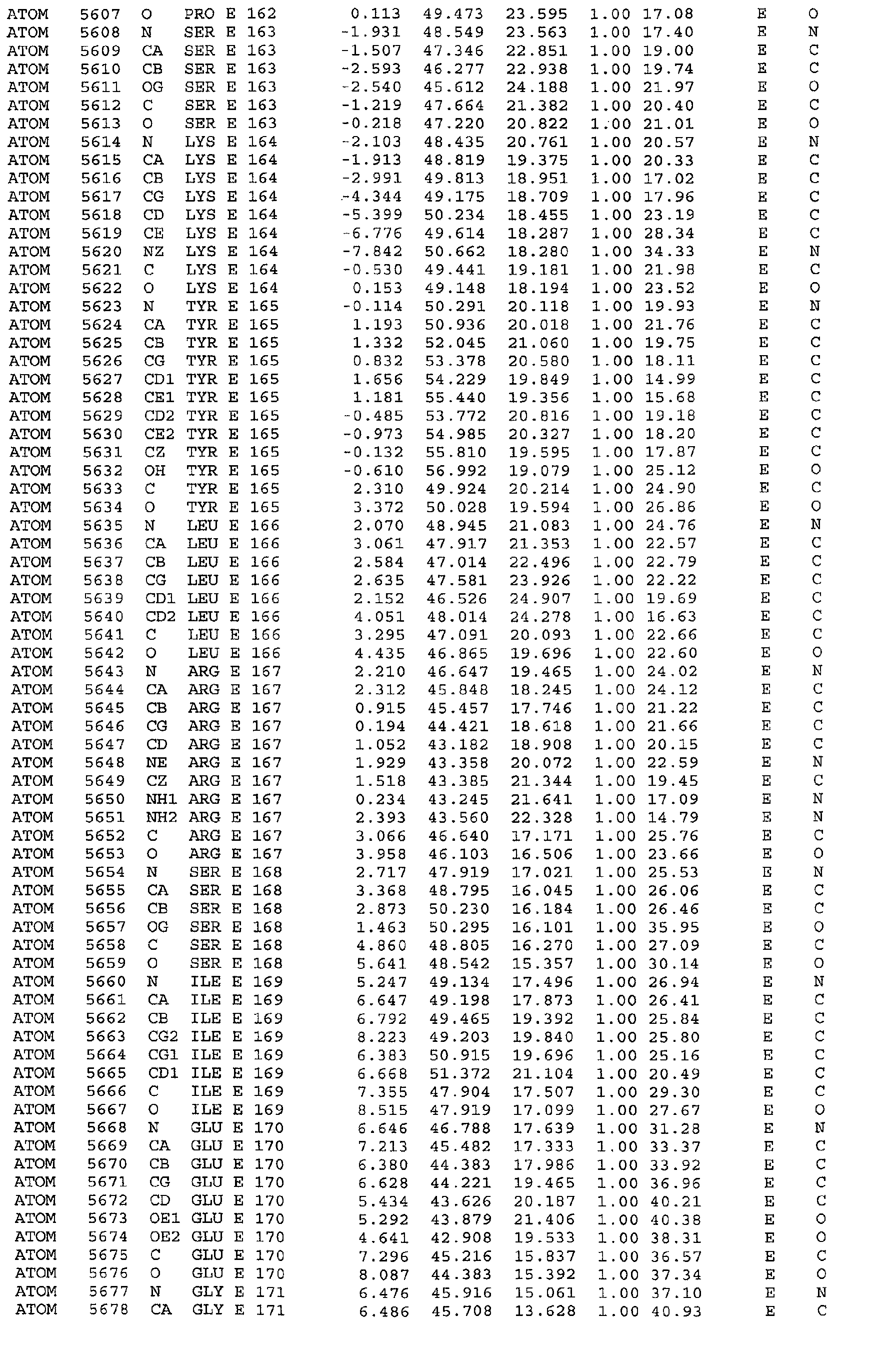

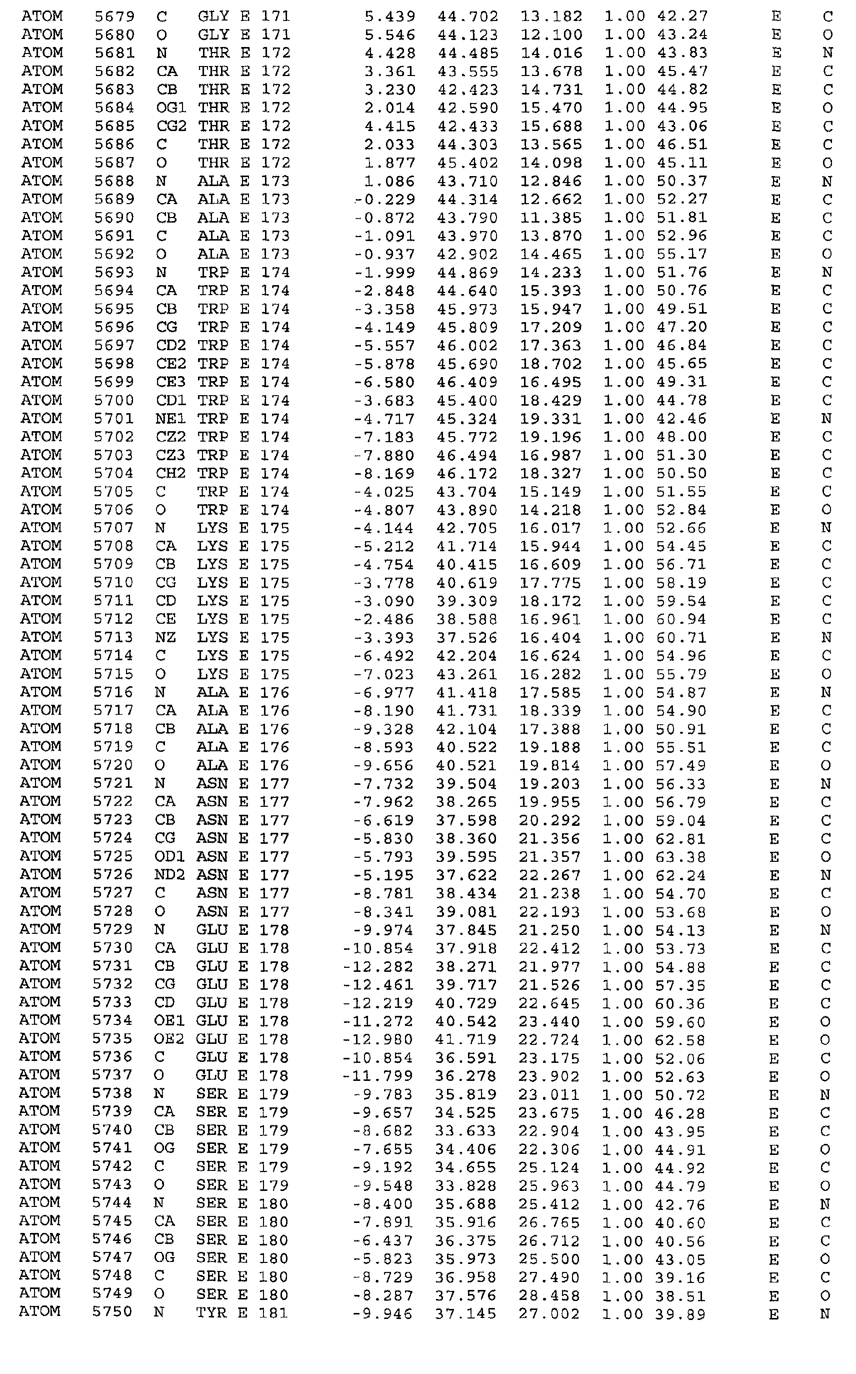

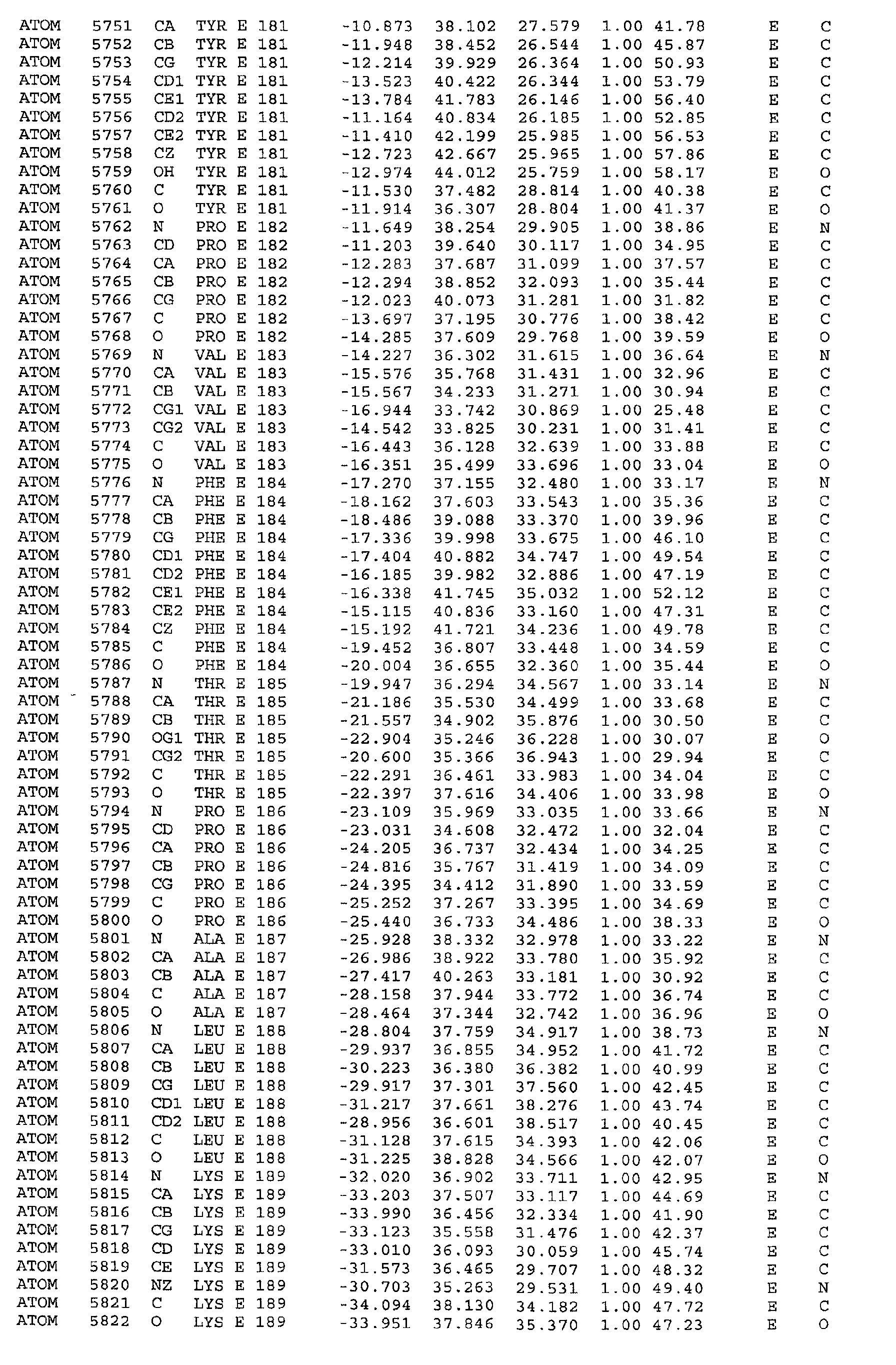

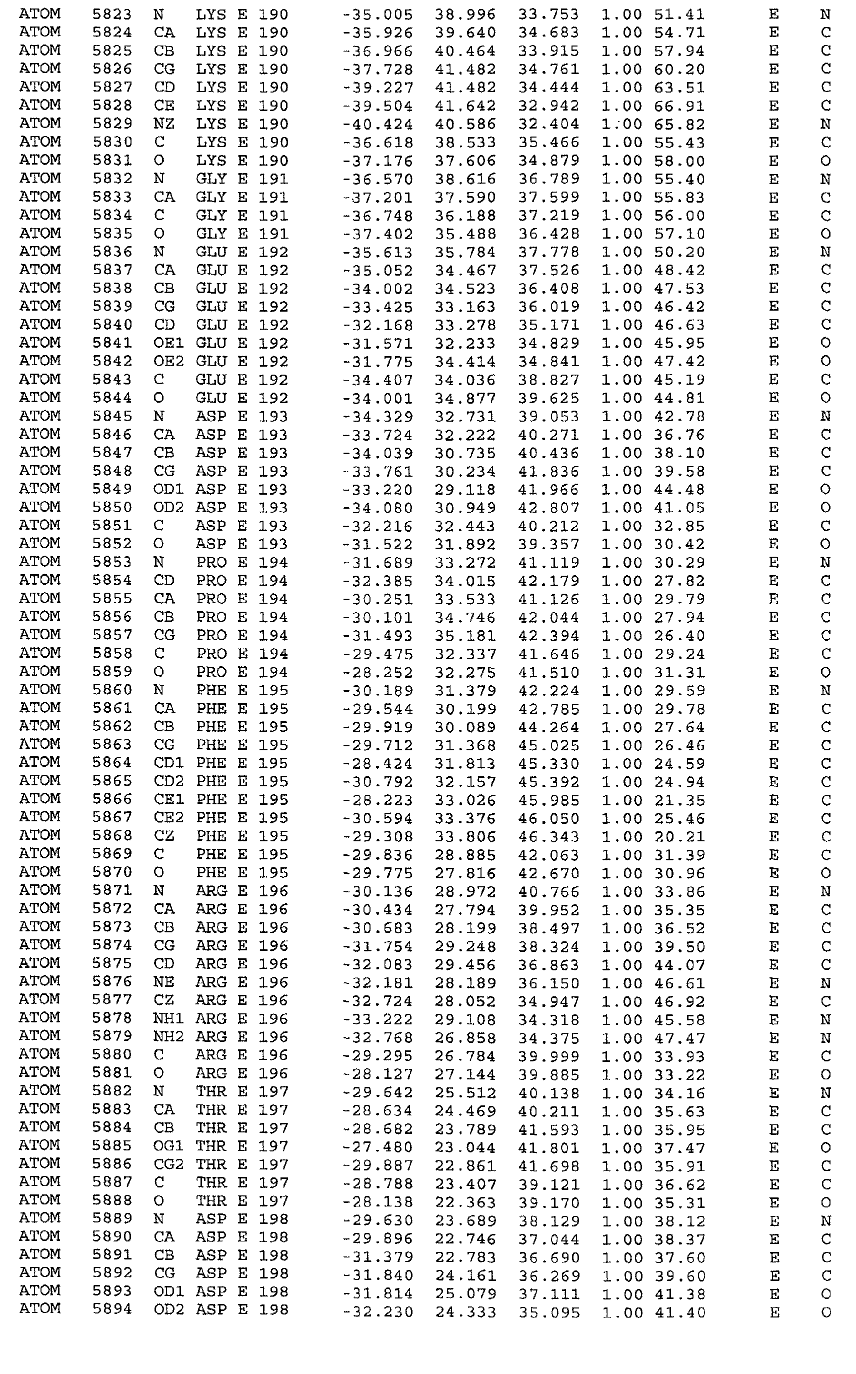

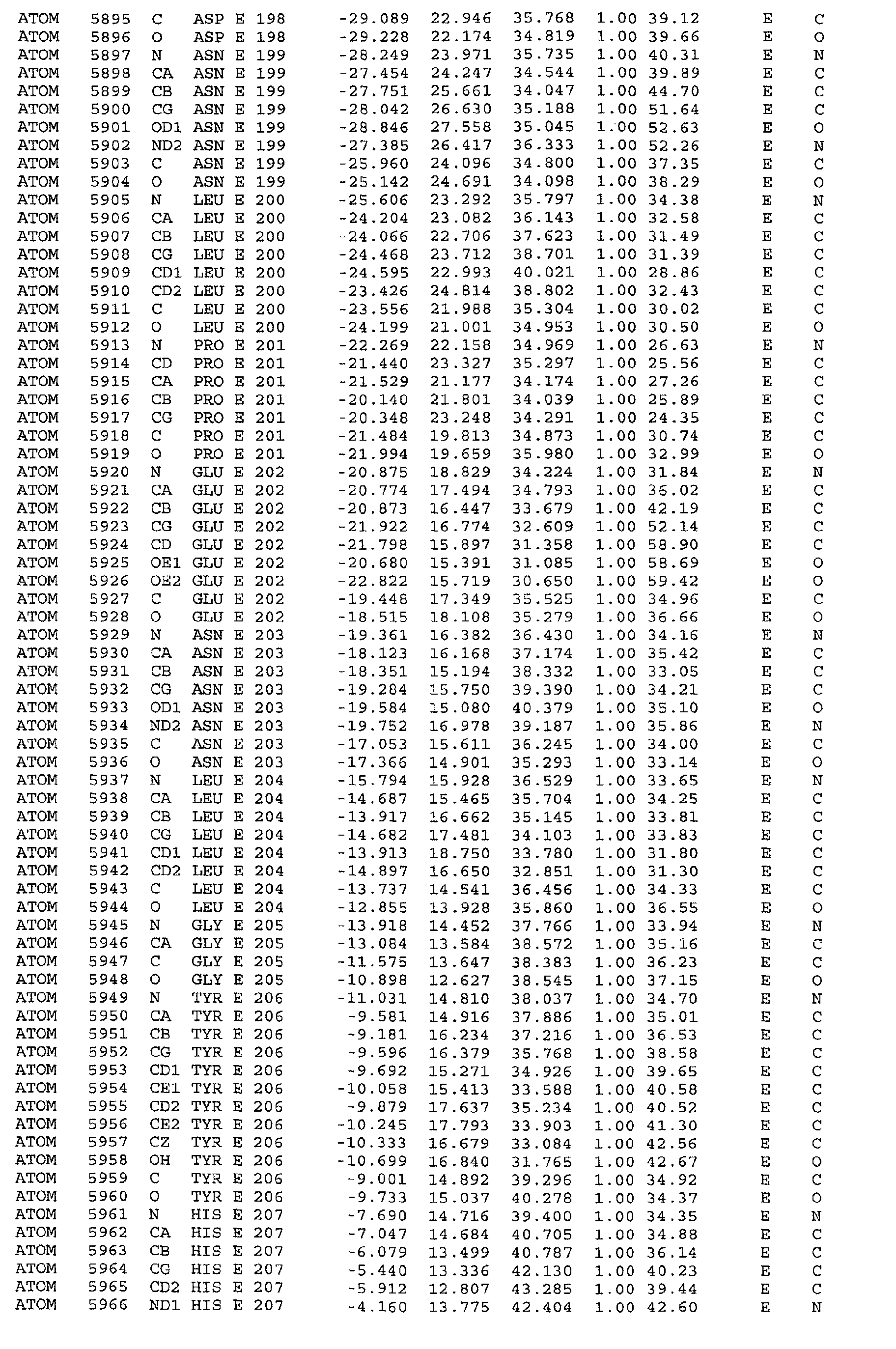

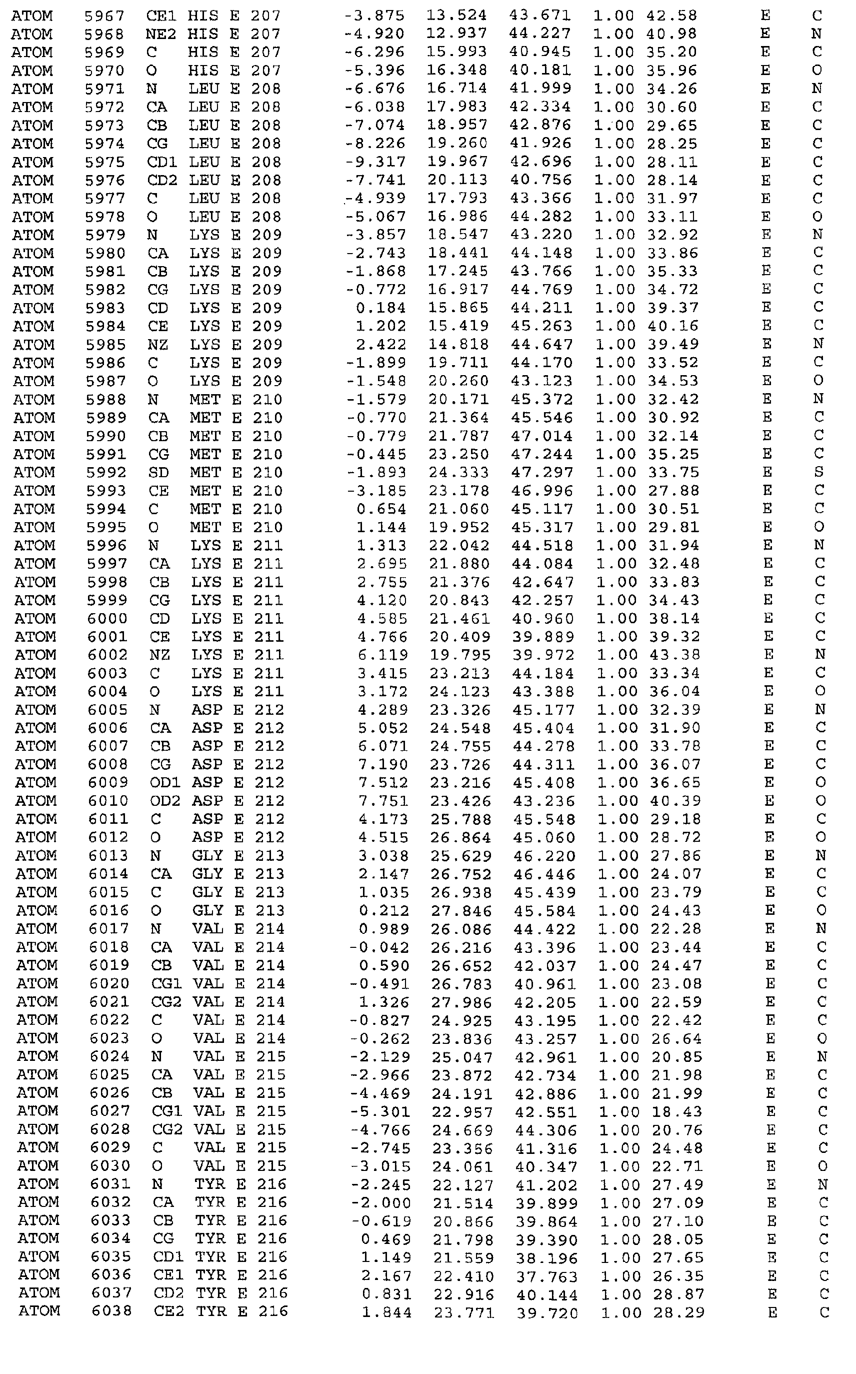

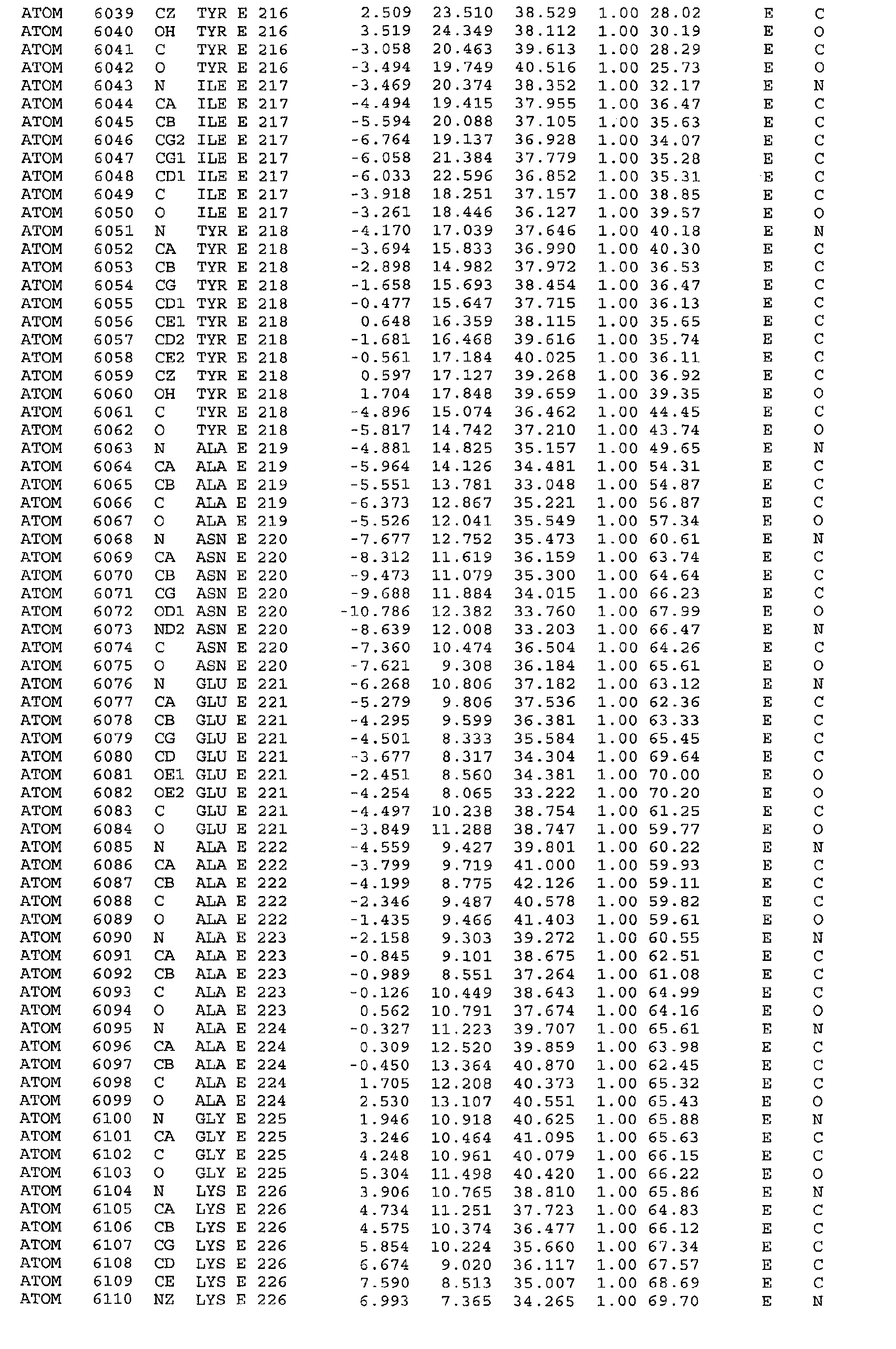

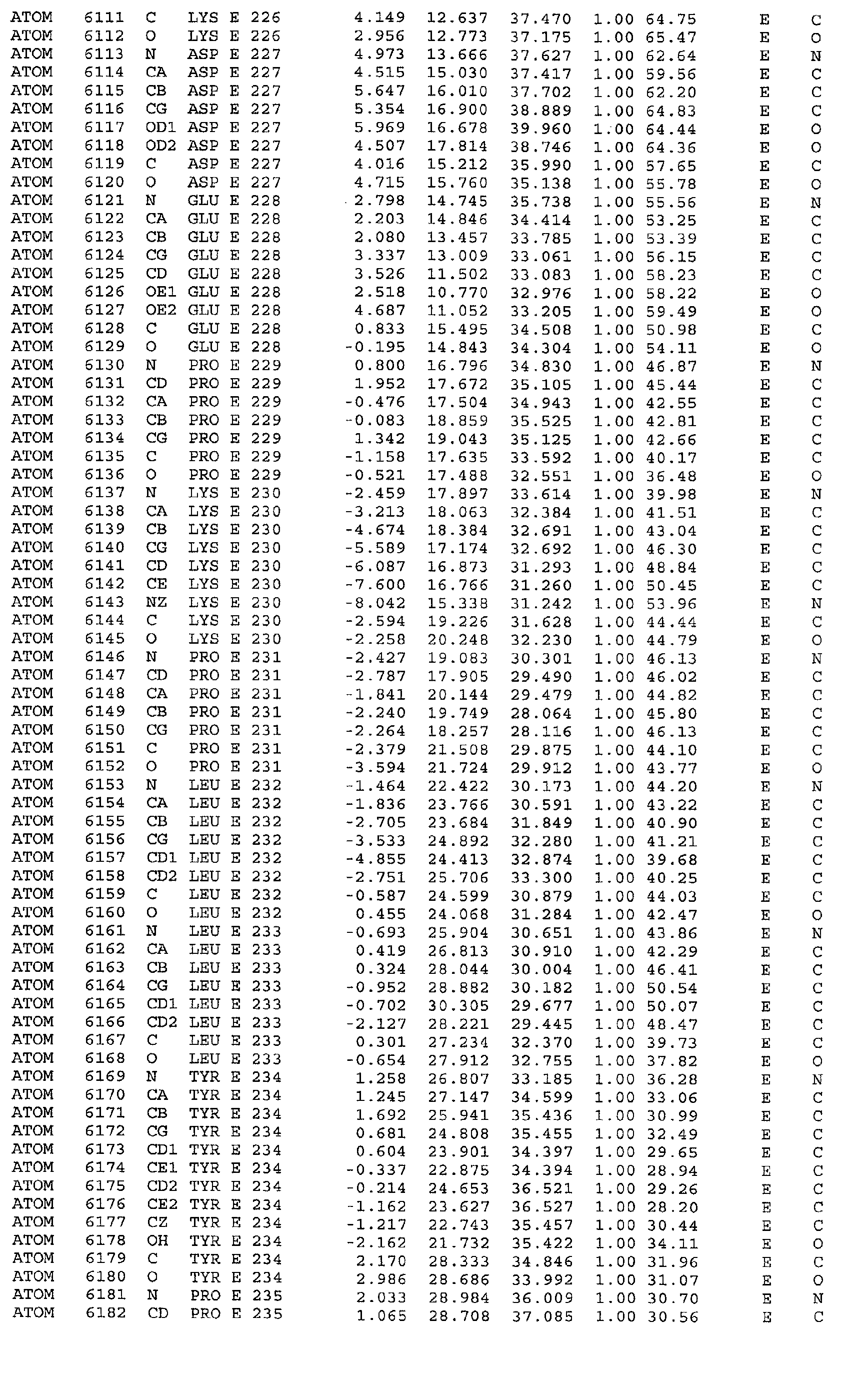

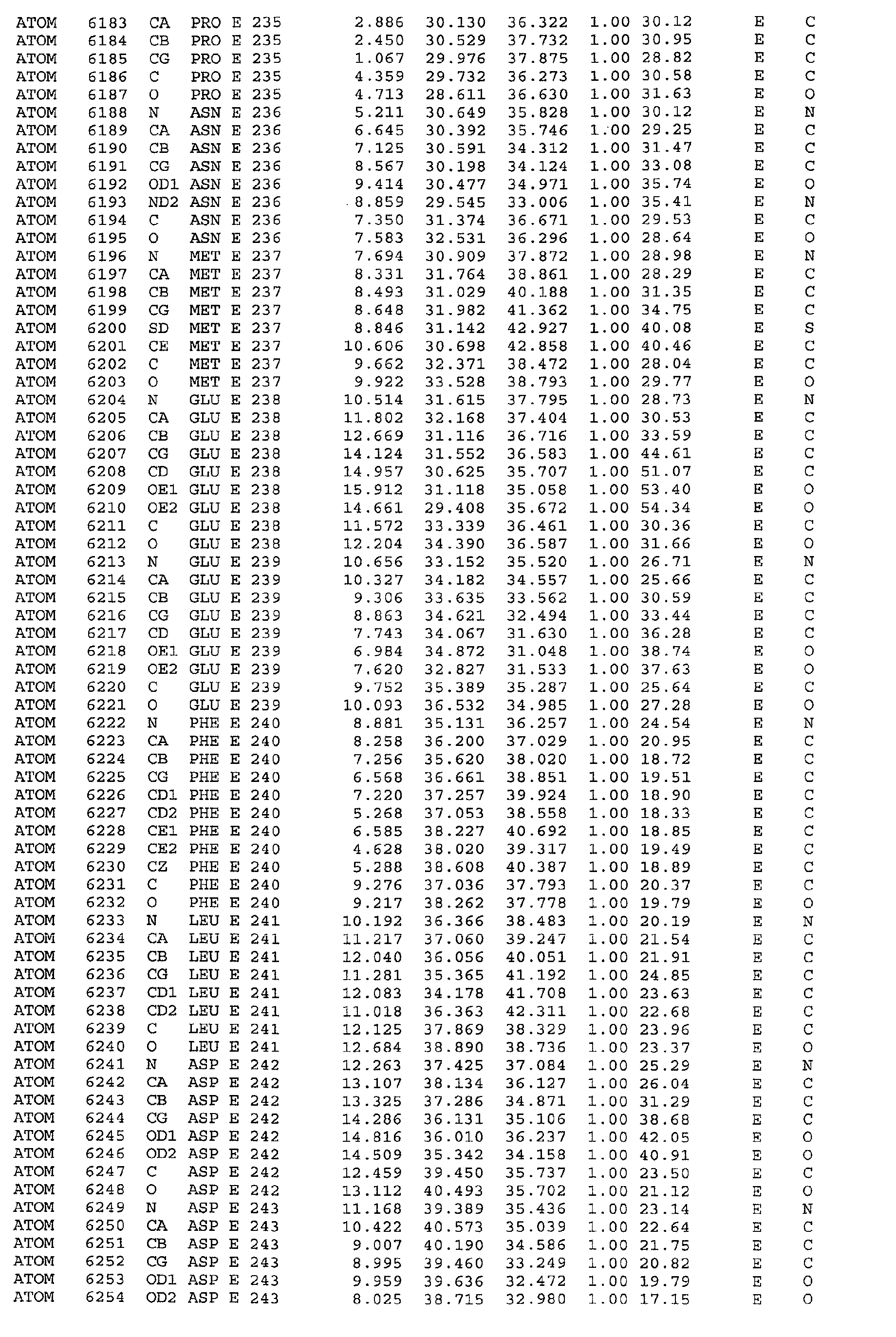

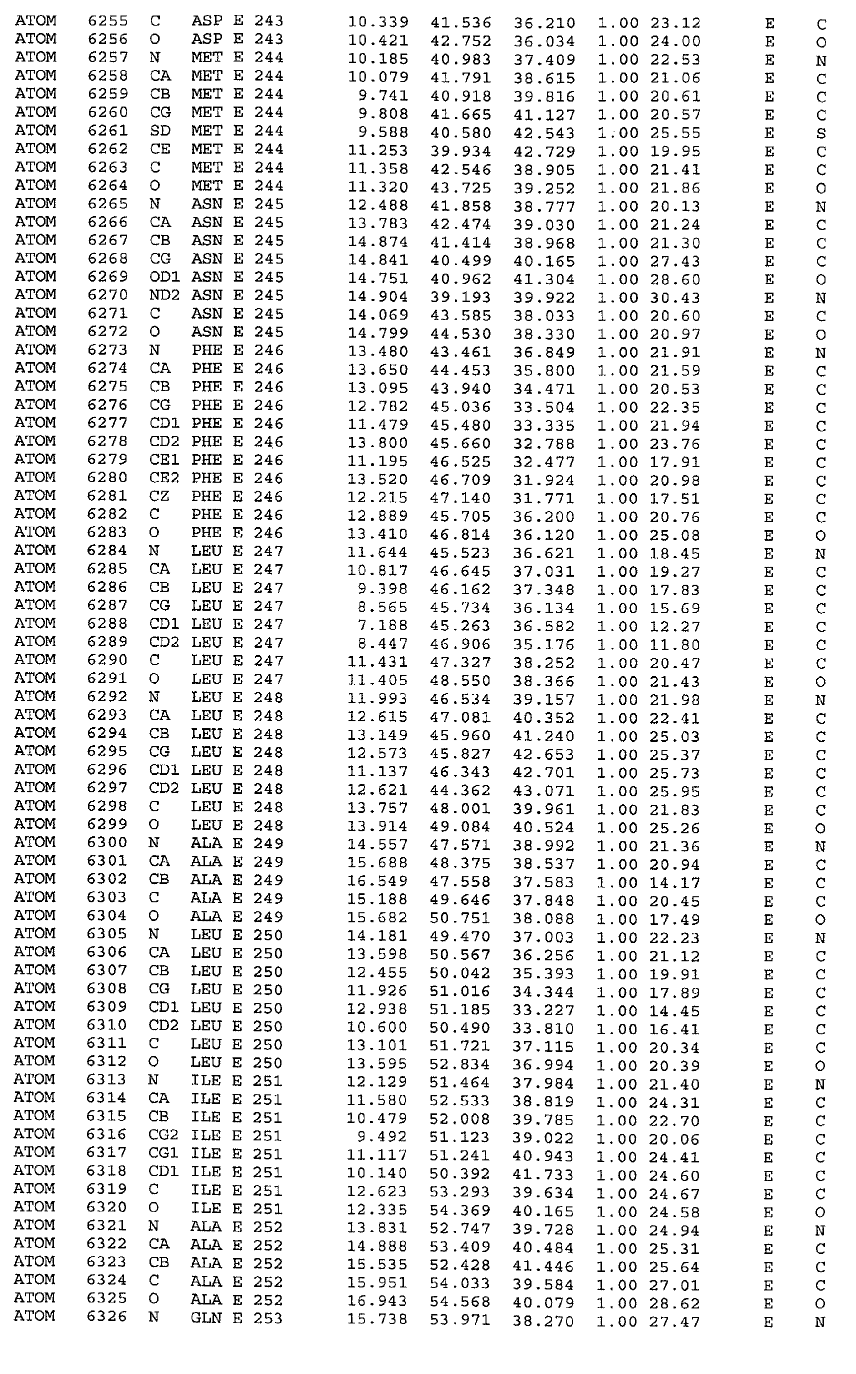

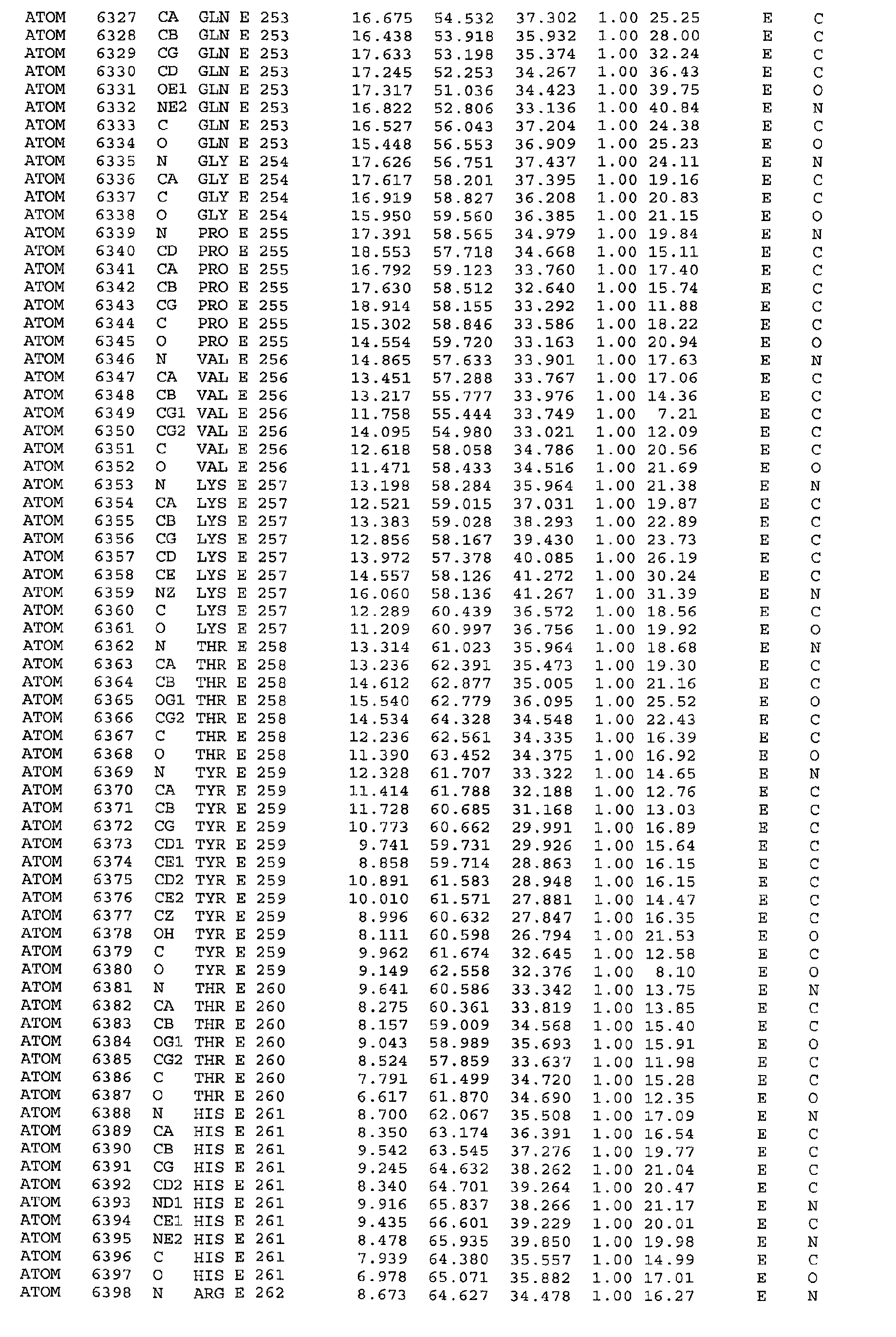

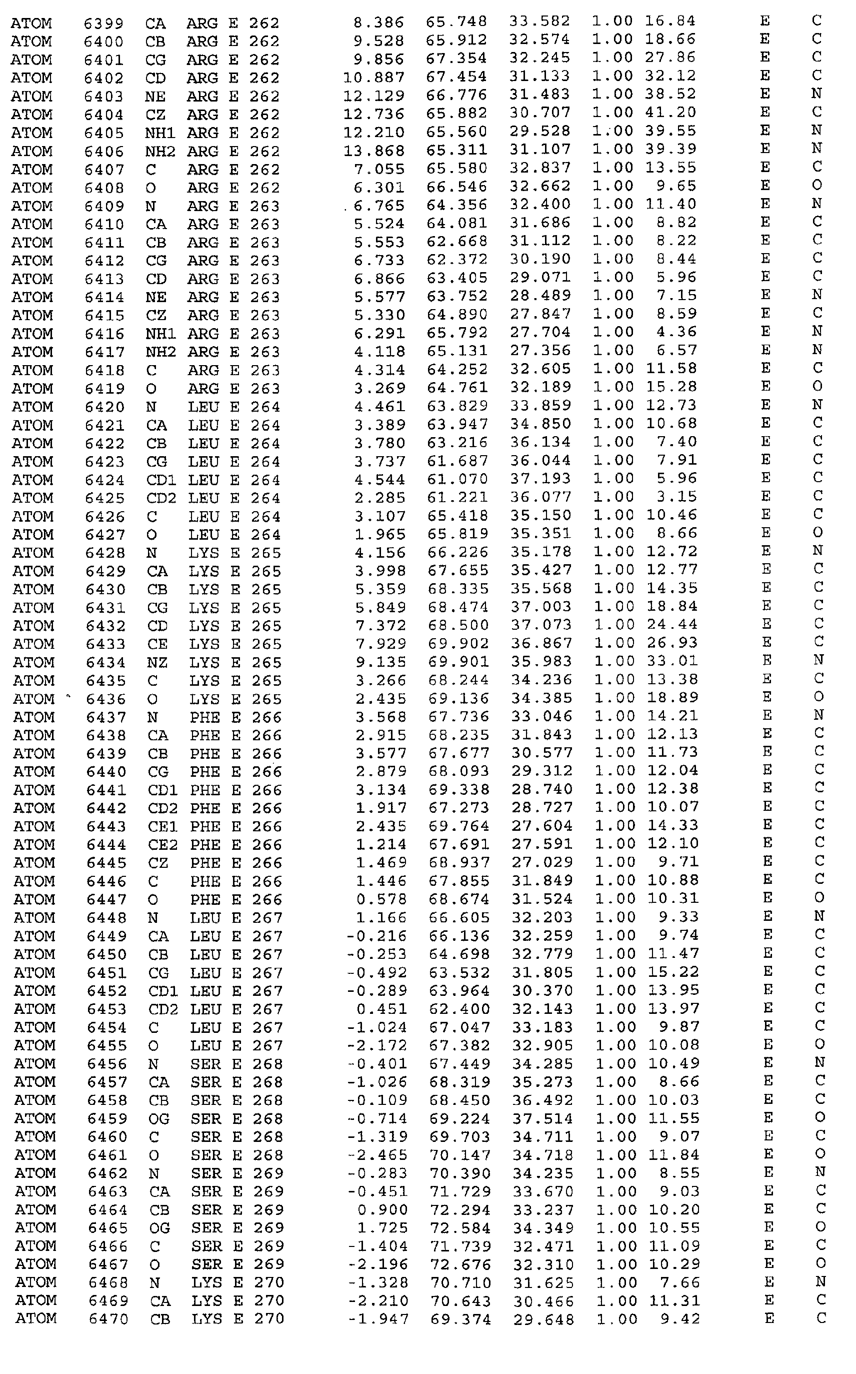

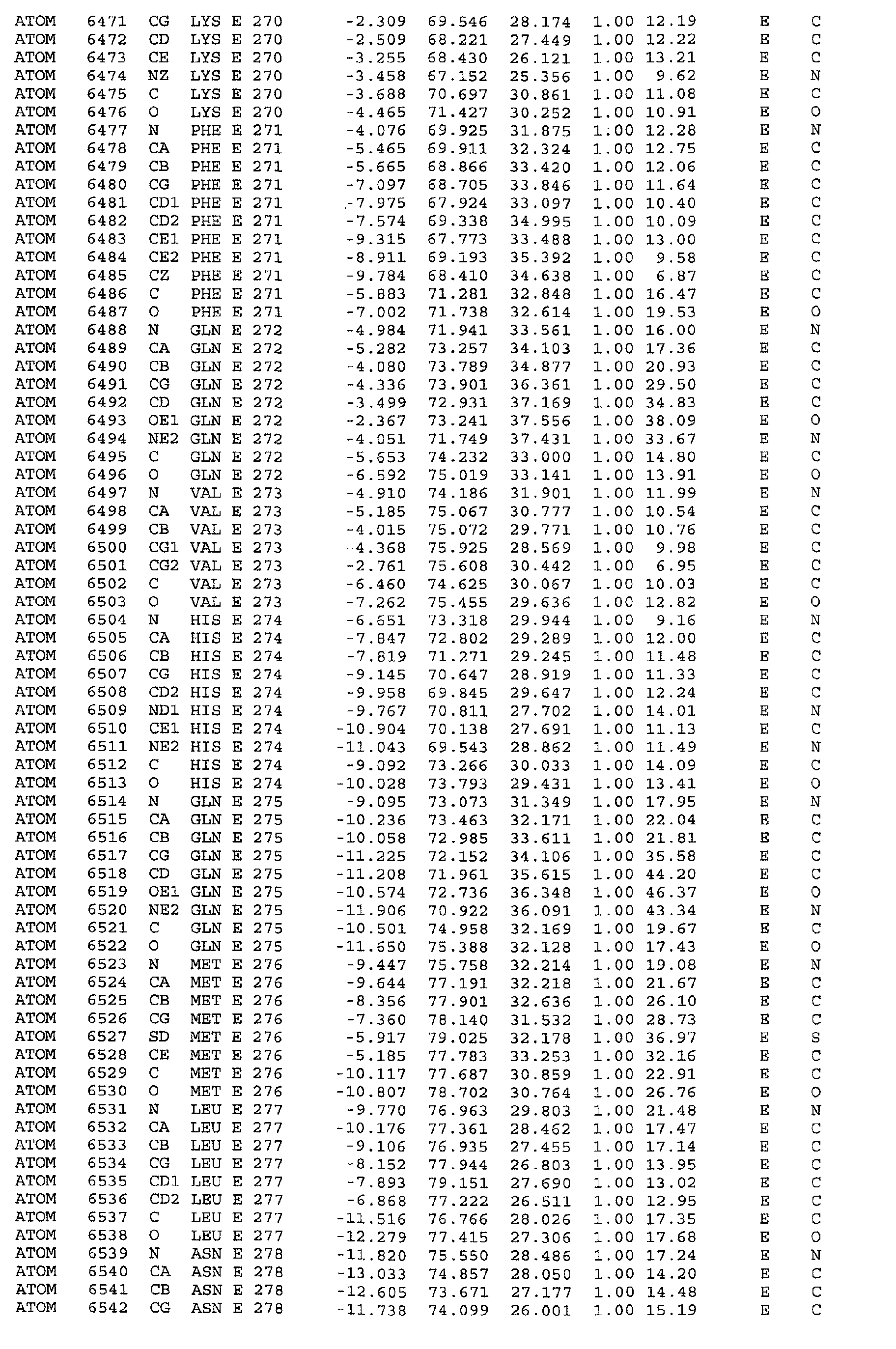

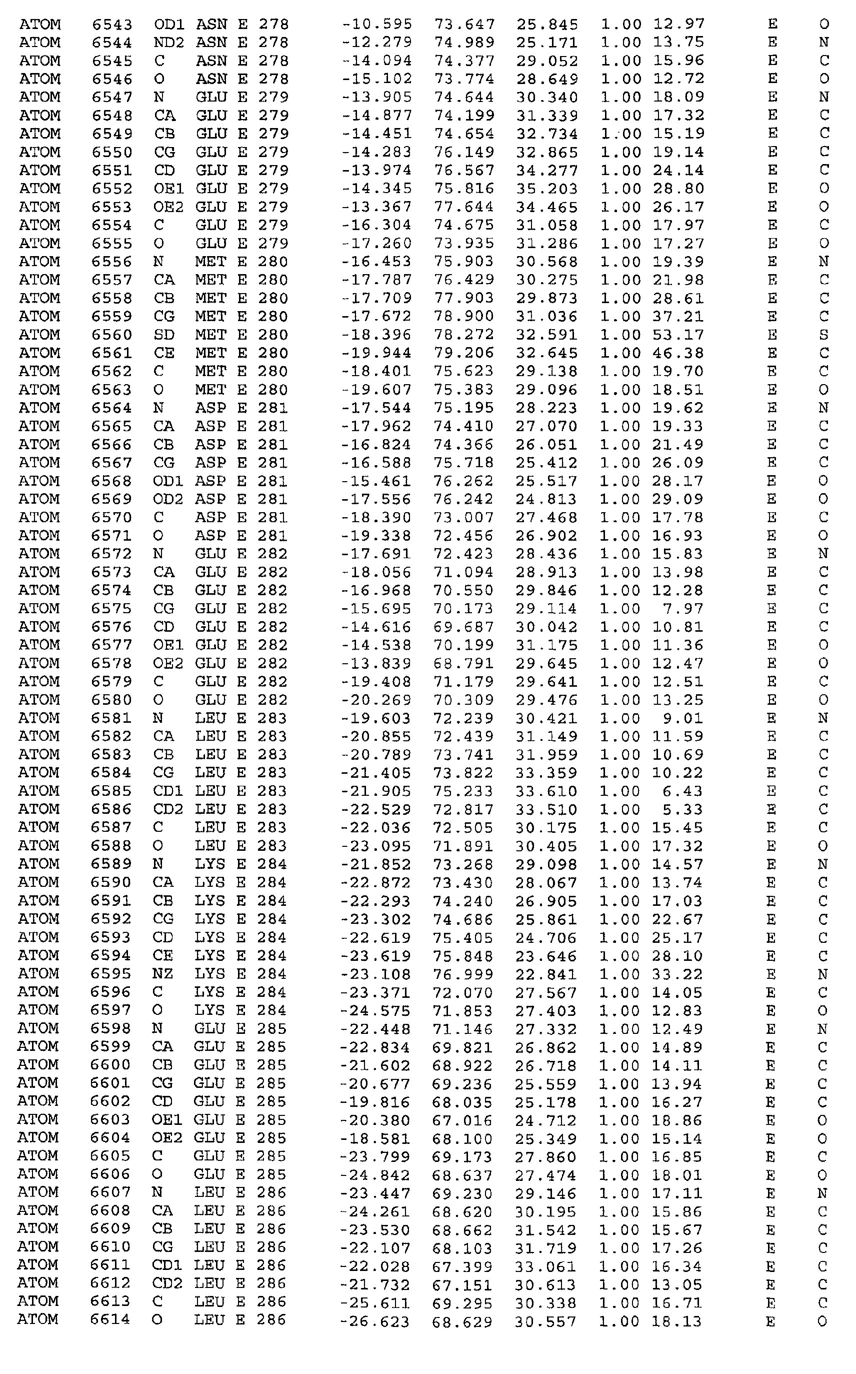

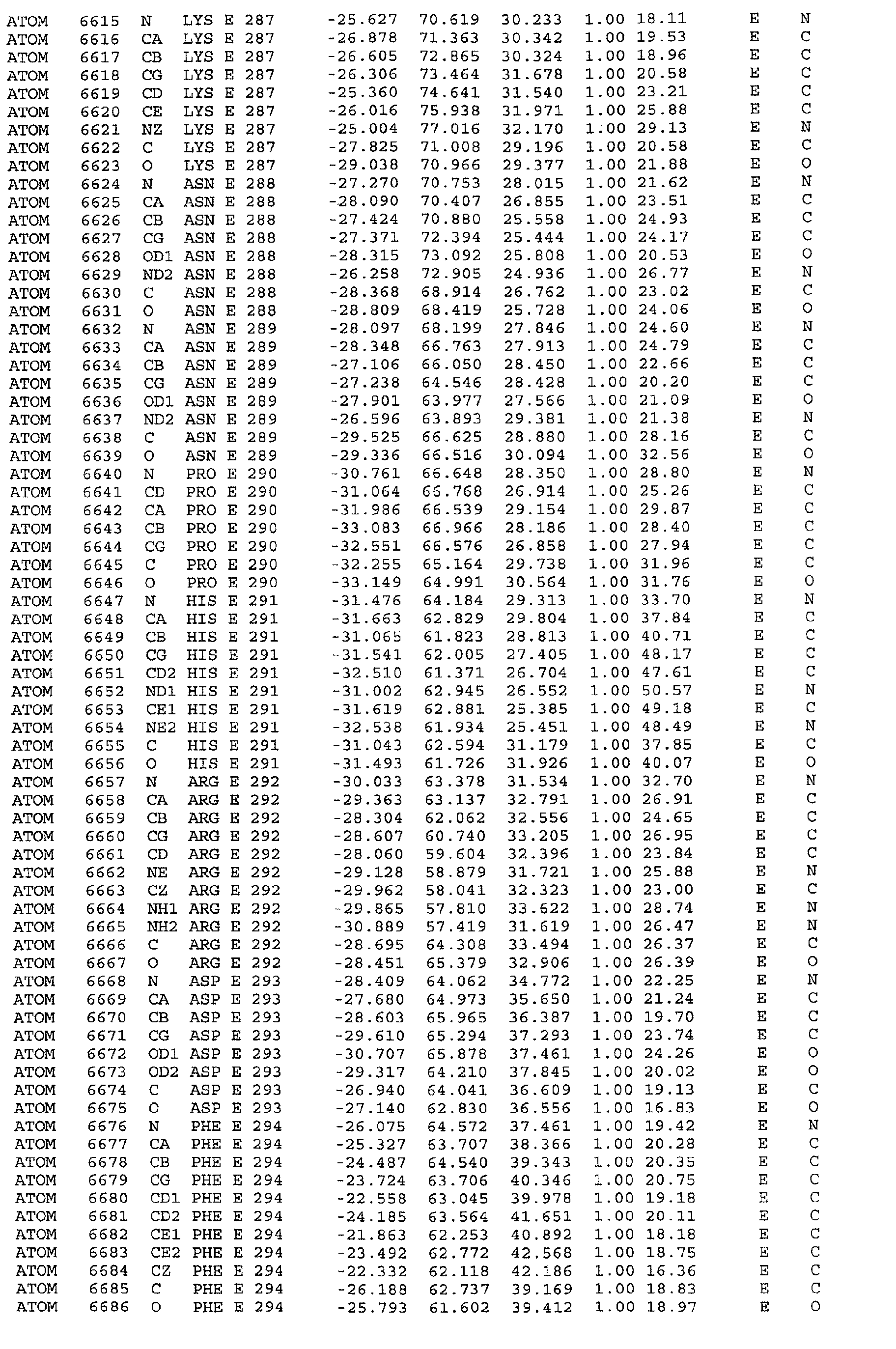

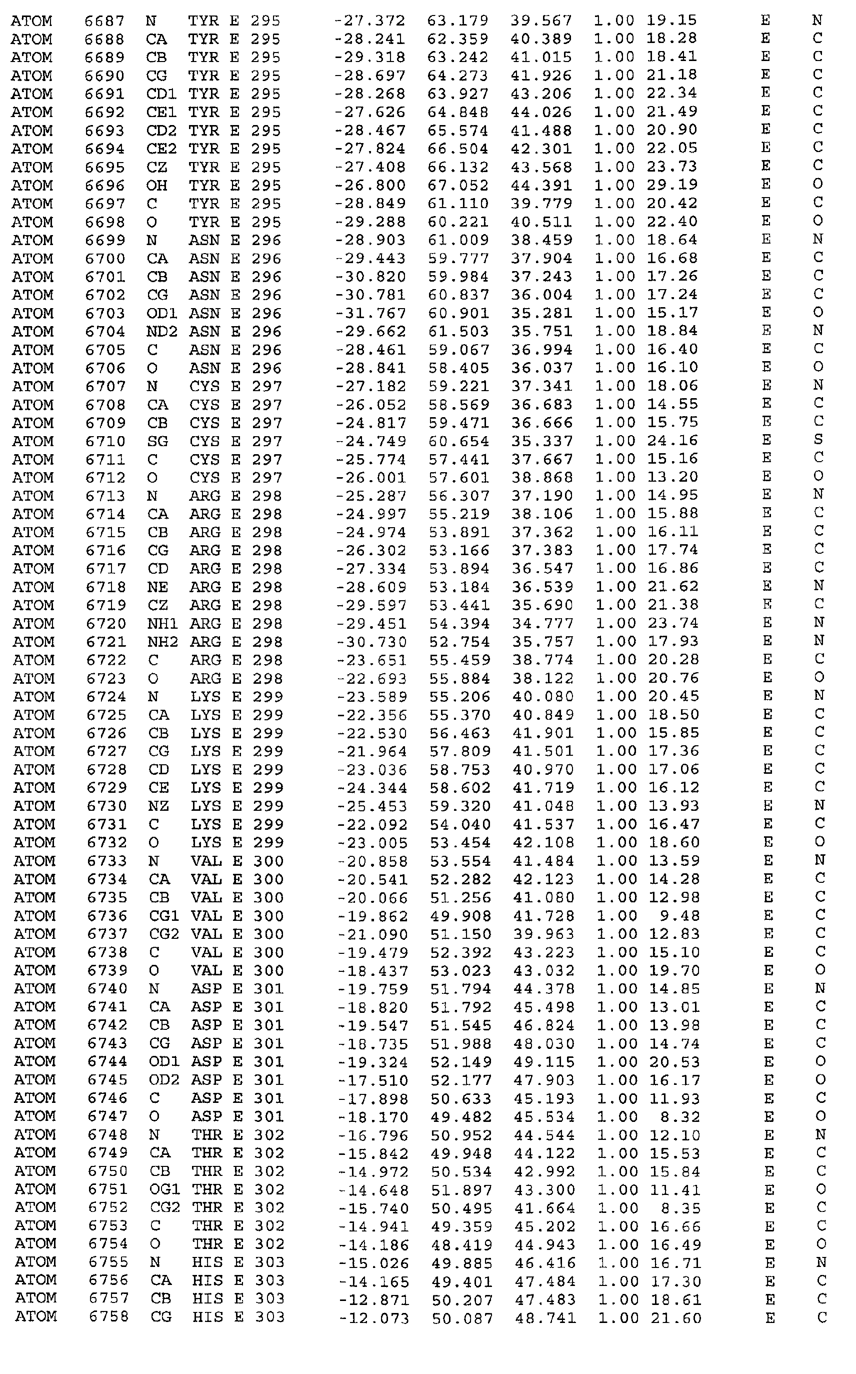

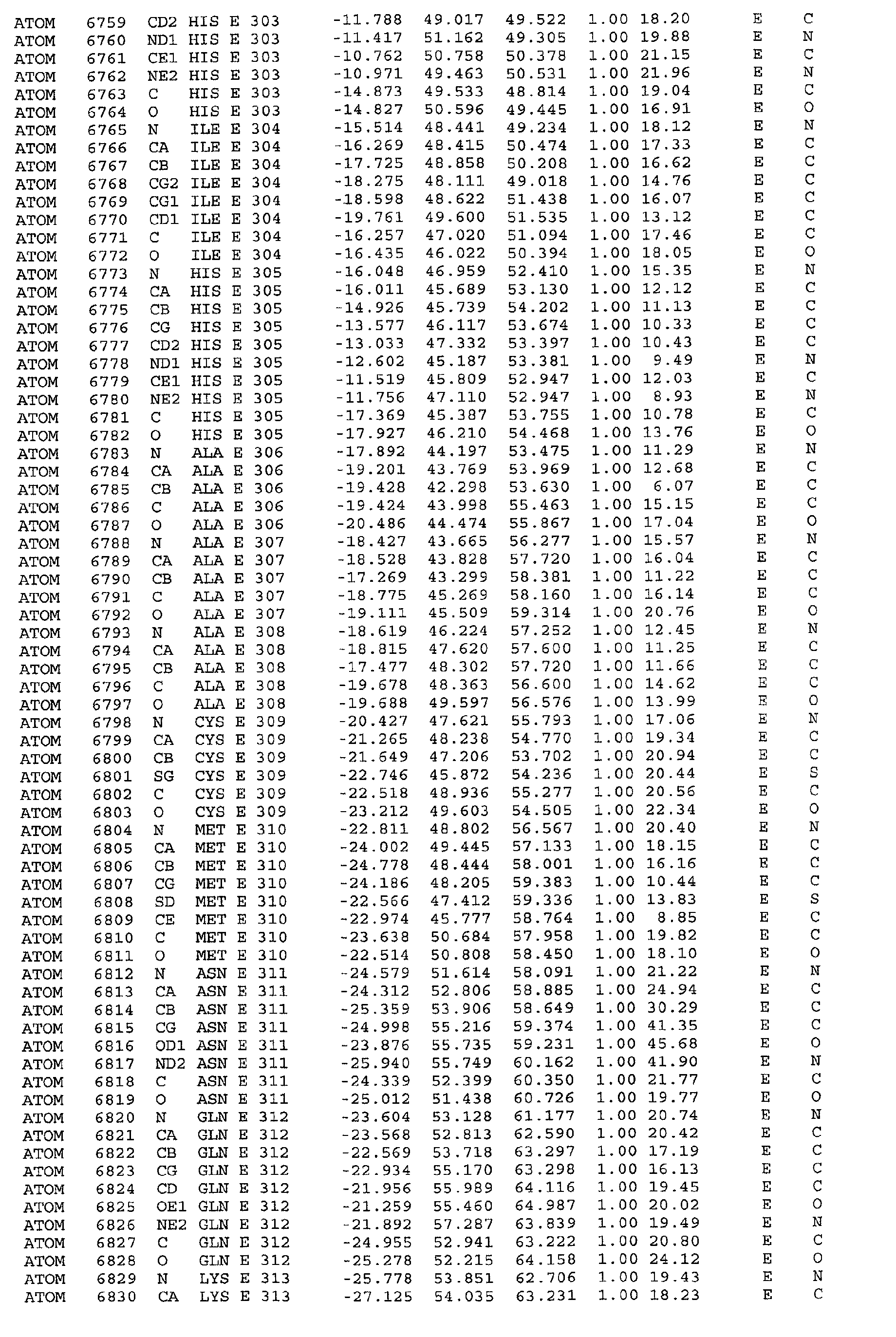

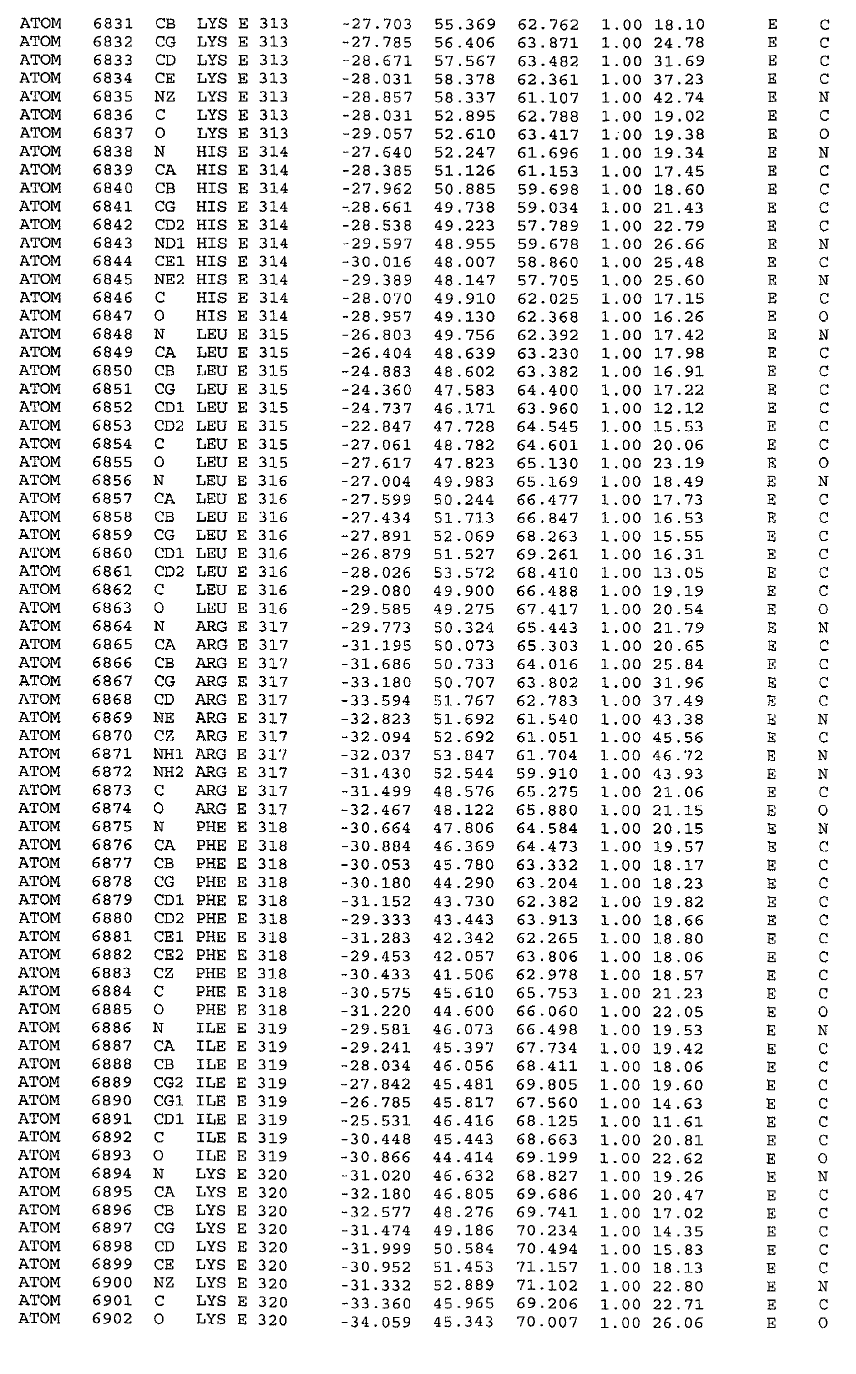

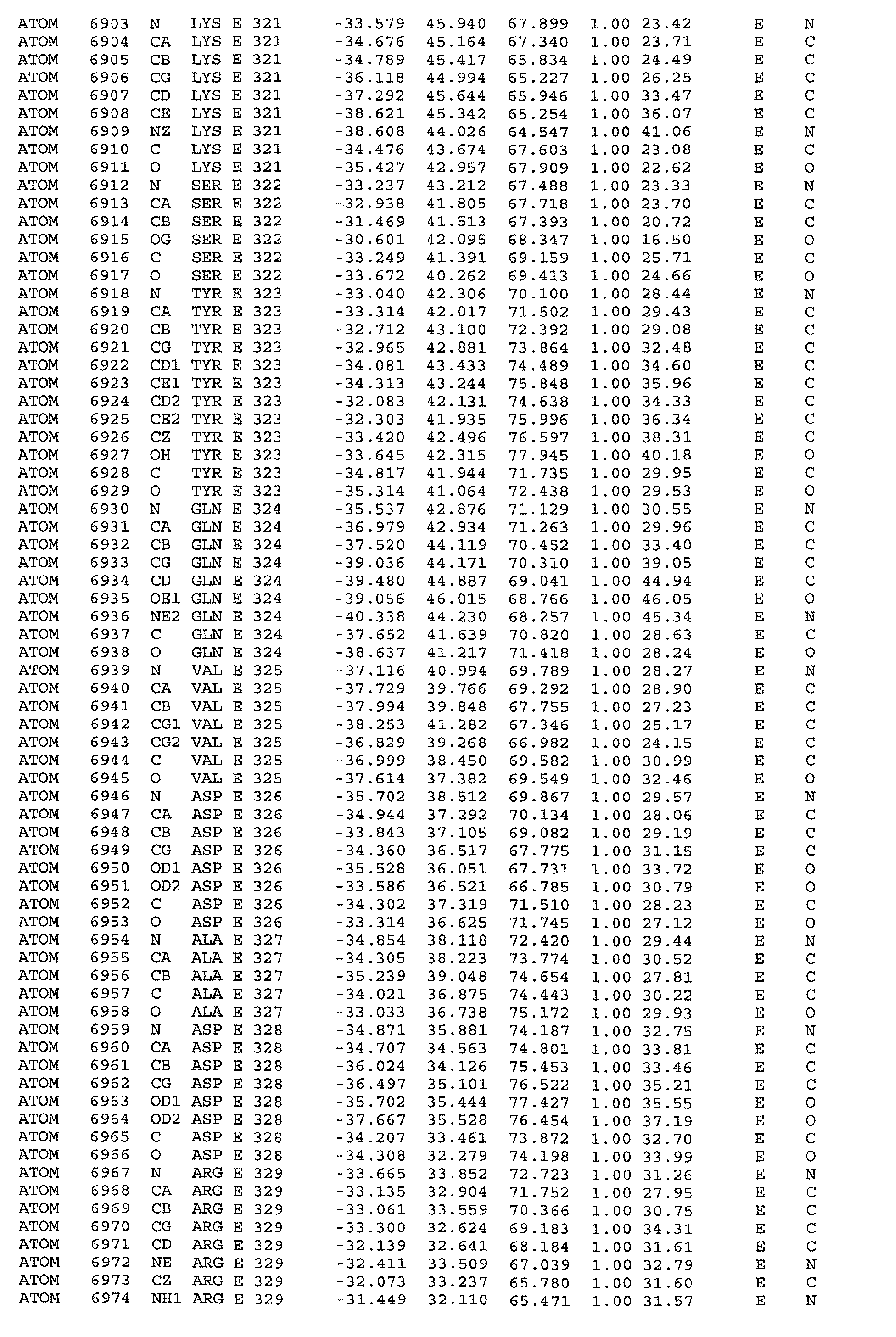

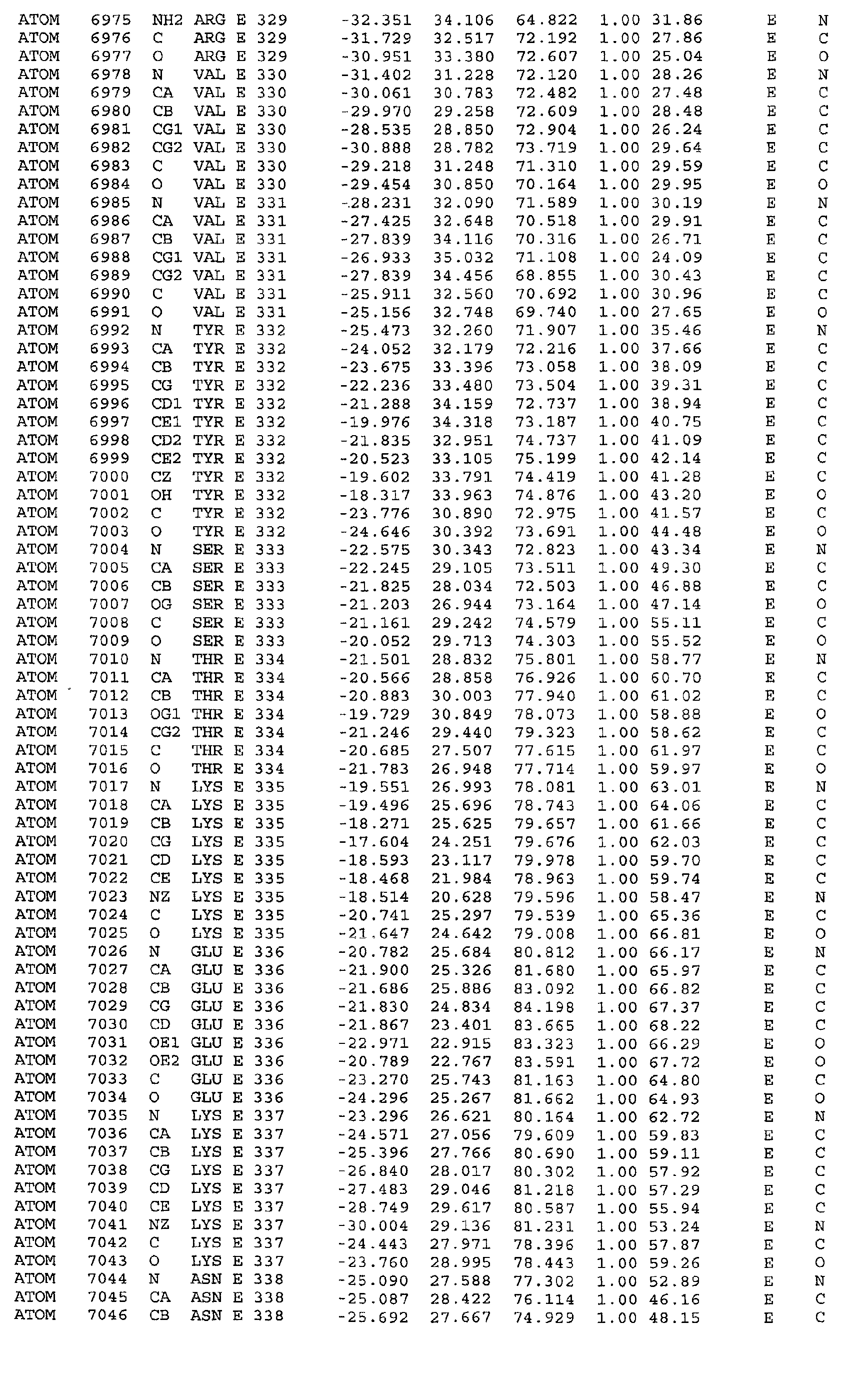

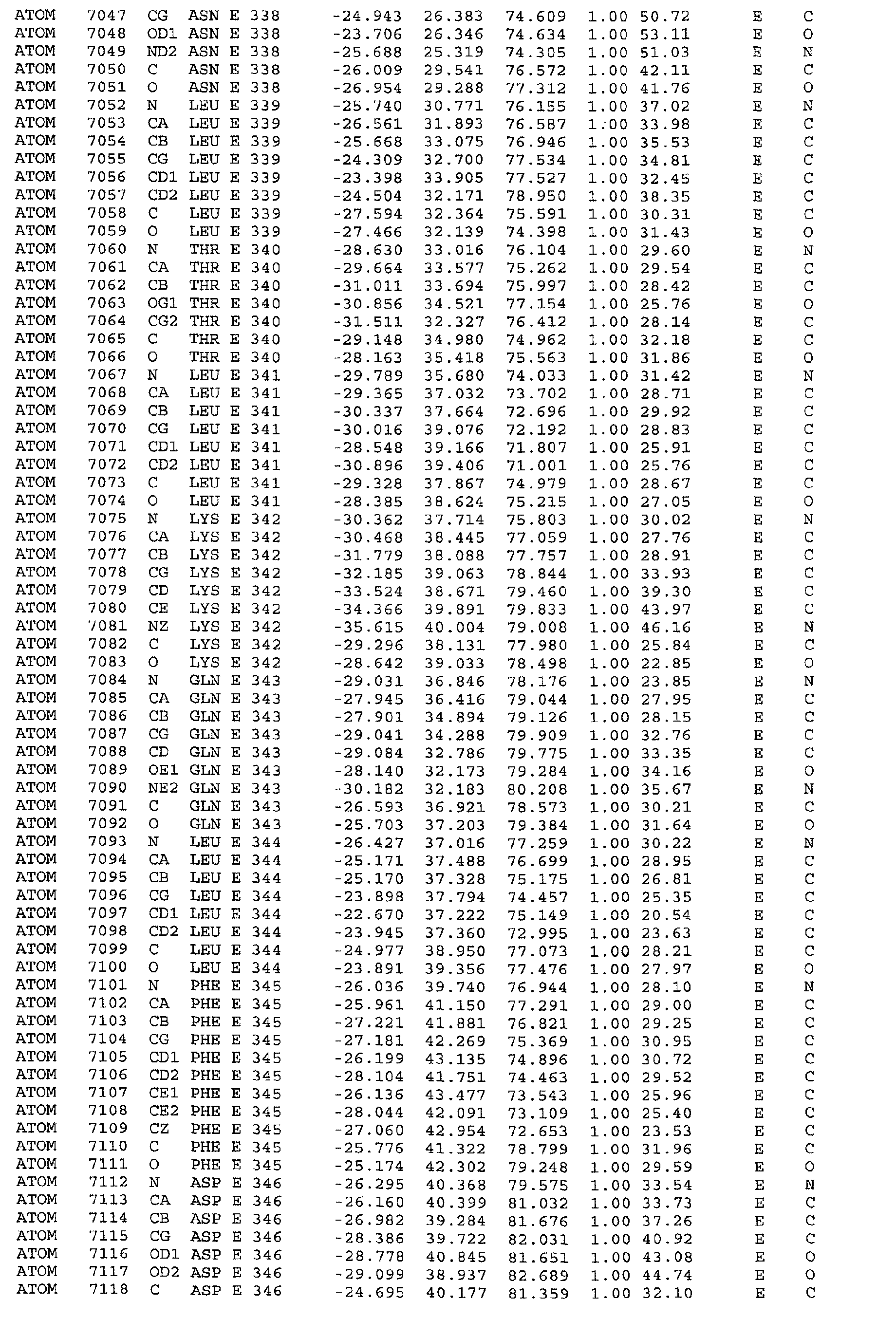

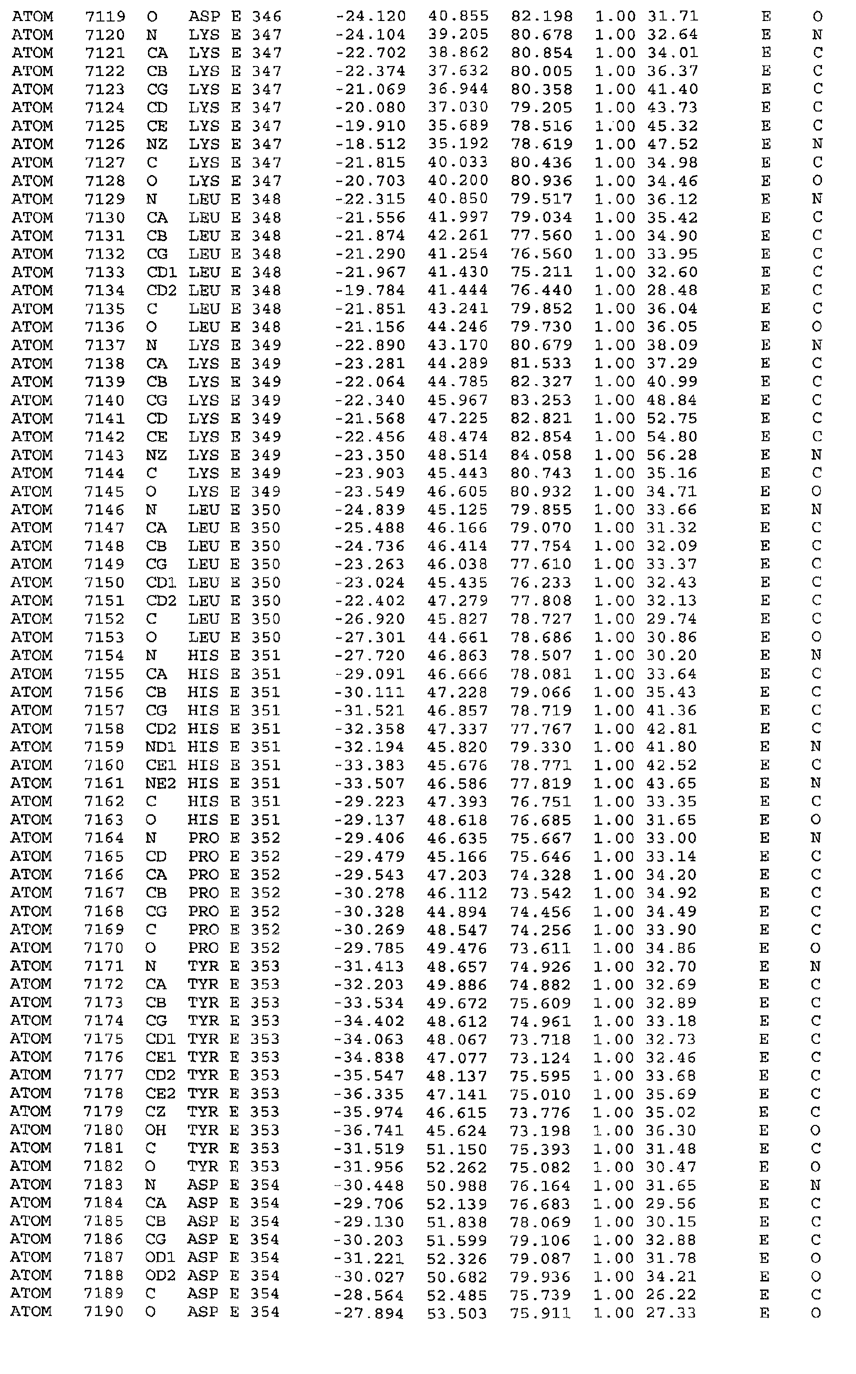

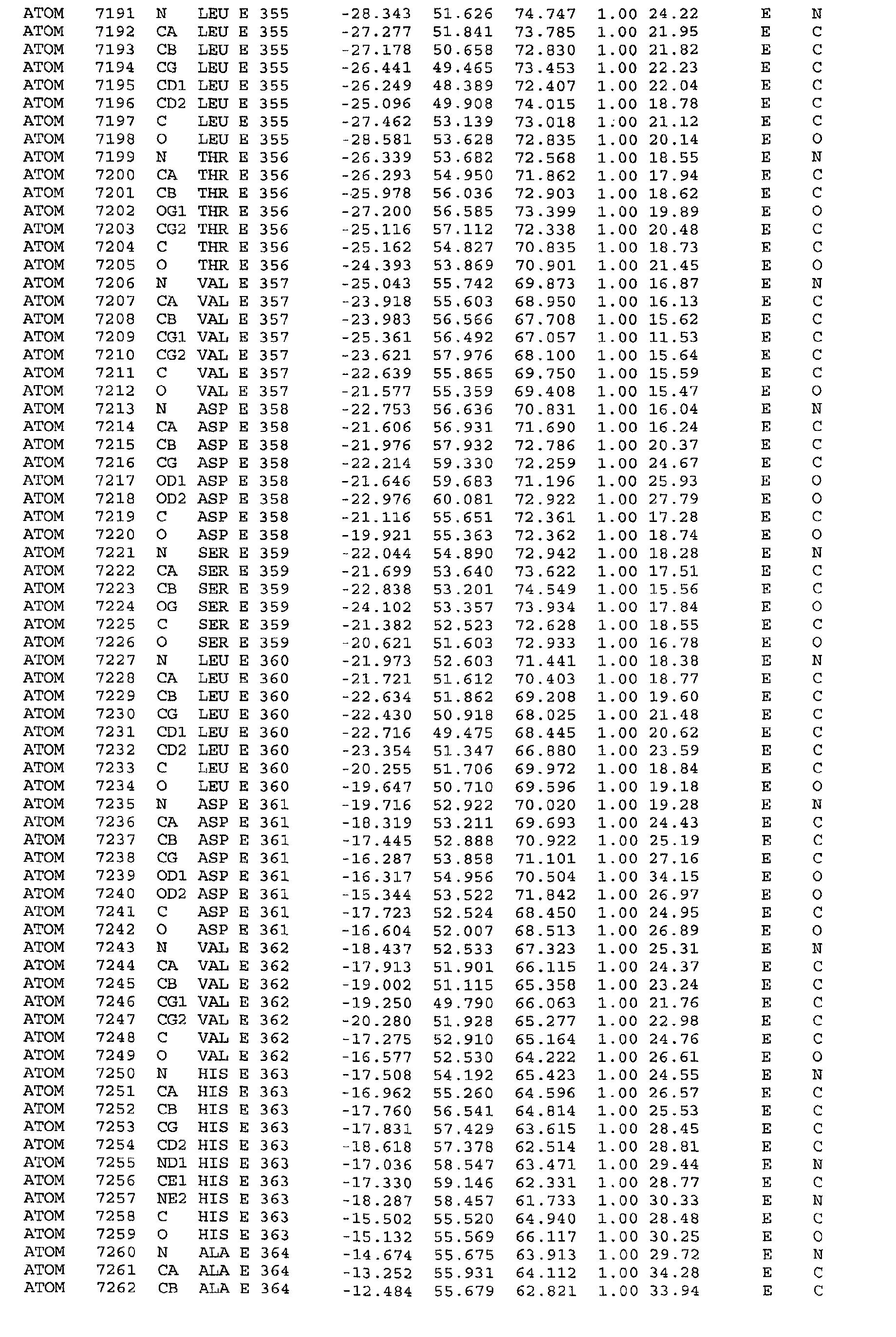

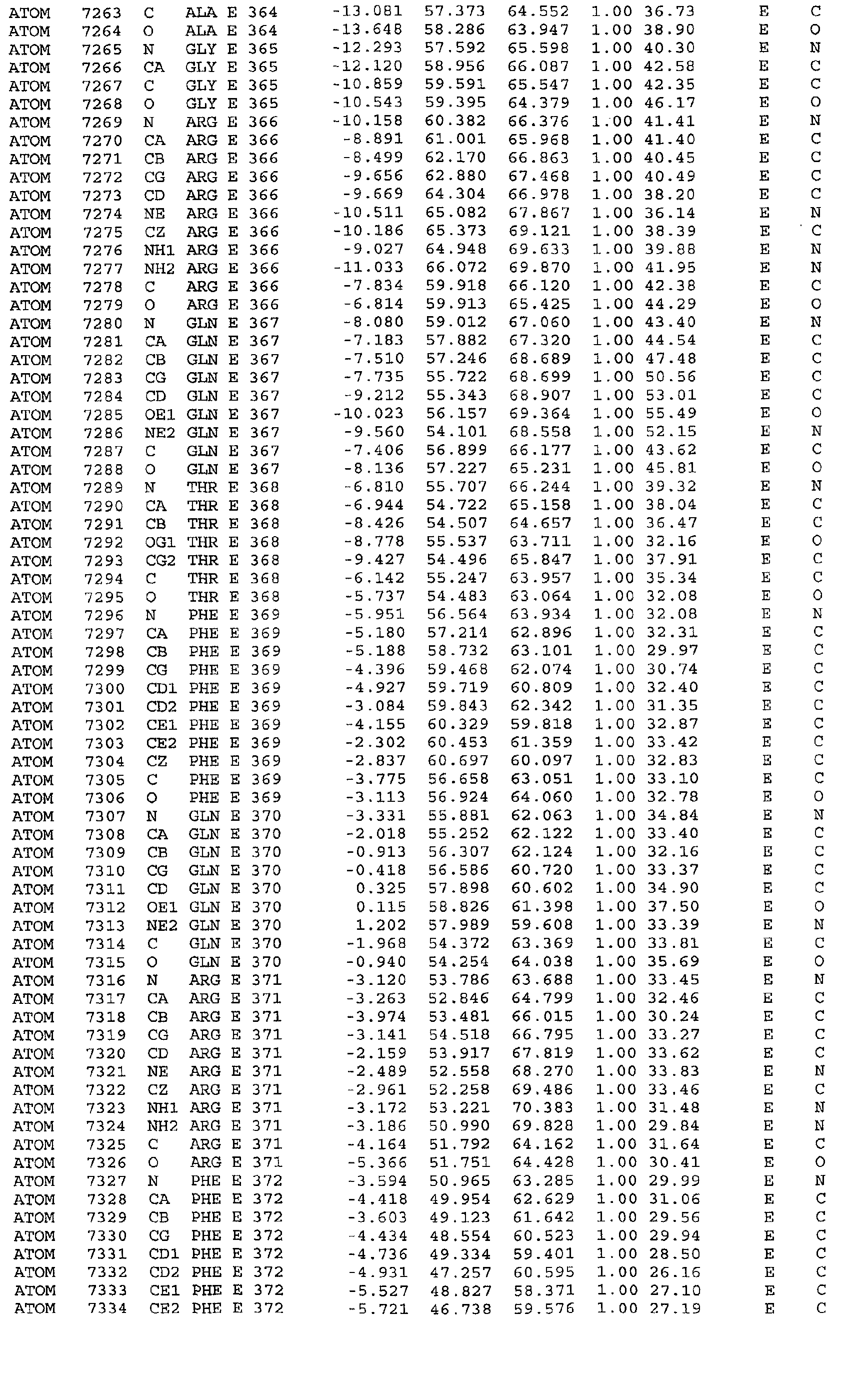

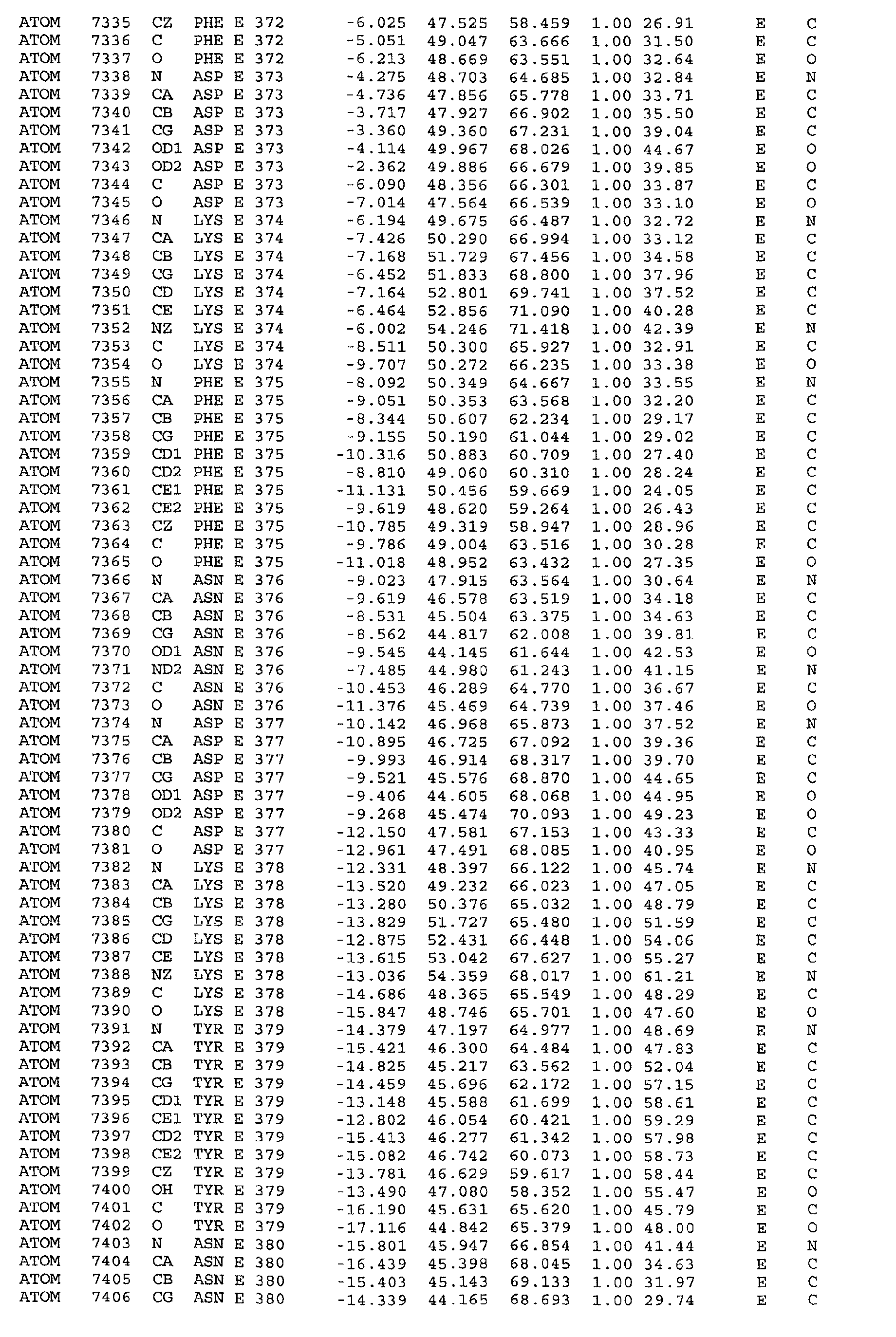

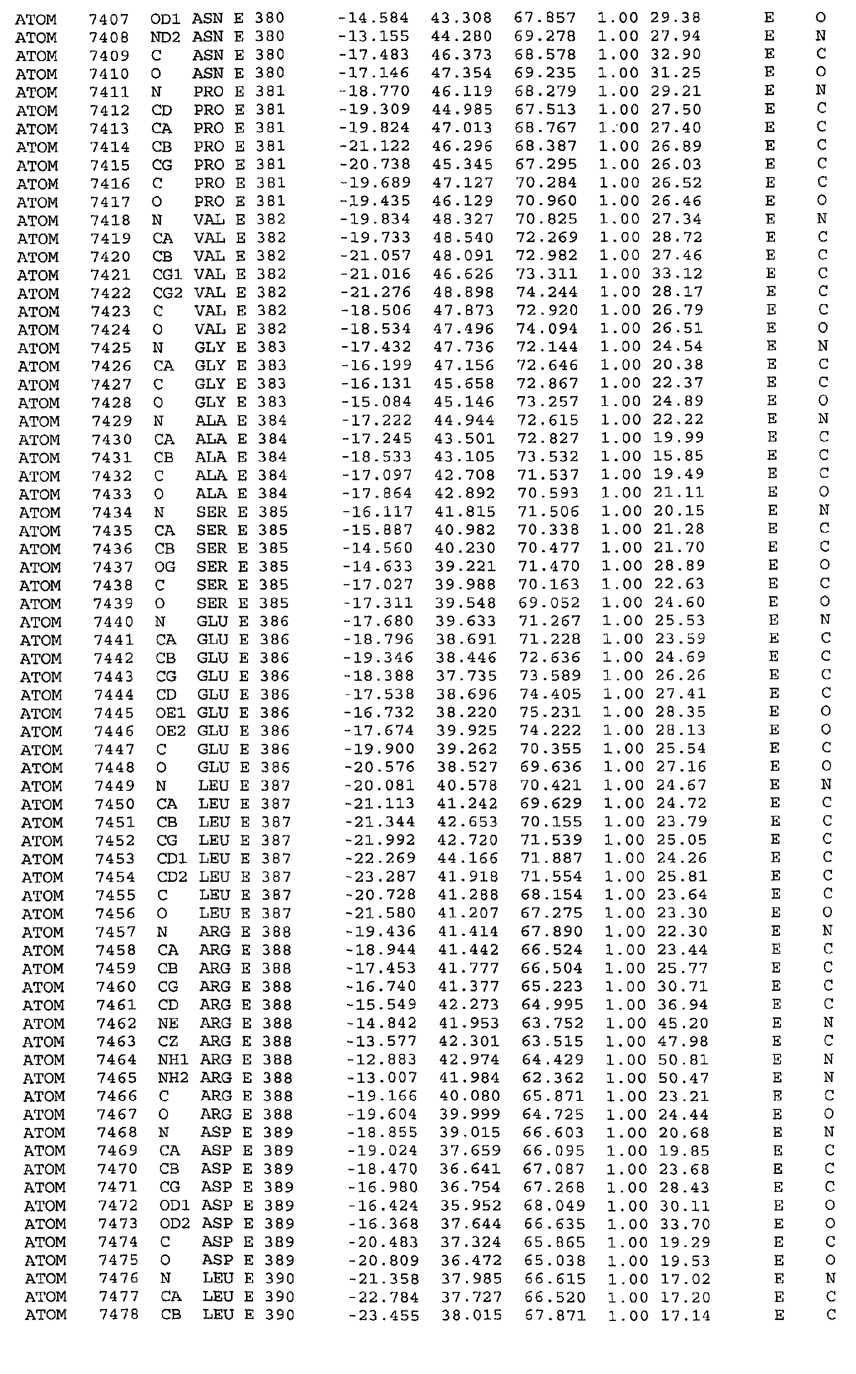

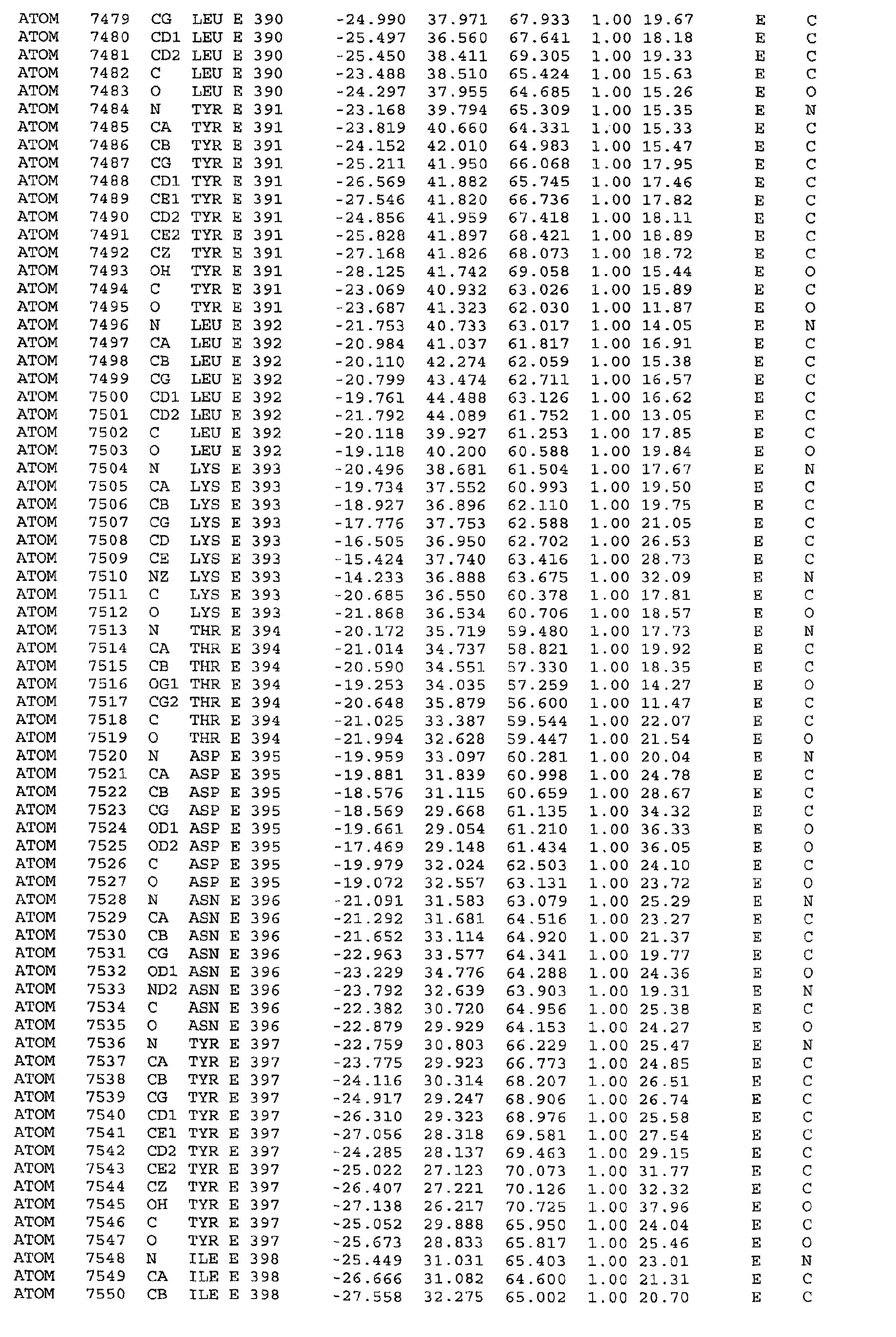

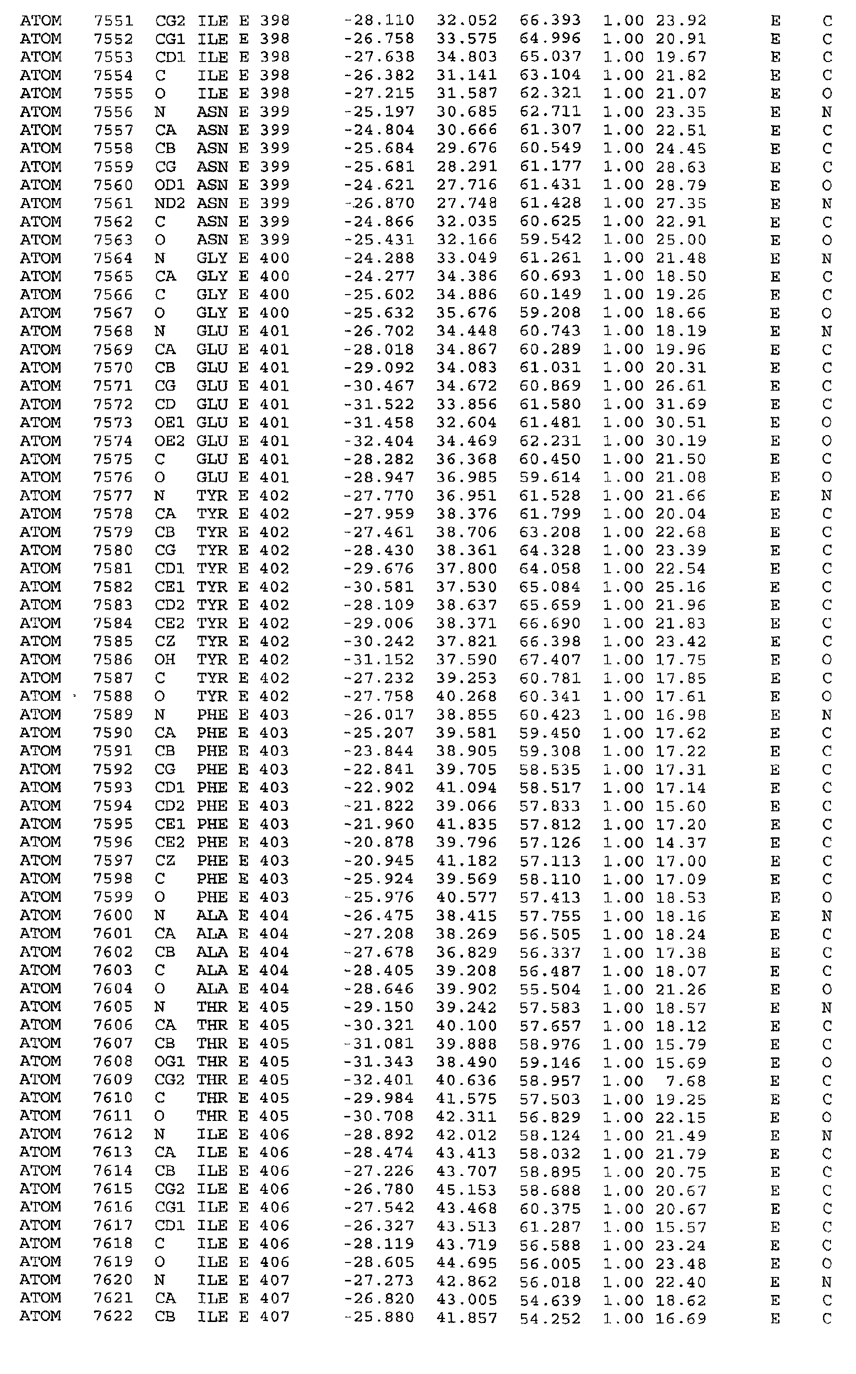

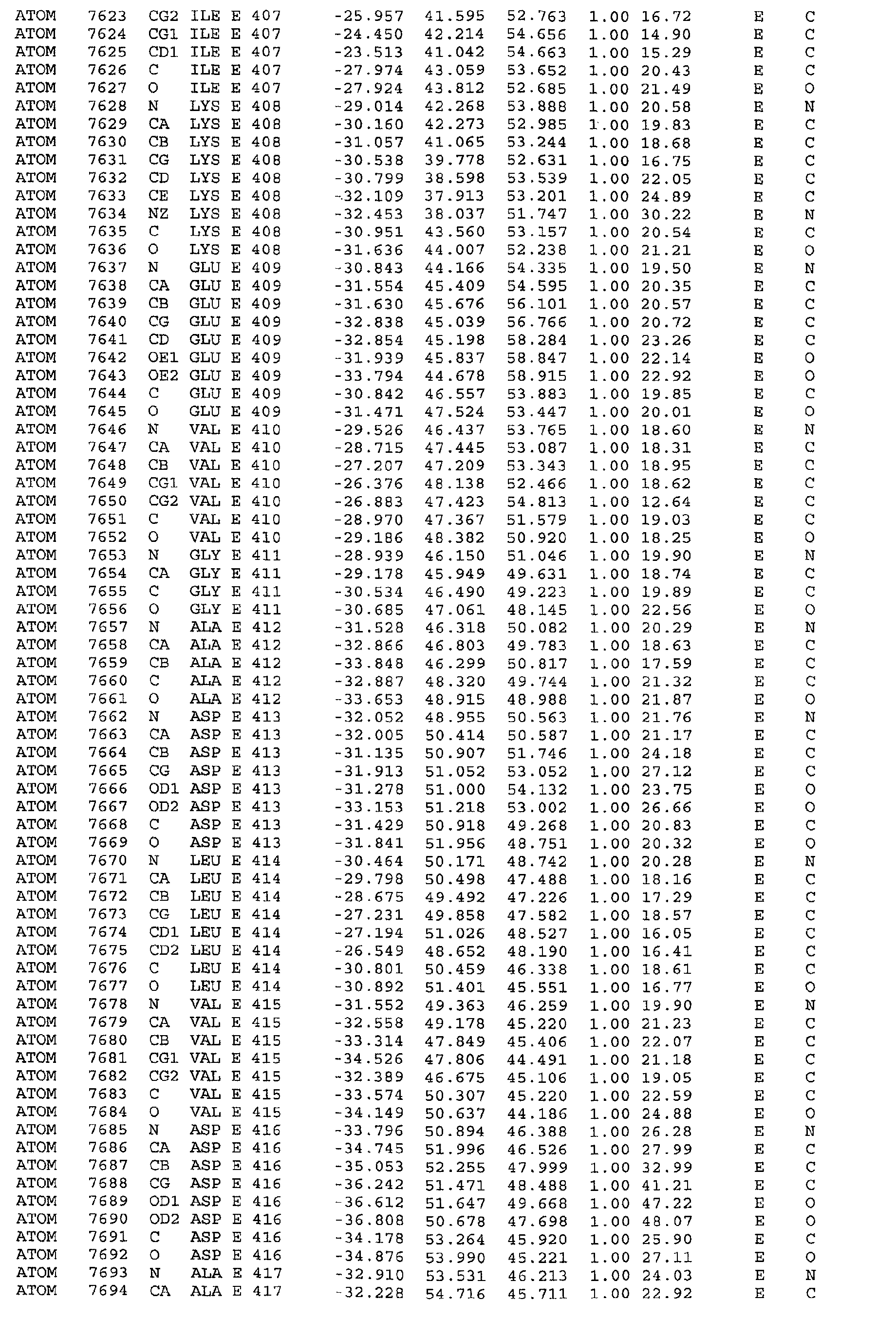

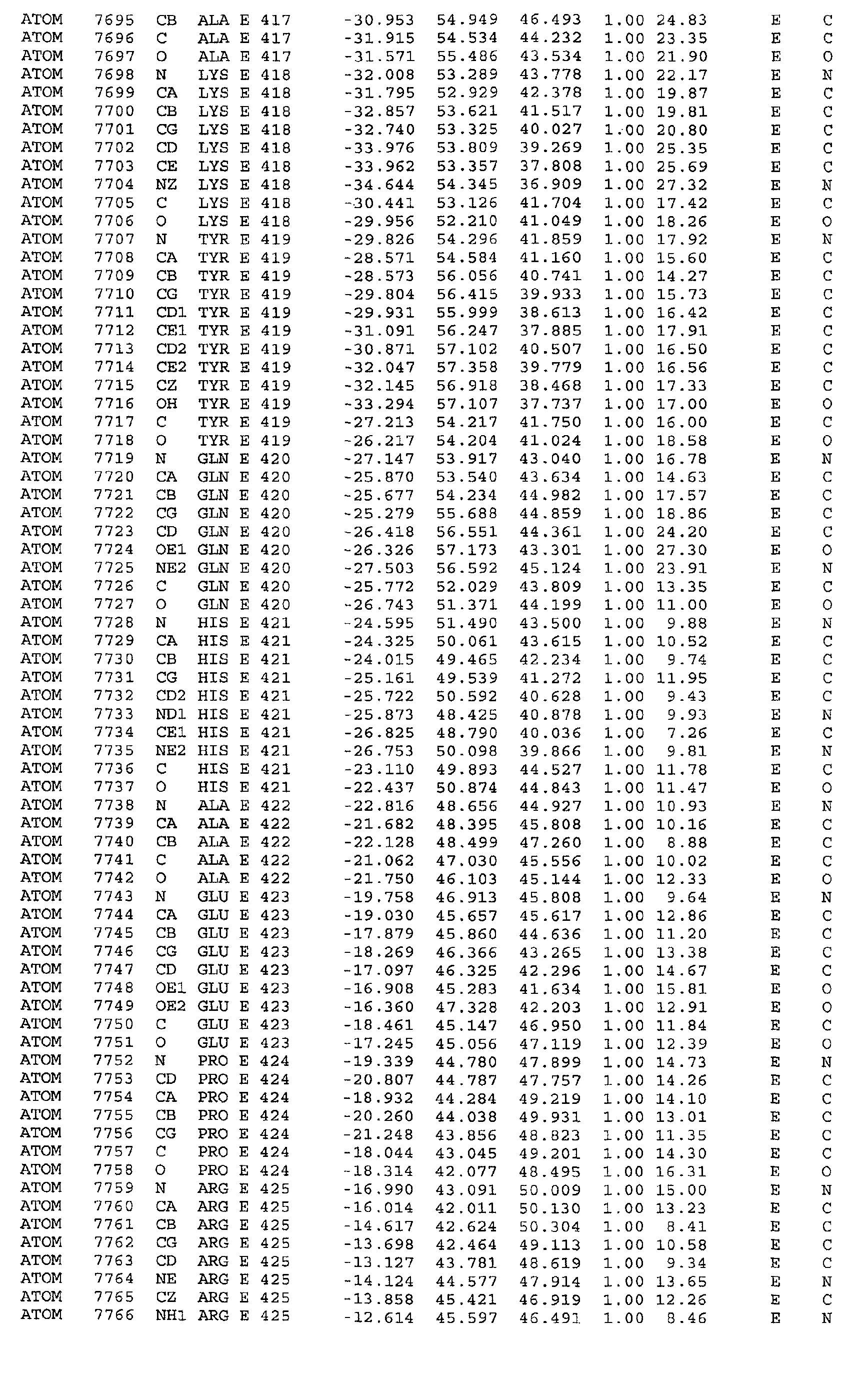

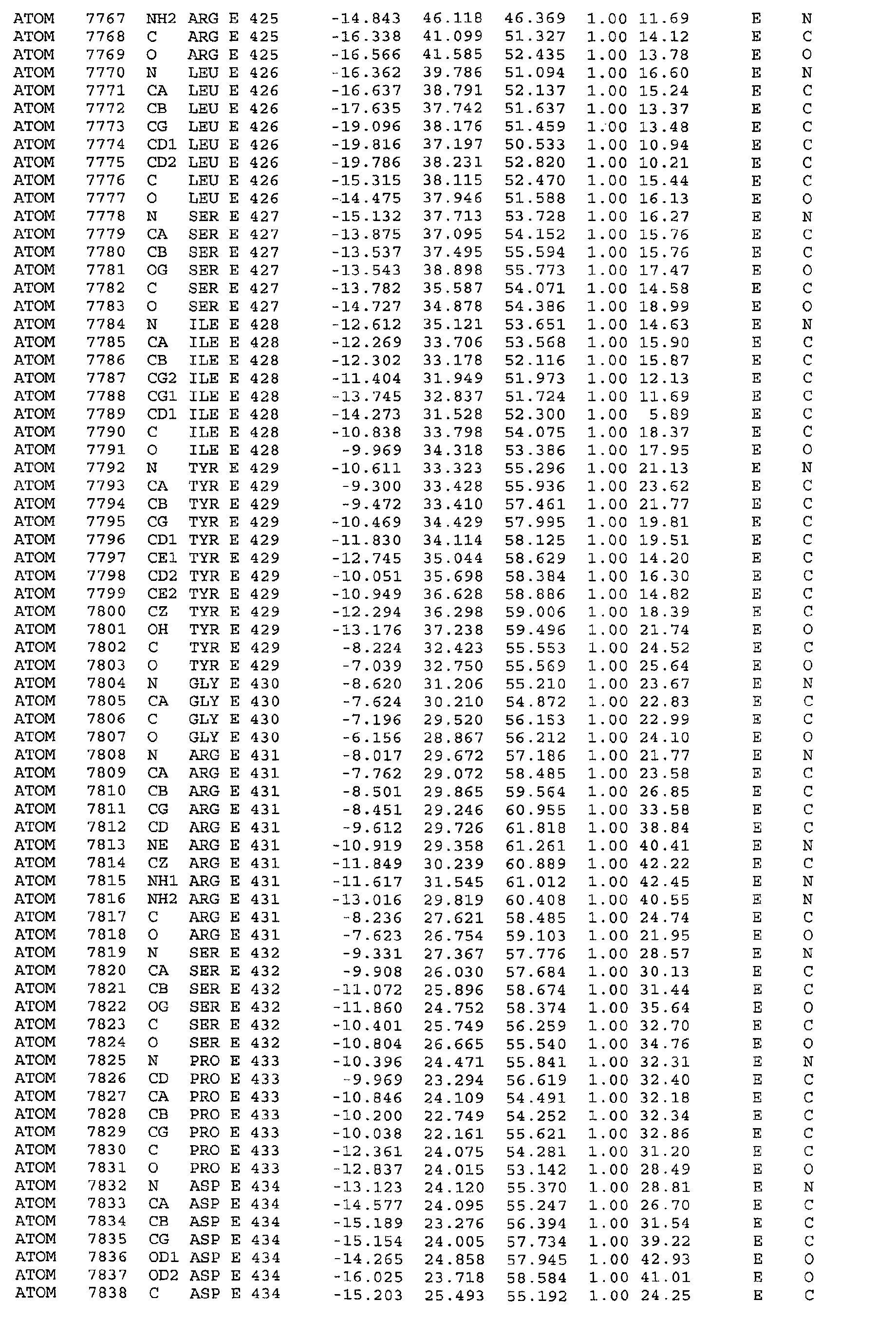

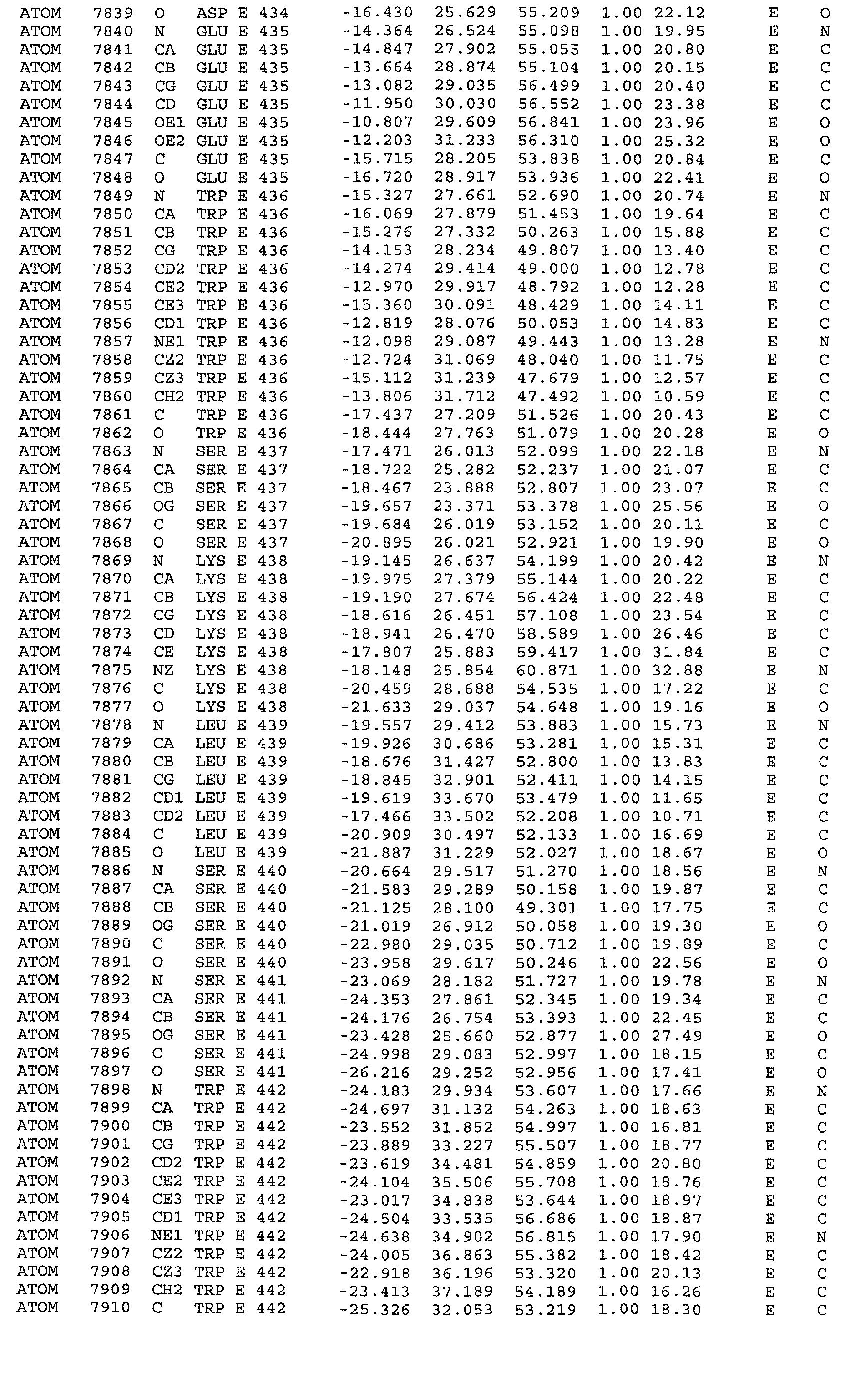

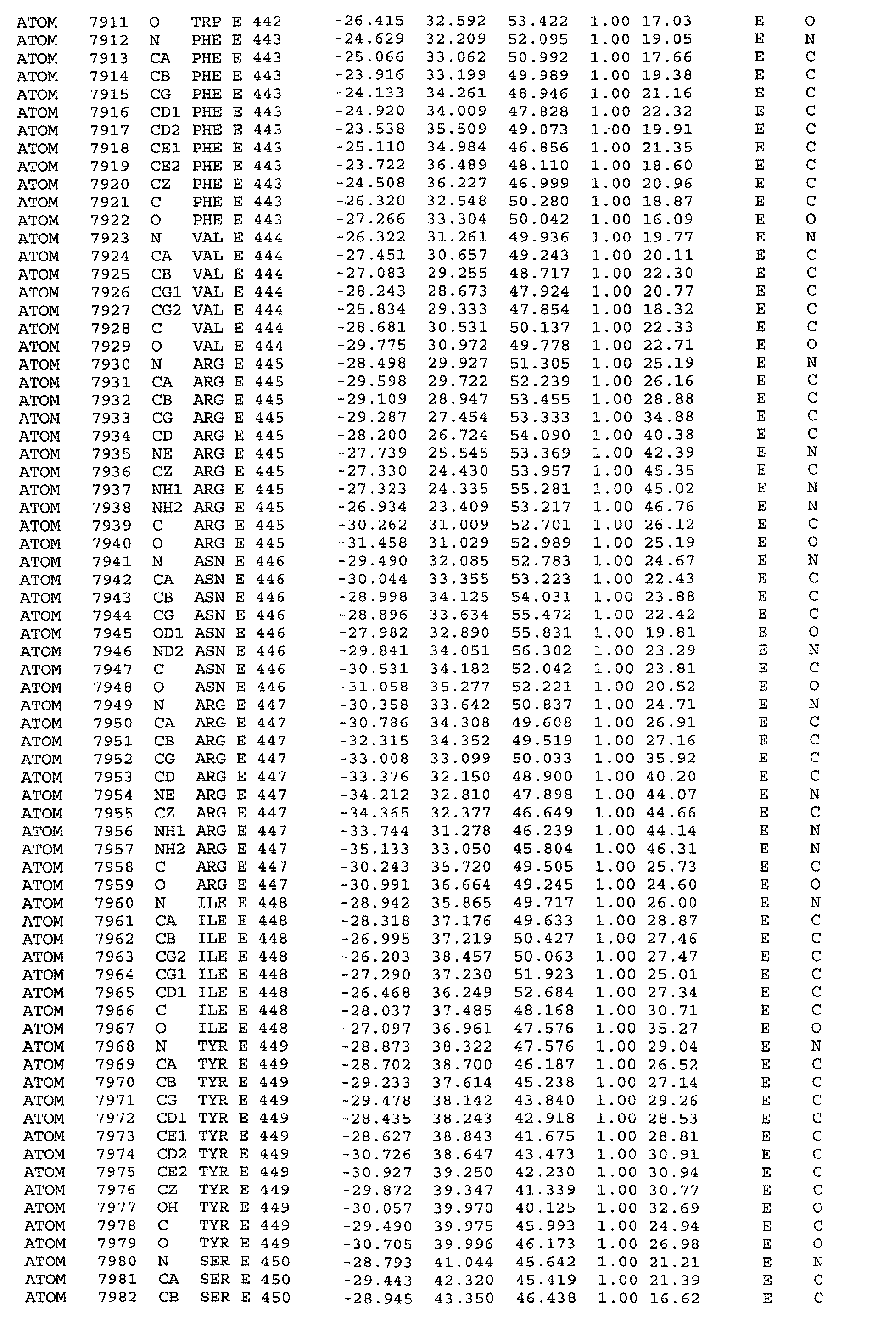

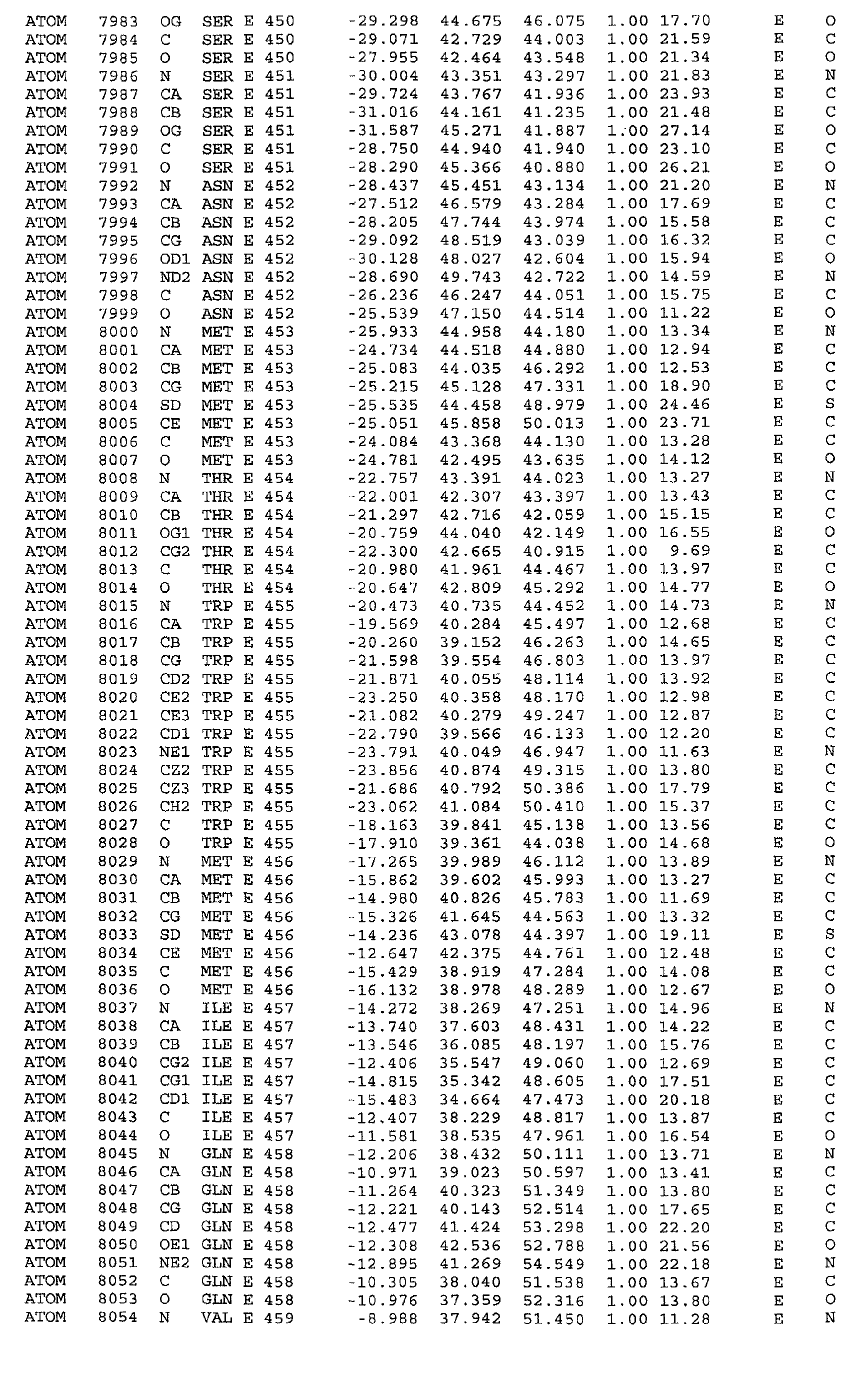

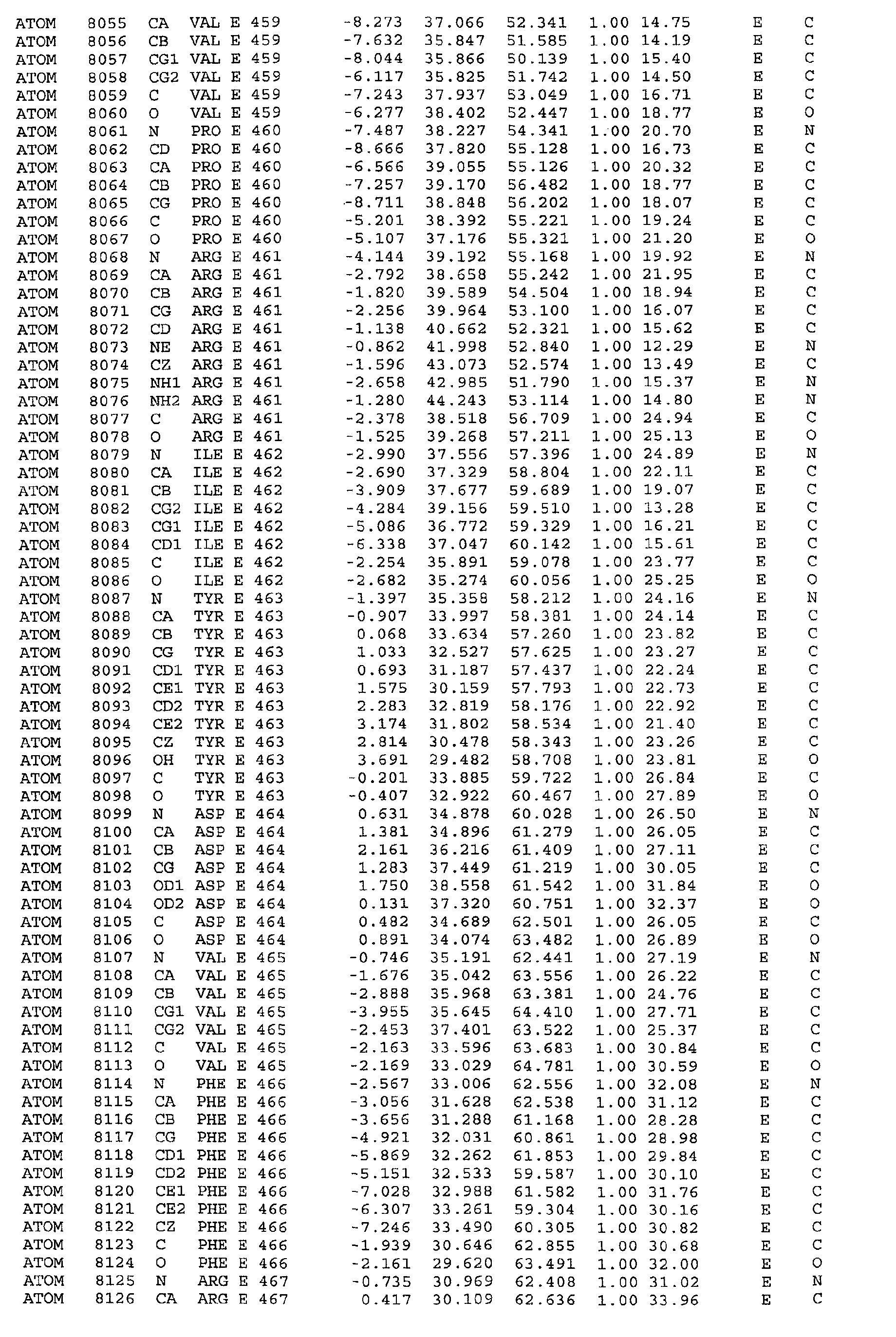

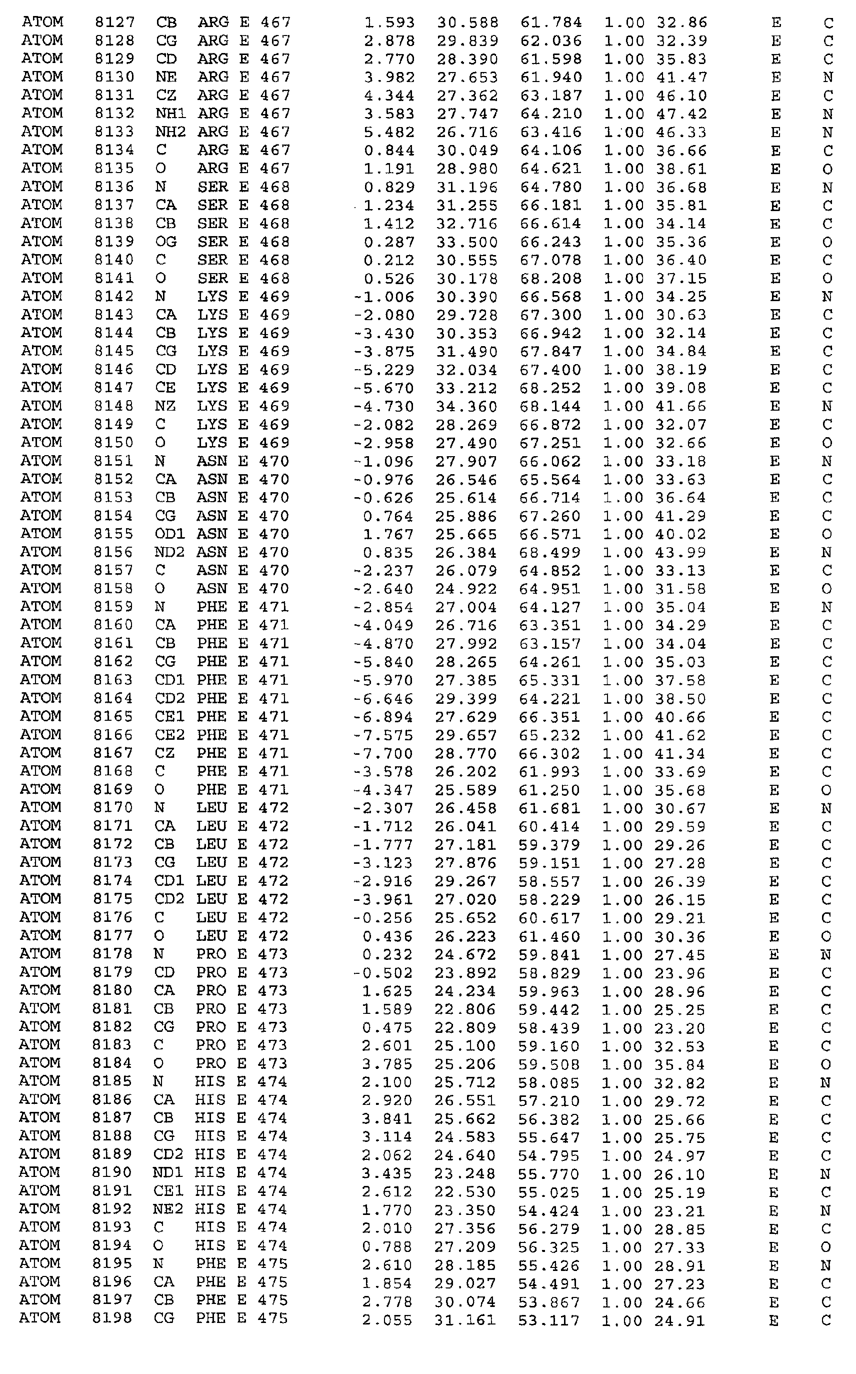

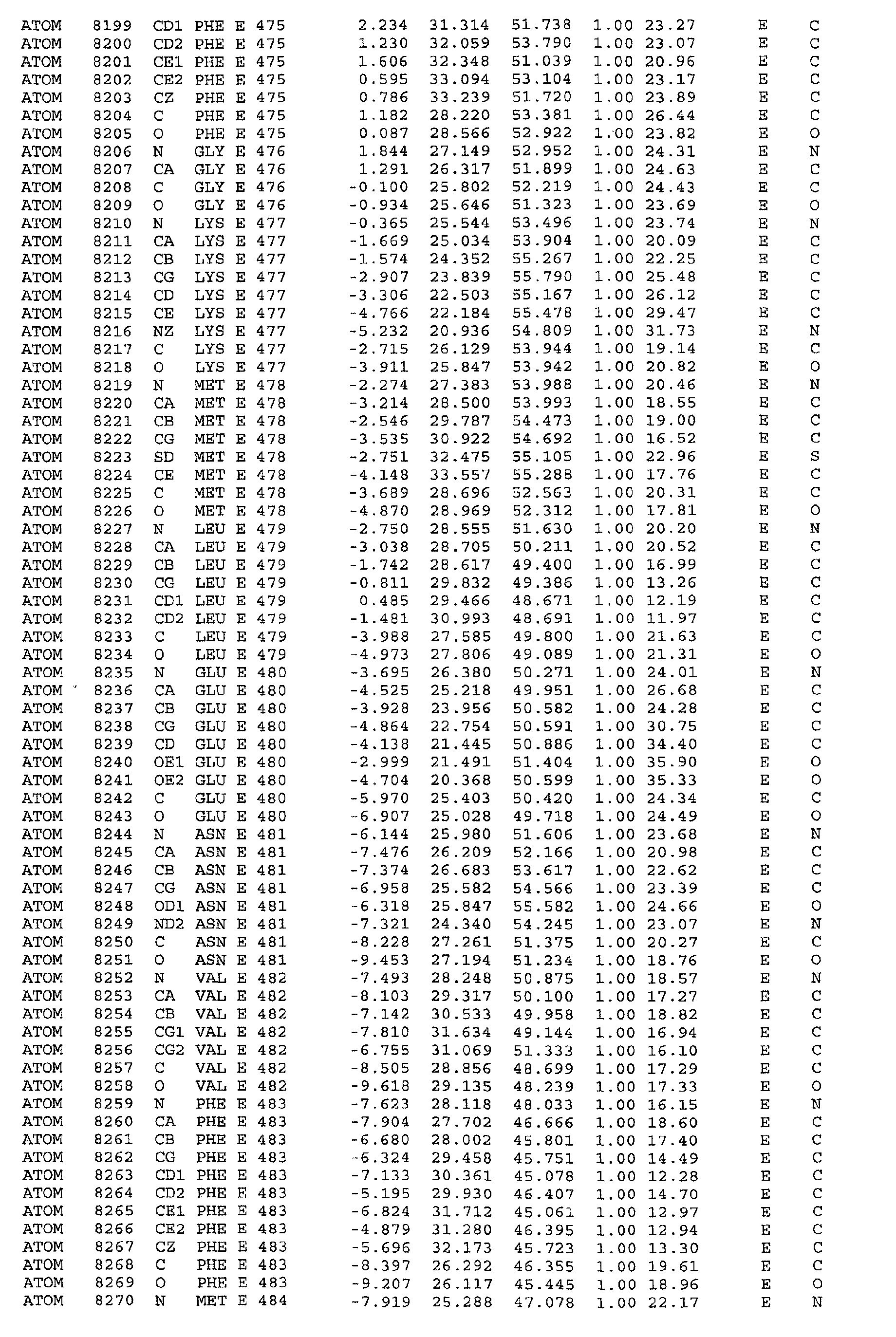

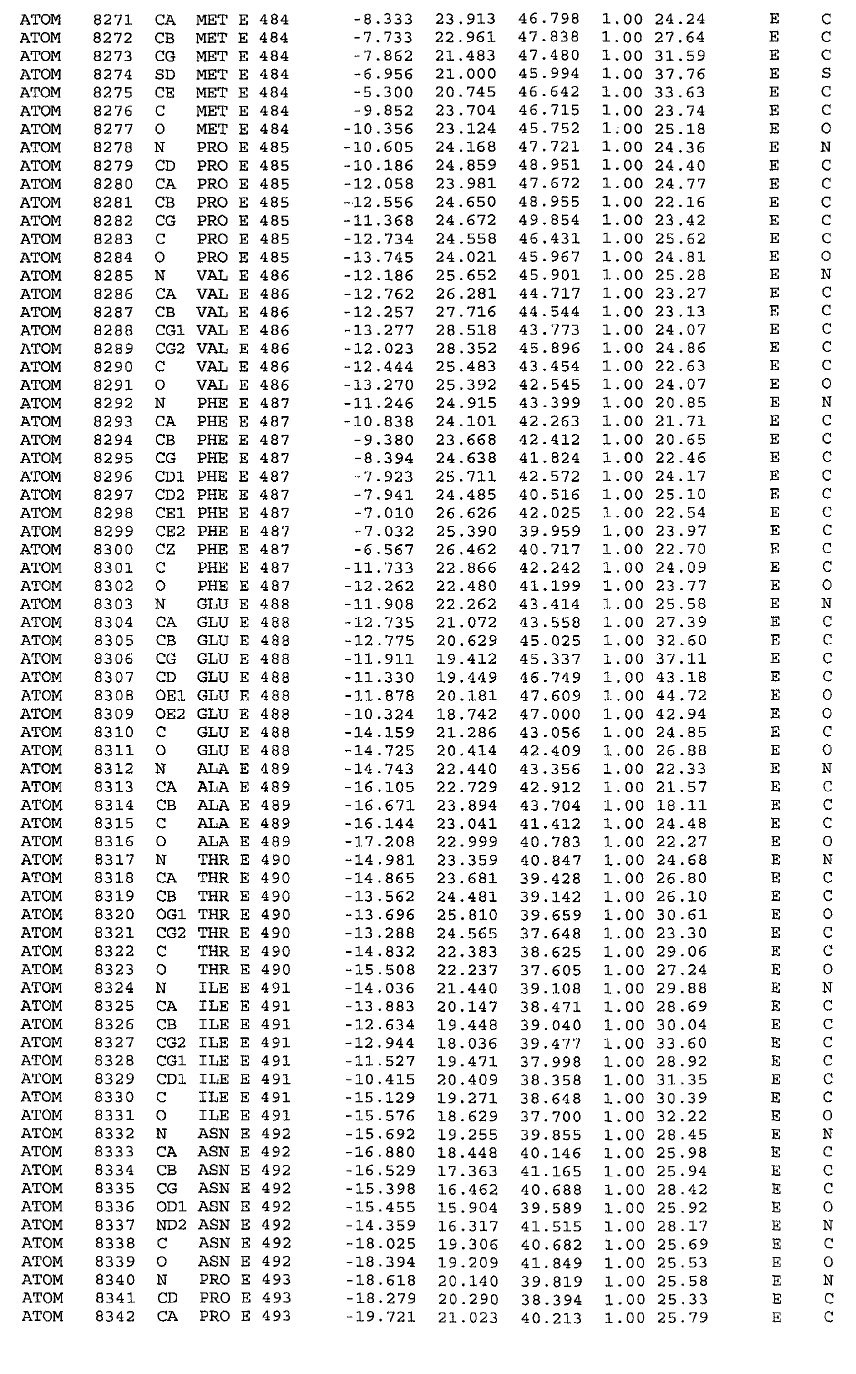

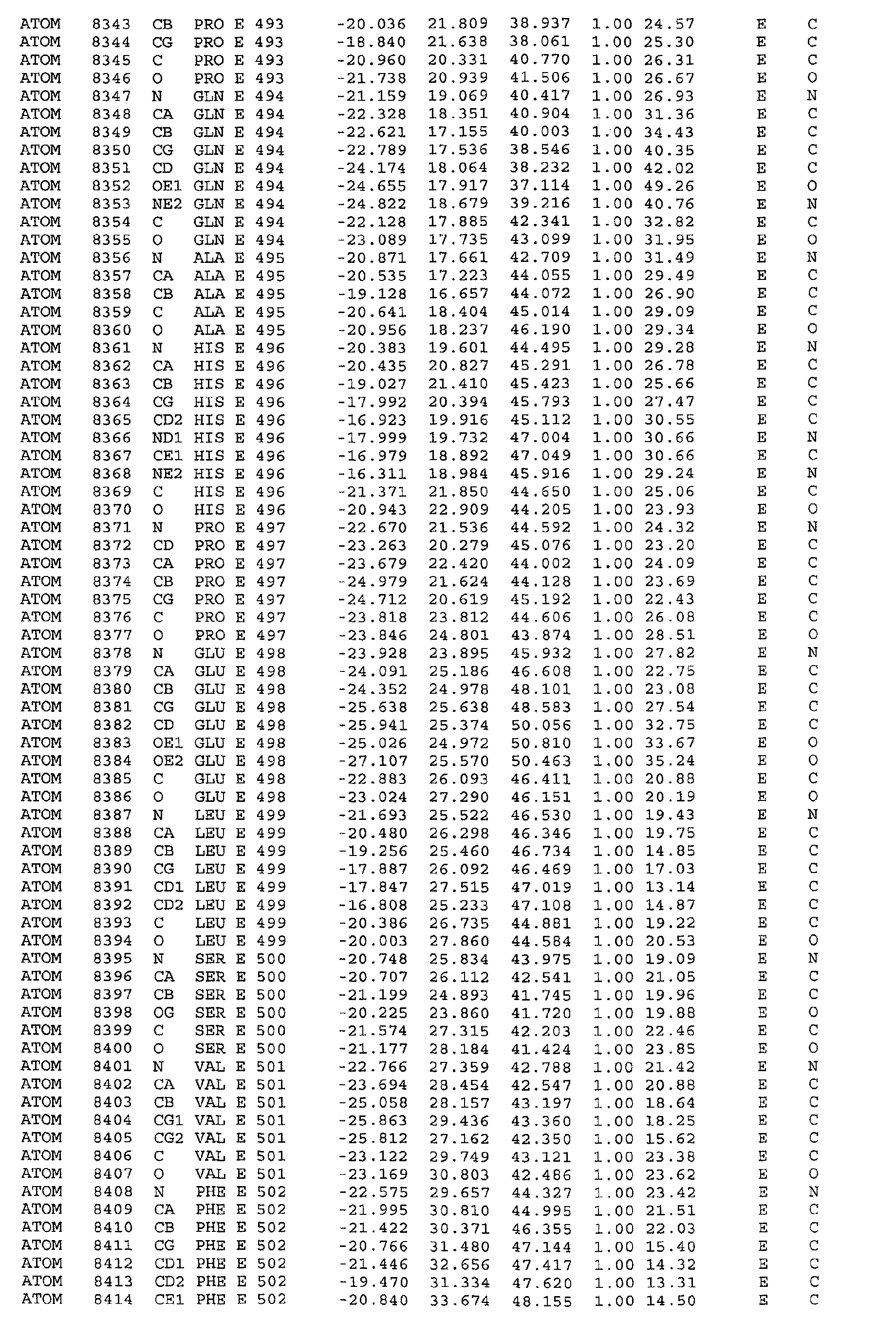

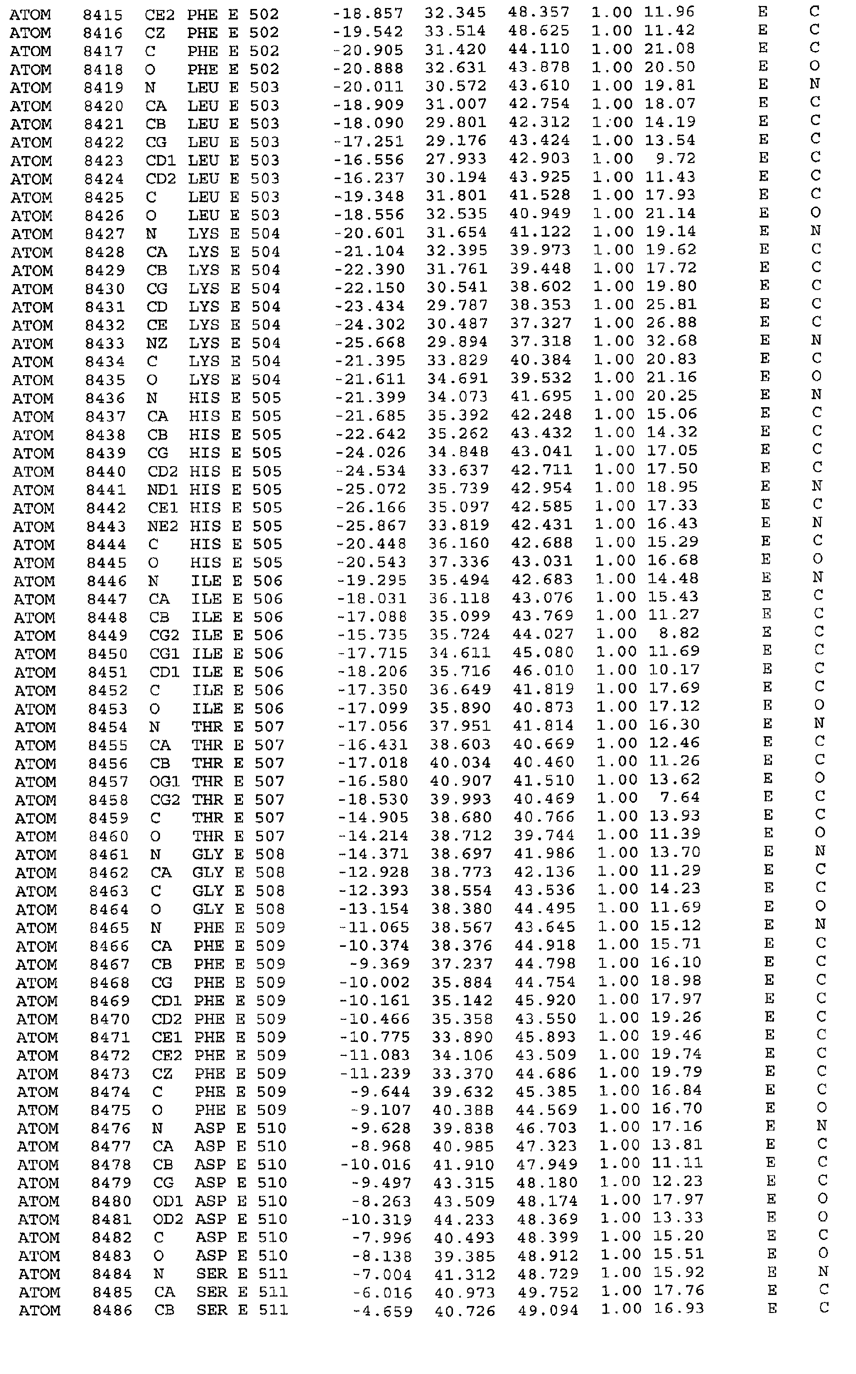

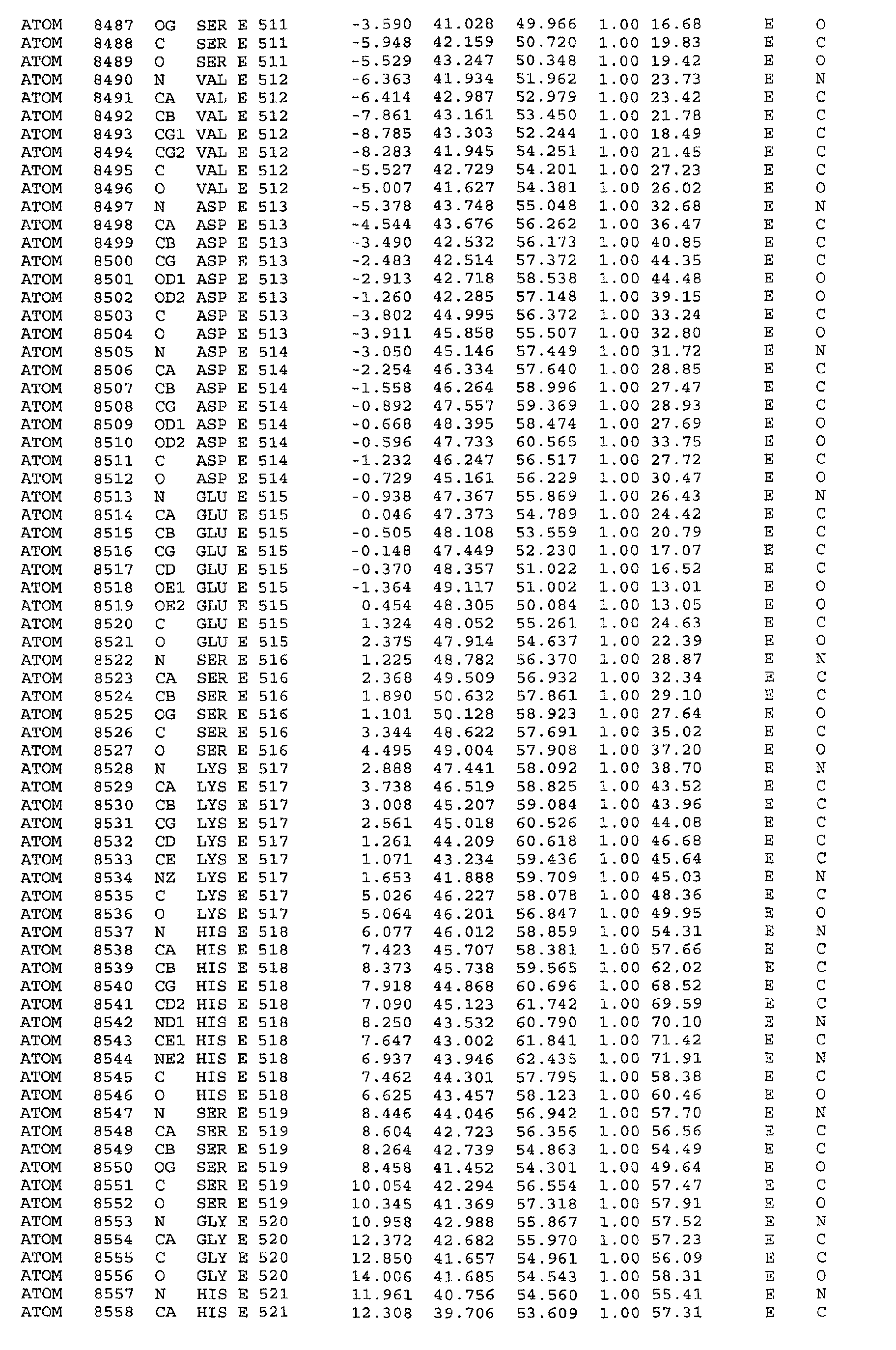

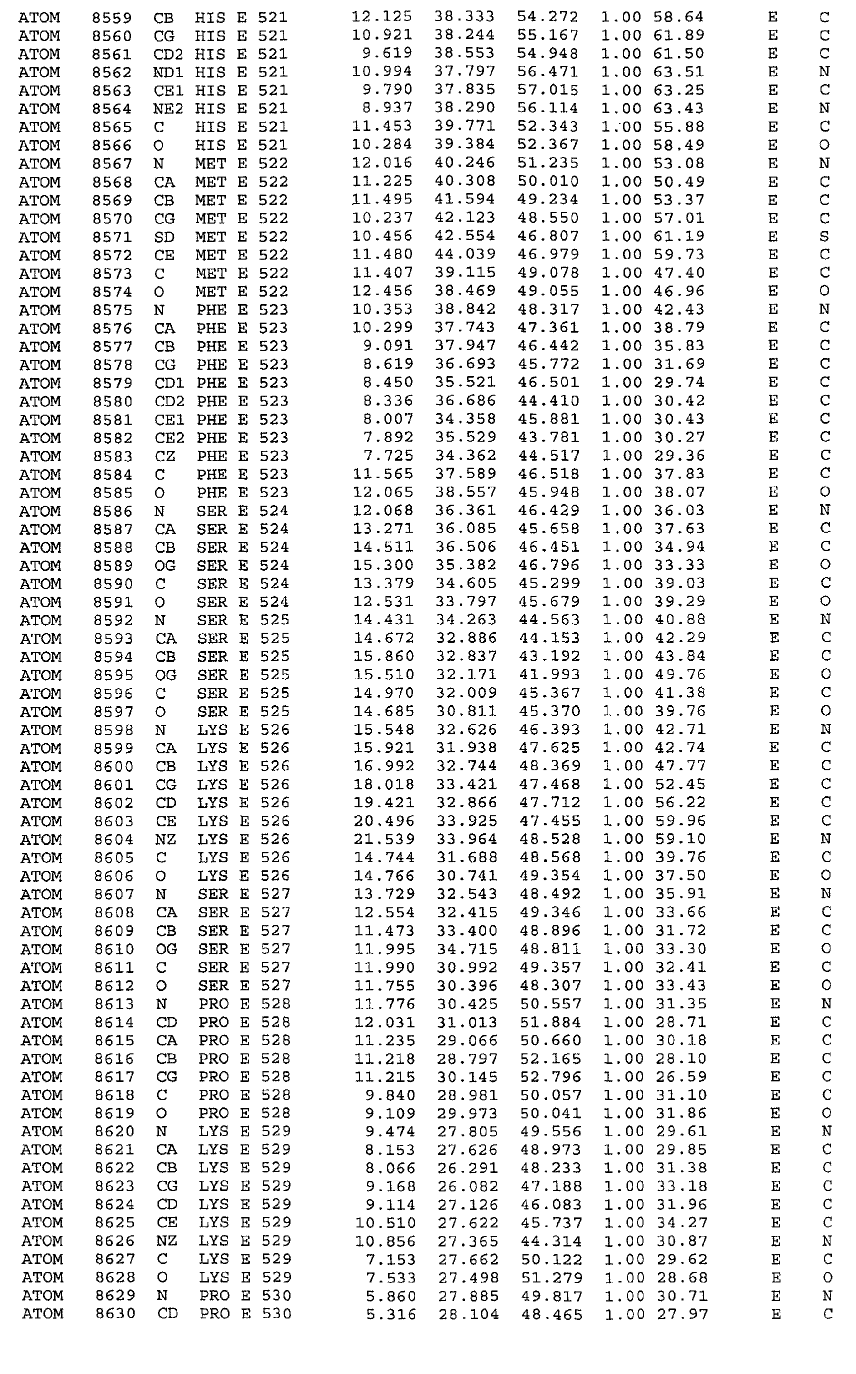

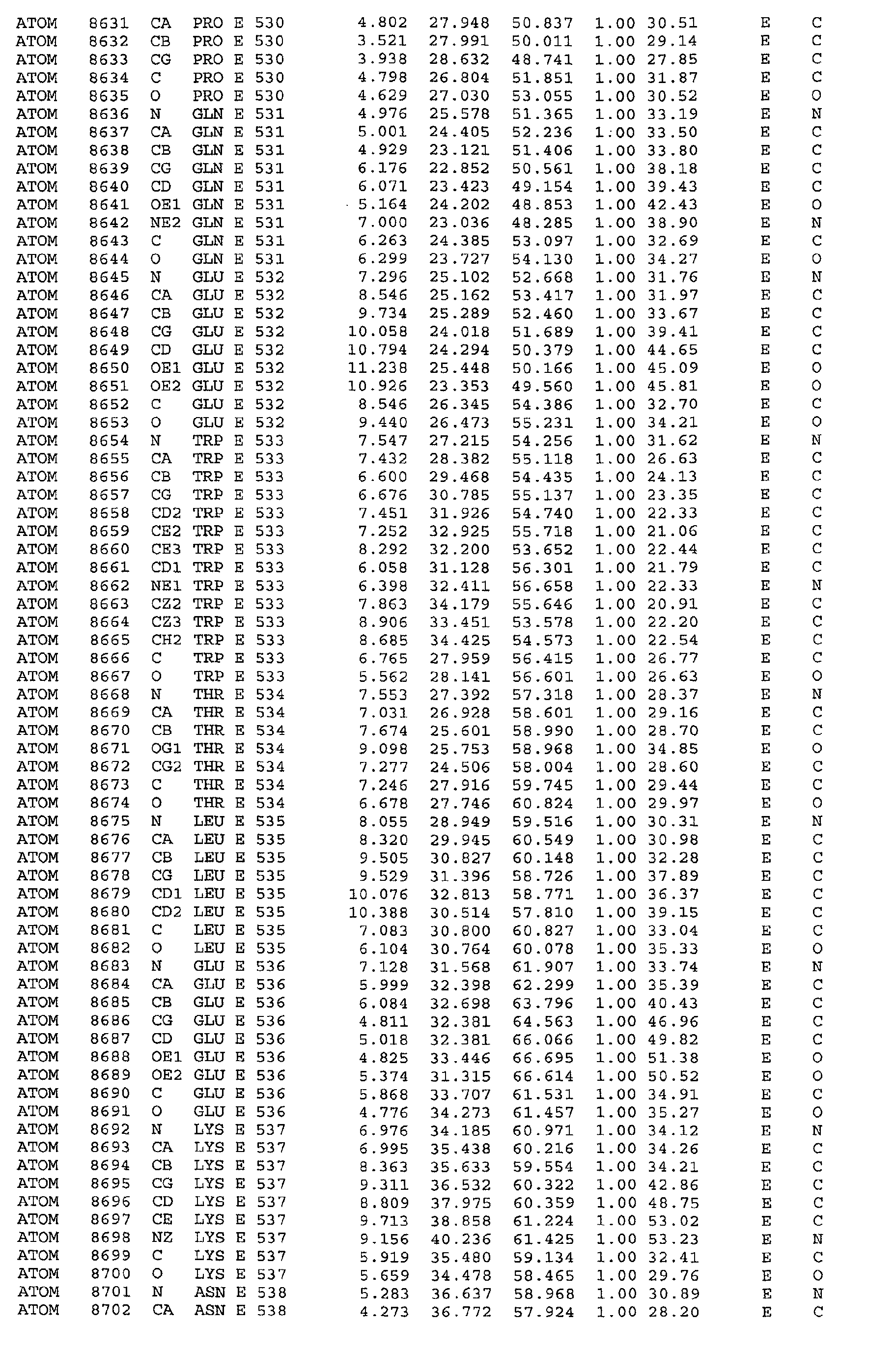

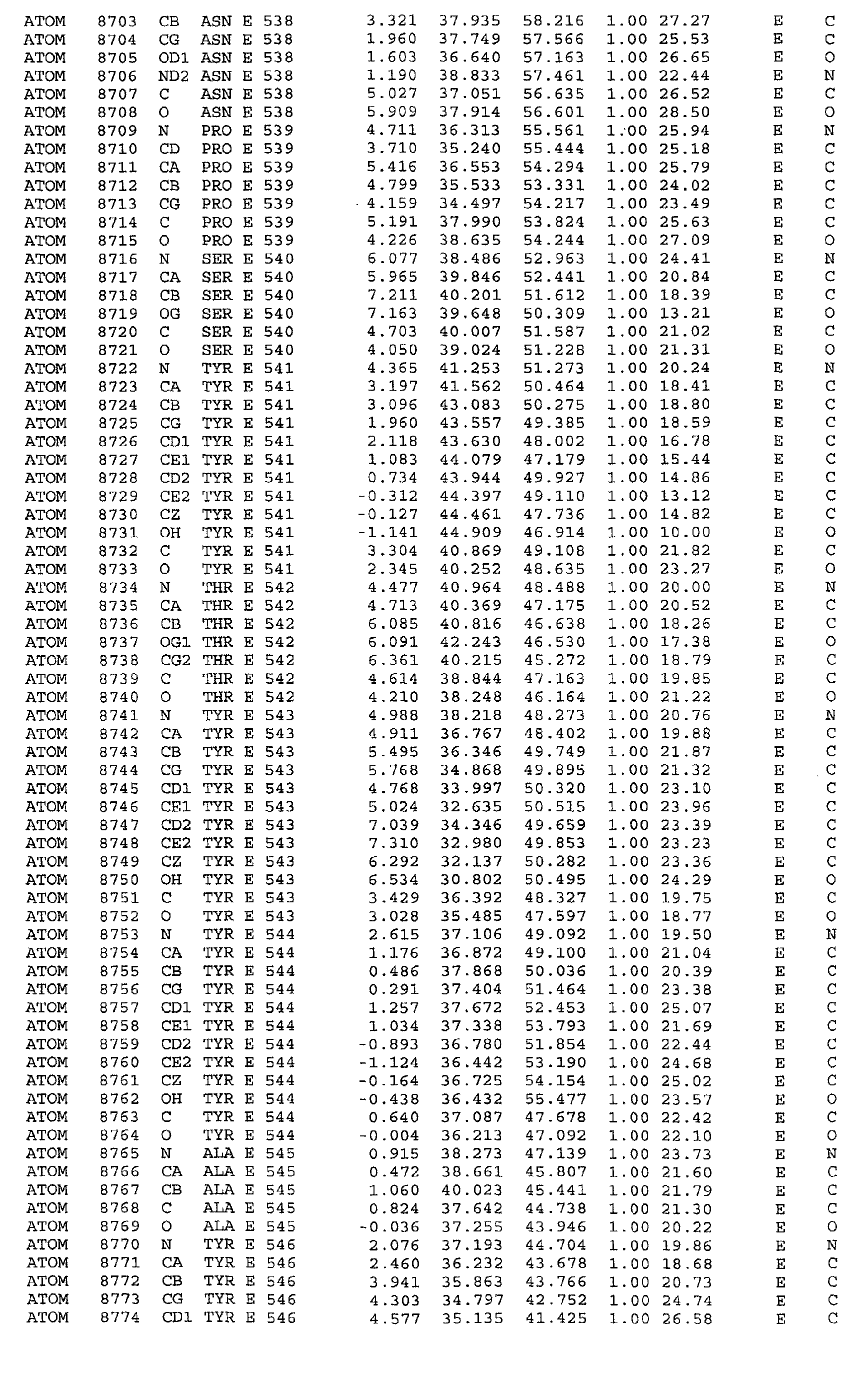

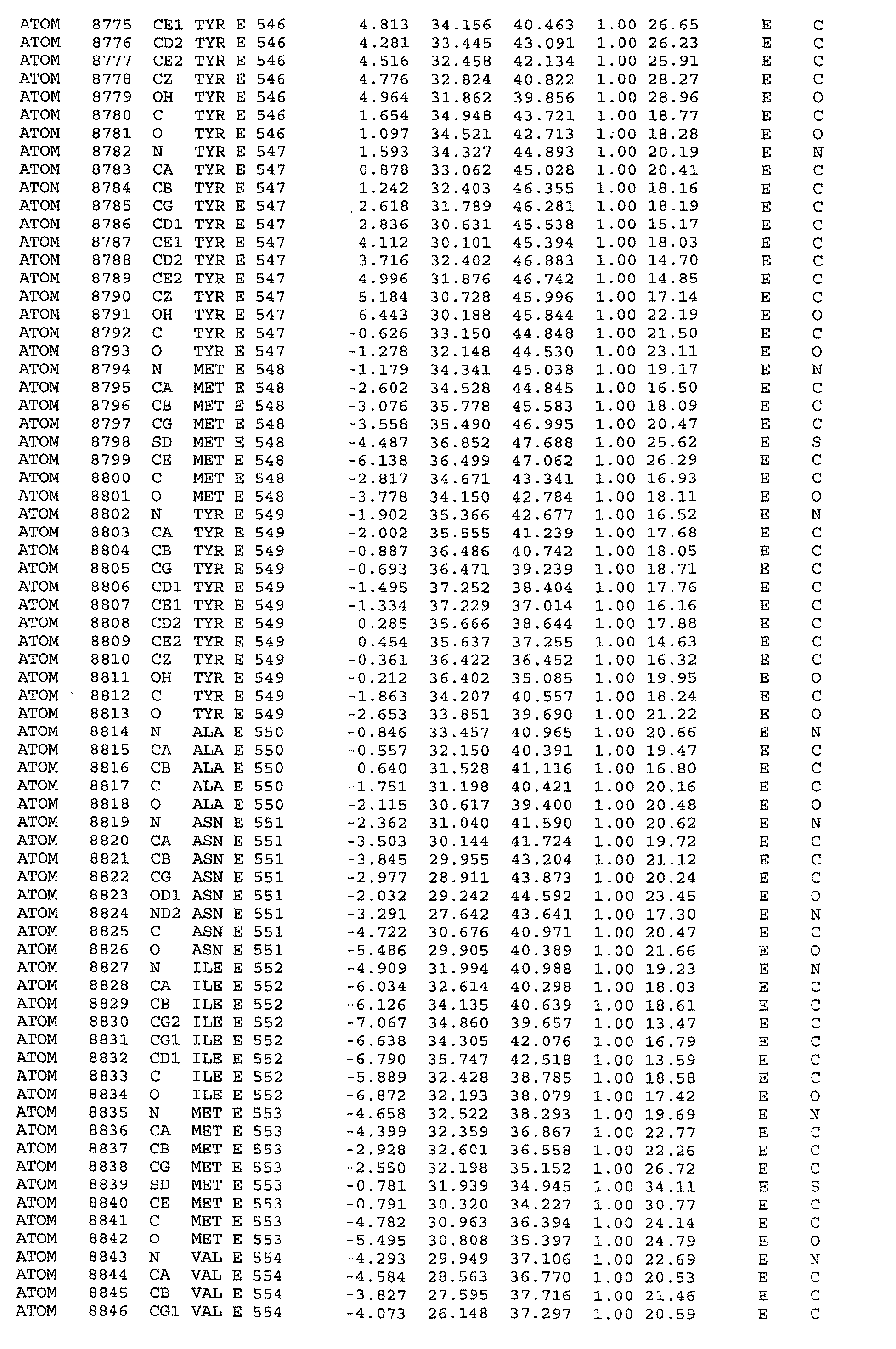

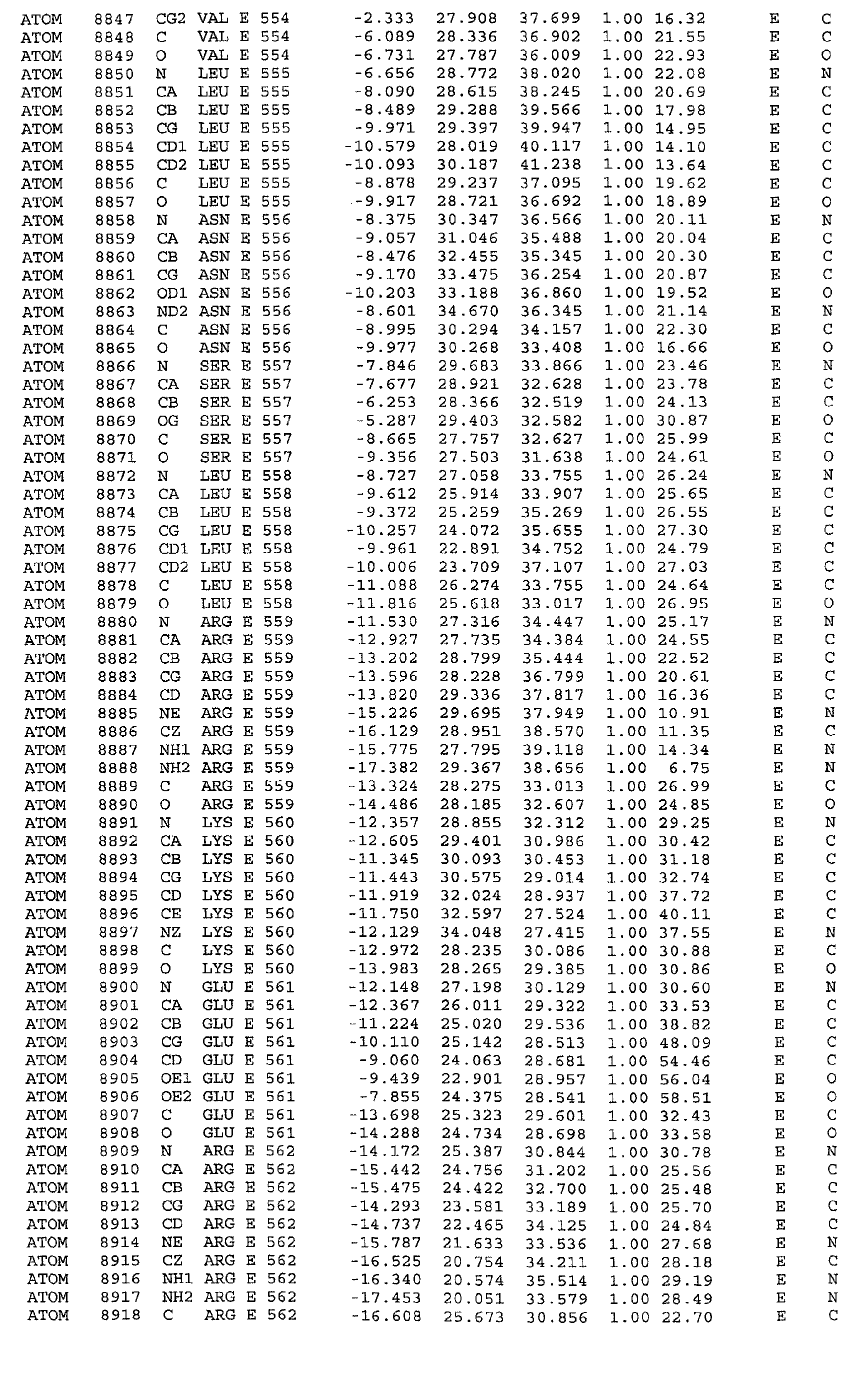

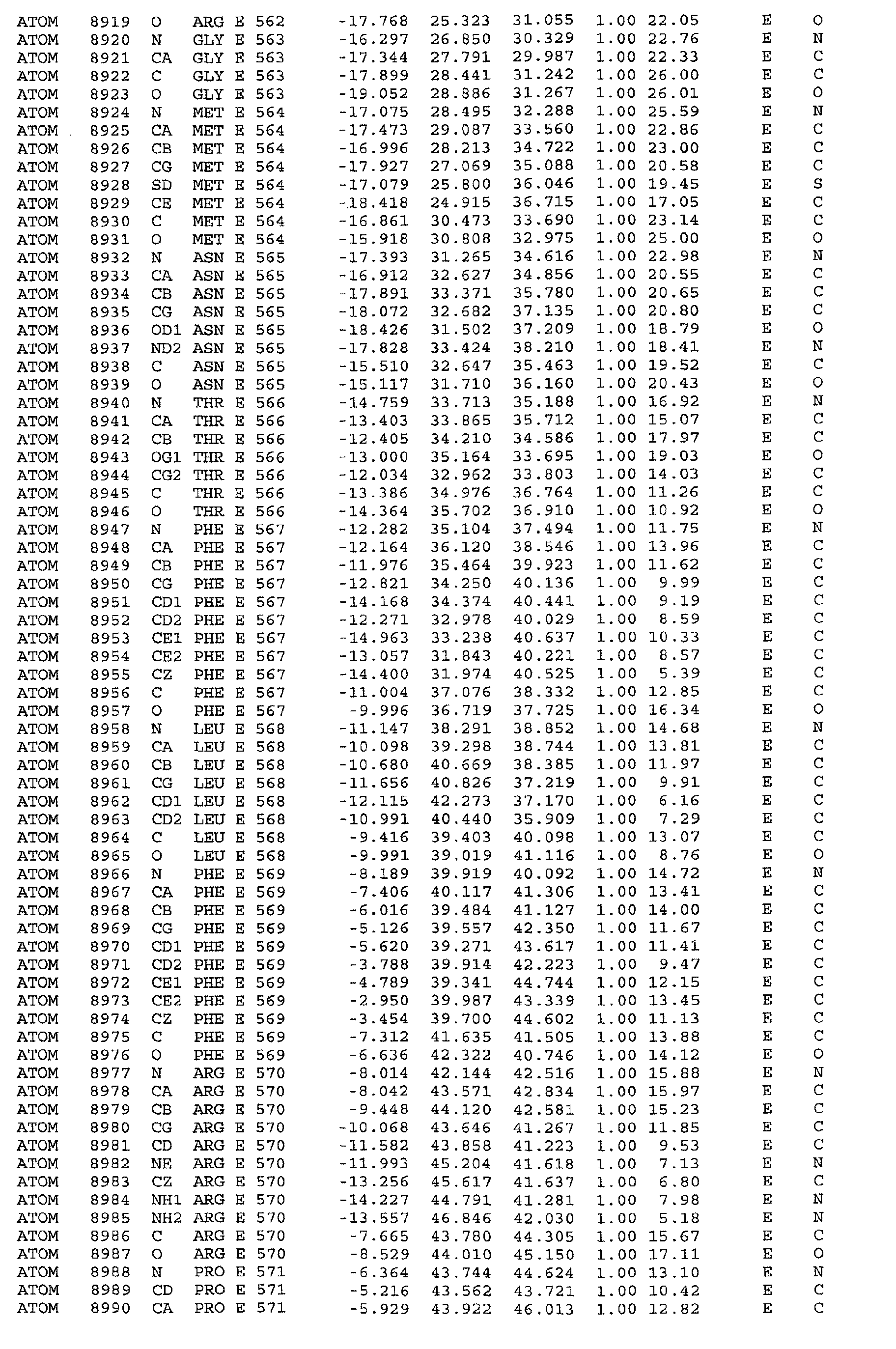

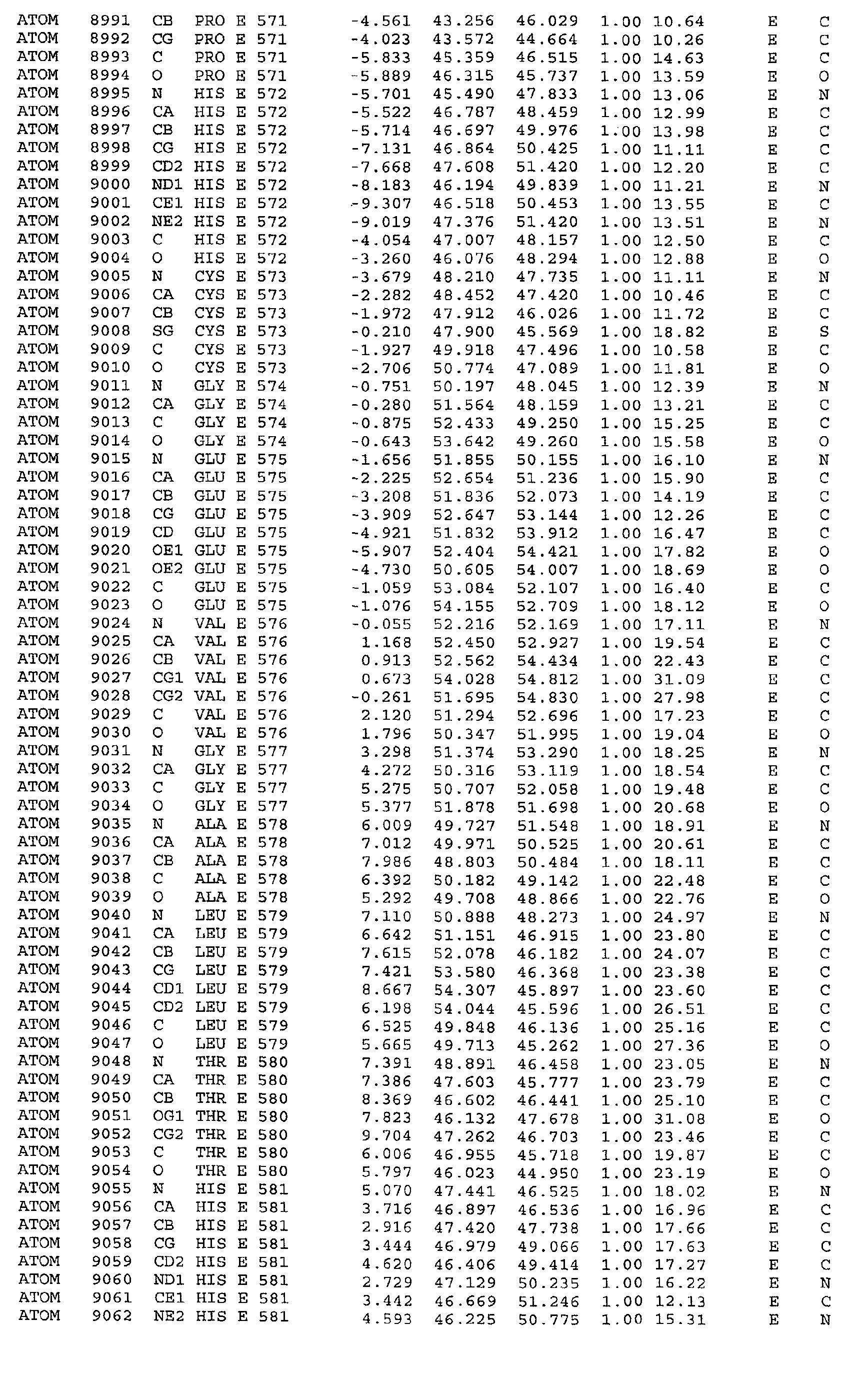

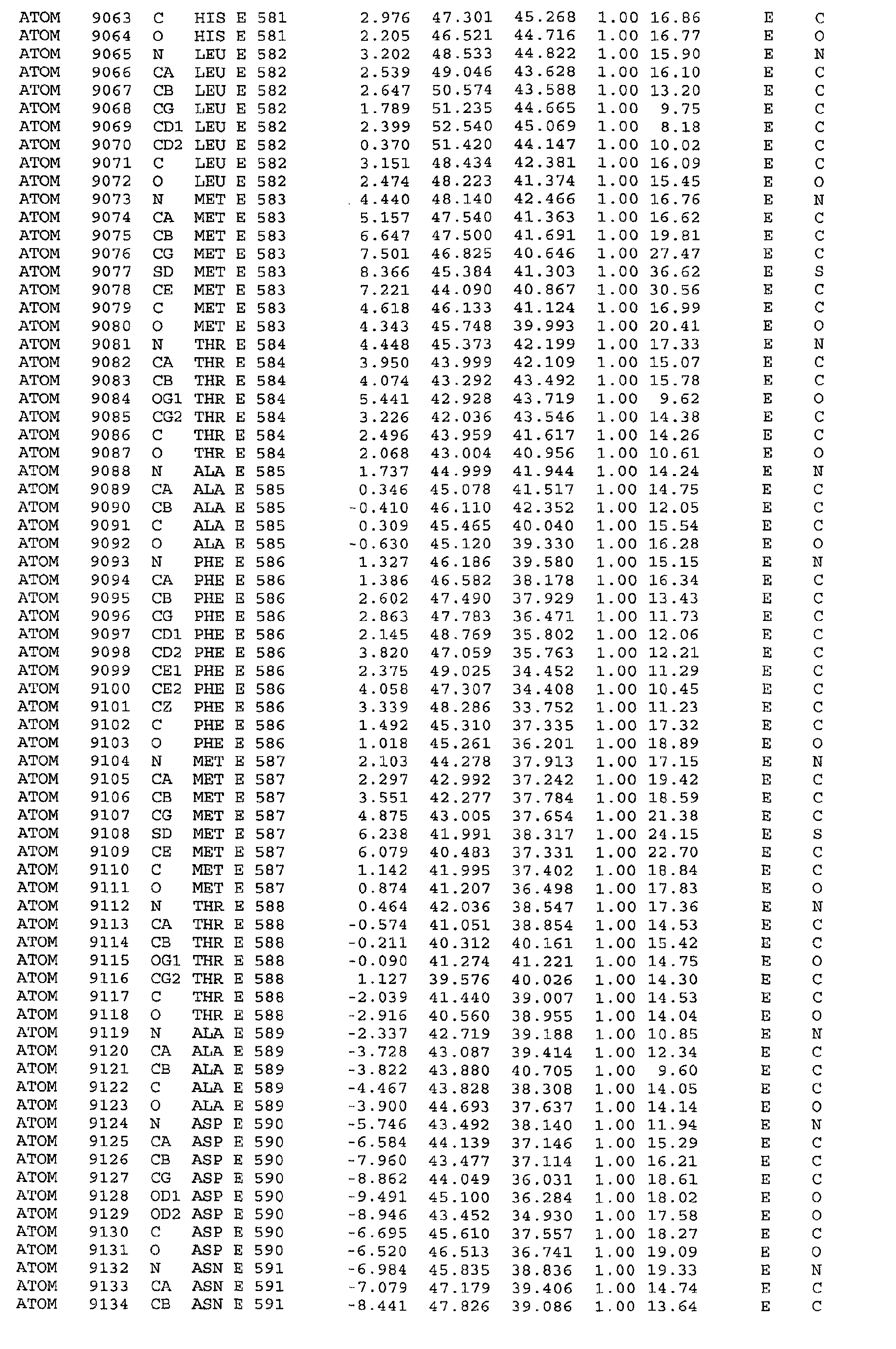

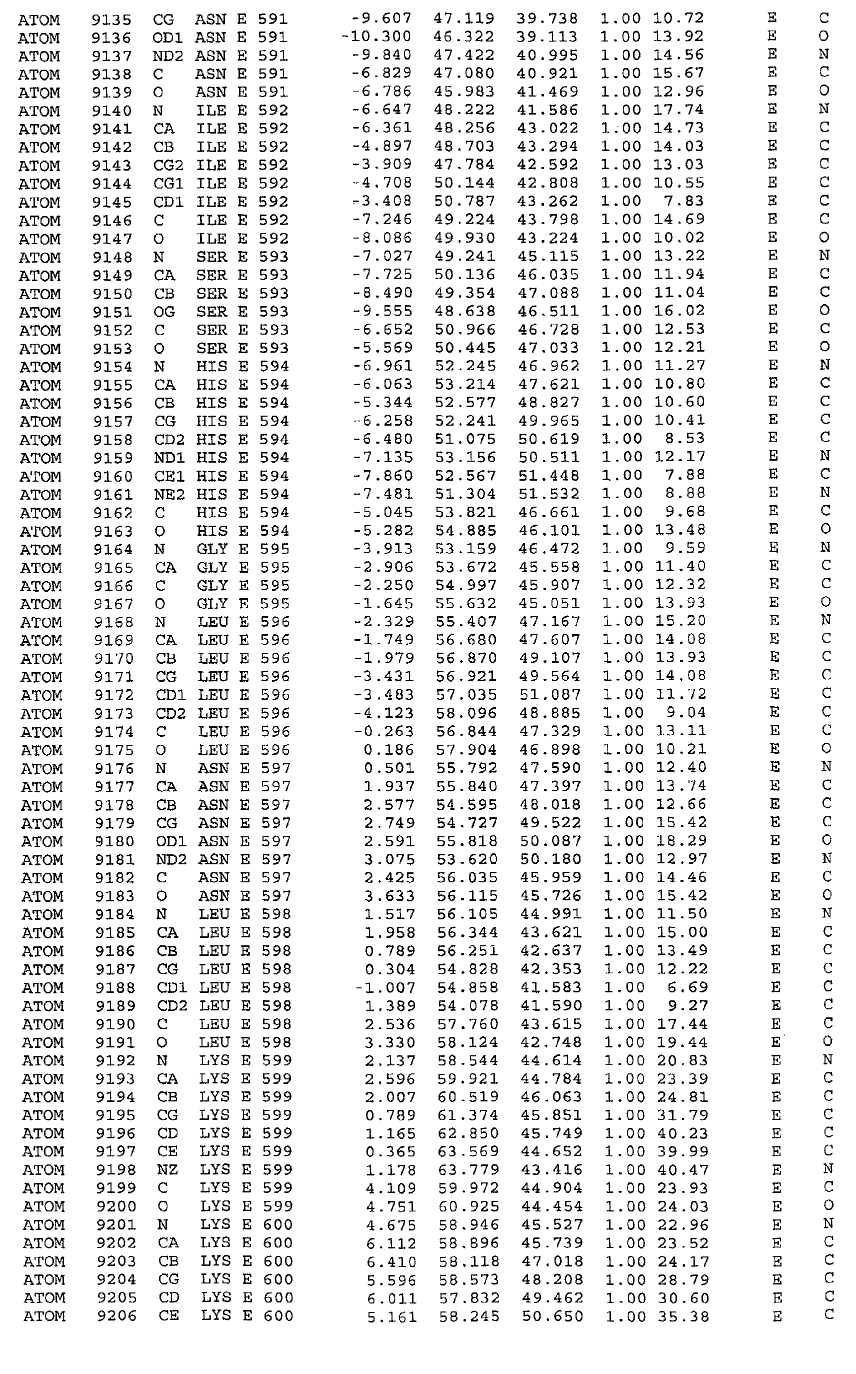

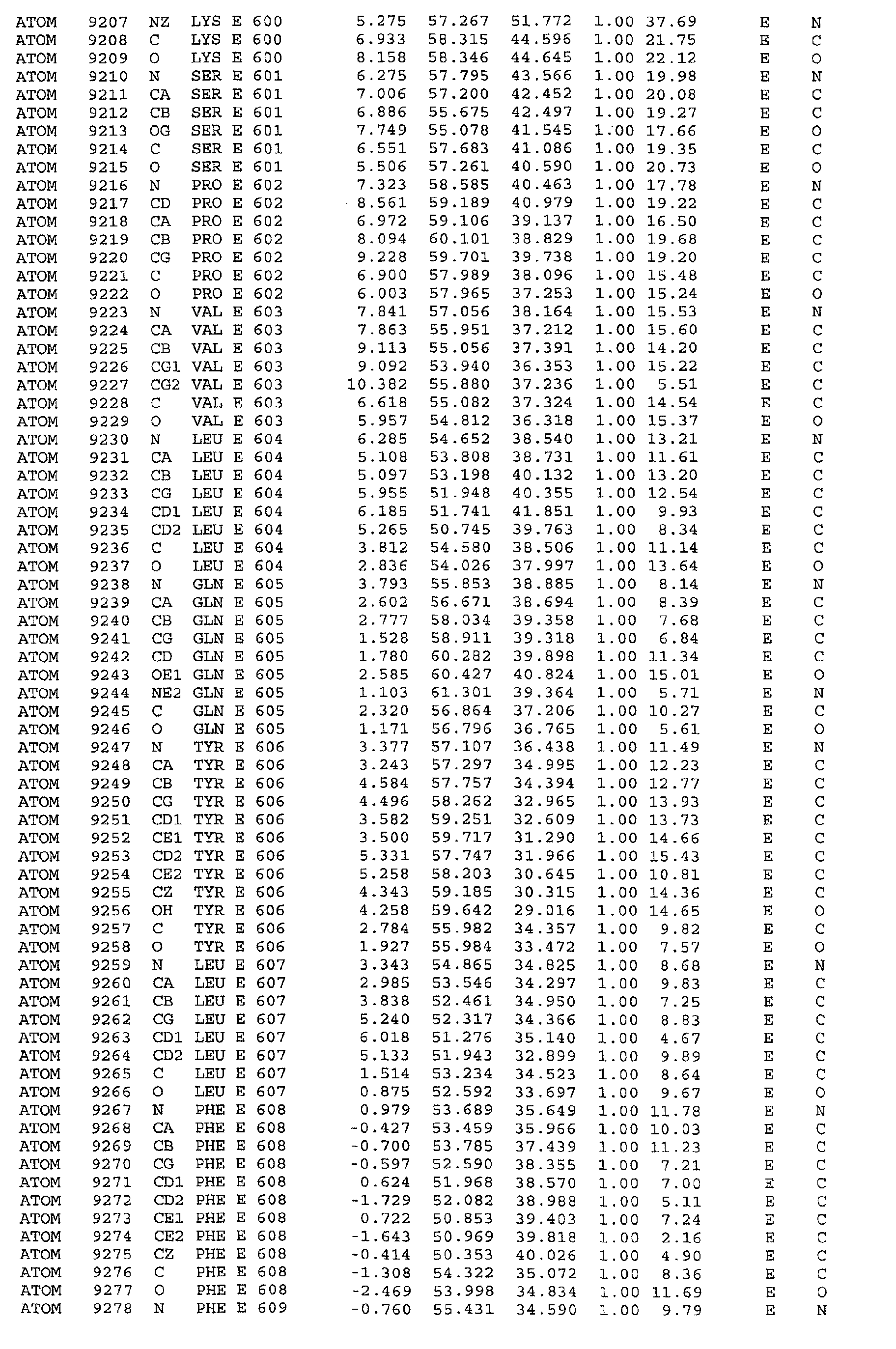

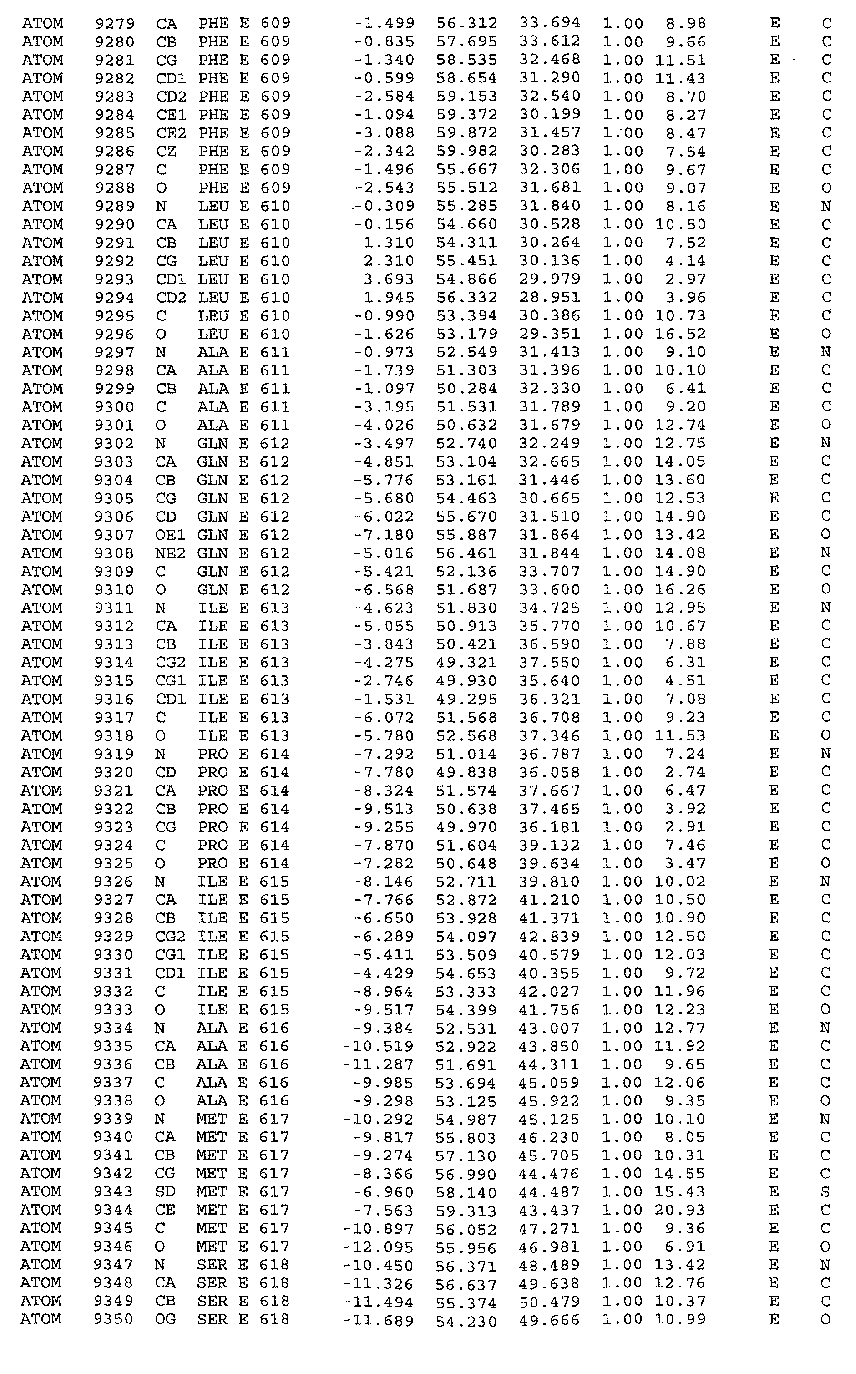

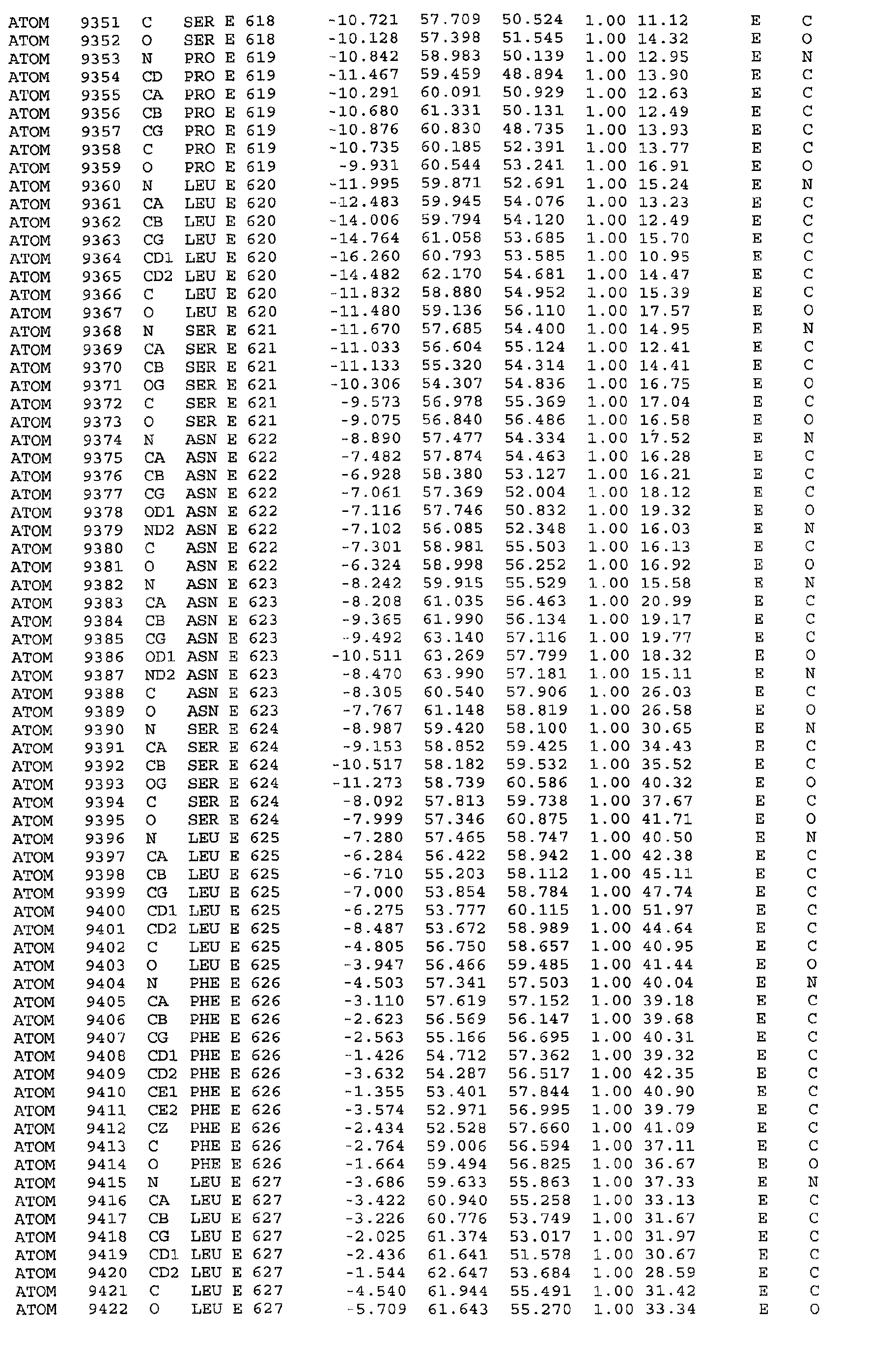

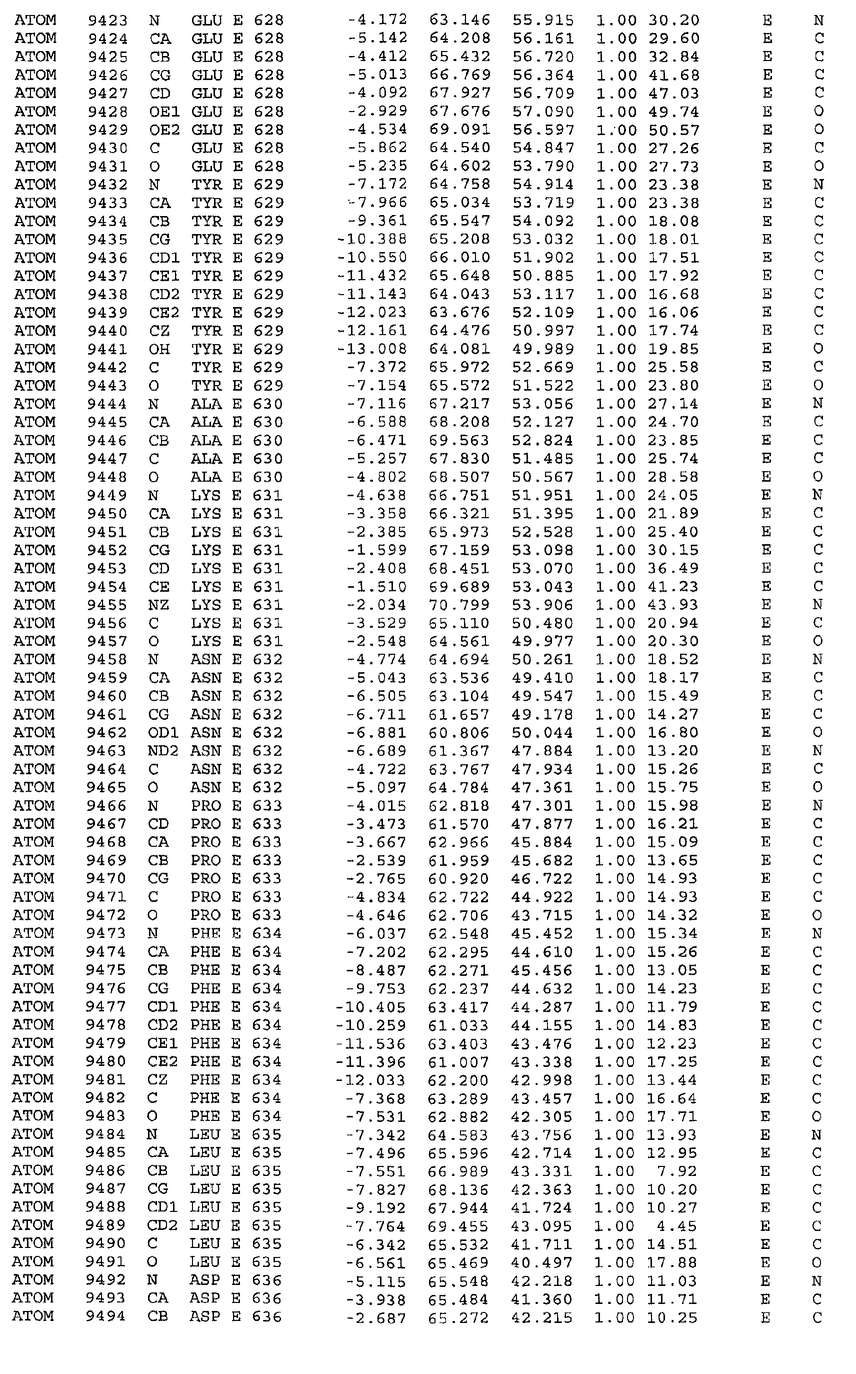

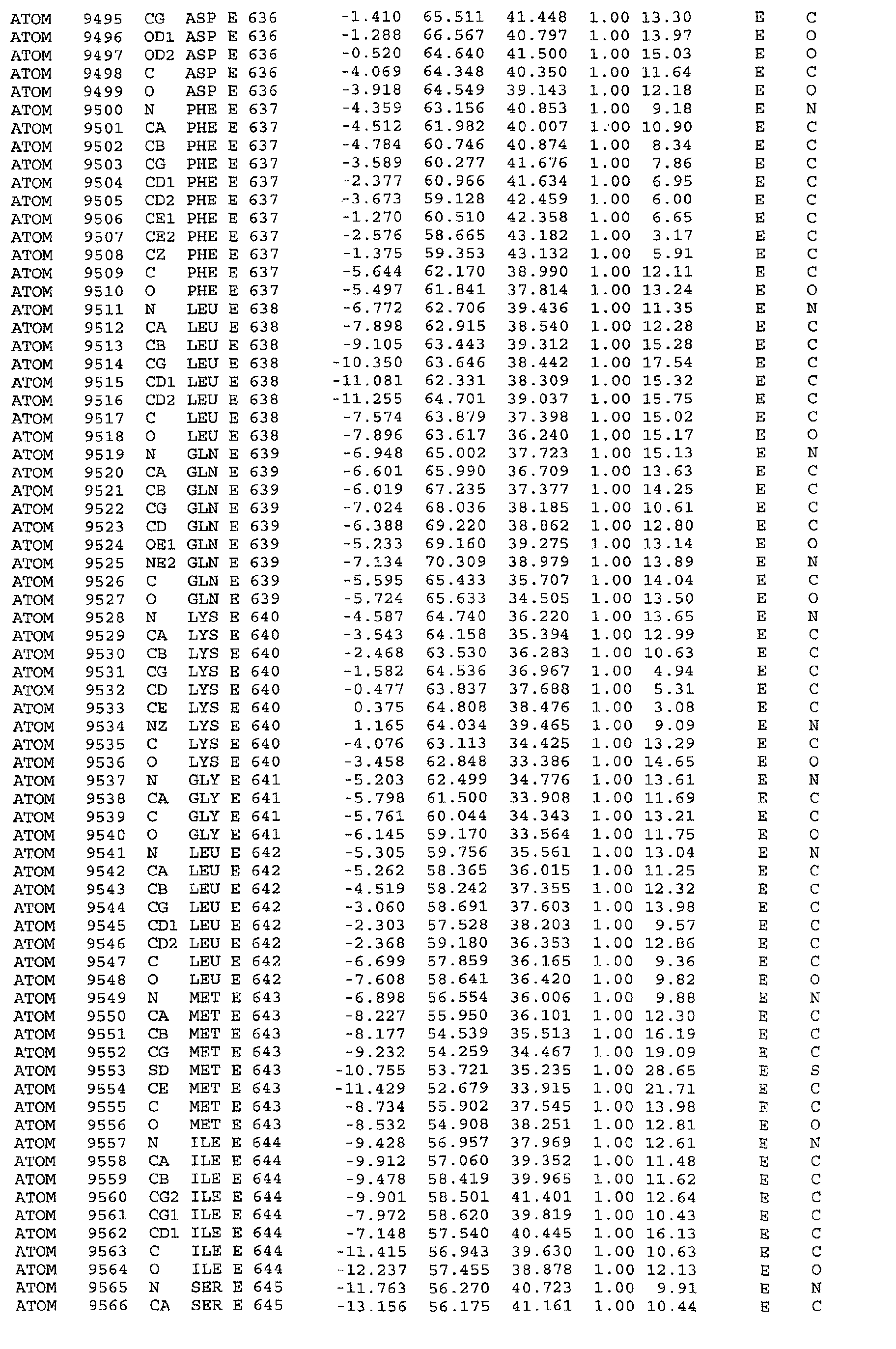

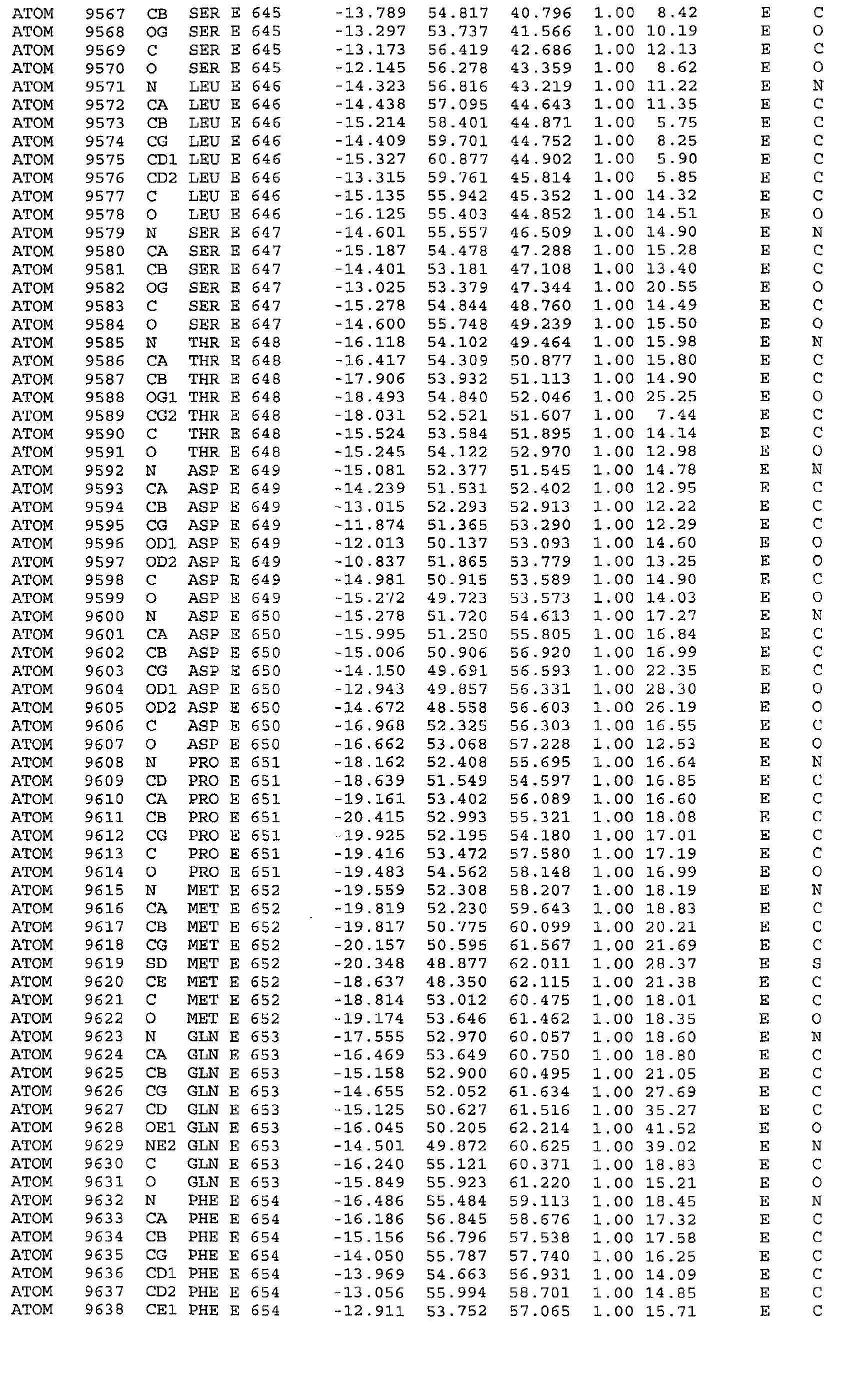

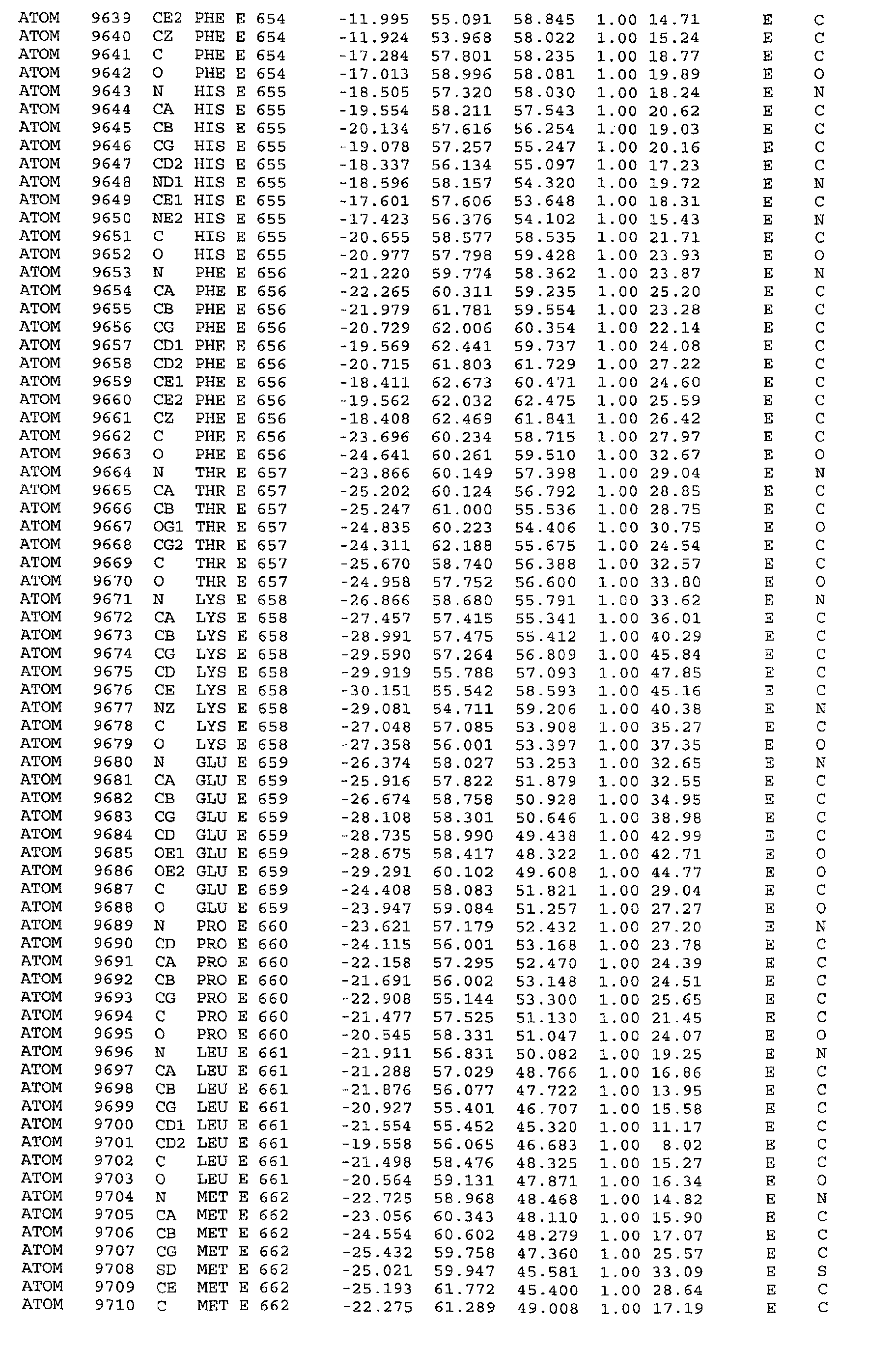

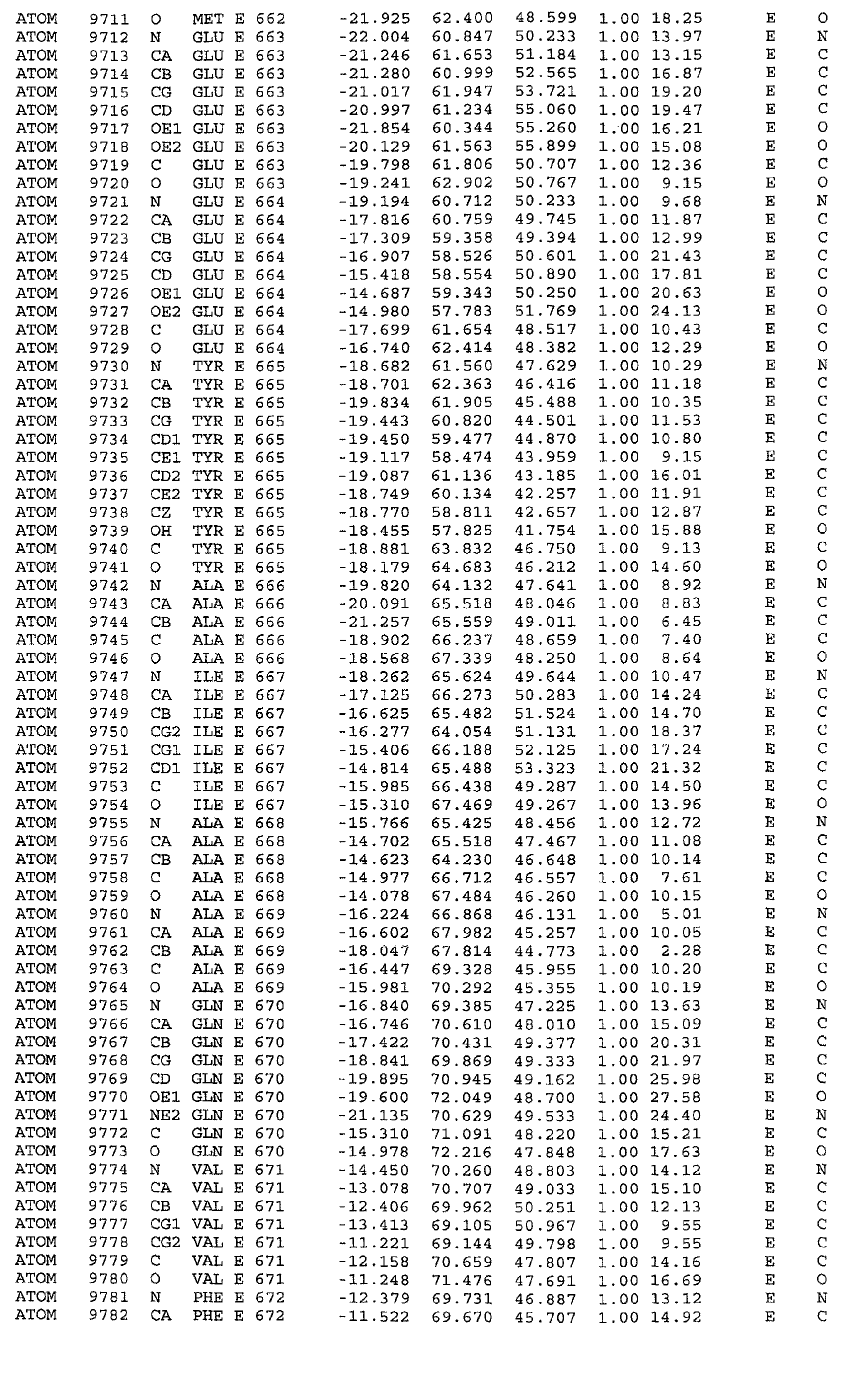

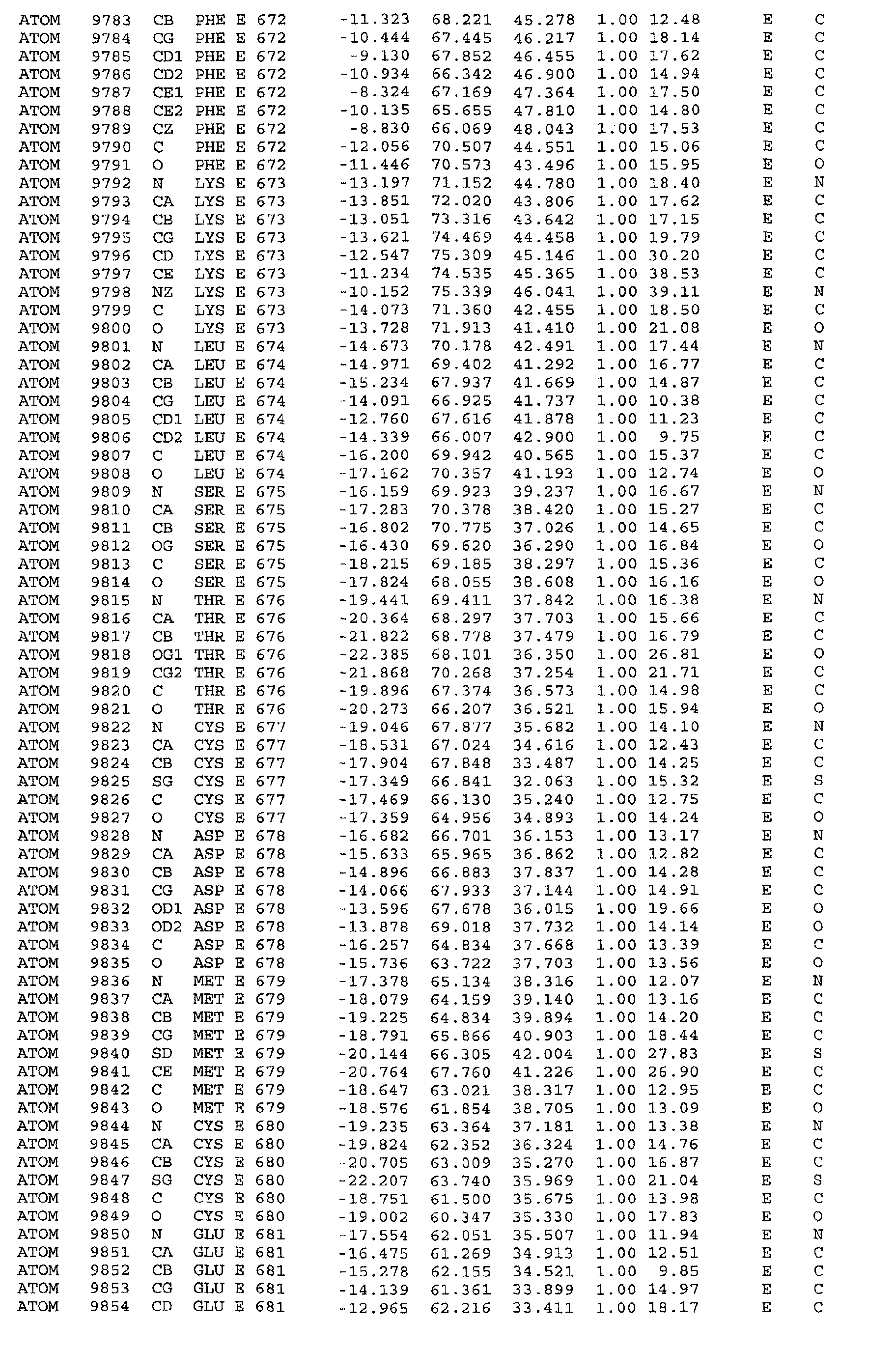

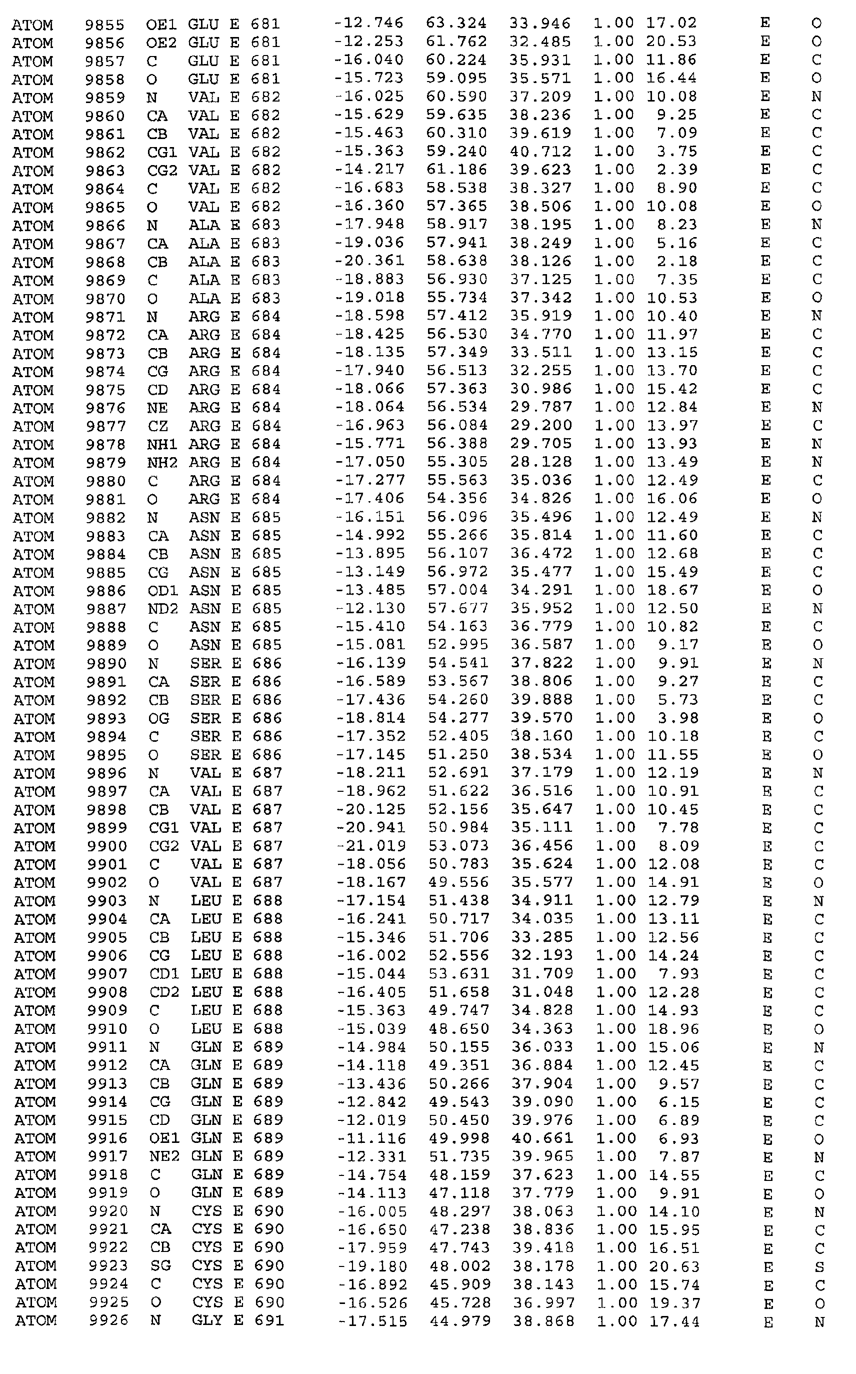

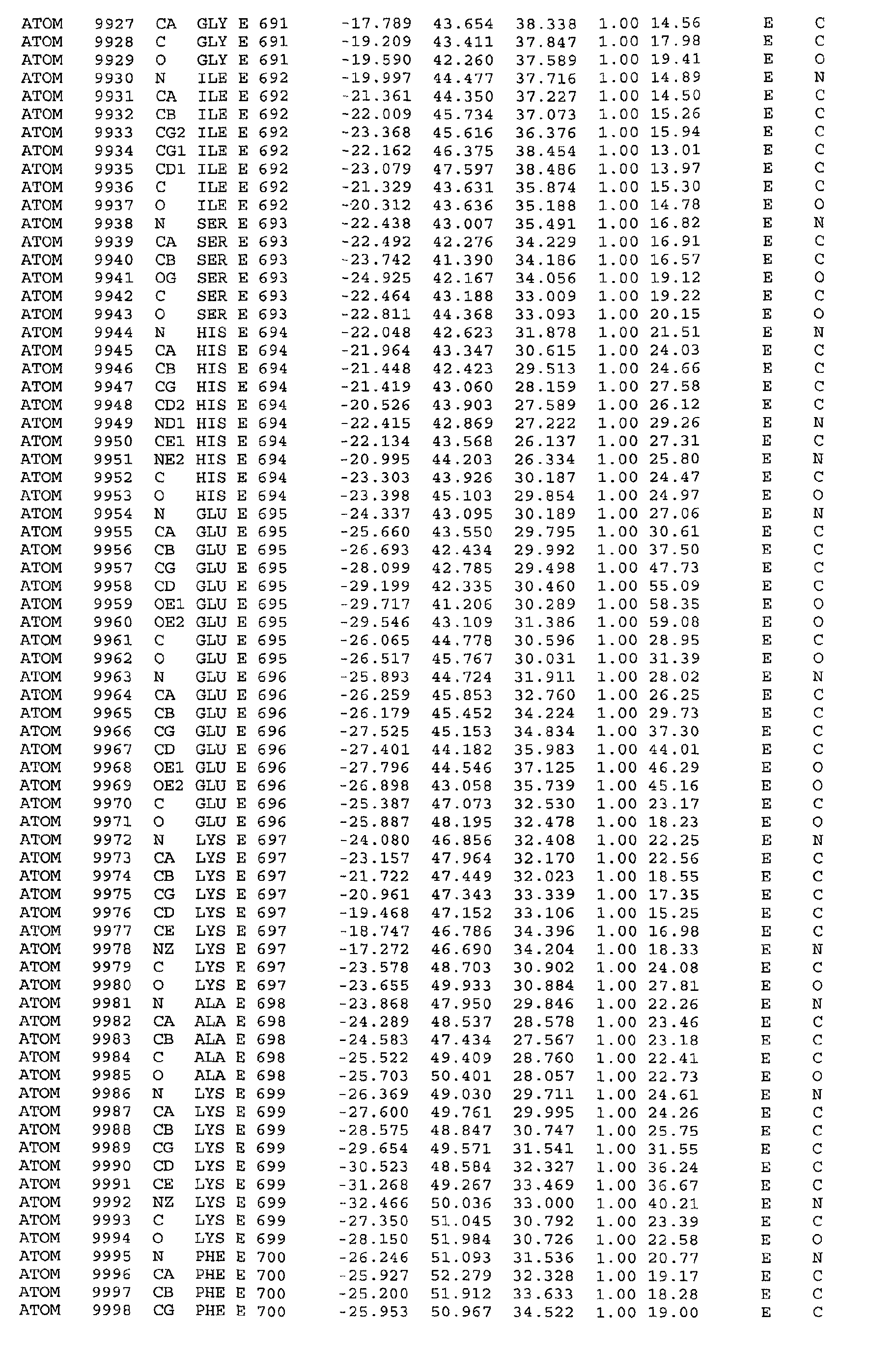

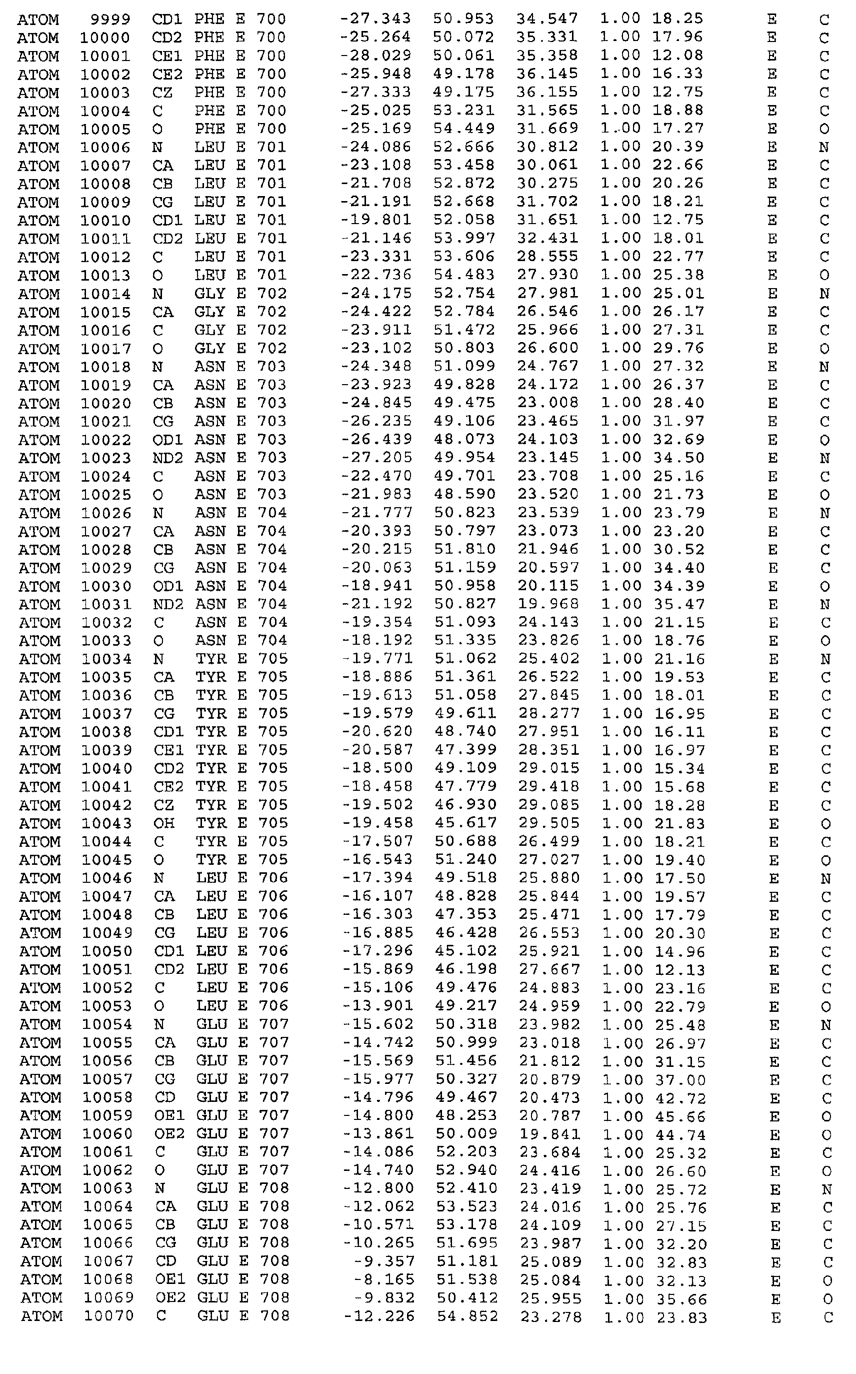

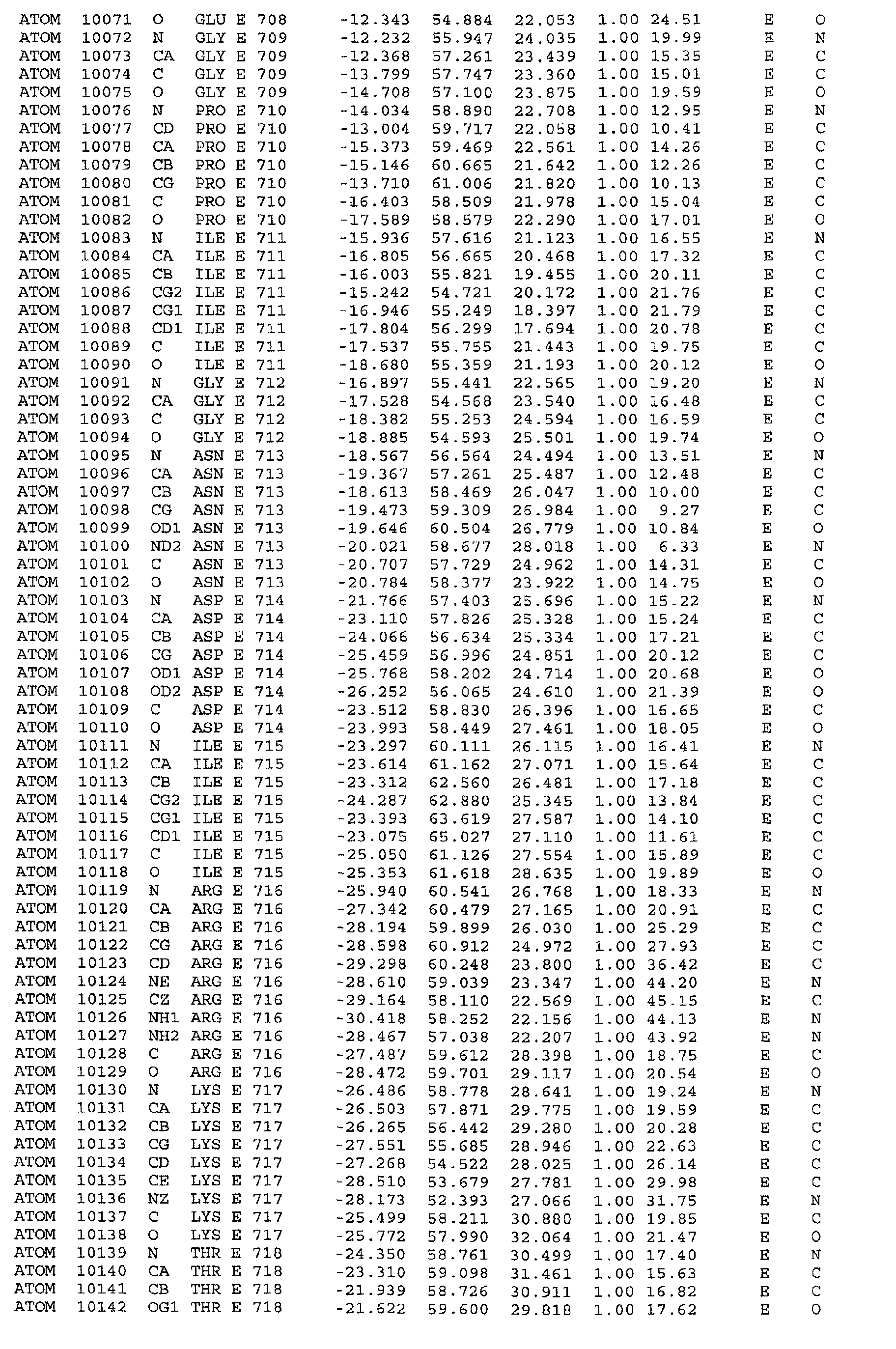

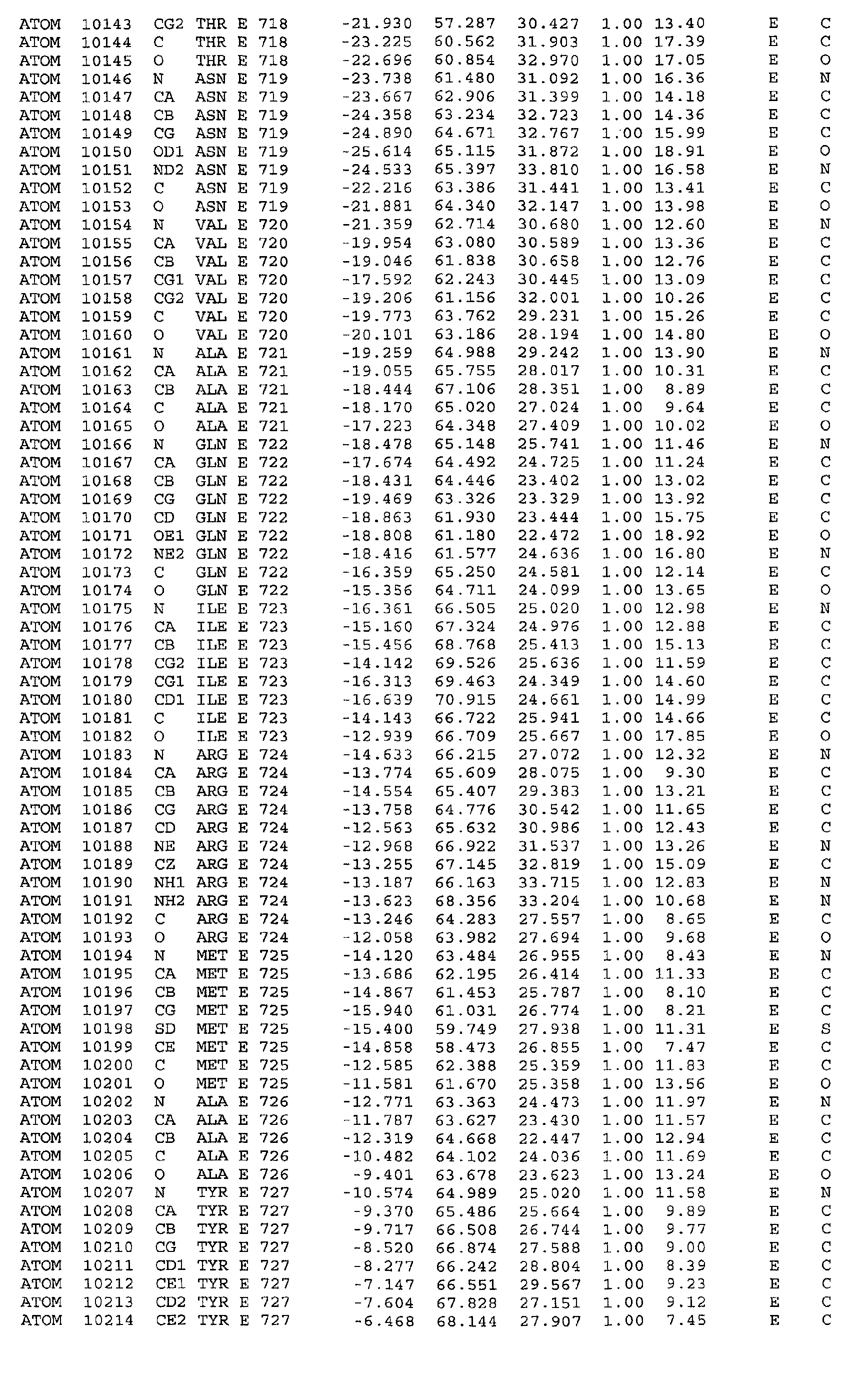

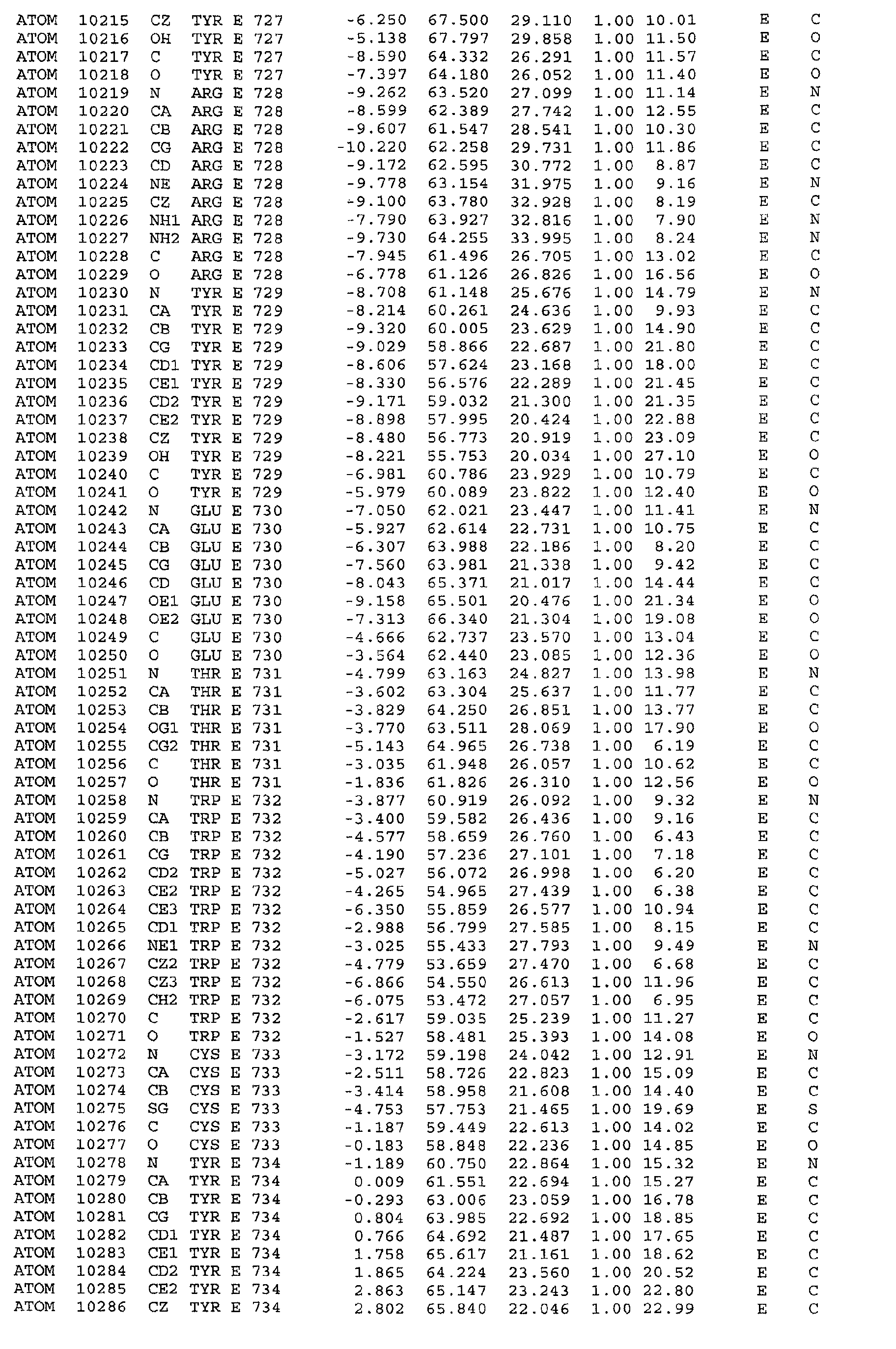

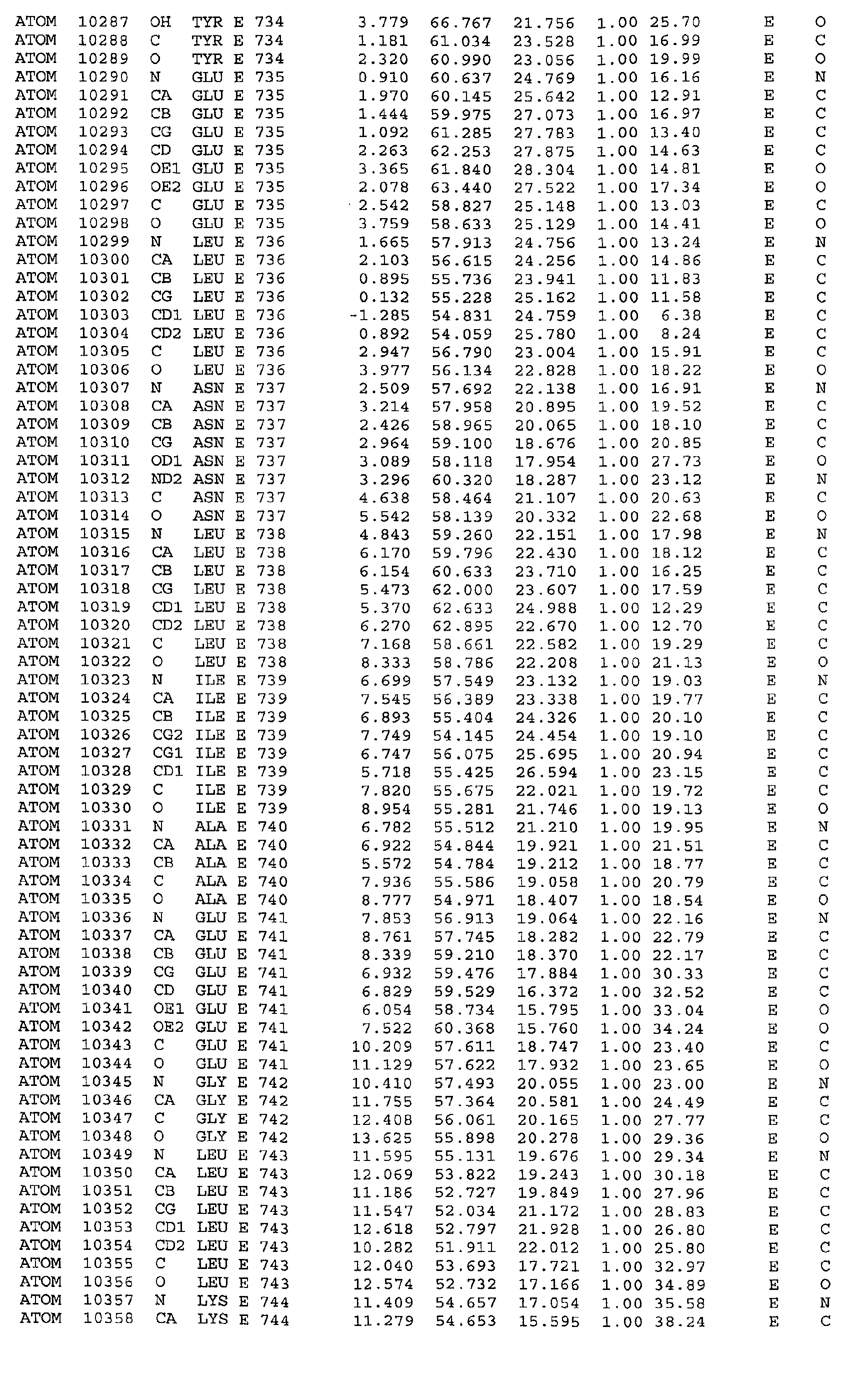

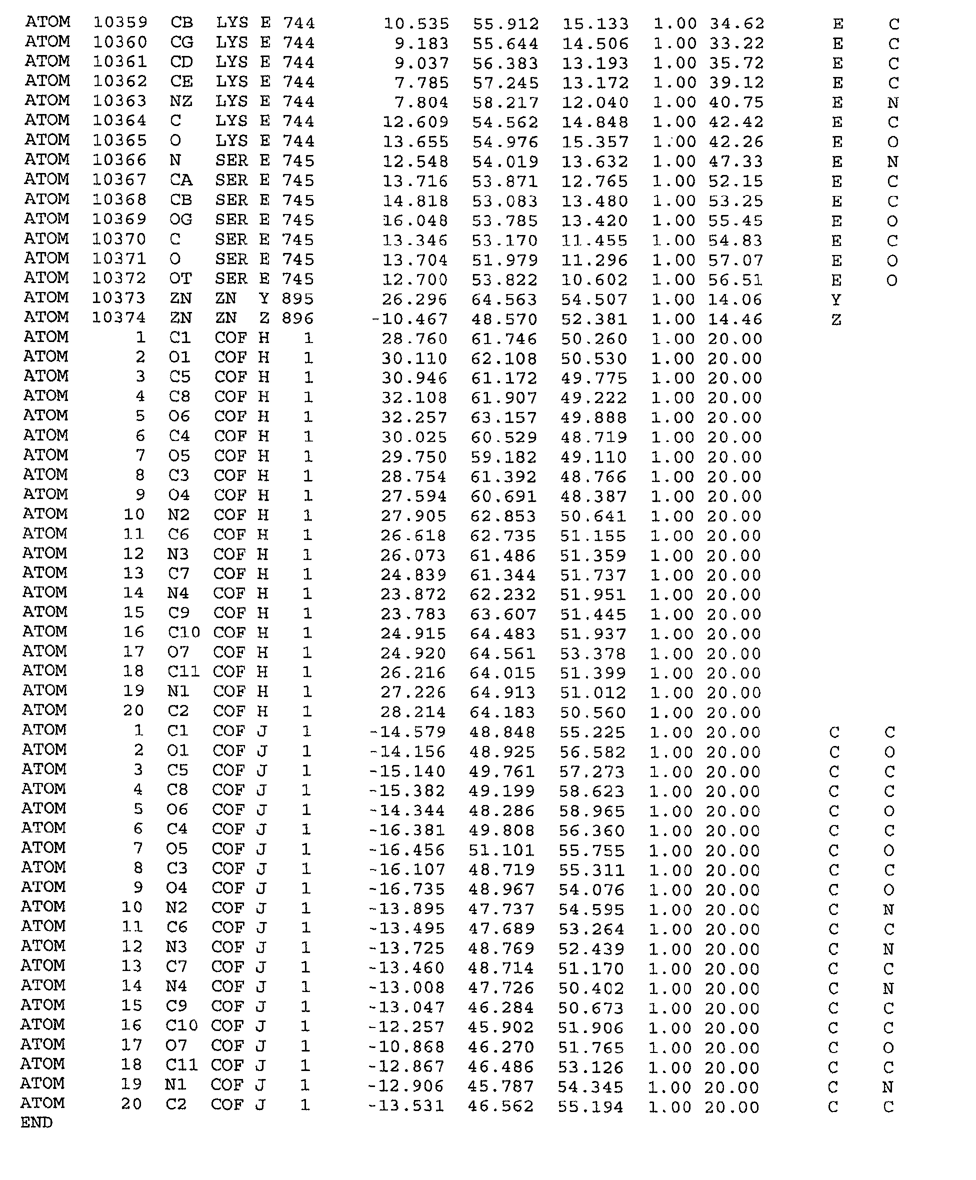

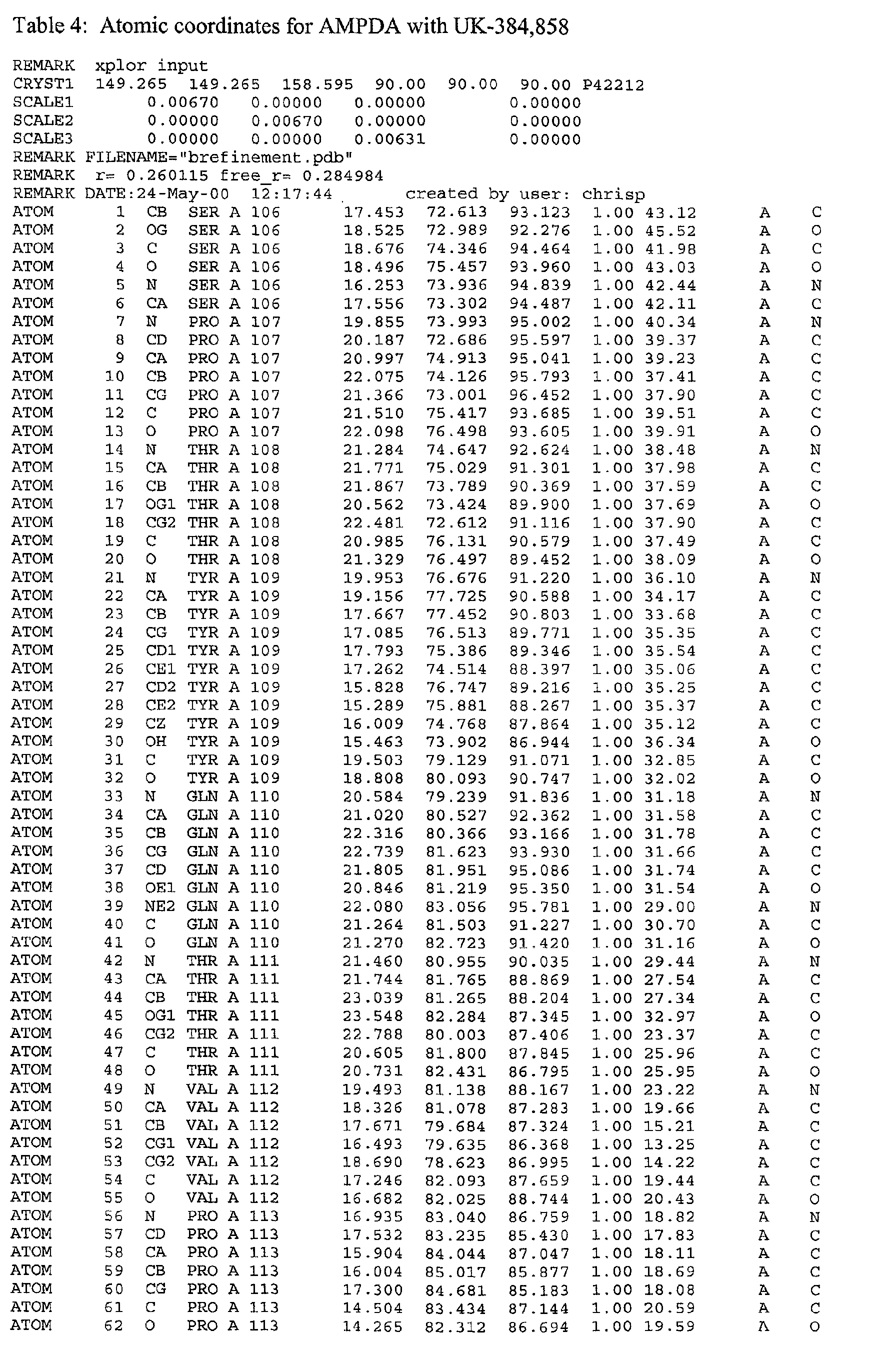

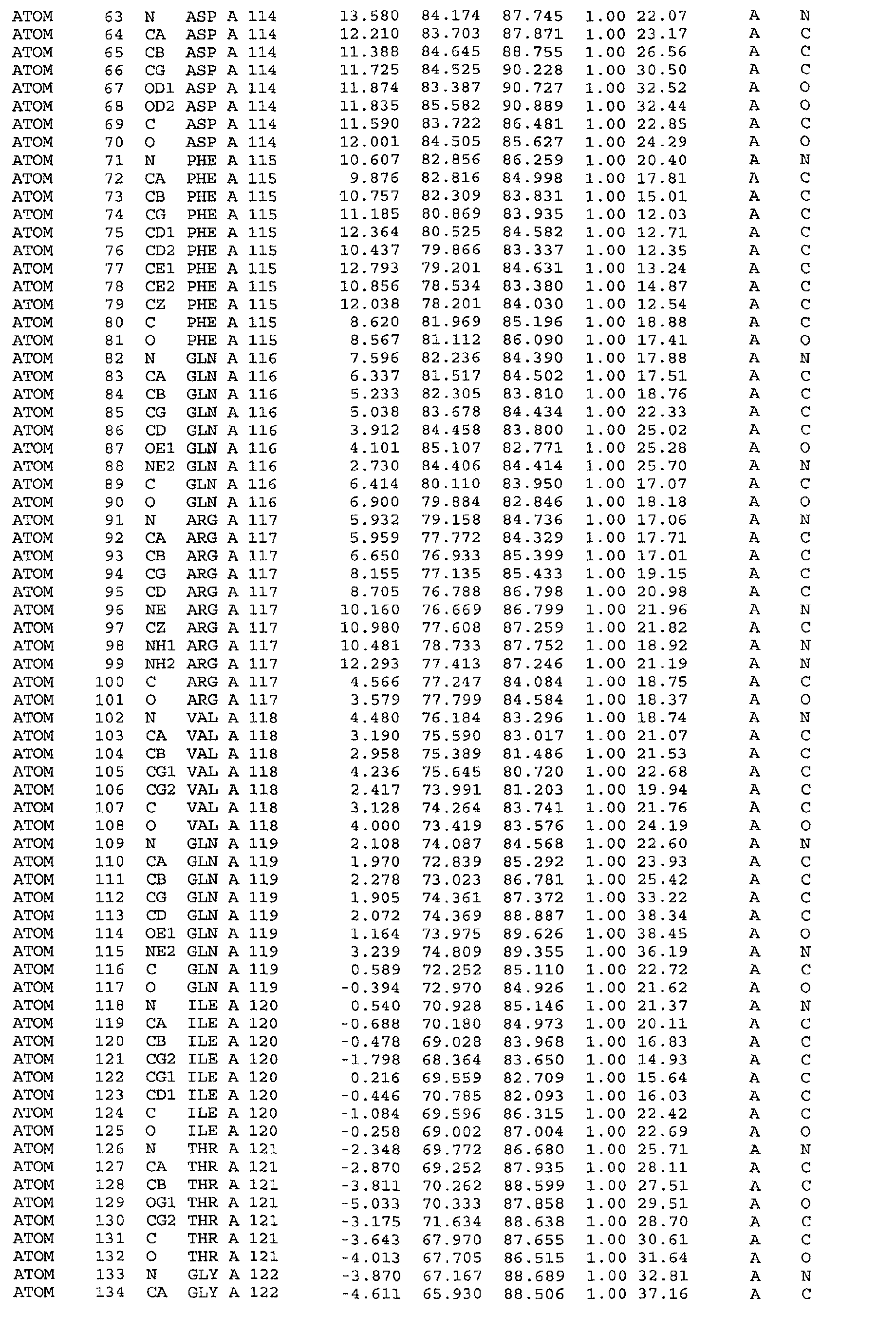

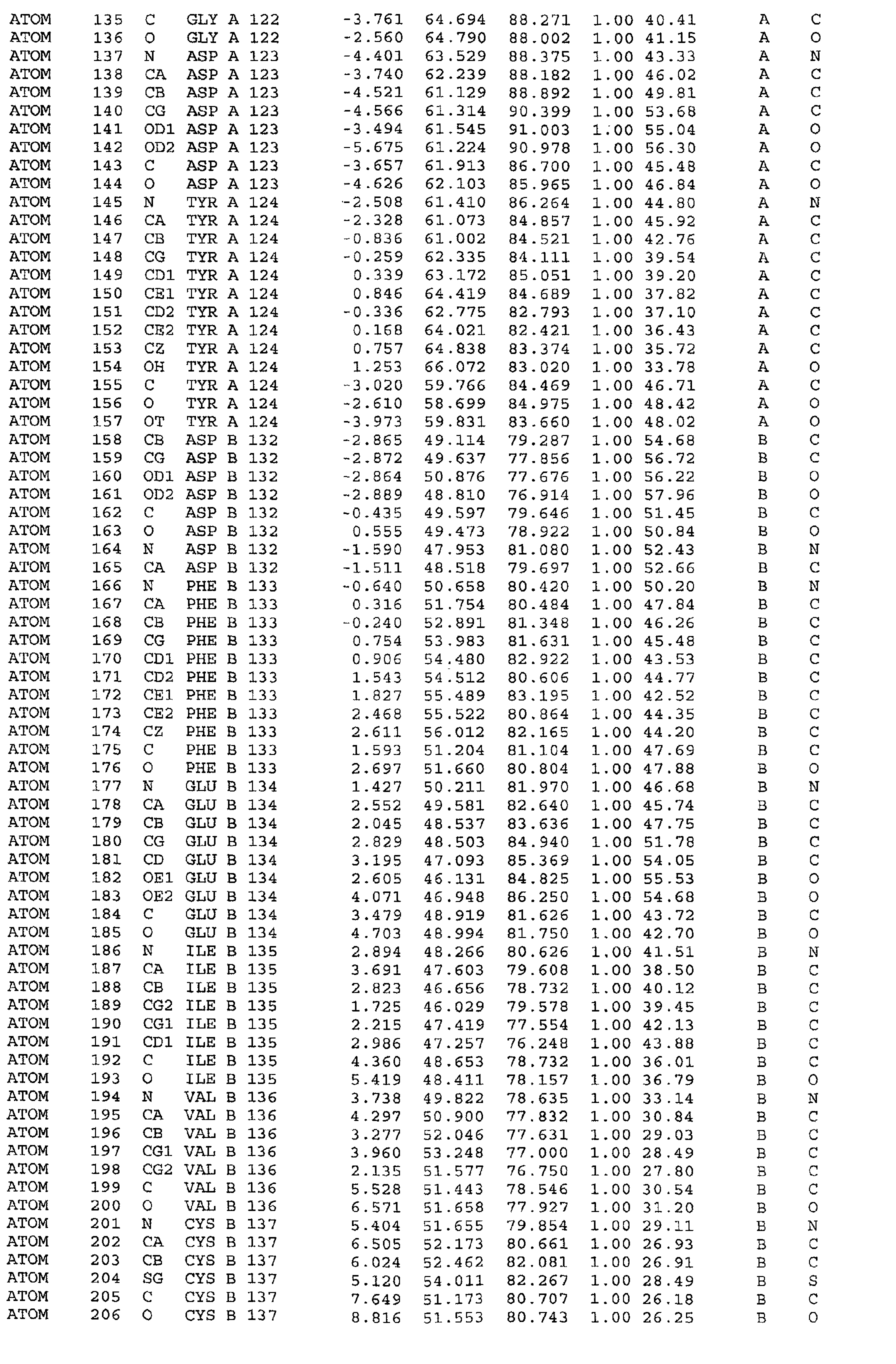

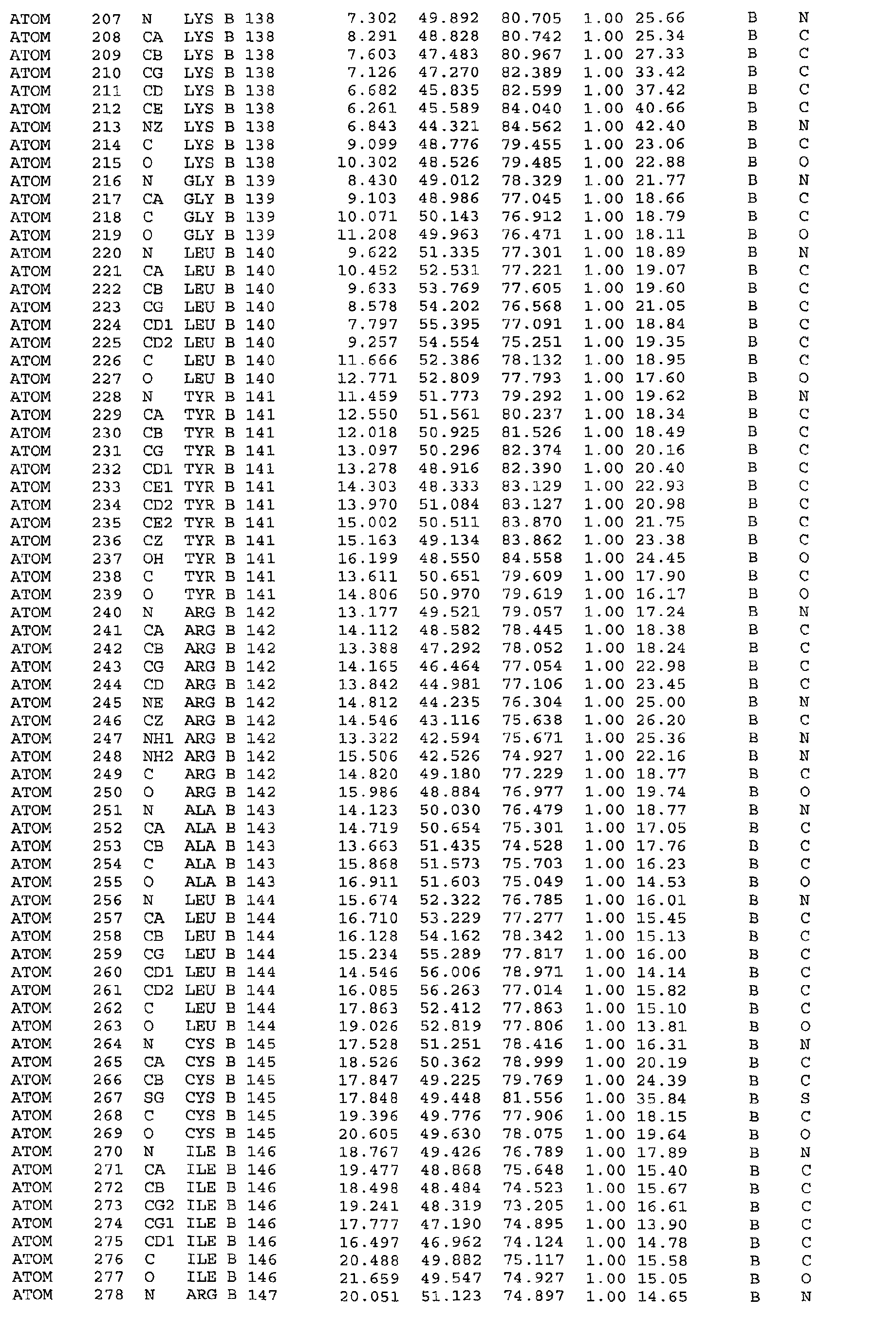

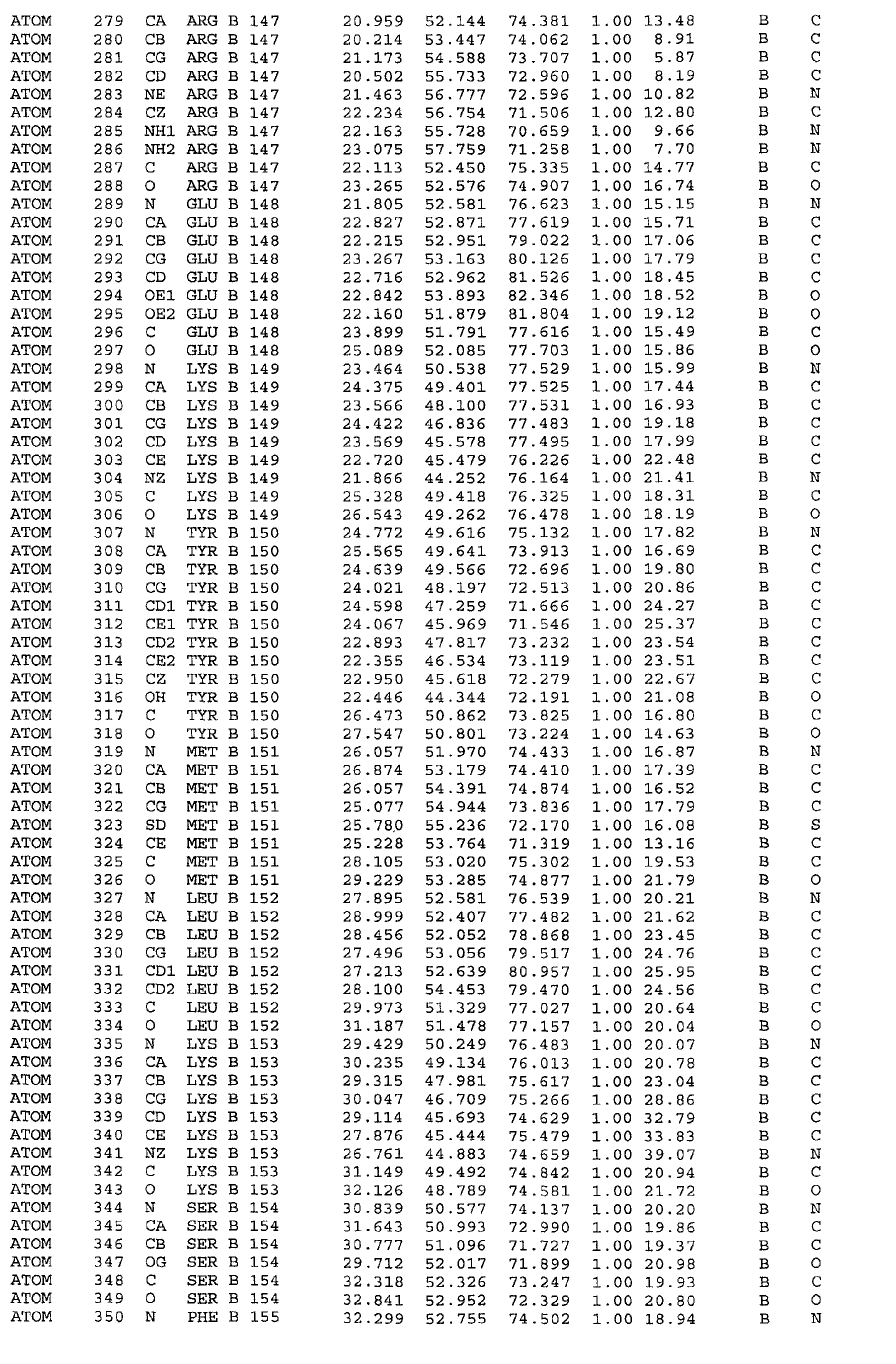

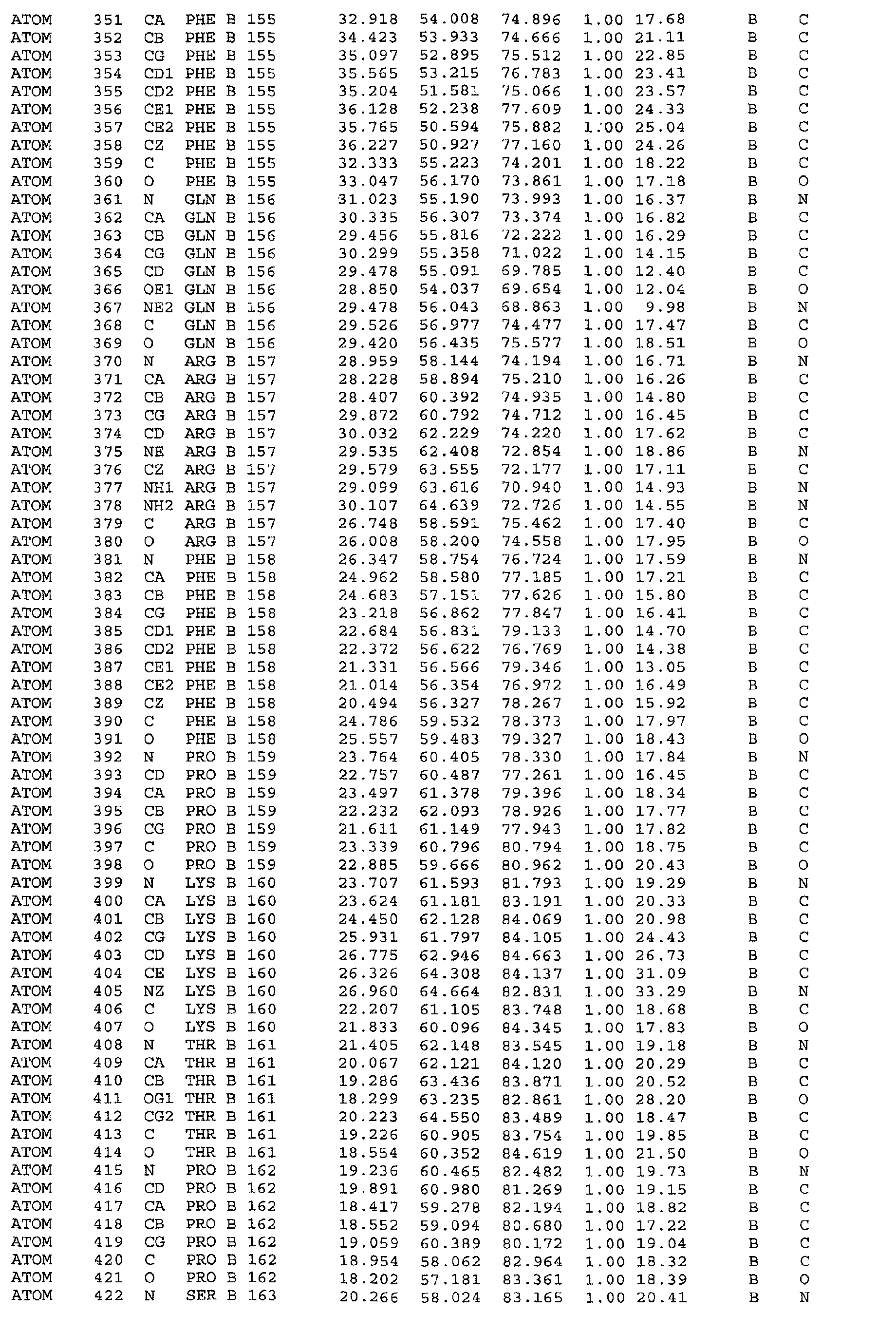

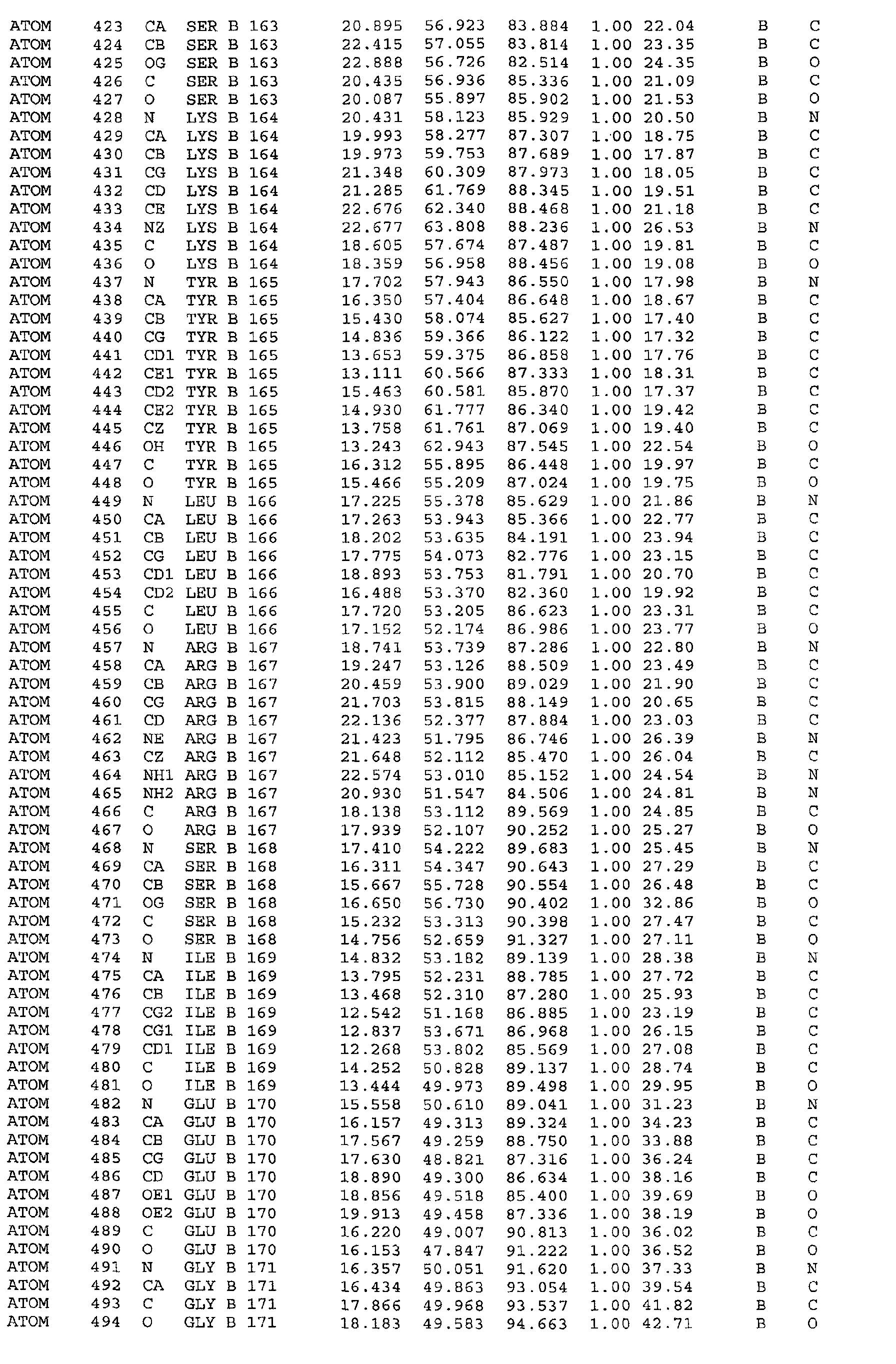

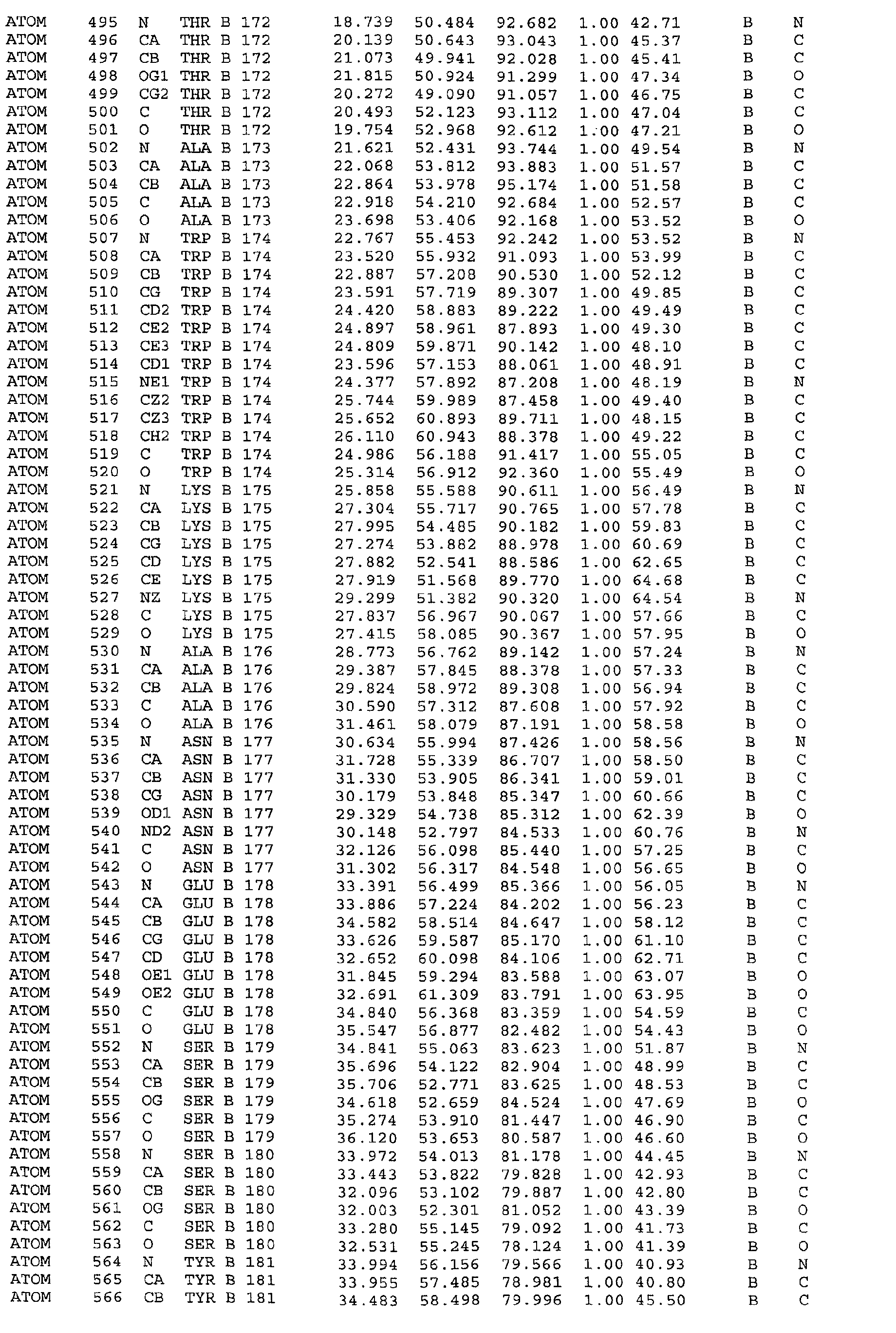

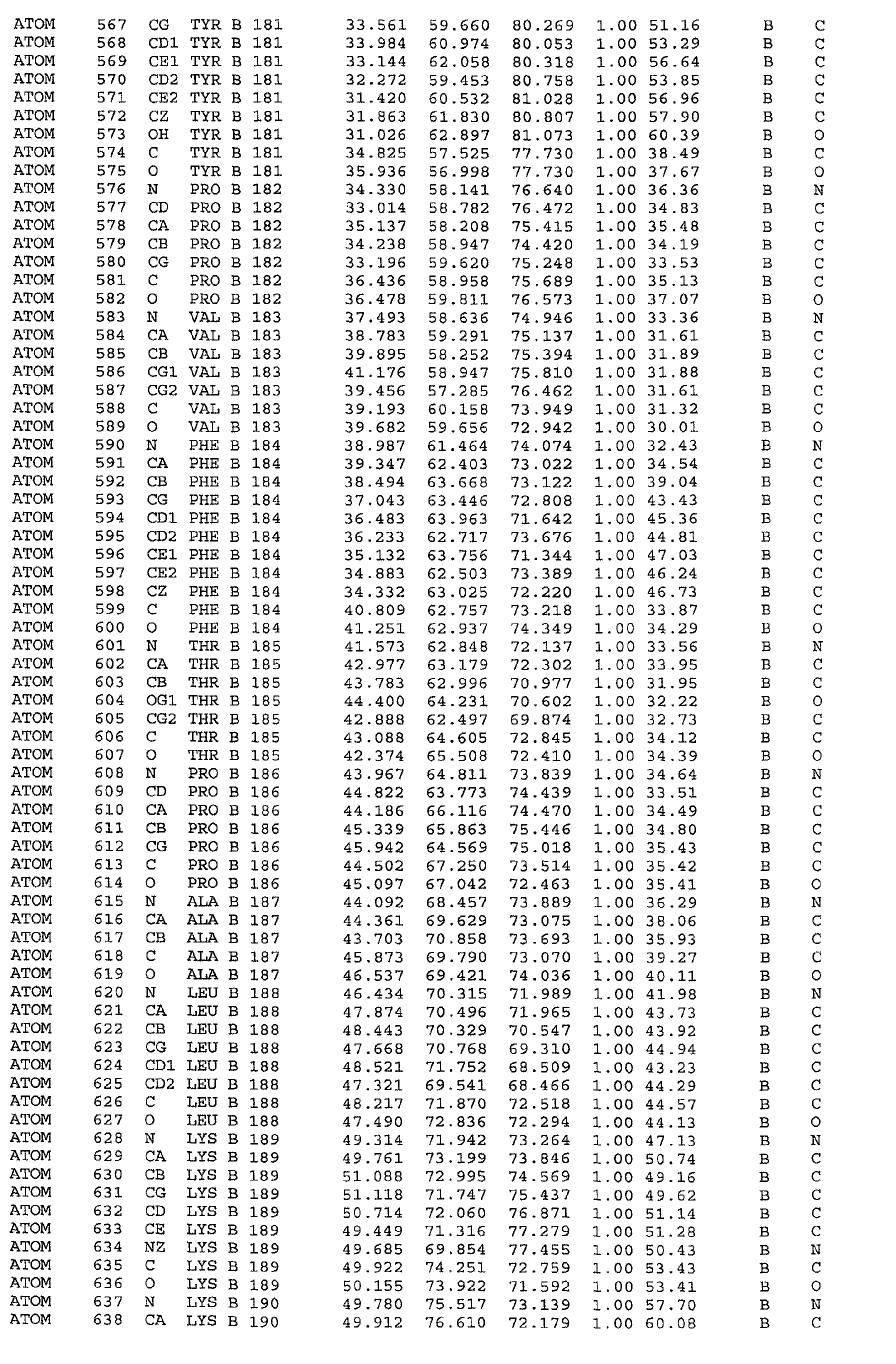

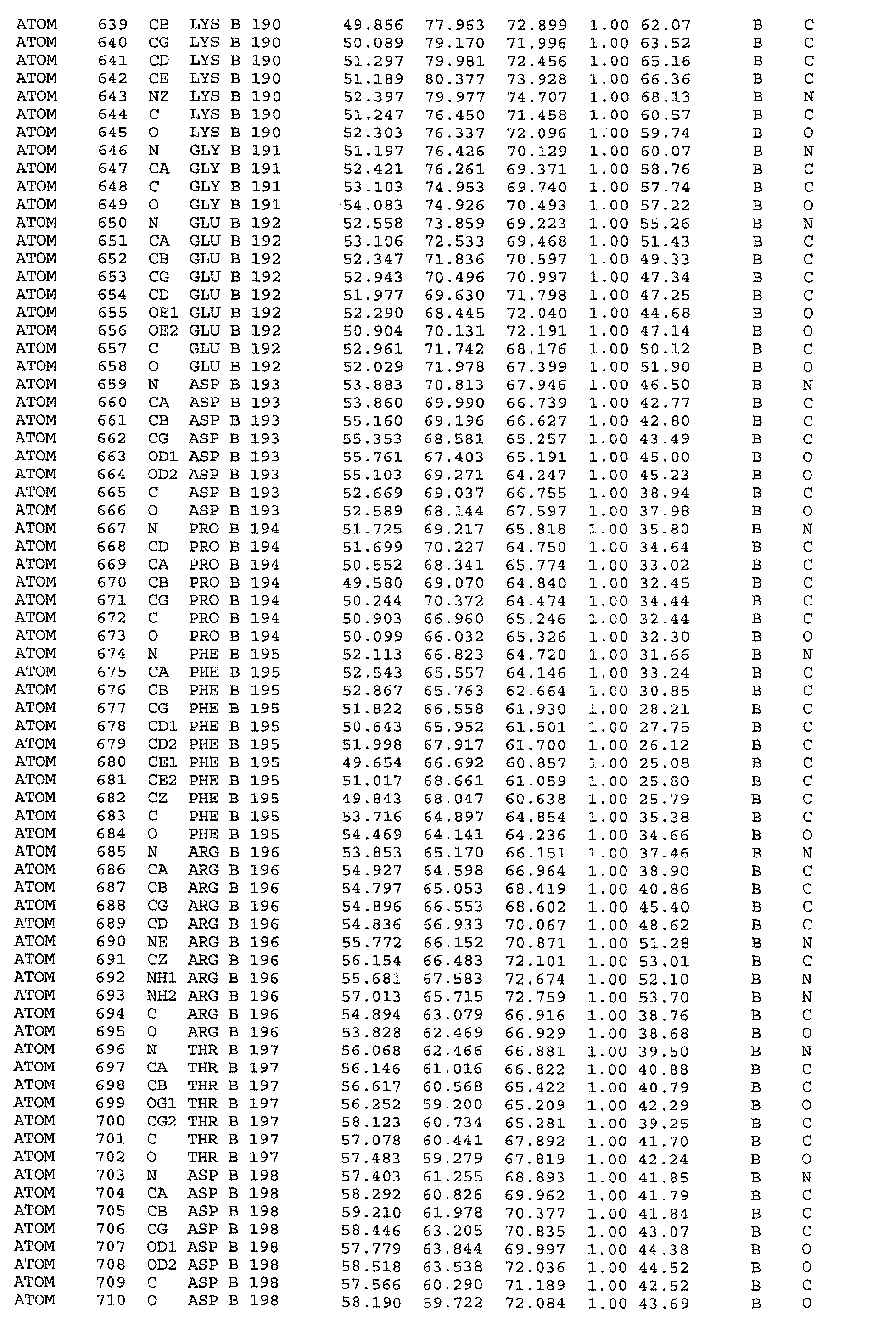

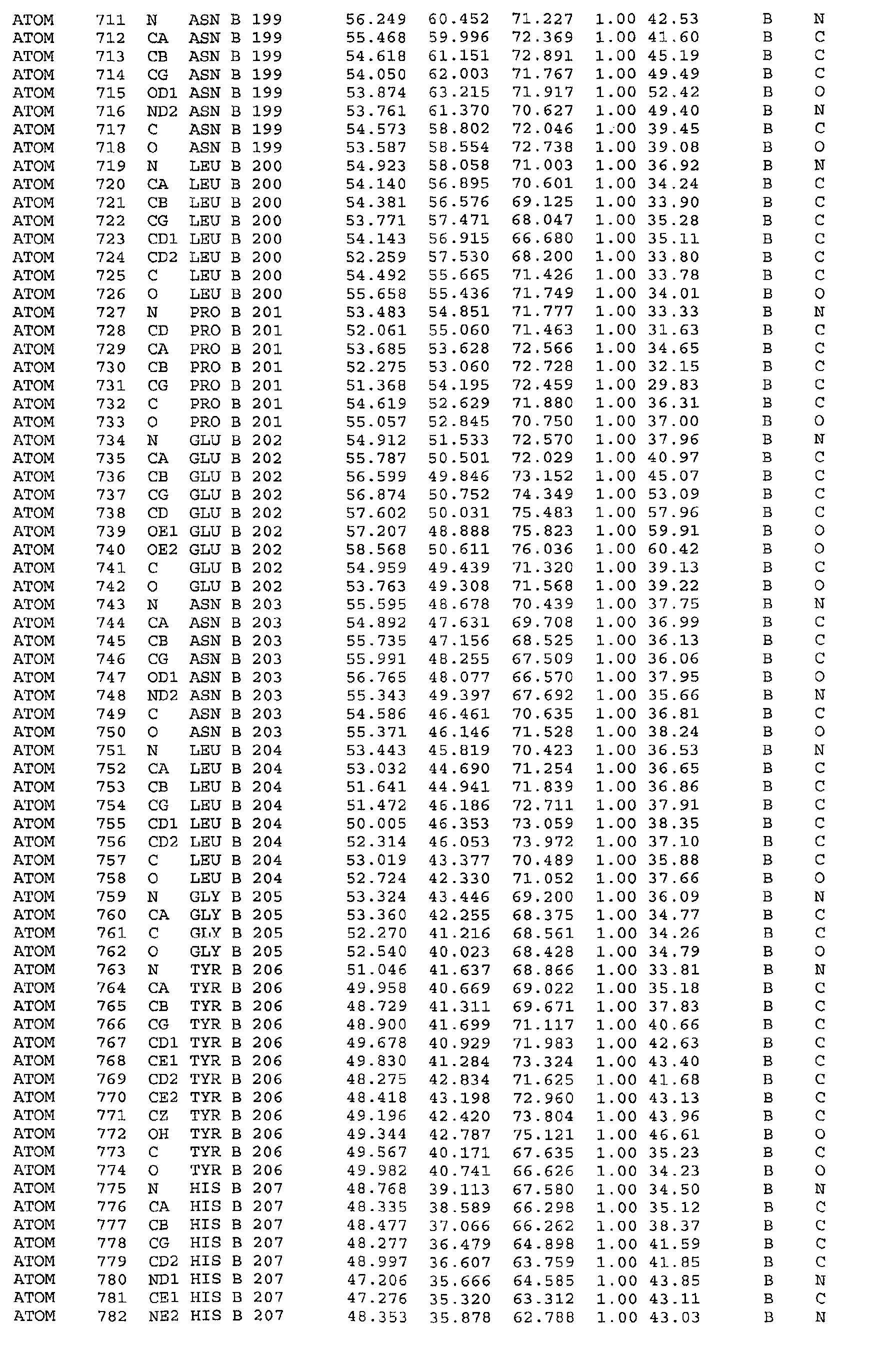

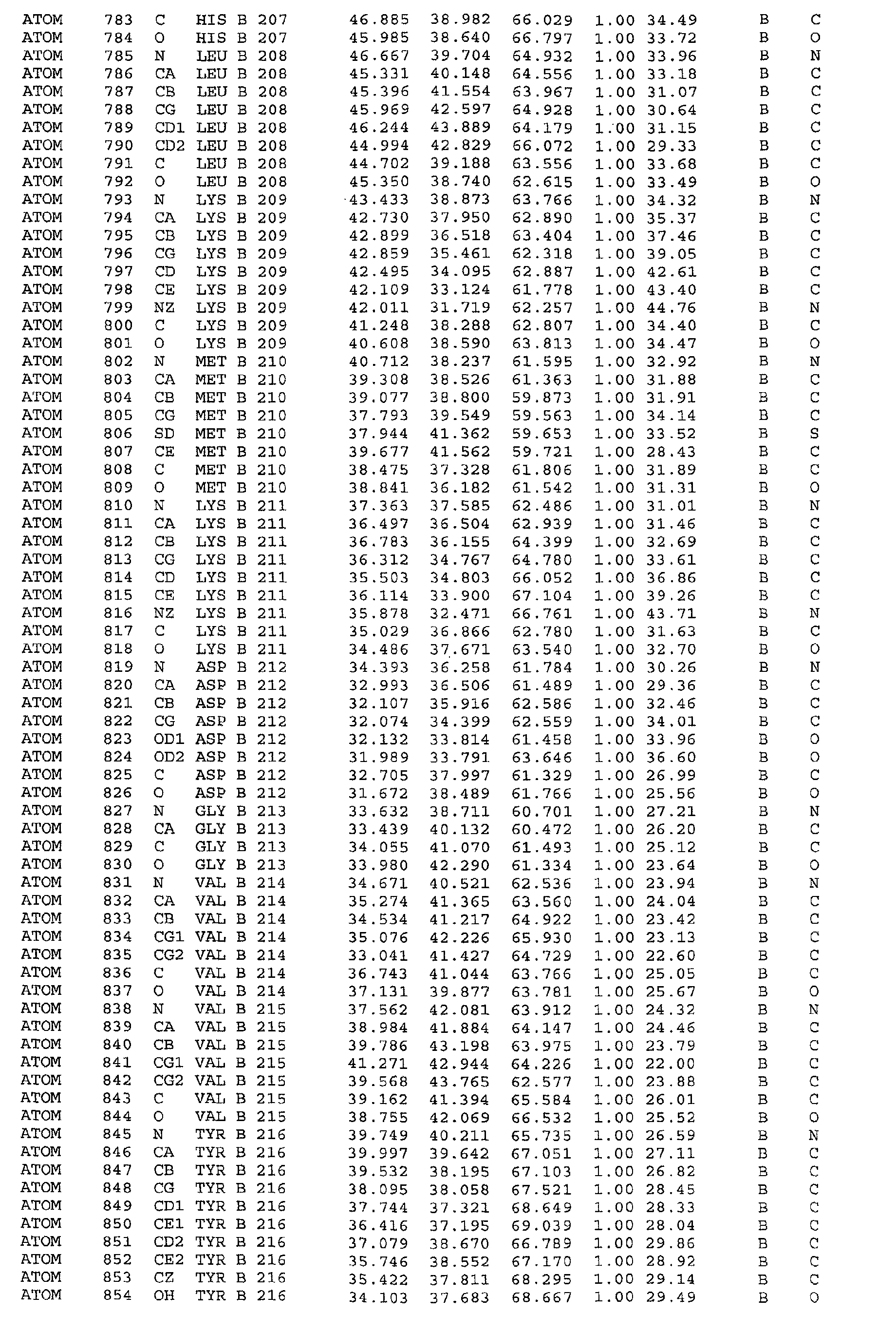

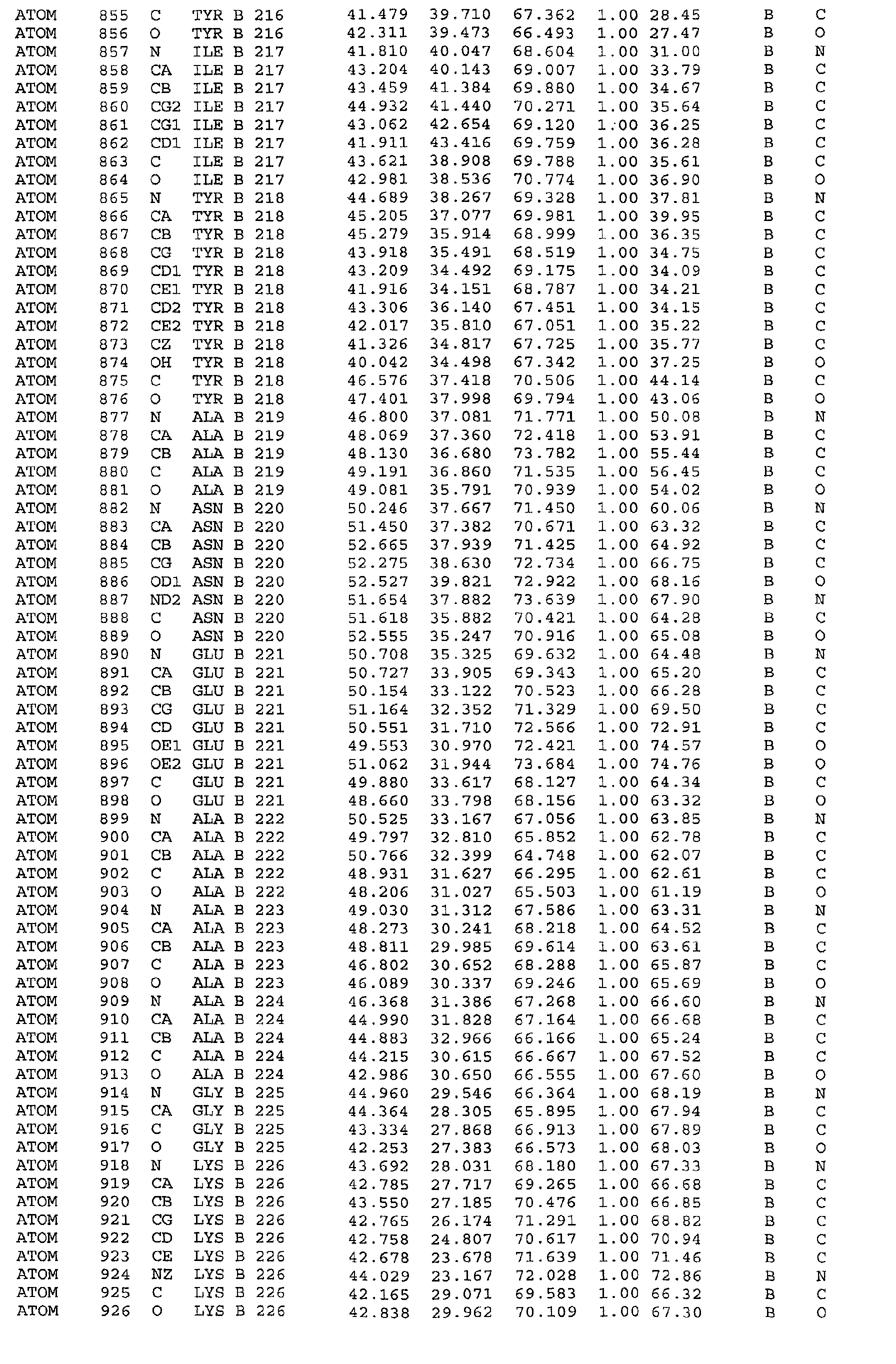

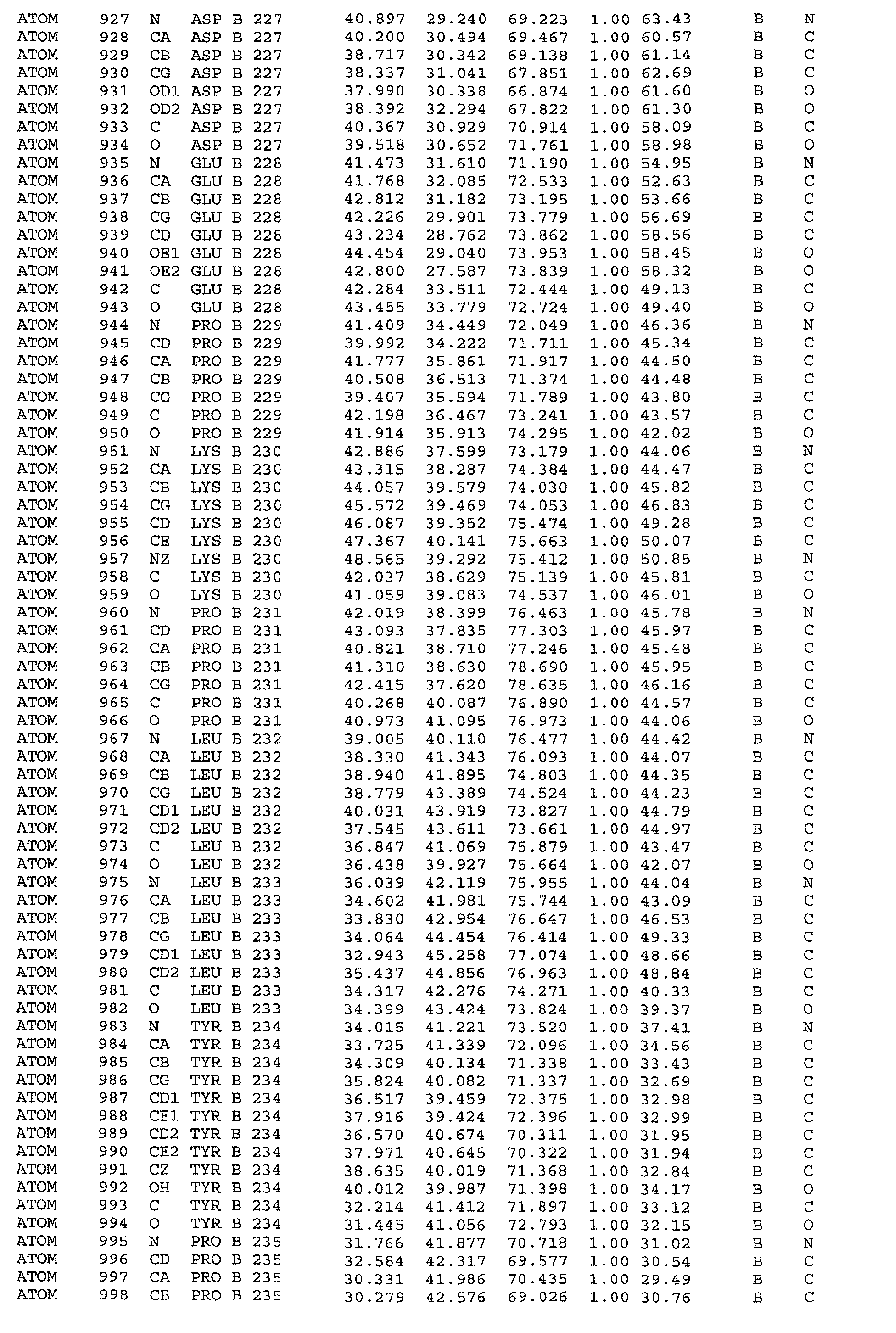

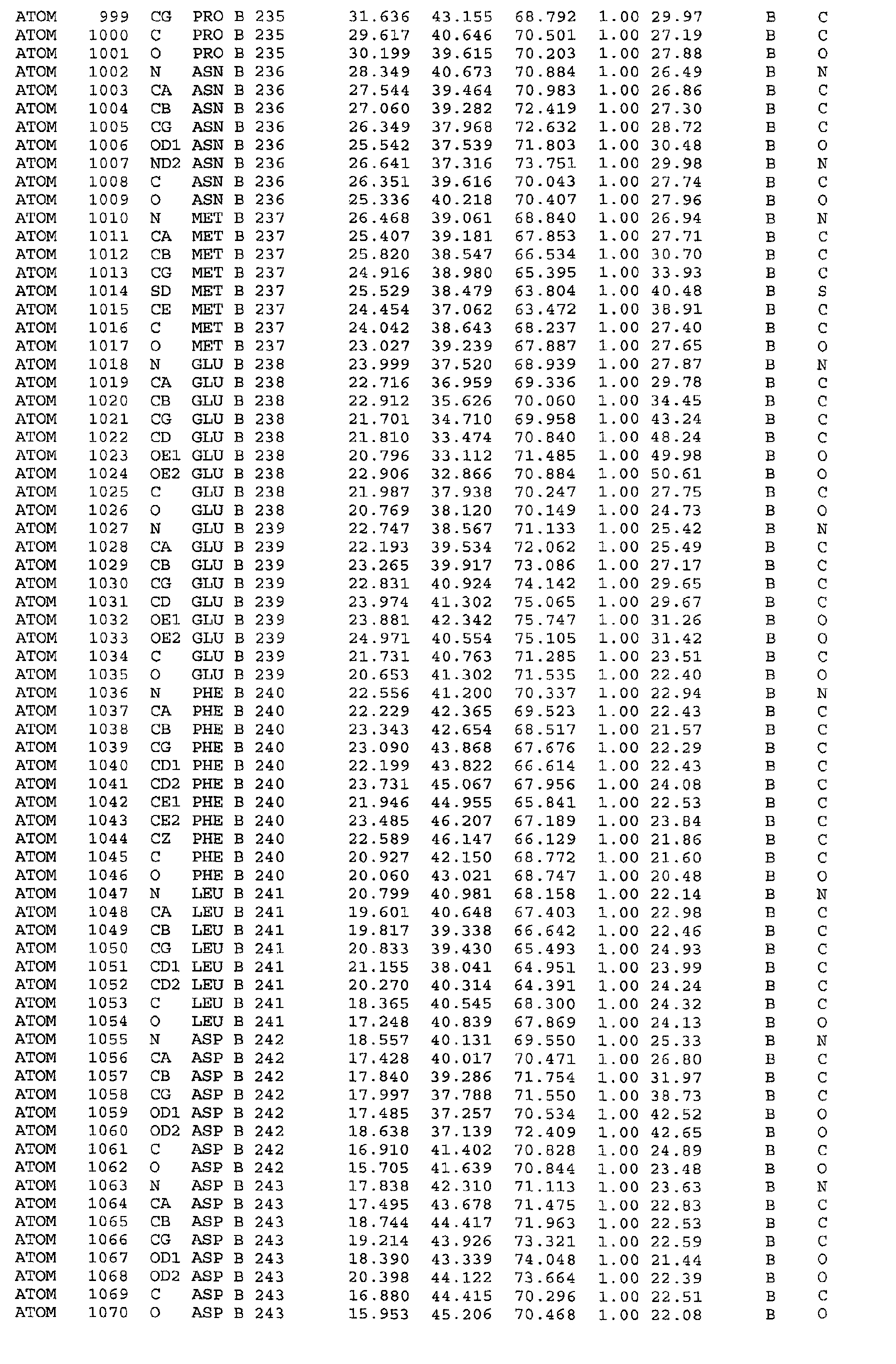

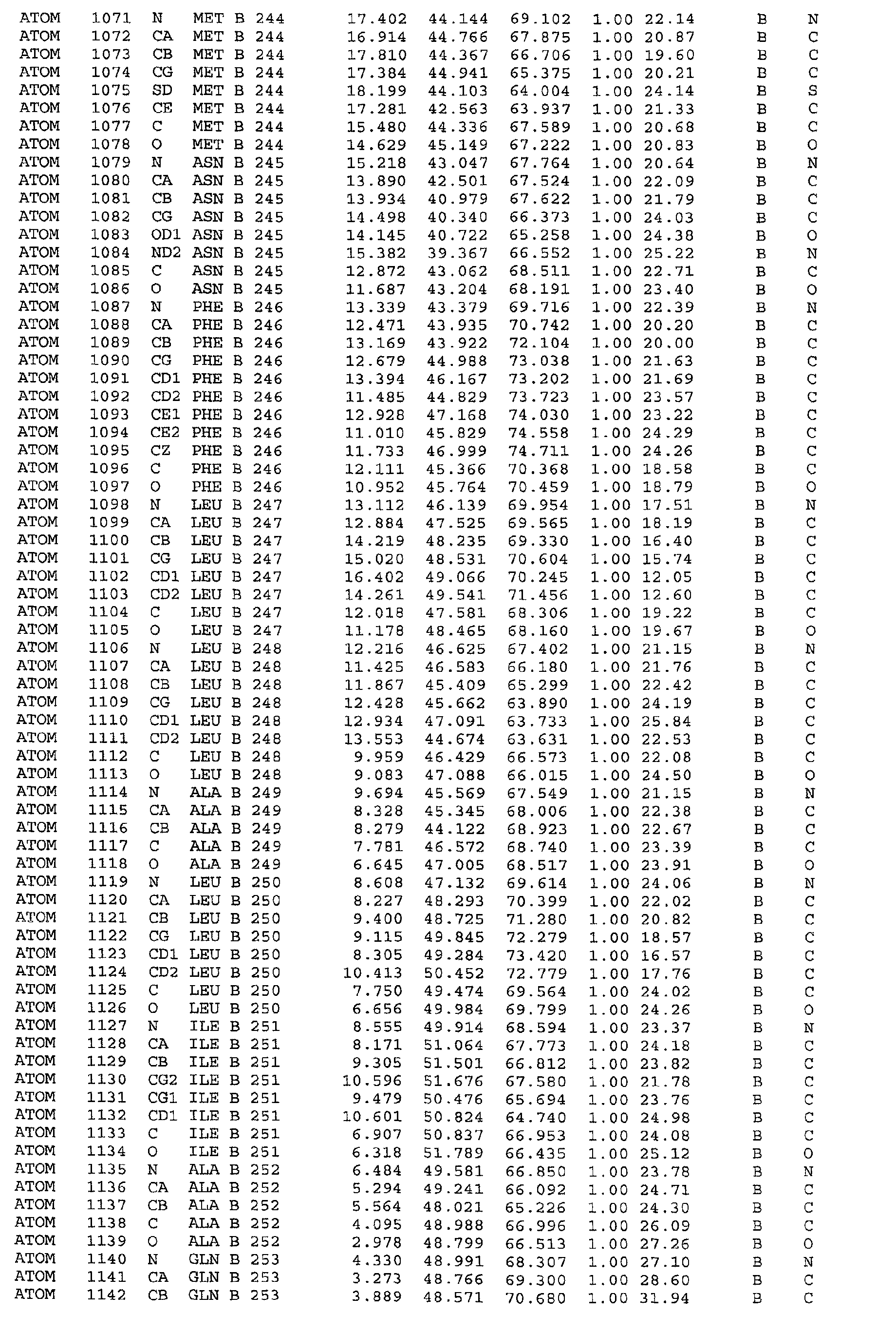

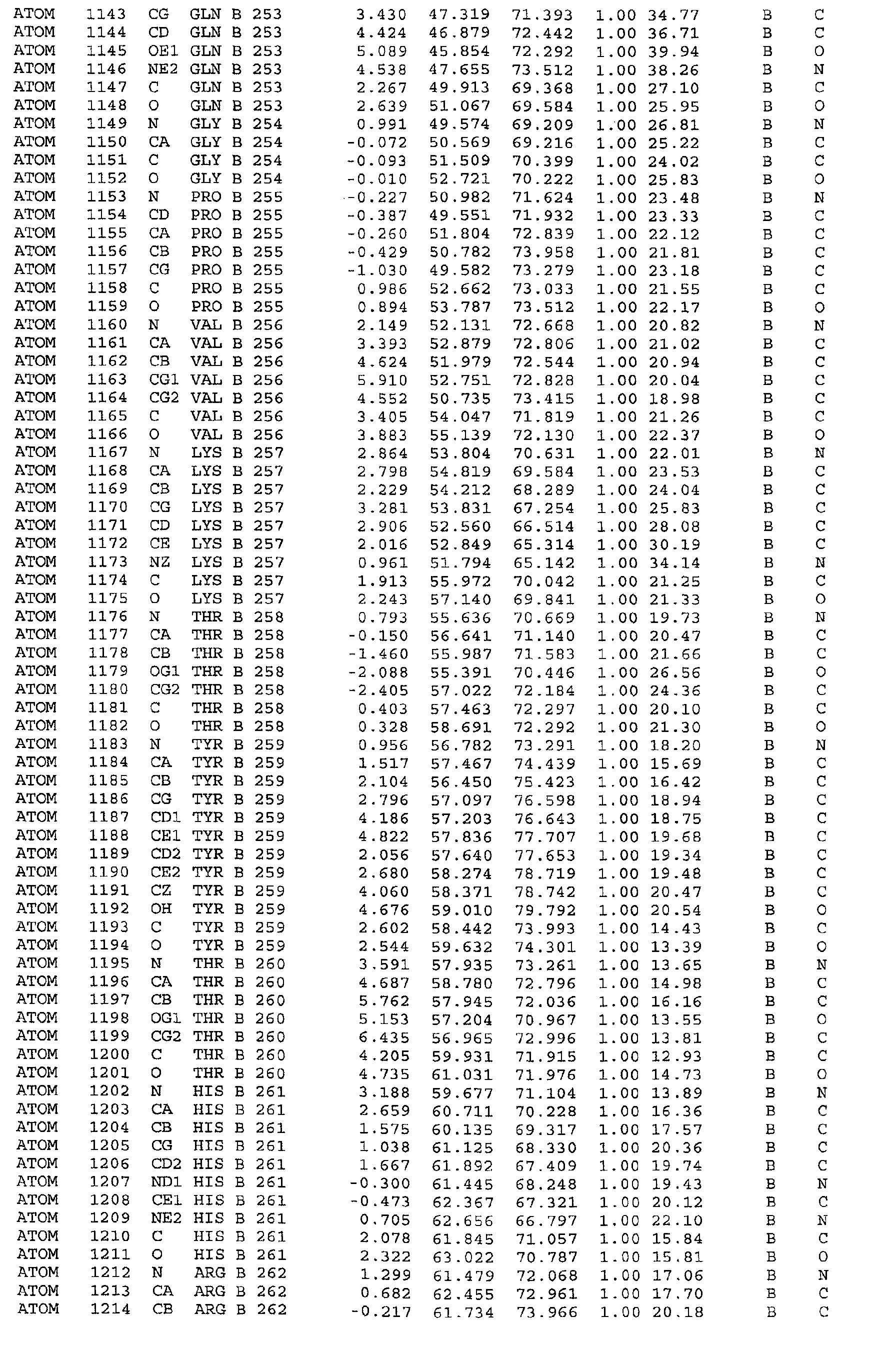

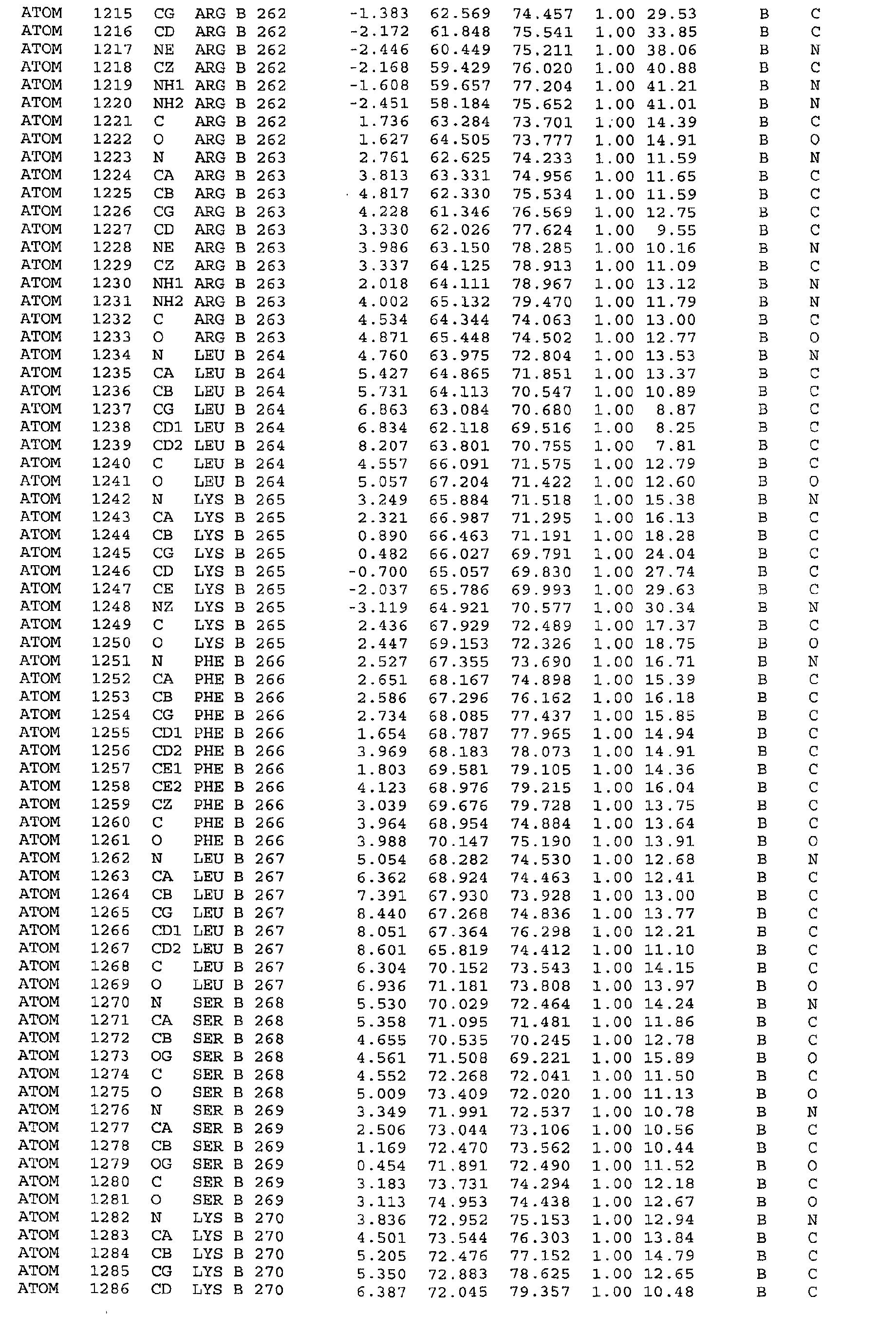

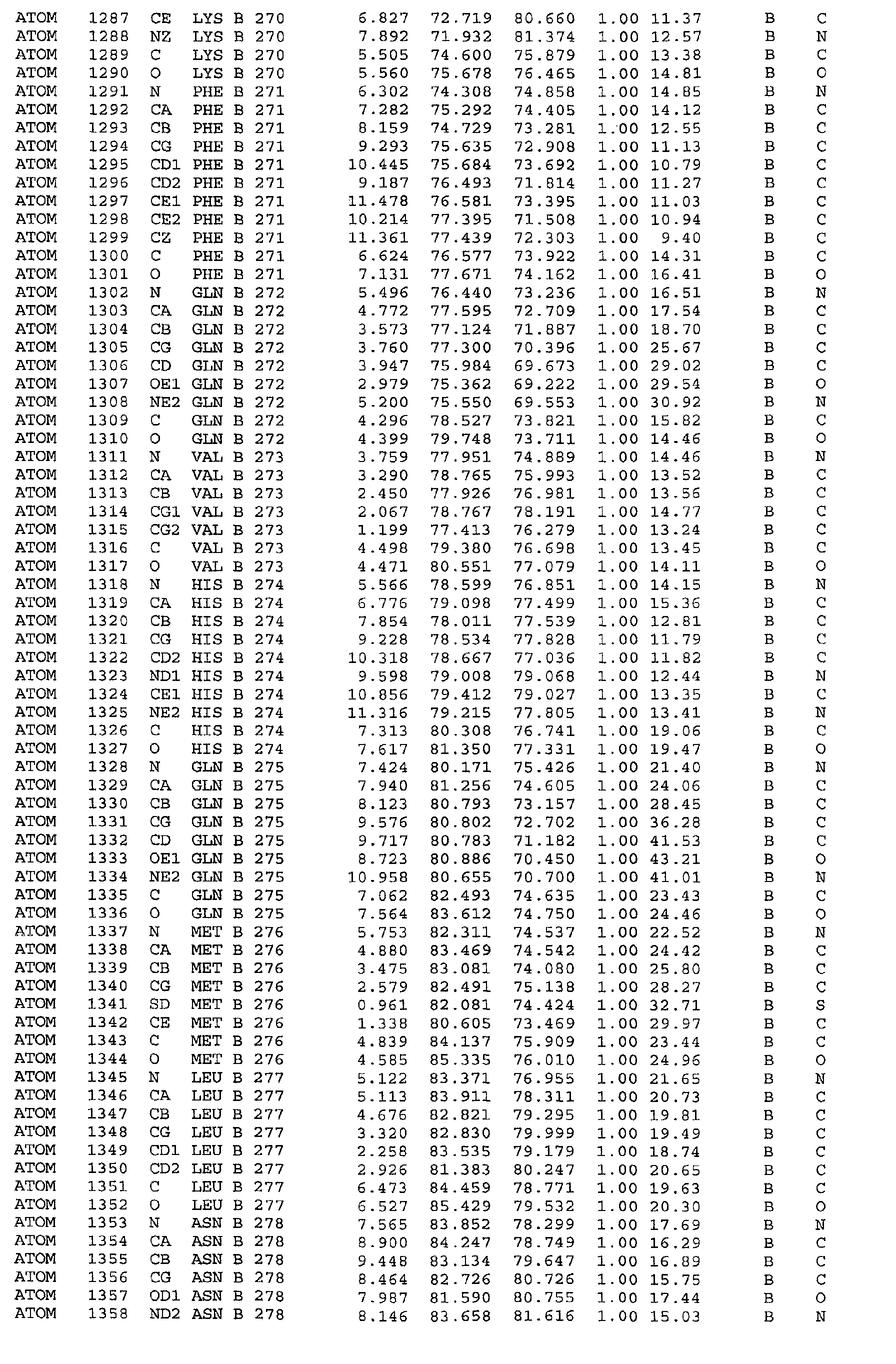

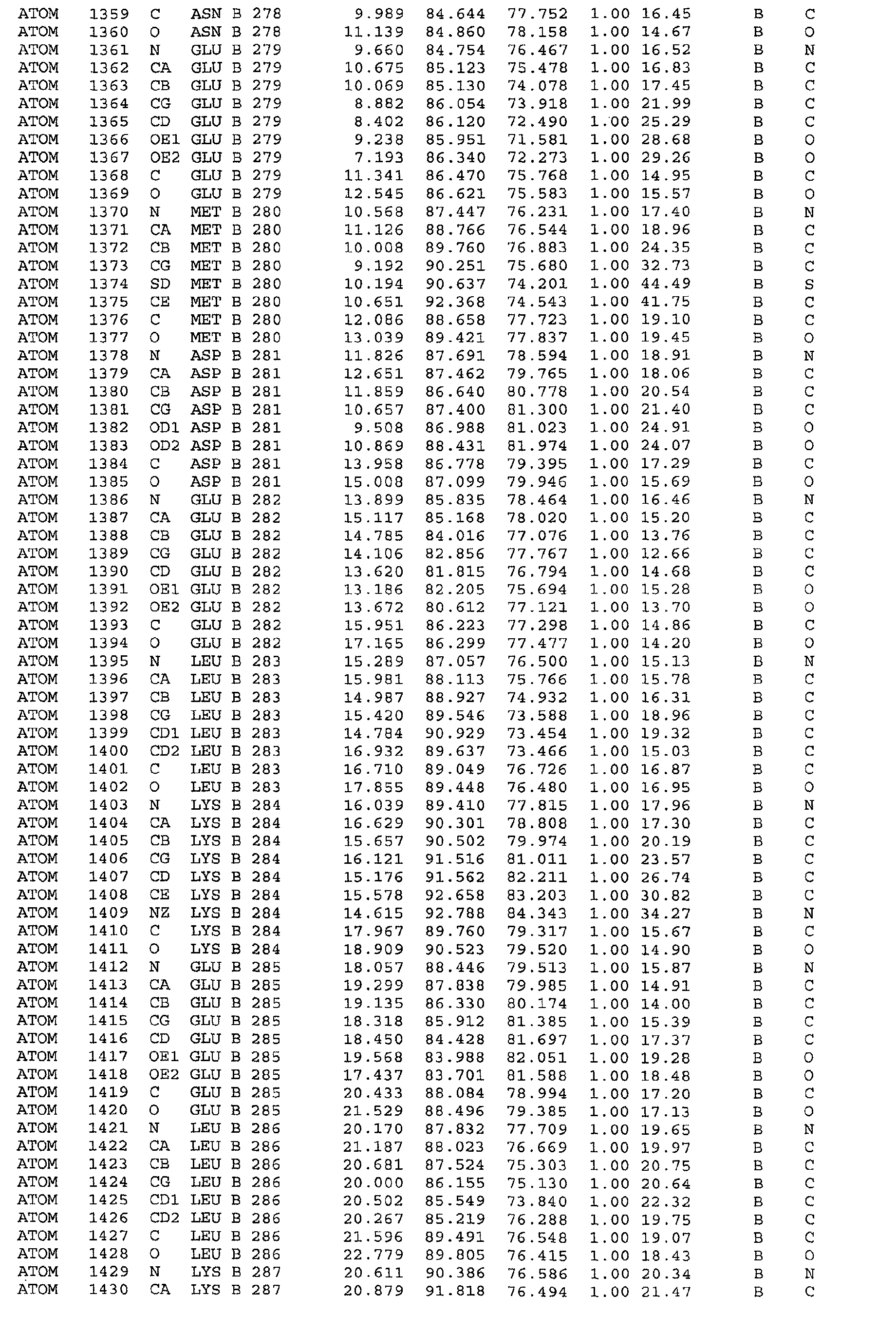

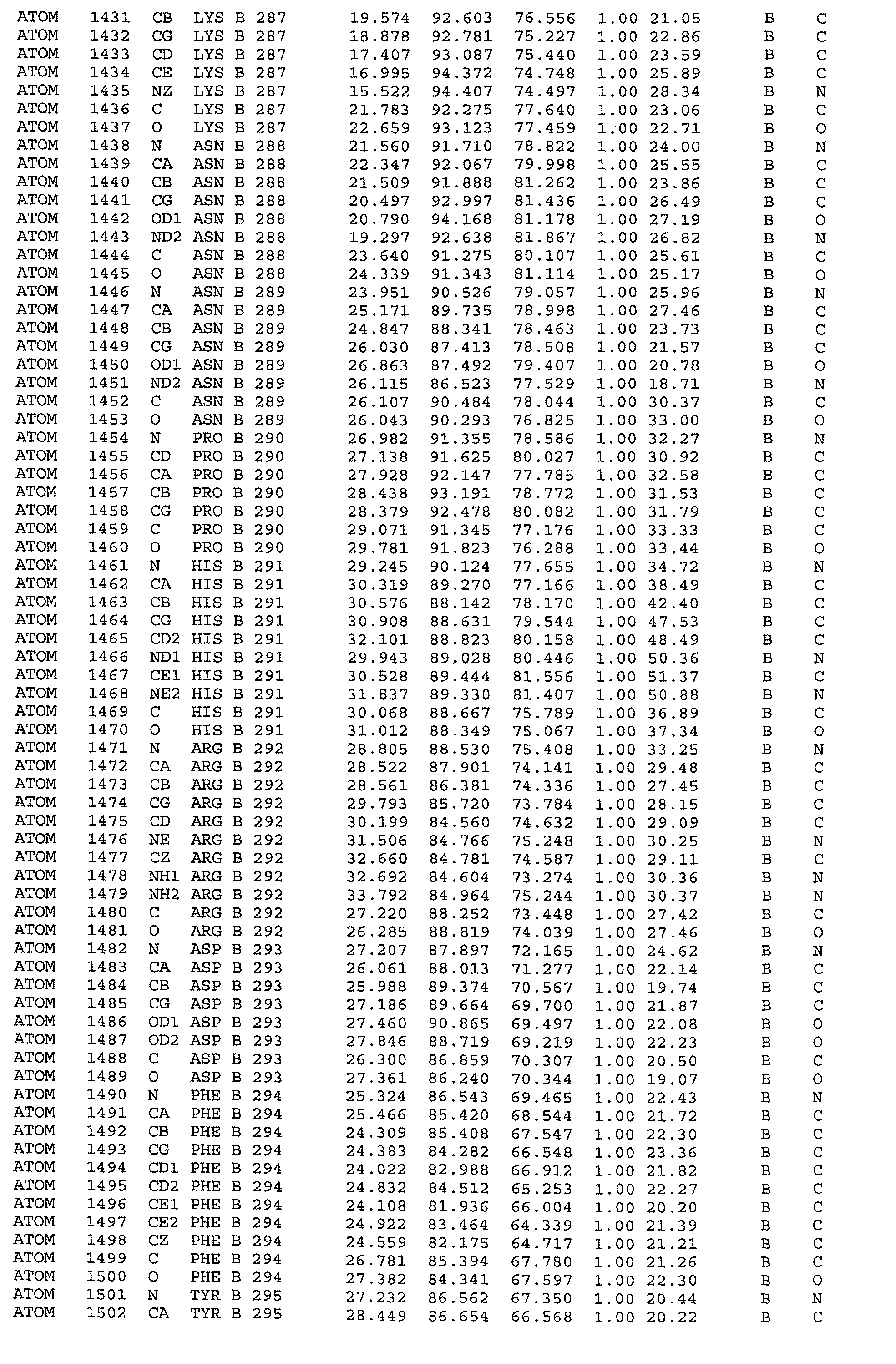

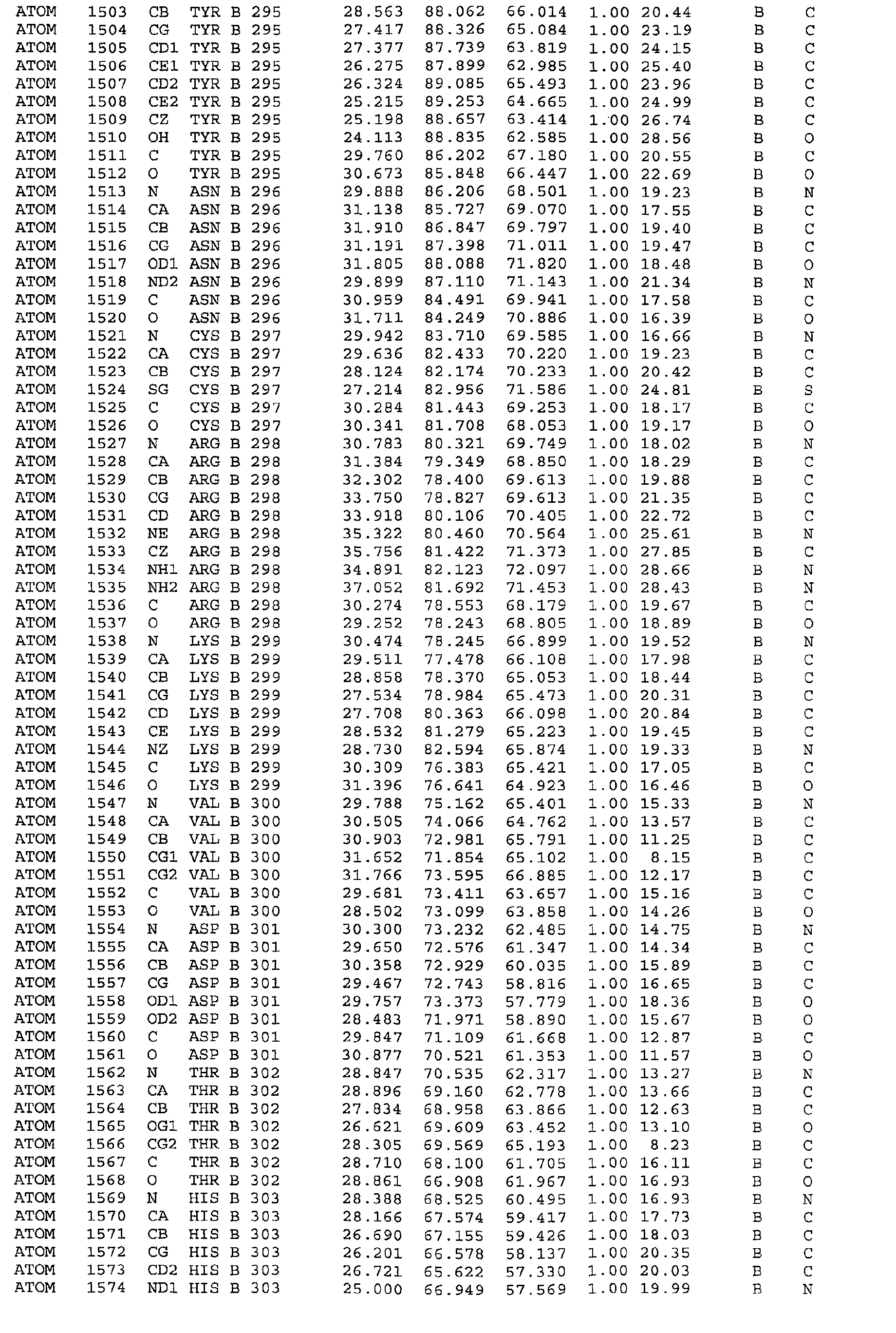

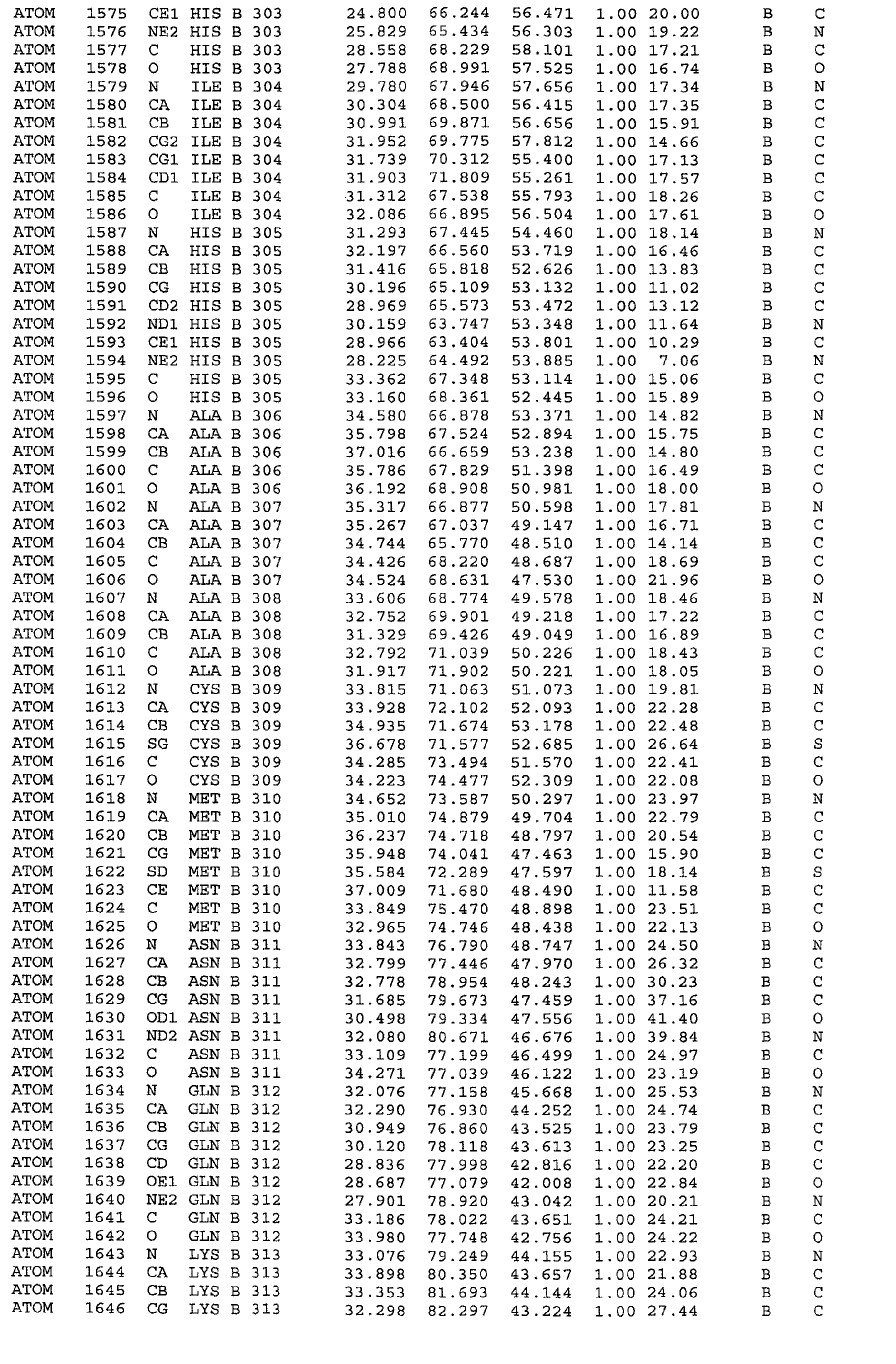

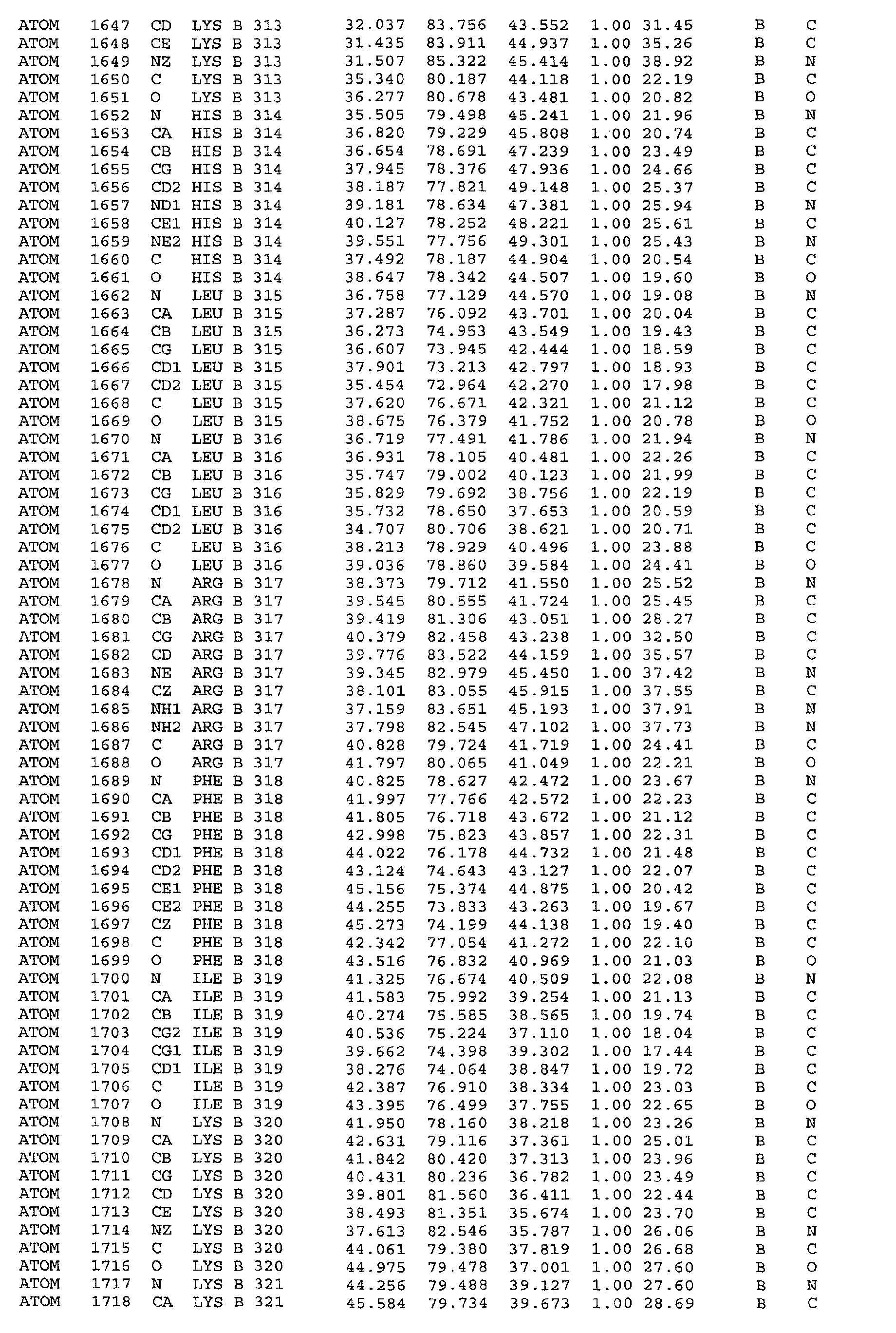

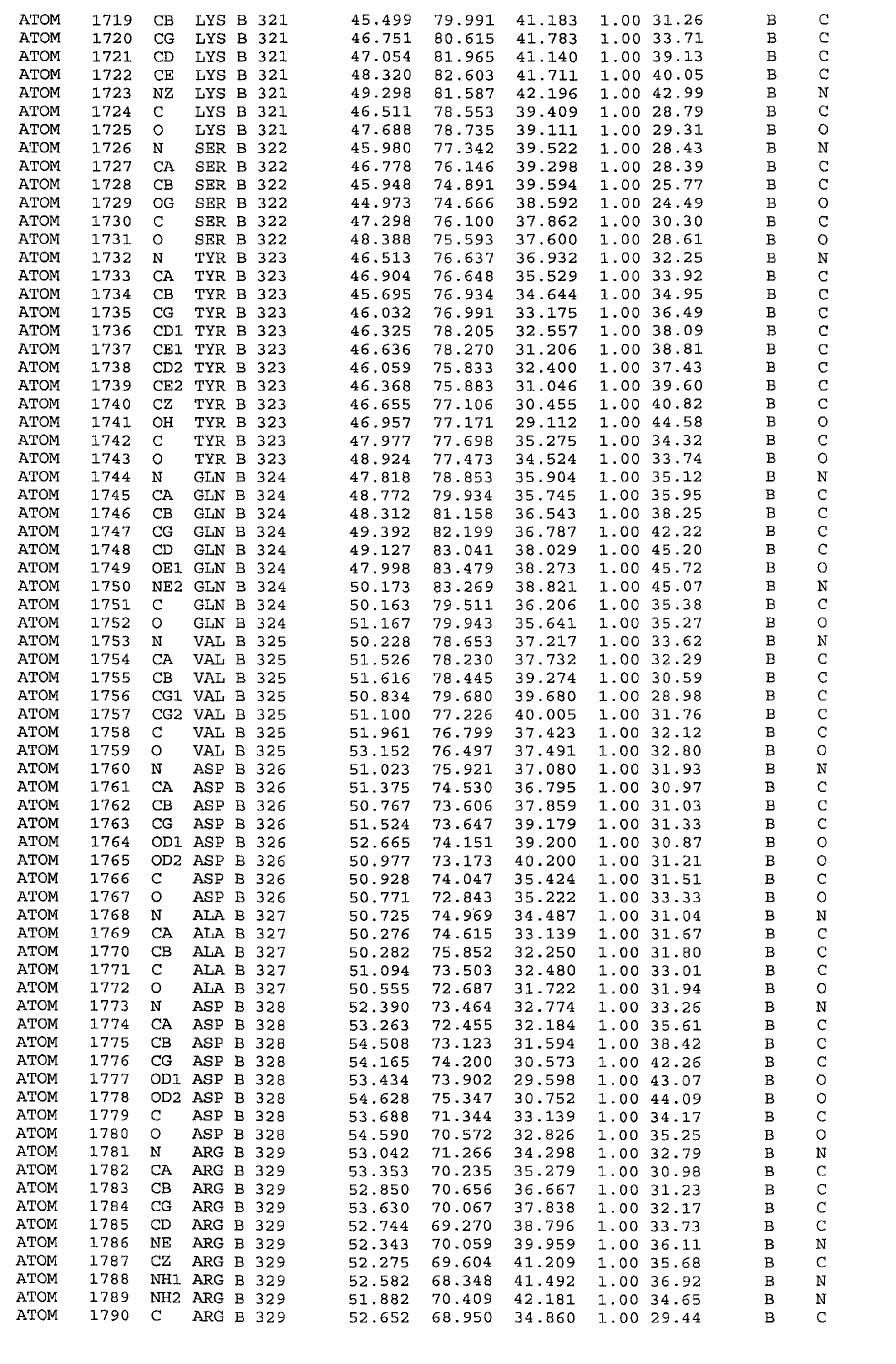

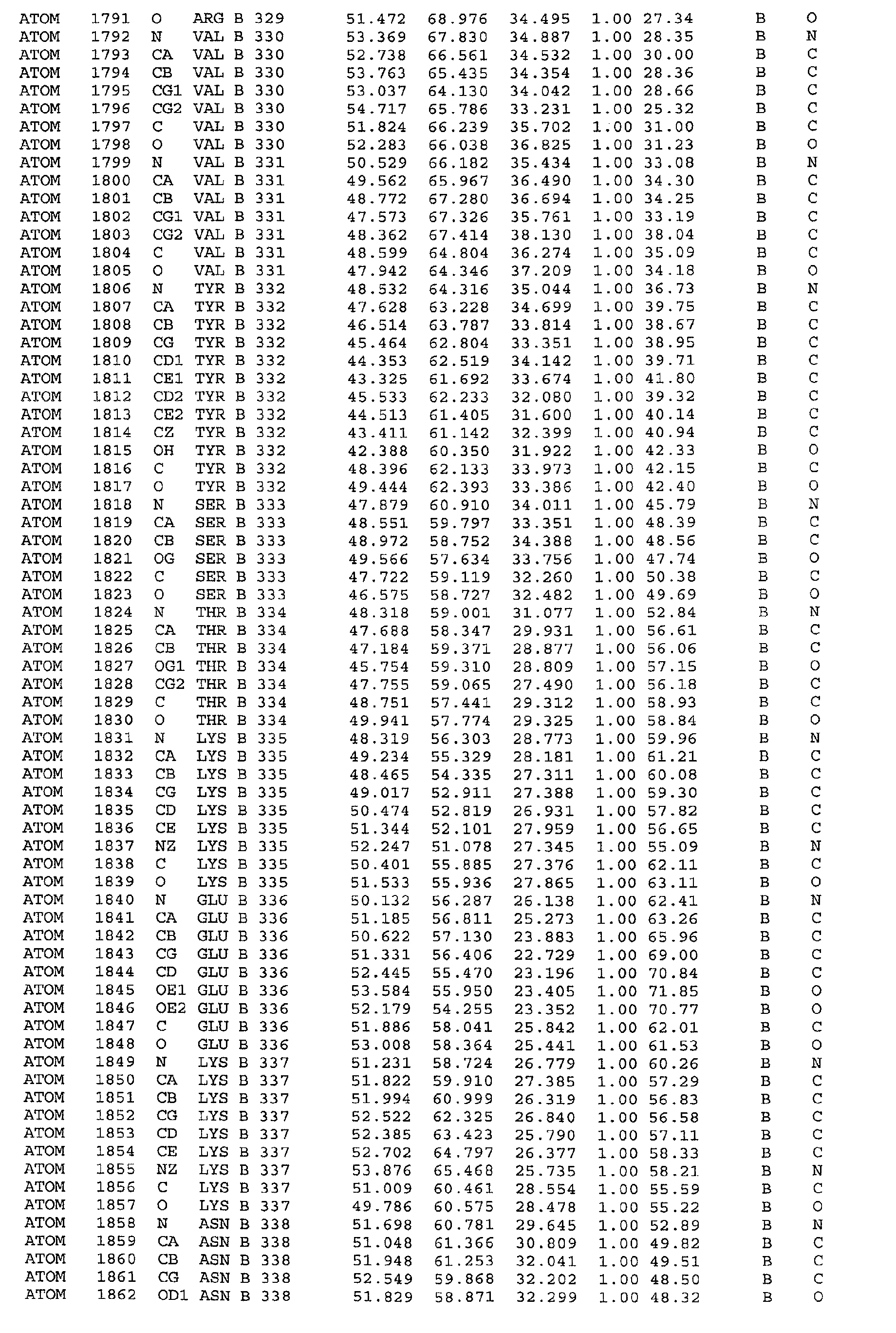

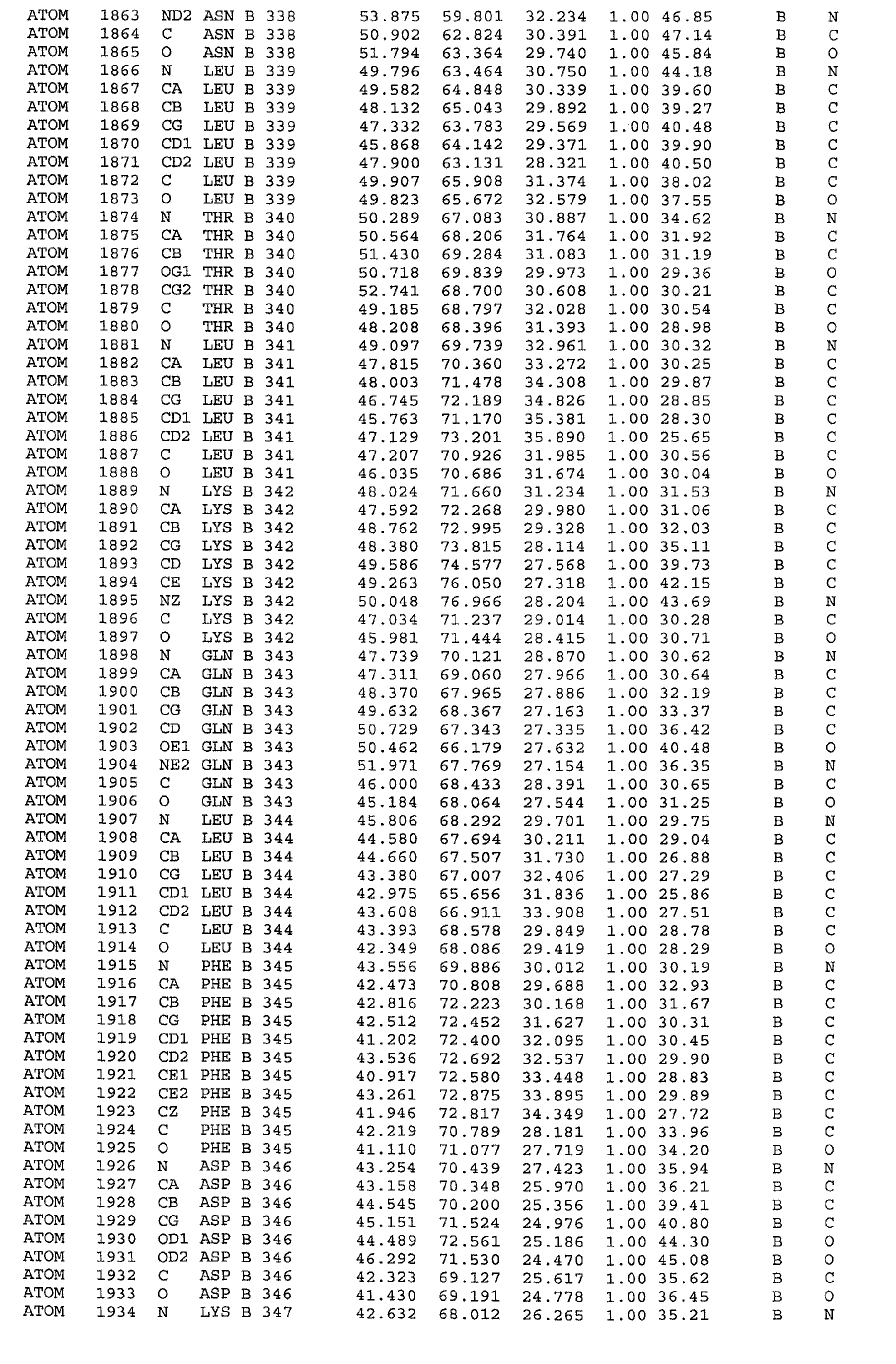

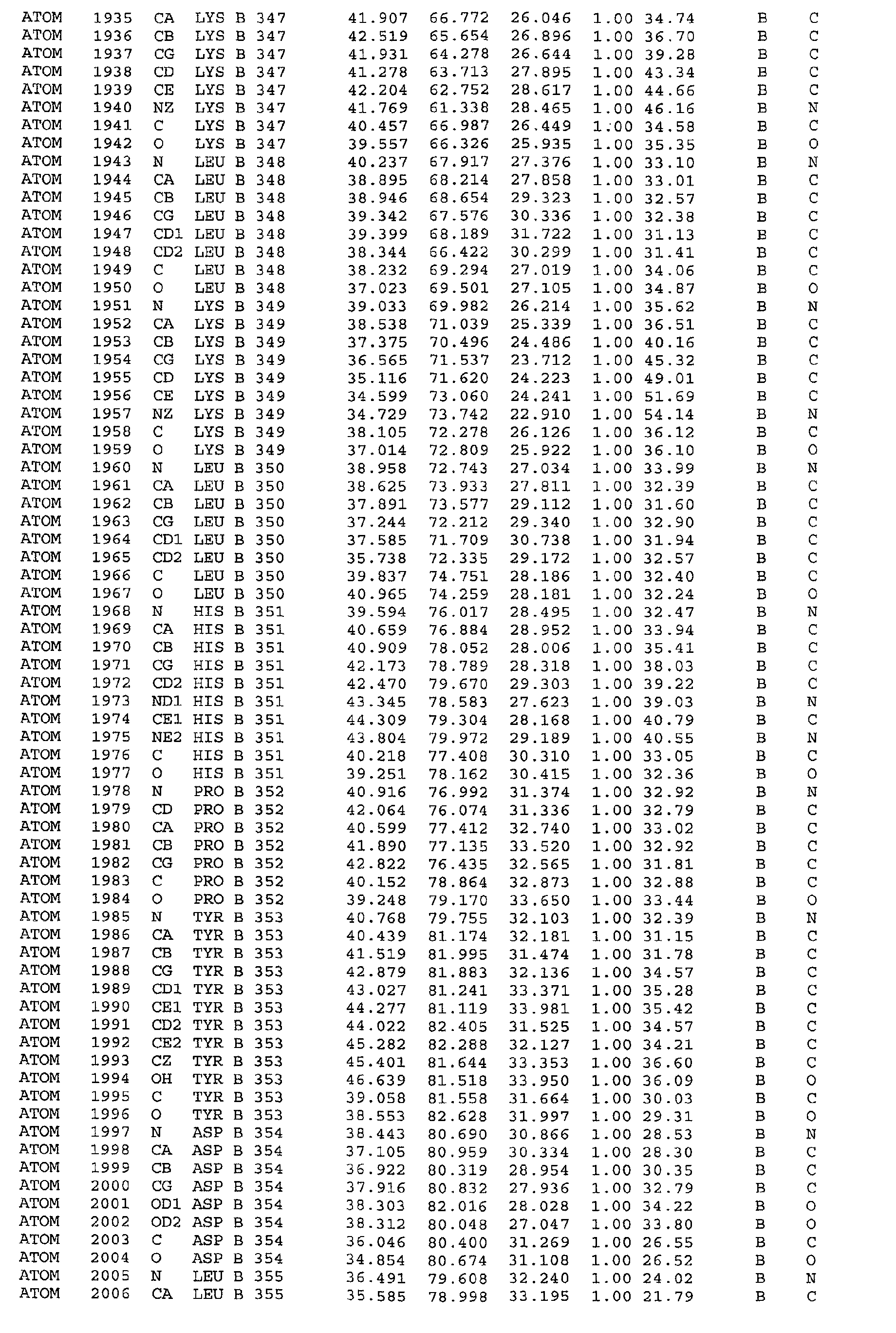

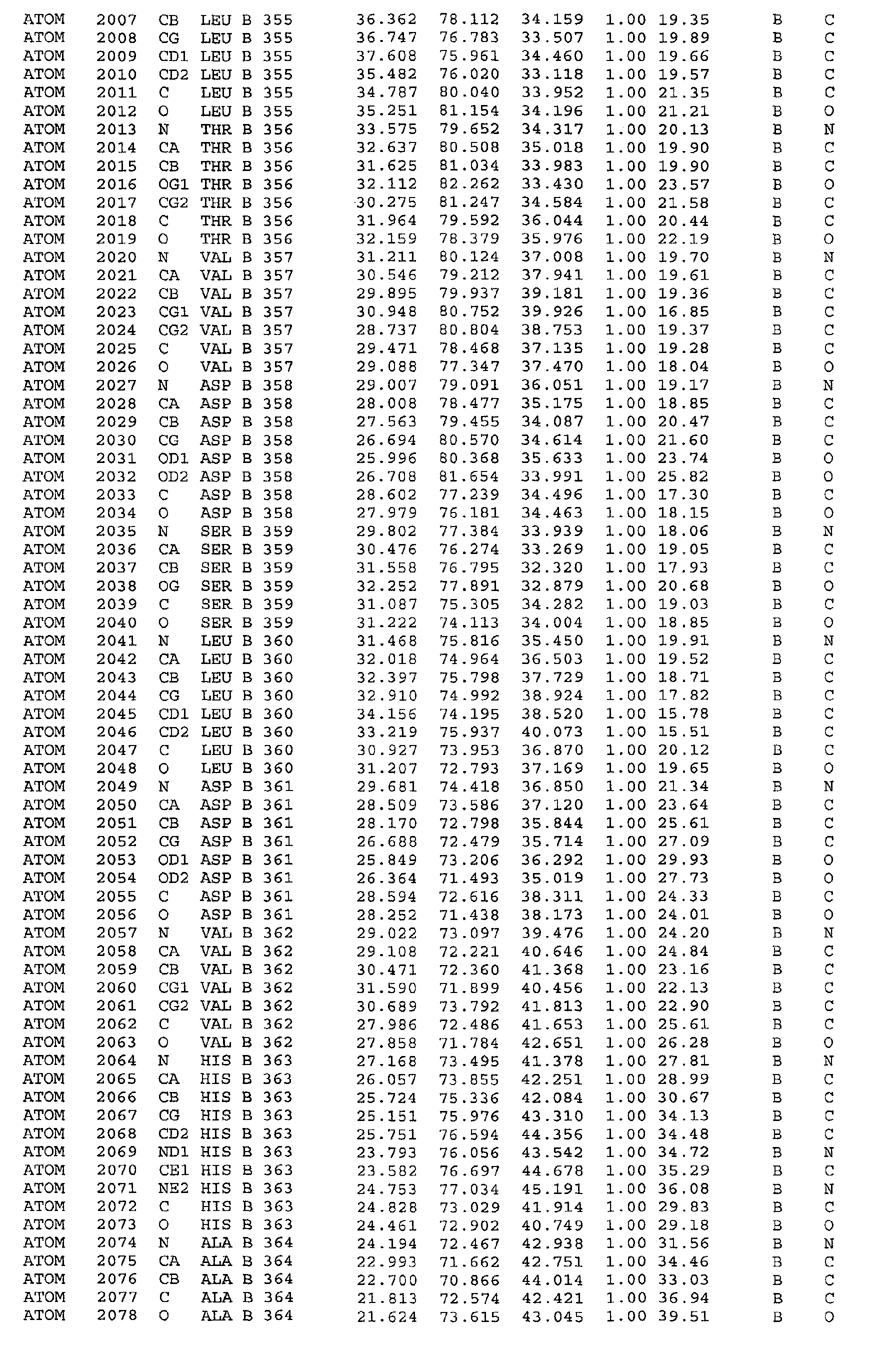

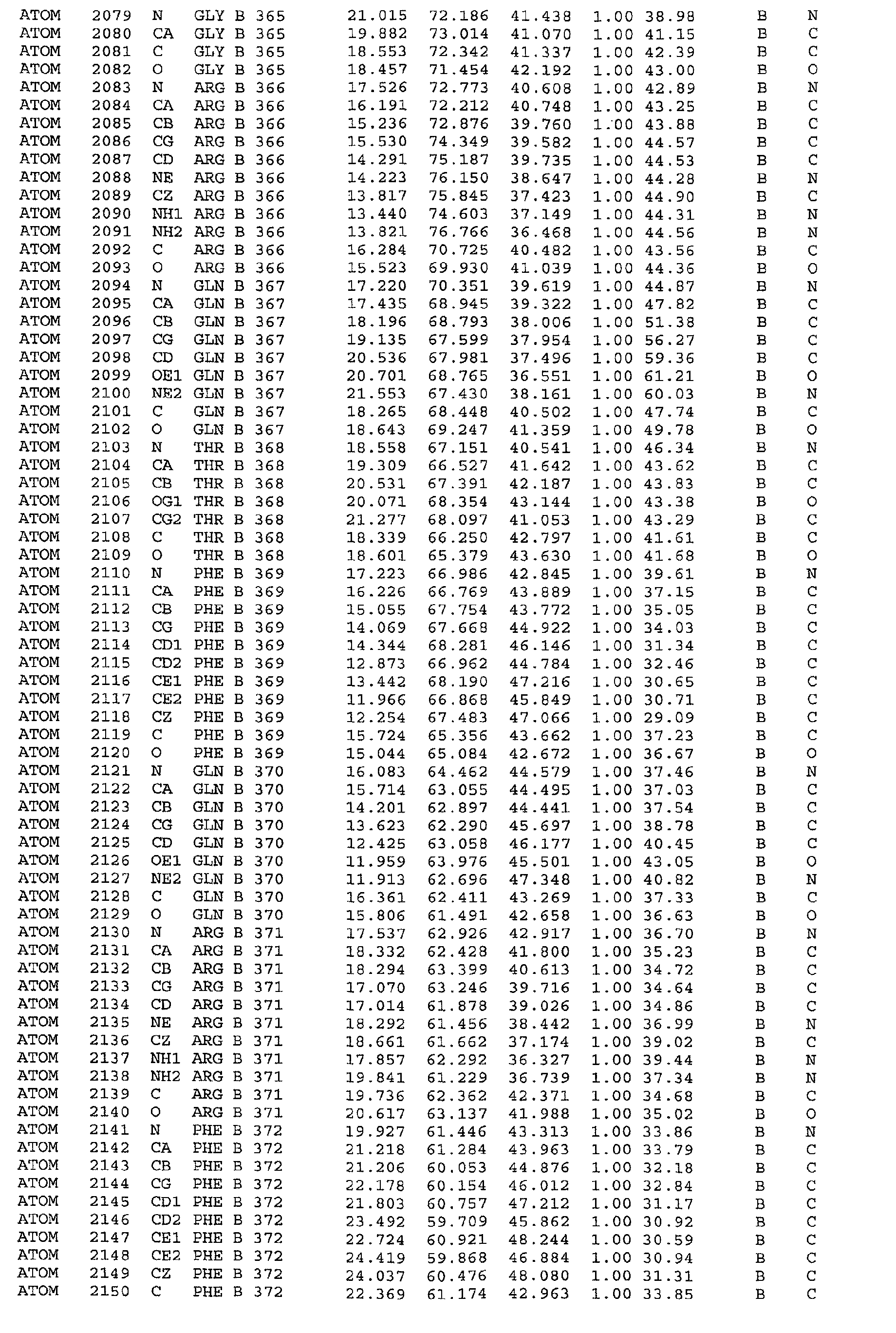

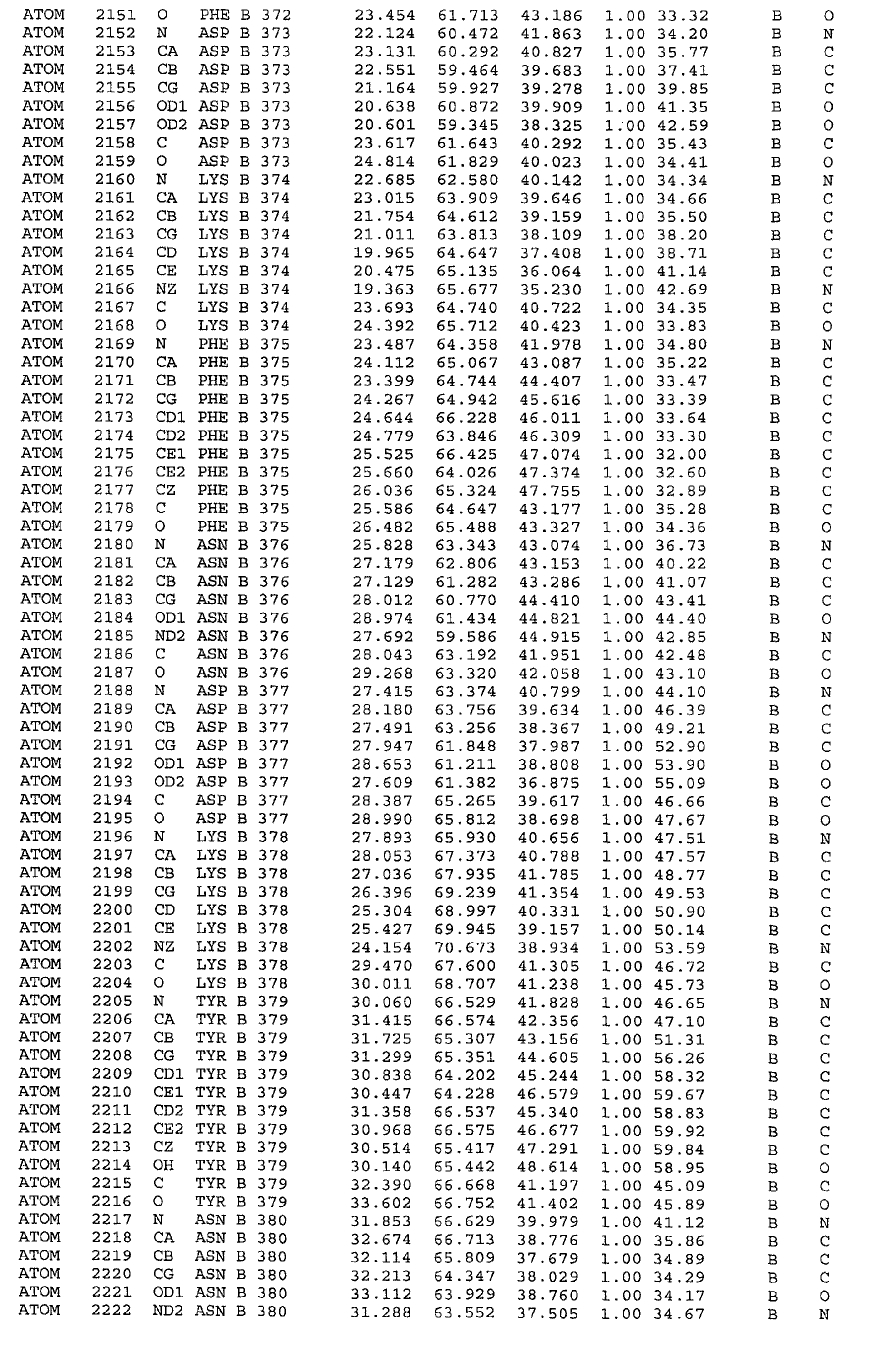

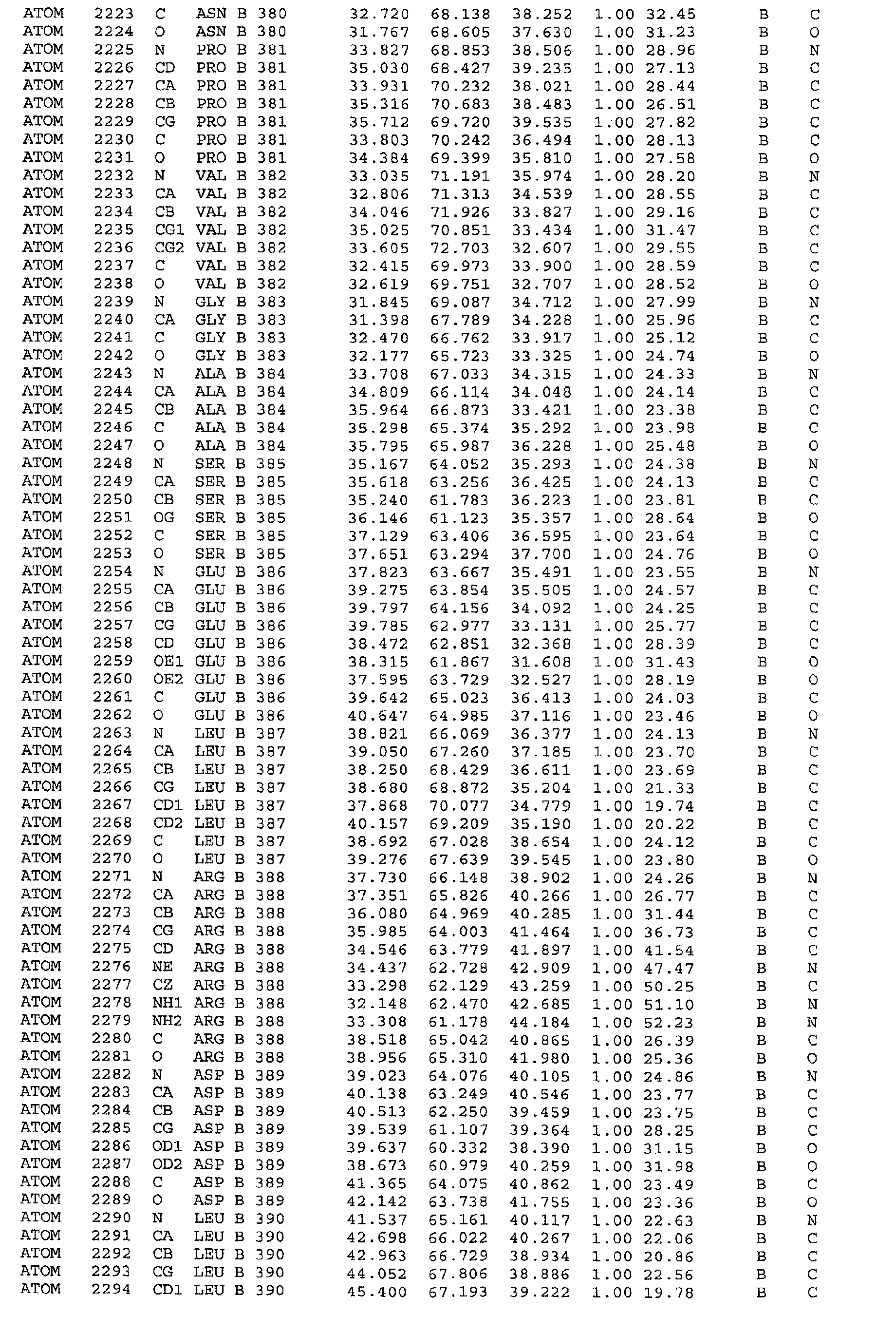

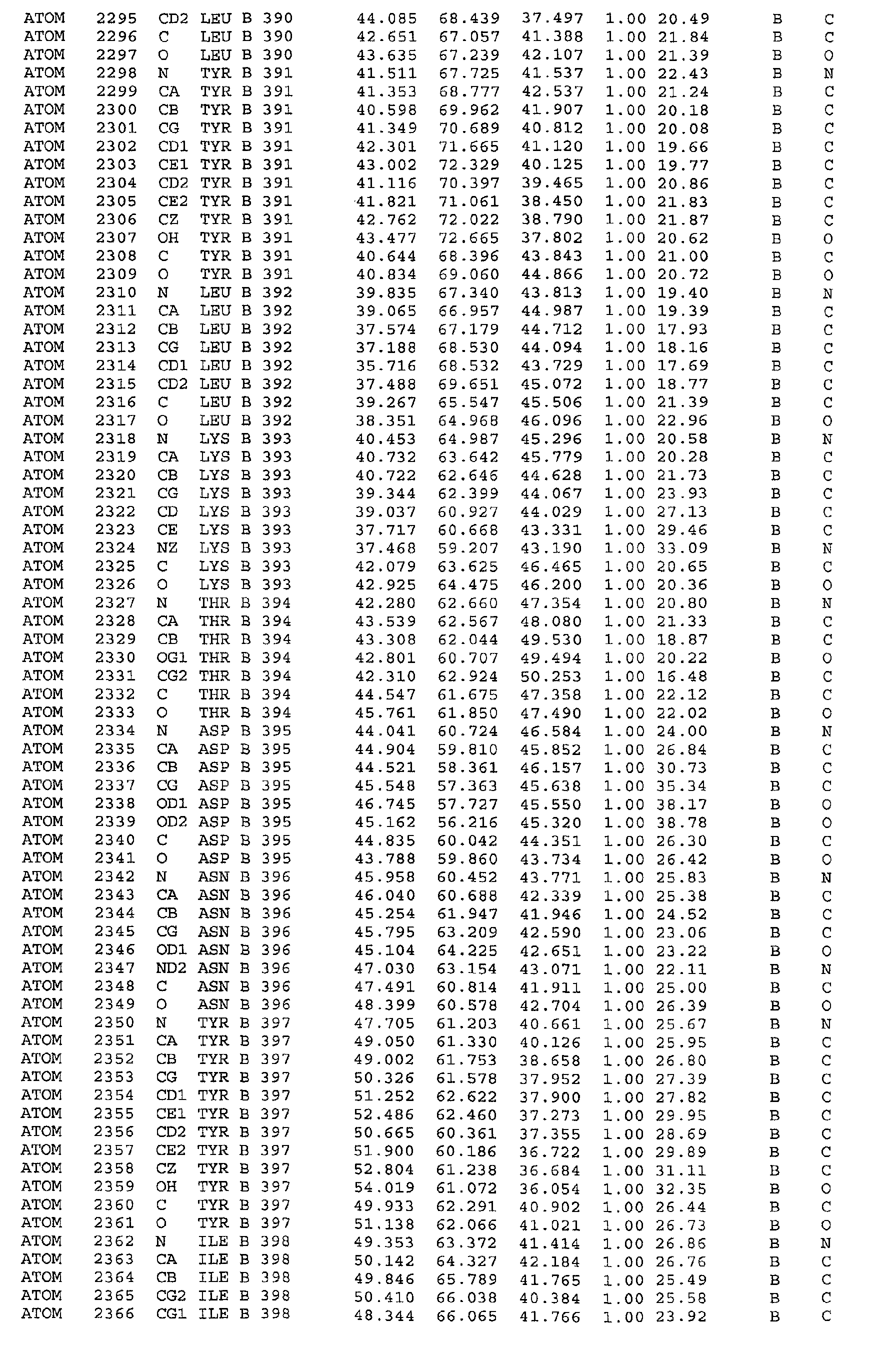

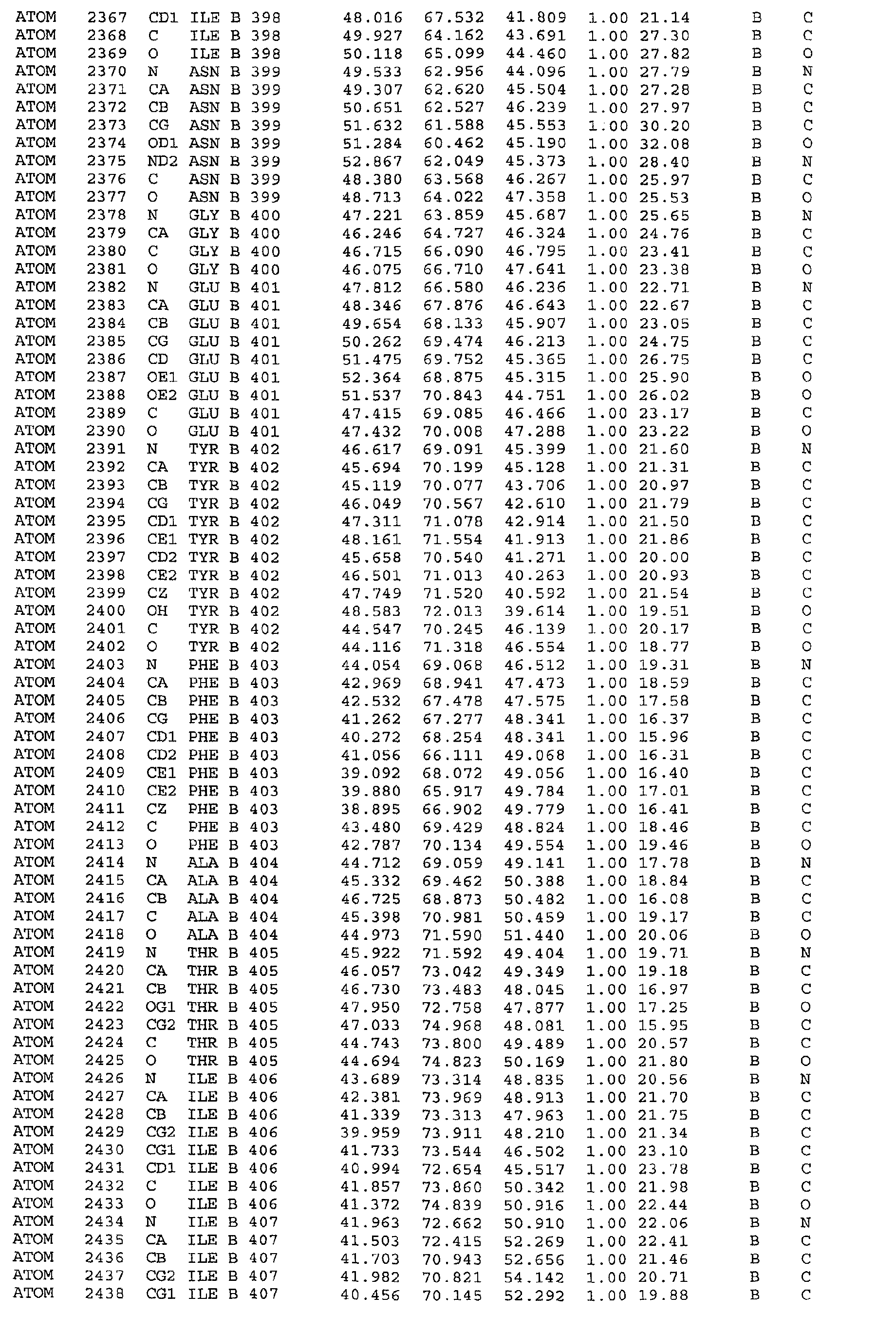

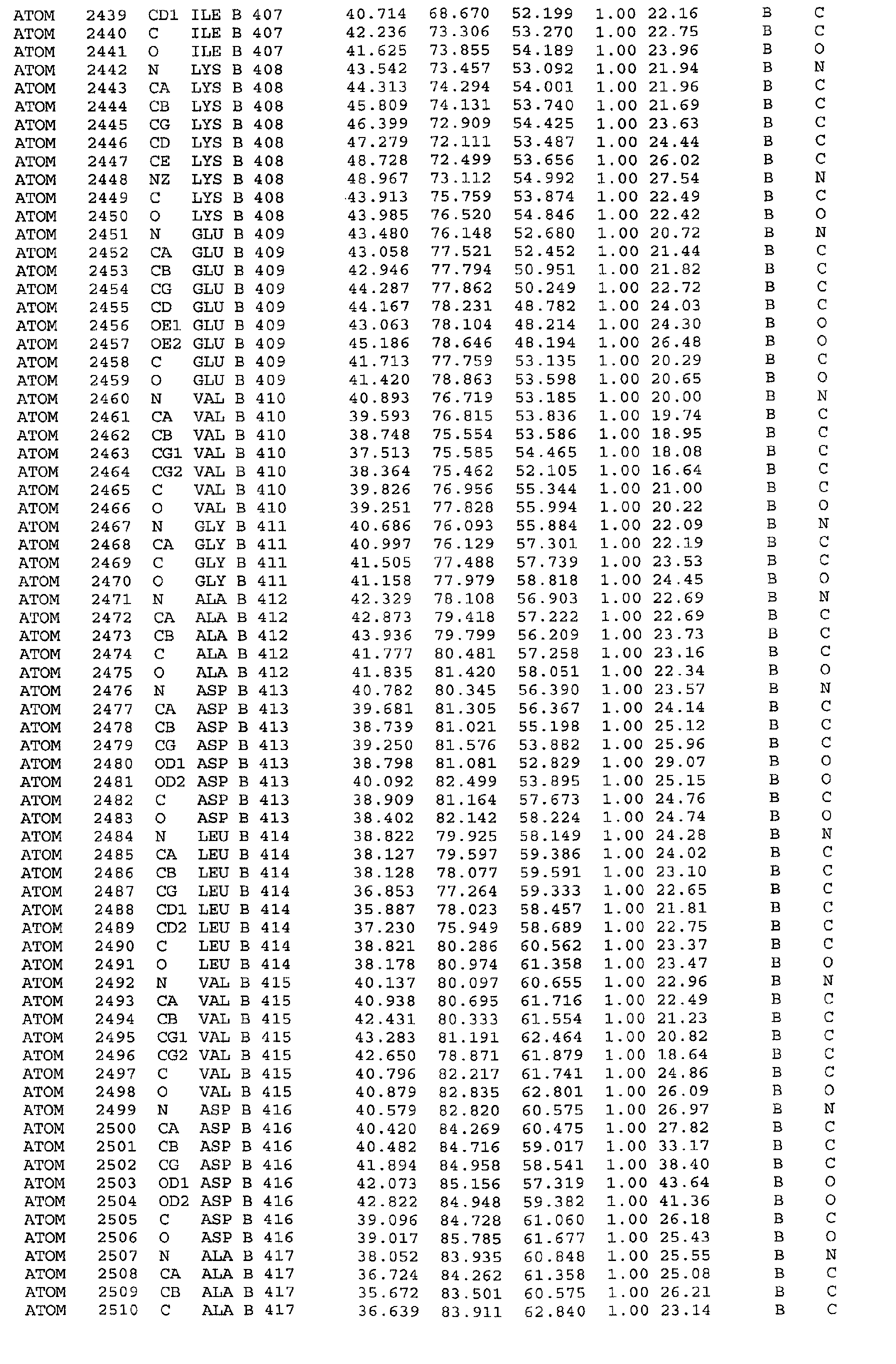

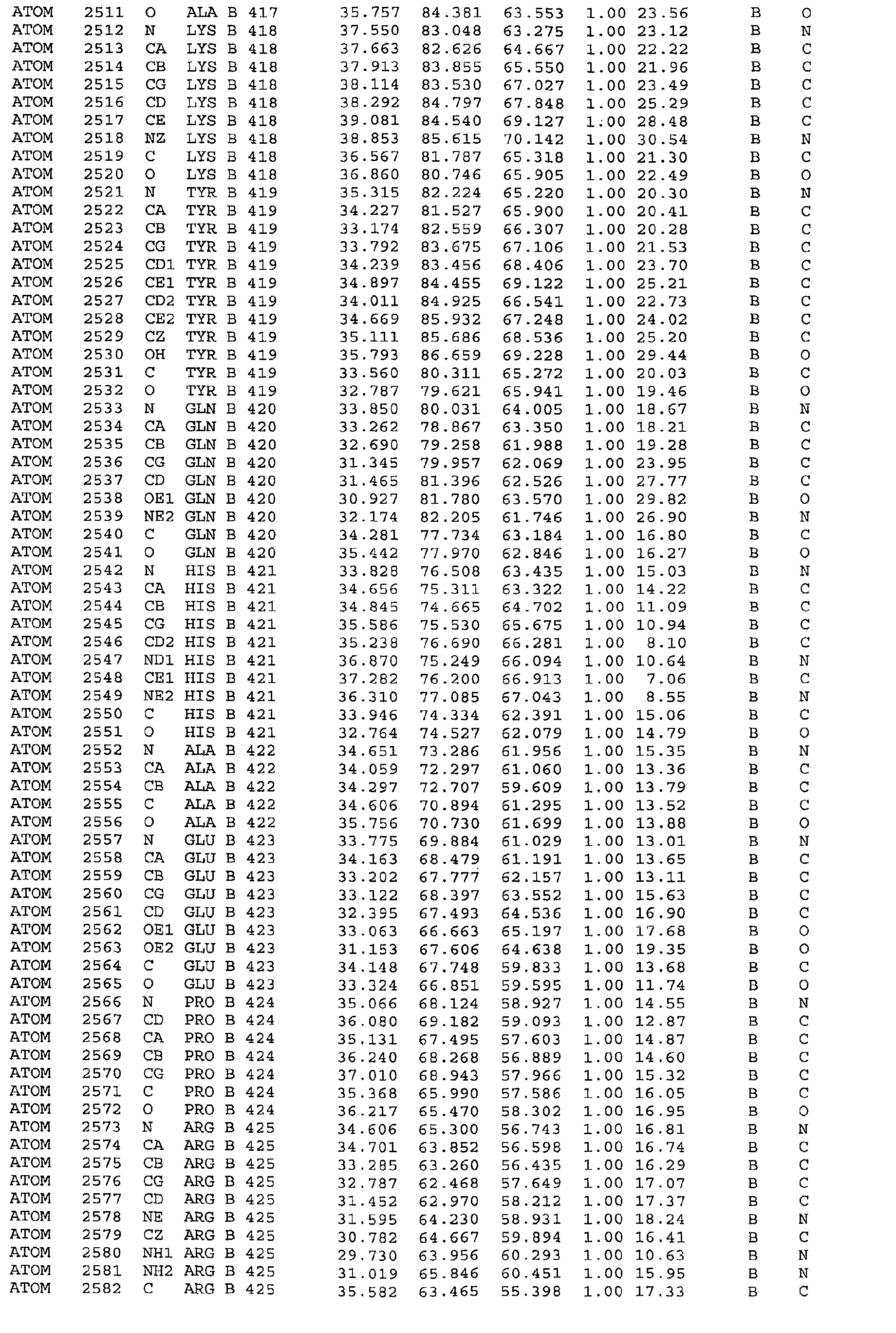

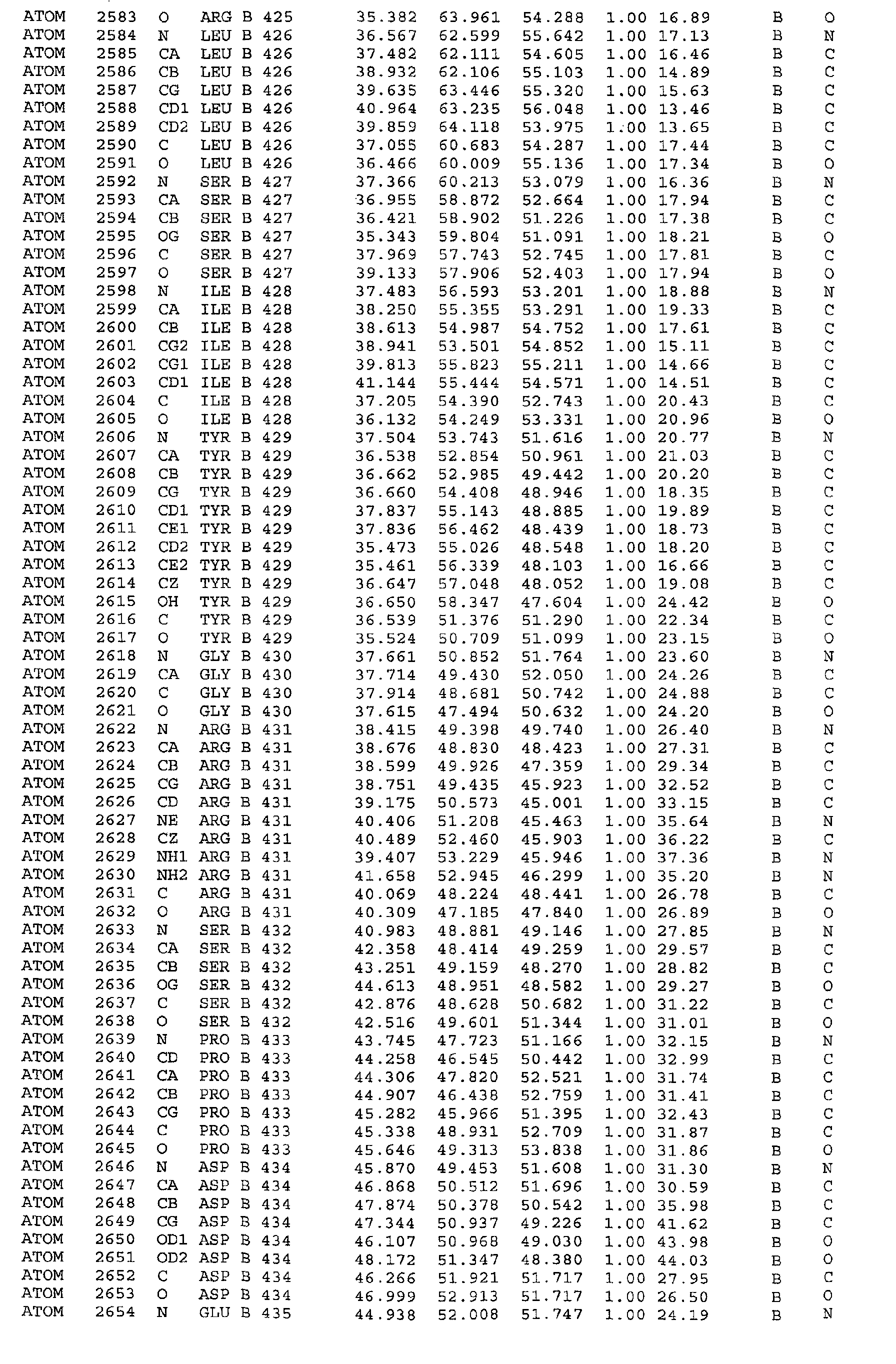

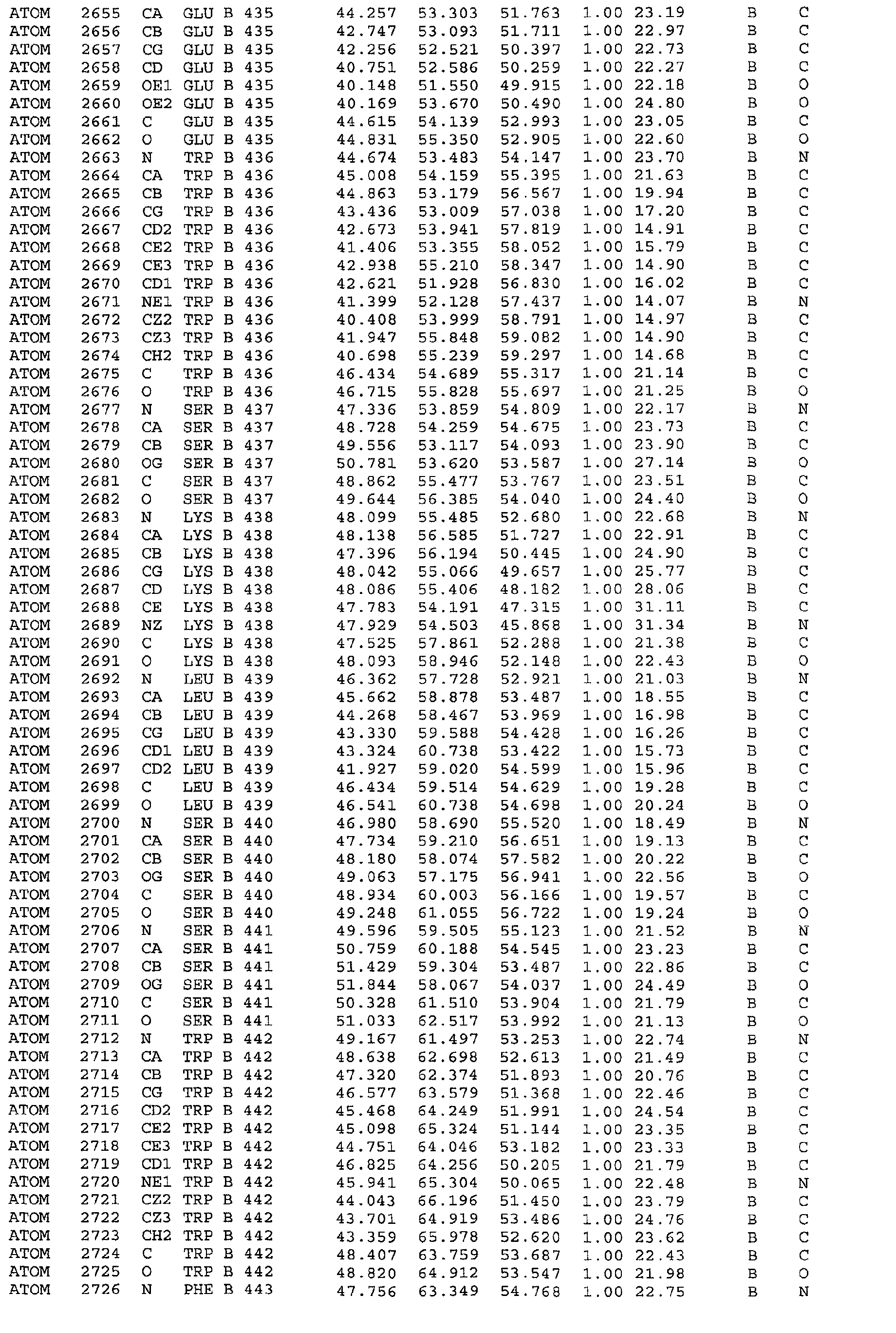

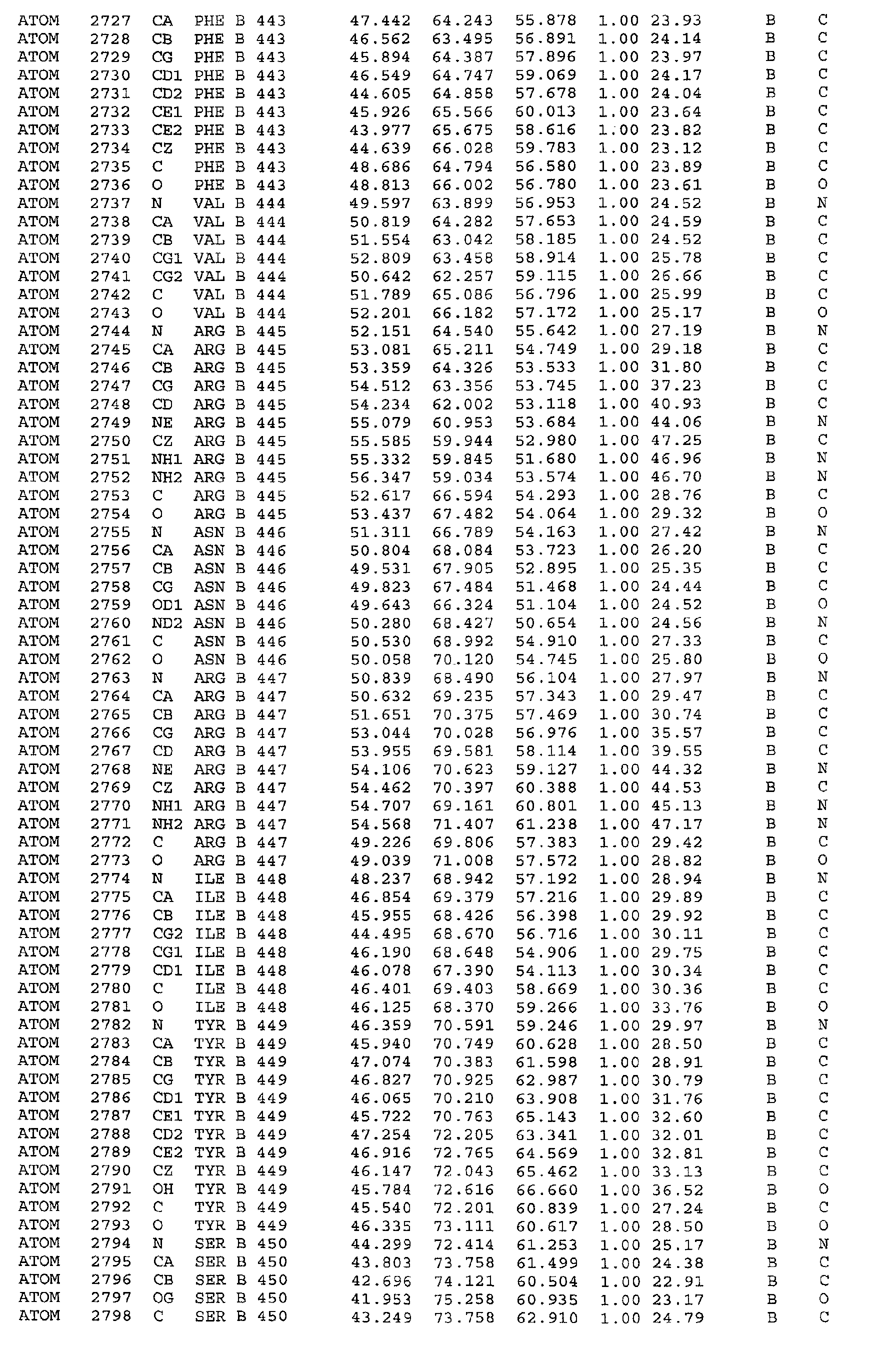

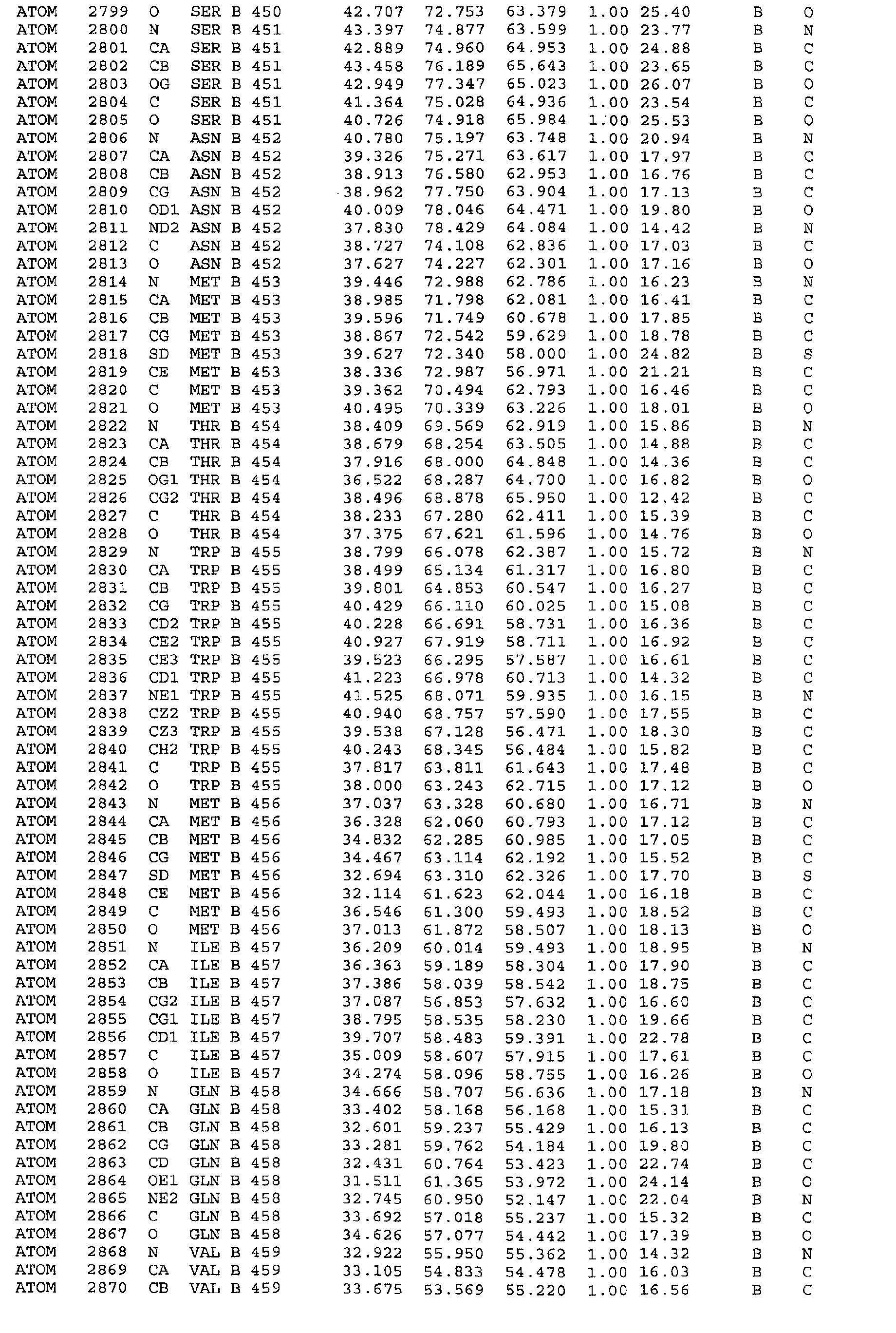

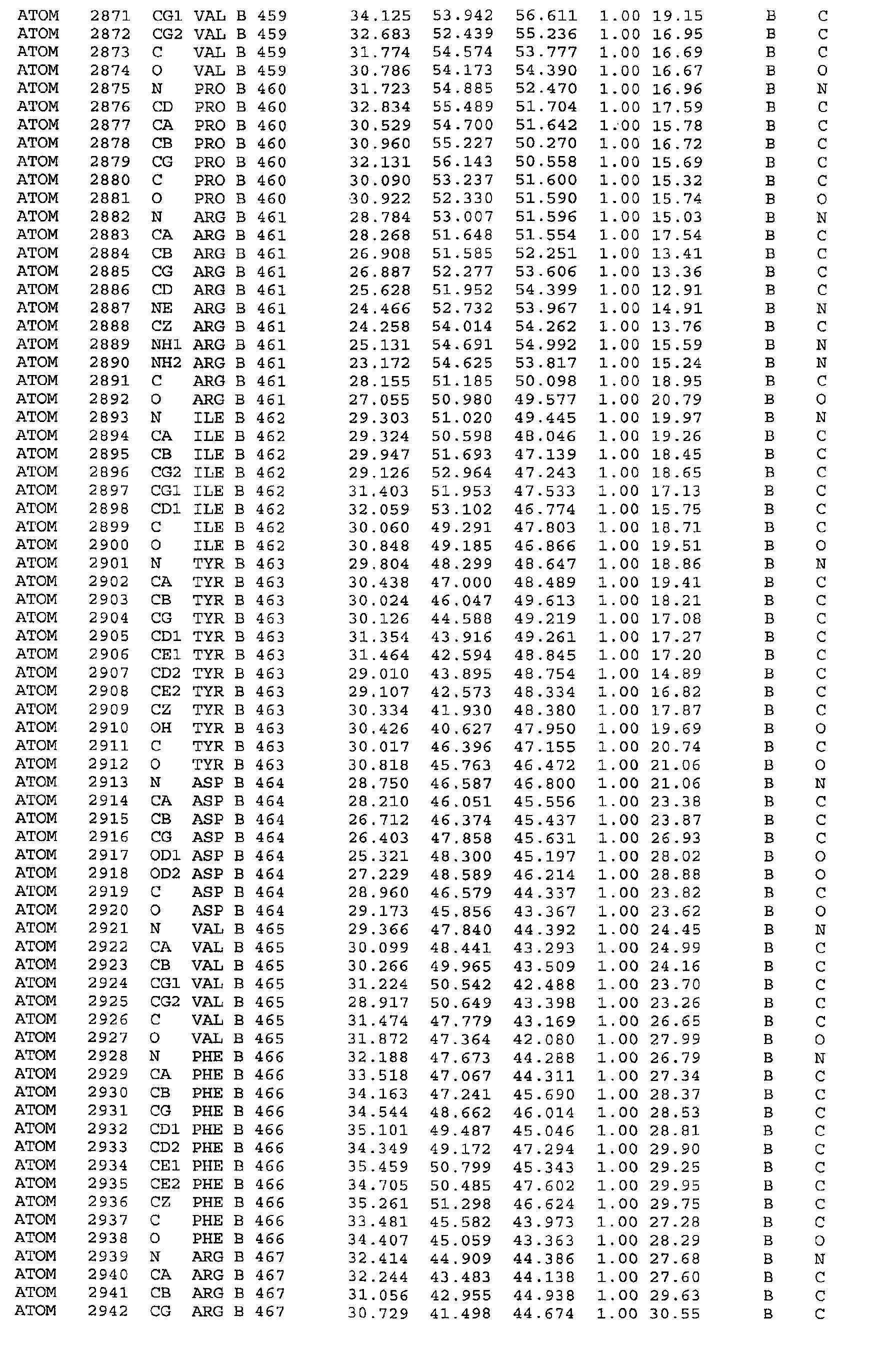

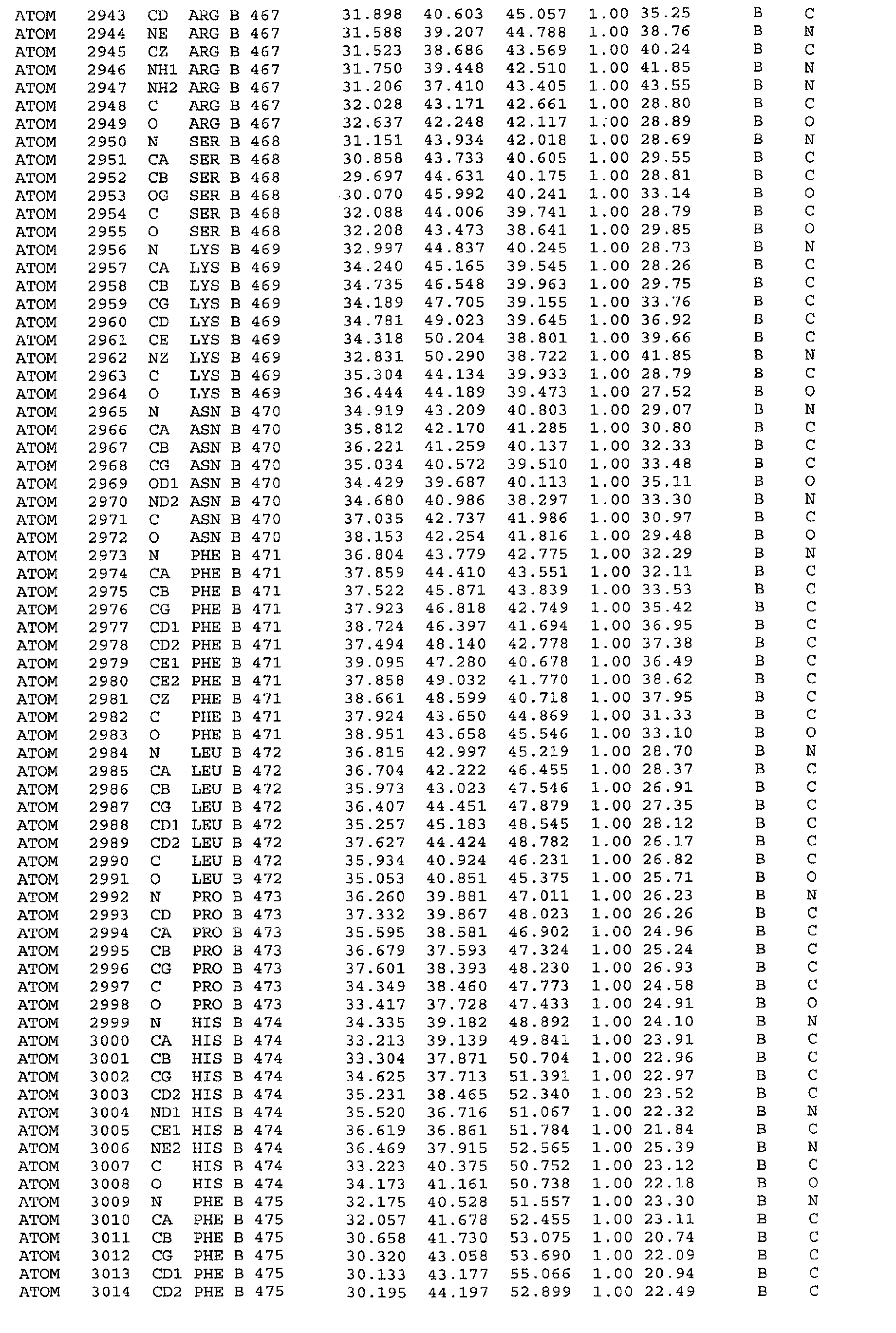

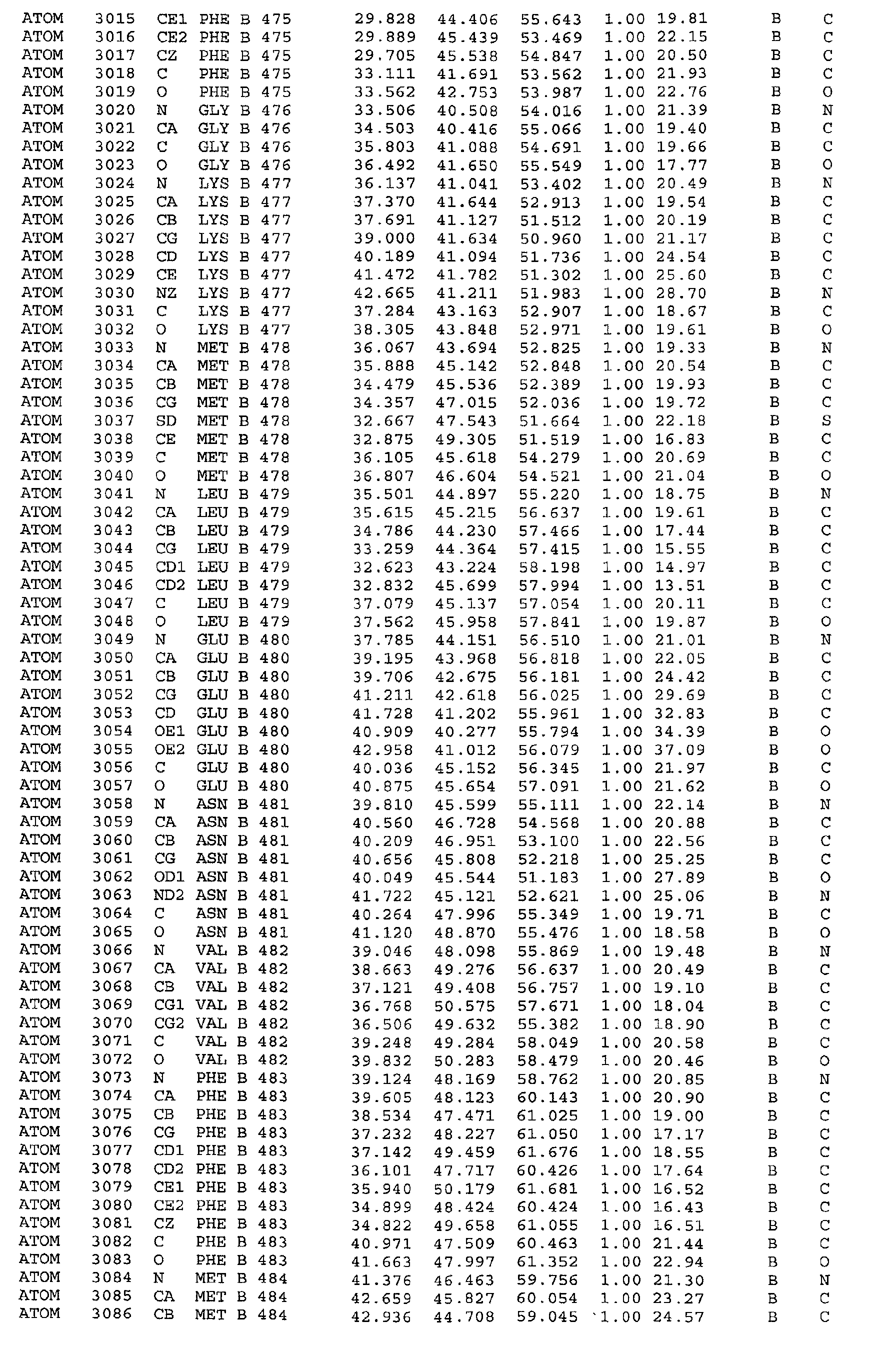

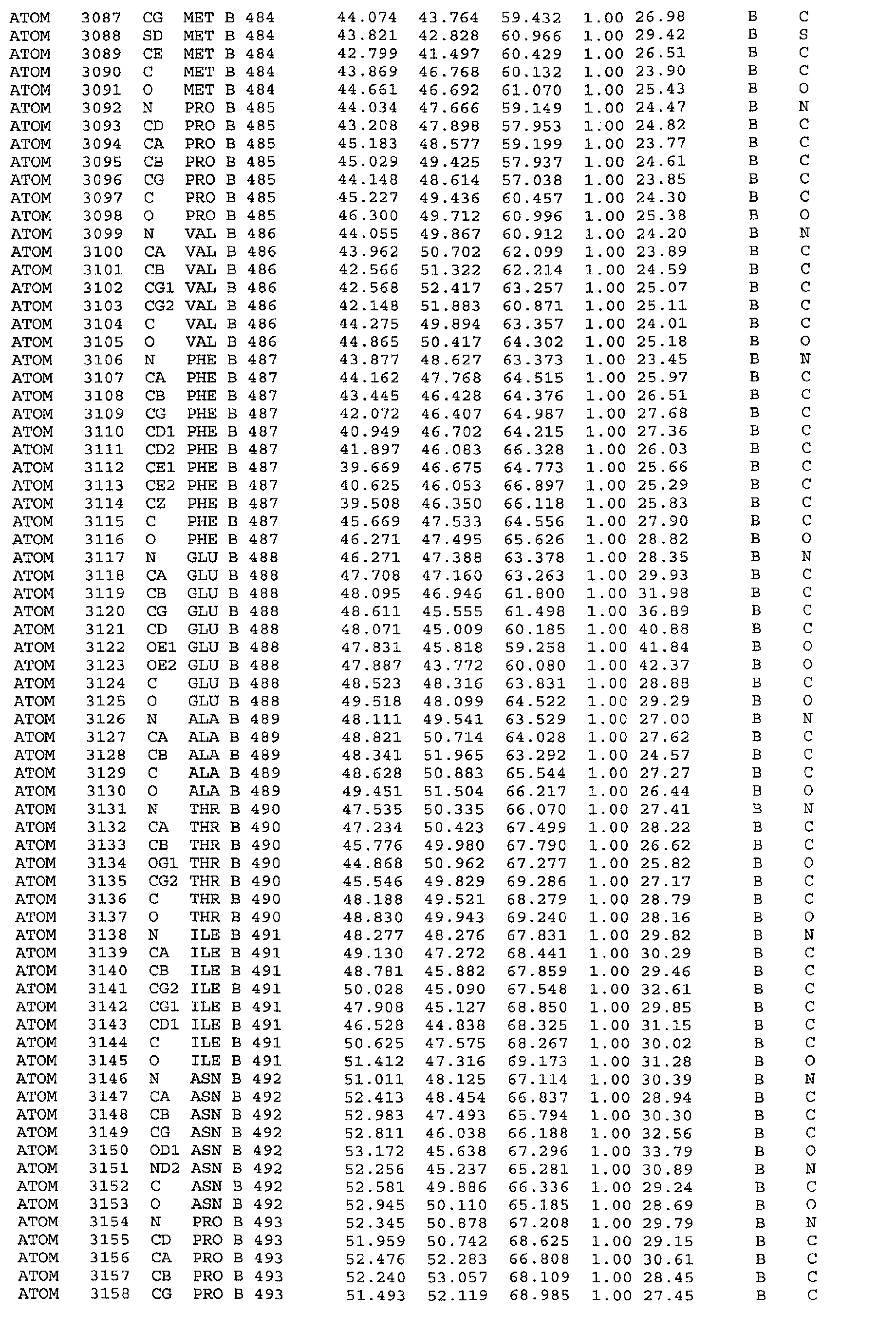

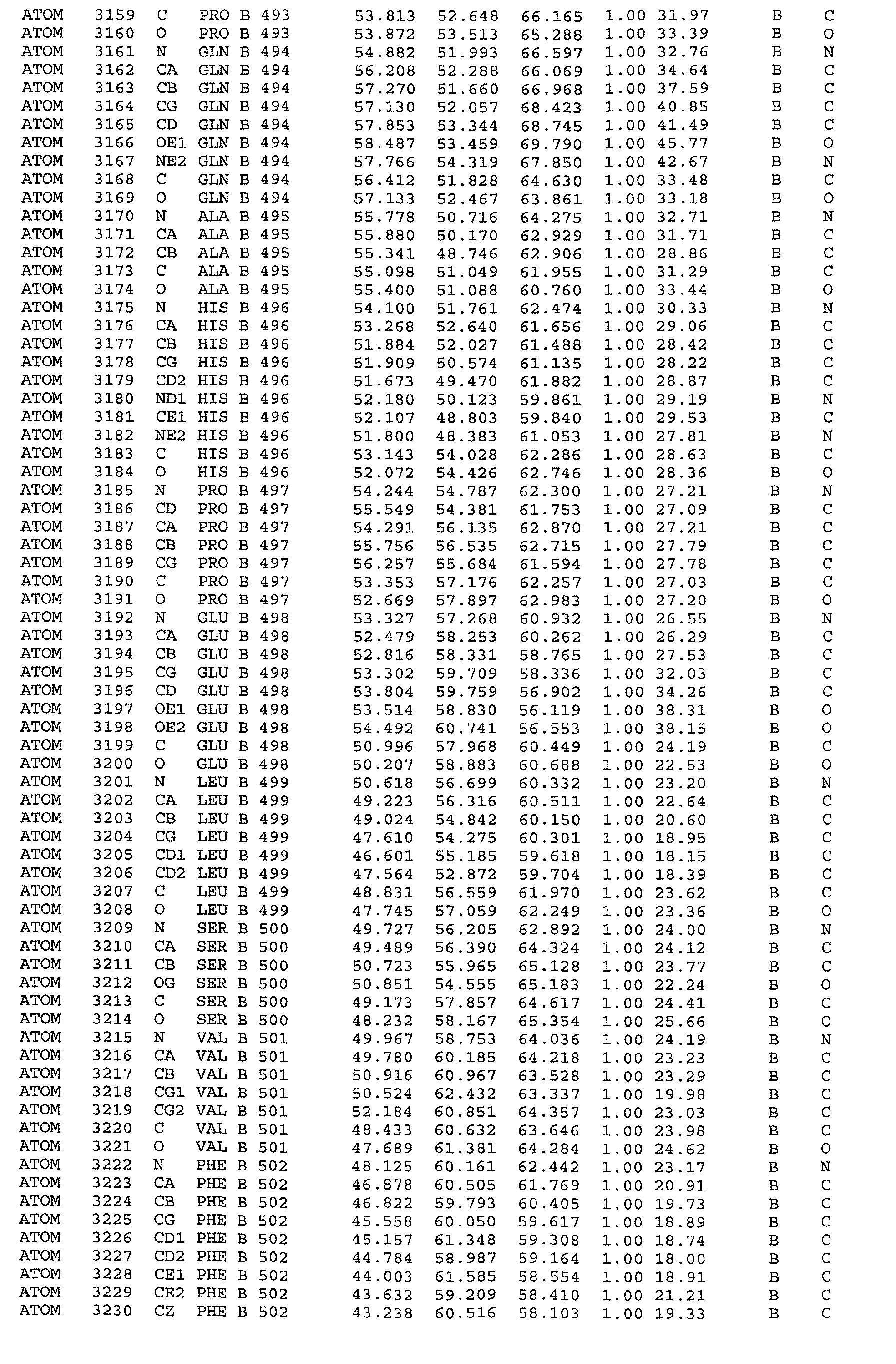

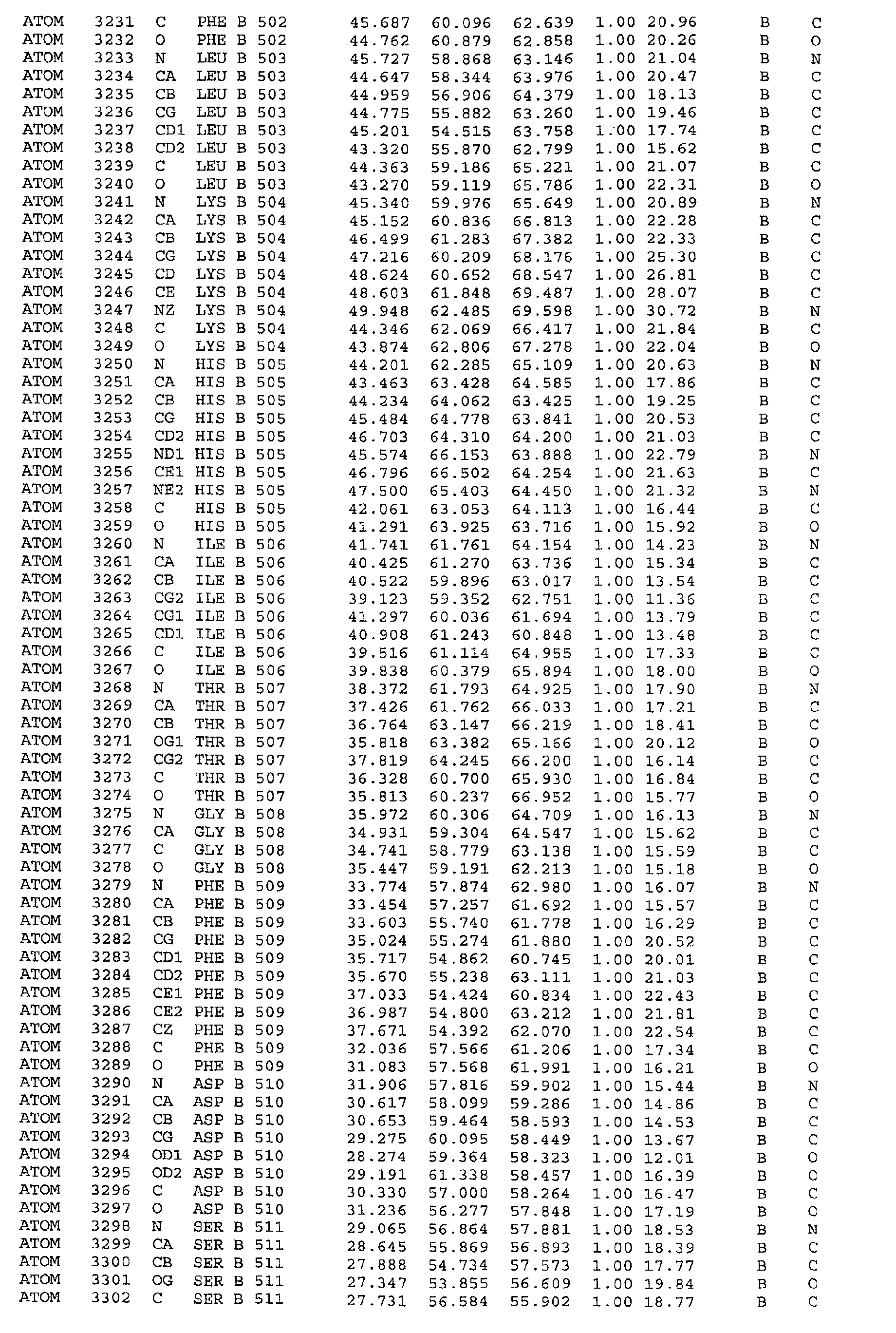

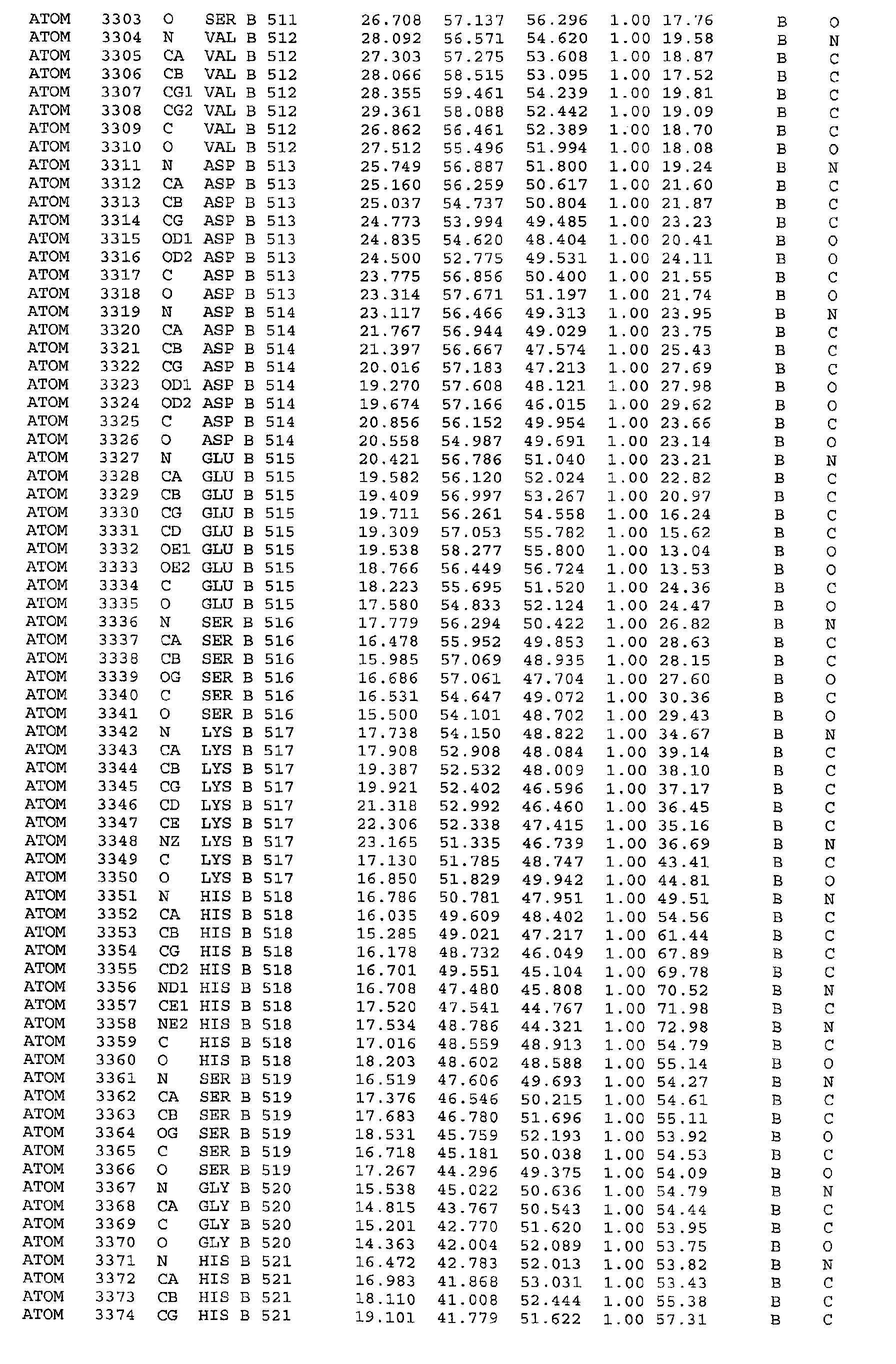

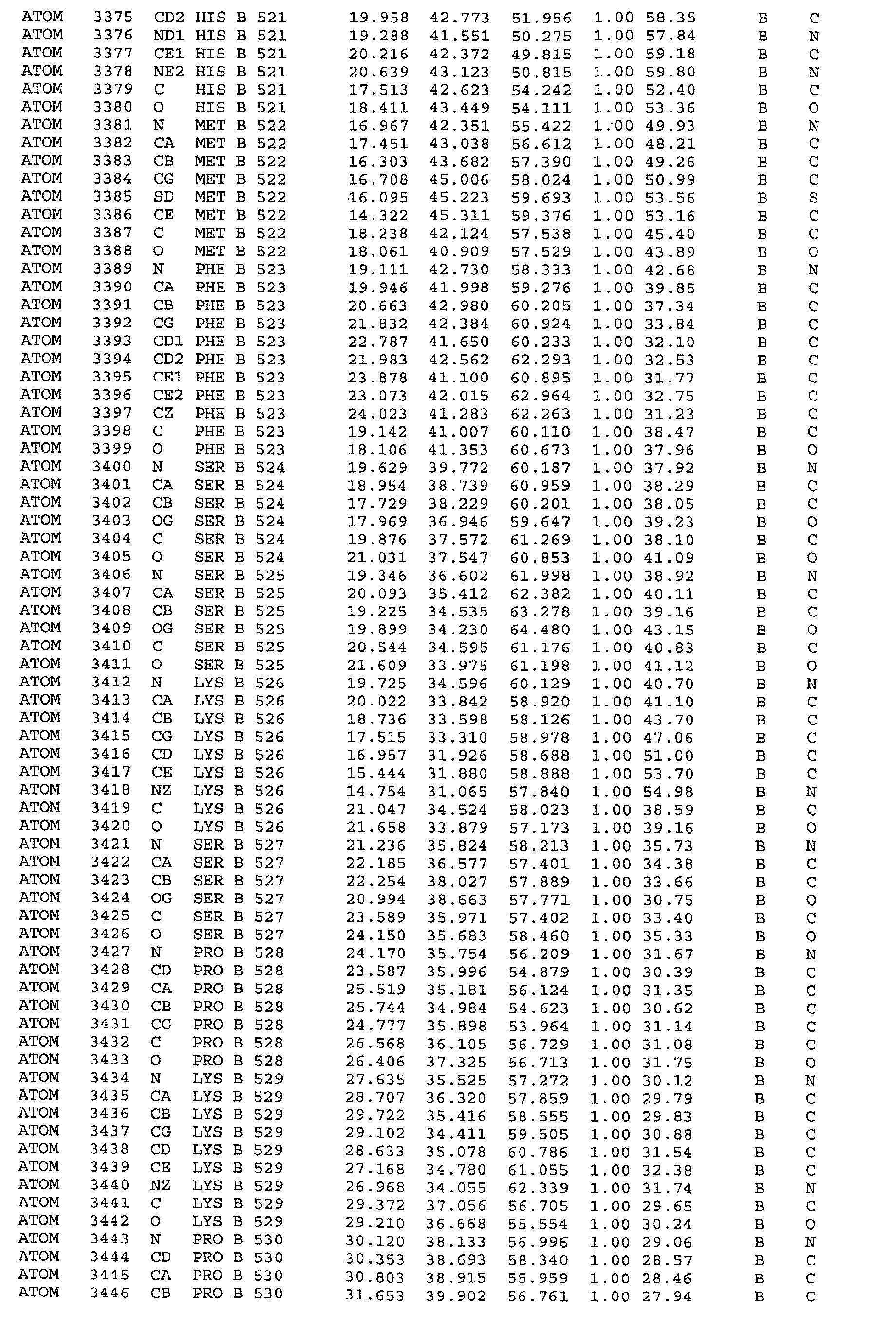

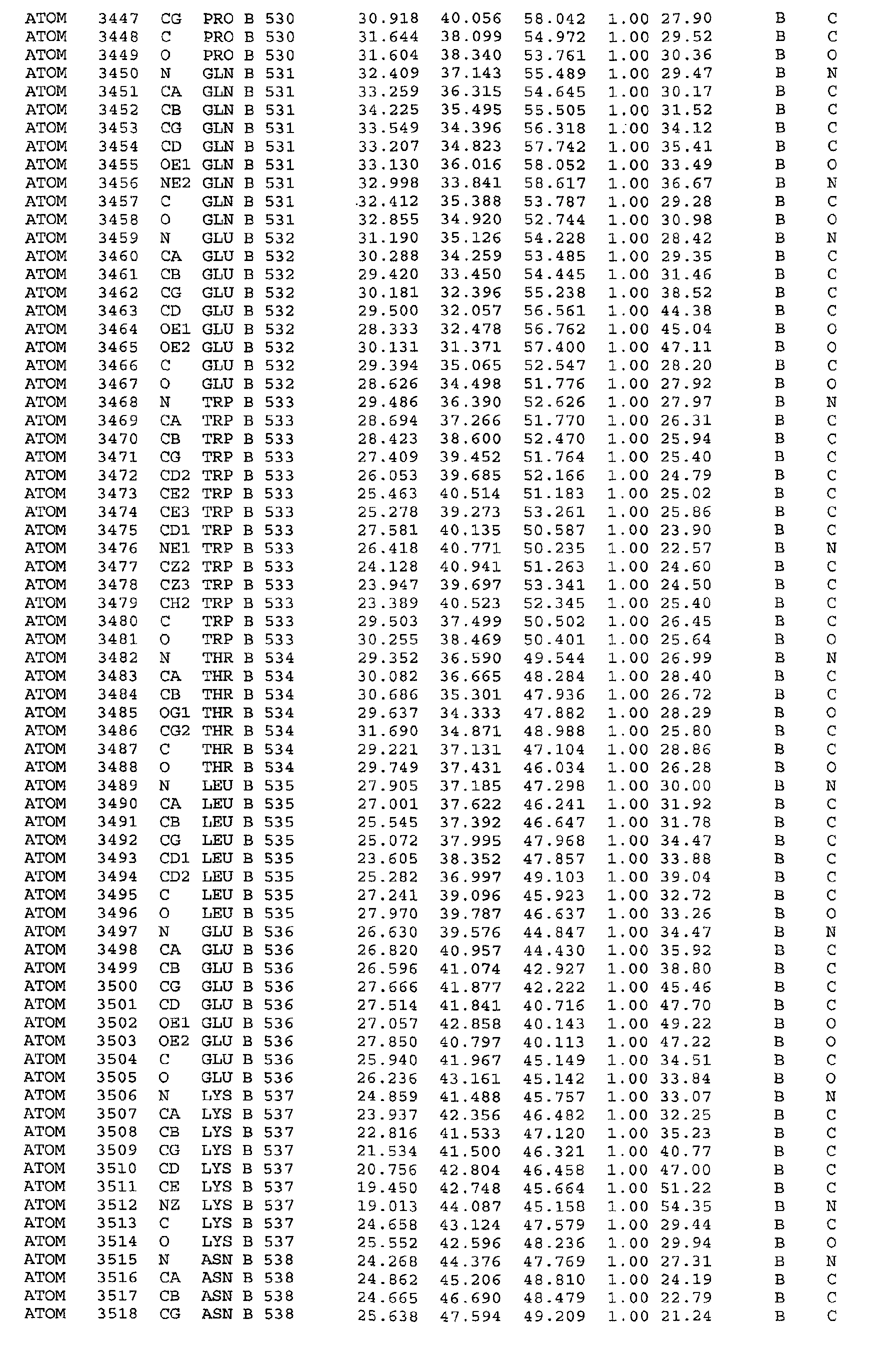

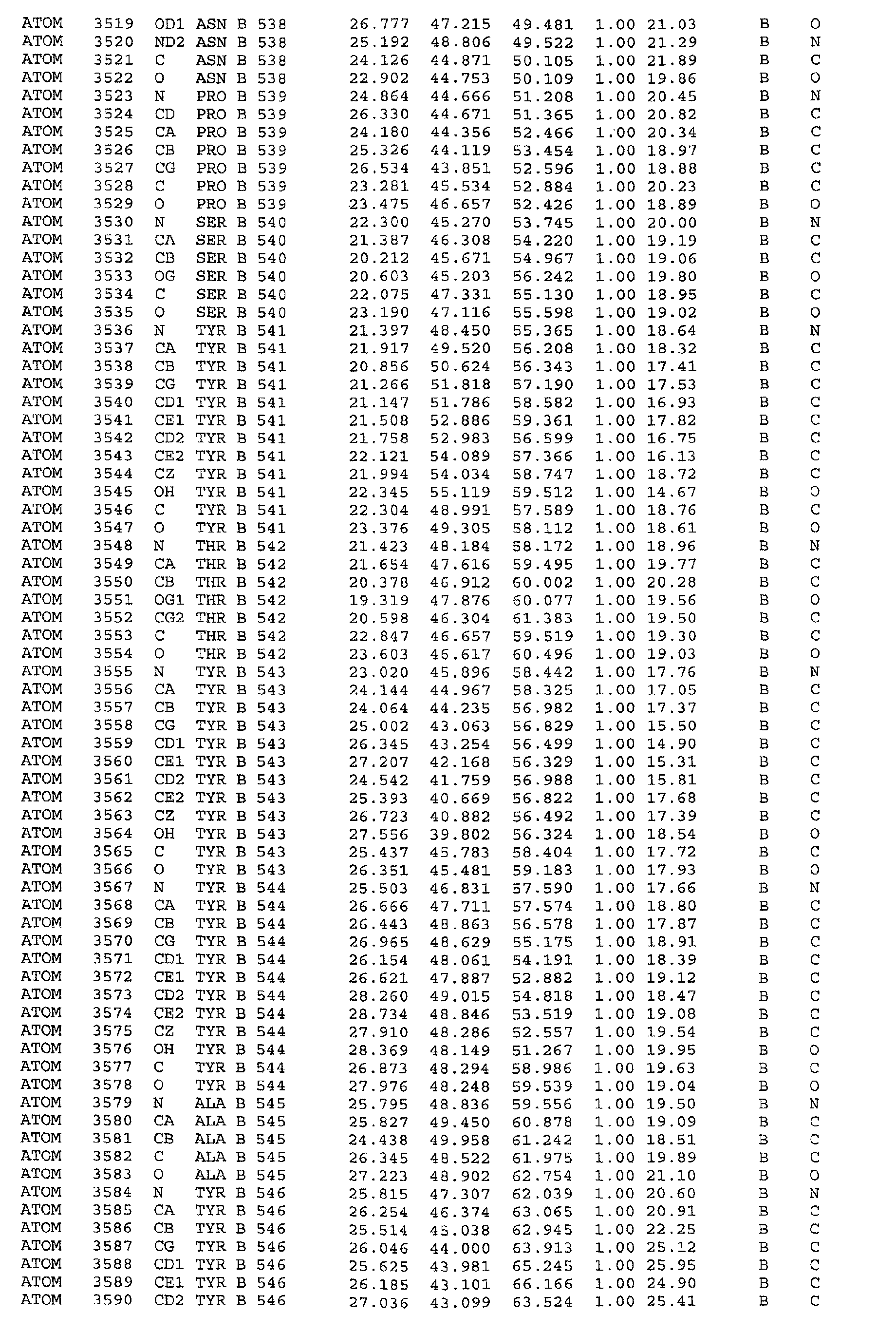

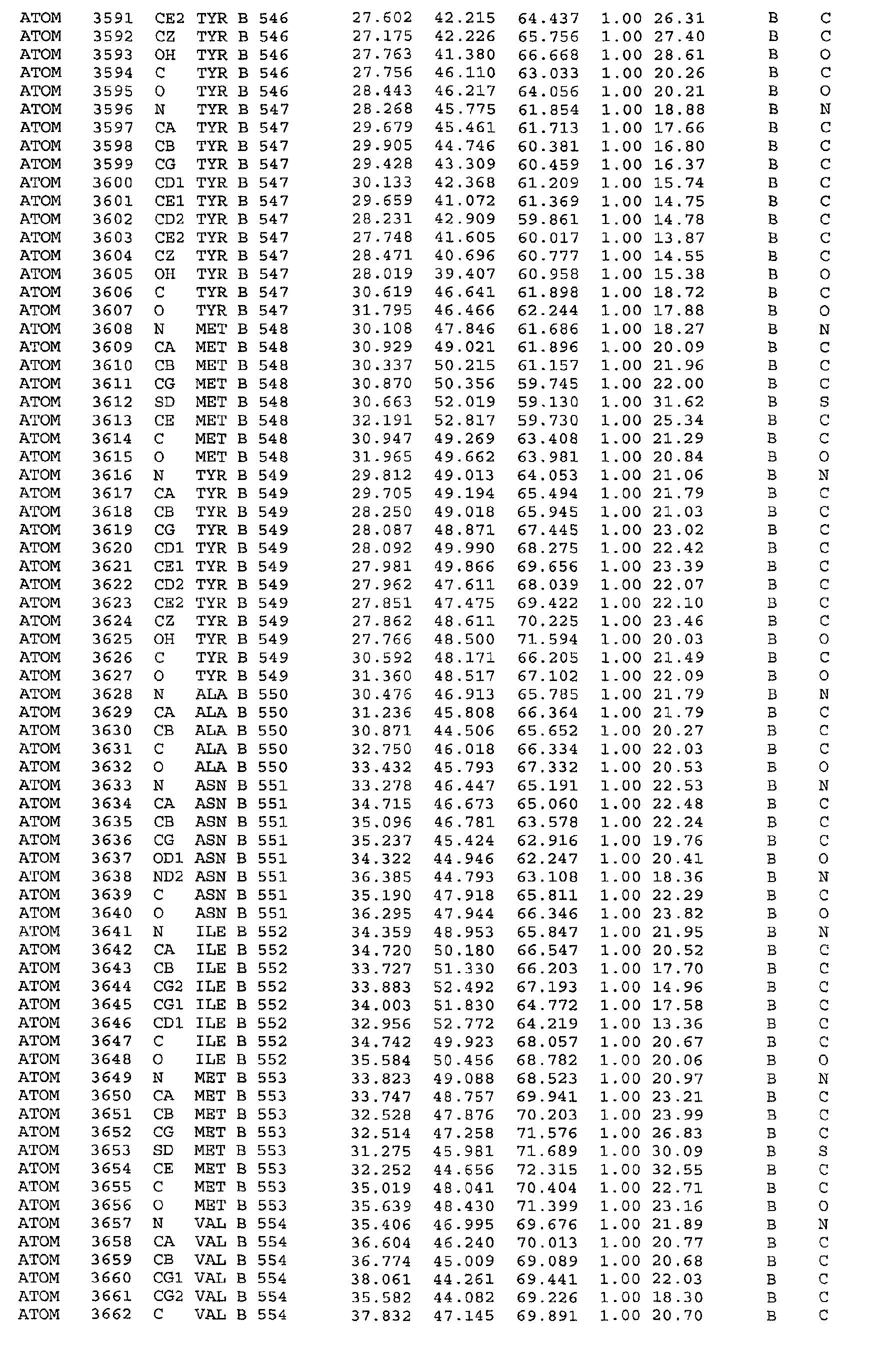

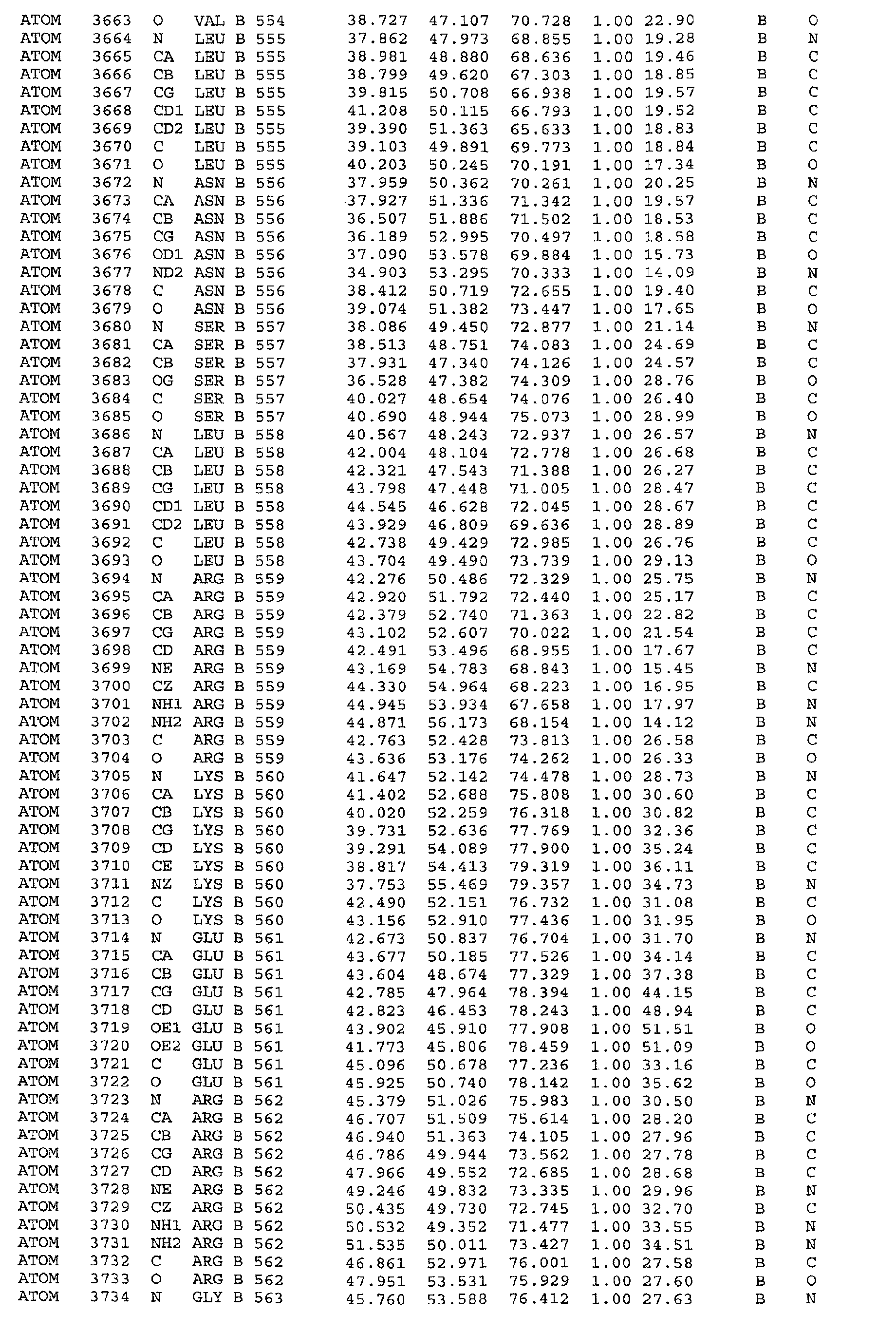

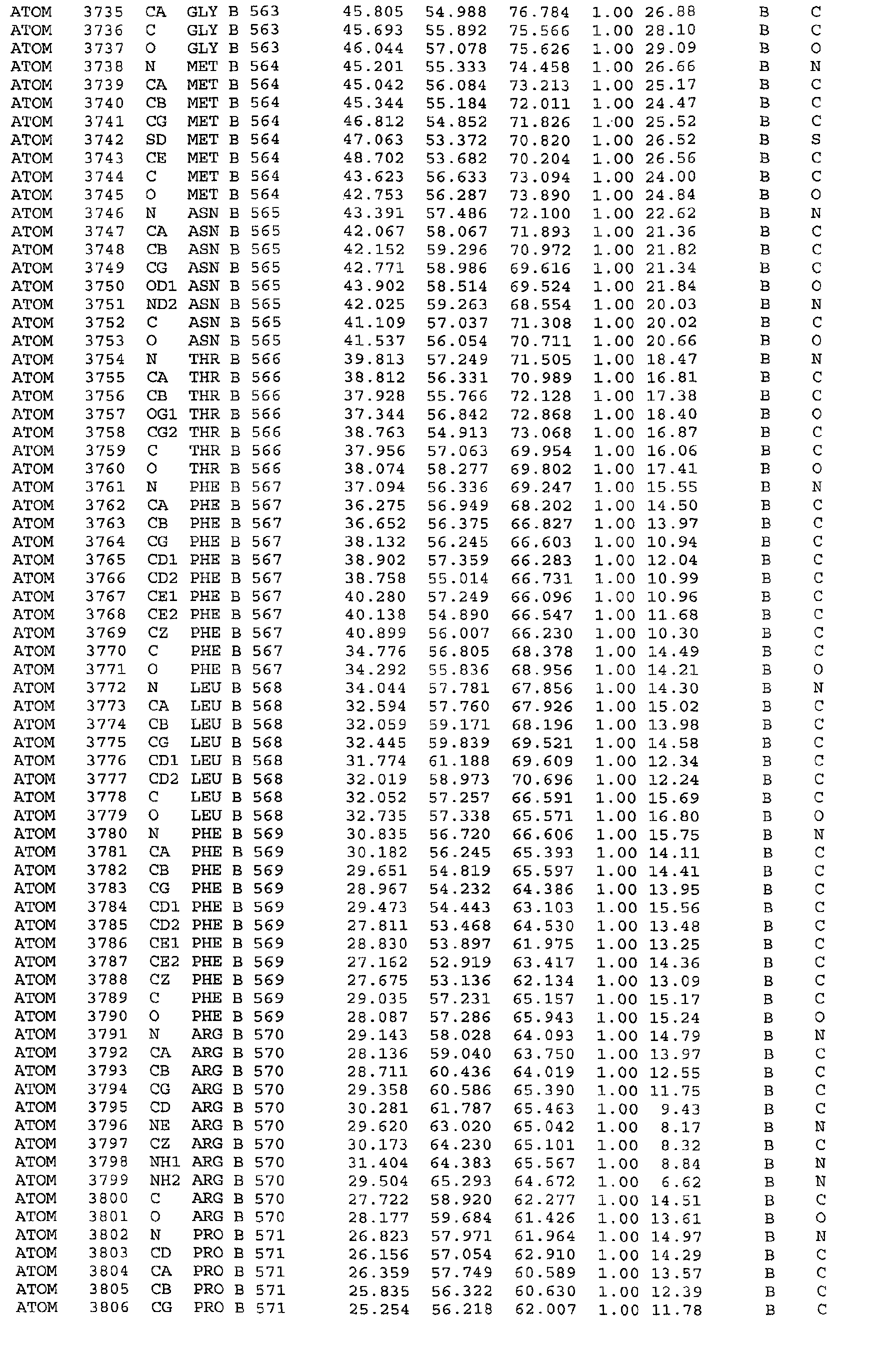

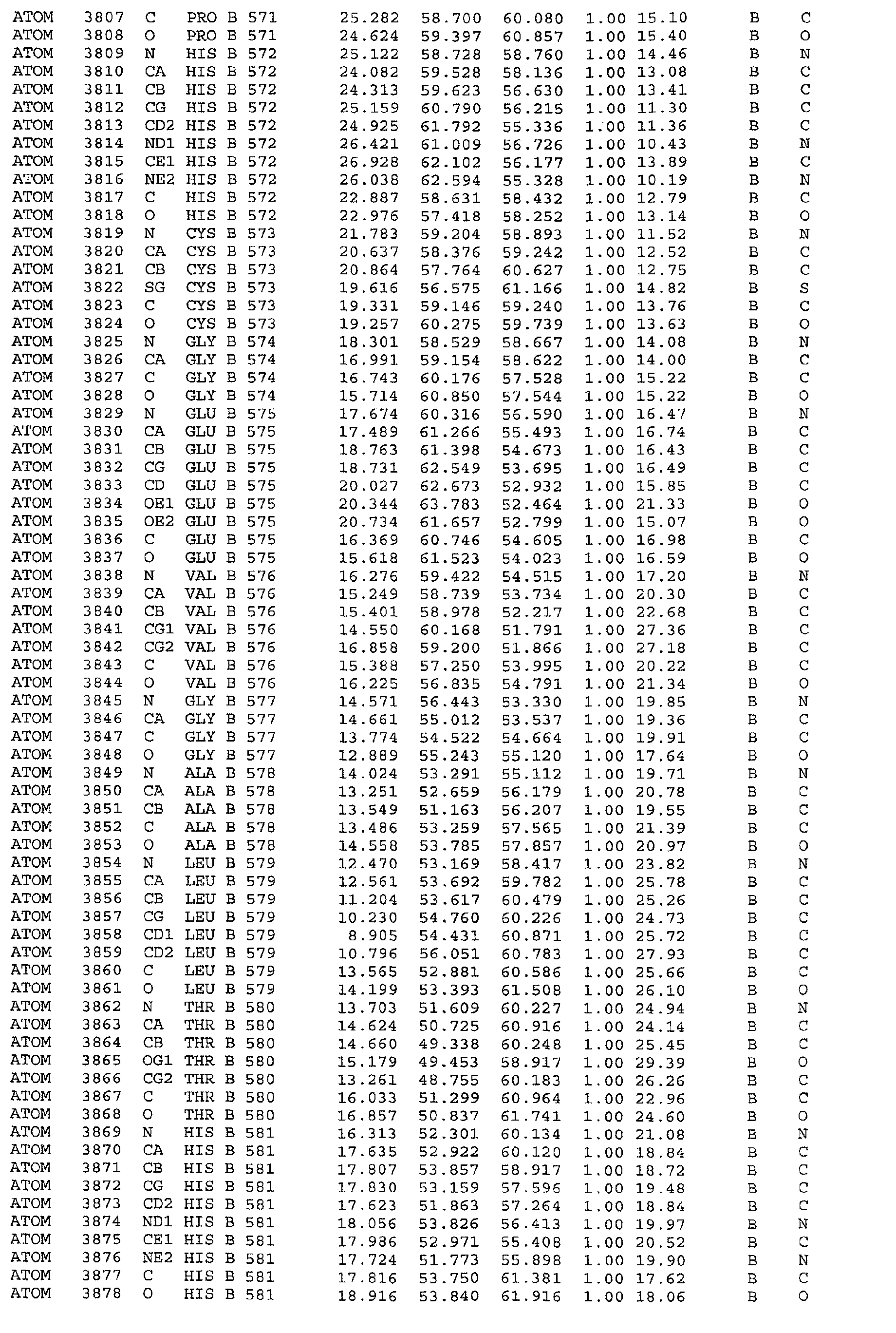

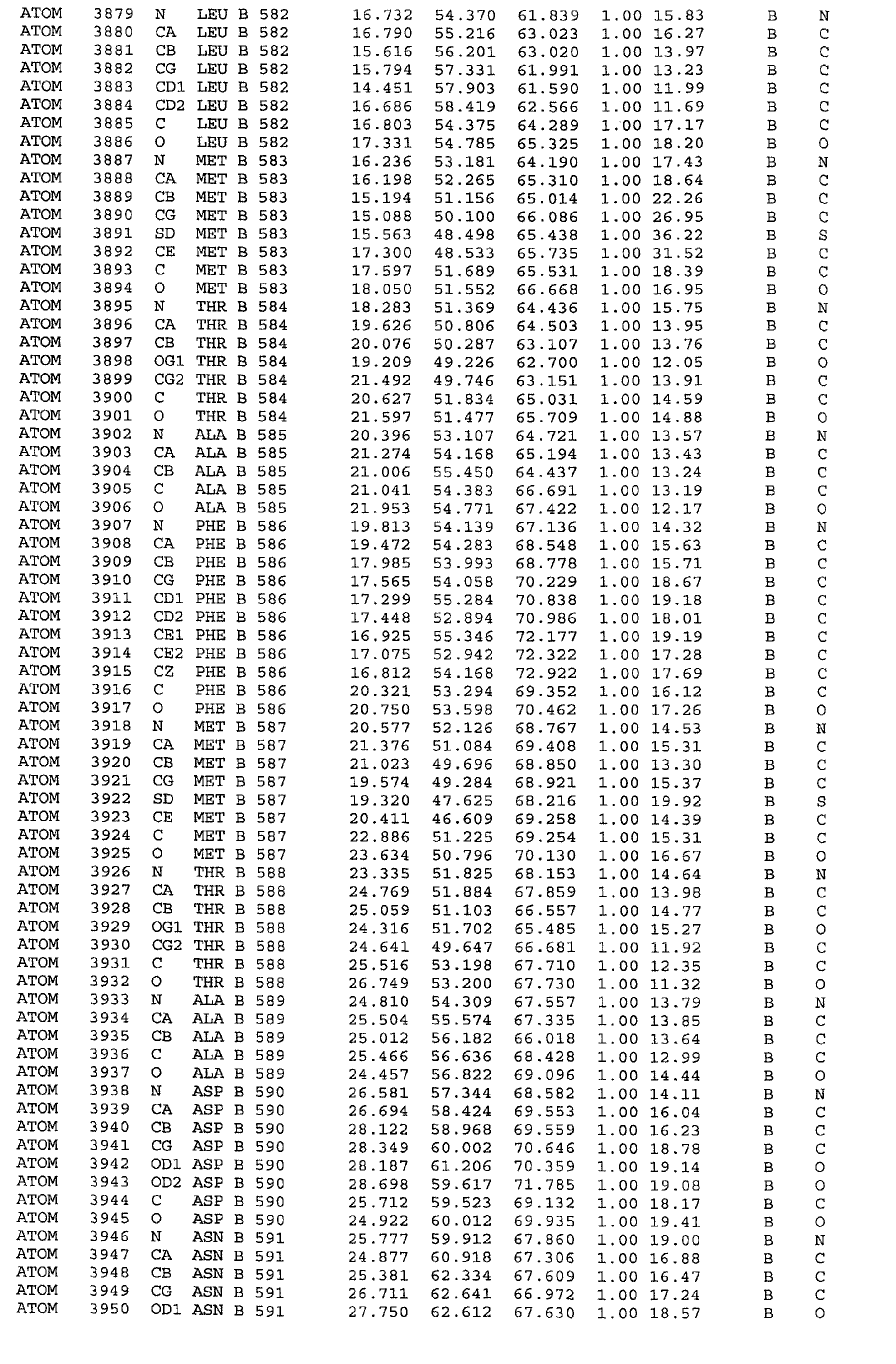

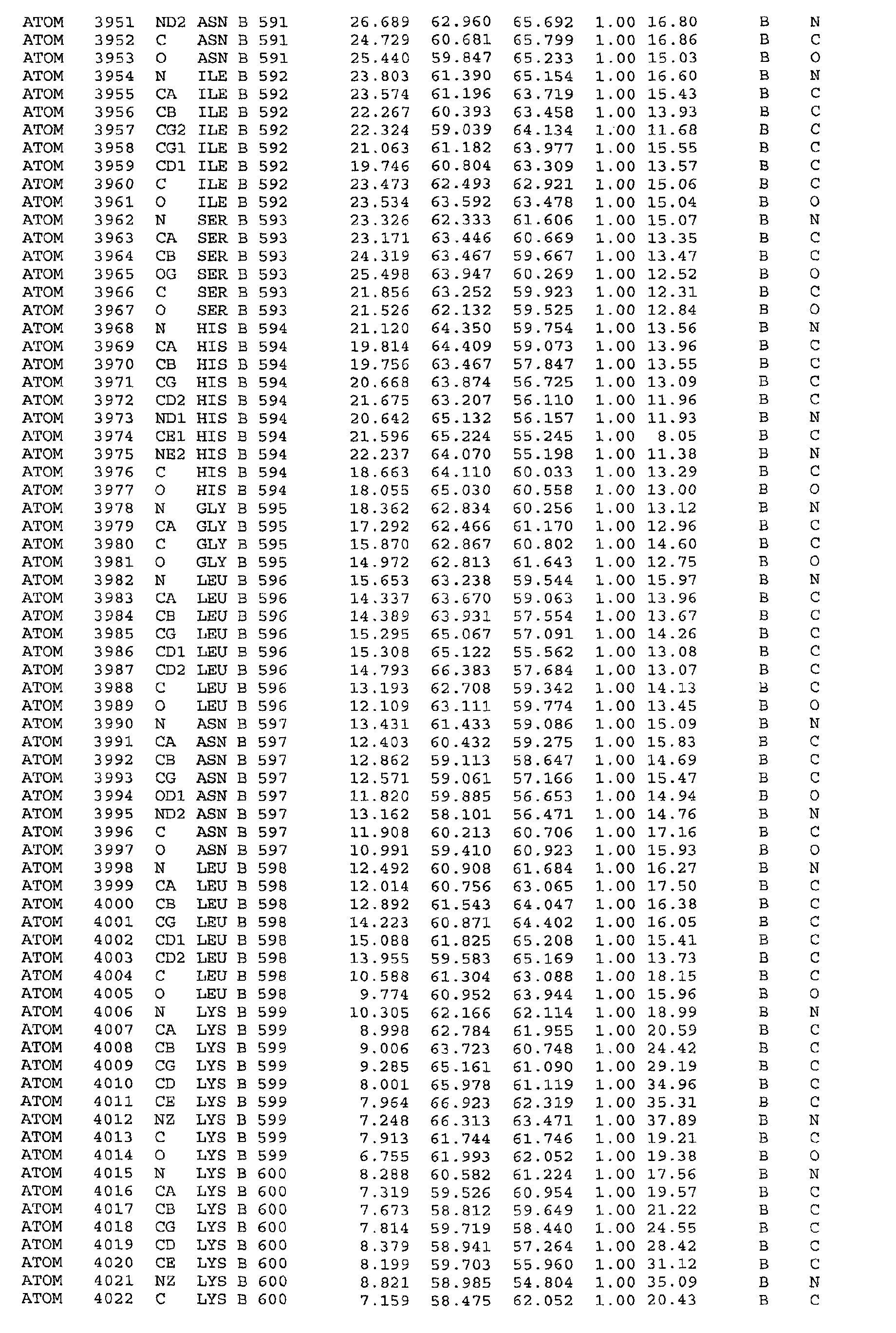

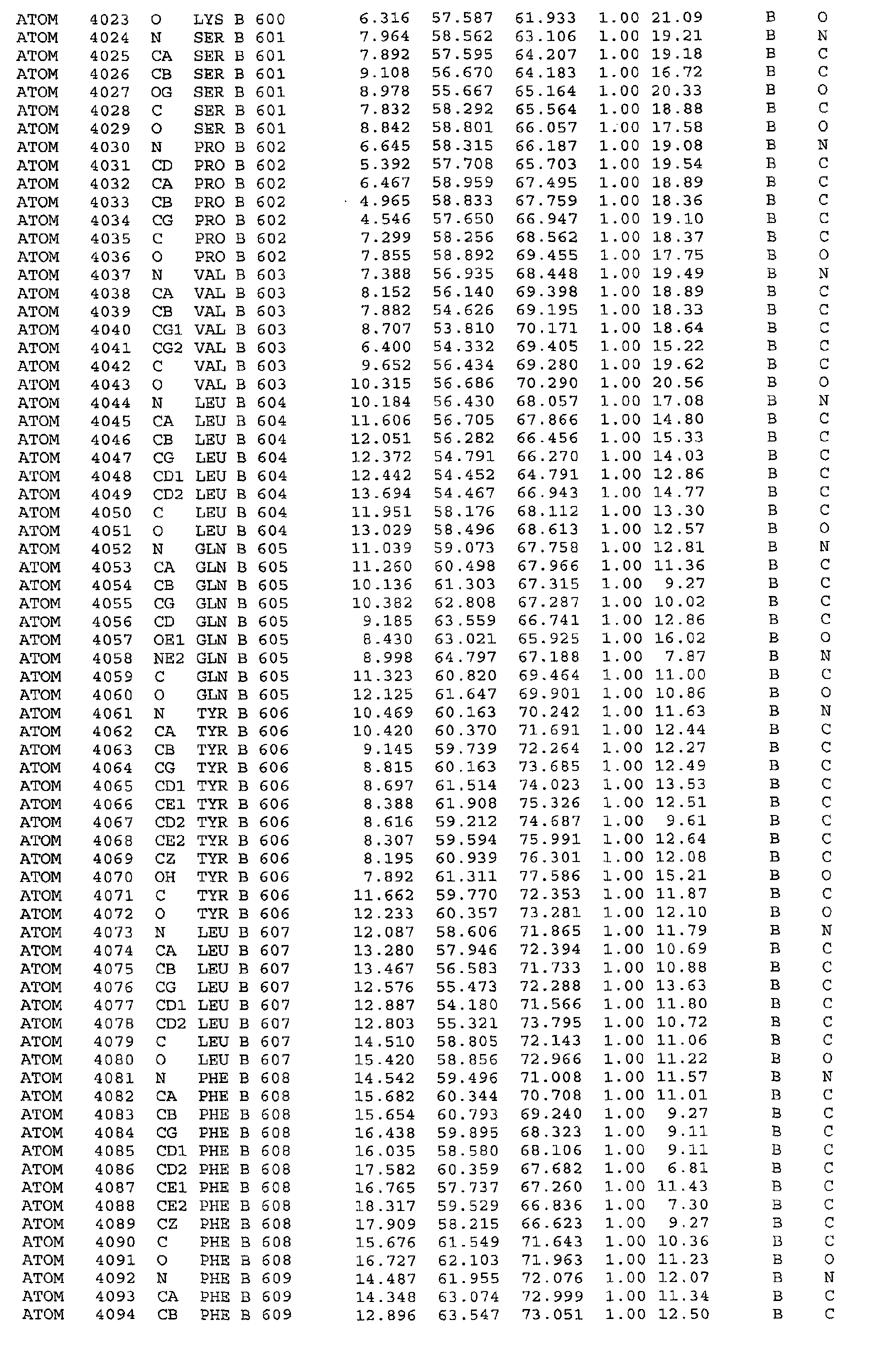

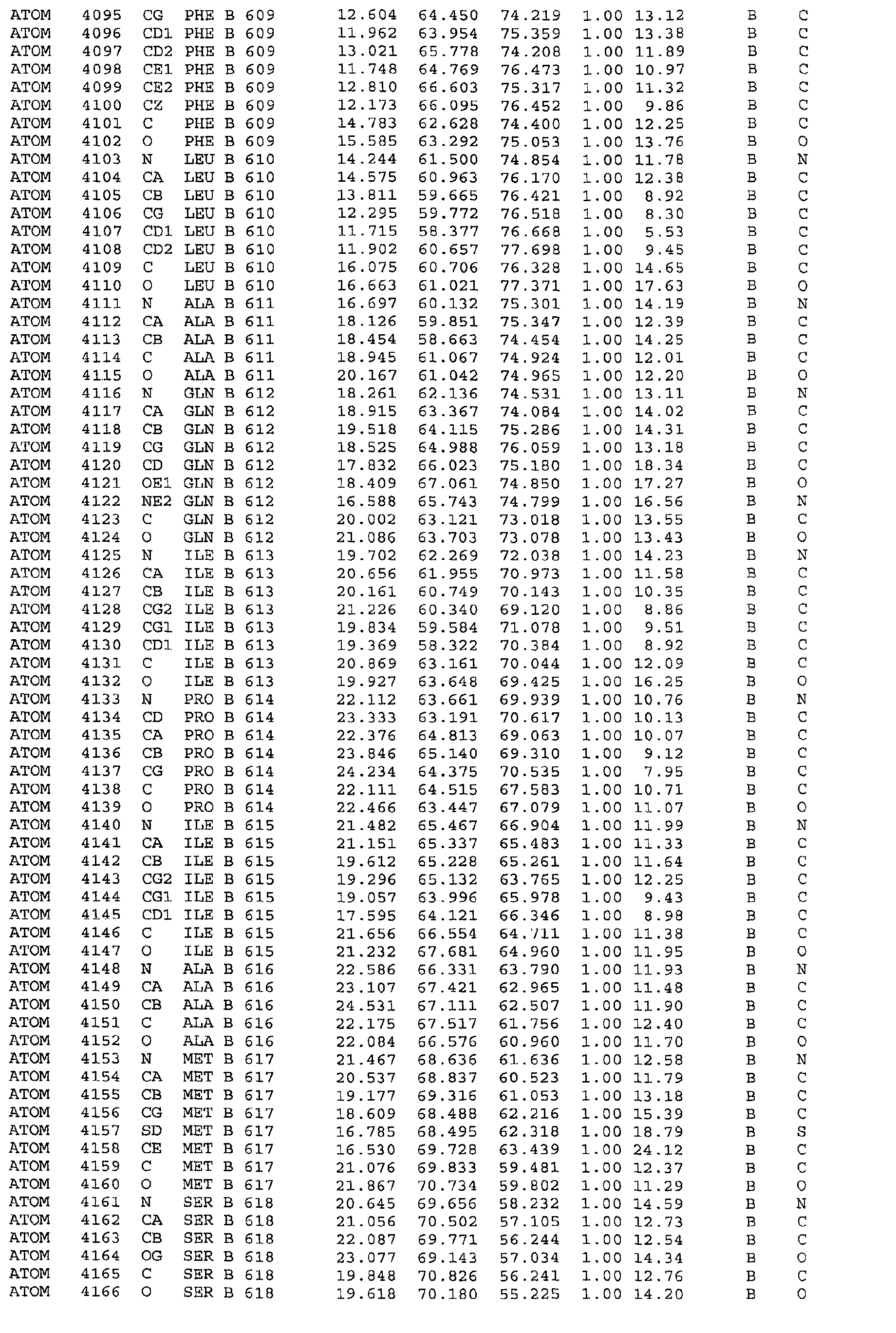

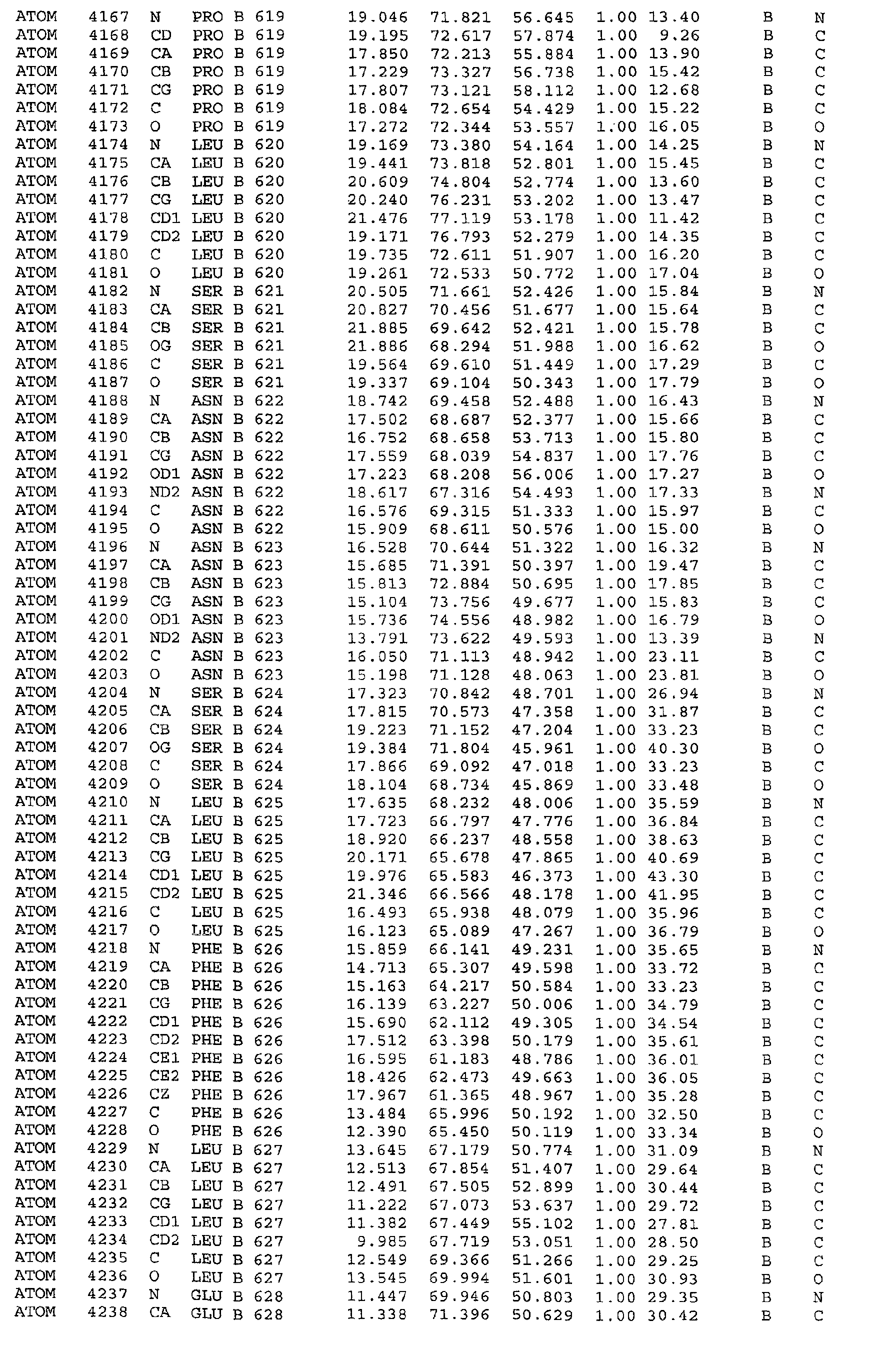

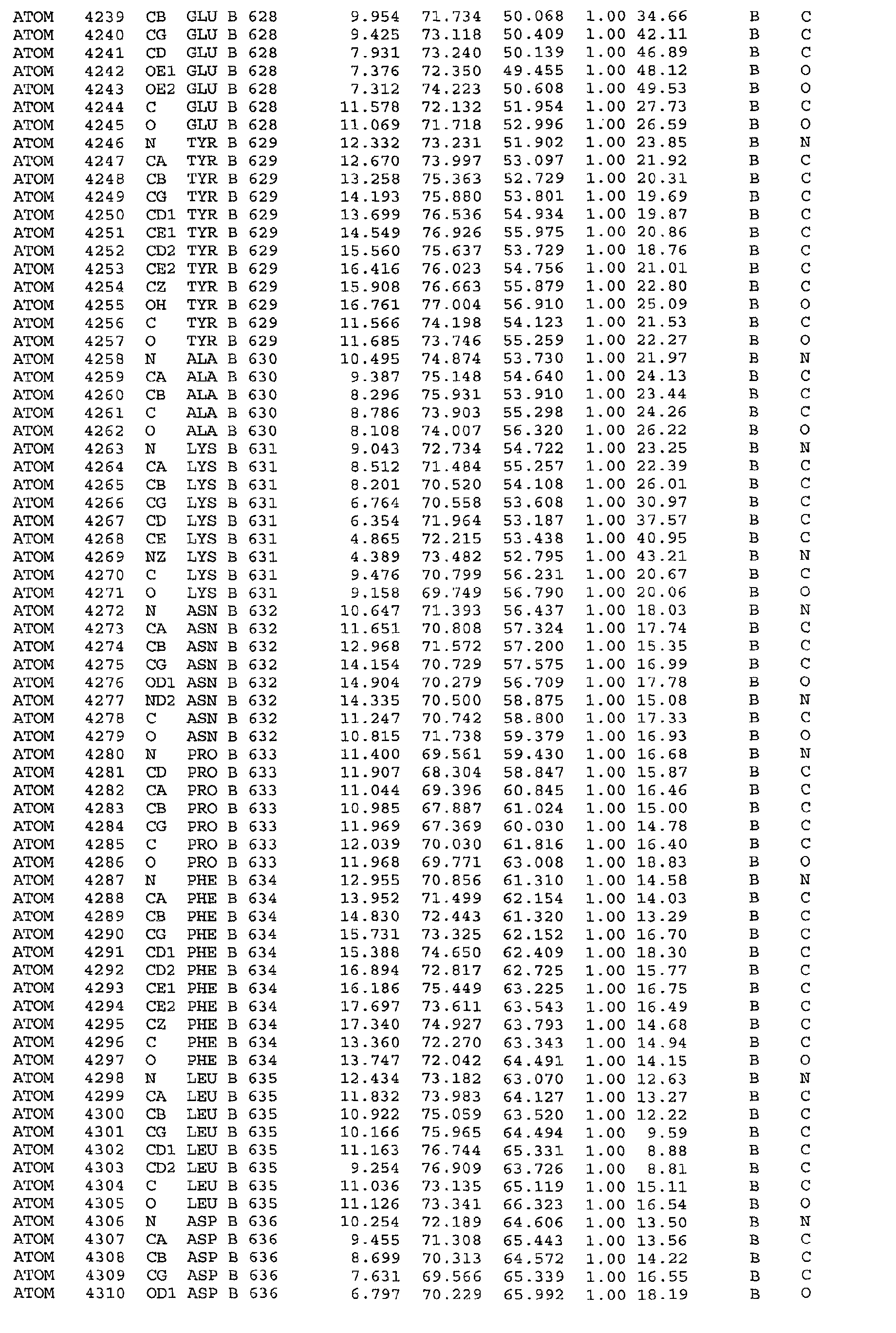

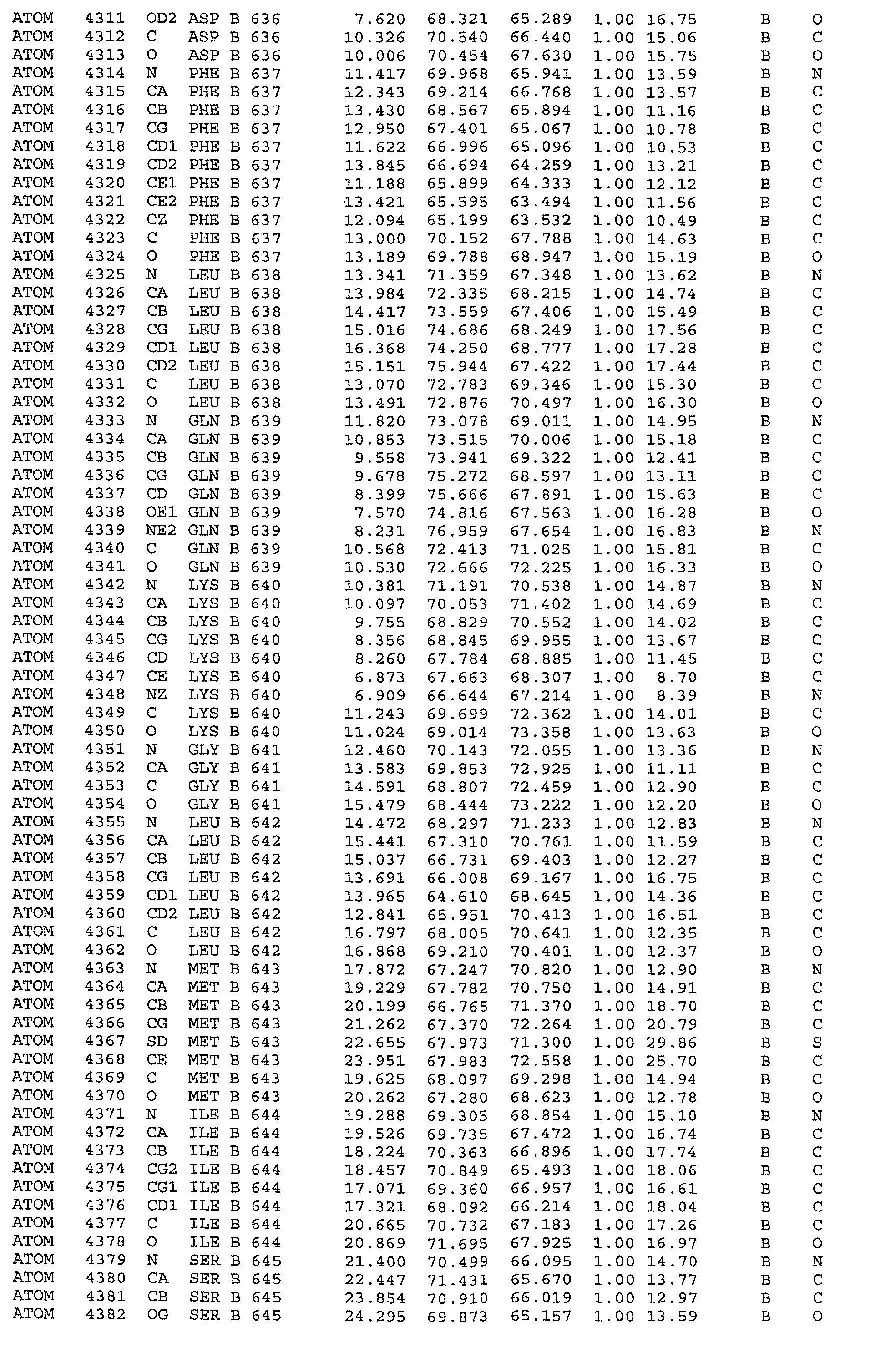

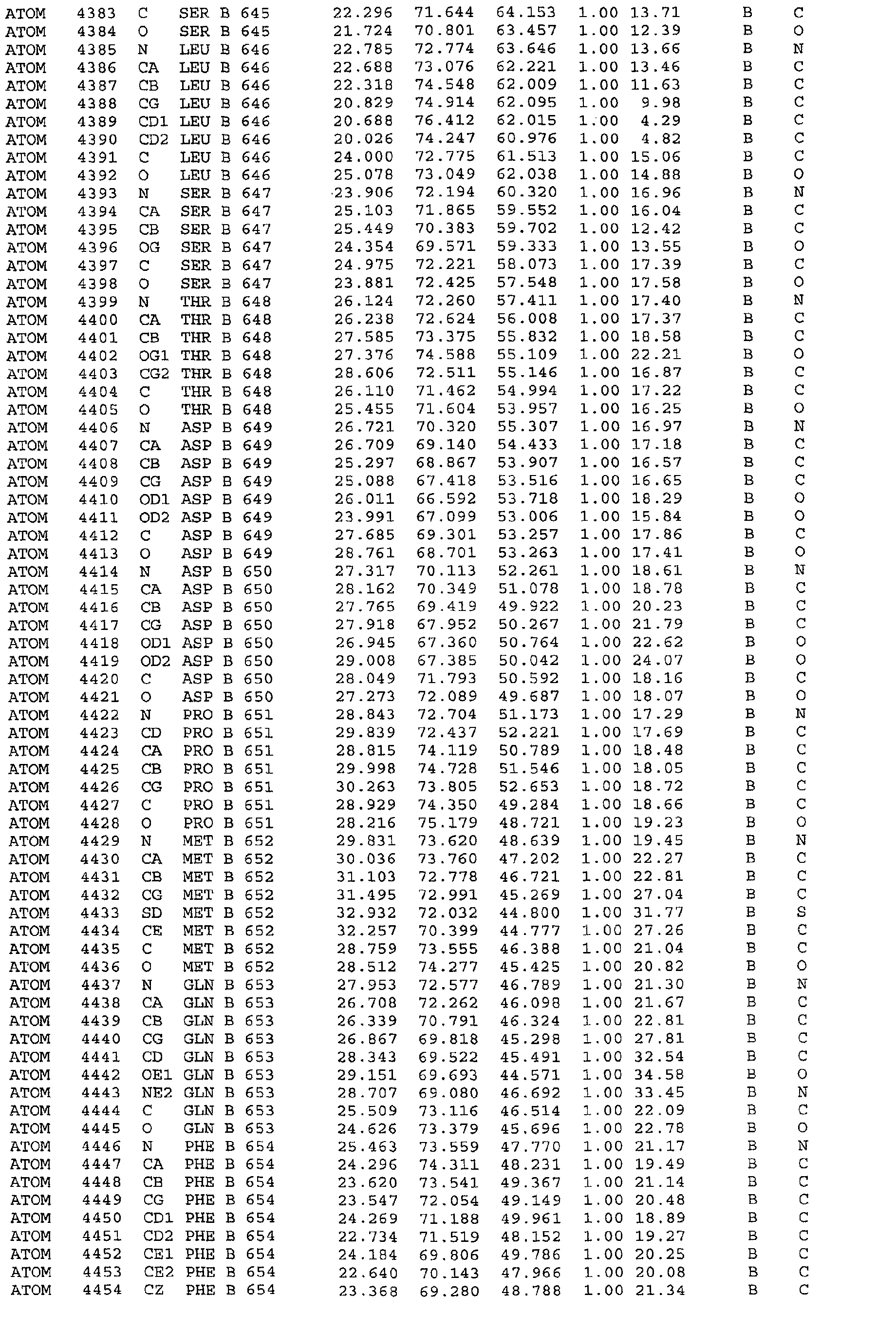

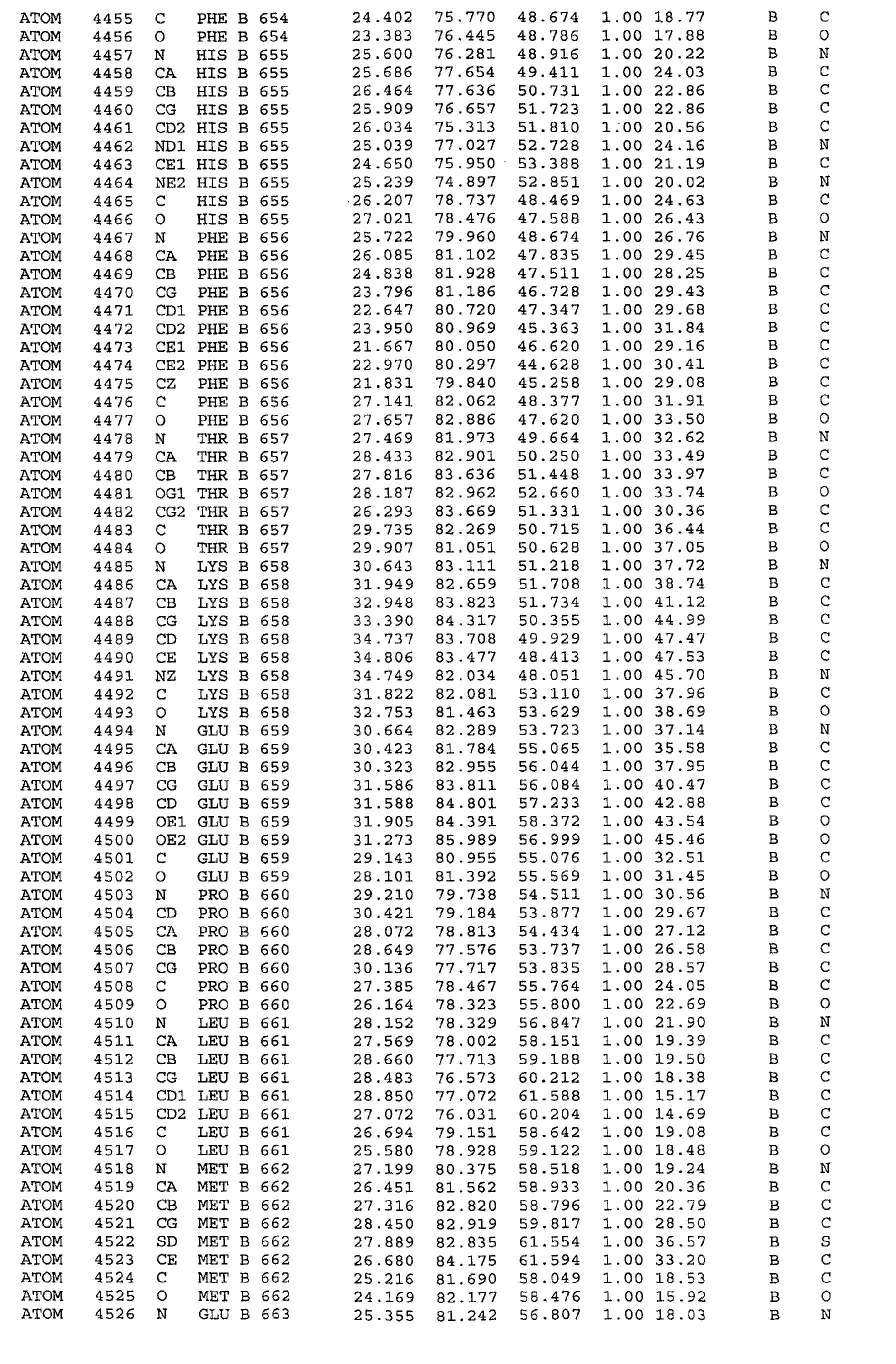

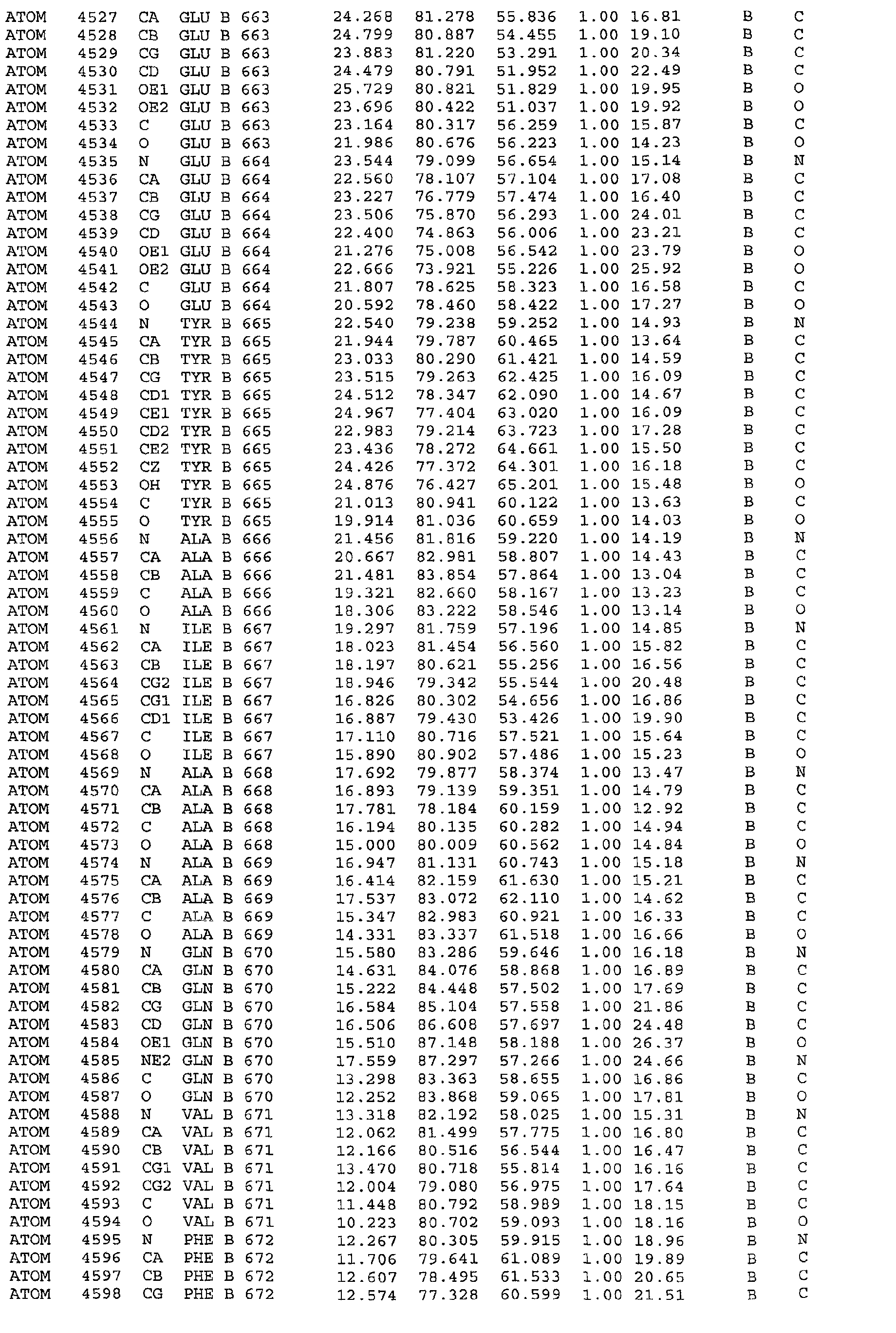

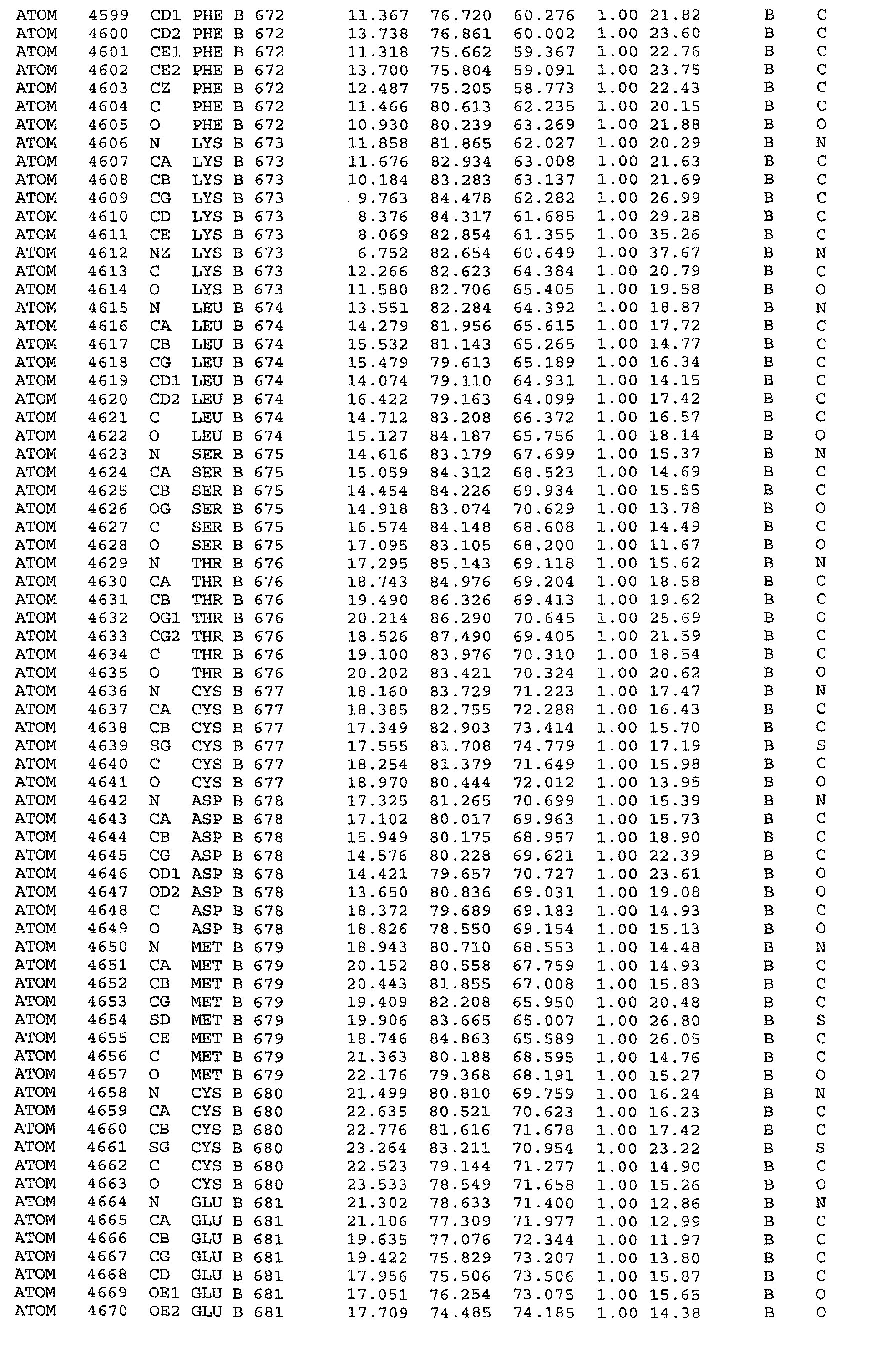

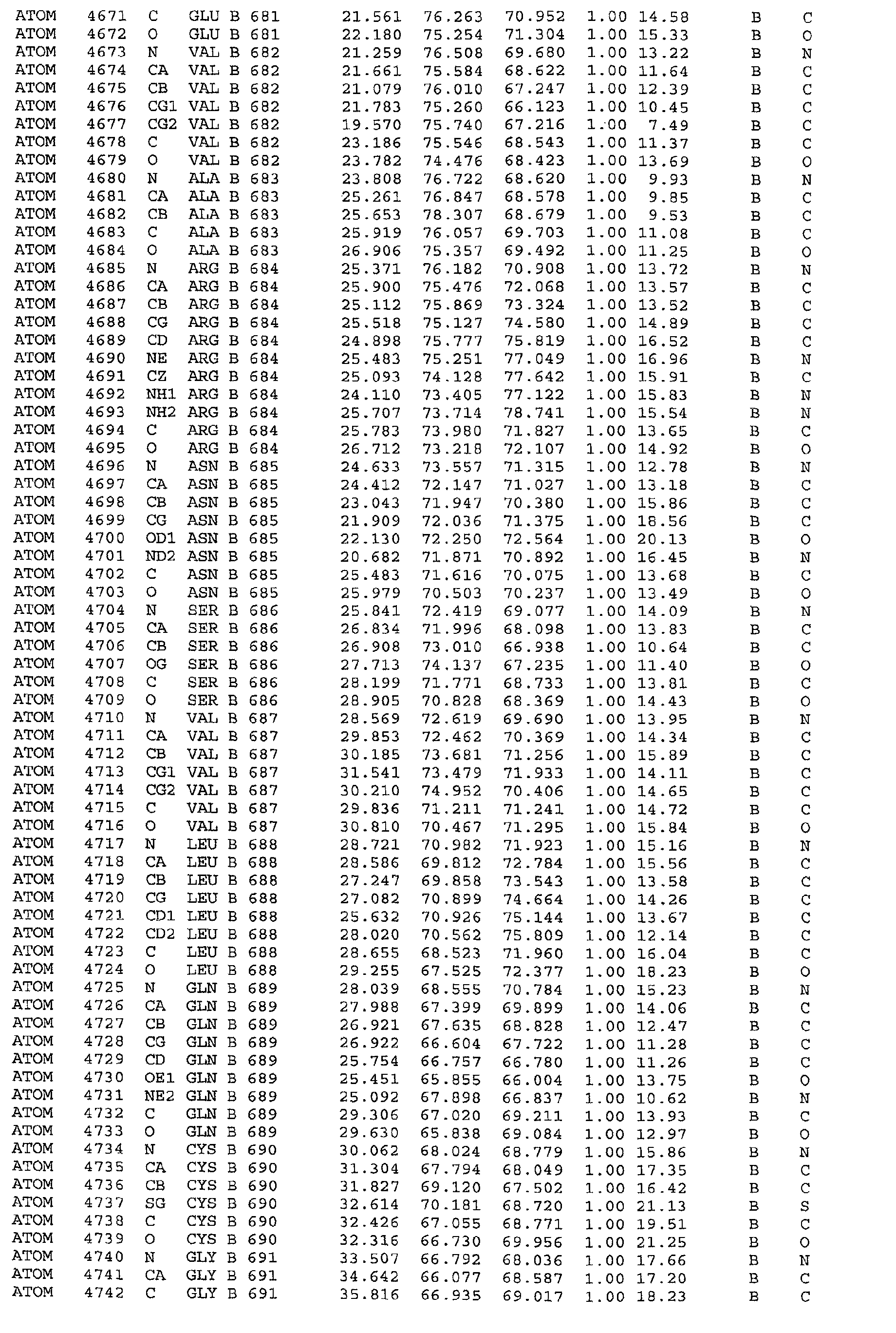

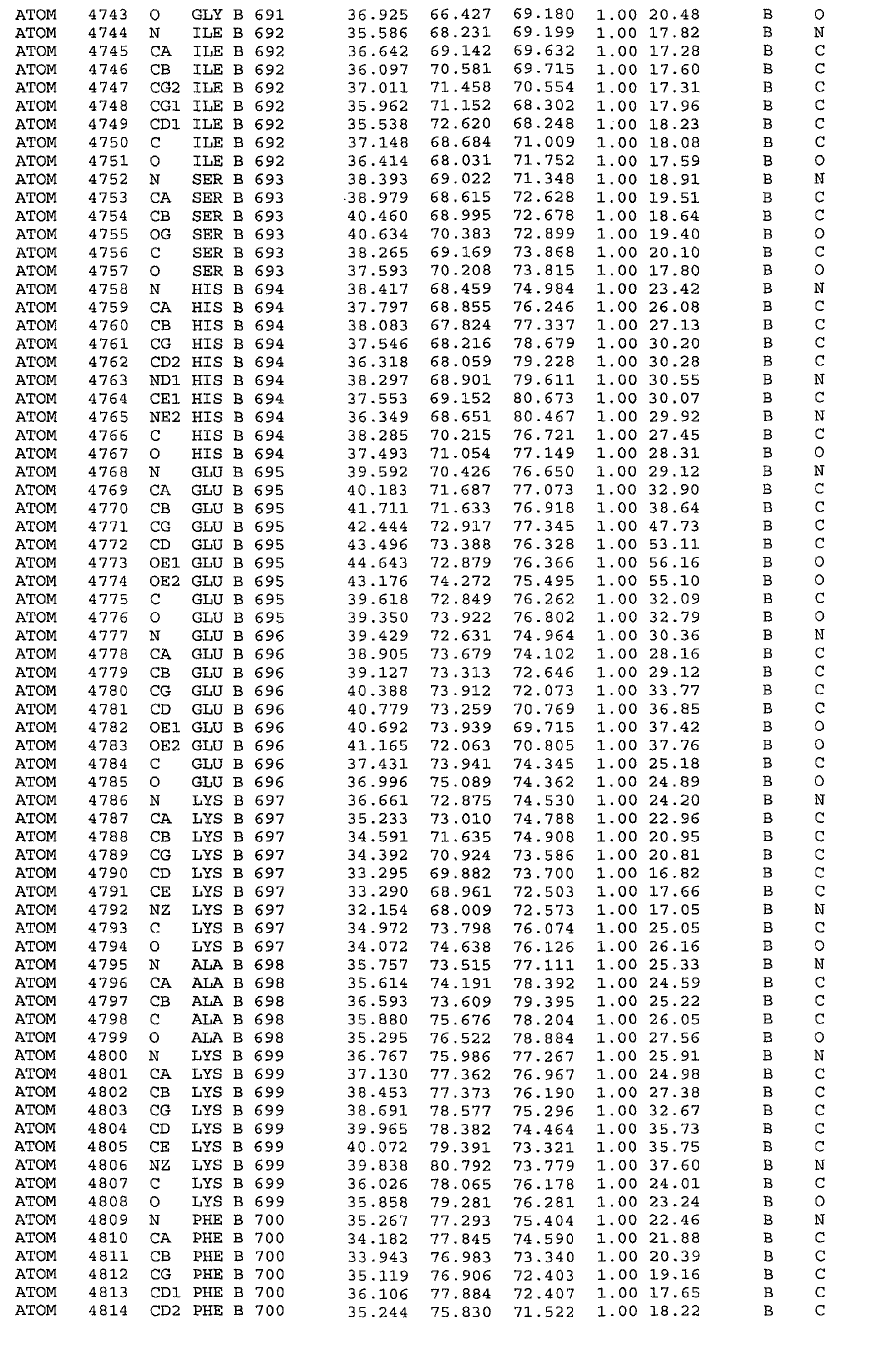

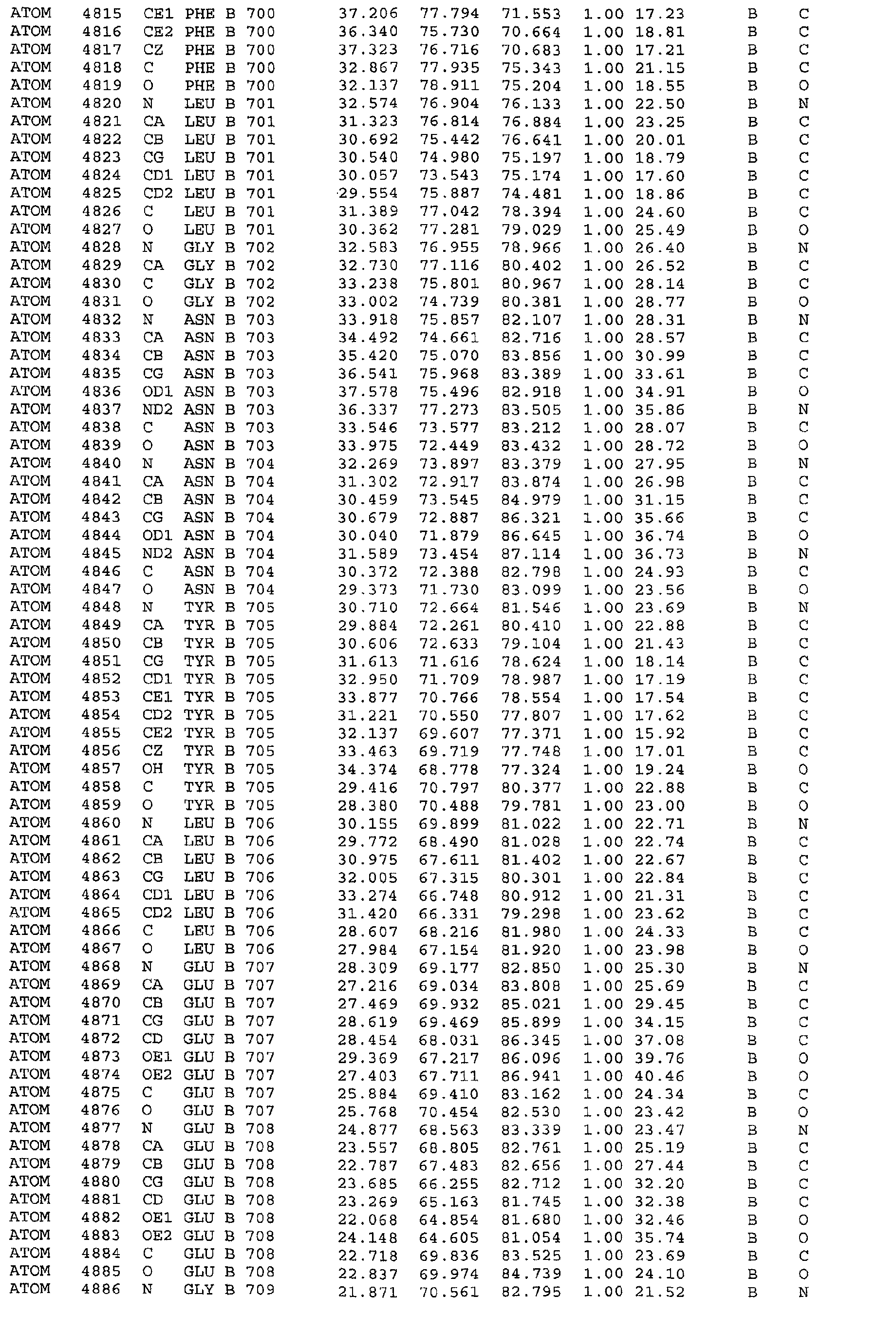

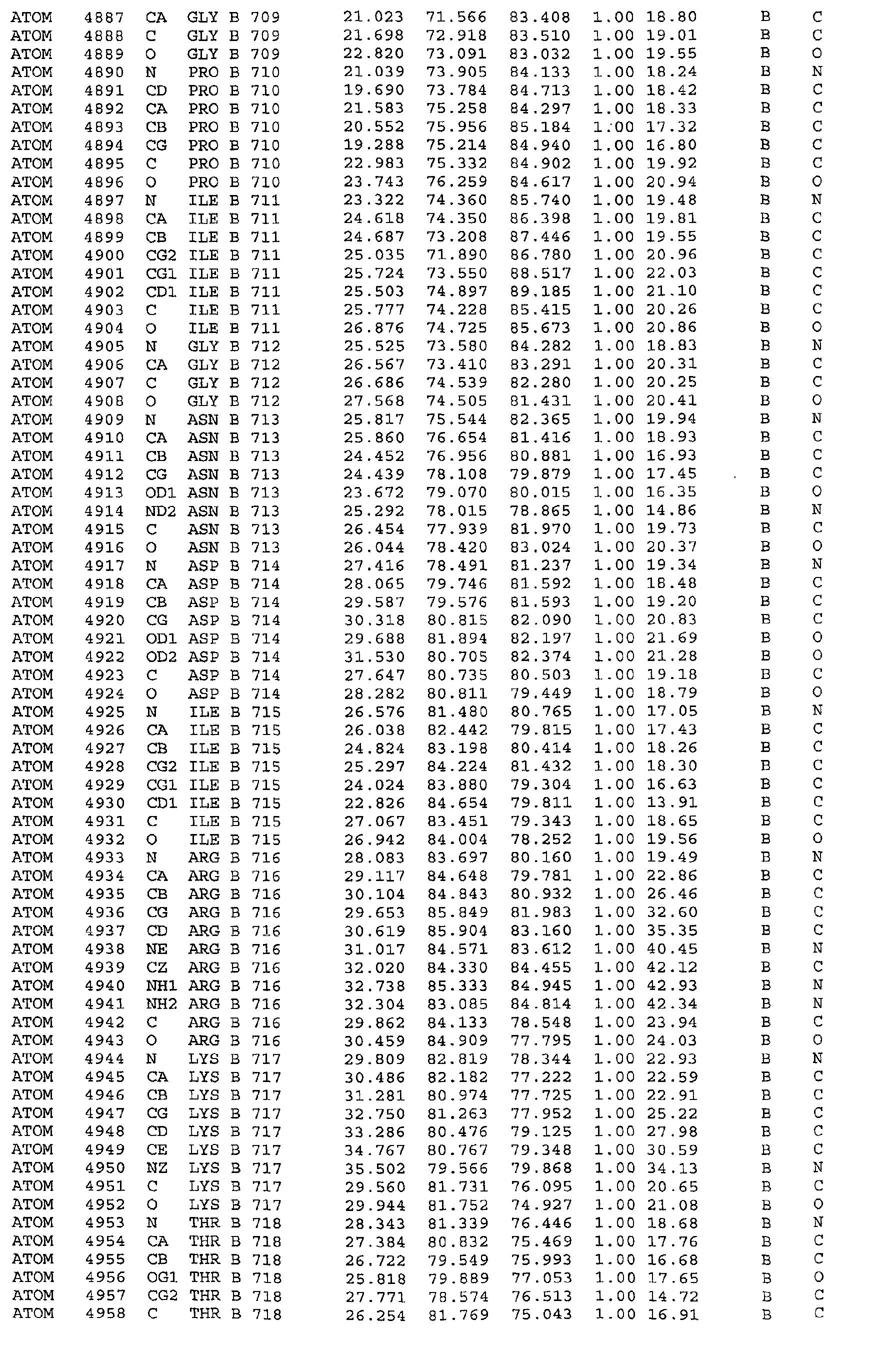

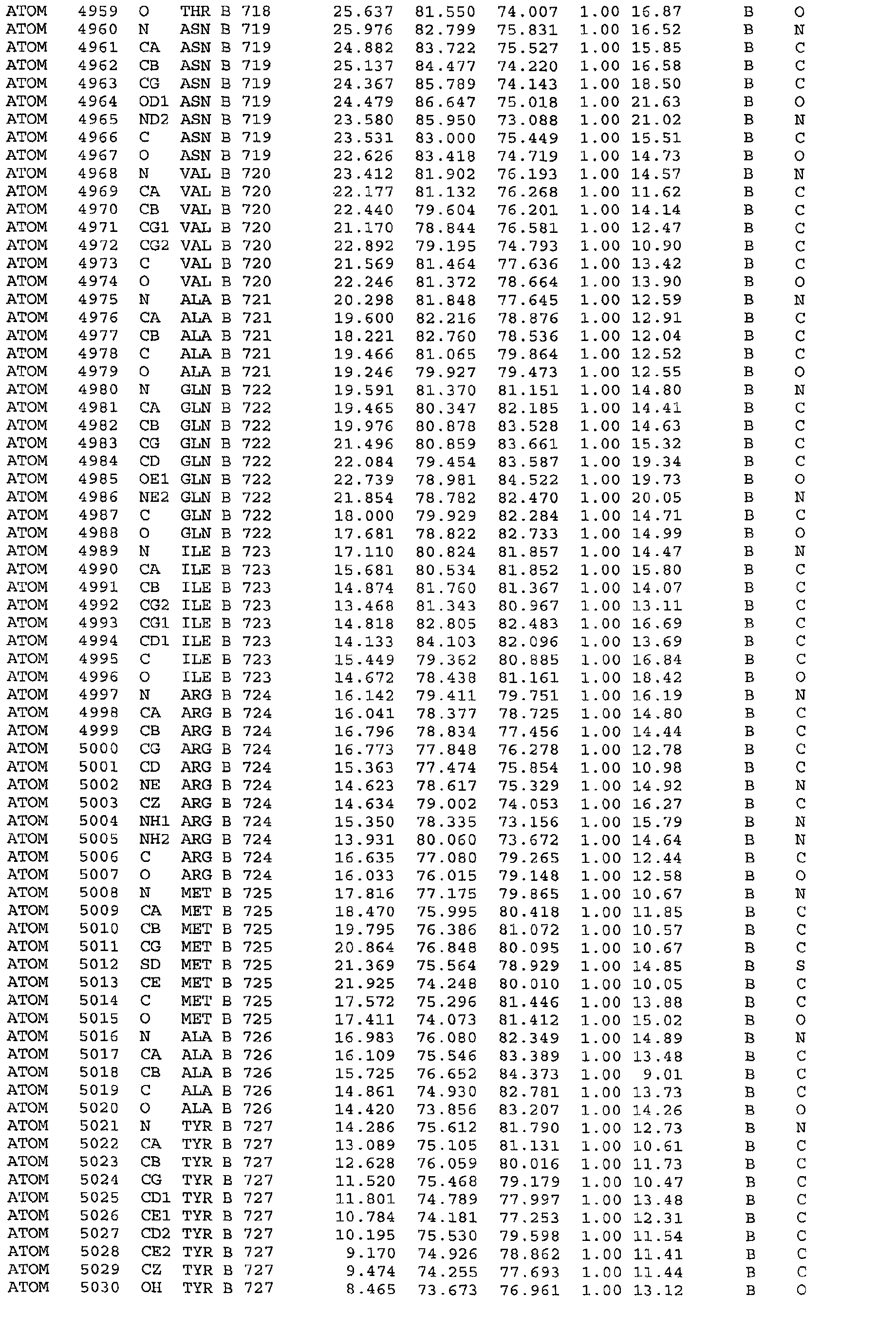

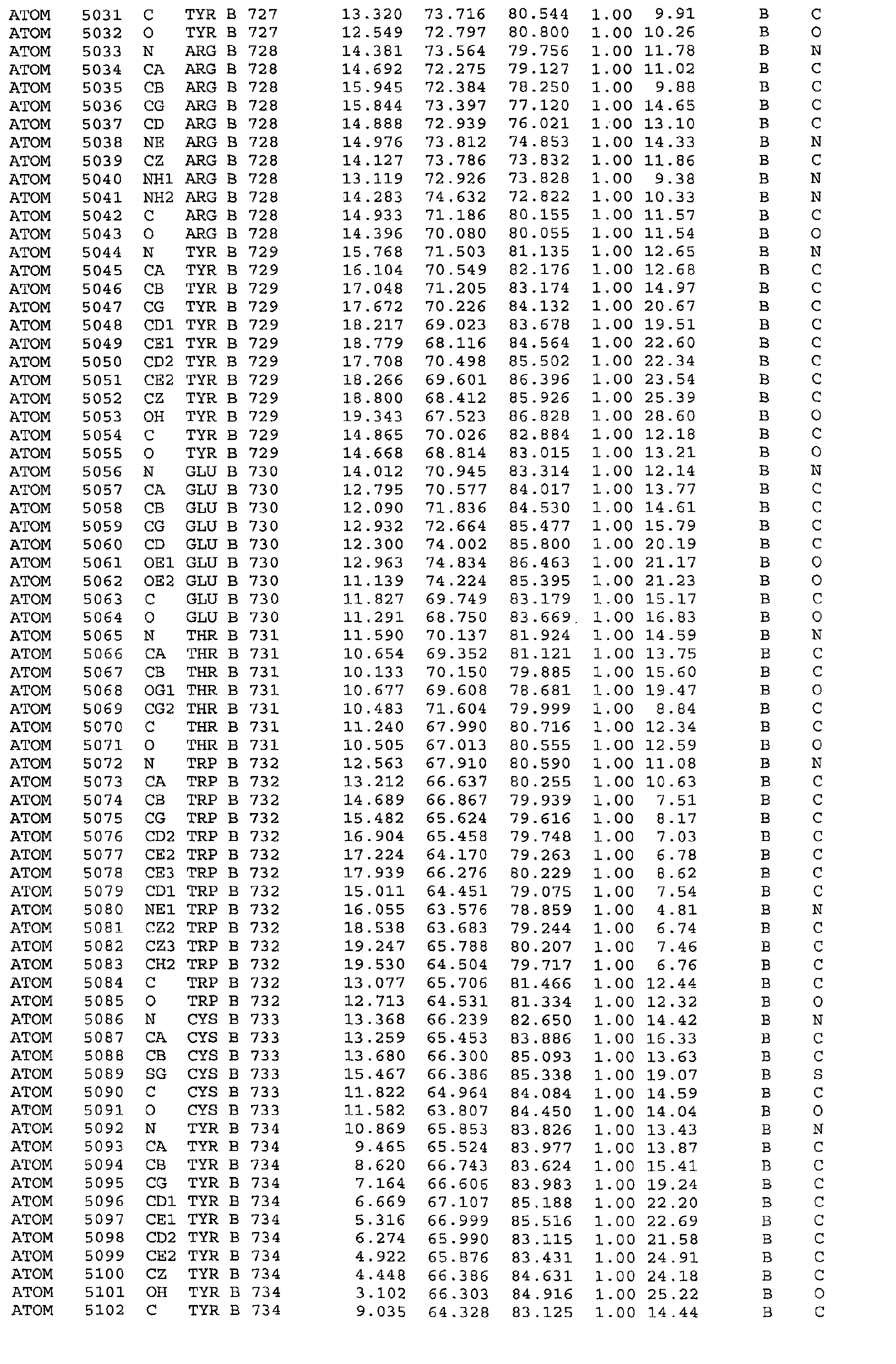

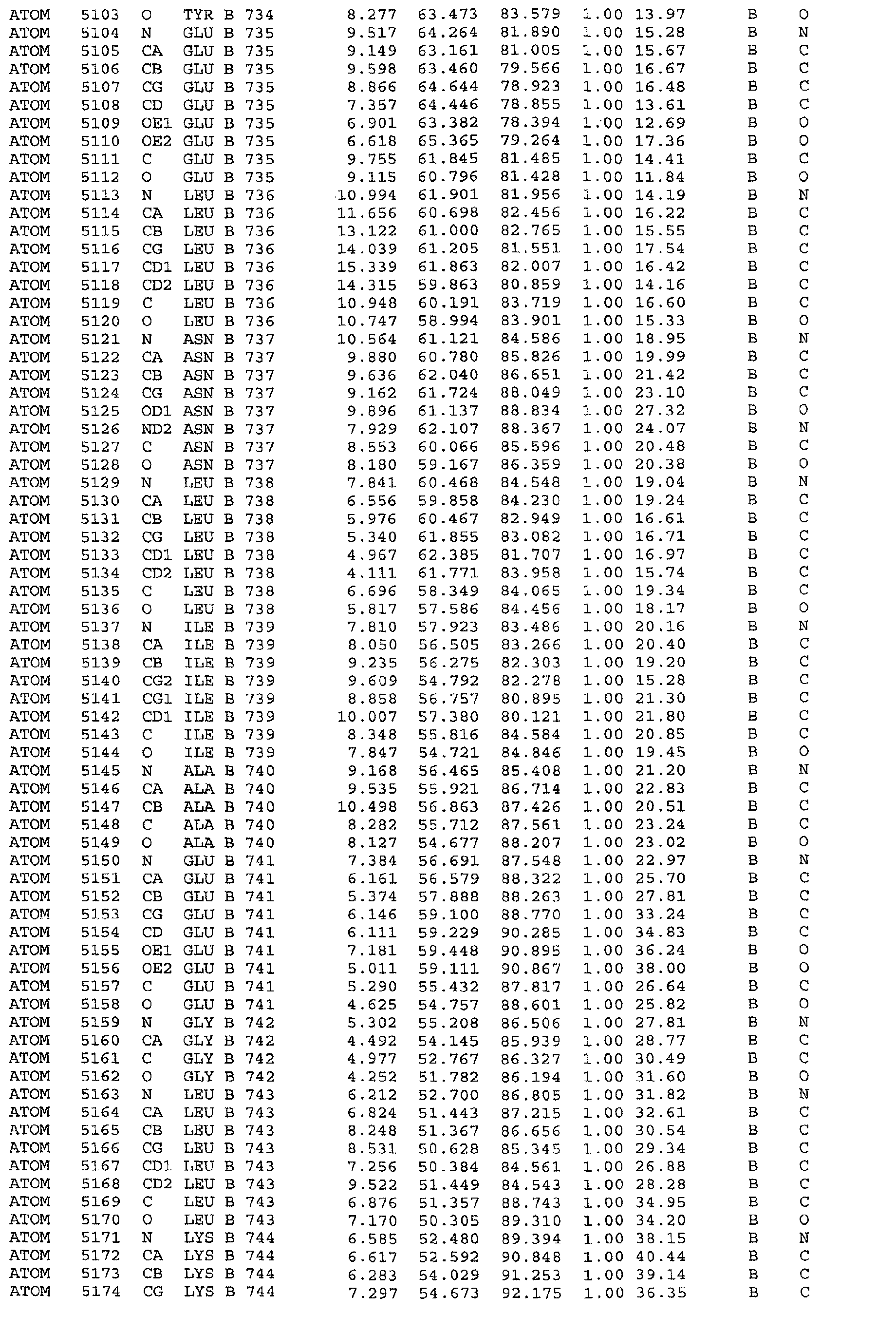

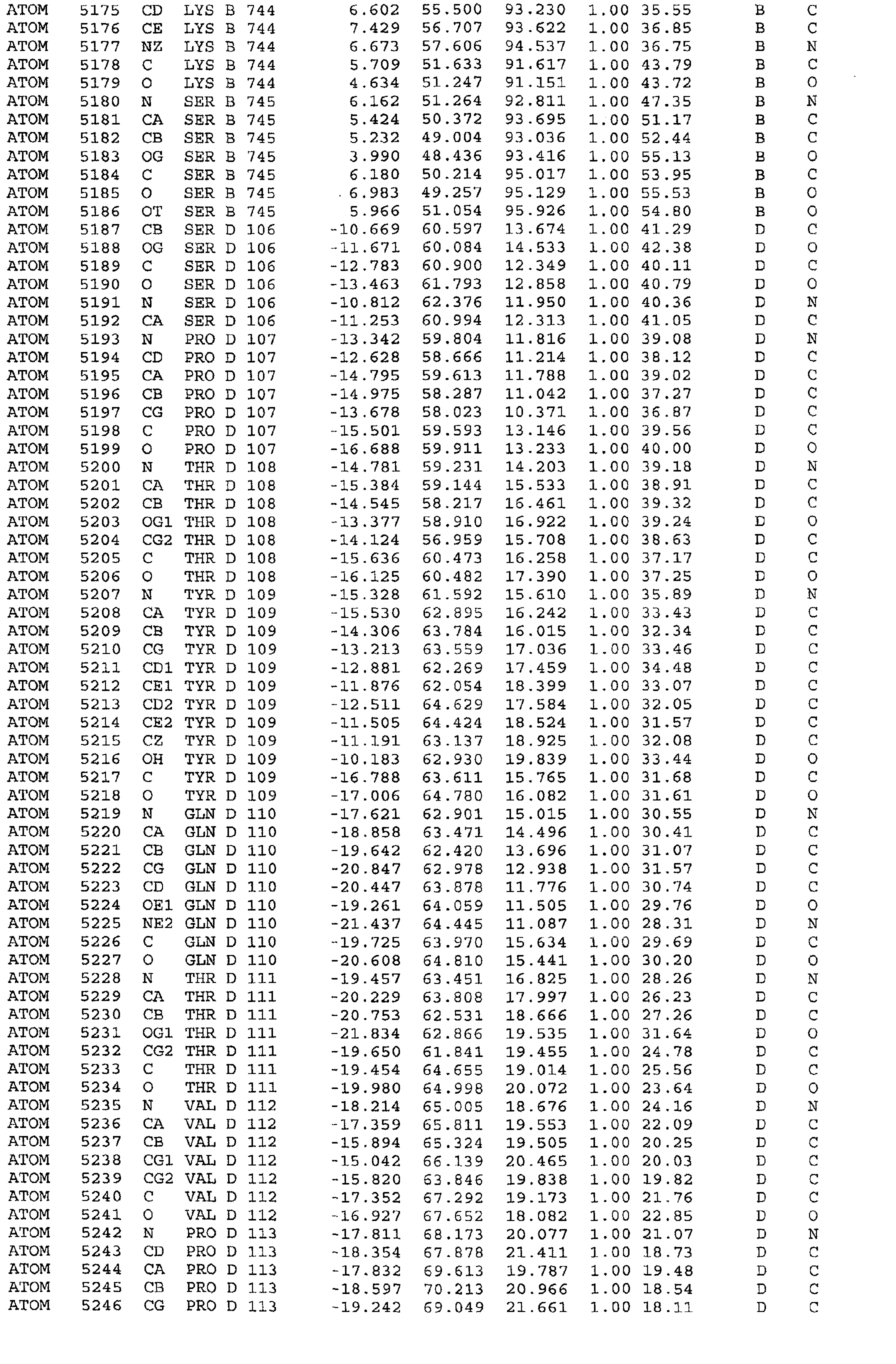

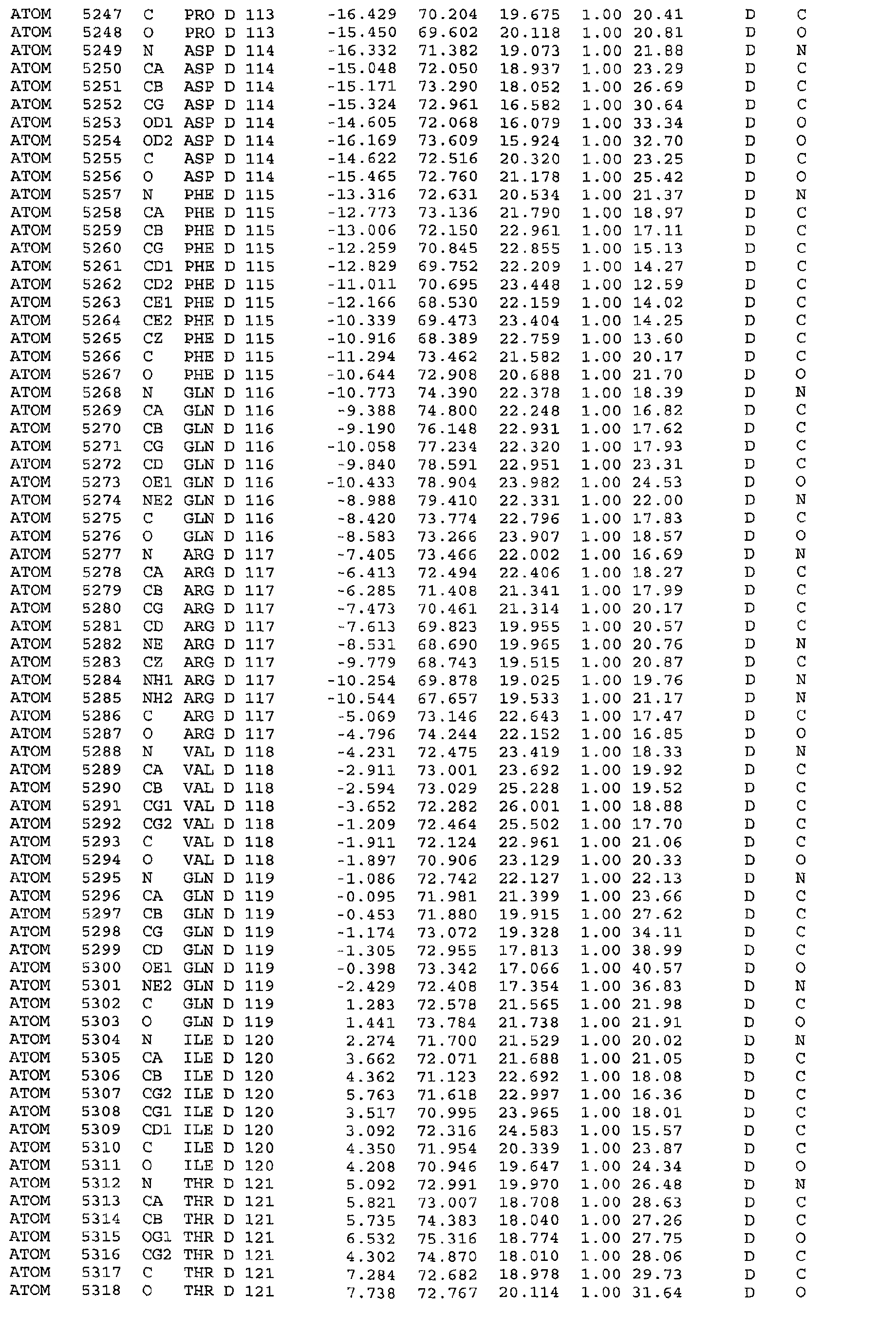

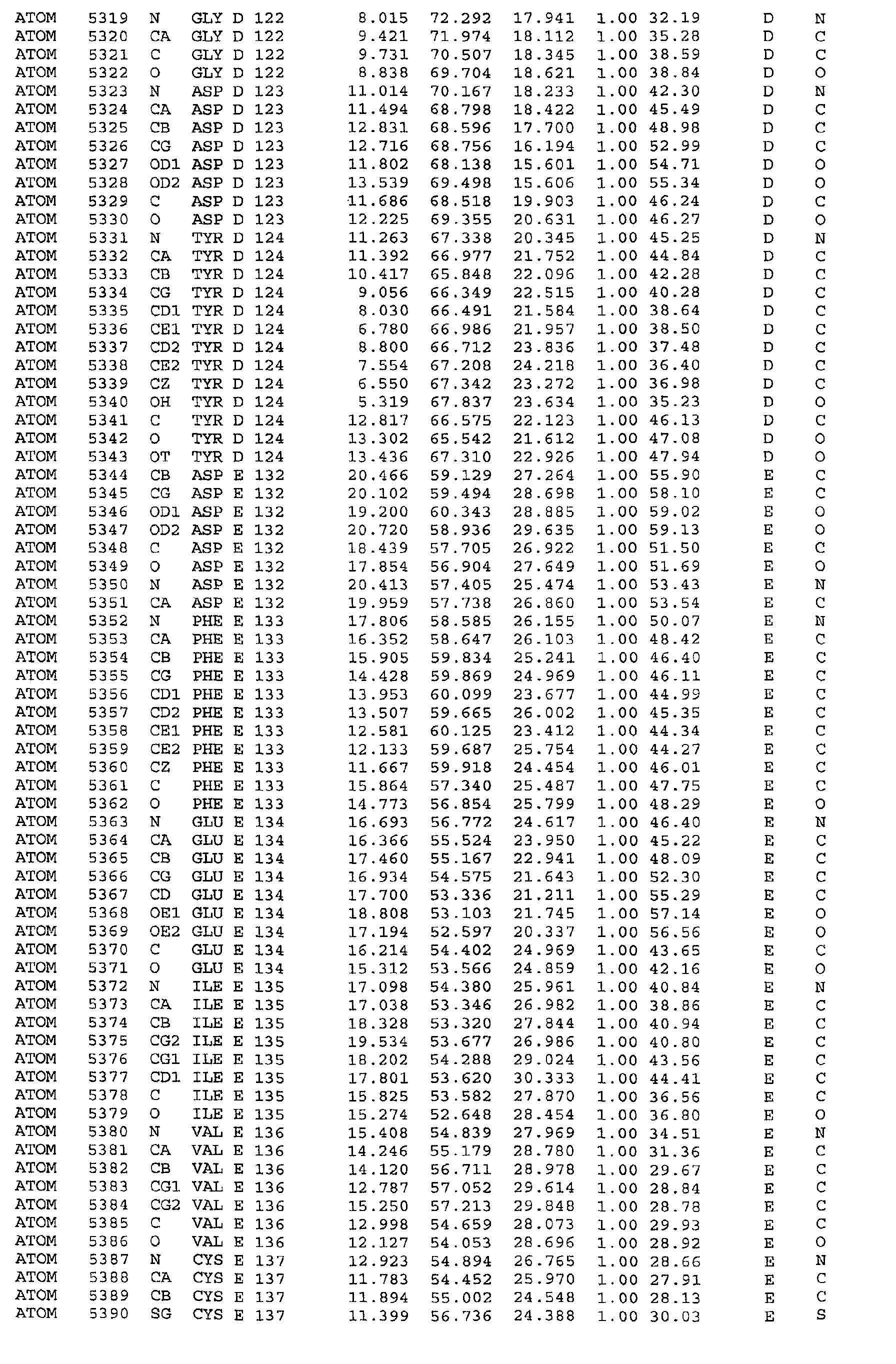

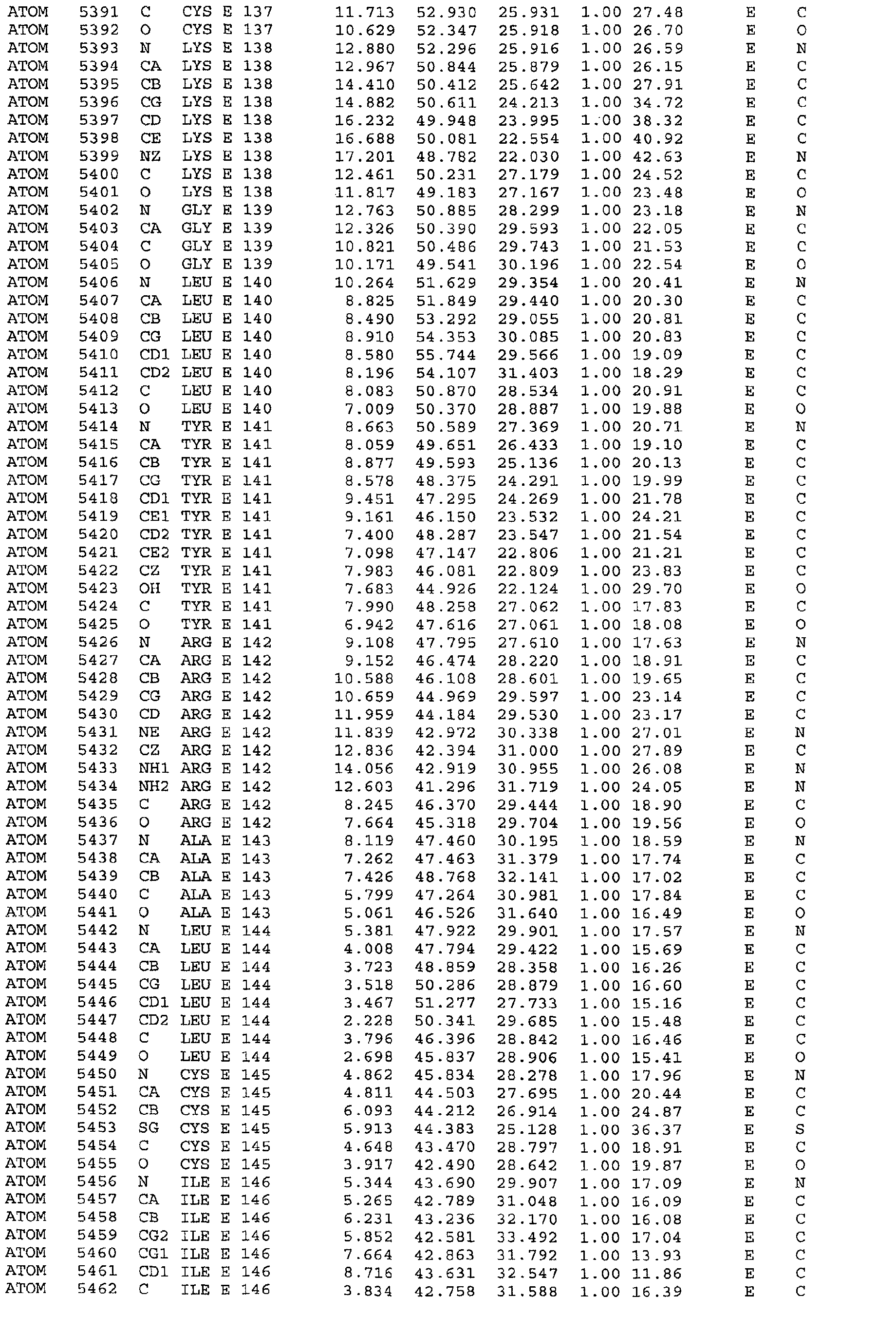

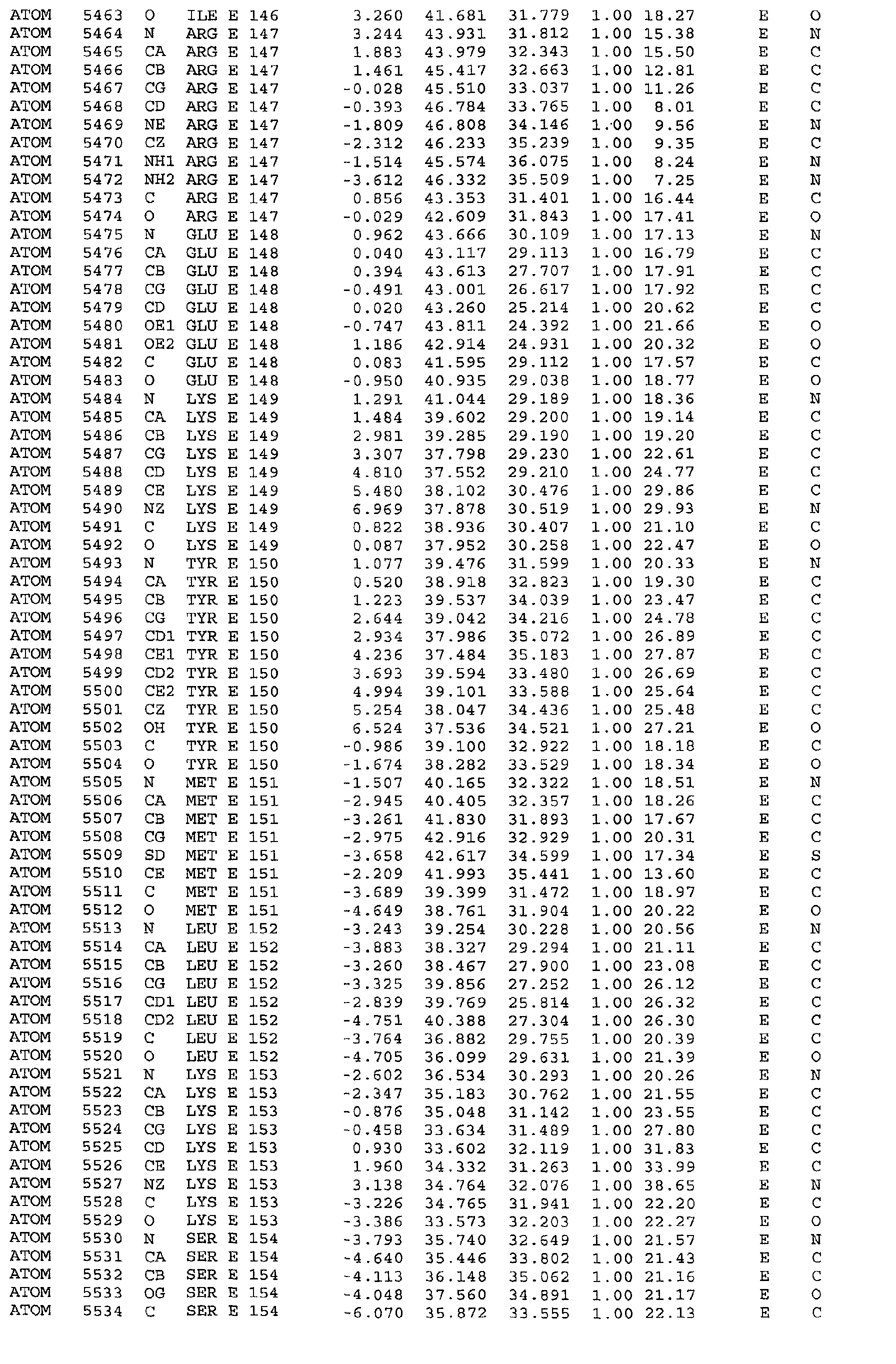

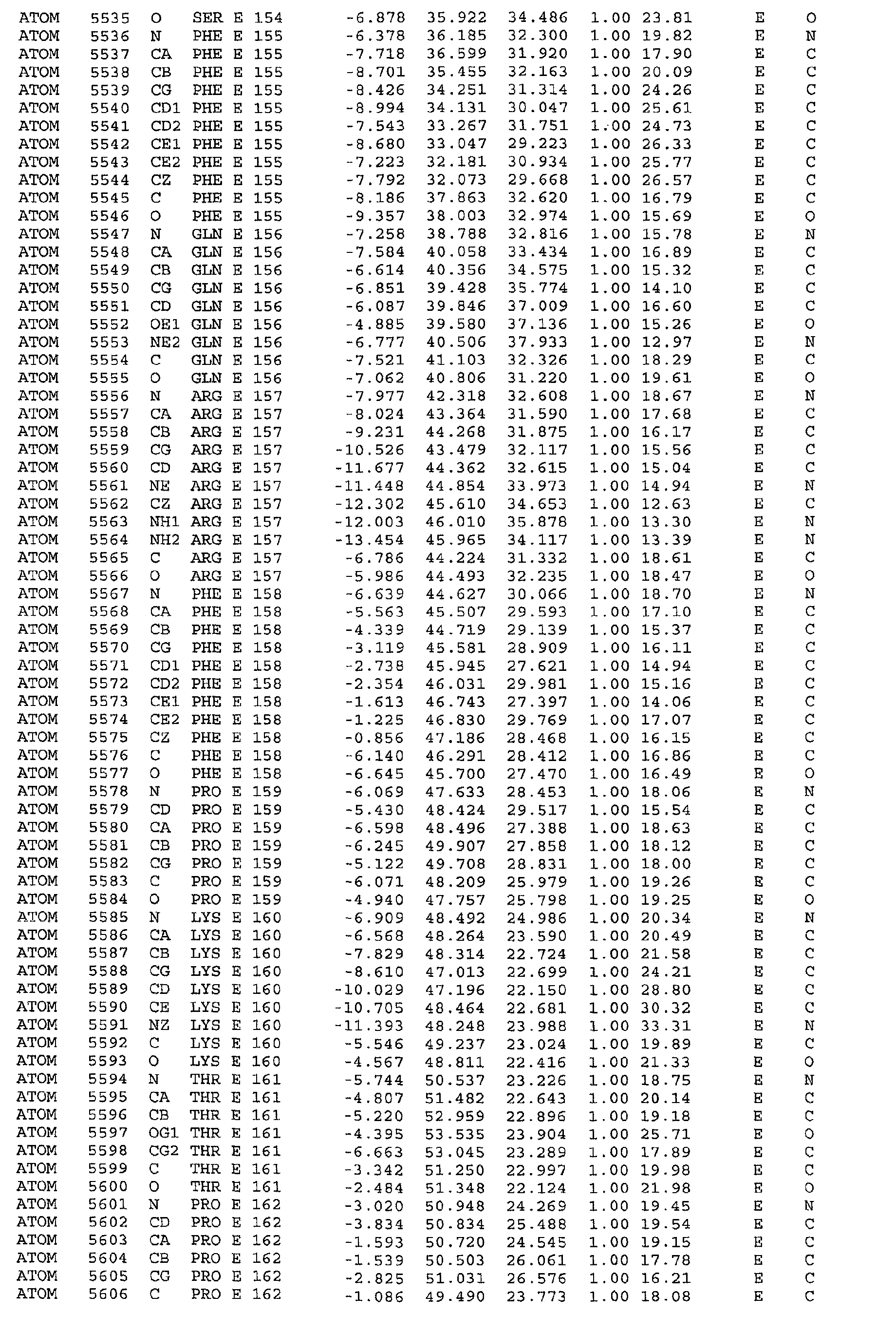

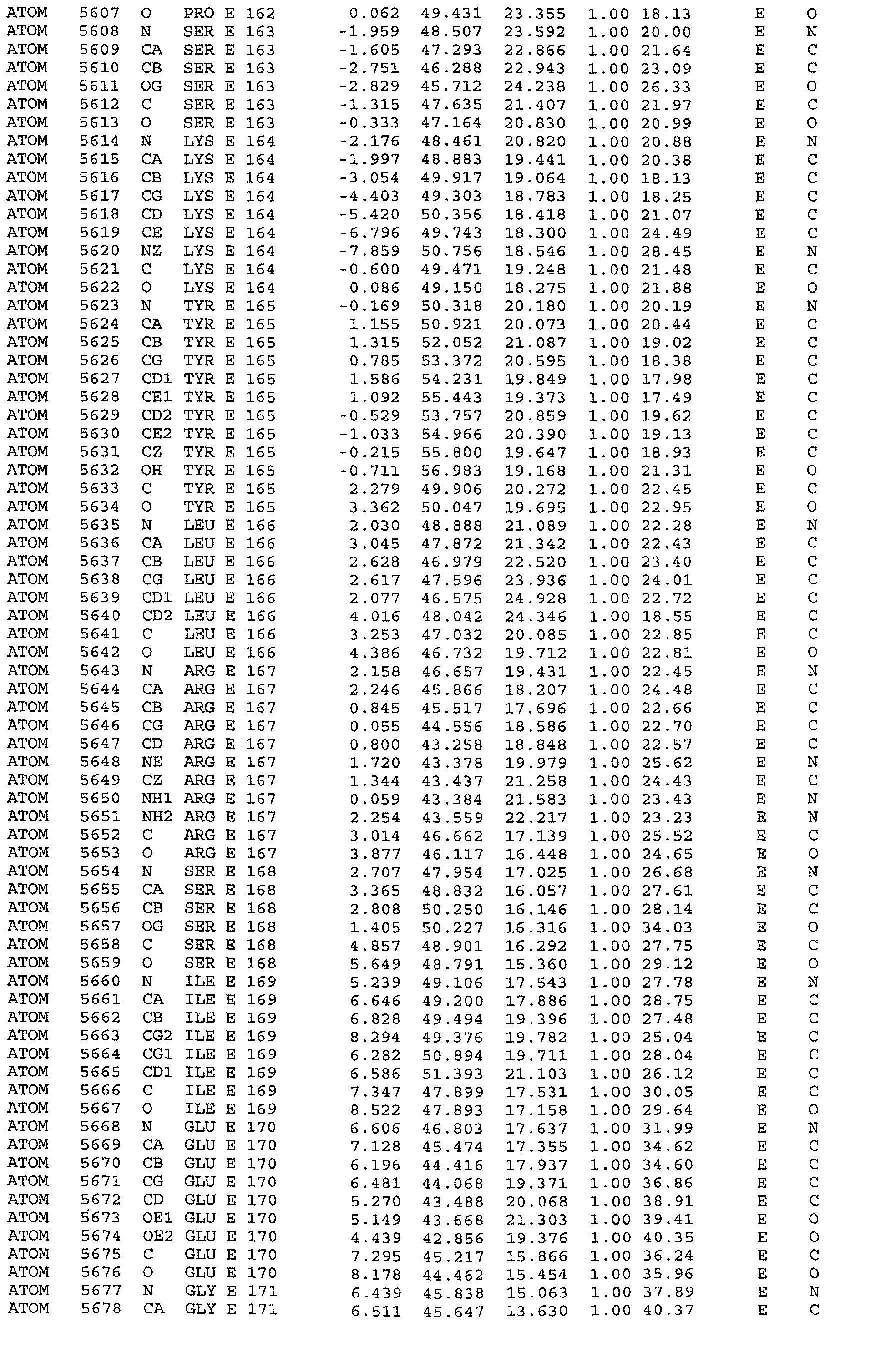

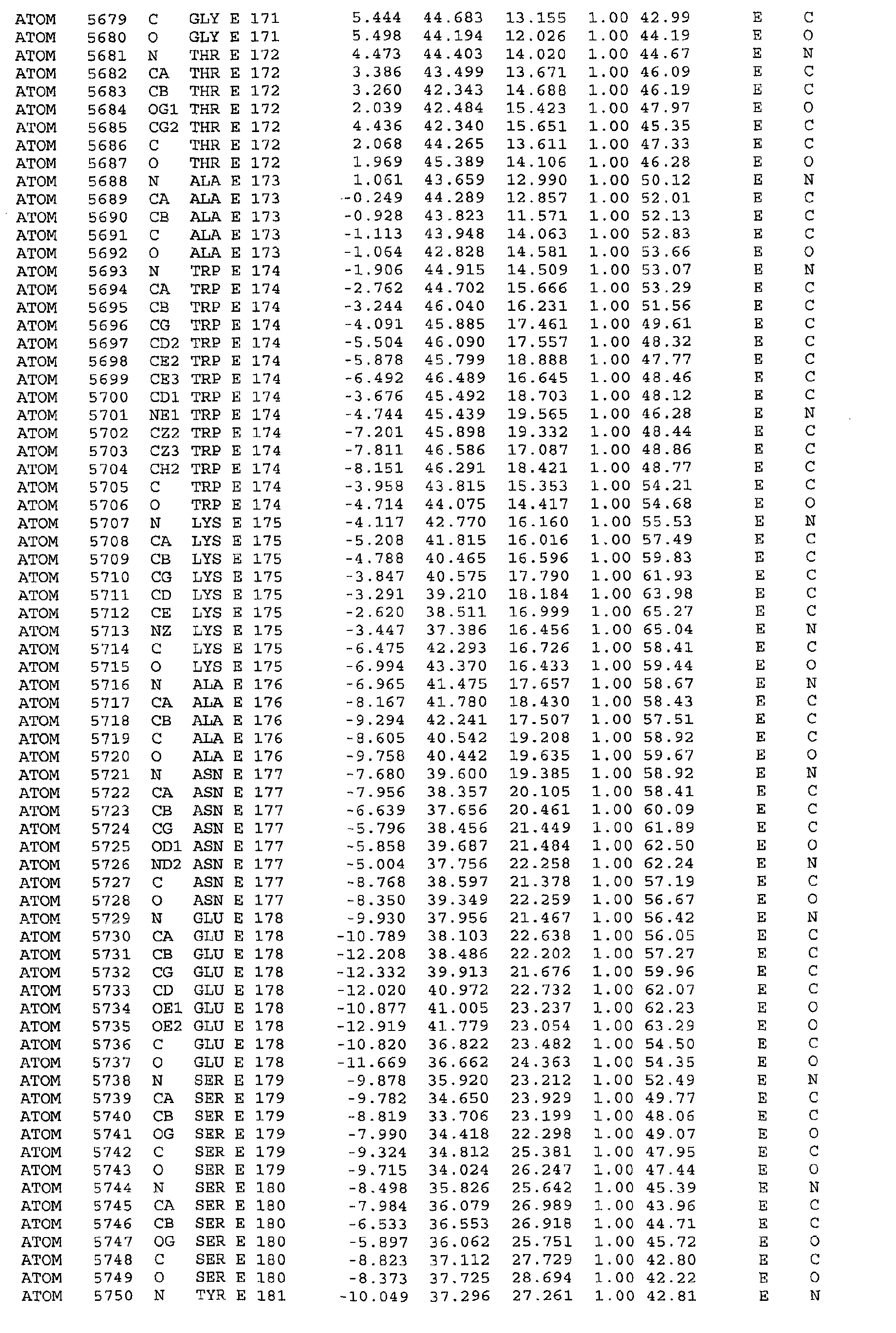

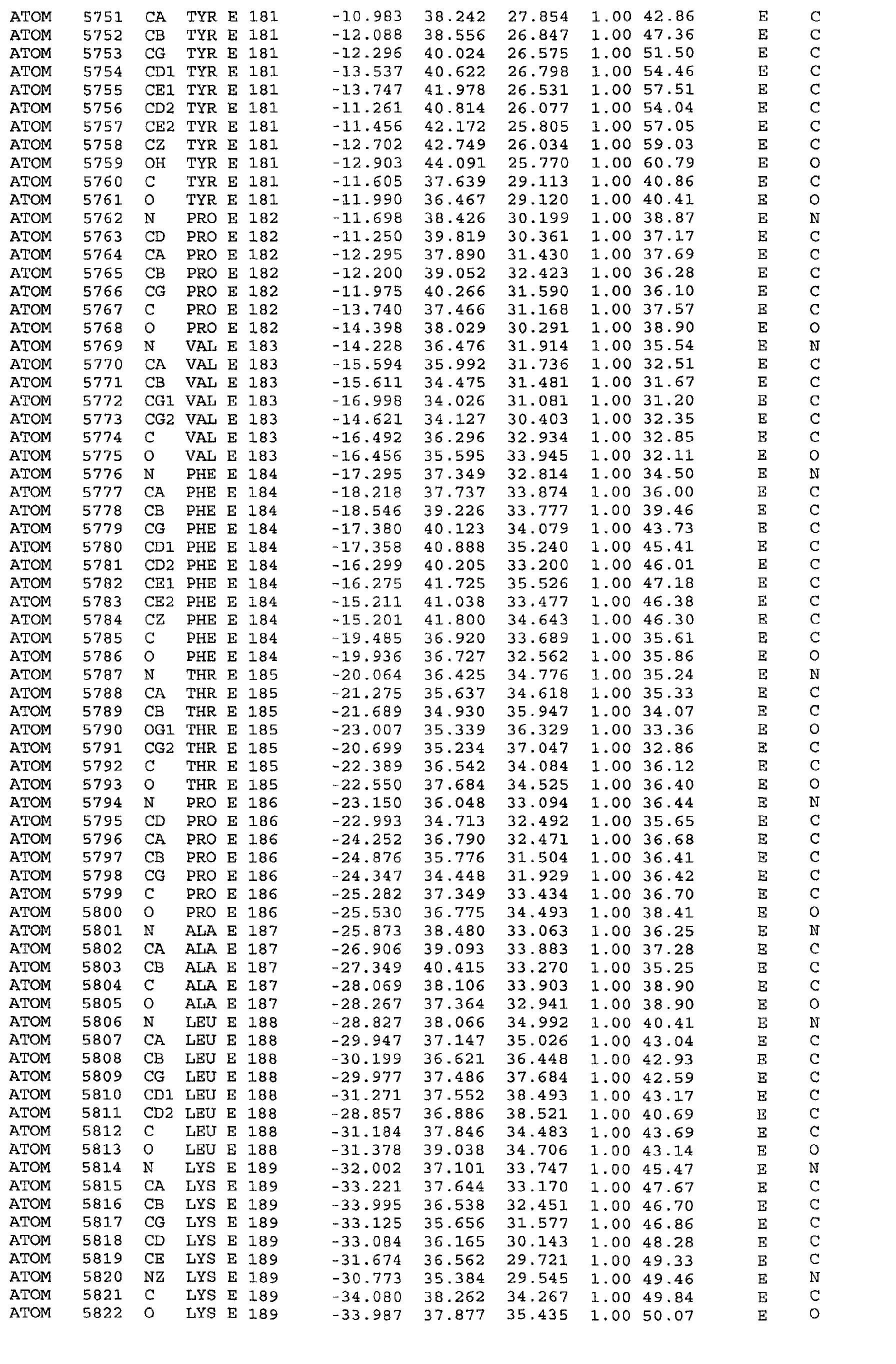

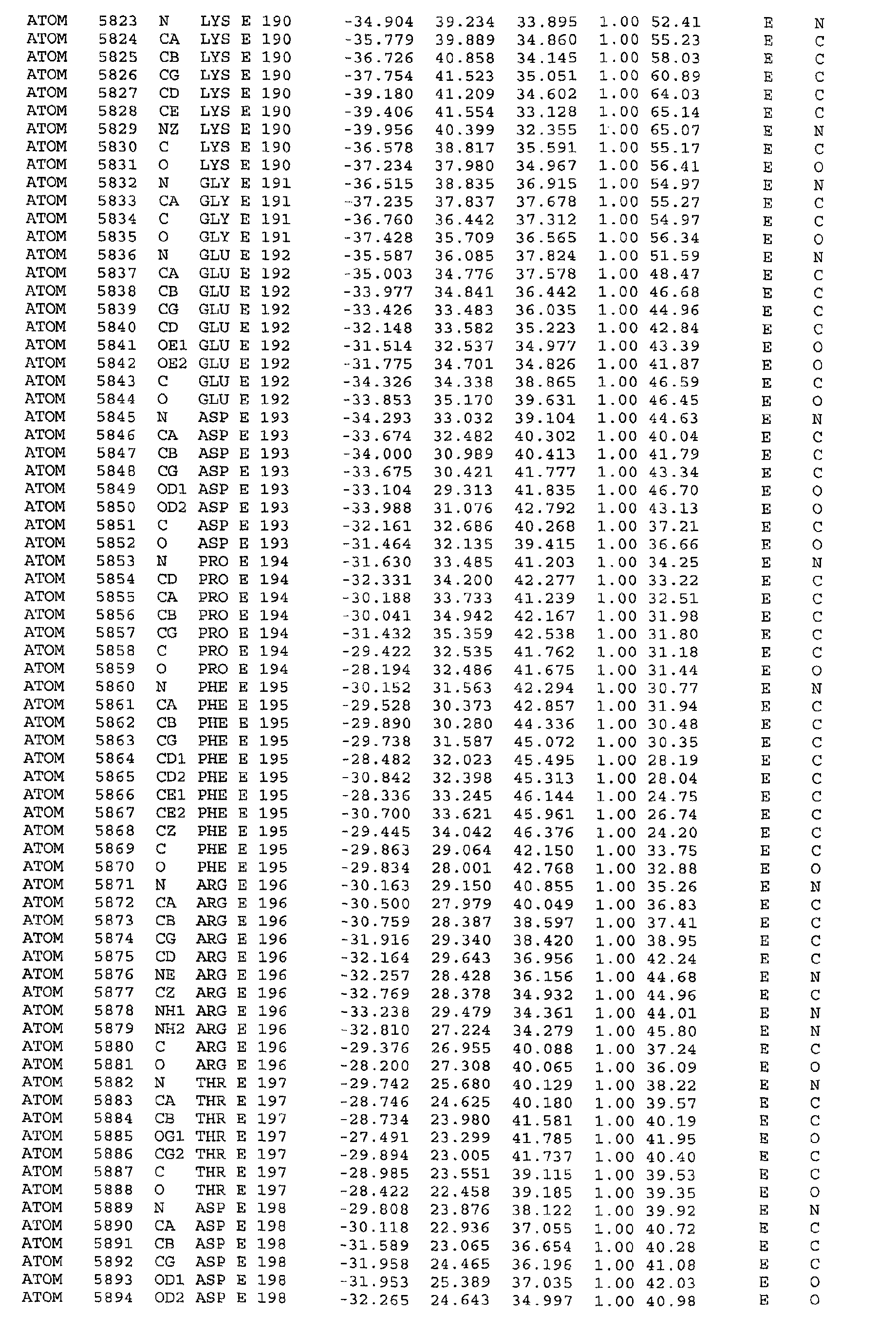

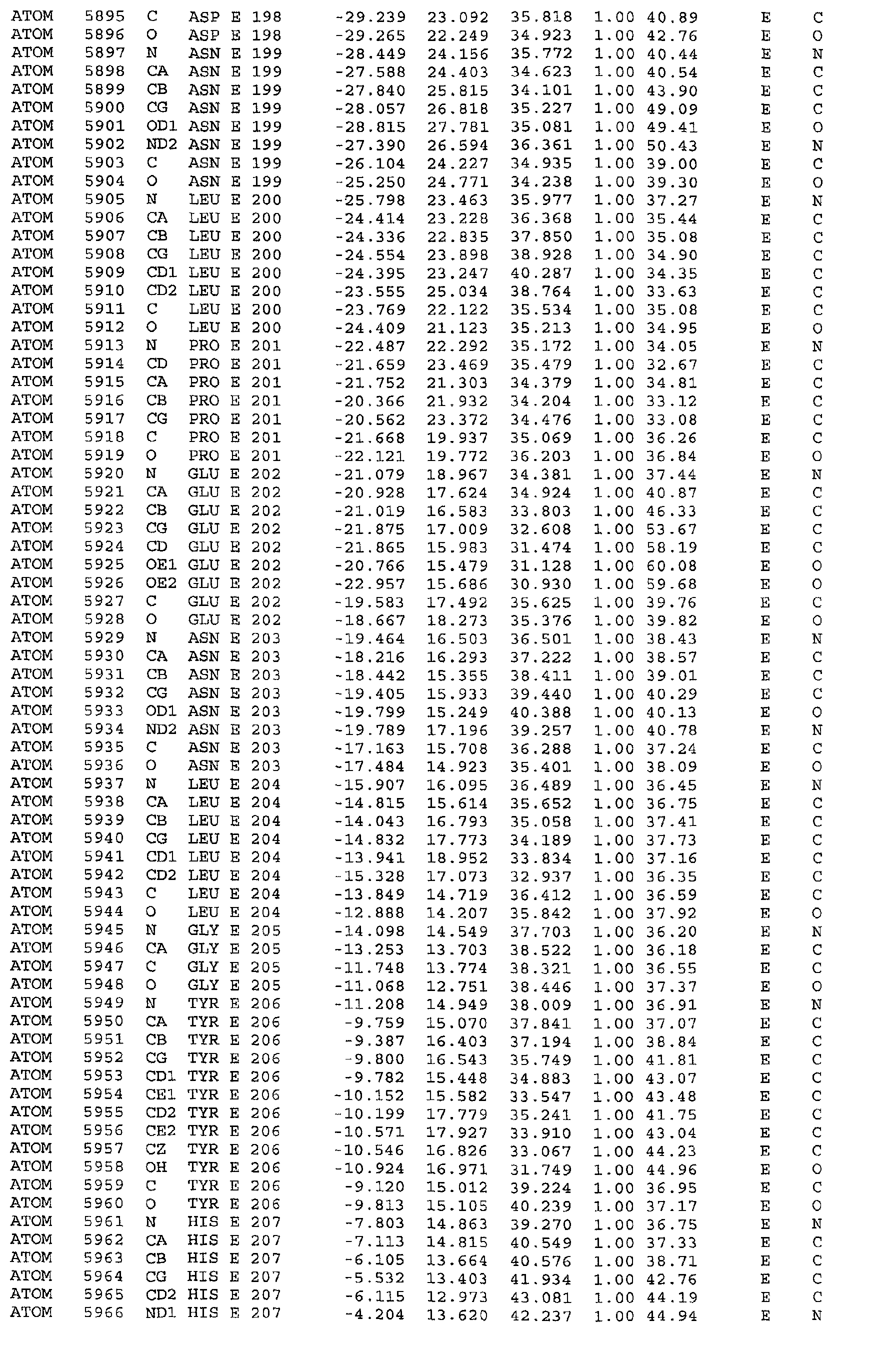

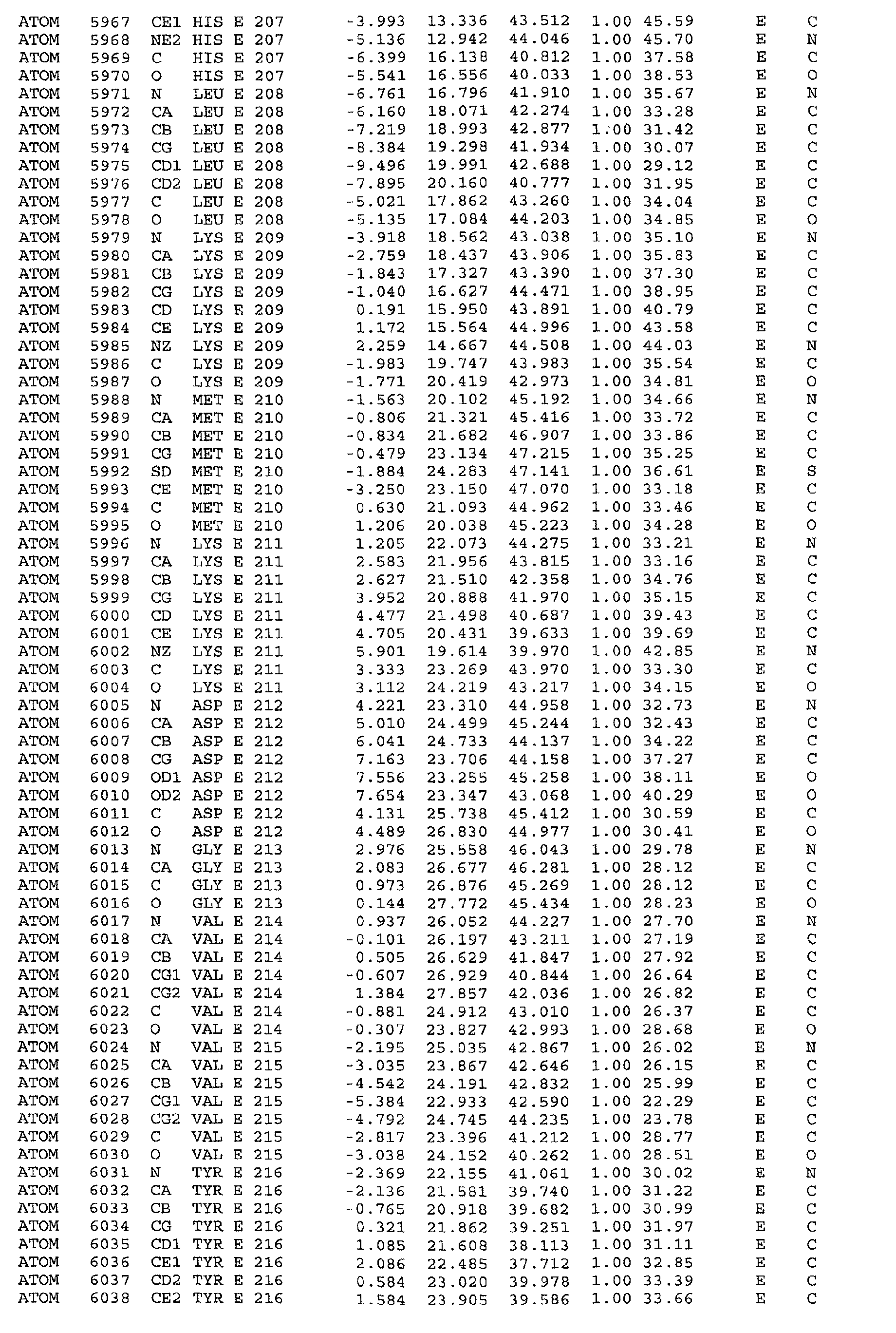

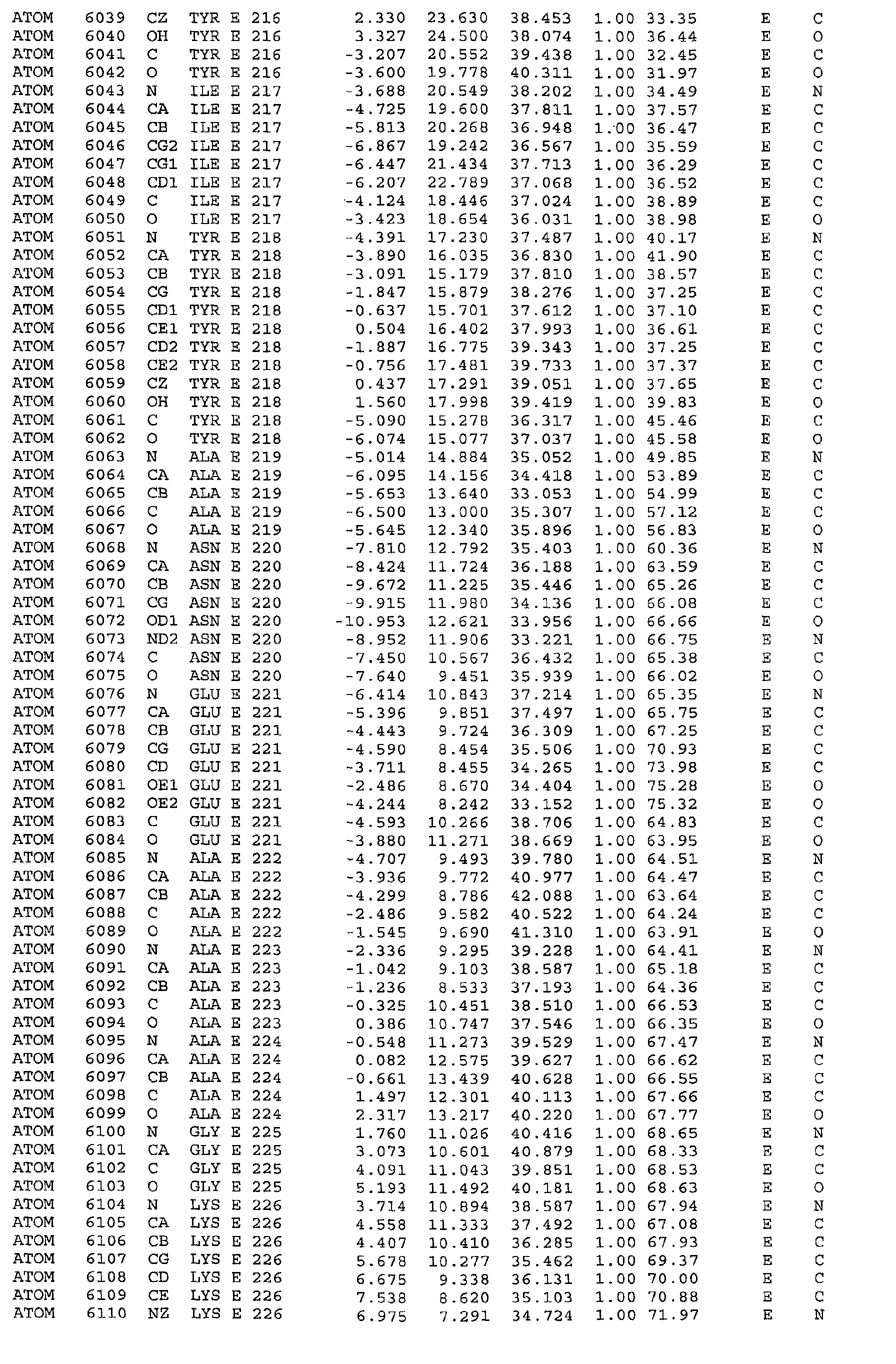

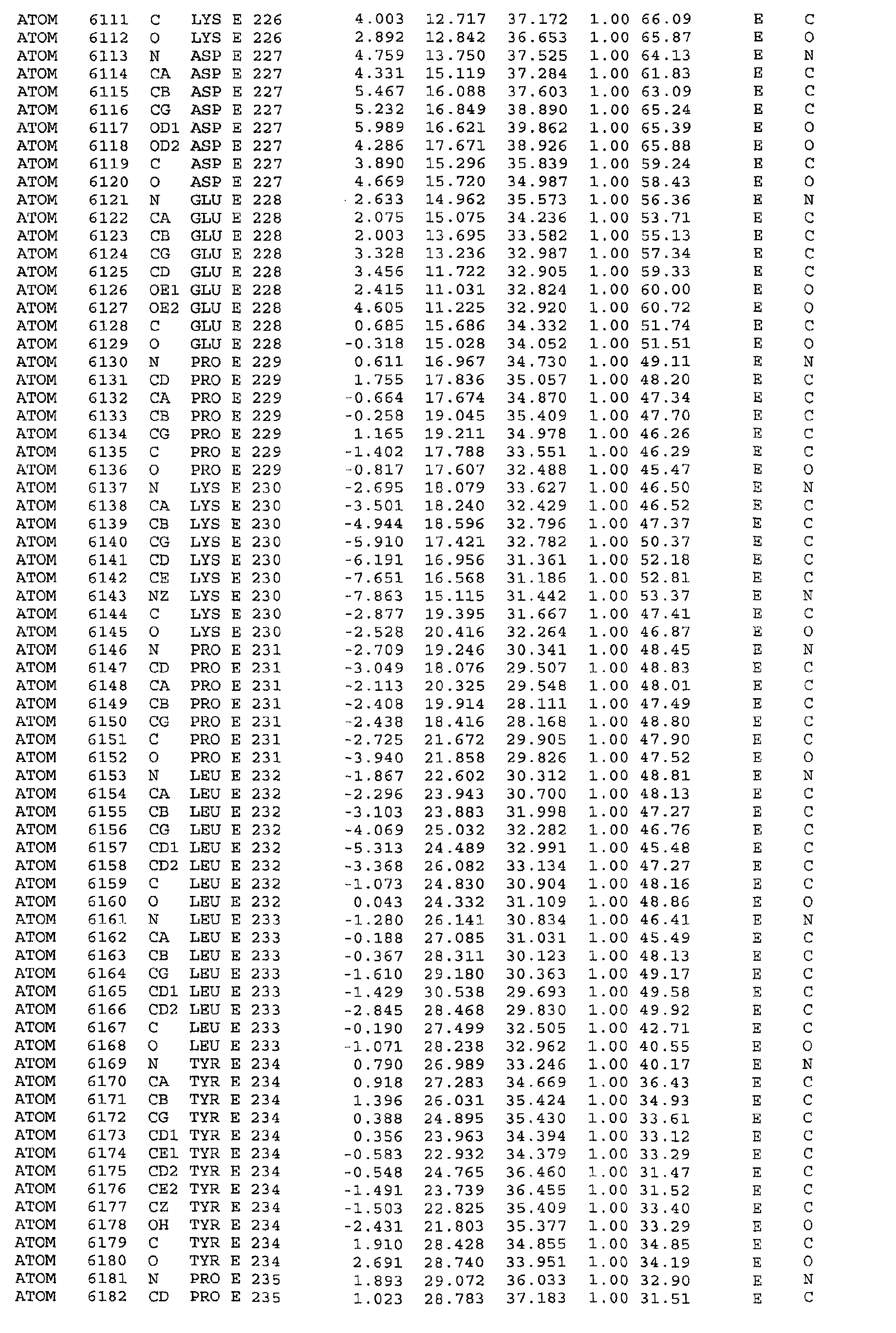

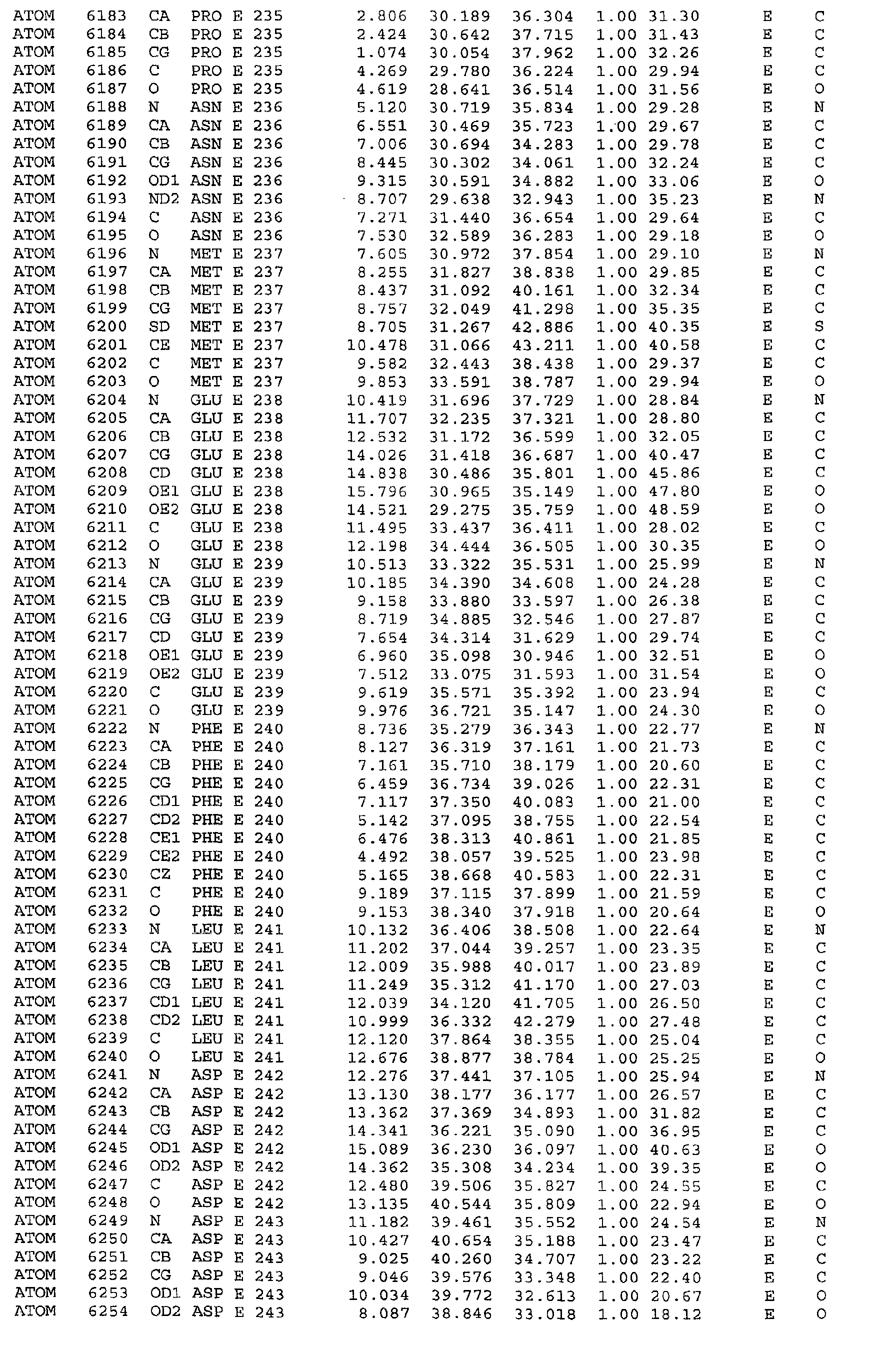

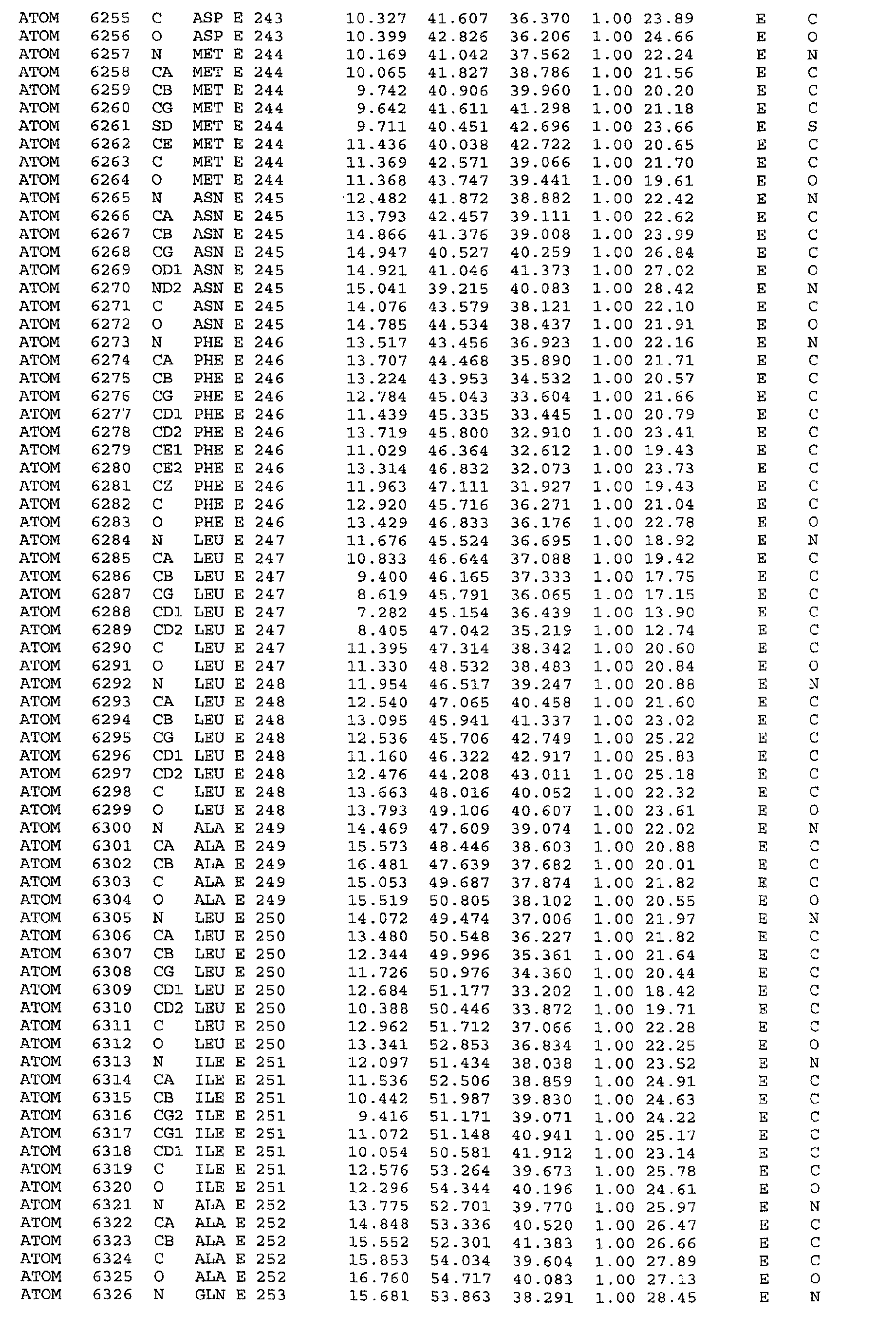

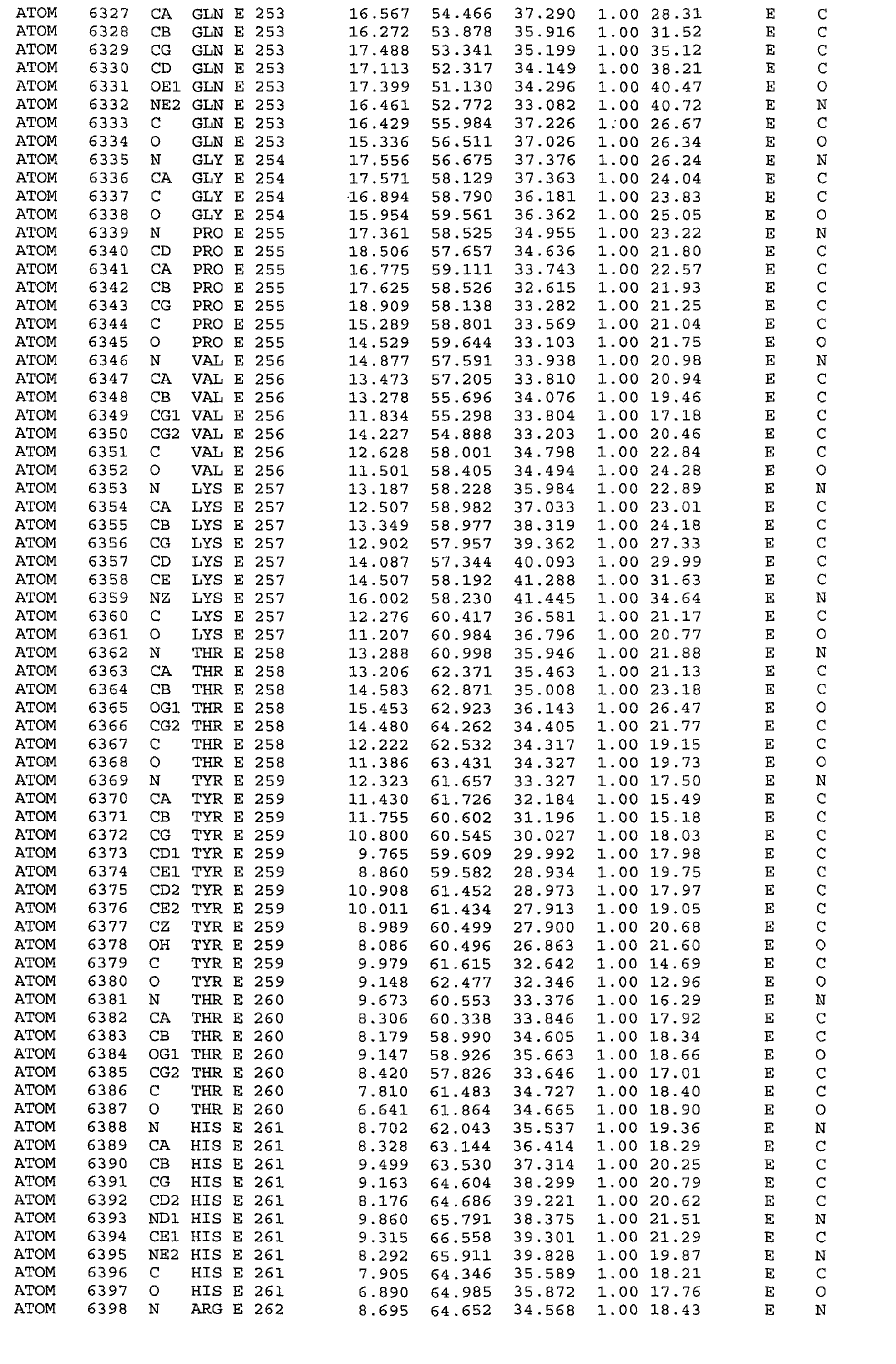

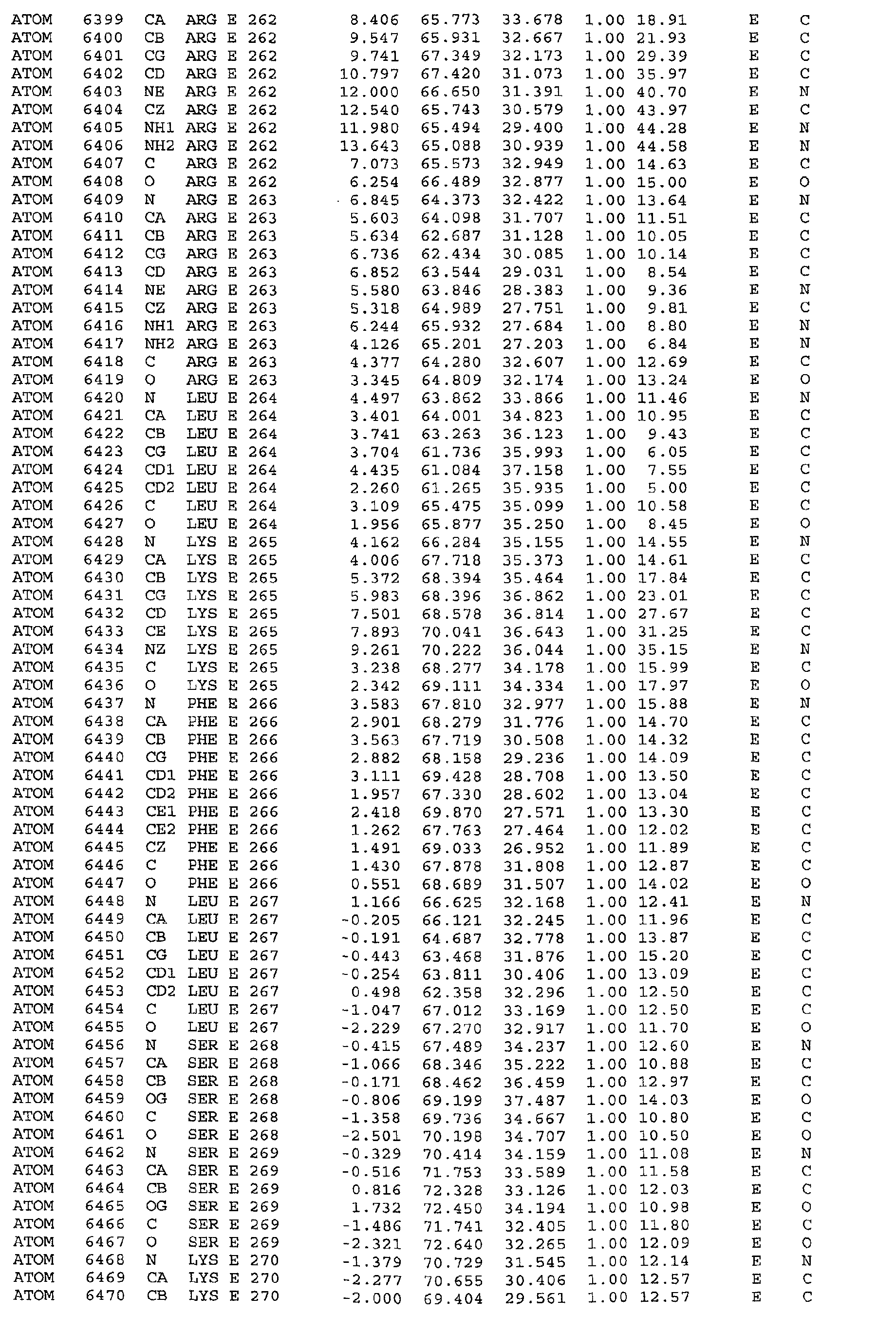

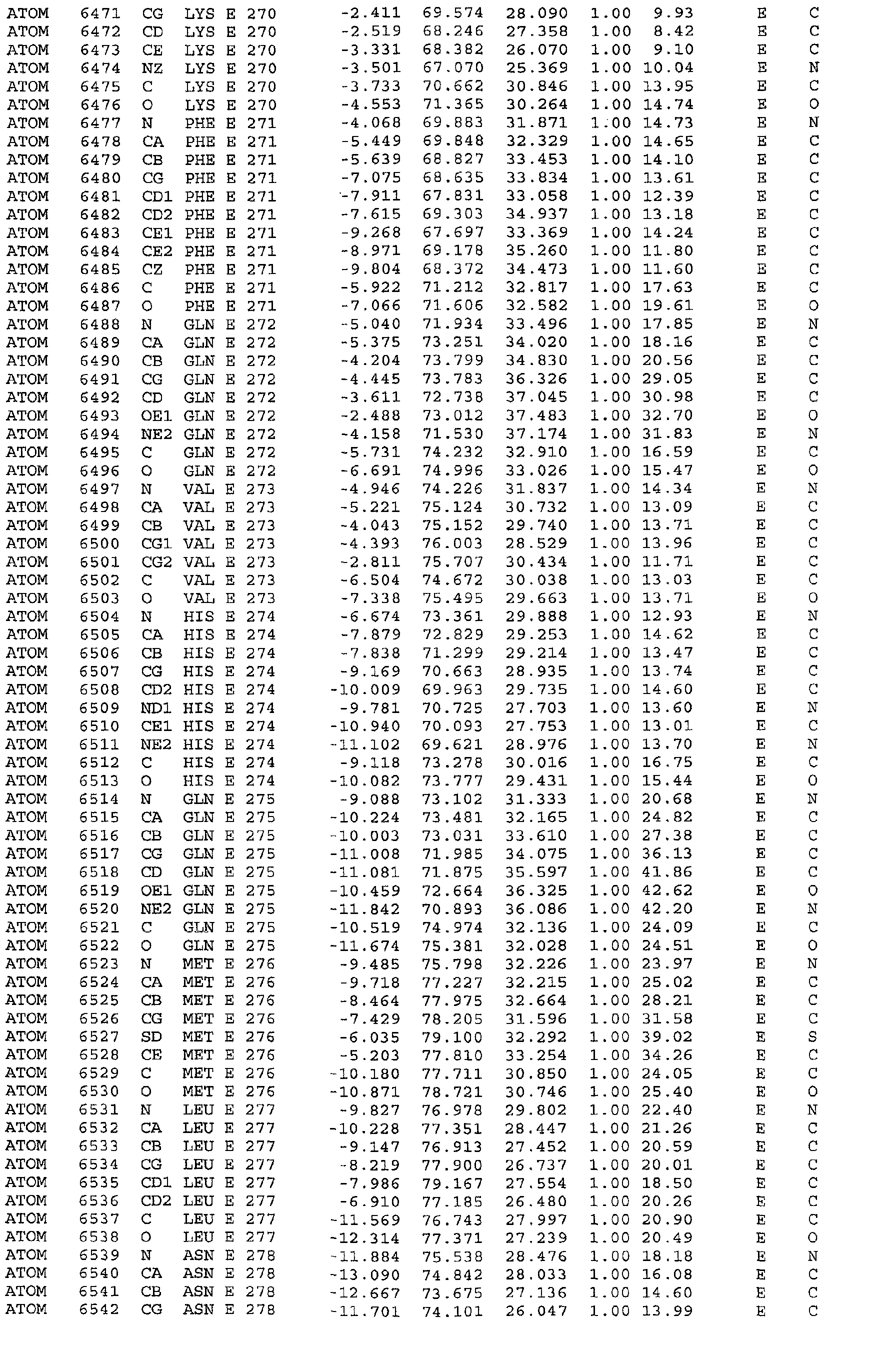

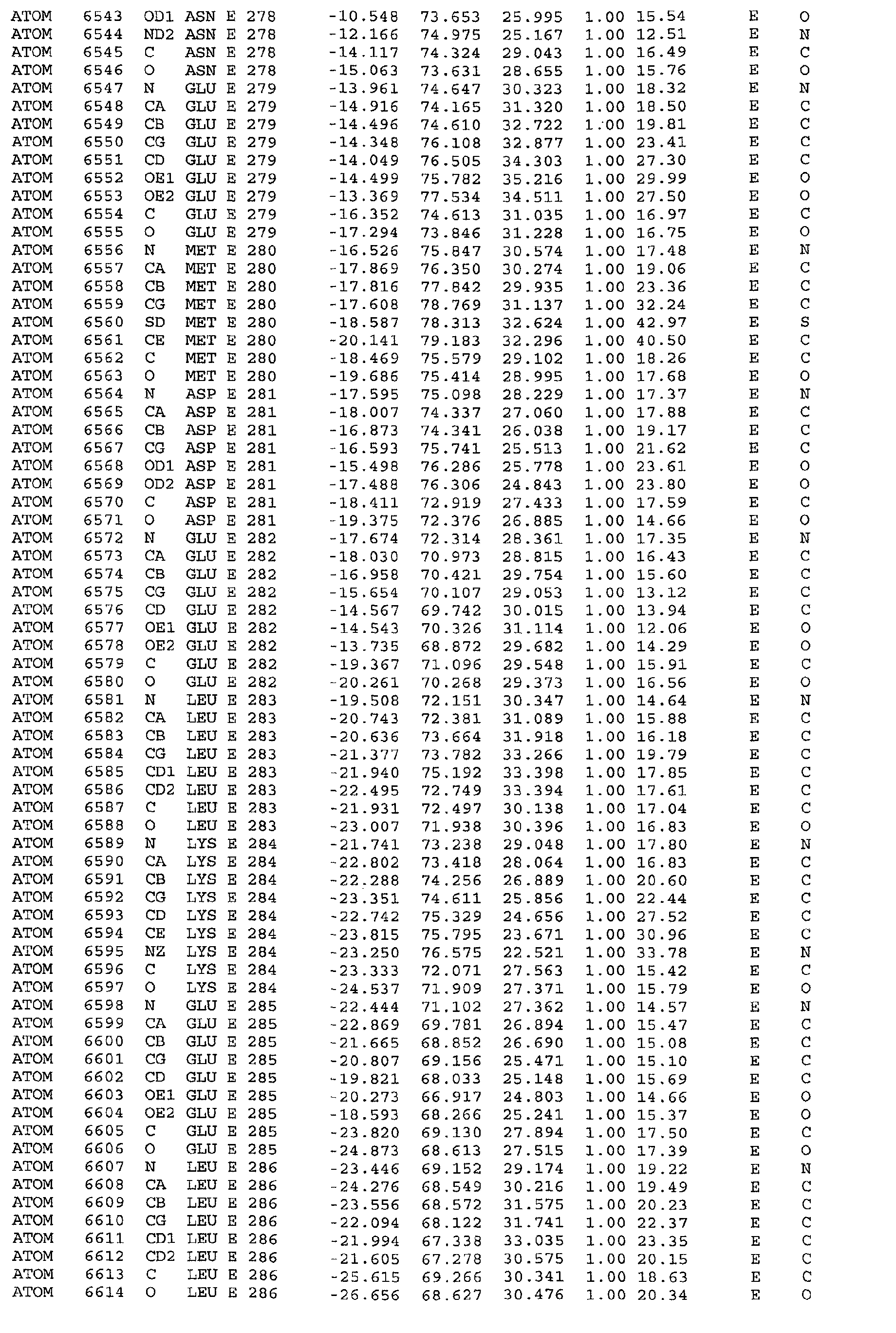

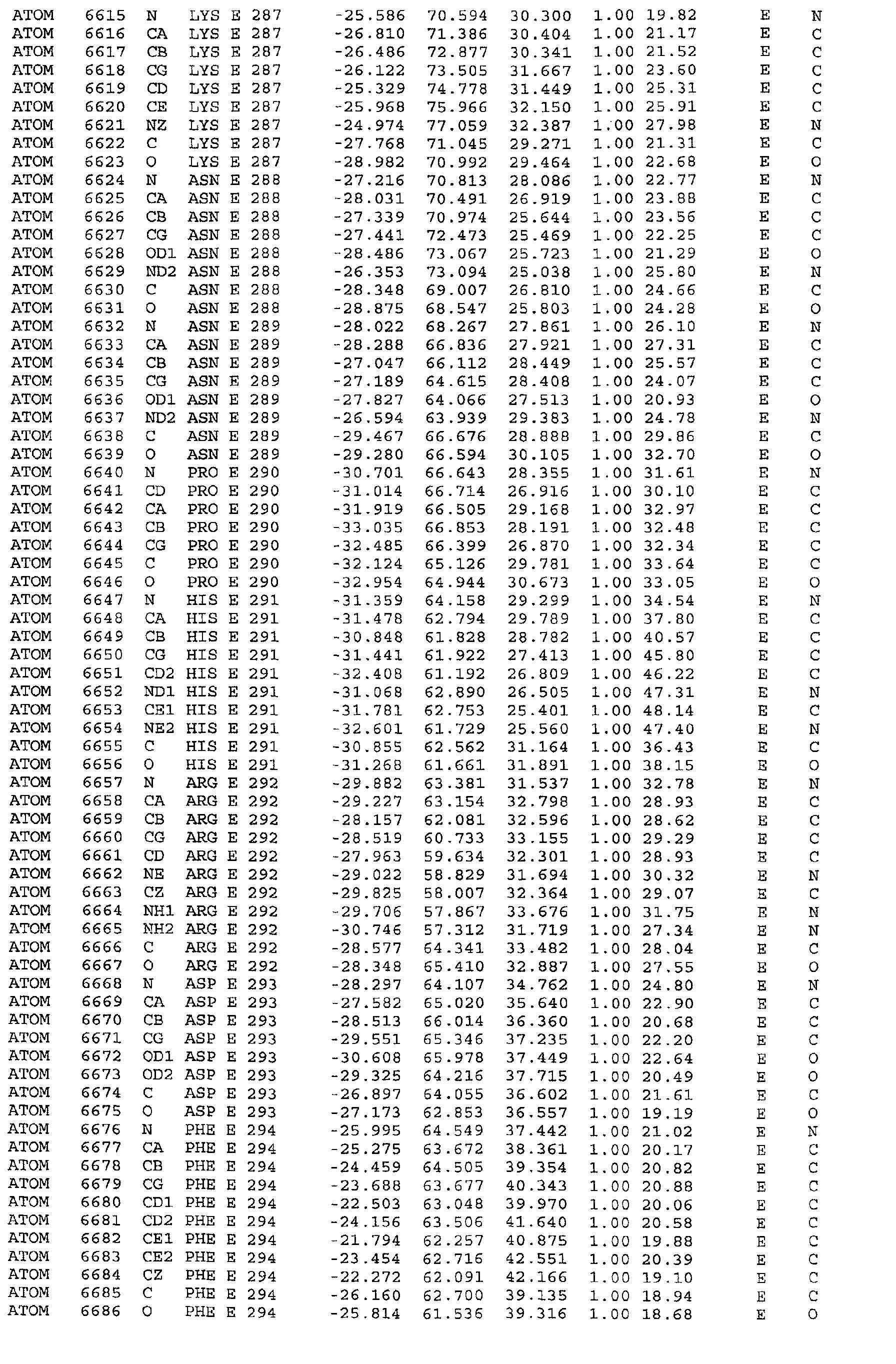

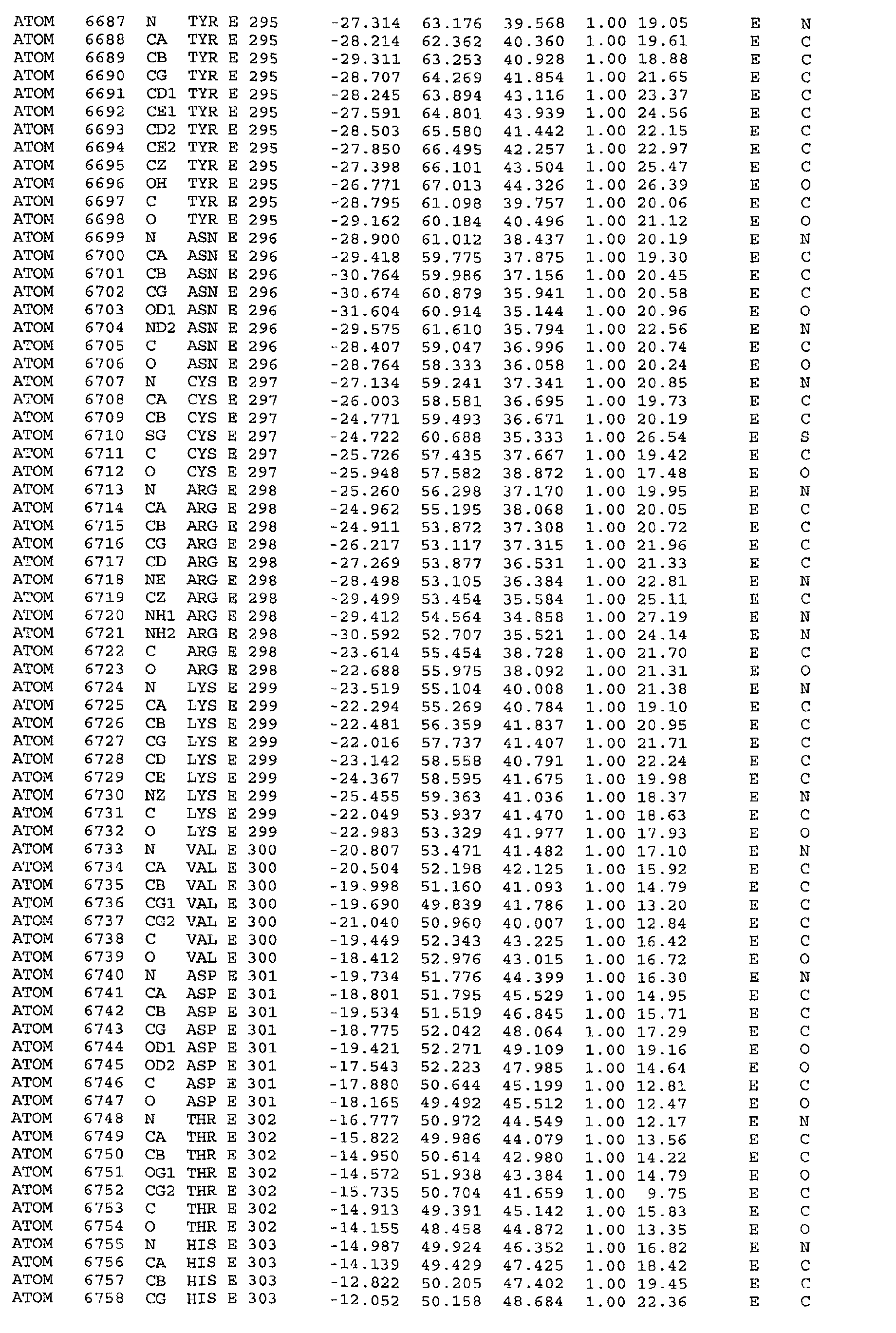

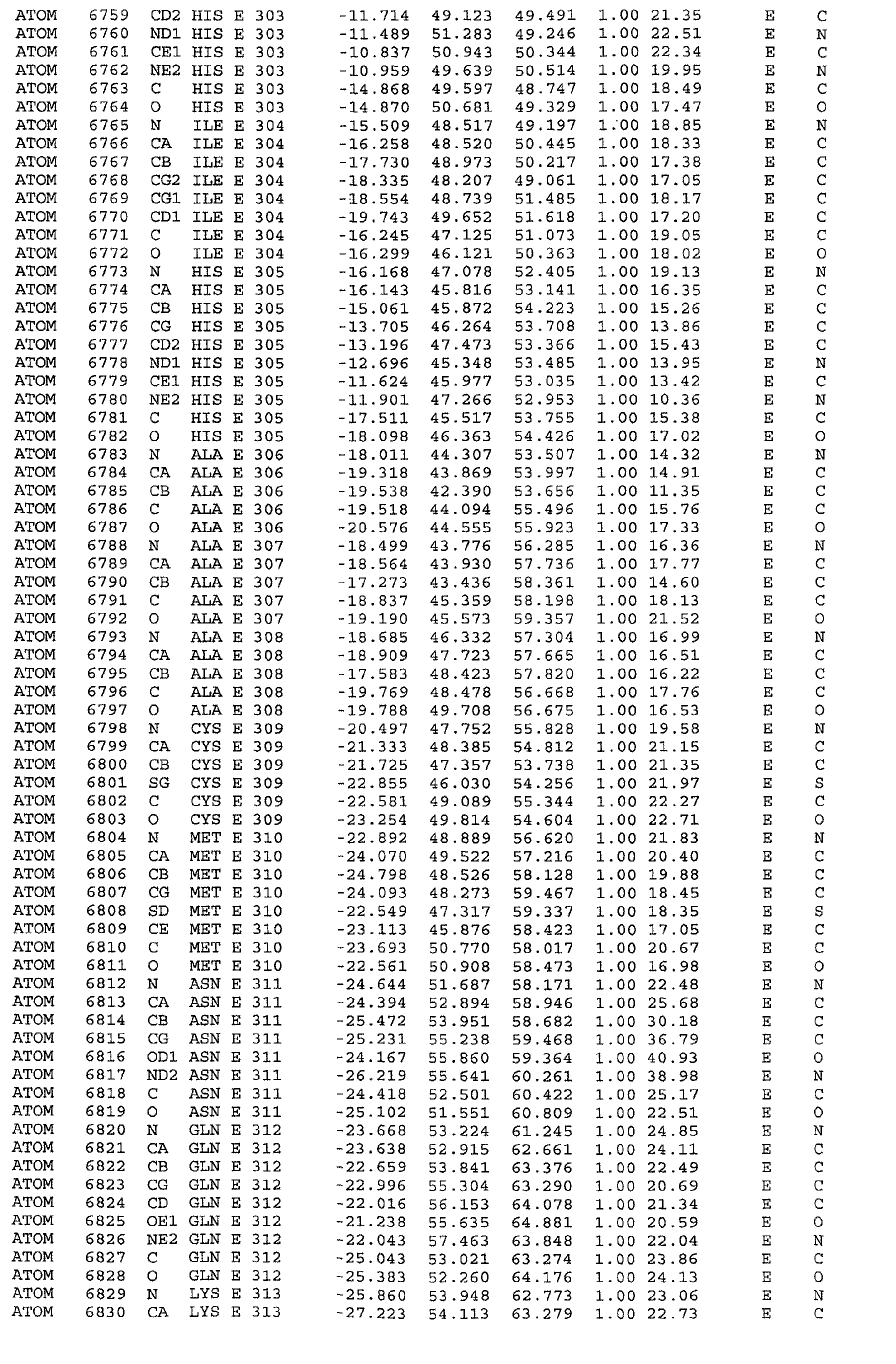

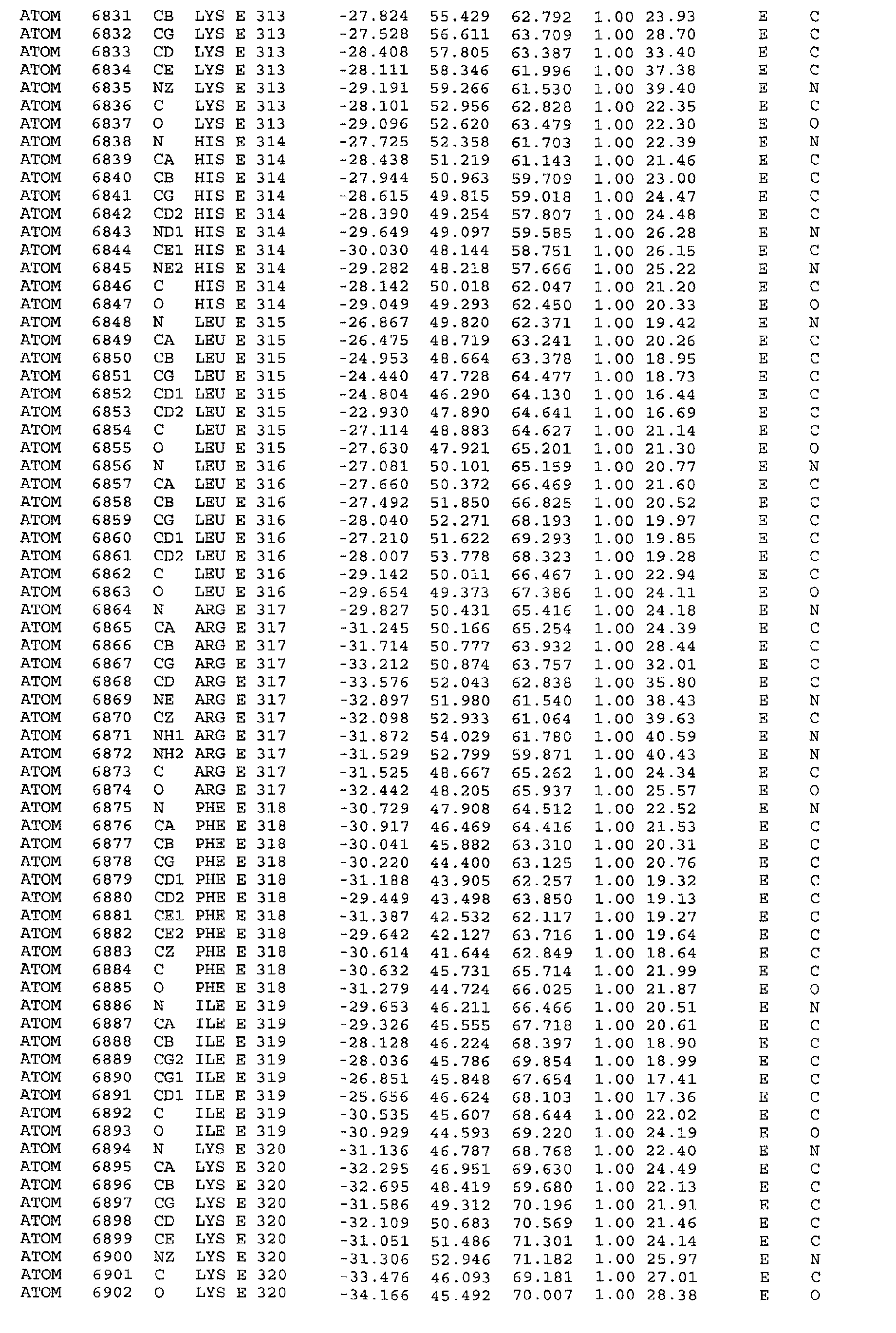

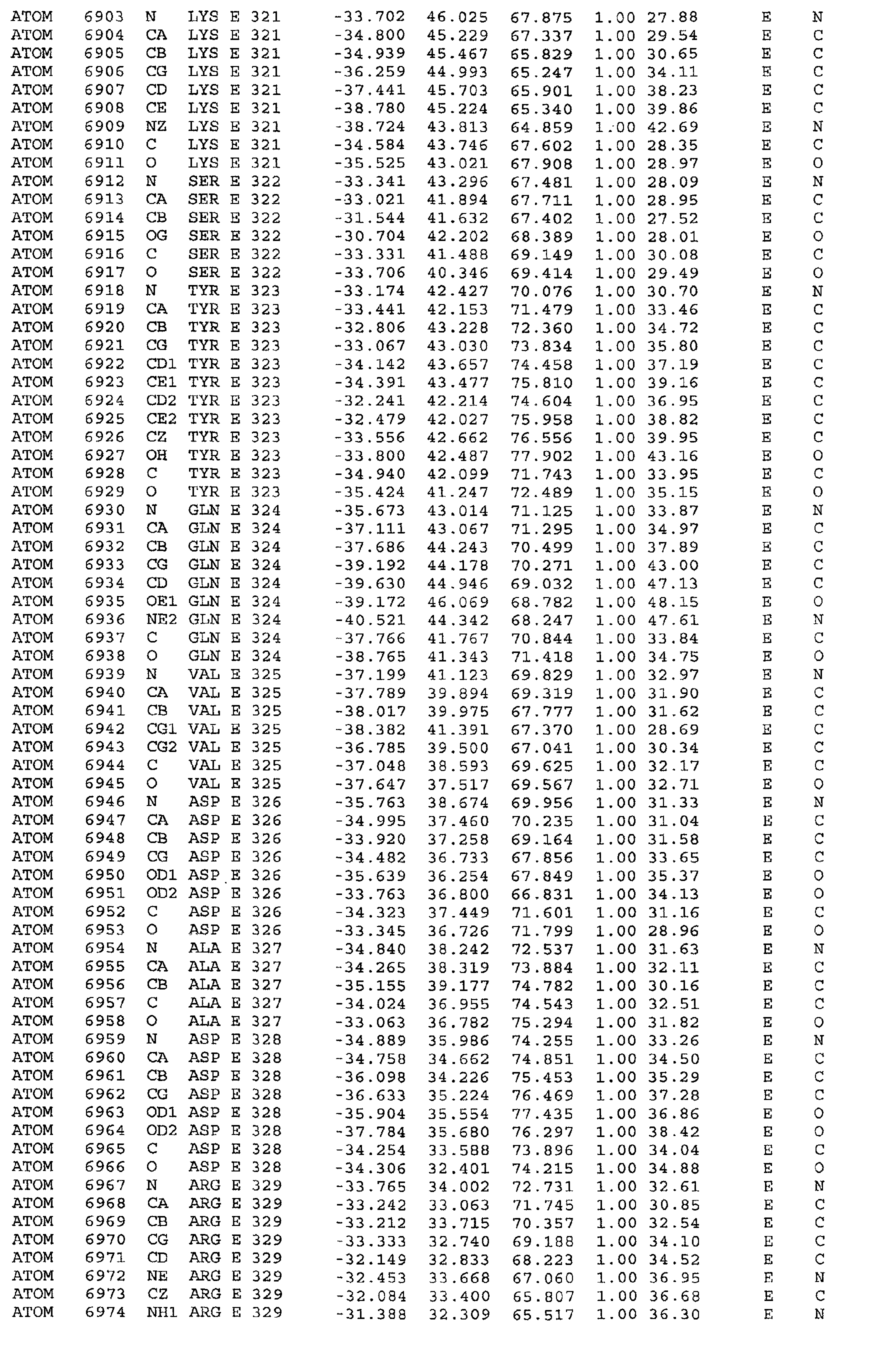

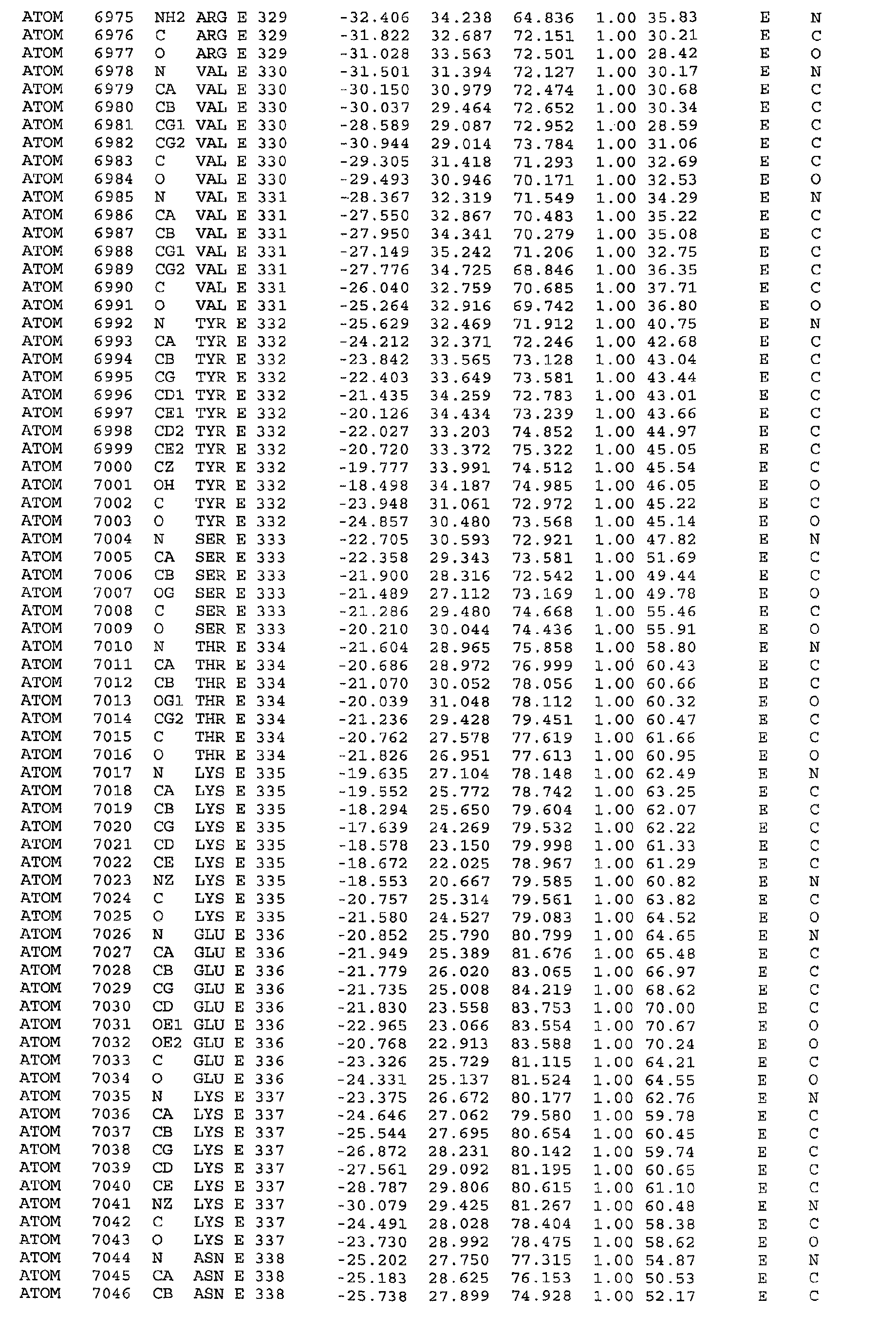

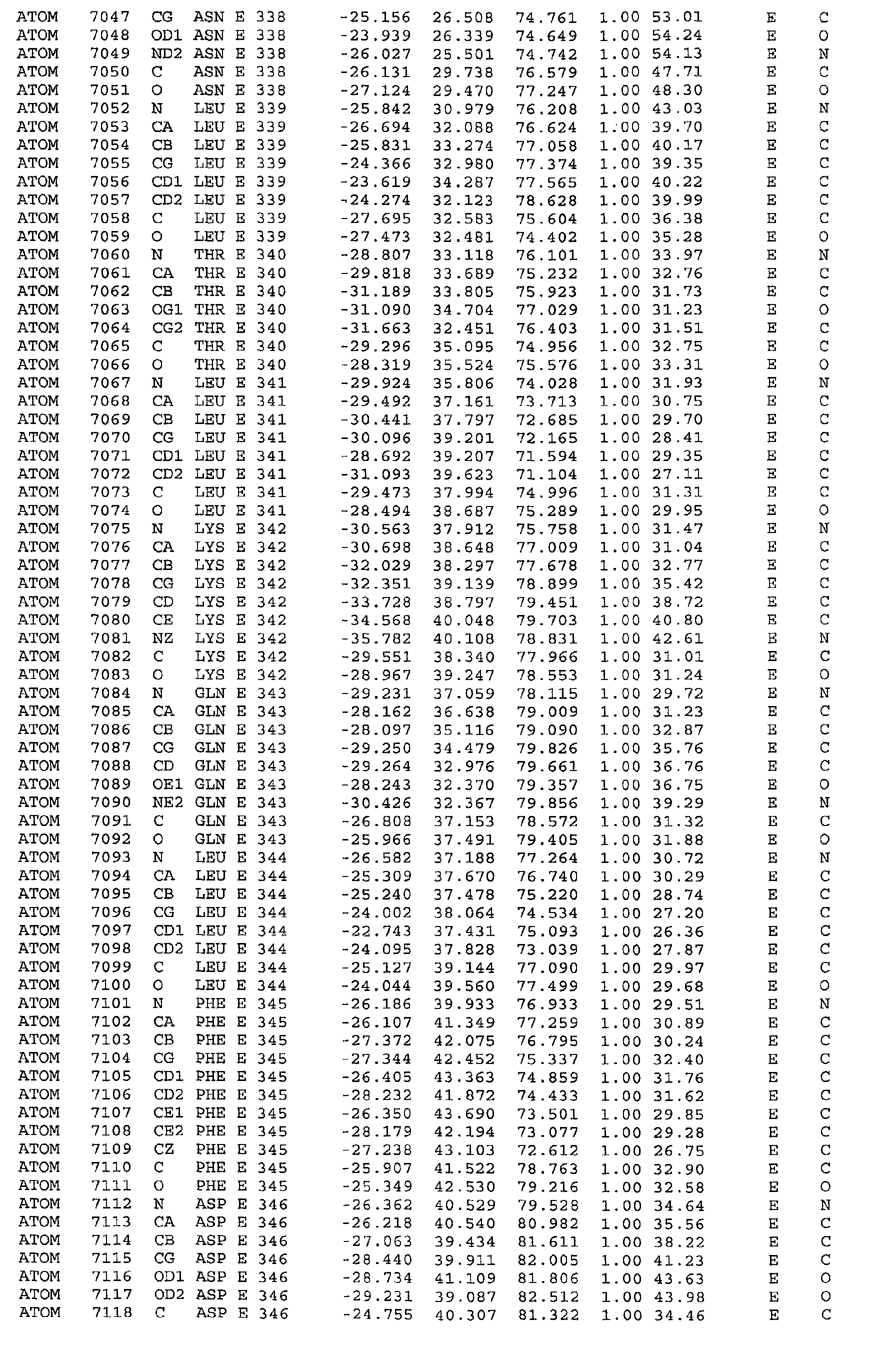

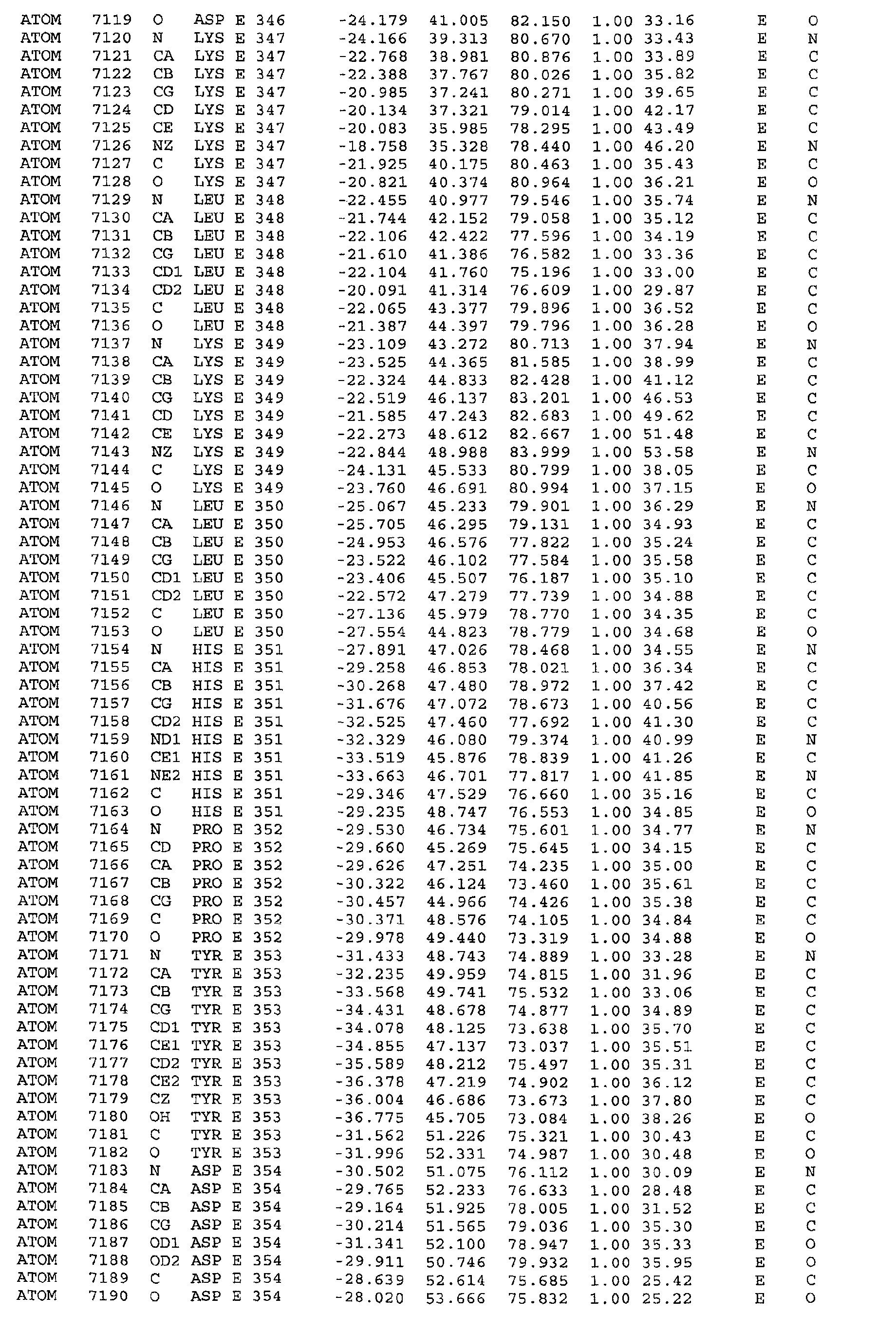

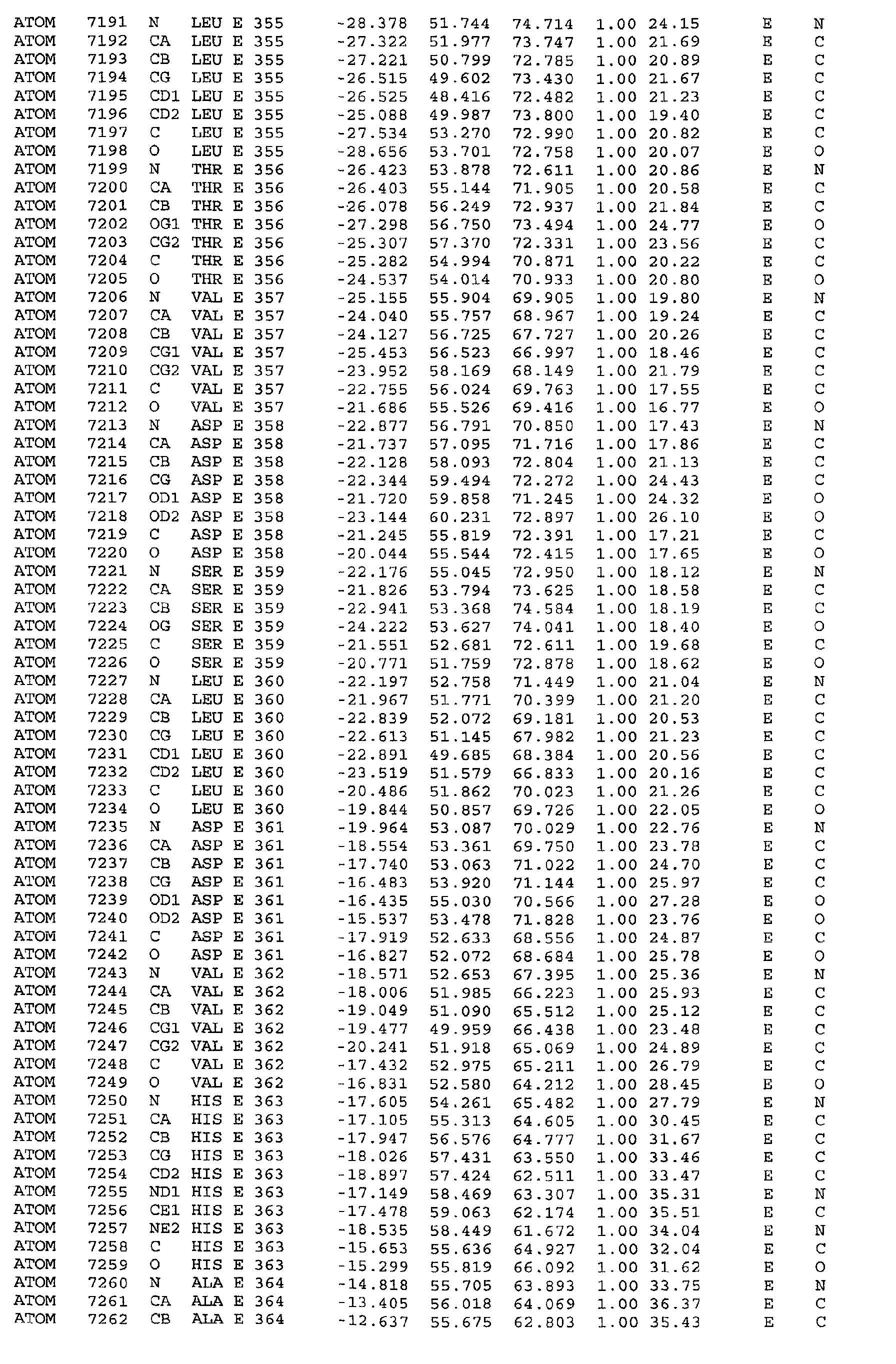

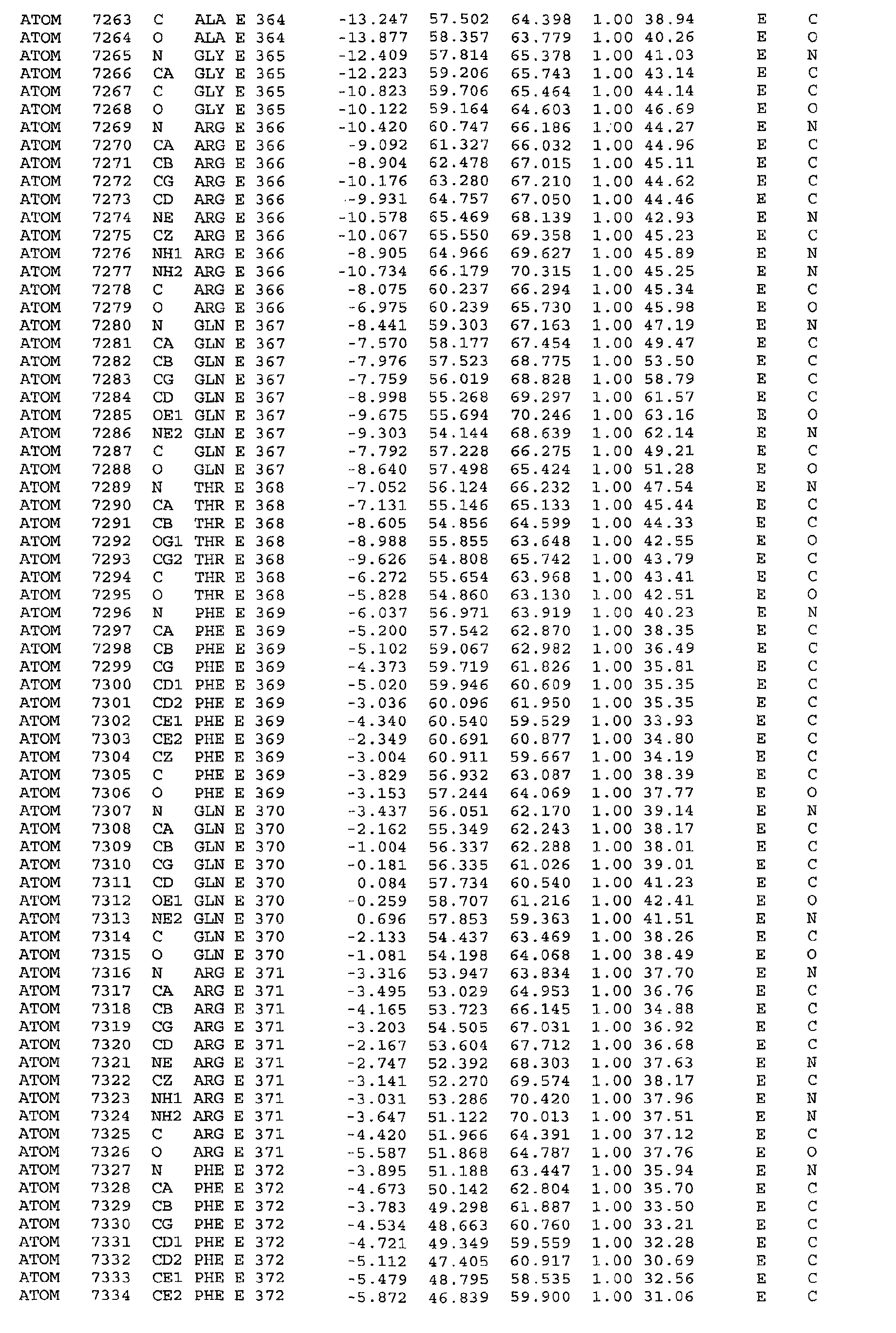

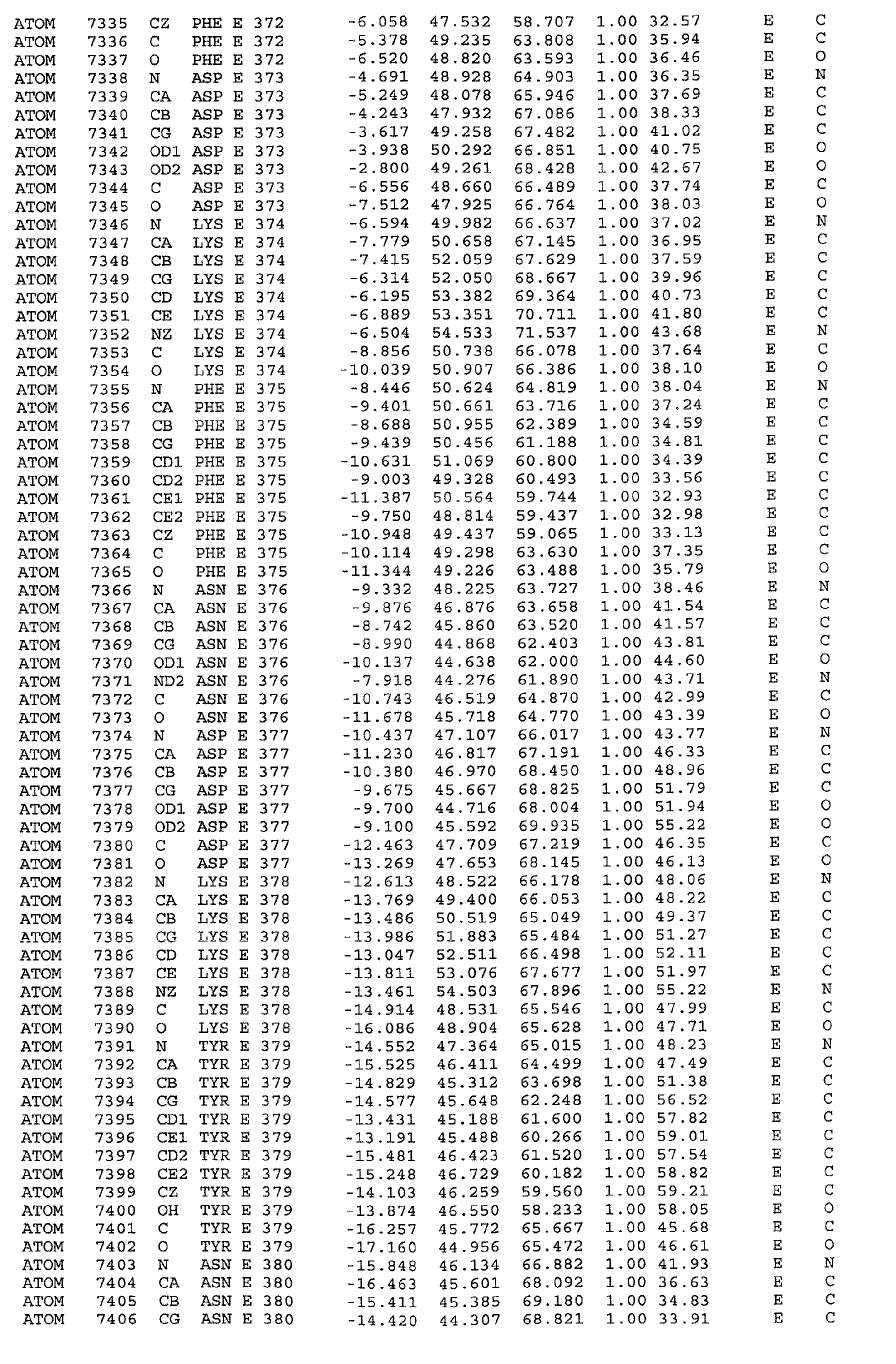

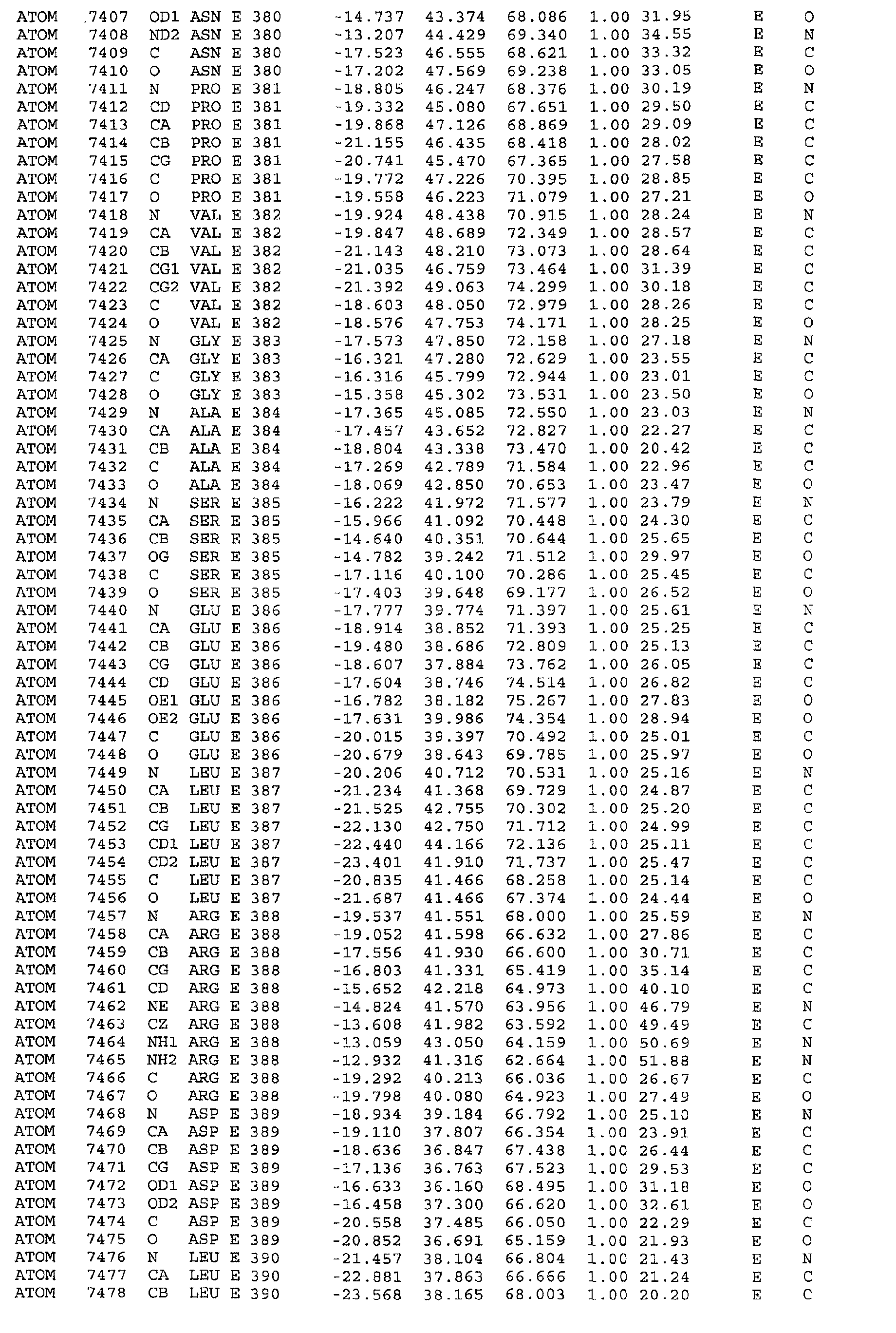

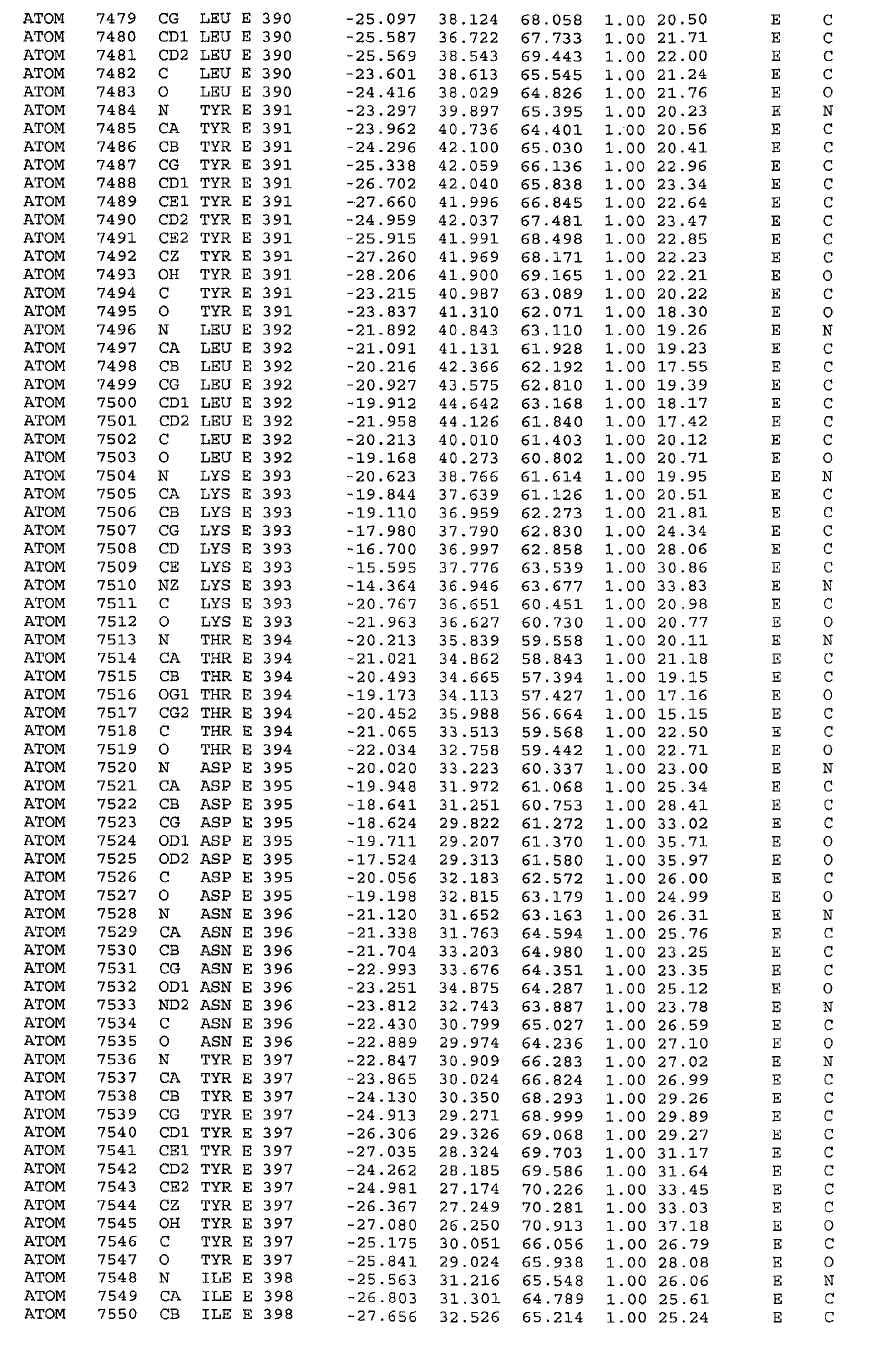

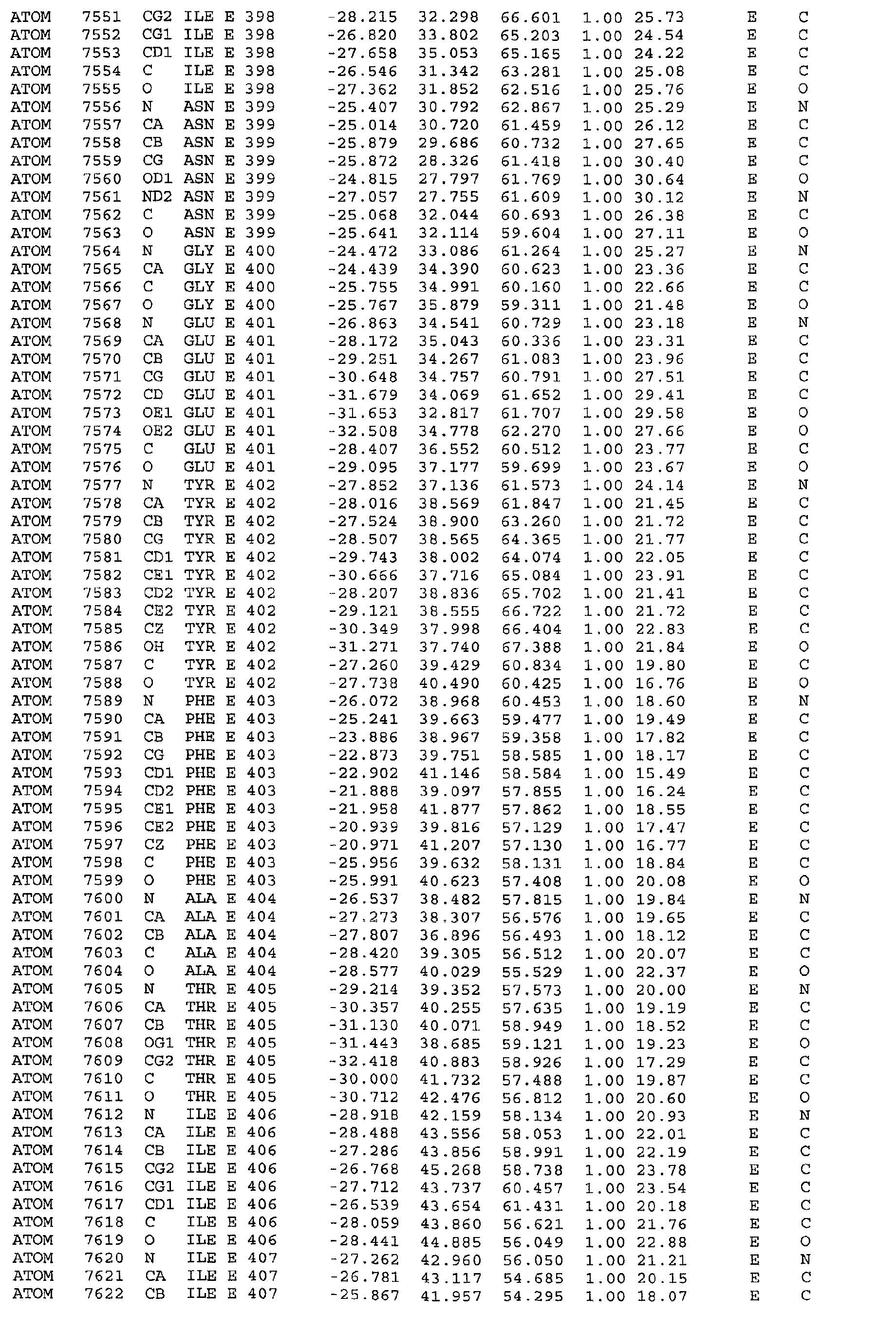

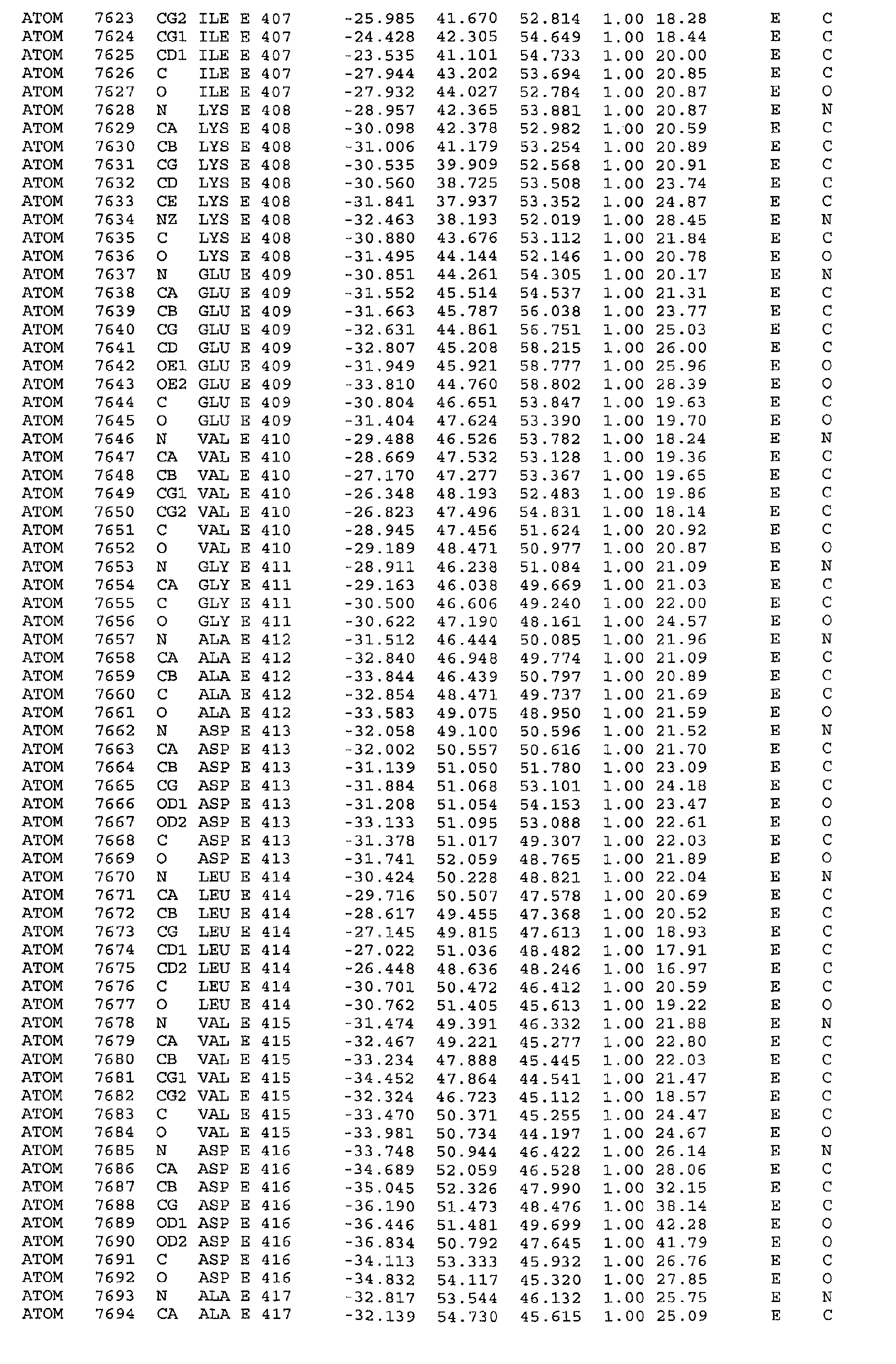

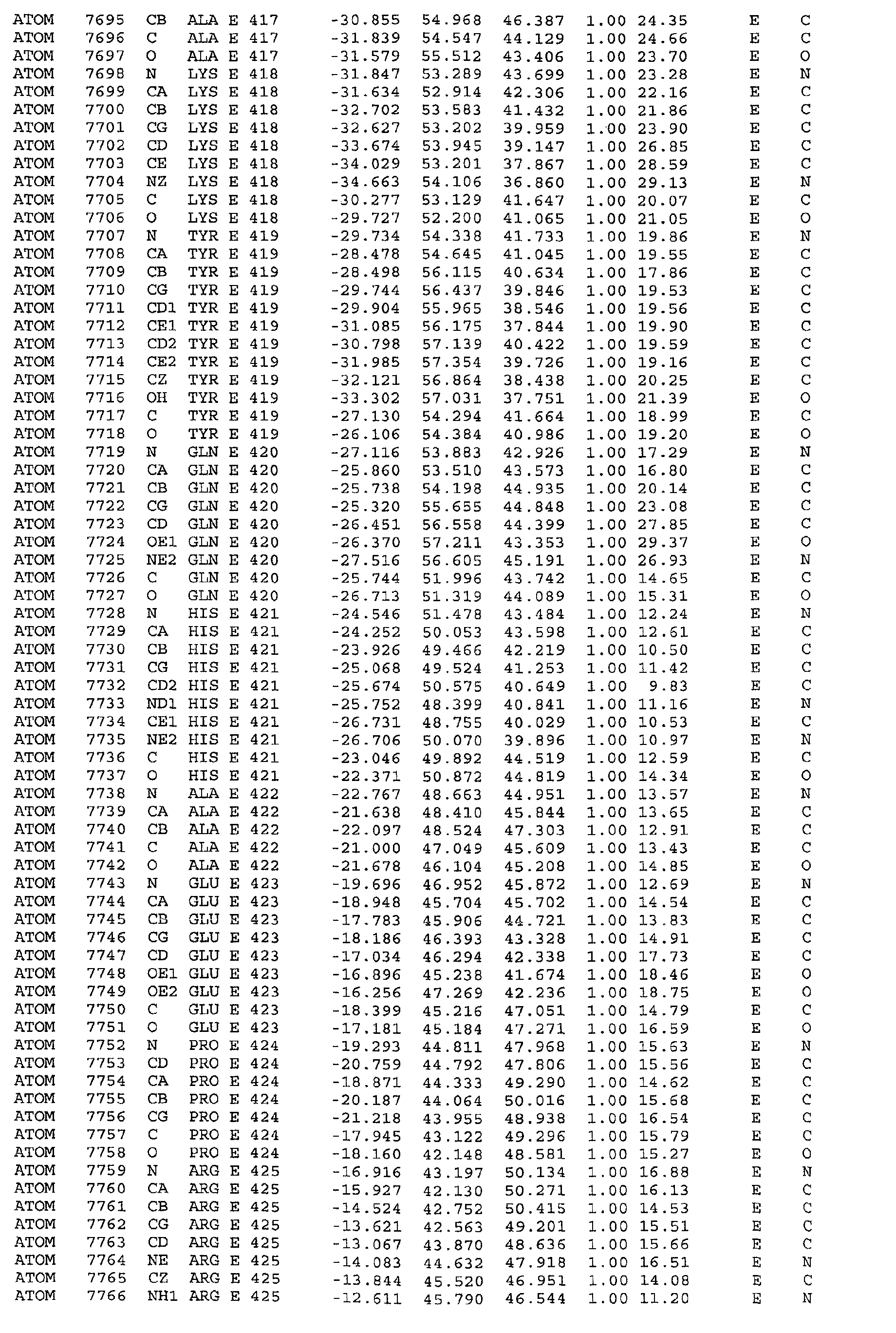

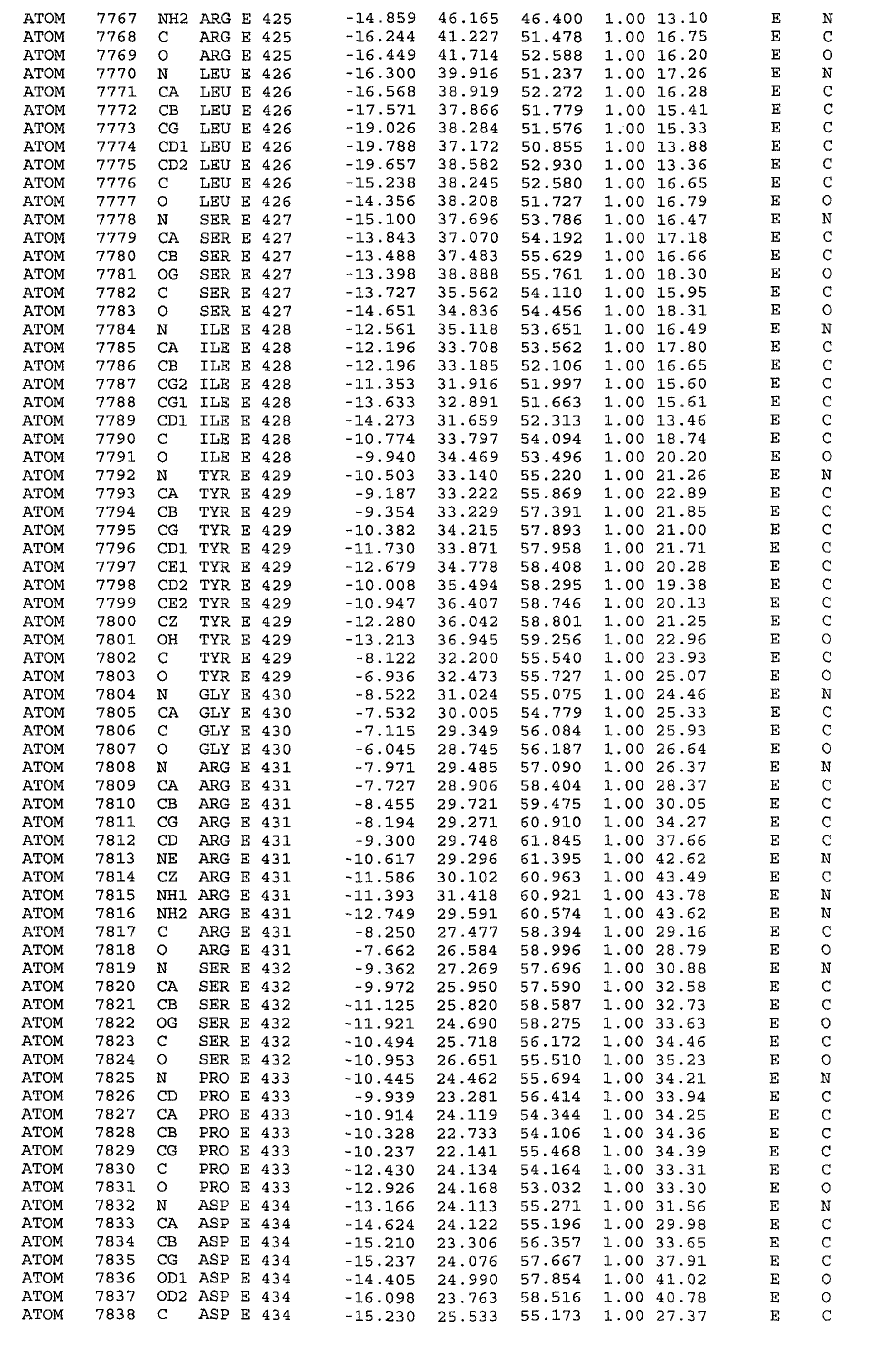

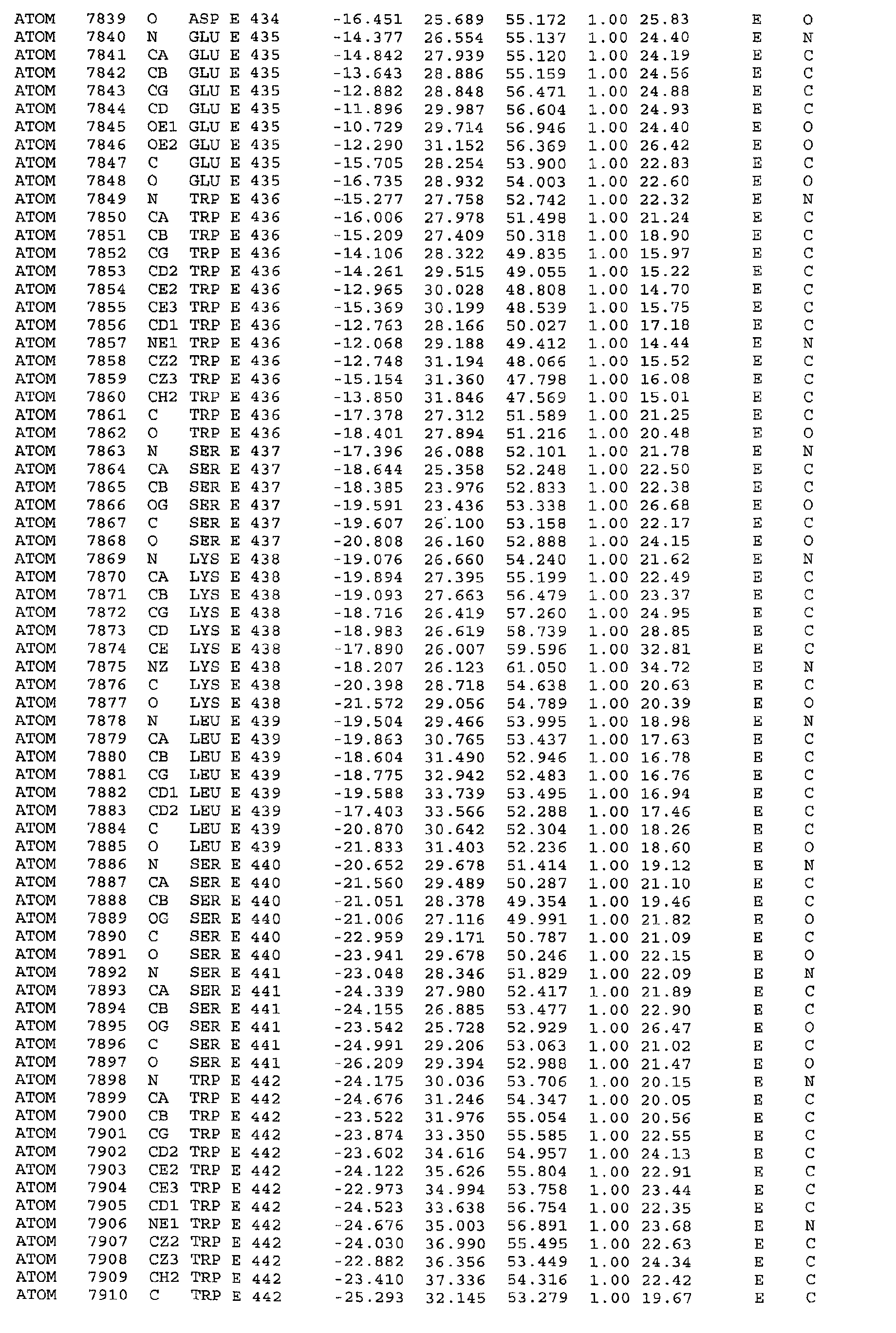

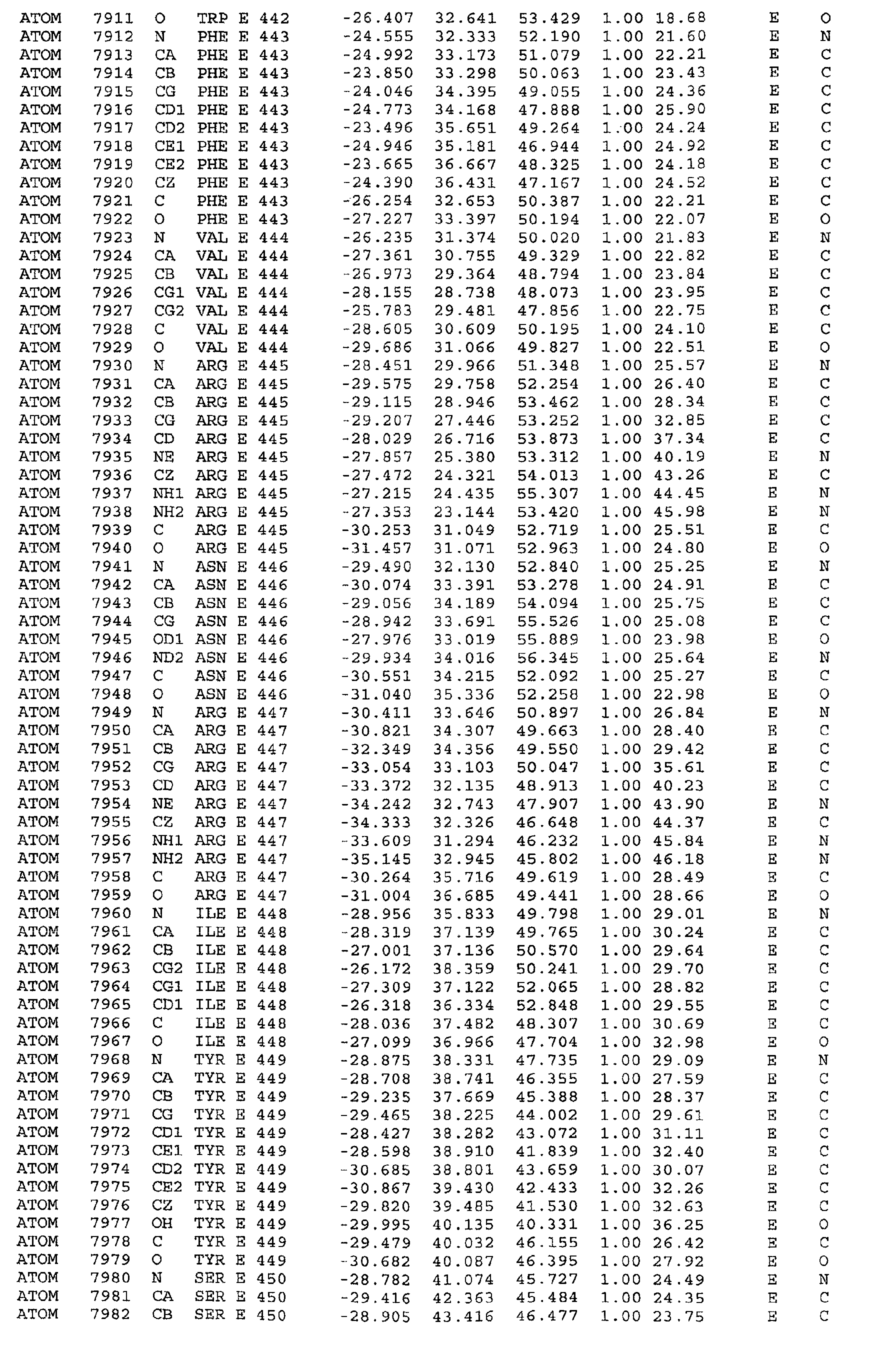

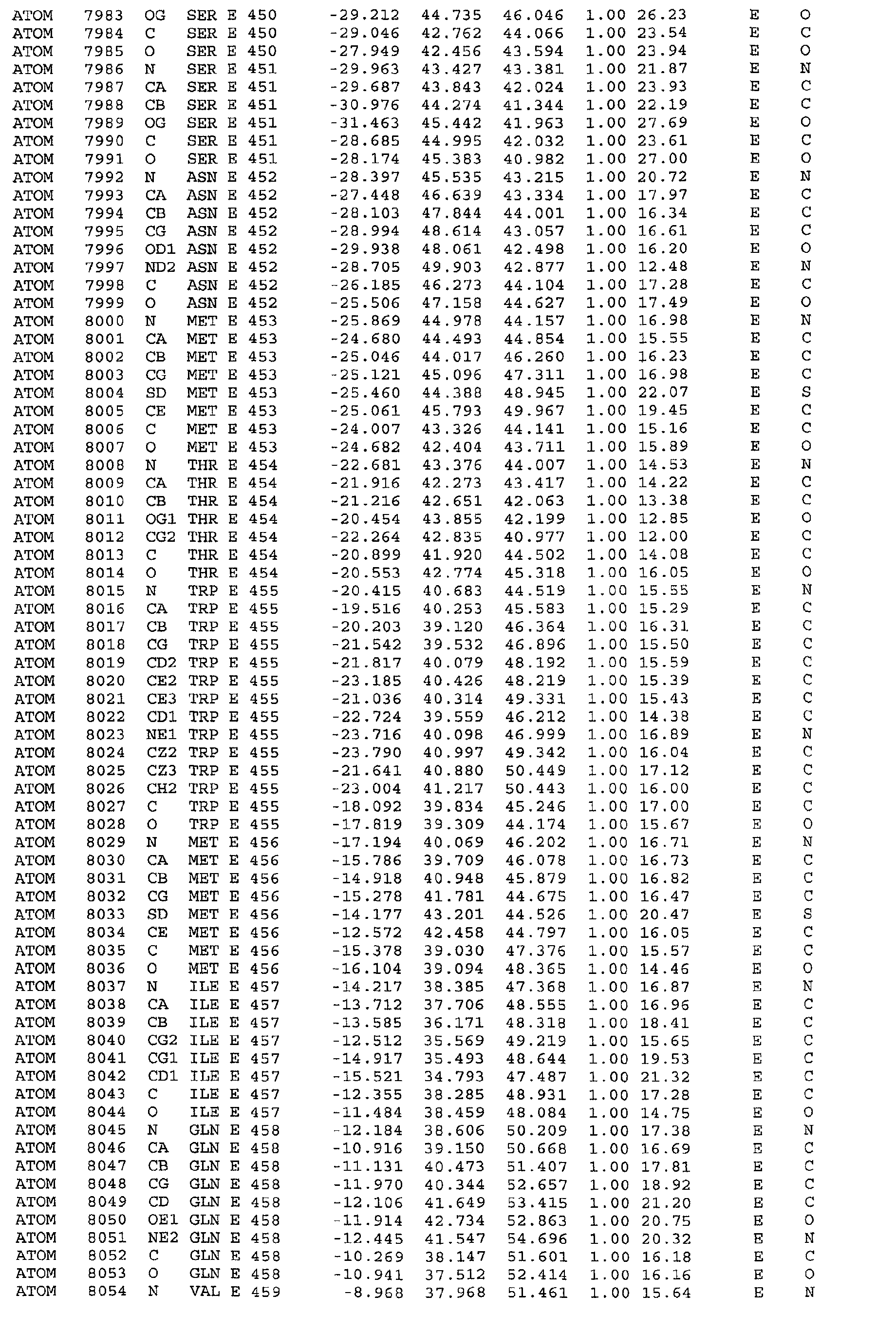

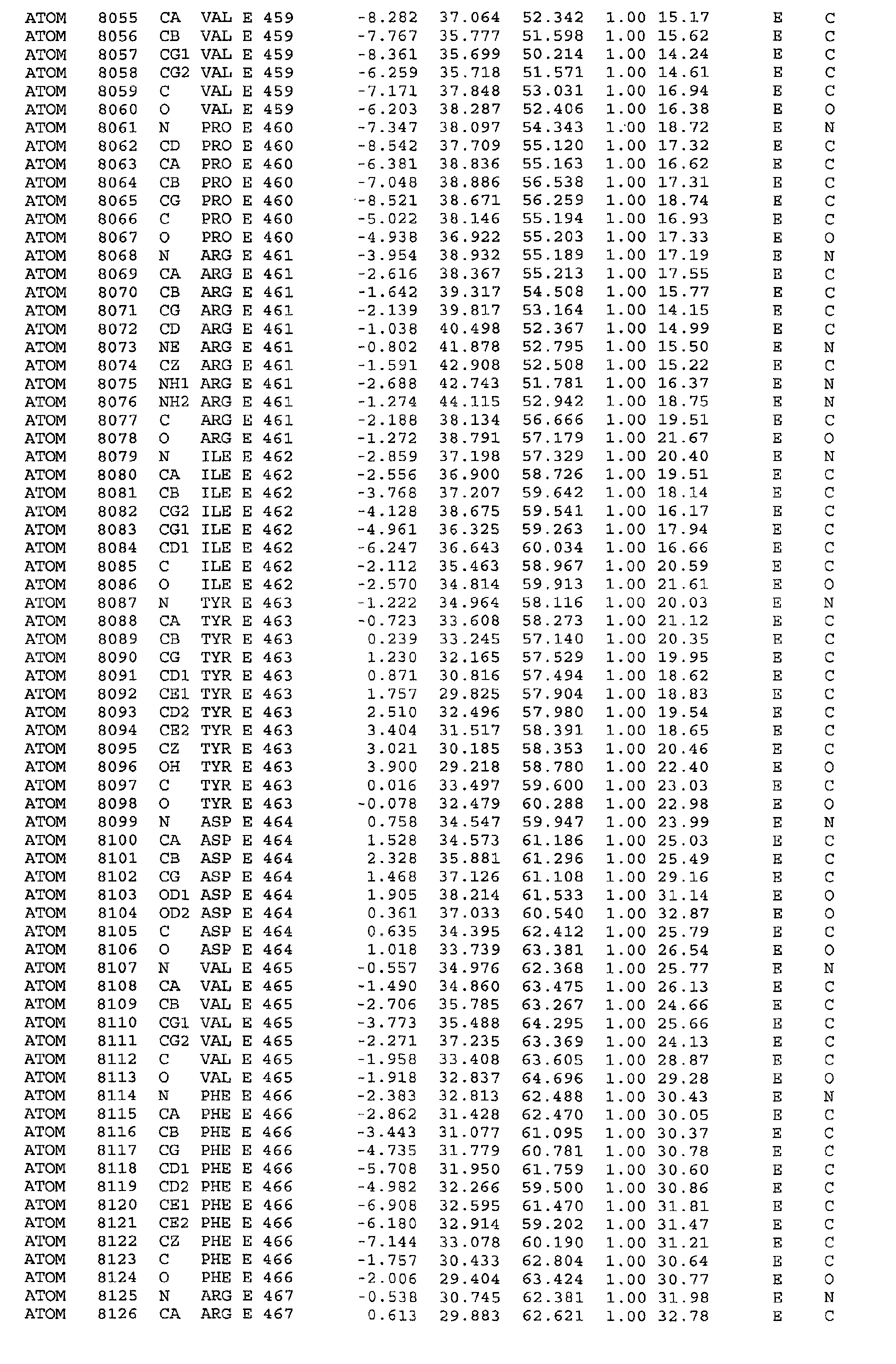

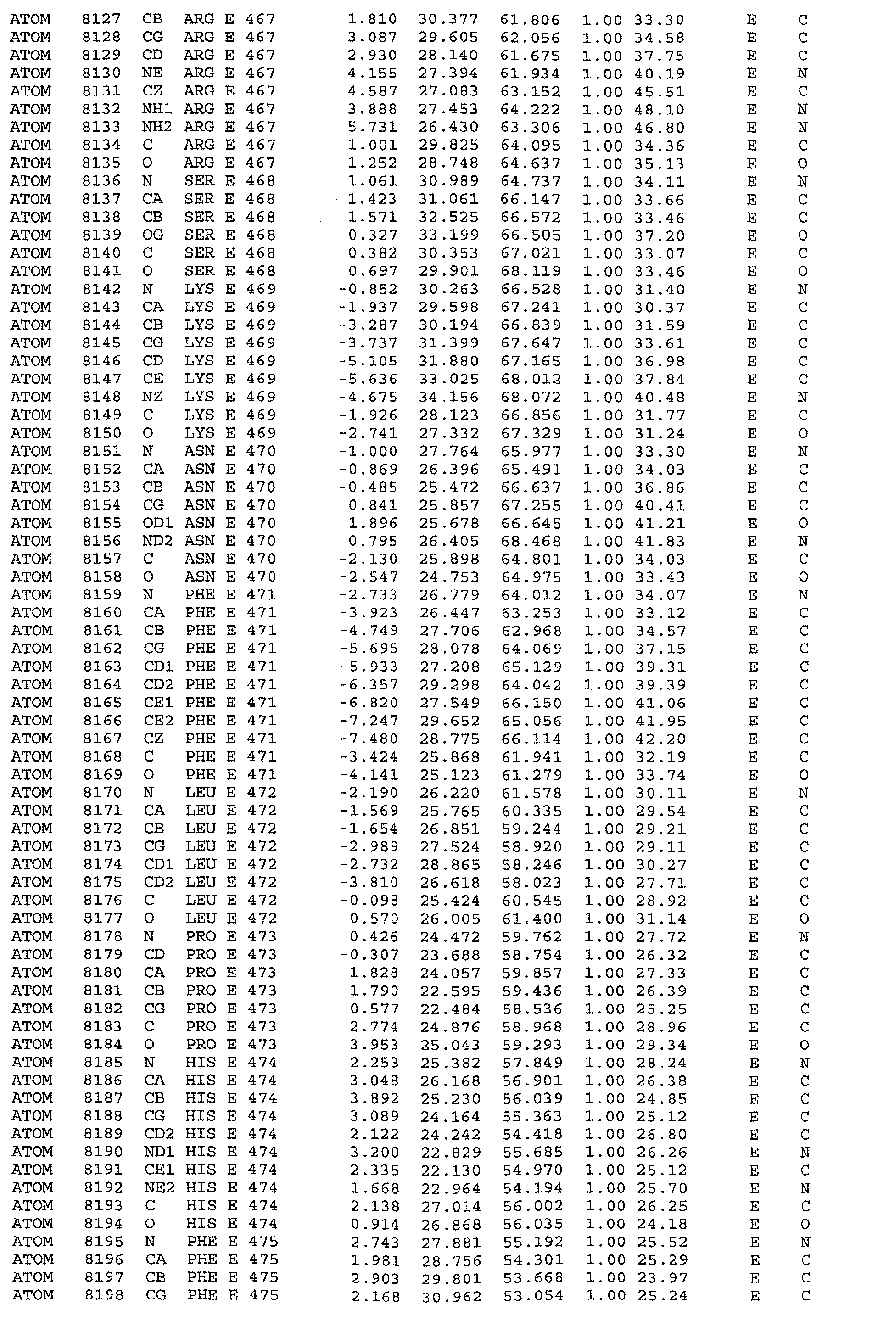

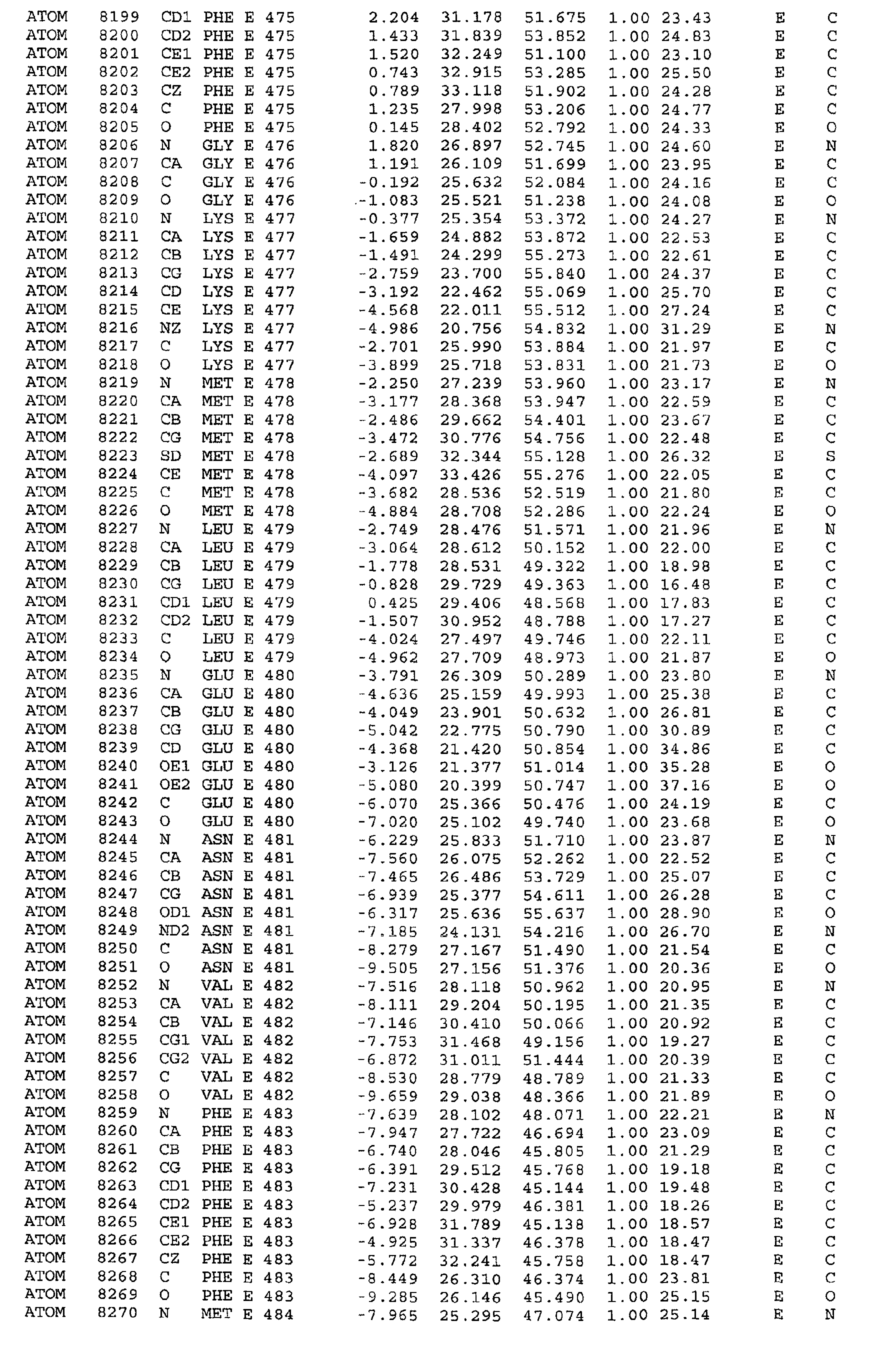

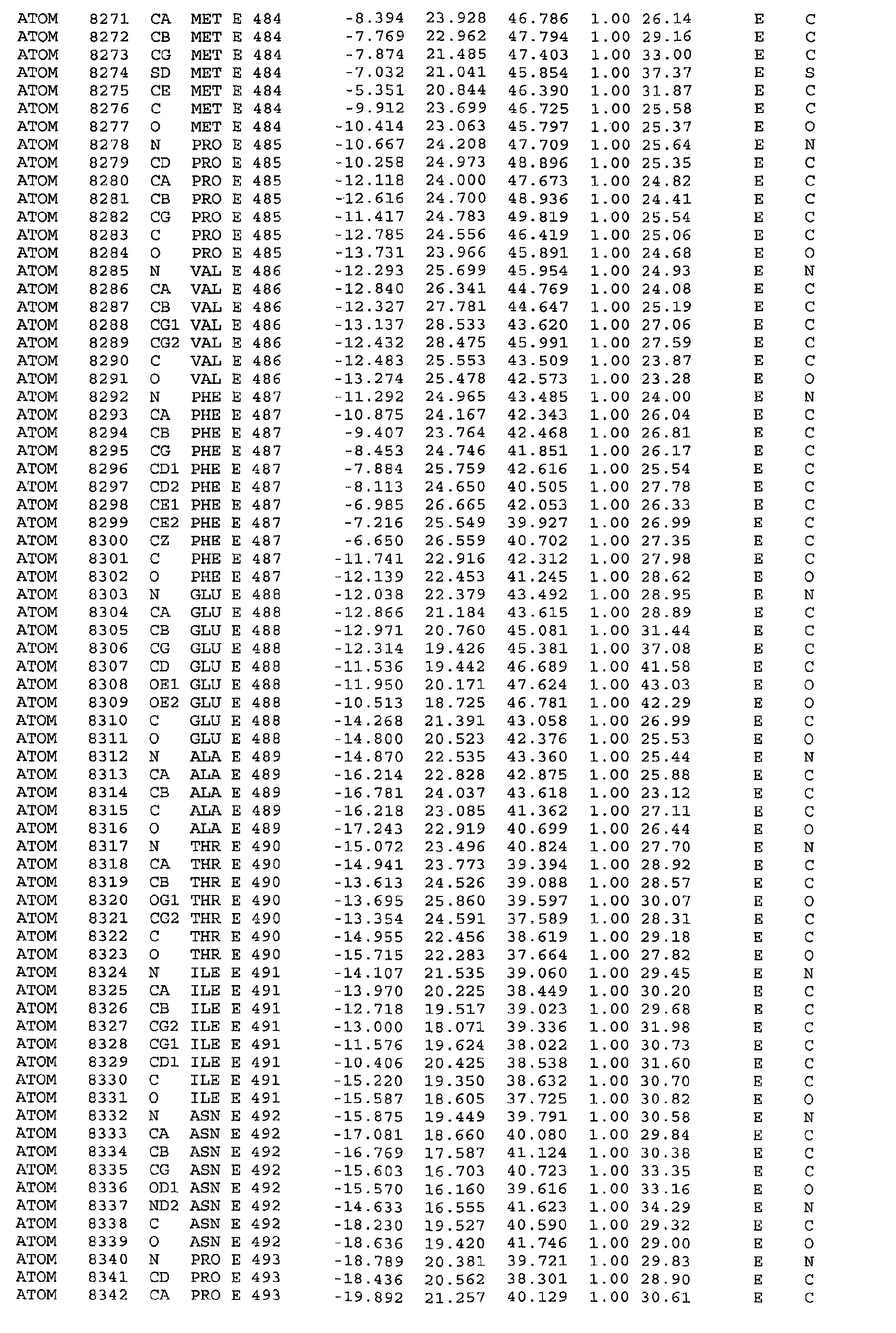

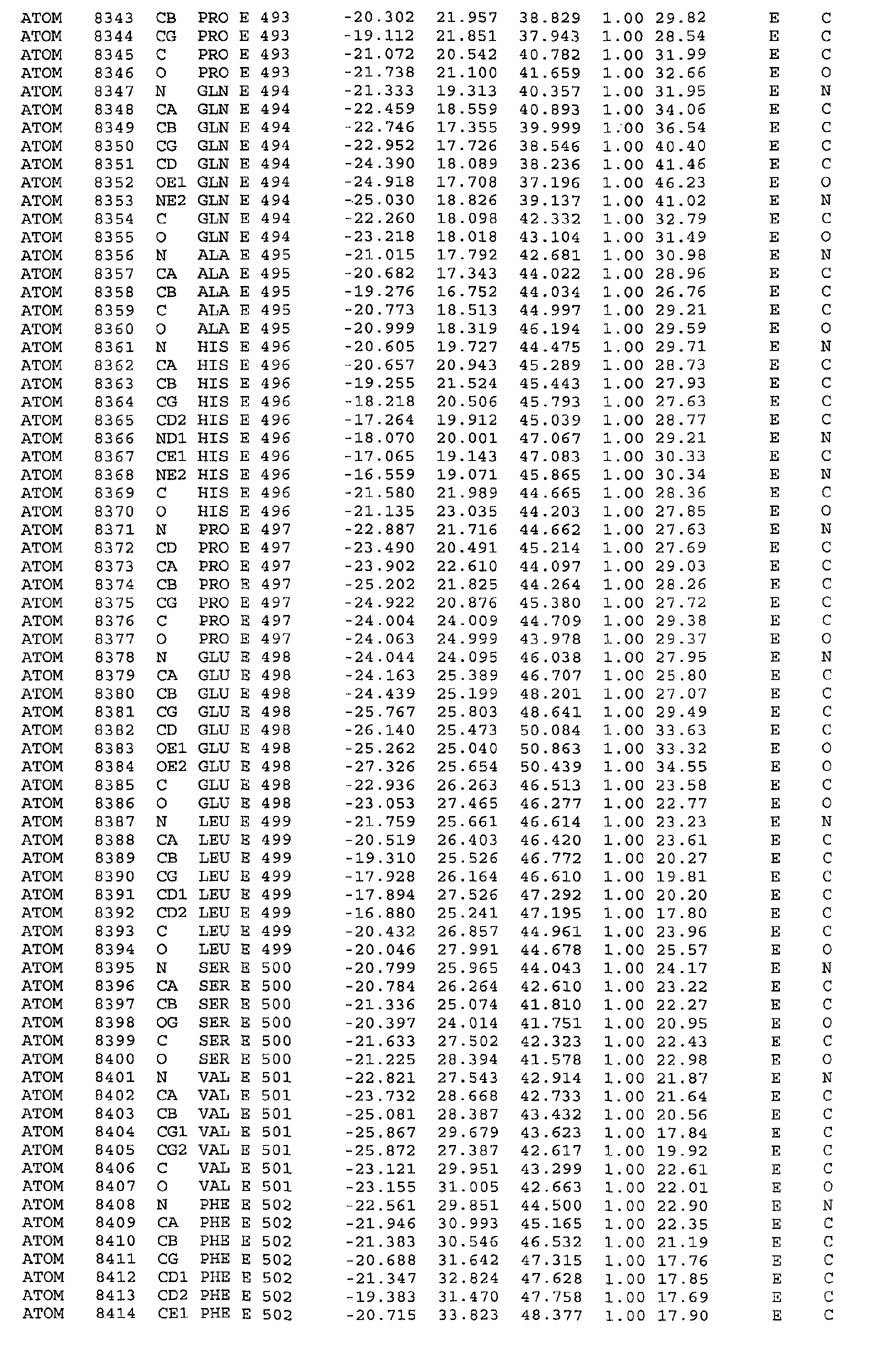

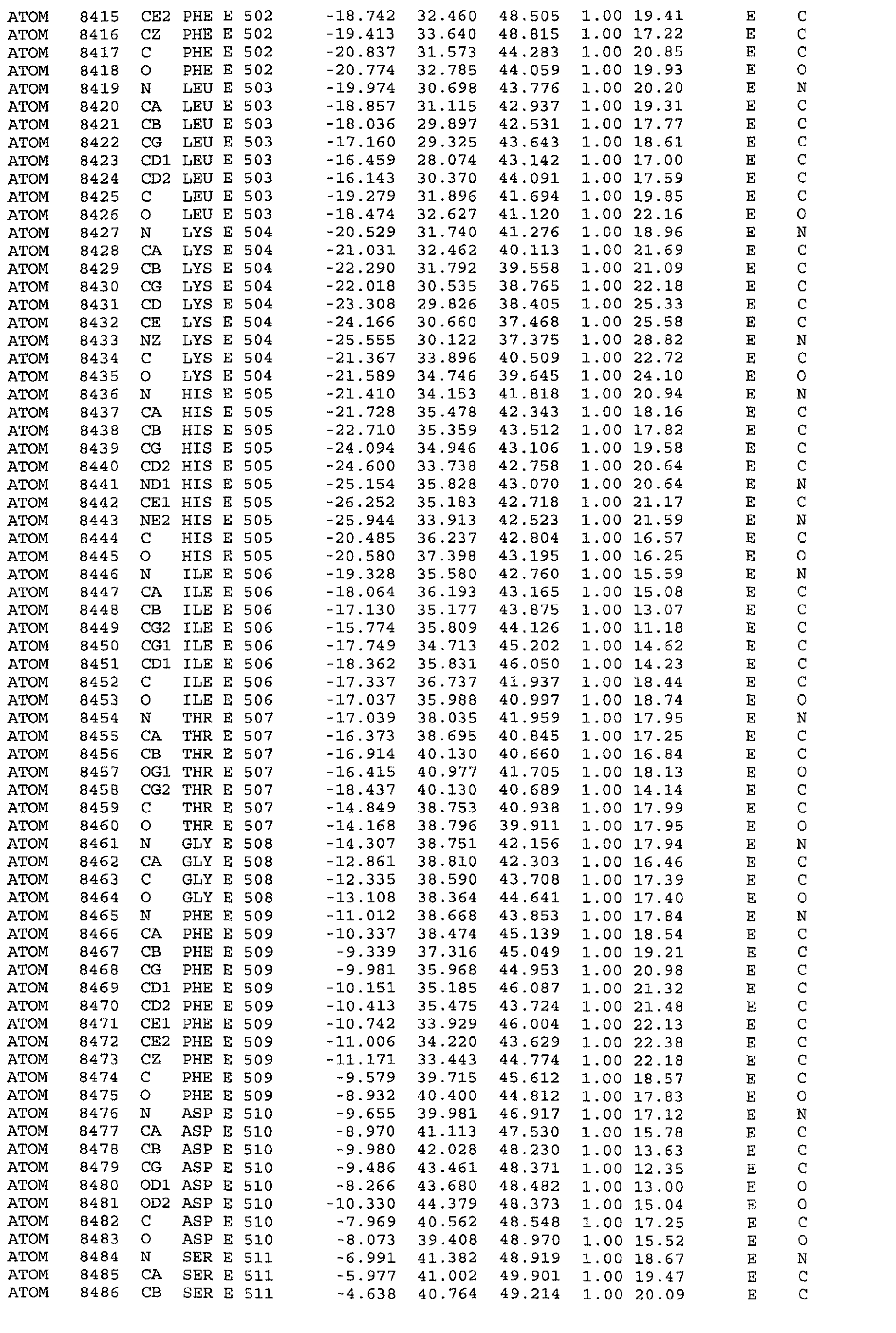

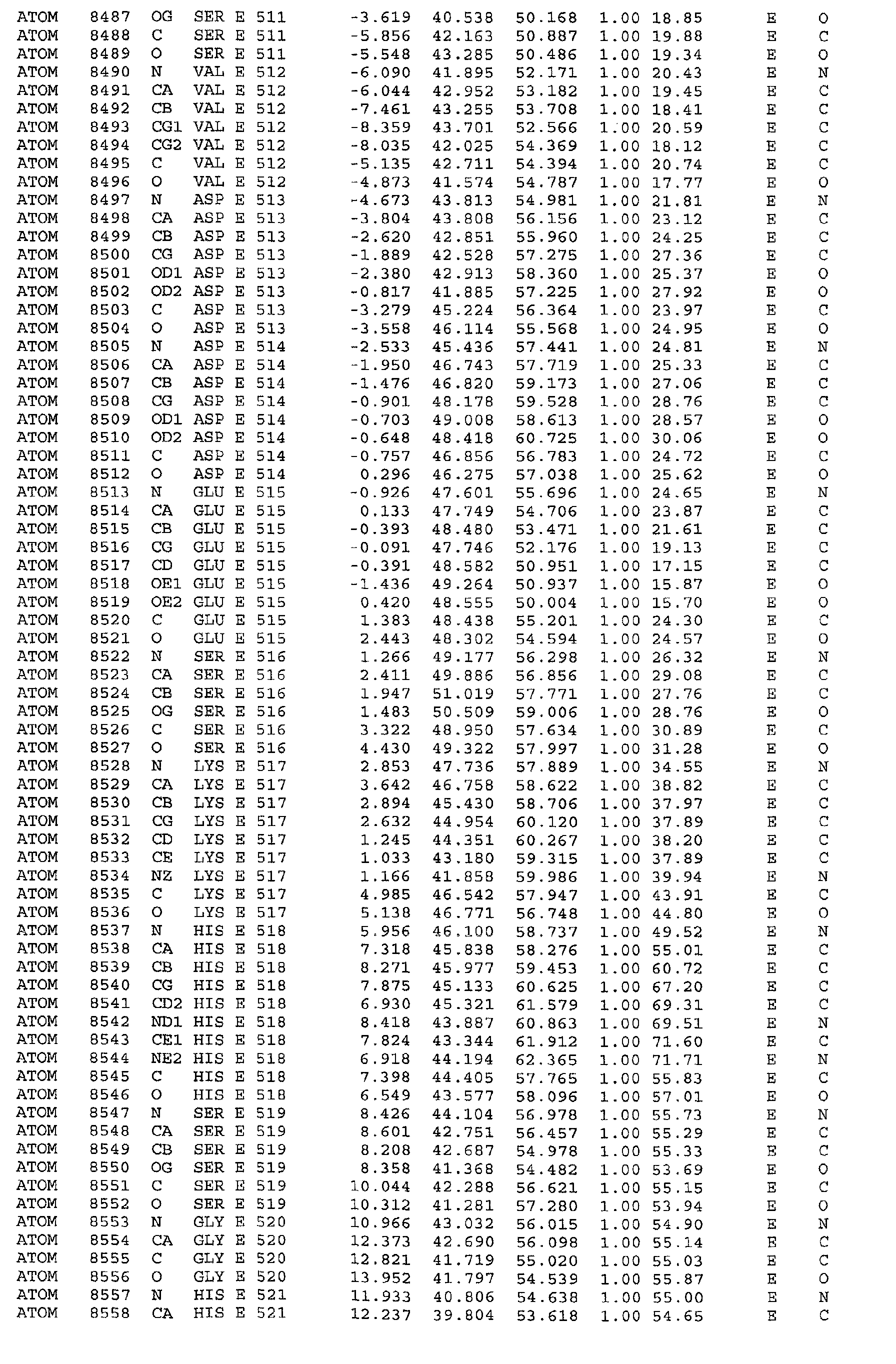

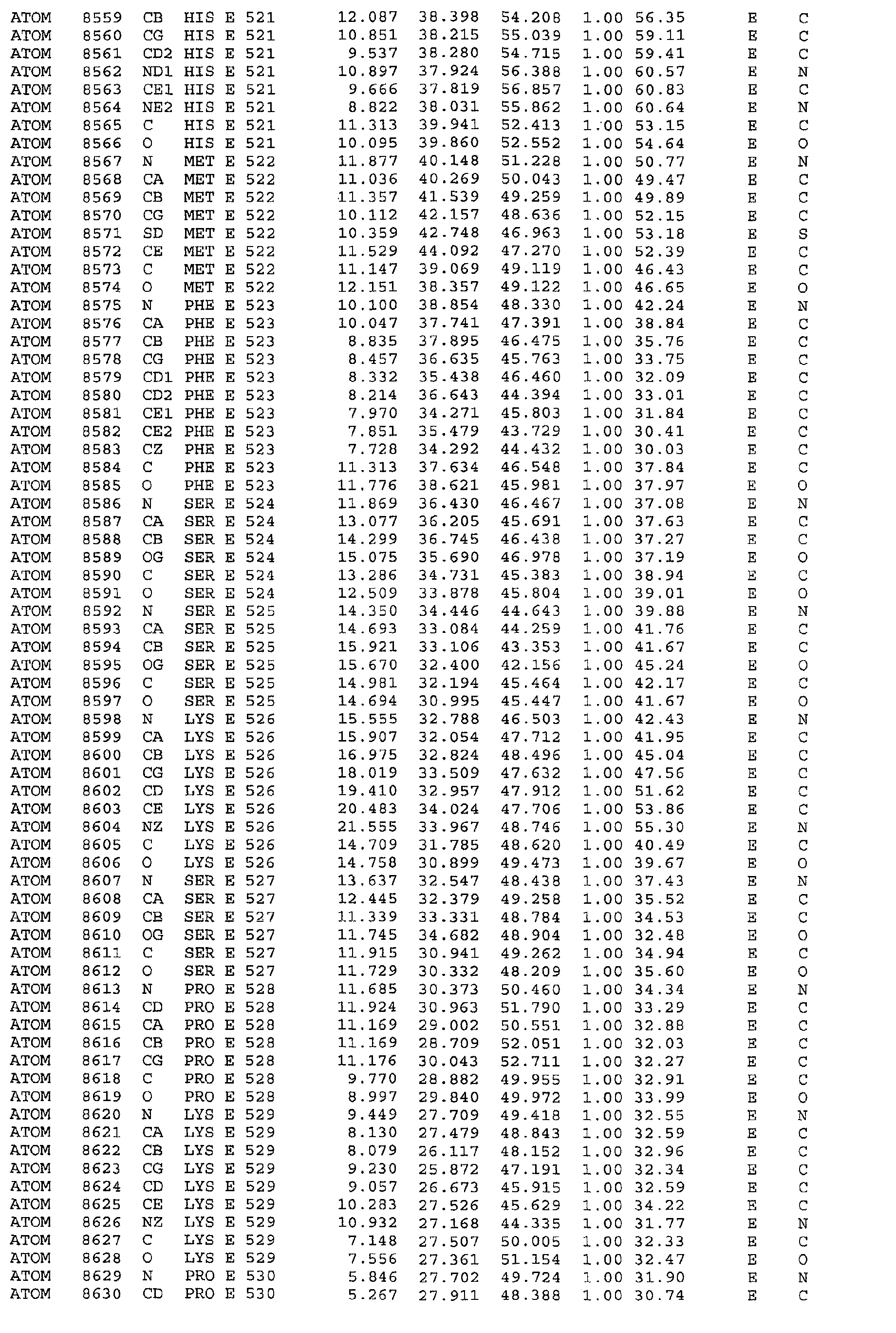

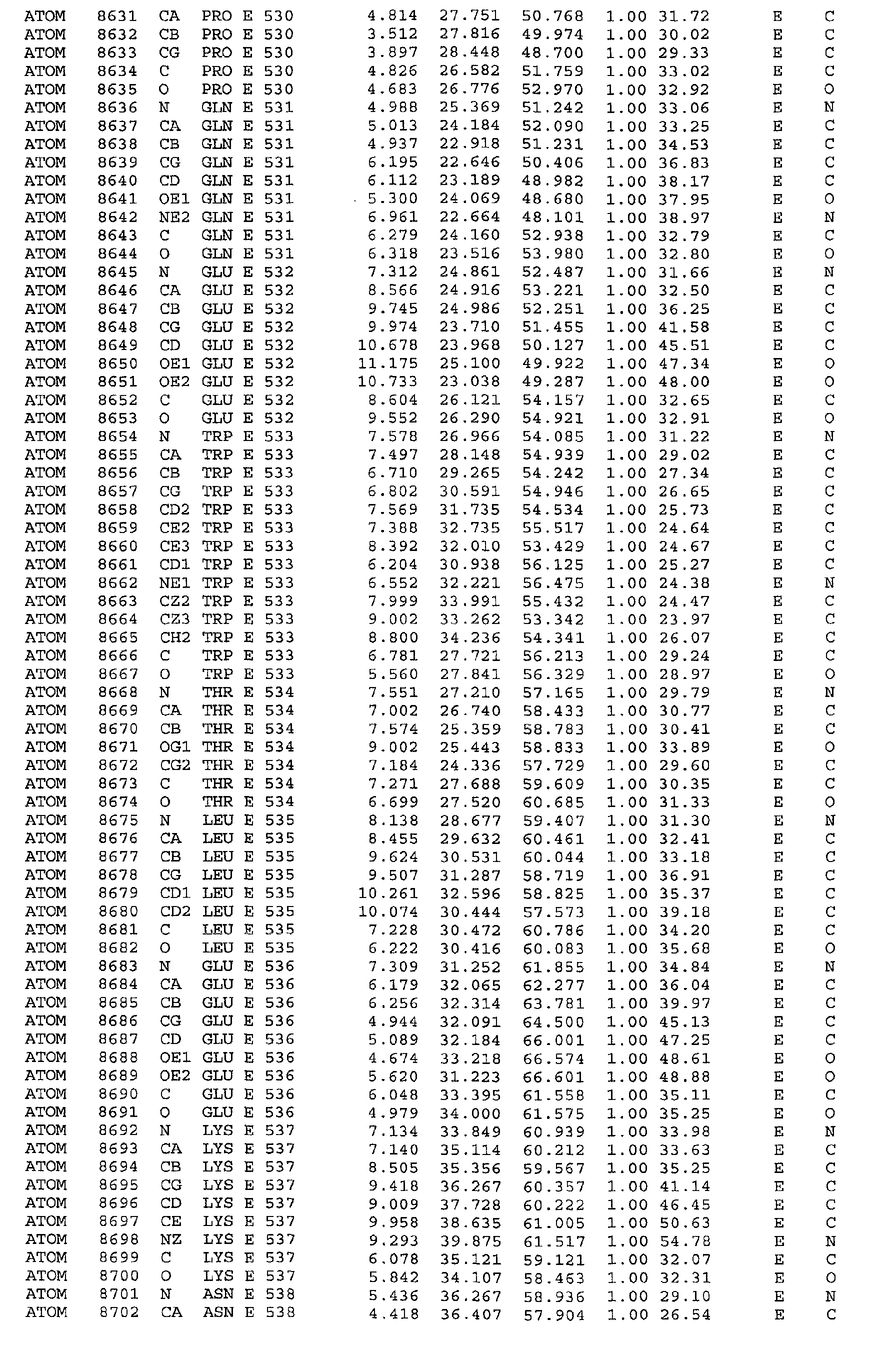

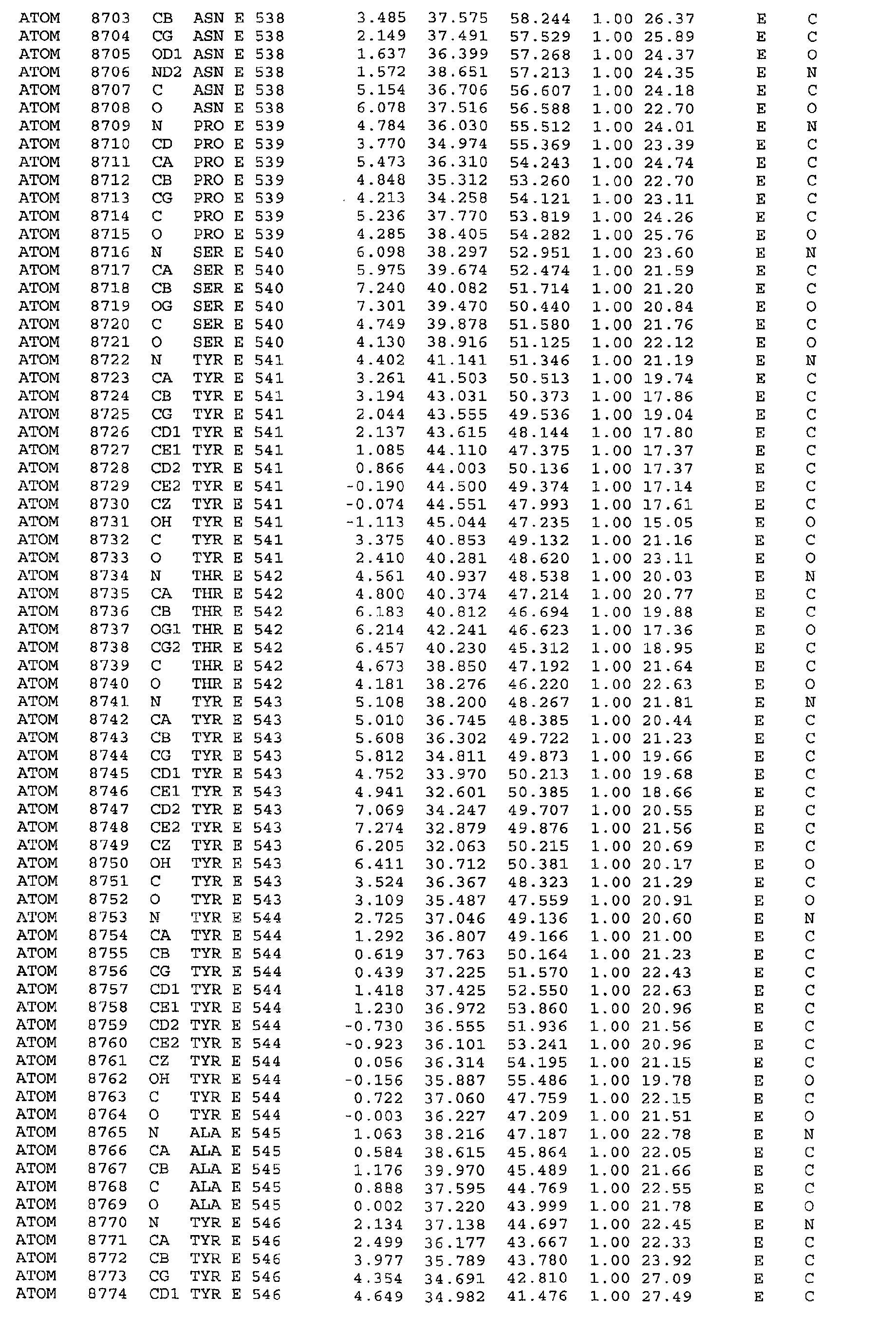

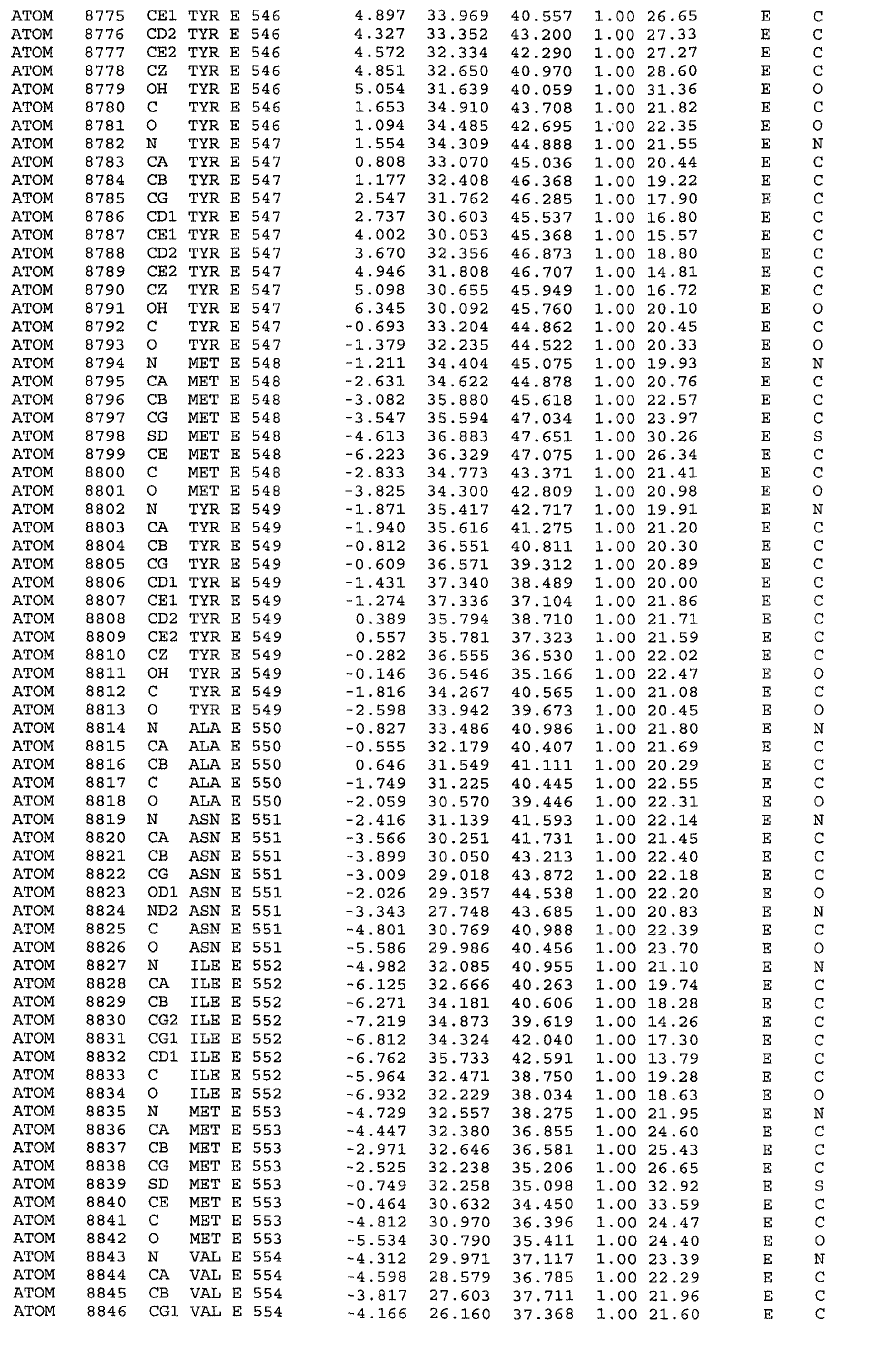

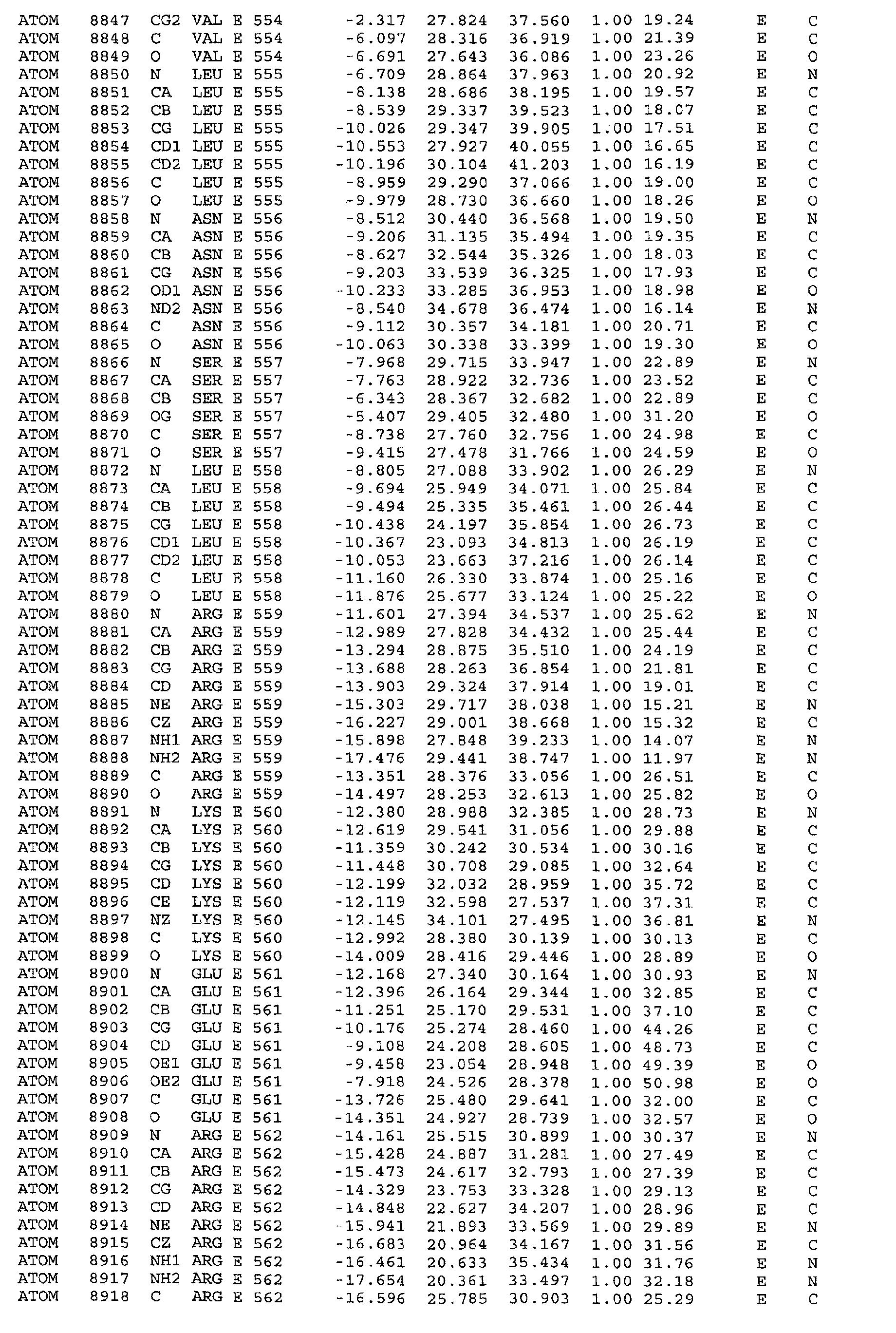

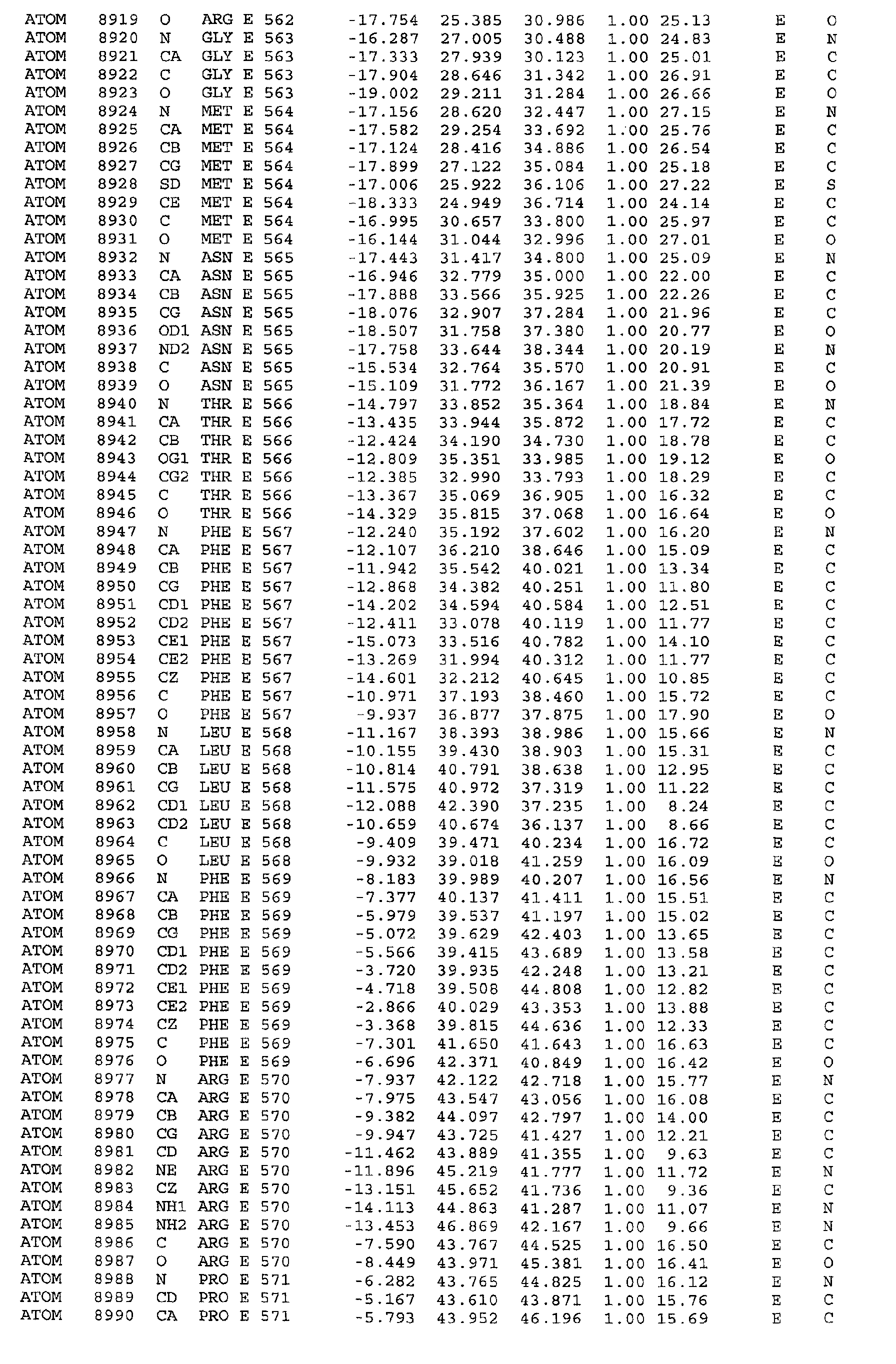

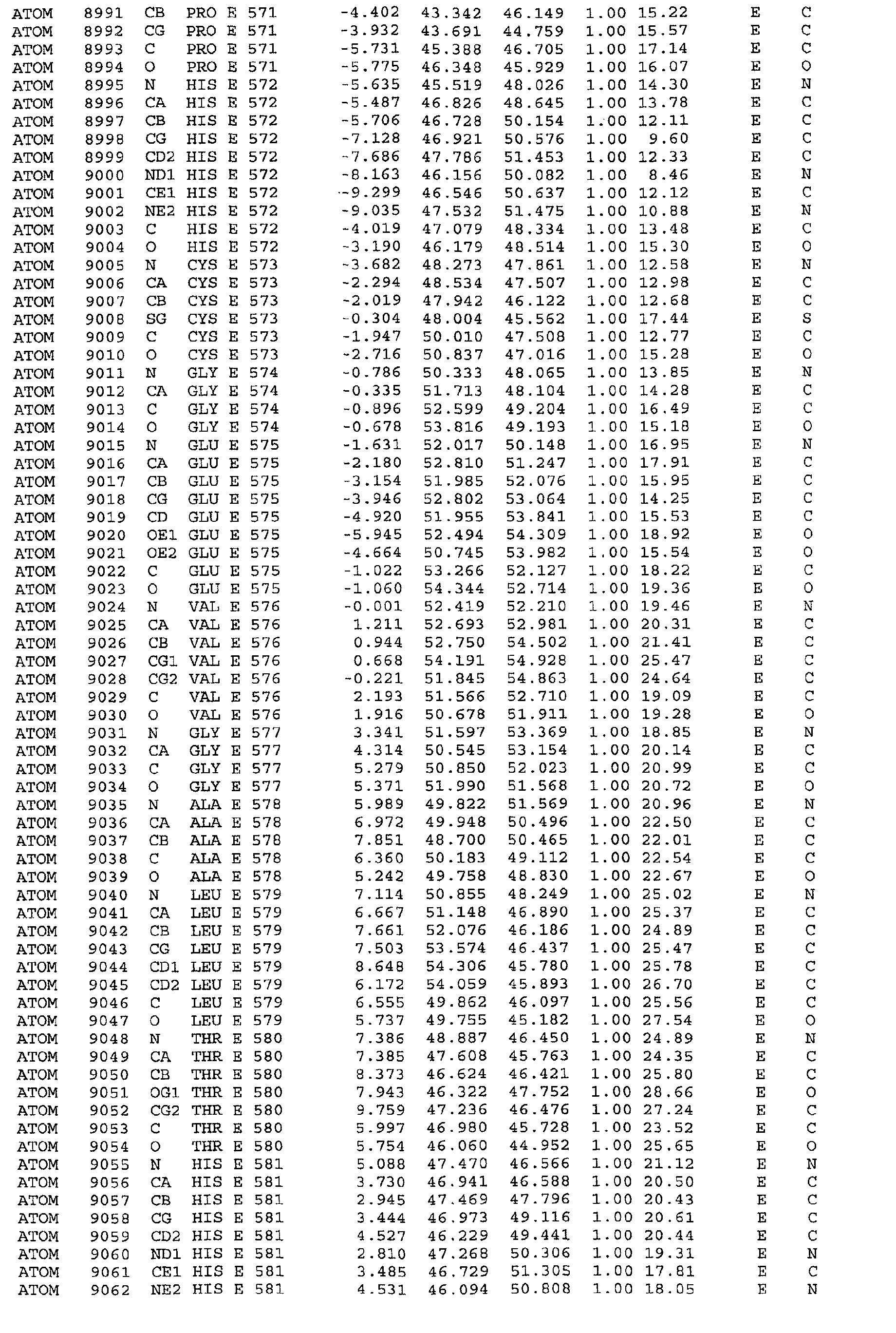

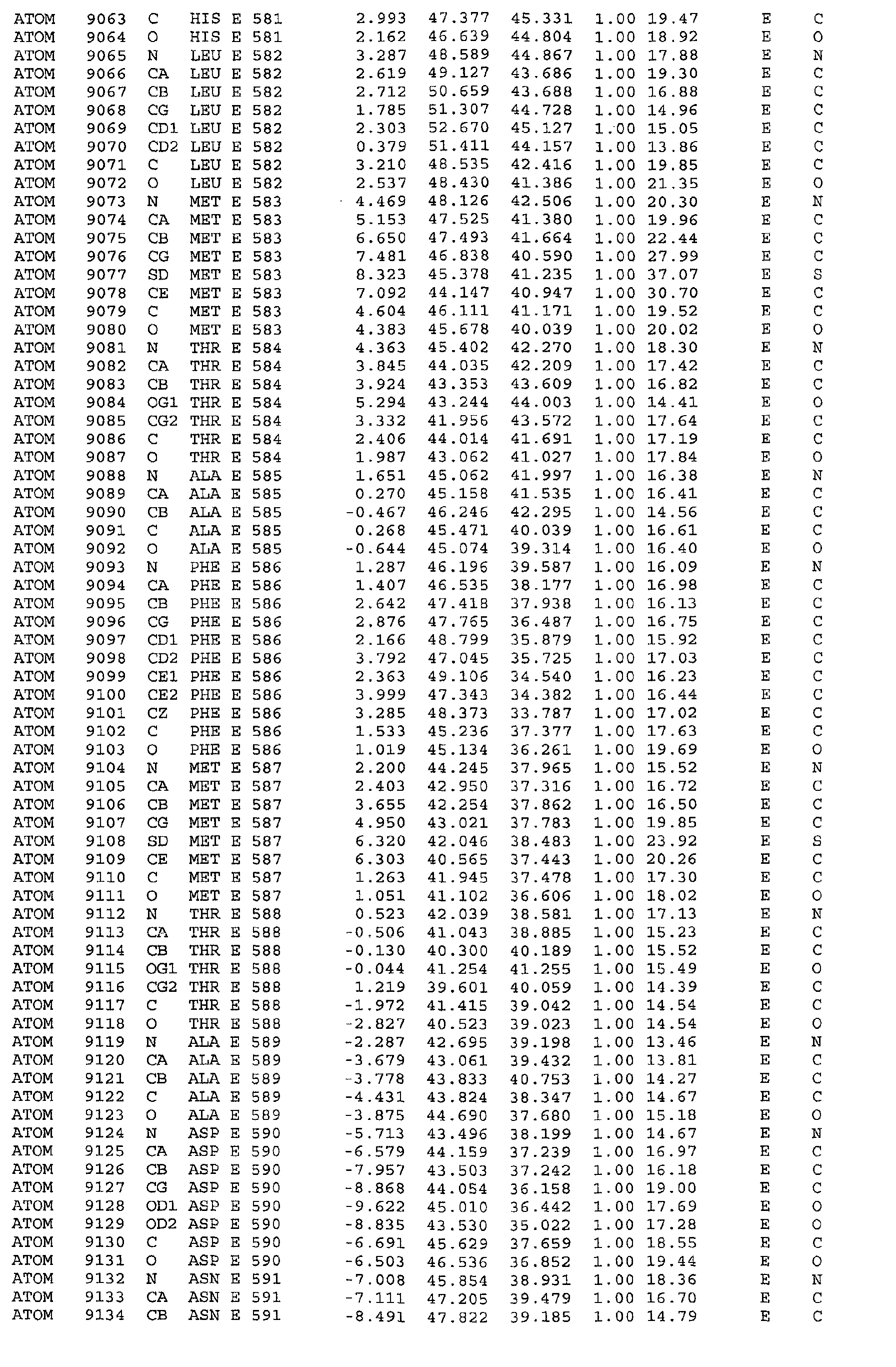

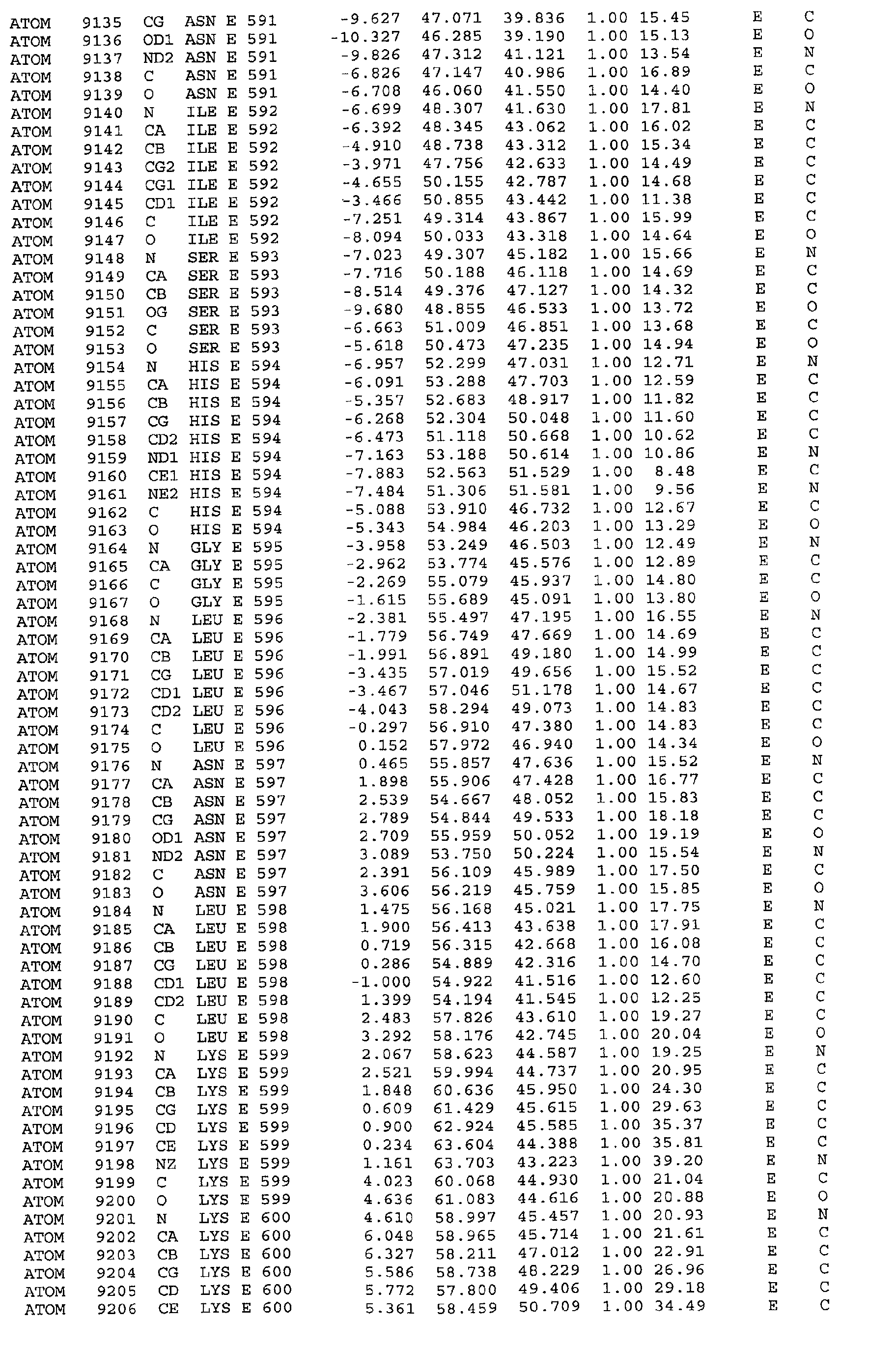

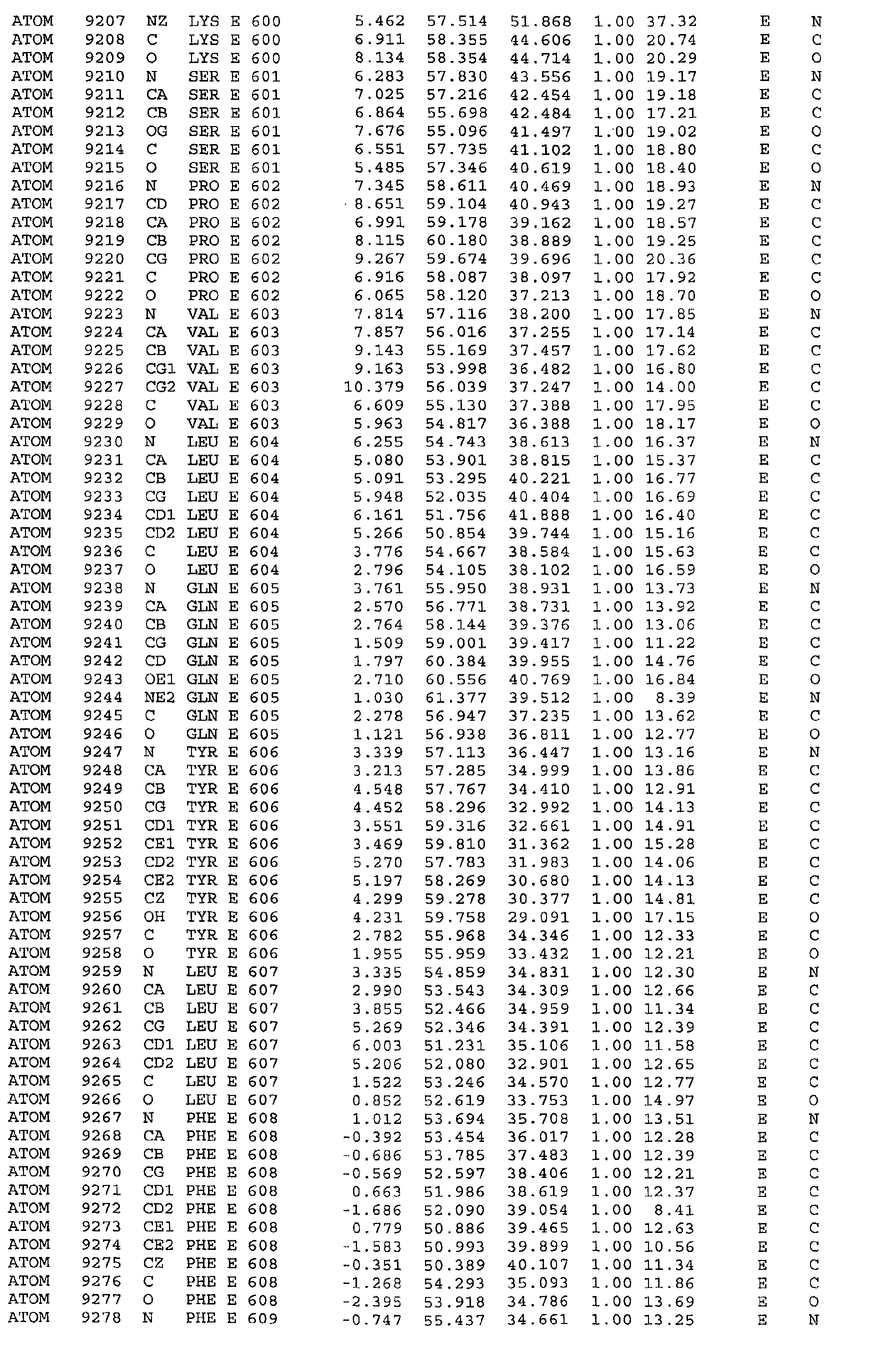

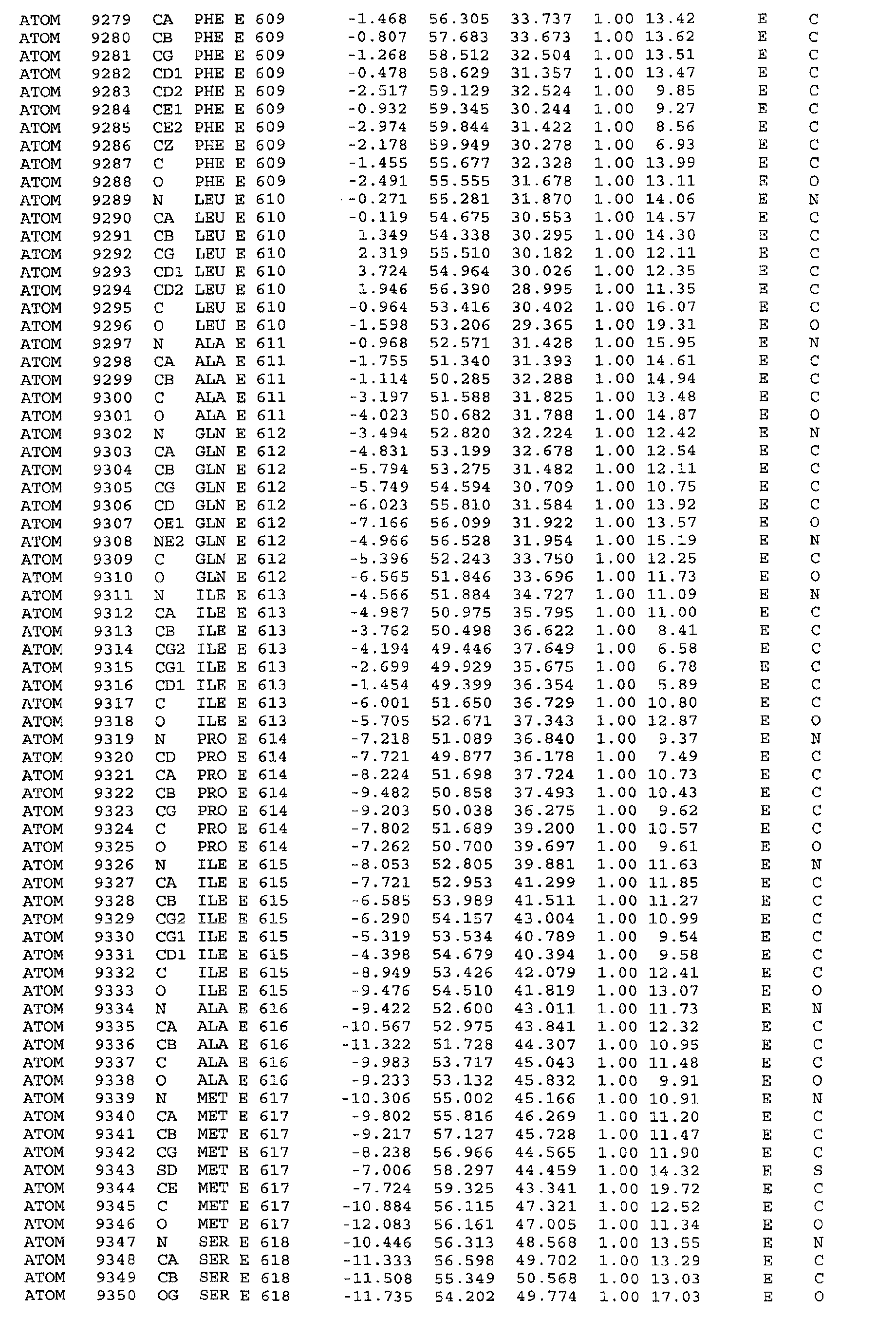

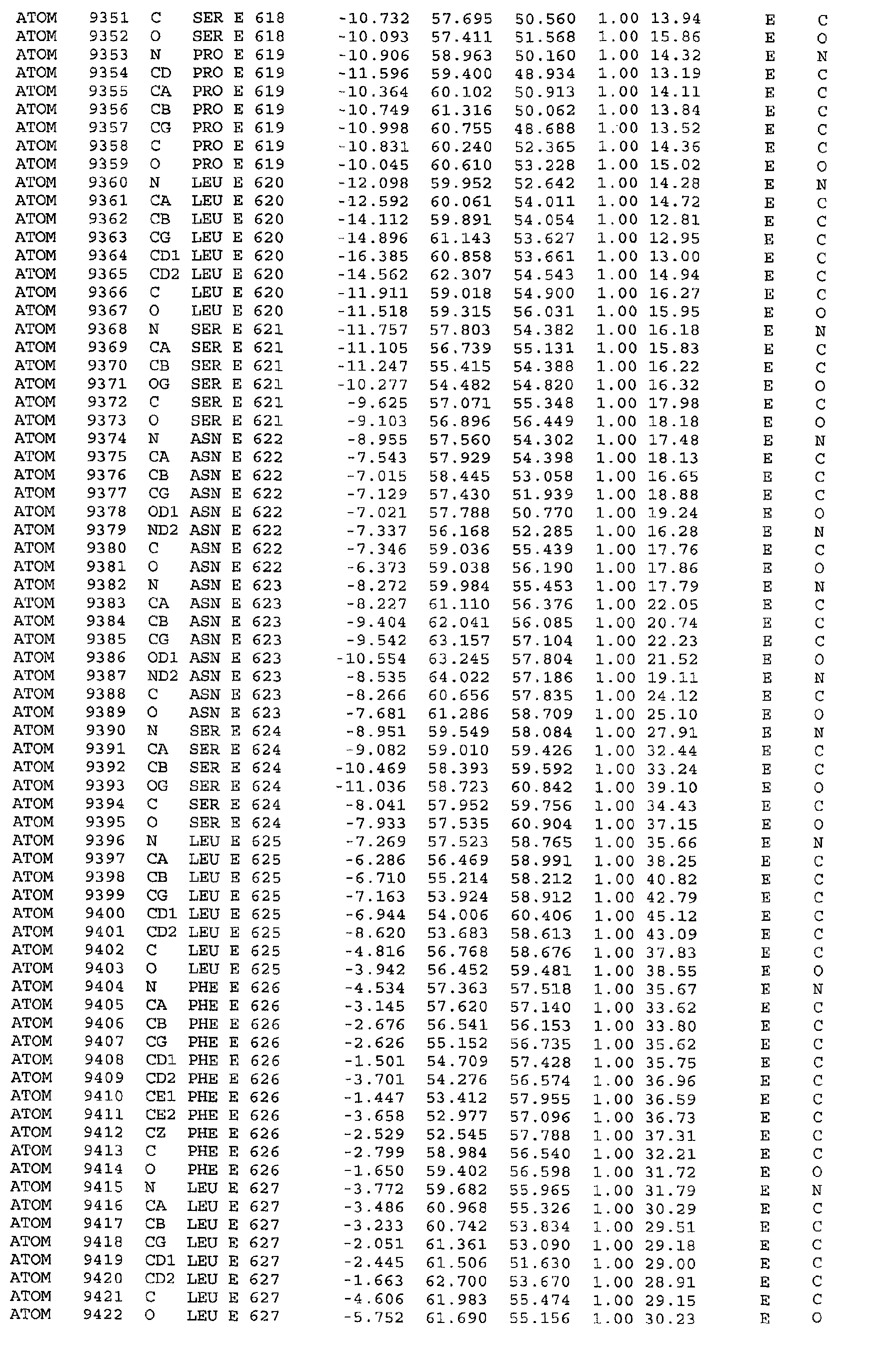

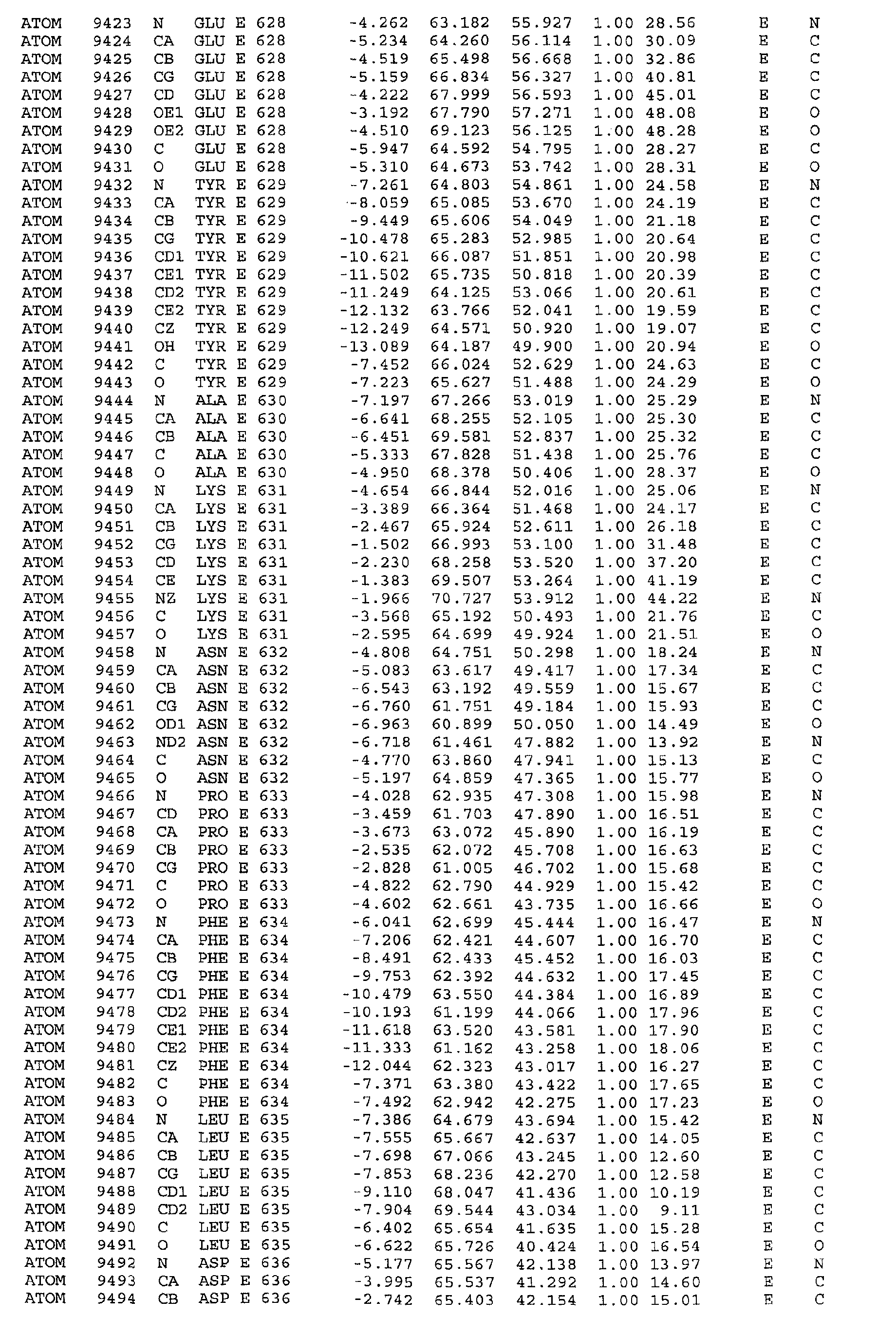

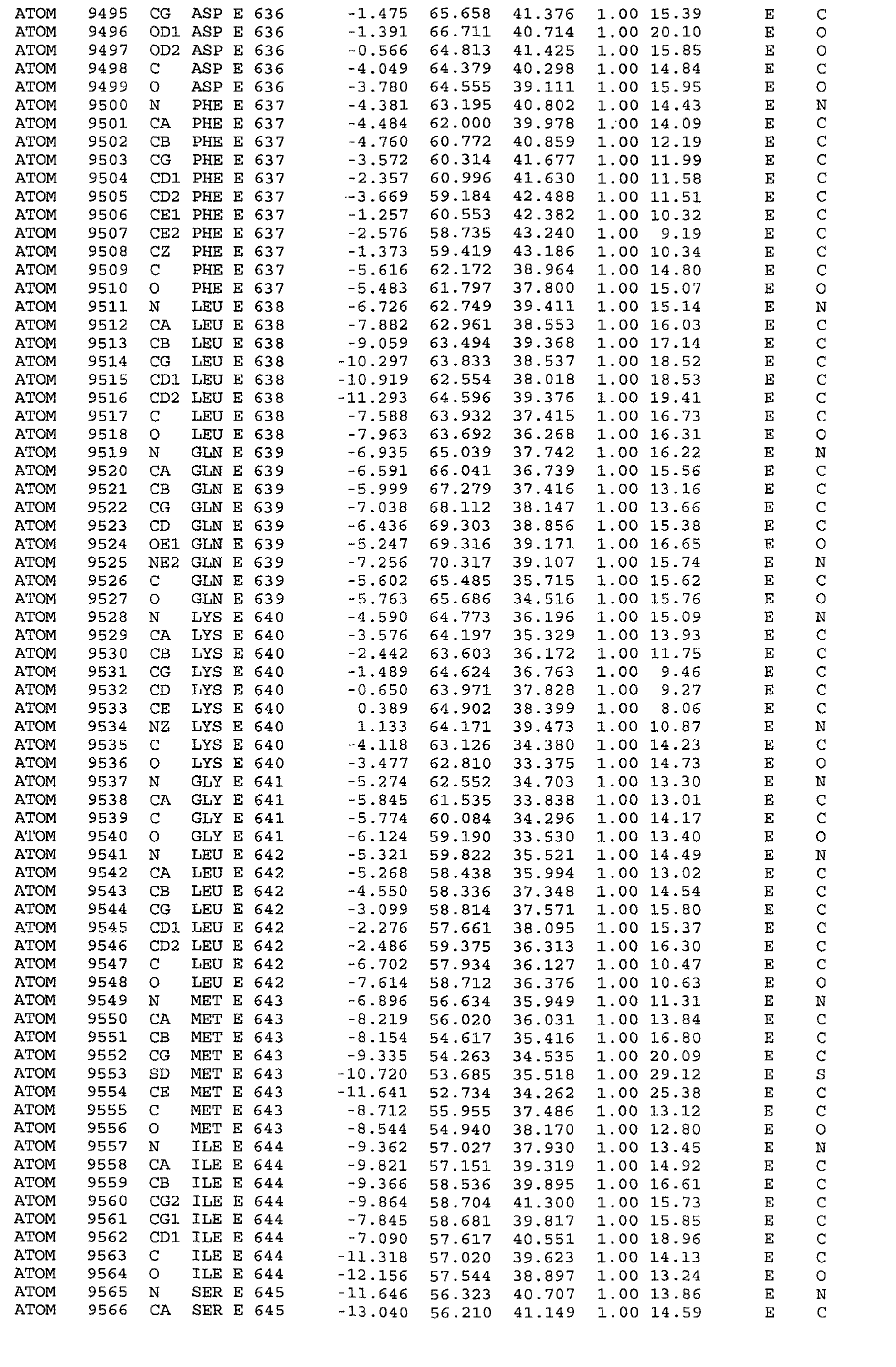

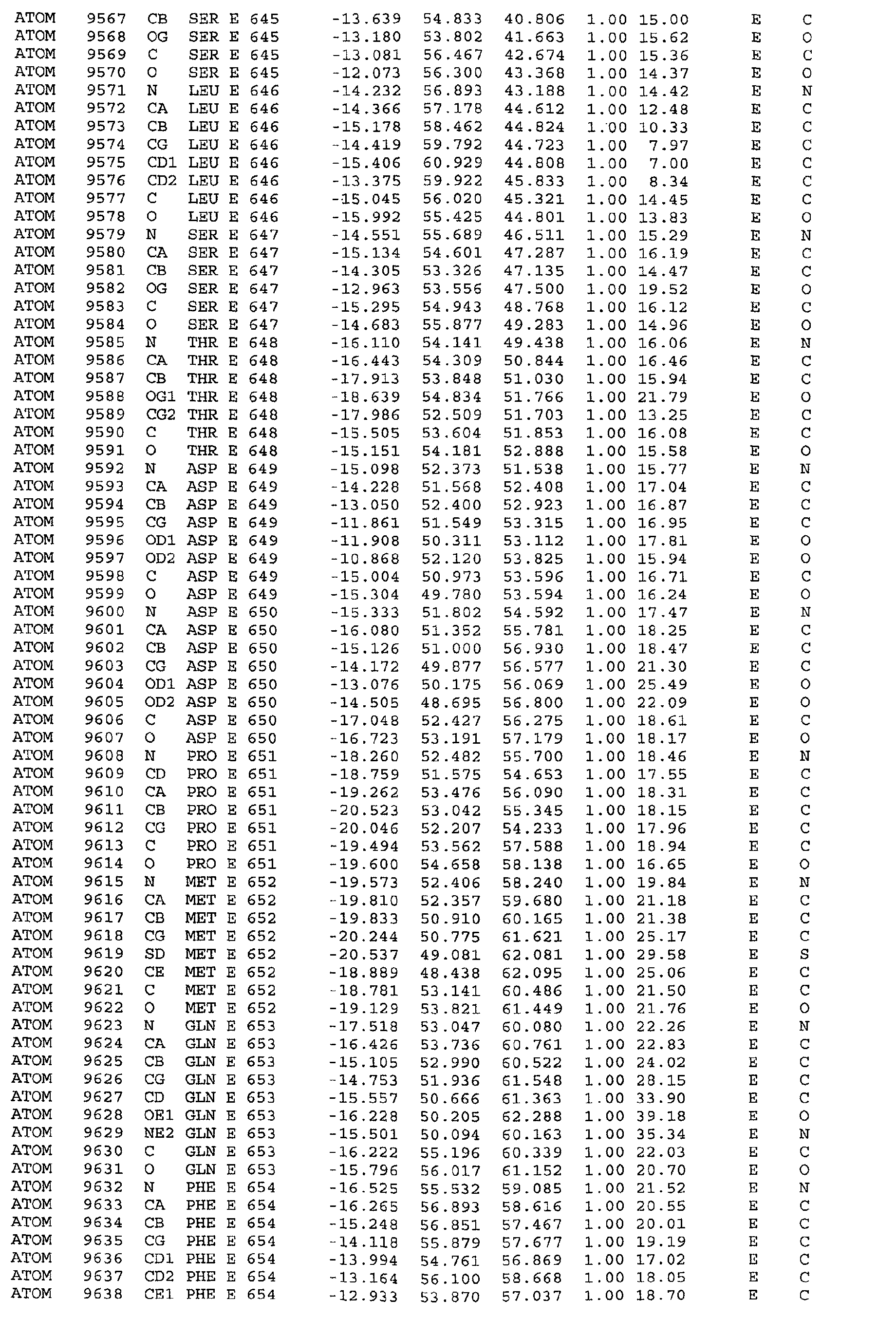

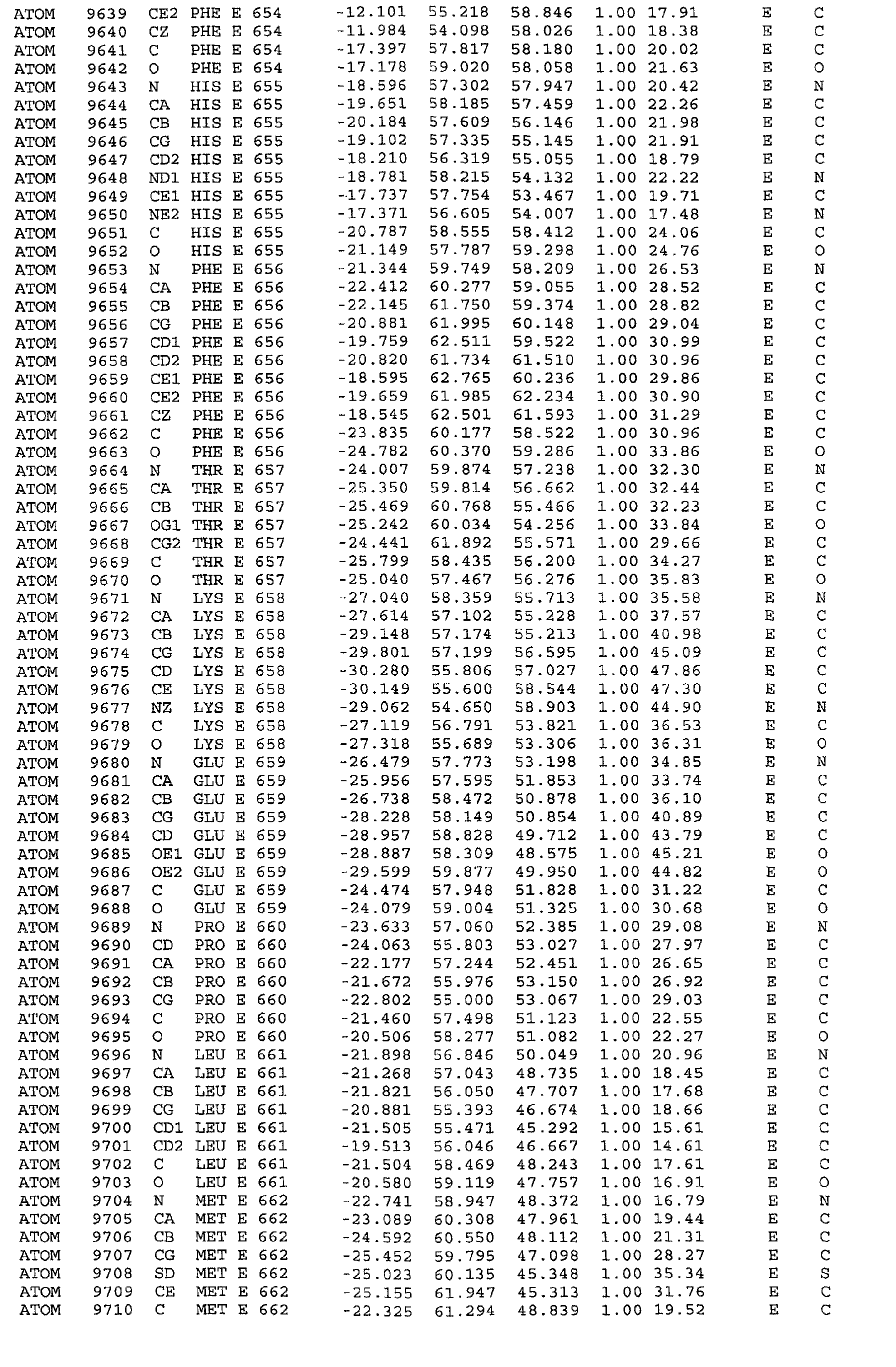

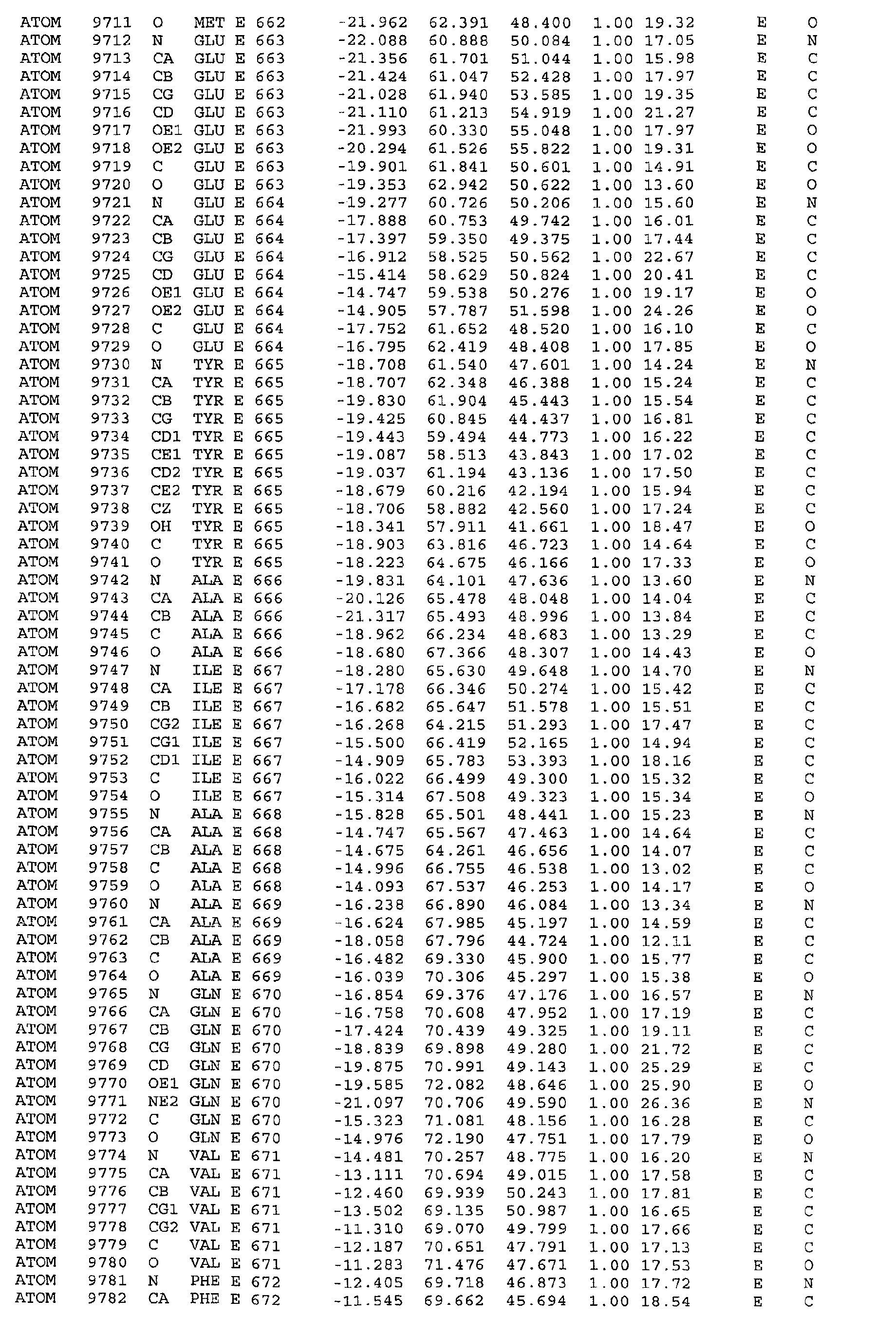

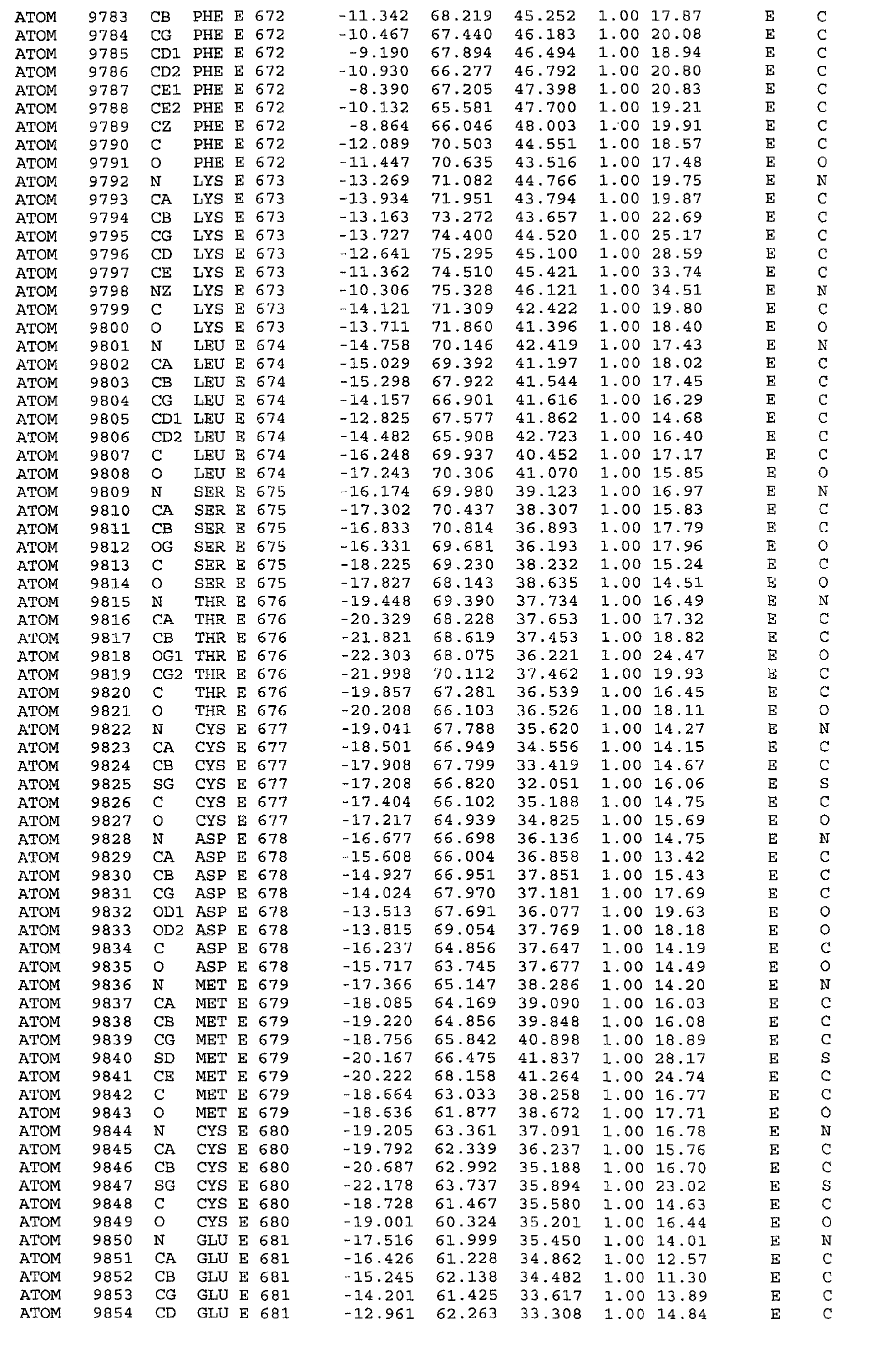

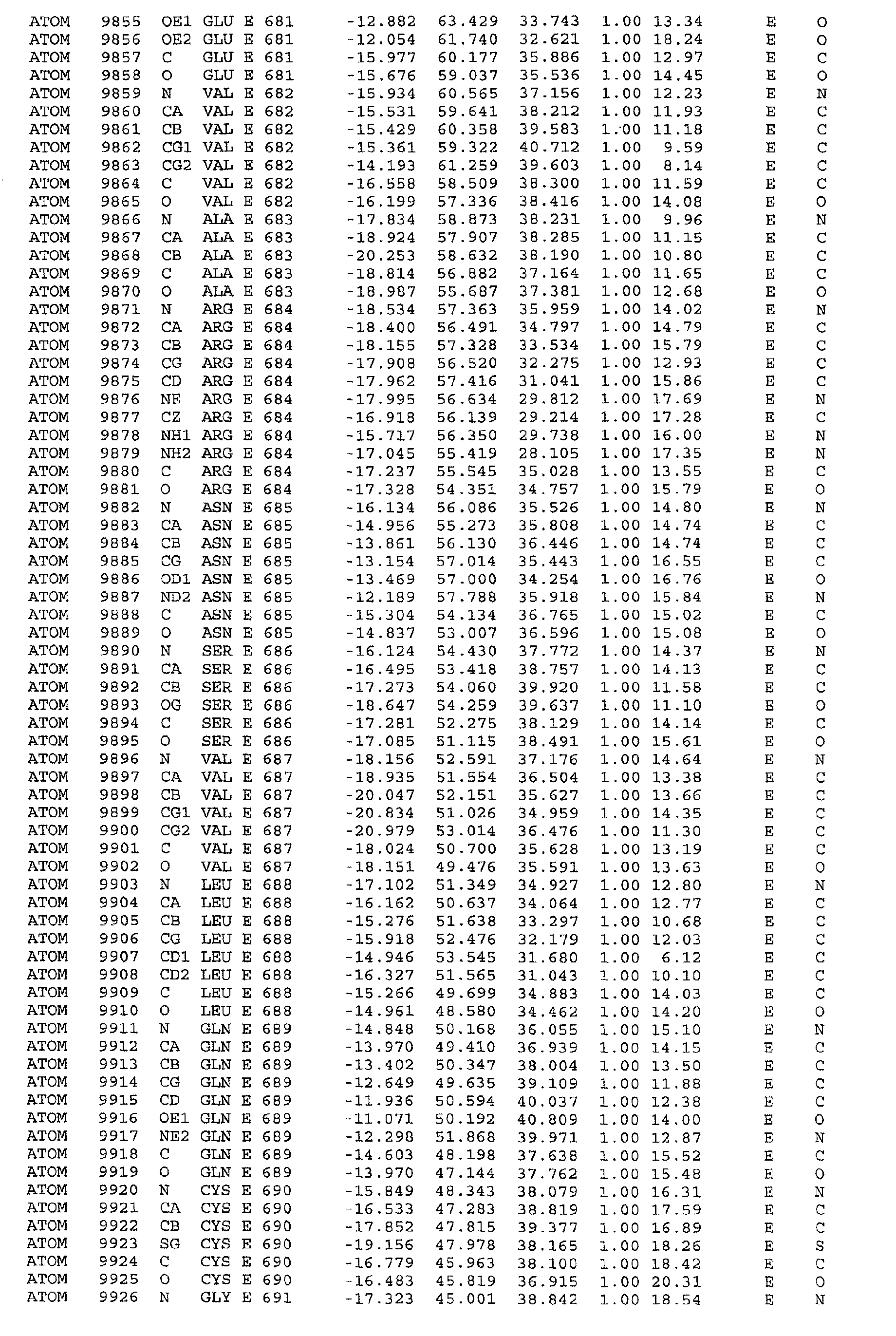

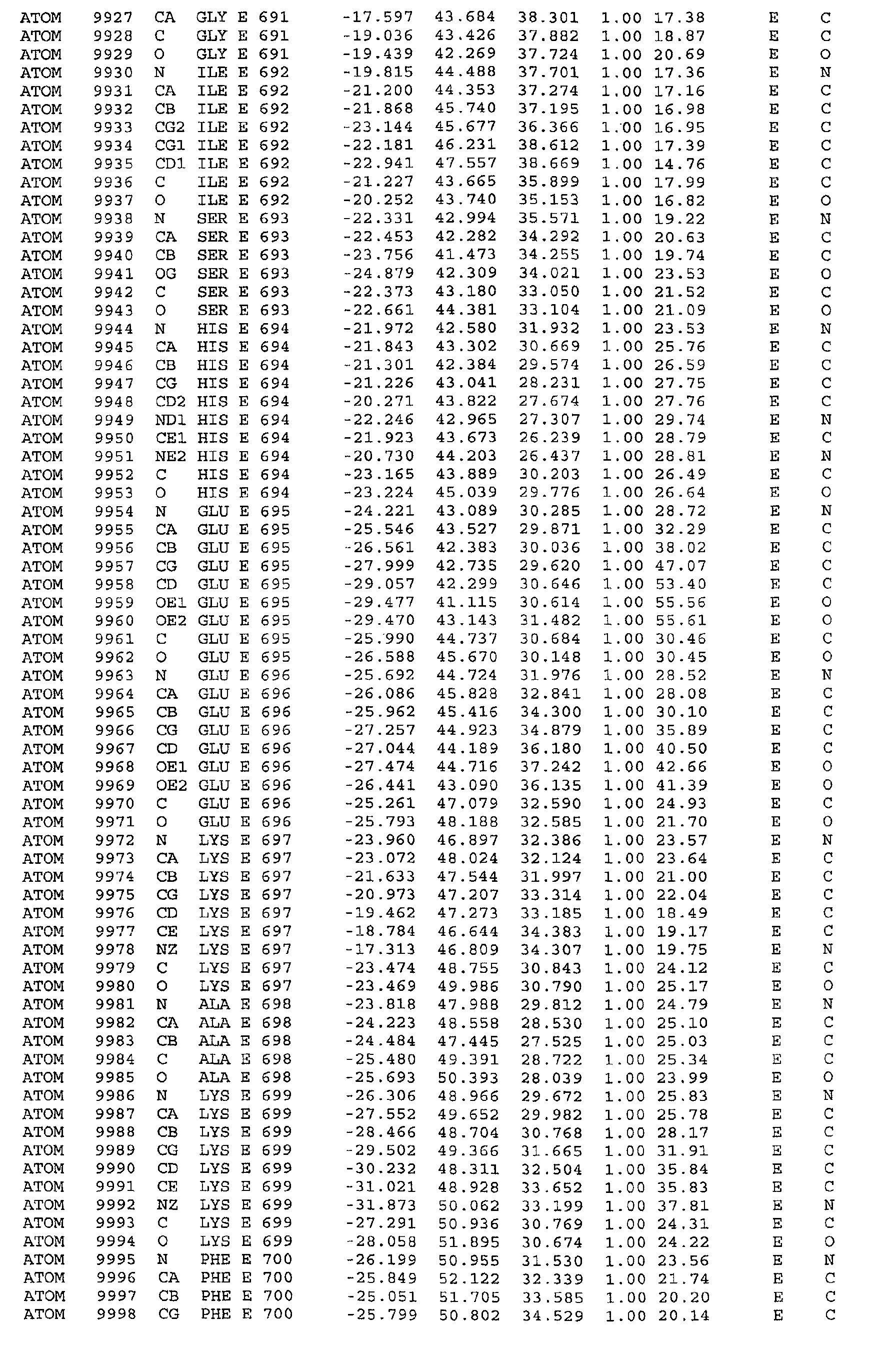

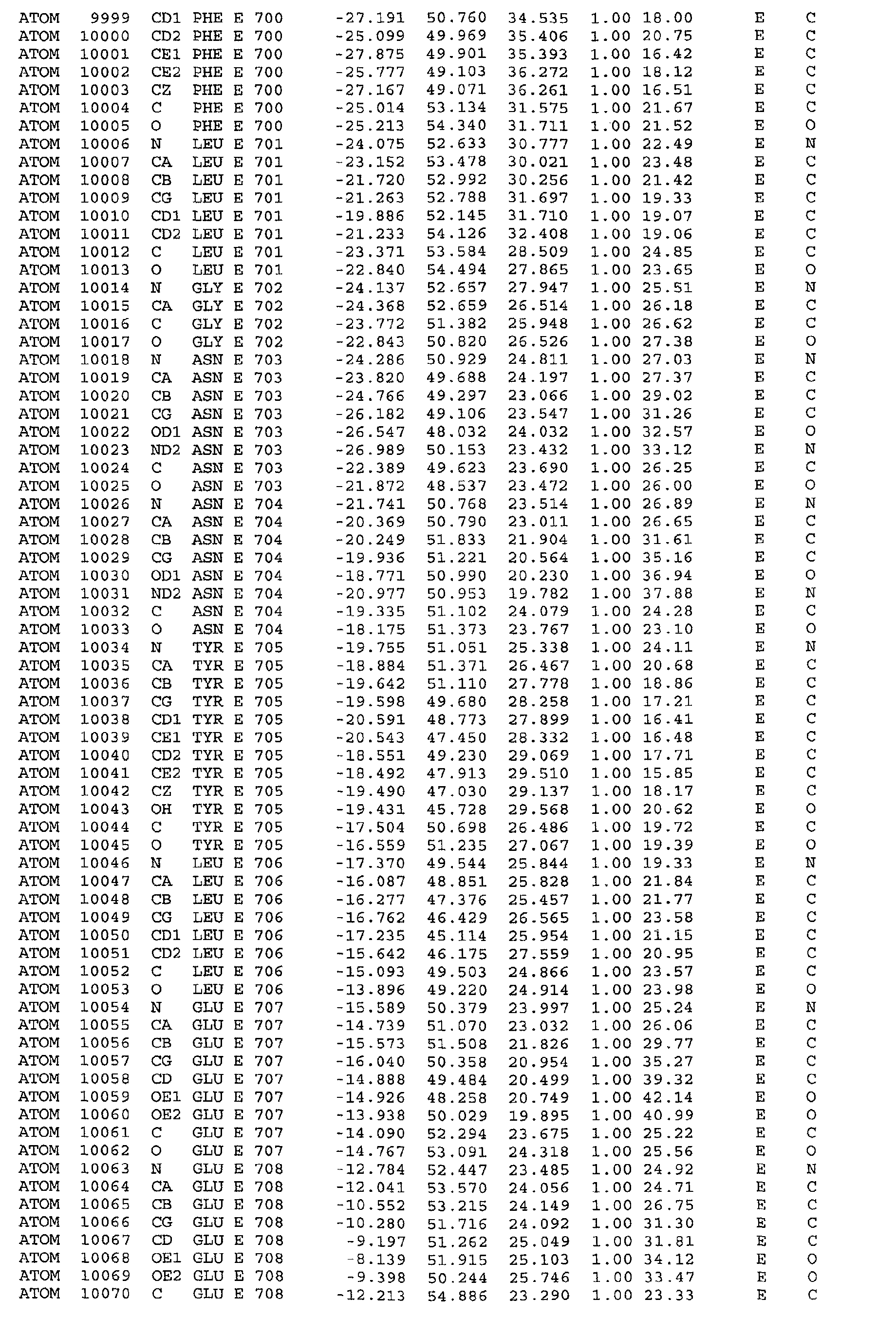

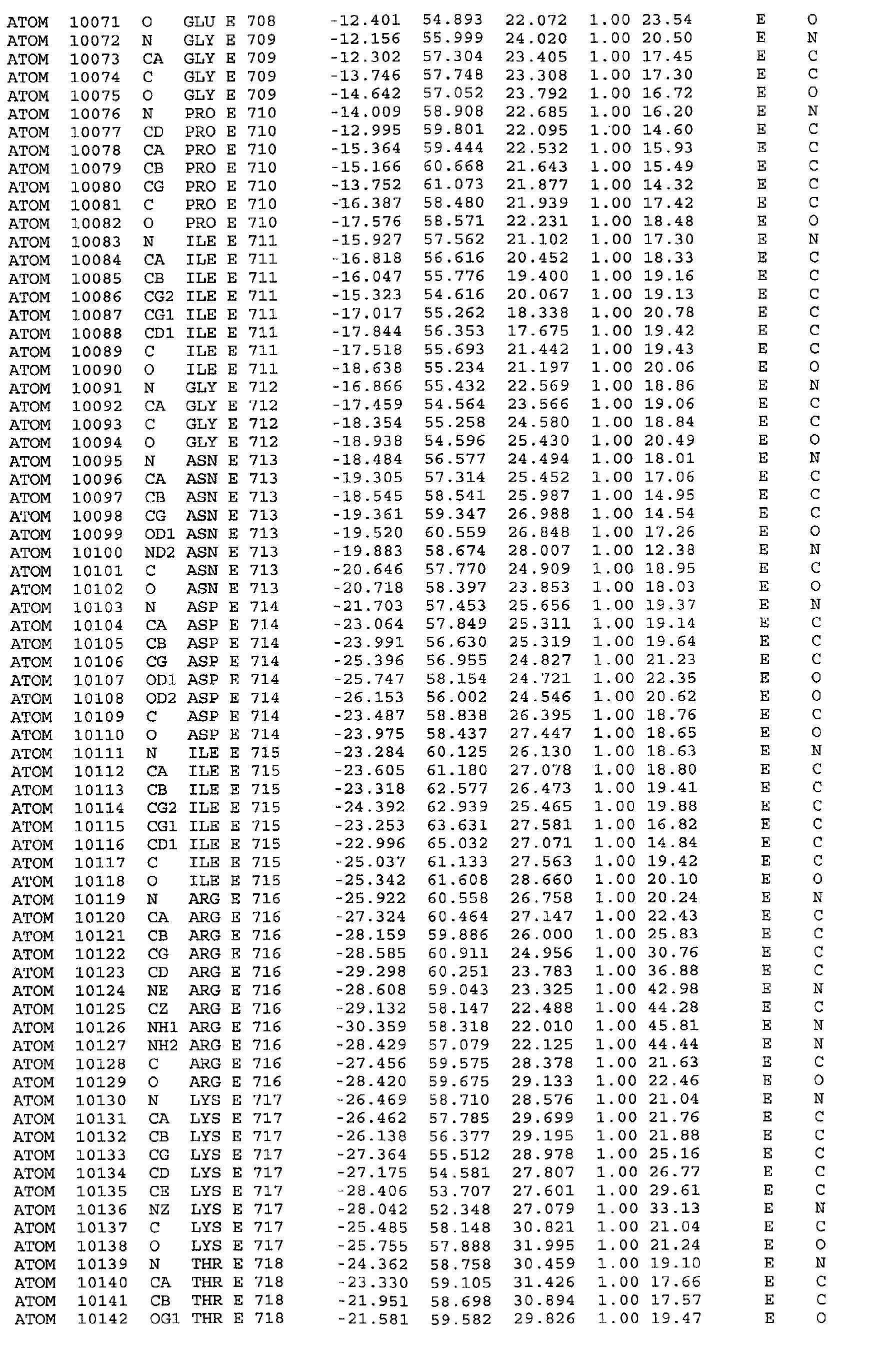

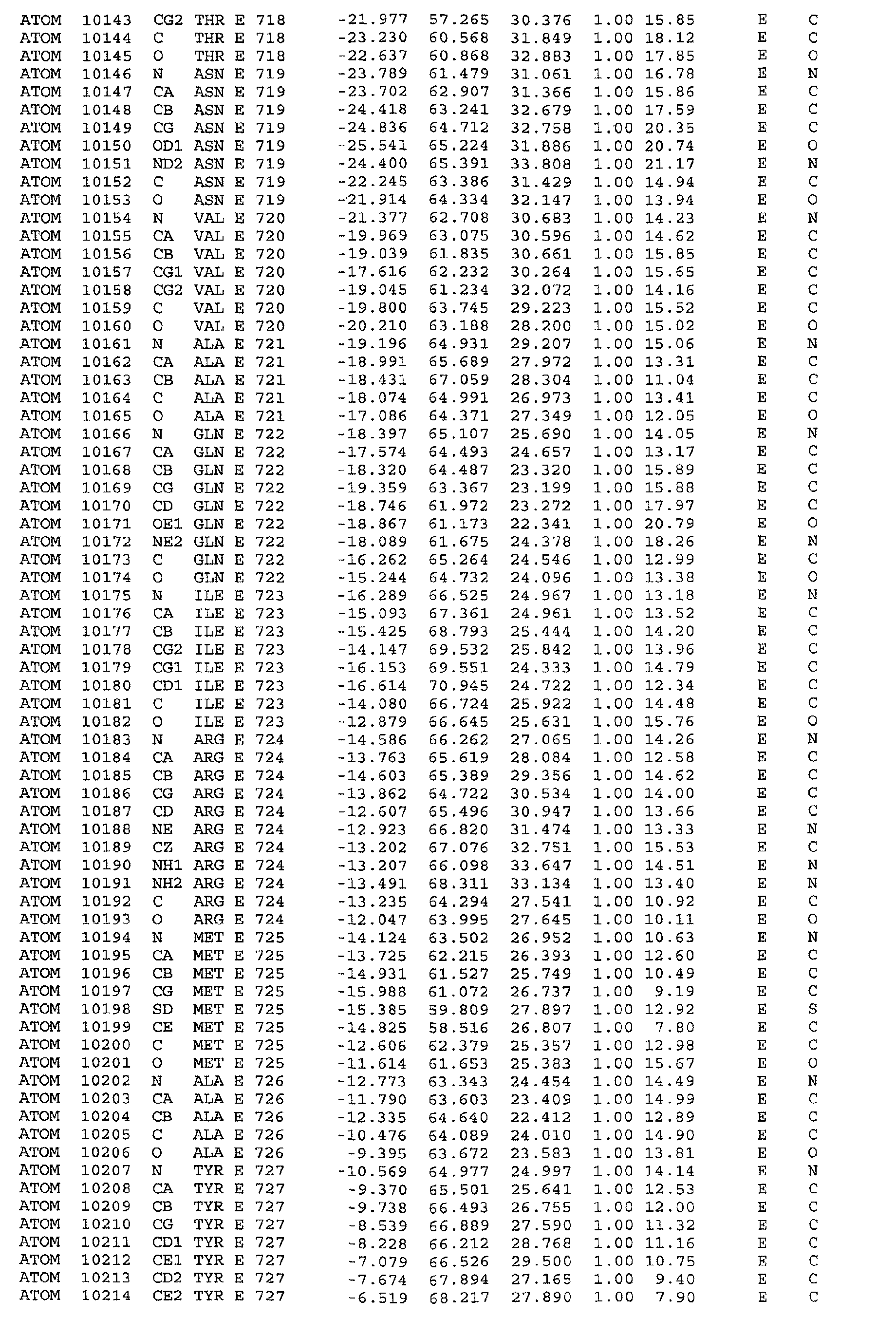

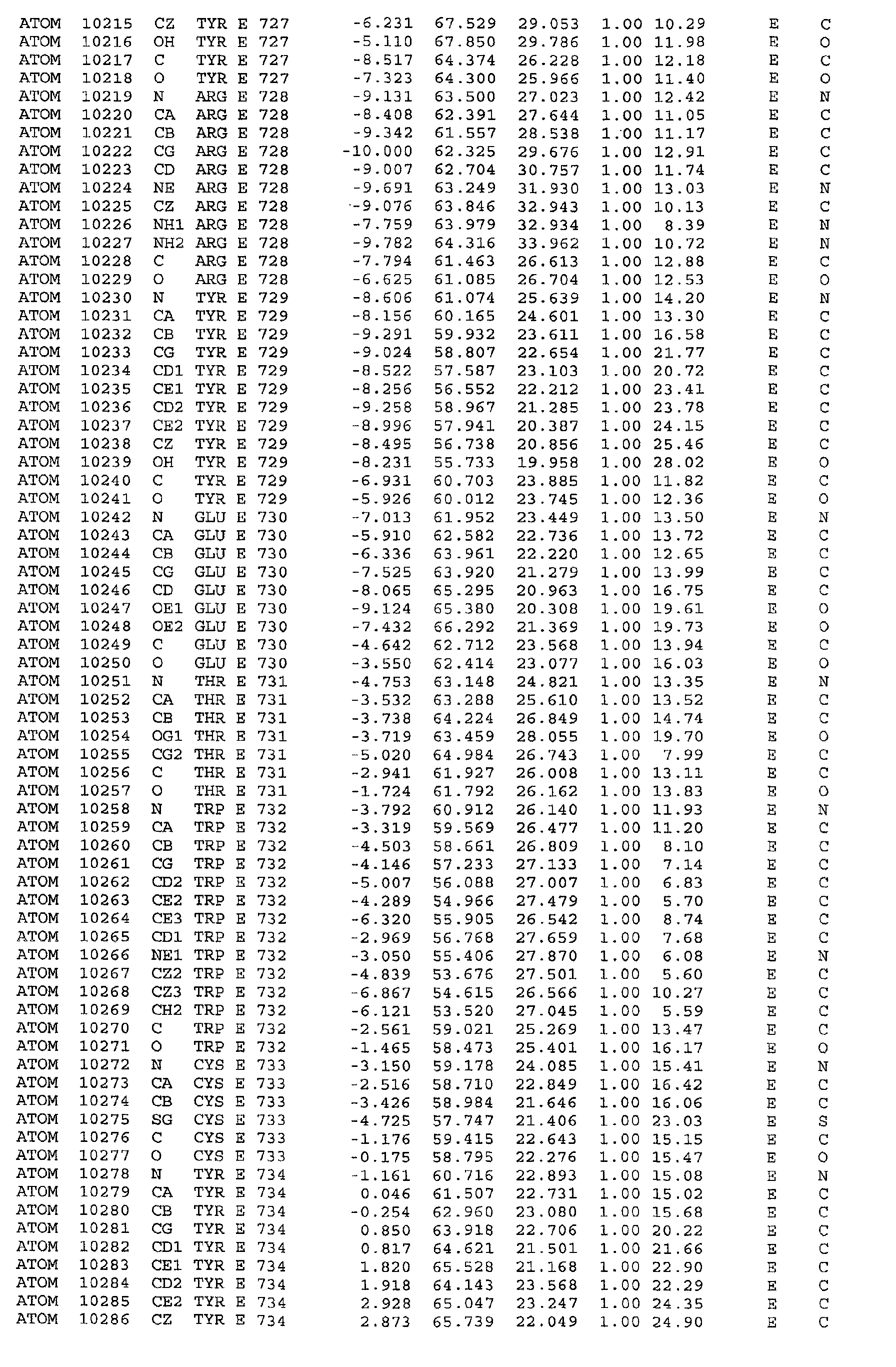

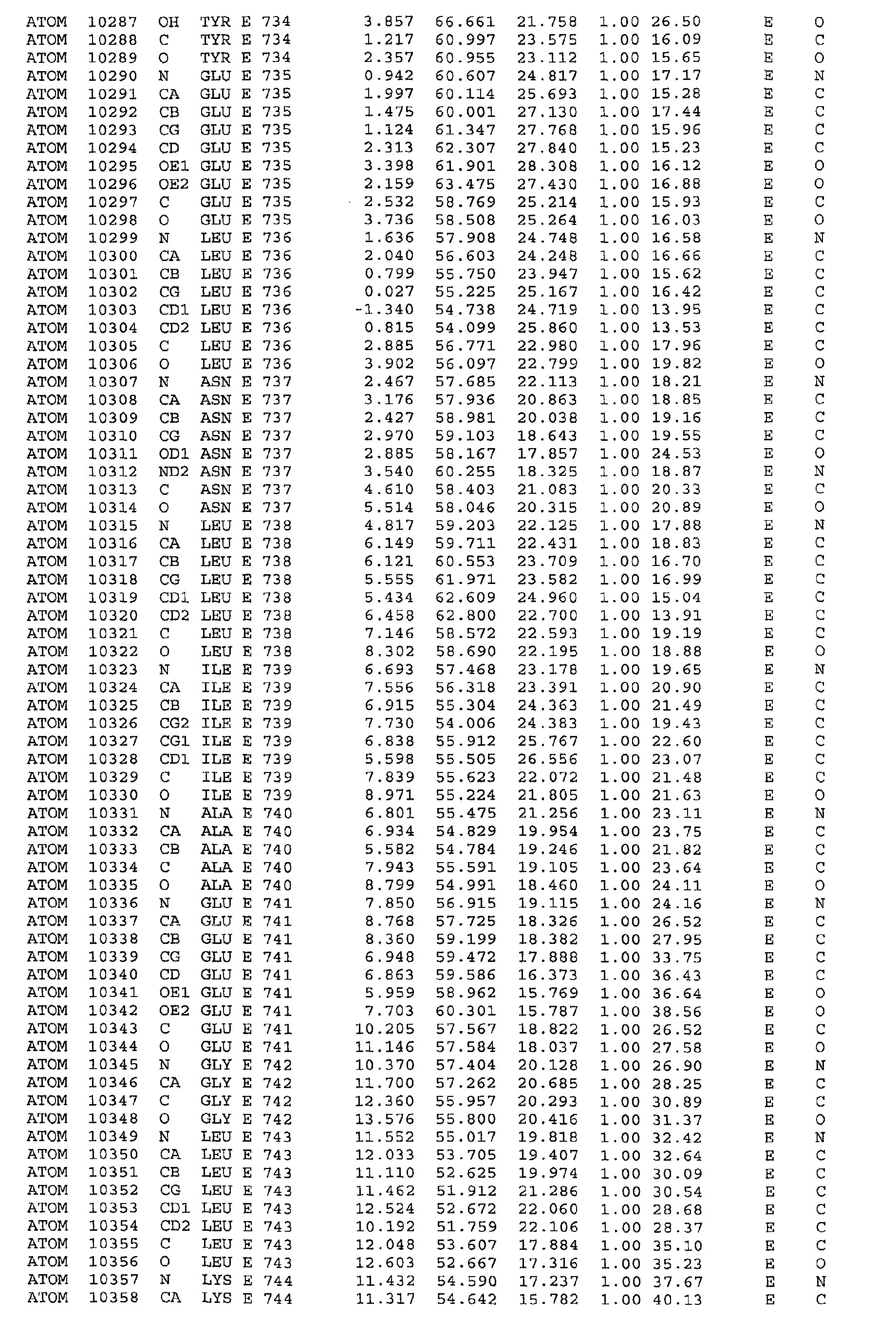

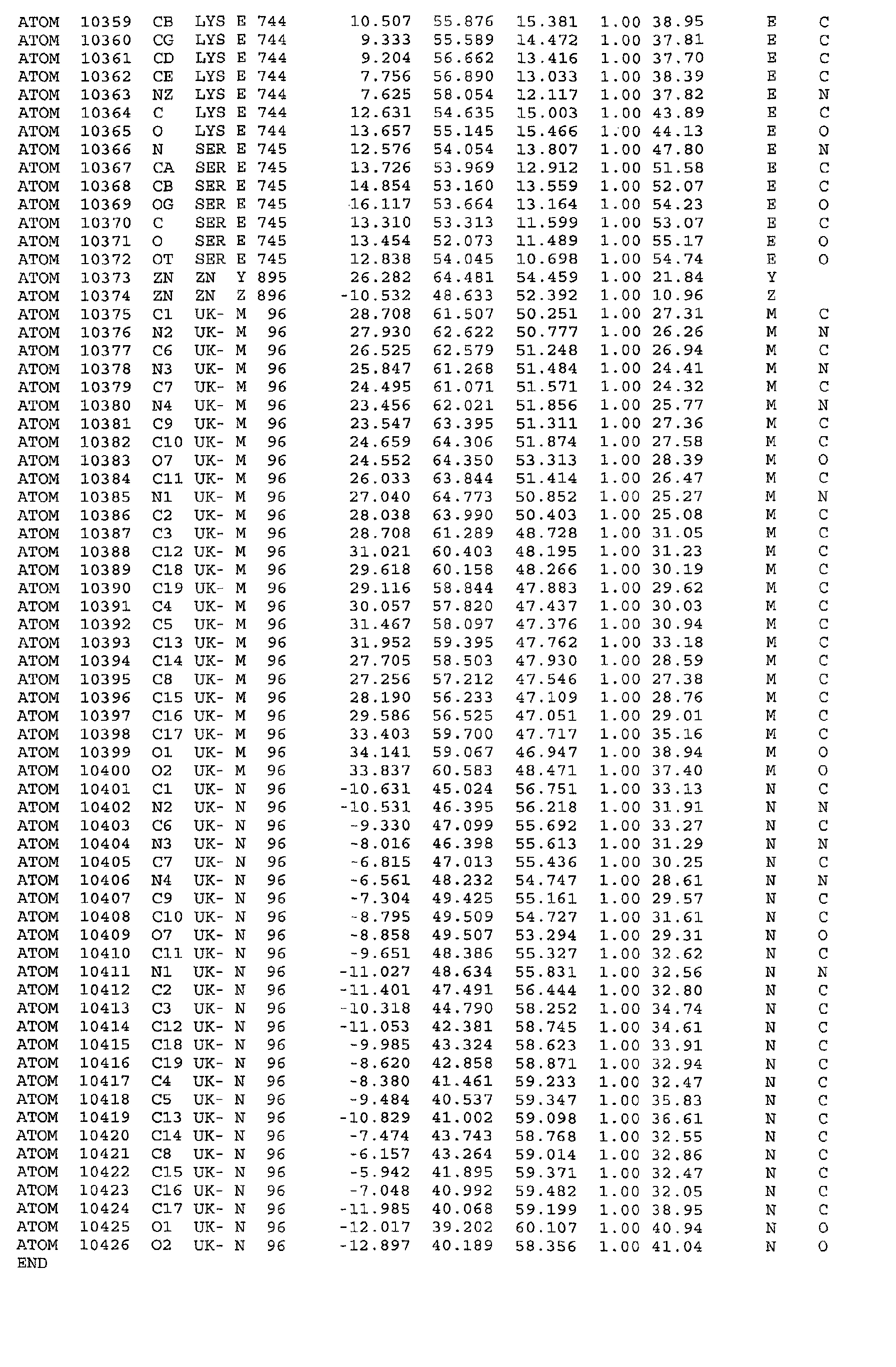

## Claims

1. An AMP deaminase (AMPDA) crystal, wherein said crystal is tetragonal.

2. The crystal as claimed in claim 1, wherein the AMPDA consists of the catalytic domain of AMPDA.

3. The crystal as claimed in claim 1 or claim 2, wherein the AMPDA is from a mammal.

4. The crystal as claimed in any one of claims 1 to 3, wherein the AMPDA is from a rabbit.

5. The crystal as claimed in claim 4, wherein the AMPDA sequence is as shown in SEQ ID NO: 2, but starting at position 96.

6. The crystal as claimed in any one of the preceding claims, which is grown using citric acid as precipitating agent.

7. The crystal as claimed in any one of the preceding claims, which is grown in the pH range of 7.80-8.20.

8. The crystal as claimed in any one of the preceding claims, which is grown in the presence of imidazole.

9. The crystal as claimed in any one of the preceding claims, wherein the crystal has a space group P4₂2₁2.

10. The crystal as claimed in any one of the preceding claims, wherein the crystal has unit cell dimensions of a=b=149Å +/- 3Å, c=159Å +/- 3Å.

11. The crystal as claimed in any one of the preceding claims, wherein the active site of AMPDA is contained in a cleft formed by the additional helices between the first and second strand of the (βα)₈ barrel fold, and the helix immediately following the third strand.

12. The crystal as claimed in any one of the preceding claims, wherein the AMPDA has a pocket which can accommodate the adenosine group of AMP, which pocket is formed by amino acid residues including, but not limited to, residues His305, Phe372, Phe375, Asp513, Glu575, His594, and Asp650.

13. The crystal as claimed in any one of the preceding claims, wherein the AMPDA has a pocket which can accommodate the ribose and phosphate groups of AMP, which pocket is formed by amino acid residues including, but not limited to, residues His305, Ala306, Ala307, Ala308, Phe375, Asn376, Tyr379, Arg388, Lys393, Ser427, Tyr429, Pro460, Ile462, Val512, and Asp513.

14. The crystal as claimed in any one of the preceding claims, which diffracts X-rays to 3.5Å or higher resolution.

15. The crystal as claimed in claim 14, having the atomic coordinates set out in Table 2, or a derivative set as expressed in any reference frame.

16. A heavy atom derivative of the crystal as claimed in any one of the preceding claims.

17. The crystal as claimed in any one of claims 1 to 15, wherein an AMPDA inhibitor has been soaked in.

18. The crystal as claimed in claim 17, wherein the inhibitor is an AMPDA transition state analogue.

19. The crystal as claimed in claim 17, wherein the inhibitor is a coformycin analogue.

20. The crystal as claimed in claim 17, wherein the inhibitor is coformycin or UK-384,858.

21. The crystal as claimed in claim 20, having the atomic coordinates set out in Table 3 or in Table 4, or a derivative set as expressed in any reference frame.

22. A crystal of AMPDA, wherein the primary sequence of the AMPDA has 90% or higher identity at amino acid level to the sequence shown in SEQ ID NO:2.

23. The use of atomic coordinates of a crystal as claimed in any one of the preceding claims for deriving the three-dimensional structure of AMPDA.

24. The use of the three-dimensional structure of AMPDA as derived in claim 23 to computationally or otherwise evaluate the binding interactions of a chemical compound with AMPDA.

25. The use of the three-dimensional structure of AMPDA as derived in claim 23 to design a compound capable of associating with the enzyme.

26. The use according to claim 24 or claim 25, wherein the compound is an inhibitor of AMPDA.

27. A method of selecting an AMPDA inhibitor compound from a group of potential AMPDA inhibitor compounds, comprising the following steps:
a) creating a three-dimensional representation of the structure of AMPDA as derived in claim 23;
b) displaying and superimposing the model of said test compound on the model of the AMPDA structure;
c) assessing whether the test compound model fits the AMPDA structure.

28. A method of selecting an AMPDA inhibitor compound from a group of potential AMPDA inhibitor compounds, comprising the following steps:
a) creating a three-dimensional representation of the active site cavity of AMPDA as derived in claim 23, in a suitable computer program;
b) displaying and superimposing the model of said test compound on the model of said active site;
c) assessing whether the test compound model fits the active site.

29. The method as claimed in claim 27 or claim 28, further comprising the following steps:
d) incorporating the test compound in a biological AMPDA activity assay;
e) determining whether the test compound inhibits AMPDA activity in this assay.

30. A compound selected by the method as claimed in claim 27, claim 28 or claim 29, or designed by the use of the three-dimensional structure as claimed in claim 24 or in claim 25.

31. A pharmaceutical composition comprising the compound of claim 30.

32. Use of a compound as claimed in claim 30 in the manufacture of a medicament for the treatment of diseases, wherein the inhibition of AMPDA is beneficial.

33. Use as claimed in claim 32, wherein the diseases are ischemia-related diseases such as congestive heart failure, peripheral vascular disease, chronic obstructive pulmonary disease, or coronary artery disease, or Alzheimer's disease.

34. The use of the atomic coordinates of AMPDA as defined in claim 15 or in claim 21, or portions thereof, to solve a crystal form of a mutant, homologue or co-complex of AMPDA.

35. An isolated and/or purified polynucleotide comprising one or more of:
a) a polynucleotide encoding the polypeptide as set forth in SEQ ID NO: 2;
b) a polynucleotide comprising a nucleotide sequence of SEQ ID NO: 1;
c) a polynucleotide comprising a nucleotide sequence that has at least 91% identity to the polynucleotide of any one of (a) to (b);
d) a polynucleotide comprising a nucleotide sequence that has at least 95% identity to the polynucleotide of any one of (a) to (c);
e) a polynucleotide comprising a nucleotide sequence which is capable of hybridising to the polynucleotide of any one of (a) to (d) under high stringency conditions;
f) a complement to the polynucleotide of any one of (a) to (e); or
g) a polynucleotide fragment of the polynucleotide of any one of (a) to (f)

36. A polypeptide comprising:
a) a polypeptide having the deduced amino acid sequence translated from the polynucleotide sequence in SEQ ID NO: 1 and variants, fragments, homologues, analogues and derivatives thereof; or
b) a polypeptide of SEQ ID NO: 2 and variants, fragments, homologues, analogues and derivatives thereof.


SEQUENCE LISTING

<110> Pfizer Ltd. (EP(GB) only)
      Pfizer Inc. (US, JP, EP except GB)

<120> Crystal Structure

<130> PCS10934A BXP

<160> 6

<170> FastSEQ for Windows Version 4.0

<210> 1
<211> 2241
<212> DNA
<213> Oryctolagus cuniculus (Rabbit)

<400> 1
atgcctctgt tcaaactccc agctgaagga aaagaactcg atgatgcaat gggcagcttc 60
gctgaaaaag tgtttgcctc agaagtcaaa gatgaggggg gccgccagga gatttcccct 120
tttgatgtgg atgagatctg tccaatttct catcatgaga tgcaagcgca catactccac 180
atggagacgc tggccacctc cccagaaggc acgaggaaaa agcgtttcca aggacggaag 240
actgttaatt tgtccattcc actcagtgaa gcatcttcca ccaaactgtc gcacattgat 300
gaatacatct ctttatctcc aacctaccag acagtccctg attttcagag agtgcagatc 360
acgggagact atgcctctgg ggtcacagtg gaagacttcg aaatagtgtg caaagggctg 420
taccgggcat tgtgtatccg ggagaaatac atgctgaagt cgtttcagag gttccccaaa 480
accccttcca agtacttgcg gagcattgaa ggcacagctt ggaaagcaaa tgagagctcc 540
tatccagtct ttacacctgc tctgaagaag ggagaggacc ctttccgaac agacaacctg 600
cccgaaaacc tgggctatca cctcaaaatg aaagacggtg tggtttacat ctatgctaac 660
gaagcagcag cgggcaaaga tgagcccaag ccacttcttt acccaaatat ggaggagttc 720
ttggatgata tgaatttttt gcttgcttta attgcccaag gacctgttaa gacctatacg 780
caccggcgtc tgaagttcct ctcctccaag ttccaggtgc accagatgct caacgagatg 840
gacgagctga aggagctgaa gaacaaccct caccgcgatt tttacaactg caggaaggtg 900
gacacccaca tccatgcagc tgcctgcatg aaccagaaac atctgctgcg cttcattaag 960
aaatcttacc aagtggatgc cgacagagtg gtctacagca ccaaagagaa gaatctaacc 1020
ctaaagcaac tttttgataa attaaaactg cacccctatg acctgactgt cgactctctg 1080
gatgttcatg ccggccgcca gaccttccag cgttttgata agttcaatga caaatacaat 1140
cctgtaggag caagtgagct gcgggatctc tacctgaaga cagacaatta cattaacggg 1200
gaatattttg ccactatcat caaggaggta ggtgcagact tggtggacgc caagtaccag 1260
catgctgagc ctcgtctgtc catctatggc cgcagccctg atgagtggag caaactttcc 1320
tcctggttcg tccgcaaccg catctacagc tctaacatga catggatgat ccaggtcccc 1380
aggatctatg atgtgtttcg atccaagaat ttccttccac acttcggaaa gatgctggag 1440
aatgtgttca tgccagtgtt tgaggcgacc atcaaccccc aagctcatcc agaactcagt 1500
gtctttctta aacatatcac tggctttgac agtgtggatg atgaaagtaa acacagtggc 1560
catatgtttt cttcaaaaag ccccaaaccc caggagtgga ccttggaaaa aaatccttcc 1620
tatacctact acgcctacta catgtatgca aacatcatgg tgctcaacag cctgagaaag 1680
gaacgaggca tgaatacatt tctgttccga cctcactgcg gggaagttgg ggctctcacc 1740
cacctcatga ccgccttcat gacagcagat aatatctctc atggcctgaa tttaaaaaag 1800
agtcctgtgt tacaatactt gtttttctta gcccagattc ctatcgccat gtcaccatta 1860
agtaacaaca gcctatttct agagtatgcc aaaaatccat ttttagattt tctccagaaa 1920
ggactaatga tctcactgtc taccgatgat ccgatgcagt tccacttcac caaggagccc 1980
ctgatggaag aatacgccat tgcagcacaa gtcttcaagc tgagtacctg tgacatgtgt 2040
gaagtggcga ggaacagtgt tctgcagtgt ggaatttctc atgaggaaaa agcaaagttt 2100
ttgggcaaca attaccttga ggaaggcccc attggaaatg atatccggaa gacgaatgta 2160
gcccaaatcc gcatggccta tcgctatgaa acctggtgtt atgaactcaa tttaattgct 2220
gagggtctta aatcaacaga a 2241

<210> 2
<211> 747
<212> PRT
<213> Oryctolagus cuniculus (Rabbit)

<400> 2

Met Pro Leu Phe Lys Leu Pro Ala Glu Gly Lys Glu Leu Asp Asp Ala
1               5                   10                  15
Met Gly Ser Phe Ala Glu Lys Val Phe Ala Ser Glu Val Lys Asp Glu
            20                  25                  30
Gly Gly Arg Gln Glu Ile Ser Pro Phe Asp Val Asp Glu Ile Cys Pro
        35                  40                  45
Ile Ser His His Glu Met Gln Ala His Ile Leu His Met Glu Thr Leu
    50                  55                  60
Ala Thr Ser Pro Glu Gly Thr Arg Lys Lys Arg Phe Gln Gly Arg Lys
65                  70                  75                  80
Thr Val Asn Leu Ser Ile Pro Leu Ser Glu Ala Ser Ser Thr Lys Leu
                85                  90                  95
Ser His Ile Asp Glu Tyr Ile Ser Leu Ser Pro Thr Tyr Gln Thr Val
            100                 105                 110
Pro Asp Phe Gln Arg Val Gln Ile Thr Gly Asp Tyr Ala Ser Gly Val
        115                 120                 125
Thr Val Glu Asp Phe Glu Ile Val Cys Lys Gly Leu Tyr Arg Ala Leu
    130                 135                 140
Cys Ile Arg Glu Lys Tyr Met Leu Lys Ser Phe Gln Arg Phe Pro Lys
145                 150                 155                 160
Thr Pro Ser Lys Tyr Leu Arg Ser Ile Glu Gly Thr Ala Trp Lys Ala
                165                 170                 175
Asn Glu Ser Ser Tyr Pro Val Phe Thr Pro Ala Leu Lys Lys Gly Glu
            180                 185                 190
Asp Pro Phe Arg Thr Asp Asn Leu Pro Glu Asn Leu Gly Tyr His Leu
        195                 200                 205
Lys Met Lys Asp Gly Val Val Tyr Ile Tyr Ala Asn Glu Ala Ala Ala
    210                 215                 220
Gly Lys Asp Glu Pro Lys Pro Leu Leu Tyr Pro Asn Met Glu Glu Phe
225                 230                 235                 240
Leu Asp Asp Met Asn Phe Leu Leu Ala Leu Ile Ala Gln Gly Pro Val
                245                 250                 255
Lys Thr Tyr Thr His Arg Arg Leu Lys Phe Leu Ser Ser Lys Phe Gln
            260                 265                 270
Val His Gln Met Leu Asn Glu Met Asp Glu Leu Lys Glu Leu Lys Asn
        275                 280                 285
Asn Pro His Arg Asp Phe Tyr Asn Cys Arg Lys Val Asp Thr His Ile
    290                 295                 300
His Ala Ala Ala Cys Met Asn Gln Lys His Leu Leu Arg Phe Ile Lys
305                 310                 315                 320
Lys Ser Tyr Gln Val Asp Ala Asp Arg Val Val Tyr Ser Thr Lys Glu
                325                 330                 335
Lys Asn Leu Thr Leu Lys Gln Leu Phe Asp Lys Leu Lys Leu His Pro
            340                 345                 350
Tyr Asp Leu Thr Val Asp Ser Leu Asp Val His Ala Gly Arg Gln Thr
        355                 360                 365
Phe Gln Arg Phe Asp Lys Phe Asn Asp Lys Tyr Asn Pro Val Gly Ala
    370                 375                 380
Ser Glu Leu Arg Asp Leu Tyr Leu Lys Thr Asp Asn Tyr Ile Asn Gly
385                 390                 395                 400
Glu Tyr Phe Ala Thr Ile Ile Lys Glu Val Gly Ala Asp Leu Val Asp
                405                 410                 415
Ala Lys Tyr Gln His Ala Glu Pro Arg Leu Ser Ile Tyr Gly Arg Ser
            420                 425                 430
Pro Asp Glu Trp Ser Lys Leu Ser Ser Trp Phe Val Arg Asn Arg Ile
        435                 440                 445
Tyr Ser Ser Asn Met Thr Trp Met Ile Gln Val Pro Arg Ile Tyr Asp
    450                 455                 460
Val Phe Arg Ser Lys Asn Phe Leu Pro His Phe Gly Lys Met Leu Glu
465                 470                 475                 480
Asn Val Phe Met Pro Val Phe Glu Ala Thr Ile Asn Pro Gln Ala His
                485                 490                 495
Pro Glu Leu Ser Val Phe Leu Lys His Ile Thr Gly Phe Asp Ser Val
            500                 505                 510
Asp Asp Glu Ser Lys His Ser Gly His Met Phe Ser Ser Lys Ser Pro
        515                 520                 525

Lys Pro Gln Glu Trp Thr Leu Glu Lys Asn Pro Ser Tyr Thr Tyr Tyr
    530                 535                 540
Ala Tyr Tyr Met Tyr Ala Asn Ile Met Val Leu Asn Ser Leu Arg Lys
545                 550                 555                 560
Glu Arg Gly Met Asn Thr Phe Leu Phe Arg Pro His Cys Gly Glu Val
                565                 570                 575
Gly Ala Leu Thr His Leu Met Thr Ala Phe Met Thr Ala Asp Asn Ile
            580                 585                 590
Ser His Gly Leu Asn Leu Lys Lys Ser Pro Val Leu Gln Tyr Leu Phe
        595                 600                 605
Phe Leu Ala Gln Ile Pro Ile Ala Met Ser Pro Leu Ser Asn Asn Ser
    610                 615                 620
Leu Phe Leu Glu Tyr Ala Lys Asn Pro Phe Leu Asp Phe Leu Gln Lys
625                 630                 635                 640
Gly Leu Met Ile Ser Leu Ser Thr Asp Asp Pro Met Gln Phe His Phe
                645                 650                 655
Thr Lys Glu Pro Leu Met Glu Glu Tyr Ala Ile Ala Ala Gln Val Phe
            660                 665                 670
Lys Leu Ser Thr Cys Asp Met Cys Glu Val Ala Arg Asn Ser Val Leu
        675                 680                 685
Gln Cys Gly Ile Ser His Glu Glu Lys Ala Lys Phe Leu Gly Asn Asn
    690                 695                 700
Tyr Leu Glu Glu Gly Pro Ile Gly Asn Asp Ile Arg Lys Thr Asn Val
705                 710                 715                 720
Ala Gln Ile Arg Met Ala Tyr Arg Tyr Glu Thr Trp Cys Tyr Glu Leu
                725                 730                 735
Asn Leu Ile Ala Glu Gly Leu Lys Ser Thr Glu
            740                 745

<210> 3
<211> 20
<212> DNA
<213> Homo sapiens

<400> 3
atgcctctgt tcaaactccc                                                20

<210> 4
<211> 21
<212> DNA
<213> Homo sapiens

<400> 4
ttctgttgat ttaagaccct c                                              21

<210> 5
<211> 21
<212> DNA
<213> Homo sapiens

<400> 5
atgaaccaga aacatctgct g                                              21

<210> 6
<211> 22
<212> DNA
<213> Homo sapiens

<400> 6
cagcagatgt ttctggttca tg                                             22